# EUROPÄISCHE PATENTSCHRIFT

(11) **EP 1 791 840 B1**
(45) Veröffentlichungstag und Bekanntmachung des Hinweises auf die Patenterteilung: **27.11.2013**
(21) Anmeldenummer: 05784773.3
(22) Anmeldetag: 08.09.2005
(51) Int. Cl.: C07D 471/04, A61K 31/4188, A61P 25/00

(54) **SUBSTITUIERTE BIZYKLISCHE IMIDAZO-3-YL-AMIN-VERBINDUNGEN**
SUBSTITUTED BICYCLIC IMIDAZO-3-YLAMINE COMPOUNDS
COMPOSÉS IMIDAZO-3-YL-AMINE BICYCLIQUES SUBSTITUÉS

(30) Priorität: 14.09.2004 DE 102004044884
(43) Veröffentlichungstag der Anmeldung: 06.06.2007
(73) Patentinhaber: Grünenthal GmbH, 52078 Aachen (DE)
(72) Erfinder: KÜHNERT, Sven, 52355 Düren (DE); OBERBÖRSCH, Stefan, 52078 Aachen (DE); SUNDERMANN, Corinna, 61381 Friedrichsdorf (DE); HAURAND, Michael, 52078 Aachen (DE); JOSTOCK, Ruth, 52223 Stolberg (DE); SCHIENE, Klaus, 41363 Jüchen (DE); TZSCHENTKE, Thomas, 52078 Aachen (DE); CHRISTOPH, Thomas, 52080 Aachen (DE); KAULARTZ, Dagmar, 52222 Stolberg (DE); ZEMOLKA, Saskia, 52066 Aachen (DE)
(74) Vertreter: Brosch, Oliver
(86) Internationale Anmeldenummer: PCT/EP2005/054436
(87) Internationale Veröffentlichungsnummer: WO 2006/029980

(56) Entgegenhaltungen:
- WO-A-02/30428
- WO-A-02/080911
- DE-A1- 19 948 438

## Beschreibung

Die vorliegende Erfindung betrifft substituierte bizyklische Imidazo-3-yl-amin-Verbindungen, Verfahren zu ihrer Herstellung, Arzneimittel enthaltend diese Verbindungen sowie deren Verwendung zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung, Obstipation oder Toleranzentwicklung. Des Weiteren sind sie bei neuropathischen Schmerzen, unter denen insbesondere Tumorpatienten leiden, häufig nicht ausreichend wirksam.

Die WO 02/080911, DE 19948438 und WO 02/130428 offenbaren bizylische Imidazo-3-yl-amin-Verbindungen.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Behandlung von Schmerzen.

Es wurde nun überraschenderweise gefunden, dass sich die substituierten bizyklischen Imidazo-3-yl-amin-Verbindungen der nachstehend angegebenen allgemeinen Formel I zur mGluR5-Rezeptor-Regulation (mGluR5 = metabotroper Glutamatrezeptor 5) eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Ein Gegenstand der vorliegenden Erfindung sind daher substituierte Imidazo-3-yl-amin-Verbindungen der allgemeinen Formel I, 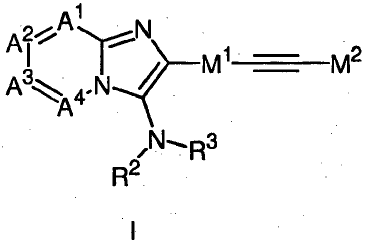 worin
A¹ für ein Stickstoffatom oder für eine G-R^{1a}-Gruppe steht,
A² für ein Stickstoffatom oder für eine C-R^{1b}-Gruppe steht,
A³ für ein Stickstoffatom oder für eine C-R^{1c}-Gruppe steht,
A⁴ für ein Stickstoffatom oder für eine C-R^{1d}-Gruppe steht,

R^{1a}, R^{1b}, R^{1c}, R^{1d}, unabhängig voneinander, jeweils für einen Wasserstoff-Rest; für einen Halogen-Rest; -NO₂; -CN; -NH₂; -NHR⁴; -NR⁵R⁶; -NH-C(=O)-R⁷; -C(=O)-R⁸, -C(=O)-NH₂; -C(=O)-NHR⁹; -C(=O)-NR¹⁰R¹¹; -C(=O)-OR¹²; -(CH₂)ₘ-C(=O)-OR¹³ mit m = 1, 2, 3, 4 oder 5; O-C(=O)-R¹⁴; -(CH₂)ₙ-O-C(=O)-R¹⁵ mit n = 1, 2, 3, 4 oder 5; -OR¹⁶; -(CH₂)ₒ-OR¹⁷ mit o = 1, 2, 3; 4 oder 5; -SR¹⁸; -(CH₂)ₚ-S(=O)ₜ-R¹⁹ mit p = 1, 2, 3, 4 oder 5 und t = 0, 1 oder 2; -NH-S(=O)₂-R²⁶R²⁷; -S(=O)₂-NR²⁸R²⁹, -SF₅; für einen linearen oder verzweigten, gesättigten oder ungesättigten, unsubstituierten oder wenigstens einfach substituierten aliphatischen Rest; für einen gesättigten oder ungesättigten, unsubstituierten oder wenigstens einfach substituierten, ggf. wenigstens ein Heteroatom als Ringglied aufweisenden cycloaliphatischen Rest, der über eine lineare oder verzweigte Alkylen-Gruppe gebunden und/oder mit einem unsubstituierten oder wenigstens einfach substituierten mono- oder polyzyklischen Ringsystem kondensiert sein kann; oder für einen unsubstituierten oder wenigstens einfach substituierten Aryl- oder Heteroaryl-Rest, der über eine lineare oder verzweigte Alkylen-Gruppe gebunden und/oder mit einem unsubstituierten oder wenigstens einfach substituierten mono- oder polyzyklischen Ringsystem kondensiert sein kann, steht,
oder ggf. R^{1a} und R^{1b} mit der sie verbindenden C-C-Brücke einen unsubstituierten oder wenigstens einfach substituierten annelierten Phenyl-Rest bilden,
oder ggf. R^{1b} und R^{1c} mit der sie verbindenden C-C-Brücke einen unsubstituierten oder wenigstens einfach substituierten annelierten Phenyl-Rest bilden,
oder ggf. R^{1c} und R^{1d} mit der sie verbindenden C-C-Brücke einen unsubstituierten oder wenigstens einfach substituierten annelierten Phenyl-Rest bilden,
R² und R³, unabhängig voneinander, jeweils für einen Wasserstoff-Rest; -C(=O)-R²⁰; -(CH₂)_{q}-C(=O)-R²¹ mit q = 1, 2, 3, 4 oder 5; -C(=O) O-R²²; -(CH₂)ᵣ-C(=O)-O-R²³ mit r = 1, 2, 3, 4 oder 5; -C(=O)-NHR²⁴; -(CH₂)ₛ-C(=O)-NHR²⁵ mit s = 1, 2, 3, 4 oder 5; für einen linearen oder verzweigten, gesättigten oder ungesättigten, unsubstituierten oder wenigstens einfach substituierten aliphatischen Rest; für einen gesättigten oder ungesättigten, unsubstituierten oder wenigstens einfach substituierten, ggf. wenigstens ein Heteroatom als Ringglied aufweisenden cycloaliphatischen Rest, der über eine lineare oder verzweigte Alkylen-Gruppe gebunden und/oder mit einem unsubstituierten oder wenigstens einfach substituierten mono- oder polyzyklischen Ringsystem kondensiert sein kann; oder für einen unsubstituierten oder wenigstens einfach substituierten Aryl- oder Heteroaryl-Rest, der über eine lineare oder verzweigte Alkylen-Gruppe gebunden und/oder mit einem unsubstituierten oder wenigstens einfach substituierten mono- oder polyzyklischen Ringsystem kondensiert sein kann, stehen,
oder R² und R³ zusammen mit dem sie verbindenden Stickstoffatom als Ringglied einen gesättigten oder ungesättigten, ggf. wenigstens ein weiteres Heteroatom als Ringglied aufweisenden heterocycloaliphatischen Rest bilden, der mit einem unsubstituierten oder wenigstens einfach substituierten mono- oder polyzyklischen Ringsystem kondensiert sein kann,
R⁴, R⁵, R⁶, R⁷, R⁹, R¹⁰, R¹¹, R¹⁴ und R¹⁵, jeweils unabhängig voneinander, für einen linearen oder verzweigten, gesättigten oder ungesättigten aliphatischen Rest oder für einen unsubstituierten oder wenigstens einfach substituierten Aryl- oder Heteroaryl-Rest, der über eine lineare oder verzweigte Alkylen-Gruppe gebunden und/oder mit einem unsubstituierten oder wenigstens einfach substituierten mono- oder polyzyklischen Ringsystem kondensiert sein kann, stehen,
R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ und R²⁹, jeweils unabhängig voneinander, für einen Wasserstoff-Rest; für einen linearen oder verzweigten, gesättigten oder ungesättigten aliphatischen Rest oder für einen unsubstituierten oder wenigstens einfach substituierten Aryl- oder Heteroaryl-Rest, der über eine lineare oder verzweigte Alkylen-Gruppe gebunden und/oder mit einem unsubstituierten oder wenigstens einfach substituierten mono- oder polyzyklischen Ringsystem kondensiert sein kann, stehen,
M¹ für einen Aryl- oder Heteroaryl-Rest steht, der mit wenigstens einem weiteren Substituenten substituiert und/oder mit einem unsubstituierten oder wenigstens einfach substituierten mono- oder polyzyklischen Ringsystem kondensiert sein kann,
M² für einen Aryl- oder Heteroaryl-Rest steht, der unsubstituiert oder wenigstens einfach substituiert und ggf. mit einem unsubstituierten oder wenigstens einfach substituierten mono- oder polyzyklischen Ringsystem kondensiert ist,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Sofern einer oder mehrere der Substituenten R^{1a}, R^{1b}, R^{1c}, R^{1d} und R² bis R²⁹ für einen gesättigten oder ungesättigten aliphatischen Rest, d.h. für einen Alkyl-, Alkenyl- oder Alkinyl-Rest, stehen, der einfach oder mehrfach substituiert ist, kann dieser mit 1, 2, 3, 4 oder 5 Substituenten unabhängig voneinander ausgewählt aus der Gruppe bestehend aus F, Cl, Br, -NO₂, -CN, -OH, -SH und -NH₂ substituiert sein. Alkenyl-Reste weisen wenigstens eine, vorzugsweise 1, 2, 3 oder 4 C-C-Doppelbindungen und Alkinyl-Reste wenigstens eine, vorzugsweise 1, 2, 3 oder 4 C-C-Dreifachbindungen auf.

Als geeignete Alkyl-, Alkenyl- und Alkinyl-Reste, die einfach oder mehrfach substituiert sein können, seien beispielsweise Methyl, Ethyl, n-Propyl, iso-Propyl, n-Butyl, iso-Butyl, sec-Butyl, tert-Butyl, n-Pentyl, 2-Pentyl, 3-Pentyl, iso-Pentyl, neo-Pentyl, n-Hexyl, 2-Hexyl, 3-Hexyl, n-Heptyl, n-Octyl, n-Nonyl, n-Decyl, -C(H)(n-C₃H₇)₂, -CH₂-CH₂-C(H)(CH₃)-(CH₂)₃-CH₃, (1,1,3,3-Tetramethyl)-butyl, (1,1)-Dimethylpentyl, (1,1)-Dimethyl-butyl, Vinyl, Ethinyl, 1-Propenyl, 2-Propenyl, 1-Propinyl, 2-Propinyl, 1-Butenyl, 2-Butenyl, 3-Butenyl, 1-Butinyl, 2-Butinyl, 3-Butinyl, 1-Pentenyl, 2-Pentenyl, 3-Pentenyl, 4-Pentenyl, 1-Pentinyl, 2-Pentinyl, 3-Pentinyl, 4-Pentinyl, Hexenyl, Hexinyl, -CH=CH-CH=CH-CH₃ und -CH₂-CH₂-CH=CH₂ genannt.

Als geeignete substituierte Alkyl- und Alkenyl-Reste seien beispielsweise Trifluormethyl, Difluormethyl, Monofluormethyl, -(CH₂)-OH, -(CH₂)-NH₂, -(CH₂)-CN, - (CH₂)-(CF₃), -(CH₂)-(CHF₂), -(CH₂)-(CH₂F), -(CH₂)-(CH₂)-OH, -(CH₂)-(CH₂)-NH₂, - (CH₂)-(CH₂)-CN, -(CF₂)-(CF₃), -(CH₂)-(CH₂)-(CF₃), -CH=CH-(CH₂)-OH, -CH=CH-(CH₂)-NH₂, -CH=CH-CN und -(CH₂)-(CH₂)-(CH₂)-OH genannt.

Sofern einer oder mehrere der Substituenten R^{1a}, R^{1b}, R^{1c}, R^{1d}, R² und R³ für einen cycloaliphatischen Rest stehen oder einen cycloaliphatischen Rest aufweisen, der einfach oder mehrfach substituiert ist, kann dieser ggf. mit 1, 2, 3, 4 oder 5 Substituenten unabhängig voneinander ausgewählt aus der Gruppe bestehend aus F, Cl, Br, I, -CN, -CF₃, -SF₅, -OH, -NH₂, -O-CF₃, -SH, -O-C₁₋₅-Alkyl, -(CH₂)-O-C₁₋₅-Alkyl, -S-C₁₋₅-Alkyl, -C₁₋₅-Alkyl, -C₂₋₅-Alkenyl, -C₂₋₅-Alkinyl, -C(=O)-O-C₁₋₅-Alkyl, - C(=O)-CF₃, -S(=O)₂-C₁₋₅-Alkyl, -S(=O)-C₁₋₅-Alkyl, -S(=O)₂-Phenyl, Oxo (=O), Thioxo (=S), -N(C₁₋₅-Alkyl)₂, -N(H)(C₁₋₅-Alkyl), -NO₂, -S-CF₃, -C(=O)-OH, -NH-S(=O)₂-C₁₋₅-Alkyl, -NH-C(=O)-C₁₋₅-Alkyl, -C(=O)-H; -C(=O)-C₁₋₅-Alkyl, -C(=O)-NH₂, -C(=O)-N(C₁₋₅-Alkyl)₂, -C(=O)-N(H)(C₁₋₅-Alkyl) und Phenyl substituiert sein, wobei die vorstehend genannten C₁₋₅-Alkyl-Reste jeweils linear oder verzweigt sein können und die Phenyl-Reste jeweils unsubstituiert oder mit 1, 2, 3 oder 4 Substituenten unabhängig voneinander ausgewählt aus der Gruppe bestehend aus F, Cl, Br, I, Methyl, Ethyl, Methoxy und Ethoxy substituiert sein können.

Besonders bevorzugt können die Substituenten, jeweils unabhängig voneinander, ausgewählt werden aus der Gruppe bestehend aus F, Cl, Br, I, -CN, Methyl, Ethyl, n-Propyl, iso-Propyl, n-Butyl, sec-Butyl, iso-Butyl, tert-Butyl, Ethenyl, Propenyl, Ethinyl, Propinyl, -OH, Oxo, Thioxo, -O-CH₃, -O-C₂H₅, -O-C₃H₇, -(CH₂)-O-CH₃, -(CH₂)-O-C₂H₅, -NH₂, -N(CH₃)₂, -N(C₂H₅)₂, -NH-CH₃, -NH-C₂H₅, -NO₂, -CF₃, -SF₅, -O-CF₃, -S-CF₃, -SH, -S-CH₃, -S-C₂H₅, -S(=O)-CH₃, -S(=O)-C₂H₅, -S(=O)₂-CH₃, -S(=O)₂-C₂H₅, - NH-S(=O)₂-CH₃, -C(=O)-OH, -C(=O)-H, -C(=O)-CH₃, -C(=O)-C₂H₅, -C(=O)-N(CH₃)₂, - C(=O)-NH-CH₃, -C(=O)-NH₂, -NH-C(=O)-CH₃, -NH-C(=O)-C₂H₅, -C(=O)-O-CH₃, - C(=O)-O-C₂H₅, -C(=O)-O-C(CH₃)₃ und Phenyl, wobei der Phenyl-Rest mit 1, 2 oder 3 Substituenten unabhängig voneinander ausgewählt aus der Gruppe bestehend aus F, Cl, Br, Methyl und Methoxy substituiert sein kann.

Sofern die cycloaliphatischen Reste eines oder mehrere Heteroatome als Ringglieder aufweisen, können diese 1, 2, 3, 4 oder 5, besonders bevorzugt 1, 2 oder 3, Heteroatom(e) als Ringglied(er) aufweisen, die jeweils unabhängig voneinander, ausgewählt werden können aus der Gruppe bestehend aus Stickstoff, Sauerstoff und Schwefel.

Beispielhaft für cycloaliphatische Rest, die einfach oder mehrfach substituiert sein können, seien Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cycloheptyl, Cyclopentenyl, Cyclohexenyl, Cycloheptenyl, Cyclooctenyl, Oxiranyl, Aziridinyl, Tetrahydrofuranyl, Tetrahydrothienyl, Pyrrolidinyl, Isoxazolidinyl, Isothioazolidinyl, Pyrazolidinyl, Oxazolidinyl, Thiazolidinyl, Imidazolidinyl, (1,2,4)-Oxadiazolidinyl, (1,2,4)-Thiadiazolidinyl, (1,2,4)-Triazolidin-3-yl, (1,3,4)-Thiadiazolidinyl, (1,3,4)-Triazolidin-1-yl, (1,3,4)-Triazolidin-2-yl, (2,3)-Dihydrofuryl, (2,5)-Dihydrofuryl, (2,3)-Dihydrothienyl, (2,5)-Dihydrothienyl, (2,3)-Dihydropyrrolyl, (2,5)-Dihydropyrrolyl, (2,3)-Dihydroisoxazolyl, (4,5)-Dihydroisoxazolyl, (2,5)-Dihydroisothiazolyl, (2,3)-Dihydropyrazolyl, (4,5)-Dihydropyrazolyl, (2,5)-Dihydropyrazolyl, (2,3)-Dihydrooxazolyl, (4,5)-Dihydrooxazolyl, (2,5)-Dihydrooxazolyl, (2,3)-Dihydrothiazolyl, (4,5)-Dihydrothiazolyl, (2,5)-Dihydrothiazolyl, (2,3)-Dihydroimidazolyl, (4,5)-Dihydroimidazolyl, (2,5)-Dihydroimidazolyl, Morpholinyl, Piperidinyl, Piperazinyl, Azocanyl, Tetrahydropyridazinyl, Tetrahydropyrimidinyl, Tetrahydropyrazinyl, (1,3,5)-Tetrahydrotriazinyl, (1,2,4)-Tetrahydrotriazin-1-yl, (1,2,4)-Tetrahydrotriazin-3-yl, (1,3)-Dihydrooxazinyl, (1,3)-Dithian-2-yl, Tetrahydropyranyl, (1,3)-Dioxolan-2-yl, (3,4,5,6)-Tetrahydropyridin-2-yl, (1,2,5,6)-Tetrahydropyridin-1-yl, (1,2,3,4)-Tetrahydropyridin-1-yl, (1,2)-Dihydropyridin-1-yl, (1,4)-Dihydropyridin-1-yl, 4H-1,3-Thiazinyl, (1,3)-Dihydrooxazin-2-yl, Azepanyl, (1,4)-Diazepanyl, Thiomorpholinyl und Dithiolanyl genannt.

Besonders bevorzugt seine als cycloaliphatische Reste, die einfach oder mehrfach substituiert sein können, Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Imidazolidinyl, Tetrahydrofuranyl (Tetrahydrofuryl), Piperidinyl, Piperazinyl, Morpholinyl, Pyrrolidinyl, Tetrahydrothiophenyl, Tetrahydropyranyl, Thiomorpholinyl, Dioxolanyl, Azepanyl, Diazepanyl, Azocanyl und Dithiolanyl genannt.

Sofern der cycloaliphatische Rest mit einem unsubstituierten oder wenigstens einfach substituierten mono- oder polyzyklischen Ringsystem kondensiert ist, können geeignete unsubstituierte oder wenigstens einfach substituierte Reste ausgewählt werden aus der Gruppe bestehend aus 2,3-Dihydro-benzo[1,4]dioxinyl; 3,4-Dihydro-2H-benzo[1,4]oxazinyl; Benzo[1,3]dioxolyl; (1,2,3,4)-Tetrahydrochinazolinyl; Indanyl; (1,2,3,4)-Tetrahydronaphthyl; 1 H-Indenyl; (1,2,3,4)-Tetrahydrochinolinyl; (1,2,3,4)-Tetrahydroisochinolinyl; (2,3)-Dihydro-1H-indolyl, (2,3)-Dihydro-1H-isoindolyl und Decahydroisochinolinyl.

Sofern die Substituenten R² und R³ zusammen mit dem sie verbindenden Stickstoffatom als Ringglied einen gesättigten oder ungesättigten heterocycloaliphatischen Rest bilden, der einfach oder mehrfach substituiert ist, kann dieser ggf. mit 1, 2, 3, 4 oder 5 Substituenten unabhängig voneinander ausgewählt aus der Gruppe bestehend aus F, Cl, Br, I, -CN, -CF₃, -SF₅, -OH, -NH₂, - O-CF₃, -SH, -O-C₁₋₅-Alkyl, -(CH₂)-O-C₁₋₅-Alkyl, -S-C₁₋₅-Alkyl, -C₁₋₅-Alkyl, -C₂₋₅-Alkenyl, -C₂₋₅-Alkinyl, -C(=O)-O-C₁₋₅-Alkyl, -C(=O)-CF₃, -S(=O)₂-C₁₋₅-Alkyl, -S(=O)-C₁₋₅-Alkyl, - S(=O)₂-Phenyl, Oxo (=O), Thioxo (=S), -N(C₁₋₅-Alkyl)₂, -N(H)(C₁₋₅-Alkyl), -NO₂, -S-CF₃, -C(=O)-OH, -NH-S(=O)₂-C₁₋₅-Alkyl, -NH-C(=O)-C₁₋₅-Alkyl, -C(=O)-H; -C(=O)-C₁₅-Alkyl, -C(=O)-NH₂, -C(=O)-N(C₁₋₅-Alkyl)₂, -C(=O)-N(H)(C₁₋₅-Alkyl) und Phenyl substituiert sein, wobei die vorstehend genannten C₁₋₅-Alkyl-Reste jeweils linear oder verzweigt sein können und die Phenyl-Reste jeweils unsubstituiert oder mit 1, 2, 3 oder 4 Substituenten unabhängig voneinander ausgewählt aus der Gruppe bestehend aus F, Cl, Br, I, Methyl, Ethyl, Methoxy und Ethoxy substituiert sein können.

Besonders bevorzugt können die Substituenten, jeweils unabhängig voneinander, ausgewählt werden aus der Gruppe bestehend aus F, Cl, Br, I, -CN, Methyl, Ethyl, n-Propyl, iso-Propyl, n-Butyl, sec-Butyl, iso-Butyl, tert-Butyl, Ethenyl, Propenyl, Ethinyl, Propinyl, -OH, Oxo, Thioxo, -O-CH₃, -O-C₂H₅, -O-C₃H₇, -(CH₂)-O-CH₃, -(CH₂)-O-C₂H₅, -NH₂, -N(CH₃)₂, -N(C₂H₅)₂, -NH-CH₃, -NH-C₂H₅, -NO₂, -CF₃, -SF₅, -O-CF₃, -S-CF₃, -SH, -S-CH₃, -S-C₂H₅, -S(=O)-CH₃, -S(=O)-C₂H₅, -S(=O)₂-CH₃, -S(=O)₂-C₂H₅, - NH-S(=O)₂-CH₃, -C(=O)-OH, -C(=O)-H, -C(=O)-CH₃, -C(=O)-C₂H₅, -C(=O)-N(CH₃)₂, - C(=O)-NH-CH₃, -C(=O)-NH₂, -NH-C(=O)-CH₃, -NH-C(=O)-C₂H₅, -C(=O)-O-CH₃, - C(=O)-O-C₂H₅, -C(=O)-O-C(CH₃)₃ und Phenyl, wobei der Phenyl-Rest mit 1, 2 oder 3 Substituenten unabhängig voneinander ausgewählt aus der Gruppe bestehend aus F, Cl, Br, Methyl und Methoxy substituiert sein kann.

Sofern die heterocycloaliphatischen Reste eines oder mehrere weitere Heteroatome als Ringglieder aufweisen, können diese 1, 2, 3, 4 oder 5, besonders bevorzugt 1, 2 oder 3, Heteroatom(e) als Ringglied(er) aufweisen, die jeweils unabhängig voneinander, ausgewählt werden können aus der Gruppe bestehend aus Stickstoff, Sauerstoff und Schwefel.

Als geeignete heterocycloaliphatische Reste, die einfach oder mehrfach substituiert sein können, seien beispielsweise Imidazolidinyl, Piperidinyl, Piperazinyl, Morpholinyl, Pyrrolidinyl, Thiomorpholinyl, Azepanyl, Diazepanyl und Azocanyl genannt.

Sofern der heterocycloaliphatische Rest mit einem unsubstituierten oder wenigstens einfach substituierten mono- oder polyzyklischen Ringsystem kondensiert ist, können geeignete unsubstituierte oder wenigstens einfach substituierte Reste ausgewählt werden aus der Gruppe bestehend aus (3,4)-Dihydro-2H-benzo[1,4]oxazinyl; (1,2,3,4)-Tetrahydrochinazolinyl; (1,2,3,4)-Tetrahydrochinolinyl; (1,2,3,4)-Tetrahydroisochinolinyl, (2,3)-Dihydro-1H-indolyl, (2,3)-Dihydro-1H-isoindolyl und Decahydroisochinolinyl.

Sofern einer oder mehrere der Substituenten R^{1a}, R^{1b}, R^{1c}, R^{1d}, R² bis R²⁹ und M¹ und M² für einen Aryl- oder Heteroaryl-Rest stehen oder einen Aryl- oder Heteroaryl-Rest aufweisen, der einfach oder mehrfach substituiert ist, kann dieser ggf. mit 1, 2, 3, 4 oder 5 Substituenten unabhängig voneinander ausgewählt aus der Gruppe bestehend aus Halogen, -CN, -CH₂-CN, -NO₂, -OH, -SH, -NH₂, -CH₂-NH₂, - C(=O)-OH, C₁₋₅-Alkyl, -(CH₂)-OH, -(CH₂)-O-C₁₋₅-Alkyl, -C₂₋₅-Alkenyl, -C₂₋₅-Alkinyl, -S-C₁₋₅-Alkyl, -O-C₁₋₅-Alkyl, -CF₃, -SF₅, -CHF₂, -CH₂F, -O-CF₃, -O-CHF₂, -O-CH₂F, - C(=O)-CF₃, -S-CF₃, -S-CHF₂, -S-CH₂F, -S(=O)₂-Phenyl, -S(=O)₂-C₁₋₅-Alkyl, -S(=O)-C₁₋₅-Alkyl, -NH-C₁₋₅-Alkyl, -N-(C₁₋₅Alkyl)₂, -CH₂-NH-C₁₋₅-Alkyl, -CH₂-N-(C₁₋₅Alkyl)₂, - C(=O)-O-C₁₋₅-Alkyl, -C(=O)-H, -C(=O)-C₁₋₅-Alkyl, -CH₂-O-C(=O)-Phenyl, -O-C(=O)-Phenyl, -NH-S(=O)₂-C₁₋₅-Alkyl, -NH-C(=O)-C₁₋₅-Alkyl, -S(=O)₂-NH₂, -S(=O)₂-NH-C₁₋₅-Alkyl, -S(=O)₂-N(C₁₋₅-Alkyl)₂, -NH-C(=NH)-NH₂, -NH-S(=O)₂-OH, -C(=O)-NH-C₁₋₅-Alkyl, -C(=O)-NH₂, -C(=O)-N(C₁₋₅-Alkyl)₂, -Si(Phenyl)₂[C₁₋₅-Alkyl], Pyrazolyl, Pyrrolyl, (1,3)-Dioxolanyl, Phenyl, Furyl (Furanyl), Thiazolyl, Thiadiazolyl, Thiophenyl (Thienyl), Phenoxy, Benzyl und Phenethyl substituiert sein, wobei die zyklischen Substituenten selbst jeweils ggf. mit 1, 2, 3 oder 4 Substituenten unabhängig voneinander ausgewählt aus der Gruppe bestehend aus F, Cl, Br, I, -CF₃, Methyl, Ethyl, Methoxy und Ethoxy substituiert sein können.

Besonders bevorzugt können die Substituenten, jeweils unabhängig voneinander ausgewählt werden aus der Gruppe bestehend aus F, Cl, Br, I, -CN, -CH₂-CN, -NO₂, -OH, -SH, Methyl, Ethyl, n-Propyl, iso-Propyl, n-Butyl, iso-Butyl, sec-Butyl, tert-Butyl, n-Pentyl, neo-Pentyl, Ethenyl, Propenyl, Ethinyl, Propinyl, -CH₂-OH, -CH₂-O-CH₃, - CH₂-O-C₂H₅, -OH, -SH, -NH₂, -CH₂-NH₂, -C(=O)-OH, -S-CH₃, -S-C₂H₅, -S(=O)-CH₃, - S(=O)-C₂H₅, -S(=O)₂-CH₃, -S(=O)₂-C₂H₅,-O-CH₃, -O-C₂H₅, -O-C₃H₇, -O-C(CH₃)₃, - CF₃, -SF₅, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, Pyrazolyl, Pyrrolyl, -N(CH₃)₂, -N(C₂H₅)₂, -NH-CH₃, -NH-C₂H₅, -CH₂-O-C(=O)-Phenyl, -S(=O)₂-NH₂, -S(=O)₂-NH-CH₃, -S(=O)₂-N(CH₃)₂, -NH-C(=NH)-NH₂, -NH-S(=O)₂-OH, -NH-S(=O)₂-CH₃, -C(=O)-O-CH₃, -C(=O)-O-C₂H₅, - C(=O)-O-C(CH₃)₃, -C(=O)-H, -C(=O)-CH₃, -C(=O)-C₂H₅, -C(=O)-C(CH₃)₃, -NH-C(=O)-CH₃, -NH-C(=O)-C₂H₅, -O-C(=O)-Phenyl, -C(=O)-NH₂, -C(=O)-NH-CH₃, -C(=O)-N(CH₃)₂, -Si(Phenyl)₂[C(CH₃)₃], Phenyl, (1,3)-Dioxolanyl, Furyl (Furanyl), Thiadiazolyl, Thiophenyl (Thienyl), Phenoxy und Benzyl, wobei die zyklischen Substituenten selbst jeweils ggf. mit 1, 2, 3 oder 4 Substituenten unabhängig voneinander ausgewählt aus der Gruppe bestehend aus F, Cl, Br, I, -CF₃, Methyl und Methoxy substituiert sein können.

Als geeignete Aryl-Reste seien beispielsweise Phenyl, 1-Naphthyl, 2-Naphthyl und Anthracenyl genannt.

Sofern einer oder mehrere der Substituenten R^{1a}, R^{1b}, R^{1c}, R^{1d}, R² bis R²⁹ und M¹ und M² für einen Heteroaryl-Rest stehen oder einen Heteroaryl-Rest aufweisen, können dessen Heteroatom(e), jeweils unabhängig voneinander, bevorzugt ausgewählt werden aus der Gruppe bestehend aus Sauerstoff, Schwefel und Stickstoff. Bevorzugt kann ein Heteroaryl-Rest ggf. 1, 2, 3, 4 oder 5, besonders bevorzugt 1, 2 oder 3 Heteroatome, aufweisen.

Als geeignete Heteroaryl-Reste seien beispielsweise Furyl (Furanyl), Thienyl (Thiophenyl), Pyrazolyl, Imidazolyl, Thiazolyl, Thiadiazolyl, Triazolyl, Pyrrolyl, Oxazolyl, Oxadiazolyl, Isoxazolyl, Pyridinyl, Pyridazinyl, Pyrimidinyl, Pyrazinyl, Pyranyl, Diazinyl, Triazinyl, Tetrazinyl, Tetrazolyl, Purinyl, Dithiazolyl und Pentazolyl genannt.

Sofern der Heteroaryl-Rest mit einem unsubstituierten oder wenigstens einfach substituierten mono- oder polyzyklischen Ringsystem kondensiert ist, können geeignete unsubstituierte oder wenigstens einfach substituierte Heteroaryl-Reste ausgewählt werden aus der Gruppe bestehend aus Indolyl, Isoindolyl, Benzo[b]furanyl, Isobenzo[b]furanyl, Indazolyl, Indolizinyl, Chinolinyl, Isochinolinyl, Chinazolinyl, Benzo[b]thiophenyl und Isobenzo[b]thiophenyl.

Sofern die Substituenten R^{1a} und R^{1b} oder R^{1b} und R^{1c} oder R^{1c} und R^{1d} zusammen mit der sie verbindenden C-C-Brücke einen annelierten Phenyl-Rest bilden, der einfach oder mehrfach substituiert ist, kann dieser mit 1, 2, 3 oder 4 Substituenten unabhängig voneinander ausgewählt aus der Gruppe bestehend aus Halogen, -CN, -CH₂-CN, -NO₂, -OH, -SH, -NH₂, -CH₂-NH₂, -C(=O)-OH, C₁₋₅-Alkyl, - (CH₂)-O-C₁₋₅-Alkyl, -C₂₋₅-Alkenyl, -C₂₋₅-Alkinyl, -S-C₁₋₅-Alkyl, -O-C₁₋₅-Alkyl, -CF₃, -SF₅, -CHF₂, -CH₂F, -O-CF₃, -O-CHF₂, -O-CH₂F, -C(=O)-CF₃, -S-CF₃, -S-CHF₂, -S-CH₂F, - S(=O)₂-Phenyl, -S(=O)₂-C₁₋₅-Alkyl, -S(=O)-C₁₋₅Alkyl, -NH-C₁₋₅-Alkyl, -N-(C₁₋₅Alkyl)₂, - CH₂-NH-C₁₋₅-Alkyl, -CH₂-N-(C₁₋₅Alkyl)₂, -C(=O)-O-C₁₋₅-Alkyl,- -C(=O)-H, -C(=O)-C₁₋₅-Alkyl, -CH₂-O-C(=O)-Phenyl, -O-C(=O)-Phenyl, -NH-S(=O)₂-C₁₋₅-Alkyl, -NH-C(=O)-C₁₅-Alkyl, -C(=O)-NH-C₁₋₅-Alkyl, -C(=O)-NH₂, -C(=O)-N(C₁₋₅-Alkyl)₂, -Si(Phenyl)₂[C₁₋₅-Alkyl], Pyrazolyl, Pyrrolyl, (1,3)-Dioxolanyl, Phenyl, Furyl (Furanyl), Thiazolyl, Thiadiazolyl, Thiophenyl (Thienyl), Phenoxy, Benzyl und Phenethyl substituiert sein, wobei die zyklischen Substituenten selbst jeweils ggf. mit 1, 2, 3 oder 4 Substituenten unabhängig voneinander ausgewählt aus der Gruppe bestehend aus F, Cl, Br, I, - CF₃, Methyl, Ethyl, Methoxy und Ethoxy substituiert sein können.

Besonders bevorzugt können die Substituenten, jeweils unabhängig voneinander ausgewählt werden aus der Gruppe bestehend aus F, Cl, Br, I, -CN, -CH₂-CN, -NO₂, -OH, -SH, Methyl, Ethyl, n-Propyl, iso-Propyl, n-Butyl, iso-Butyl, sec-Butyl, tert-Butyl, n-Pentyl, neo-Pentyl, Ethenyl, Propenyl, Ethinyl, Propinyl, -CH₂-O-CH₃, -CH₂-O-C₂H₅, -OH, -SH, -NH₂, -CH₂-NH₂, -C(=O)-OH, -S-CH₃, -S-C₂H₅, -S(=O)-CH₃, -S(=O)-C₂H₅, - S(=O)₂-CH₃, -S(=O)₂-C₂H₅,-O-CH₃, -O-C₂H₅, -O-C₃H₇, -O-C(CH₃)₃, -CF₃, -SF₅, - CHF₂, -CH₂F, -O-CF₃, -O-CHF₂, -O-CH₂F, -C(=O)-CF₃, -S-CF₃, -S-CHF₂, -S-CH₂F, - S(=O)₂-Phenyl, Pyrazolyl, Pyrrolyl, -N(CH₃)₂, -N(C₂H₅)₂, -NH-CH₃, -NH-C₂H₅, -CH₂-O-C(=O)-Phenyl, -NH-S(=O)₂-CH₃, -C(=O)-O-CH₃, -C(=O)-O-C₂H₅, -C(=O)-O-C(CH₃)₃, - C(=O)-H, -C(=O)-CH₃, -C(=O)-C₂H₅, -C(=O)-C(CH₃)₃, -NH-C(=O)-CH₃, -NH-C(=O)-C₂H₅, -O-C(=O)-Phenyl, -C(=O)-NH₂, -C(=O)-NH-CH₃, -C(=O)-N(CH₃)₂, - Si(Phenyl)₂[C(CH₃)₃], Phenyl, (1,3)-Dioxolanyl, Furyl (Furanyl), Thiadiazolyl, Thiophenyl (Thienyl), Phenoxy und Benzyl, wobei die zyklischen Substituenten selbst jeweils ggf. mit 1, 2, 3 oder 4 Substituenten unabhängig voneinander ausgewählt aus der Gruppe bestehend aus F, Cl, Br, I, -CF₃, Methyl und Methoxy substituiert sein können.

Unter einem mono- bzw. polyzyklischen Ringsystem werden im Sinne der vorliegenden Erfindung mono- bzw. polyzyklische Kohlenwasserstoffreste verstanden, die gesättigt, ungesättigt oder aromatisch sein und ggf. eines oder mehrere Heteroatome als Ringglieder aufweisen können. Ein solches mono- bzw. polyzyklisches Ringsystem kann beispielsweise mit einem cycloaliphatischen Rest, einem Aryl-Rest oder einem Heteroaryl-Rest kondensiert (anneliert) sein. Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt, ungesättigt oder aromatisch sein. Die Heteroatome jedes Ringes können, jeweils unabhängig voneinander, bevorzugt ausgewählt werden aus der Gruppe bestehend aus Sauerstoff, Stickstoff und Schwefel. Vorzugsweise enthält ein Ring 0, 1, 2 oder 3 Heteroatome. Vorzugsweise sind die jeweiligen Ringe des mono- oder polyzyklischen Ringsystems 5-, 6- oder 7-gliedrig, besonders bevorzugt 5- oder 6-gliedrig.

Sofern einer oder mehrere der Substituenten R^{1a}, R^{1b}, R^{1c}, R^{1d}, R² bis R²⁹ und M¹ und M² ein monozyklisches oder polyzyklisches Ringsystem aufweisen, das einfach oder mehrfach substituiert ist, kann dieses ggf. mit 1, 2, 3, 4 oder 5 Substituenten substituiert sein, die unabhängig voneinander ausgewählt werden können aus der Gruppe bestehend aus Halogen, -CN, -NO₂, -OH, -SH, -NH₂, Oxo (=O), Thioxo (=S), -C(=O)-OH, -C₁₋₅-Alkyl, -C₂₋₅-Alkenyl, -C₂₋₅-Alkinyl, -(CH₂)-O-C₁₋₅-Alkyl, -S-C₁₋₅-Alkyl, -O-C₁₋₅-Alkyl, -CF₃,-SF₅, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, -S(=O)₂-C₁₋₅-Alkyl, - S(=O)-C₁₋₅-Alkyl, -NH-C₁₋₅-Alkyl, -N(C₁₋₅Alkyl)(C₁₋₅-Alkyl), -C(=O)-O-C₁₋₅-Alkyl, - C(=O)-H, -C(=O)-C₁₋₅-Alkyl, -CH₂-O-C(=O)-Phenyl, -O-C(=O)-Phenyl, -NH-S(=O)₂-C₁₅-Alkyl, -NH-C(=O)-C₁₋₅-Alkyl, -C(=O)-NH₂, -C(=O)-NH-C₁₋₅-Alkyl, -C(=O)-N(C₁₋₅-Alkyl)₂, Pyrazolyl, Phenyl, Furyl (Furanyl), Thiadiazolyl, Thiophenyl (Thienyl), Phenoxy und Benzyl, wobei die zyklischen Substituenten selbst jeweils ggf. mit 1, 2, 3 oder 4 Substituenten unabhängig voneinander ausgewählt aus der Gruppe bestehend aus F, Cl, Br, I, Methyl, Ethyl, Methoxy und Ethoxy substituiert sein können.

Besonders bevorzugt können die Substituenten, jeweils unabhängig voneinander, ausgewählt werden aus der Gruppe bestehend aus F, Cl, Br, I, -CN, -NO₂, -OH, -SH, Methyl, Ethyl, n-Propyl, iso-Propyl, n-Butyl, iso-Butyl, sec-Butyl, tert.-Butyl, n-Pentyl, neo-Pentyl, Ethenyl, Propenyl, Ethinyl, Propinyl, -CH₂-O-CH₃, -CH₂-O-C₂H₅, -OH, - SH, -NH₂, Oxo, Thioxo, -C(=O)-OH, -S-CH₃, -S-C₂H₅, -S(=O)-CH₃, -S(=O)₂-CH₃, - S(=O)-C₂H₅, -S(=O)₂-C₂H₅, -O-CH₃, -O-C₂H₅, -O-C₃H₇, -O-C(CH₃)₃, -CF₃, -SF₅, - CHF₂, -CH₂F, -O-CF₃, -O-CHF₂, -O-CH₂F, -C(=O)-CF₃, -S-CF₃, -S-CHF₂, -S-CH₂F, - S(=O)₂-Phenyl, Pyrazolyl, -N(CH₃)₂, -N(C₂H₅)₂, -NH-CH₃, -NH-C₂H₅, -CH₂-O-C(=O)-Phenyl, -NH-S(=O)₂-CH₃, -C(=O)-O-CH₃, -C(=O)-O-C₂H₅, -C(=O)-H, -C(=O)-CH₃, - C(=O)-C₂H₅, -NH-C(=O)-CH₃, -NH-C(=O)-C₂H₅, -O-C(=O)-Phenyl, -C(=O)-NH₂, - C(=O)-NH-CH₃, -C(=O)-N(CH₃)₂, Phenyl, Furyl (Furanyl), Thiadiazolyl, Thiophenyl (Thienyl), Phenoxy und Benzyl, wobei die zyklischen Substituenten selbst jeweils ggf. mit 1, 2, 3 oder 4 Substituenten unabhängig voneinander ausgewählt aus der Gruppe bestehend aus F, Cl, Br, I, Methyl und Methoxy substituiert sein können.

Sofern einer der vorstehend genannten Substituenten R^{1a}, R^{1b}, R^{1c}, R^{1d} und R² bis R²⁹ eine lineare oder verzweigte Alkylen-Gruppe aufweist, kann die Alkylen-Gruppe vorzugsweise ausgewählt werden aus der Gruppe bestehend aus -(CH₂)-, -(CH₂)₂-, - C(H)(CH₃)-, -(CH₂)₃-, -(CH₂)₄-, -(CH₂)₅- und -C(C₂H₅)(H)-.

Der Fachmann versteht, daß einige der erfindungsgemäßen substituierten Imidazo-3-yl-amin-Verbindungen der allgemeinen Formel I in Form von Tautomeren vorliegen können, die ebenfalls Gegenstand der vorliegenden Erfindung sind und jeweils auch als Wirkstoffe in den untenstehend beschriebenen Arzneimitteln vorliegen können.

Bevorzugt sind substituierte Imidazo-3-yl-amin-Verbindungen der allgemeinen Formel I,
worin
A¹ für ein Stickstoffatom oder für eine C-R^{1a}-Gruppe steht,
A² für ein Stickstoffatom oder für eine C-R^{1b}-Gruppe steht,
A³ für ein Stickstoffatom oder für eine C-R^{1c}-Gruppe steht,
A⁴ für ein Stickstoffatom oder für eine C-R^{1d}-Gruppe steht,

R^{1a}, R^{1b}, R^{1c}, R^{1d}, unabhängig voneinander, jeweils für einen Wasserstoff-Rest; für einen Halogen-Rest; -NO₂; -CN; -NH₂; -NHR⁴; -NR⁵R⁶; -NH-C(=O)-R⁷; -C(=O)-R⁸, - C(=O)-NH₂; -C(=O)-NHR⁹; -C(=O)-NR¹⁰R¹¹; -C(=O)-OR¹²; -(CH₂)ₘ-O(=O)-OR¹³ mit m = 1, 2, 3, 4 oder 5; -O-C(=O)-R¹⁴; -(CH₂)ₙ-O-C(=O)-R¹⁵ mit n = 1, 2, 3, 4 oder 5; - OR¹⁶; -(CH₂)ₒ-O-R¹⁷ mit o = 1, 2, 3; 4 oder 5; -SR¹⁸; -(CH₂)ₚ-S(=O)ₜ-R¹⁹ mit p = 1, 2, 3, 4 oder 5 und t = 0,1 oder 2; -NH-S(=O)₂-R²⁶R²⁷; -S(=O)₂-NR²⁸R²⁹, -SF₅; für einen linearen oder verzweigten, gesättigten oder ungesättigten, unsubstituierten oder wenigstens einfach substituierten aliphatischen C₁₋₁₀-Rest; für einen gesättigten oder ungesättigten, unsubstituierten oder wenigstens einfach substituierten, ggf. wenigstens ein Heteroatom als Ringglied aufweisenden cycloaliphatischen C₃₋₈-Rest, der über eine lineare oder verzweigte C₁₋₅-Alkylen-Gruppe gebunden sein kann; oder für einen unsubstituierten oder wenigstens einfach substituierten 5- bis 14-gliedrigen Aryl- oder Heteroaryl-Rest, der über eine lineare oder verzweigte C₁₋₅-Alkylen-Gruppe gebunden sein kann, steht,
oder ggf. R^{1a} und R^{1b} mit der sie verbindenden C-C-Brücke einen unsubstituierten oder wenigstens einfach substituierten annelierten Phenyl-Rest bilden,
oder ggf. R^{1b} und R^{1c} mit der sie verbindenden C-C-Brücke einen unsubstituierten oder wenigstens einfach substituierten annelierten Phenyl-Rest bilden,
oder ggf. R^{1c} und R^{1d} mit der sie verbindenden C-C-Brücke einen unsubstituierten oder wenigstens einfach substituierten annelierten Phenyl-Rest bilden,
R² und R³, unabhängig voneinander, jeweils für einen Wasserstoff-Rest; -C(=O)-R²⁰, -(CH₂)_{q}-C(=O)-R²¹ mit q = 1, 2, 3, 4 oder 5; -C(=O)-O-R²²; -(CH₂)ᵣ-C(=O)-O-R²³ mit r = 1, 2, 3, 4 oder 5; -C(=O)-NHR²⁴; -(CH₂)ₛ-C(=O)-NHR²⁵ mit s = 1, 2, 3, 4 oder 5; für einen linearen oder verzweigten, gesättigten oder ungesättigten, unsubstituierten oder wenigstens einfach substituierten aliphatischen C₁₋₁₆-Rest; für einen gesättigten oder ungesättigten, unsubstituierten oder wenigstens einfach substituierten, ggf. wenigstens ein Heteroatom als Ringglied aufweisenden cycloaliphatischen C₄₋₈-Rest, der über eine lineare oder verzweigte C₁₋₅-Alkylen-Gruppe gebunden sein kann; oder für einen unsubstituierten oder wenigstens einfach substituierten 5- bis 14-gliedrigen Aryl- oder Heteroaryl-Rest, der über eine lineare oder verzweigte C₁₋₅-Alkylen-Gruppe gebunden sein kann, stehen,
oder R² und R³ zusammen mit dem sie verbindenden Stickstoffatom als Ringglied einen gesättigten oder ungesättigten, ggf. wenigstens ein weiteres Heteroatom als Ringglied aufweisenden heterocycloaliphatischen C₄₋₁₀-Rest bilden, der ggf. mit einem unsubstituierten oder wenigstens einfach substituierten mono- oder polyzyklischen Ringsystem kondensiert ist, wobei die Ringe des Ringsystems jeweils 5-, 6- oder 7-gliedrig sind;
R⁴, R⁵, R⁶, R⁷, R⁹, R¹⁰, R¹¹, R¹⁴ und R¹⁵, jeweils unabhängig voneinander, für einen linearen oder verzweigten, gesättigten oder ungesättigten aliphatischen C₁₋₄-Rest oder für einen unsubstituierten oder wenigstens einfach substituierten 5- bis 14-gliedrigen Aryl- oder Heteroaryl-Rest, der über eine lineare oder verzweigte C₁₋₅-Alkylen-Gruppe gebunden sein kann, stehen,
R⁸, R¹² , R¹³ , R¹⁶, R¹⁷, R¹⁸, R¹⁹, R²⁰, R²¹, R²², R²³, R²⁴, R²⁵, R²⁶, R²⁷, R²⁸ und R²⁹, jeweils unabhängig voneinander, für einen Wasserstoff-Rest; für einen linearen oder verzweigten, gesättigten oder ungesättigten aliphatischen C₁₋₄-Rest oder für einen unsubstituierten oder wenigstens einfach substituierten 5- bis 14-gliedrigen Aryl- oder Heteroaryl-Rest, der über eine lineare oder verzweigte C₁₋₅-Alkylen-Gruppe gebunden sein kann, stehen,
M¹ für einen 5- oder 6-gliedrigen Aryl- oder Heteroaryl-Rest steht, der mit wenigstens einem weiteren Substituenten substituiert sein kann und ggf. mit einem unsubsituierten oder wenigstens einfach substituierten mono- oder bizyklischem Ringsystem kondensiert ist, wobei die Ringe des Ringsystems jeweils 5-, 6- oder 7-gliedrig sind, und
M² für einen 5- oder 6-gliedrigen Aryl- oder Heteroaryl-Rest steht, der unsubstituiert oder wenigstens einfach substituiert und mit einem unsubstituierten oder wenigstens einfach substituiertem mono- oder bizyklischem Ringsystem kondensiert sein kann, wobei die Ringe des Ringsystems jeweils 5-, 6- oder 7-gliedrig sind,
wobei
die vorstehend genannten cycloaliphatischen Reste ggf. 1, 2, 3, 4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,
die vorstehend genannten heterocycloaliphatischen Reste ggf. weitere 1, 2, 3, 4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,
die Ringe des mono- oder polyzyklischen Ringsystems jeweils ggf. 0, 1, 2 oder 3 Heteroatom(e) als Ringglied(er) aufweisen, die unabhängig voneinander aus der Gruppe bestehend aus Sauerstoff, Stickstoff und Schwefel ausgewählt sind;
und
die vorstehend genannten Heteroaryl-Reste ggf. 1, 2, 3, 4 oder 5 Heteroatom(e) als Ringglied(er) aufweisen können, die jeweils unabhängig voneinander, ausgewählt werden können aus der Gruppe bestehend aus Sauerstoff, Schwefel und Stickstoff;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt sind ferner substituierte Imidazo-3-yl-amin-Verbindungen der vorstehend angegebenen allgemeinen Formel I, in denen
A¹ für eine C-R^{1a}-Gruppe steht,
A² für eine C-R^{1b}-Gruppe steht,
A³ für eine C-R^{1c}-Gruppe steht und
A⁴ für eine C-R^{1d}-Gruppe steht,
oder
A¹ für ein Stickstoffatom steht,
A² für eine C-R^{1b}-Gruppe steht,
A³ für eine C-R^{1c}-Gruppe steht und
A⁴ für eine C-R^{1d}-Gruppe steht,
oder
A¹ für eine C-R^{1a}-Gruppe steht,
A² für ein Stickstoffatom steht,
A³ für eine C-R^{1c}-Gruppe steht und
A⁴ für eine C-R^{1d}-Gruppe steht,
oder
A¹ für eine C-R^{1a}-Gruppe steht,
A² für eine C-R^{1b}-Gruppe steht,
A³ für ein Stickstoffatom steht und
A⁴ für eine C-R^{1d}-Gruppe steht,
oder
A¹ für eine C-R^{1a}-Gruppe steht,
A² für eine C-R^{1b}-Gruppe steht,
A³ für eine C-R^{1c}-Gruppe steht und
A⁴ für ein Stickstoffatom steht,
oder
A¹ und A³ jeweils für ein Stickstoffatom stehen,
A² für eine C-R^{1b}-Gruppe steht und
A⁴ für eine C-R^{1d}-Gruppe steht,
und jeweils die übrigen Reste R^{1a}, R^{1b}, R^{1c}, R^{1d}, R² bis R²⁹, M¹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte Imidazo-3-yl-amin-Verbindungen der vorstehend angegeben allgemeinen Formel I, worin
A¹ für eine C-R^{1a}-Gruppe steht,
A² für eine C-R^{1b}-Gruppe steht,
A³ für eine C-R^{1c}-Gruppe steht und
A⁴ für eine C-R^{1d}-Gruppe steht,
oder
A¹ für ein Stickstoffatom steht,
A² für eine C-R^{1b}-Gruppe steht,
A³ für eine C-R^{1c}-Gruppe steht und
A⁴ für eine C-R^{1d}-Gruppe steht,
oder
A¹ für eine C-R^{1a}-Gruppe steht,
A² für ein Stickstoffatom steht,
A³ für eine C-R^{1c}-Gruppe steht und
A⁴ für eine C-R^{1d}-Gruppe steht,
oder
A¹ für eine C-R^{1a}-Gruppe steht,
A² für eine C-R^{1b}-Gruppe steht,
A³ für eine C-R^{1c}-Gruppe steht und
A⁴ für ein Stickstoffatom steht,
und jeweils die übrigen Reste R^{1a}, R^{1b}, R^{1c}, R^{1d}, R² bis R²⁹, M¹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte Imidazo-3-yl-amin-Verbindungen der vorstehend angegebenen allgemeinen Formel I, worin
A¹ für eine C-R^{1a}-Gruppe steht,
A² für eine C-R^{1b}-Gruppe steht,
A³ für eine C-R^{1c}-Gruppe steht und
A⁴ für eine C-R^{1d}-Gruppe steht,
oder
A¹ für eine C-R^{1a}-Gruppe steht,
A² für ein Stickstoffatom steht,
A³ für eine C-R^{1c}-Gruppe steht und
A⁴ für eine C-R^{1d}-Gruppe steht,
oder
A¹ für ein Stickstoffatom steht,
A² für eine C-R^{1b}-Gruppe steht,
A³ für eine C-R^{1c}-Gruppe steht und
A⁴ für eine C-R^{1d}-Gruppe steht,
und jeweils die übrigen Reste R¹⁸, R^{1b}, R^{1c}, R^{1d}, R² bis R²⁹, M¹ und M² die vorstehend genannte Bedeutung haben, jeweils ggf. in form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte Imidazo-3-yl-amin-Verbindungen der vorstehend angegebenen allgemeinen Formel I, worin R^{1a}, R^{1b}, R^{1c}, R^{1d}, unabhängig voneinander, jeweils für -H; -F; -Cl, -Br; -I; -NO₂; -CN; -CF₃; -SF₅; -NH₂; -S(=O)₂-NH₂; -NHR⁴; -NR⁵R⁶; -C(=O)-OR¹²; -(CH₂)ₘ-C(=O)-OR¹³ mit m = 1, 2 oder 3; -O-C(=O)-R¹⁴; -OR¹⁶; -(CH₂)ₒ-O-R¹⁷ mit o = 1, 2 oder 3; für einen linearen oder verzweigten C₁-₁₀-Alkyl-Rest; oder für einen unsubstituierten oder wenigstens einfach substituierten 5- oder 6-gliedrigen Aryl- oder Heteroaryl-Rest, der über eine lineare oder verzweigte C₁₋₄-Alkylen-Gruppe gebunden sein kann, steht,
vorzugsweise R^{1a}, R^{1b}, R^{1c}, R^{1d}, unabhängig voneinander, jeweils für -H; -F; -Cl, -Br; -I; -NO₂; -CN; -CF₃; -SF₅; -NH₂; -S(=O)₂-NH₂; -NHR⁴; -NR⁵R⁶; -C(=O)-OR¹²; -(CH₂)ₘ-C(=O)-OR¹³ mit m = 1, 2 oder 3; -O-C(=O)-R¹⁴; -OR¹⁶; -(CH₂)ₒ-O-R¹⁷ mit o = 1, 2 oder 3; für einen linearen oder verzweigten Alkyl-Rest ausgewählt aus der Gruppe bestehend aus Methyl, Ethyl, n-Propyl, iso-Propyl, n-Butyl, iso-Butyl, sec-Butyl und tert-Butyl, oder für einen Aryl- oder Heteroaryl-Rest ausgewählt aus der Gruppe bestehend aus Phenyl, Furanyl, Thiophenyl und Pyridinyl, der jeweils unsubstituiert oder mit 1, 2, 3, 4 oder 5 Substituenten unabhängig voneinander ausgewählt aus der Gruppe bestehend aus F, Cl, Br, -CN, Methyl, Ethyl, n-Propyl, iso-Propyl, n-Butyl, sec-Butyl, iso-Butyl, tert-Butyl, -OH, -O-CH₃, -O-C₂H₅ substituiert und ggf. über eine - (CH₂)-, -(CH₂)₂- oder -(CH₂)₃-Gruppe gebunden ist, steht,
besonders bevorzugt R¹⁸, R^{1b}, R^{1c}, R^{1d}, unabhängig voneinander, jeweils für -H; -F; - Cl, -Br; -CF₃; -CN; -SF₅; -C(=O)-OR¹²; -S(=O)₂-NH₂; -(CH₂)ₘ-C(=O)-OR¹³ mit m = 1 oder 2; -OR¹⁶; -(CH₂)ₒ-O-R¹⁷ mit o = 1 oder 2; für einen linearen oder verzweigten Alkyl-Rest ausgewählt aus der Gruppe bestehend aus Methyl, Ethyl, n-Propyl, iso-Propyl, n-Butyl, iso-Butyl, sec-Butyl und tert-Butyl, oder für einen Phenyl-Rest, der unsubstituiert oder mit 1, 2 oder 3 Substituenten unabhängig voneinander ausgewählt aus der Gruppe bestehend aus F, Cl, Br, Methyl, Ethyl, n-Propyl, iso-Propyl, n-Butyl, sec-Butyl, iso-Butyl, tert-Butyl, -O-CH₃, -O-C₂H₅ substituiert und ggf. über eine -(CH₂)-, -(CH₂)₂- oder -(CH₂)₃-Gruppe gebunden ist, steht,
ganz besonders bevorzugt R^{1a}, R^{1b}, R^{1c}, R^{1d}, unabhängig voneinander, jeweils für -H; -F; -Cl, -Br; -CF₃; -CN; -SF₅; -S(=O)₂-NH₂; -C(=O)-OR¹²; -(CH₂)ₘ-C(=O)-OR¹³ mit m = 1; -OR¹⁶; -(CH₂)ₒ-O-R¹⁷ mit o = 1; für einen linearen oder verzweigten Alkyl-Rest ausgewählt aus der Gruppe bestehend aus Methyl, Ethyl, n-Propyl, iso-Propyl, n-Butyl, iso-Butyl, sec-Butyl und tert-Butyl; oder für einen unsubstituierten Phenyl-Rest, der ggf. über eine -(CH₂)-, -(CH₂)₂- oder -(CH₂)₃-Gruppe gebunden ist, steht,
und jeweils die übrigen Reste A¹, A², A³, A⁴, R² bis R²⁹, M¹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte Imidazo-3-yl-amin-Verbindungen der vorstehend angegebenen allgemeinen Formel I, worin R^{1a} und R^{1b} oder R^{1b} und R^{1c} oder R^{1c} und R^{1d} zusammen mit der sie verbindenden C-C-Brücke einen annelierten Phenyl-Rest bilden, der mit 1, 2, 3 oder 4 Substituenten unabhängig voneinander ausgewählt aus der Gruppe bestehend aus F, Cl, Br, -CN, Methyl, Ethyl, n-Propyl, iso-Propyl, n-Butyl, sec-Butyl, iso-Butyl, tert-Butyl, -OH, -O-CH₃, -O-C₂H₅ substituiert sein kann;
und jeweils die übrigen Reste A¹, A², A³, A⁴, R² bis R²⁹, M¹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Der Fachmann versteht, dass sich in dem Fall, dass R^{1a} und R^{1b} zusammen mit der sie verbindenden C-C-Brücke einen annelierten Phenyl-Rest bilden, die folgende allgemeine Formel Ia ergibt:

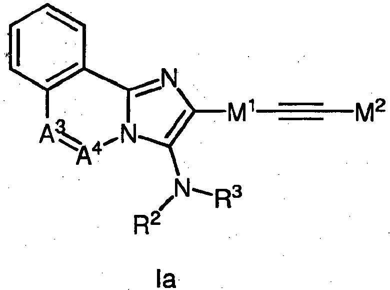

Der Fachmann versteht, dass sich in dem Fall, dass R^{1b} und R^{1c} zusammen mit der sie verbindenden C-C-Brücke einen annelierten Phenyl-Rest bilden, die folgende allgemeine Formel Ib ergibt:

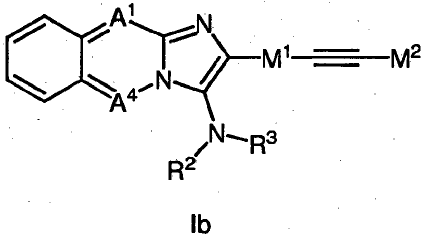

Der Fachmann versteht, dass sich in dem Fall, dass R^{1c} und R^{1d} zusammen mit der sie verbindenden C-C-Brücke einen annelierten Phenyl-Rest bilden, die folgende allgemeine Formel Ic ergibt:

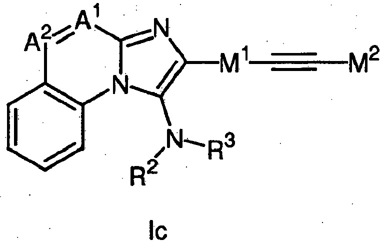

Weiterhin bevorzugt sind substituierte Imidazo-3-yl-amin-Verbindungen der vorstehend angegebenen allgemeinen Formel I, worin R² und R³, unabhängig voneinander, jeweils für einen Wasserstoff-Rest; -C(=O)-R²⁰; -(CH₂)_{q}-C(=O)-R²¹ mit q = 1, 2, 3, 4 oder 5; -(CH₂)ᵣ-C(=O)-O-R²³ mit r = 1, 2, 3, 4 oder 5; -C(=O)-NHR²⁴; für einen linearen oder verzweigten C₁₋₁₆ Alkyl-Rest; für einen unsubstituierten oder wenigstens einfach substituierten C₄₋₈ Cycloalkyl-Rest, der über eine lineare oder verzweigte C₁₋₃-Alkylen-Gruppe gebunden sein kann; oder für einen unsubstituierten oder wenigstens einfach substituierten, 5- oder 6-gliedrigen Aryl- oder Heteroaryl-Rest, der über eine lineare oder verzweigte C₁₋₃-Alkylen-Gruppe gebunden sein kann, stehen,
vorzugsweise R² und R³, unabhängig voneinander, jeweils für einen Wasserstoff-Rest; -C(=O)-R²⁰; -(CH₂)_{q}-C(=O)-R²¹ mit q = 1, 2 oder 3; -(CH₂)ᵣ-C(=O)-O-R²³ mit r = 1, 2 oder 3; für einen linearen oder verzweigten C₁₋₁₀ Alkyl-Rest; für einen C₄₋₈ Cycloalkyl-Rest, der jeweils unsubstituiert oder mit 1, 2, 3, 4 oder 5 Substituenten unabhängig voneinander ausgewählt aus der Gruppe bestehend aus F, Cl, Br, -CN, - CF₃, -SF₅, Methyl, Ethyl, n-Propyl, iso-Propyl, n-Butyl, sec-Butyl, iso-Butyl, tert-Butyl, -OH, -O-CH₃ und -O-C₂H₅ substituiert und ggf. über eine lineare oder verzweigte C₁₃-Alkylen-Gruppe gebunden ist; oder für einen Aryl- oder Heteroaryl-Rest ausgewählt aus der Gruppe bestehend aus Phenyl, Thiophenyl, Furanyl und Pyridinyl, der jeweils unsubstituiert oder mit 1, 2, 3, 4 oder 5 Substituenten unabhängig voneinander ausgewählt aus der Gruppe bestehend aus F, Cl, Br, -CN, -CF₃, Methyl, Ethyl, n-Propyl, iso-Propyl, n-Butyl, sec-Butyl, iso-Butyl, tert-Butyl, -OH, -O-CH₃ und -O-C₂H₅ substituiert und ggf. über eine linear oder verzweigte C₁₋₃-Alkylen-Gruppe gebunden ist, stehen,
besonders bevorzugt R² und R³, unabhängig voneinander, jeweils für einen Wasserstoff-Rest; -C(=O)-R²⁰; -(CH₂)_{q}-C(=O)-R²¹ mit q = 1 oder 2; -(CH₂)ᵣC(=O)-O-R²³ mit r = 1 oder 2; für einen Alkyl-Rest ausgewählt aus der Gruppe bestehend aus Methyl; Ethyl; Propyl; iso-Propyl; n-Butyl; tert-Butyl; sec-Butyl; iso-Butyl; n-Pentyl; n-Hexyl; n-Heptyl; n-Octyl; (1,1,3,3)-Tetramethyl-butyl; für einen unsubstituierten Cyclopentyl- oder Cyclohexyl-Rest, der jeweils über eine -(CH₂)-, -(CH₂)₂- oder-(CH₂)₃-Gruppe gebunden sein kann; oder für einen Phenyl- oder Pyridinyl-Rest, der jeweils unsubstituiert oder mit 1, 2 oder 3 Substituenten unabhängig voneinander ausgewählt aus der Gruppe bestehend aus F, Cl, Br, -CN, -CF₃, Methyl, Ethyl, n-Propyl, iso-Propyl, tert-Butyl, -O-CH₃ und -O-C₂H₅ substituiert und ggf. über eine - (CH₂)-, -(CH₂)₂-, -C(H)(CH₃)- oder -(CH₂)₃-Gruppe gebunden ist, stehen,
und jeweils die übrigen Reste A¹, A², A³, A⁴, R^{1a}, R^{1b}, R^{1c}, R^{1d}, R⁴ bis R²⁹, M¹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte Imidazo-3-yl-amin-Verbindungen der vorstehend angegebenen allgemeinen Formel I, worin einer der Reste R² und R³ für einen Wasserstoff-Rest oder für einen linearen oder verzweigten C₁₋₁₆ Alkyl-Rest steht und der andere dieser beiden Reste für einen Wasserstoff-Rest; -C(=O)-R²⁰; - (CH₂)_{q}-C(=O)-R²¹ mit q = 1, 2, 3, 4 oder 5; -(CH₂)ᵣ-C(=O)-O-R²³ mit r = 1, 2, 3, 4 oder 5; -C(=O)-NHR²⁴; für einen linearen oder verzweigten C₁₋₁₆ Alkyl-Rest; für einen unsubstituierten oder wenigstens einfach substituierten C₄₋₈ Cycloalkyl-Rest, der über eine lineare oder verzweigte C₁₋₃-Alkylen-Gruppe gebunden sein kann; oder für einen unsubstituierten oder wenigstens einfach substituierten, 5- oder 6-gliedrigen Aryl- oder Heteroaryl-Rest, der über eine lineare oder verzweigte C₁₋₃-Alkylen-Gruppe gebunden sein kann, steht,
vorzugsweise einer der beiden Reste R² und R³ für einen Wasserstoff-Rest oder für einen linearen oder verzweigten C₁₋₁₀-Alkyl-Rest steht und der andere dieser beiden Reste für einen Wasserstoff-Rest; -C(=O)-R²⁰; -(CH₂)_{q}-C(=O)-R²¹ mit q = 1, 2 oder 3; -(CH₂)ᵣ-C(=O)-O-R²³ mit r = 1, 2 oder 3; für einen linearen oder verzweigten C₁₋₁₀ Alkyl-Rest; für einen C₄₋₈ Cycloalkyl-Rest, der jeweils unsubstituiert oder mit 1, 2, 3, 4 oder 5 Substituenten unabhängig voneinander ausgewählt aus der Gruppe bestehend aus F, Cl, Br, -CN, -CF₃, -SF₅, Methyl, Ethyl, n-Propyl, iso-Propyl, n-Butyl, sec-Butyl, iso-Butyl, tert-Butyl, -OH, -O-CH₃ und -O-C₂H₅ substituiert und ggf. über eine lineare oder verzweigte C₁₋₃-Alkylen-Gruppe gebunden ist; oder für einen Aryl- oder Heteroaryl-Rest ausgewählt aus der Gruppe bestehend aus Phenyl, Thiophenyl, Furanyl und Pyridinyl, der jeweils unsubstituiert oder ggf. mit 1, 2, 3, 4 oder 5 Substituenten unabhängig voneinander ausgewählt aus der Gruppe bestehend aus F, Cl, Br, -CN, -CF₃, Methyl, Ethyl, n-Propyl, iso-Propyl, n-Butyl, sec-Butyl, iso-Butyl, tert-Butyl, -OH, -O-CH₃ und -O-C₂H₅ substituiert und ggf. über eine linear oder verzweigte C₁₋₃-Alkylen-Gruppe gebunden ist, steht,
besonders bevorzugt einer der beiden Reste R² und R³ für einen Wasserstoff-Rest oder für einen Alkyl-Rest ausgewählt aus der Gruppe bestehend aus Methyl; Ethyl; Propyl; iso-Propyl; n-Butyl; tert-Butyl; sec-Butyl; iso-Butyl; n-Pentyl; n-Hexyl; n-Heptyl; n-Octyl; (1,1,3,3)-Tetramethyl-butyl steht und der andere dieser beiden Reste für einen Wasserstoff-Rest; -C(=O)-R²⁰; -(CH₂)_{q}-C(=O)-R²¹ mit q = 1 oder 2; -(CH₂)ᵣ-C(=O)-O-R²³ mit r = 1 oder 2; für einen Alkyl-Rest ausgewählt aus der Gruppe bestehend aus Methyl; Ethyl; Propyl; iso-Propyl; n-Butyl; tert-Butyl; sec-Butyl; iso-Butyl; n-Pentyl; n-Hexyl; n-Heptyl; n-Octyl; (1,1,3,3)-Tetramethyl-butyl, (1,1)-Dimethyl-pentyl und (1,1)-Dimethyl-butyl; für einen unsubstituierten Cyclopentyl-oder Cyclohexyl-Rest, der jeweils über eine -(CH₂)-, -(CH₂)₂- oder -(CH₂)₃-Gruppe gebunden sein kann; oder für einen Phenyl-, 2-Pyridinyl-, 3-Pyridinyl-, 4-Pyridinyl-, 2-Furanyl-, 3-Furanyl-, 2-Thiophenyl- oder 3-Thiophenyl-Rest, der jeweils unsubstituiert oder ggf. mit 1, 2, 3, 4 oder 5 Substituenten unabhängig voneinander ausgewählt aus der Gruppe bestehend aus F, Cl, Br, -CN, -CF₃, Methyl, Ethyl, n-Propyl, iso-Propyl, tert-Butyl, -O-CH₃ und -O-C₂H₅ substituiert und ggf. über eine -(CH₂)-, -(CH₂)₂-, - C(H)(CH₃)- oder-(CH₂)₃-Gruppe gebunden ist, steht,
und jeweils die übrigen Reste A¹, A², A³, A⁴, R^{1a}, R^{1b}, R^{1c}, R^{1d}, R⁴ bis R²⁹, M¹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte Imidazo-3-yl-amin-Verbindungen der vorstehend angegebenen allgemeinen Formel I, worin R² und R³ zusammen mit dem sie verbindenden Stickstoffatom als Ringglied einen Rest bilden ausgewählt aus der Gruppe bestehend aus Imidazolidinyl, Piperidinyl, Piperazinyl, Morpholinyl, Pyrrolidinyl, Thiomorpholinyl, Azepanyl, Diazepanyl und Azocanyl, der jeweils unsubstituiert oder ggf. mit 1, 2, 3, 4 oder 5 Substituenten unabhängig voneinander ausgewählt aus der Gruppe bestehend aus F, Cl, Br, I, -CN, Methyl, Ethyl, n-Propyl, iso-Propyl, n-Butyl, sec-Butyl, iso-Butyl, tert-Butyl, -OH, Oxo, Thioxo, -O-CH₃, -O-C₂H₅, -O-C₃H₇, -NH₂, -N(CH₃)₂, -N(C₂H₅)₂, -NH-CH₃, -NH-C₂H₅, -O-CF₃, -S-CF₃, - S(=O)-CH₃, -S(=O)-C₂H₅, -S(=O)₂-CH₃, -S(=O)₂-C₂H₅, -C(=O)-OH, -C(=O)-H, -C(=O)-CH₃, -C(=O)-C₂H₅, -C(=O)-N(CH₃)₂, -C(=O)-NH-CH₃, -C(=O)-NH₂, -C(=O)-O-CH₃, - C(=O)-O-C₂H₅ und -C(=O)-O-C(CH₃)₃ substituiert sein kann,
vorzugsweise R² und R³ zusammen mit dem sie verbindenden Stickstoffatom als Ringglied einen Rest bilden ausgewählt aus der Gruppe bestehend aus Imidazolidinyl, Piperidinyl, Piperazinyl, Morpholinyl, Pyrrolidinyl, Thiomorpholinyl, Azepanyl, Diazepanyl und Azocanyl;
und jeweils die übrigen Reste A¹, A², A³, A⁴, R^{1a}, R^{1b}, R^{1c}, R^{1d}, R⁴ bis R²⁹, M¹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ferner sind substituierte Imidazo-3-yl-amin-Verbindungen der vorstehend angegebenen allgemeinen Formel I bevorzugt, worin R⁴, R⁵, R⁶, R⁷, R⁹, R¹⁰, R¹¹, R¹⁴ und R¹⁵, jeweils unabhängig voneinander, für einen linearen oder verzweigten C₁₋₄ Alkyl-Rest oder für einen unsubstituierten oder wenigstens einfach substituierten 5-bis 14-gliedrigen Aryl- oder Heteroaryl-Rest, der über eine lineare oder verzweigte C₁₋₅-Alkylen-Gruppe gebunden sein kann, stehen,
vorzugsweise R⁴, R⁵, R⁶, R⁷, R⁹, R¹⁰, R¹¹, R¹⁴ und R¹⁵, jeweils unabhängig voneinander, für einen Alkyl-Rest ausgewählt aus der Gruppe bestehend aus Methyl, Ethyl, n-Propyl, iso-Propyl, n-Butyl, iso-Butyl, sec-Butyl und tert-Butyl, oder für einen unsubstituierten oder wenigstens einfach substituierten 5- oder 6-gliedrigen Aryl- oder Heteroaryl-Rest stehen, der über eine lineare oder verzweigte C₁₋₃-Alkylen-Gruppe gebunden sein kann, stehen,
besonders bevorzugt R⁴, R⁵, R⁶, R⁷, R⁹, R¹⁰, R¹¹, R¹⁴ und R¹⁵, jeweils unabhängig voneinander, für einen Alkyl-Rest ausgewählt aus der Gruppe bestehend aus Methyl, Ethyl, n-Propyl und iso-Propyl, oder für einen Phenyl-Rest, der jeweils unsubstituiert oder mit 1, 2 oder 3 Substituenten unabhängig voneinander ausgewählt aus der Gruppe bestehend aus F, Cl, Br, -CN, -CF₃, -SF₅, Methyl, Ethyl, n-Propyl, iso-Propyl, tert-Butyl, -O-CH₃ und -O-C₂H₅ substituiert und ggf. über eine -(CH₂)-, -(CH₂)₂- oder -(CH₂)₃-Gruppe gebunden ist, stehen,
und jeweils die übrigen Reste A¹, A², A³, A⁴, R^{1a}, R^{1b}, R^{1c}, R^{1d}, R², R³, R⁸, R¹², R¹³ und R¹⁶ bis R²⁹, M¹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte Imidazo-3-yl-amin-Verbindungen der vorstehend angegebenen allgemeinen Formel I, worin R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ und R²⁹, jeweils unabhängig voneinander, für einen Wasserstoff-Rest; für einen linearen oder verzweigten C₁₋₈-Alkyl-Rest, oder für einen unsubstituierten oder wenigstens einfach substituierten 5- bis 14-gliedrigen Aryl- oder Heteroaryl-Rest, der über eine lineare oder verzweigte C₁₋₅-Alkylen-Gruppe gebunden sein kann, stehen,
vorzugsweise R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ und R²⁹, jeweils unabhängig voneinander, für einen Wasserstoff-Rest; für einen linearen oder verzweigten C₁₋₄-Alkyl-Rest, oder für einen unsubstituierten oder wenigstens einfach substituierten 5- oder 6-gliedrigen Aryl- oder Heteroaryl-Rest, der über eine lineare oder verzweigte C₁₋₃-Alkylen-Gruppe gebunden sein kann, stehen,
besonders bevorzugt R⁸, R¹², R¹³, R¹⁶, R¹⁷, R¹⁸, R¹⁹, R²⁰, R²¹, R²², R²³, R²⁴, R²⁵, R²⁸, R²⁷, R²⁸ und R²⁹, jeweils unabhängig voneinander, für einen Wasserstoff-Rest; für einen Alkyl-Rest ausgewählt aus der Gruppe bestehend aus Methyl, Ethyl, n-Propyr, iso-Propyl und tert-Butyl, oder für einen Phenyl-Rest, der jeweils unsubstituiert oder mit 1, 2 oder 3 Substituenten unabhängig voneinander ausgewählt aus der Gruppe bestehend aus F, Cl, Br, -CN, -CF₃, -SF₅, Methyl, Ethyl, n-Propyl, iso-Propyl, tert-Butyl, -O-CH₃ und -O-C₂H₅ substituiert und ggf. über eine -(CH₂)-, -(CH₂)₂- oder - (CH₂)₃-Gruppe gebunden ist, stehen,
und jeweils die übrigen Reste A¹, A², A³, A⁴, R^{1a}, R^{1b}, R^{1c}, R^{1d}, R² bis R⁷, R⁹ bis R¹¹, R¹⁴ und R¹⁵, M¹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte Imidazo-3-yl-amin-Verbindungen der vorstehend angegebenen allgemeinen Formel I, worin M¹ für einen 5- oder 6-gliedrigen Aryl- oder Heteroaryl-Rest steht, der mit wenigstens einem weiteren Substituenten substituiert sein kann, wobei der Heteroaryl-Rest ggf. 1, 2, 3, 4 oder 5 Heteroatom(e) als Ringglied(er) aufweist, die unabhängig voneinander ausgewählt sind aus der Gruppe bestehend aus Stickstoff, Sauerstoff und Schwefel,

vorzugsweise M¹ für einen Aryl- oder Heteroaryl-Rest ausgewählt aus der Gruppe bestehend aus Phenyl, Furanyl, Thiophenyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Imidazolyl, Oxadiazolyl, Triazolyl, Diazinyl, Triazinyl, Tetrazinyl und Tetrazolyl steht, der mit wenigstens einem weiteren Substituenten substituiert sein kann, steht,
besonders bevorzugt für einen Rest ausgewählt aus der Gruppe bestehend aus den Resten 1 bis 38 steht, 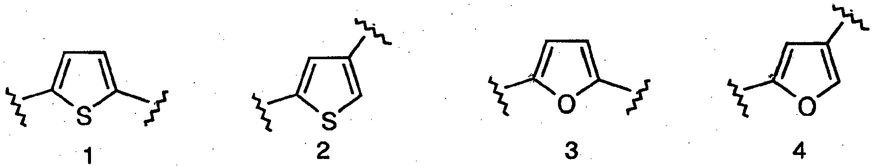 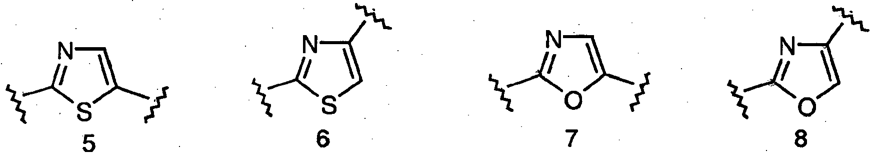 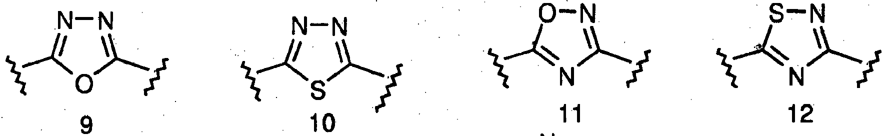 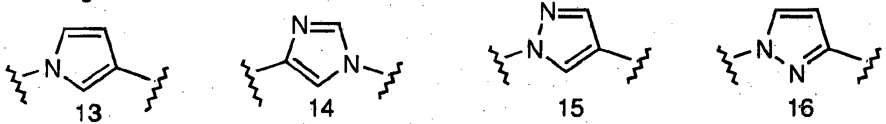 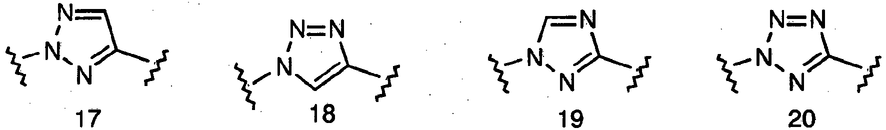 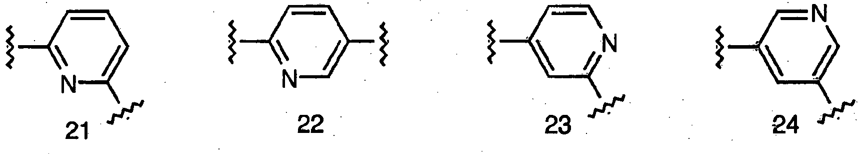 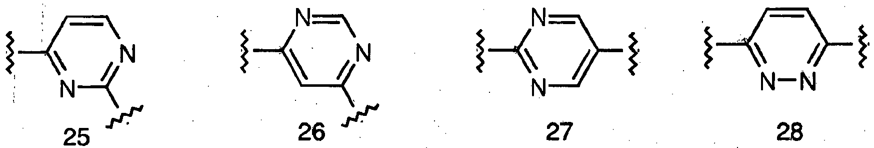 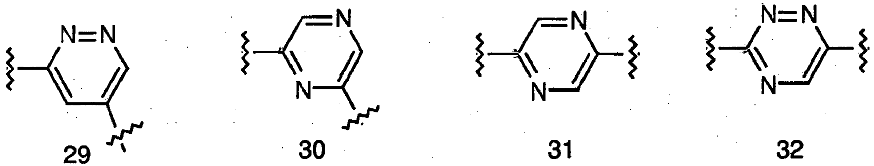 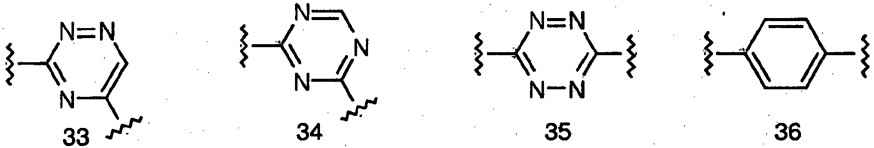 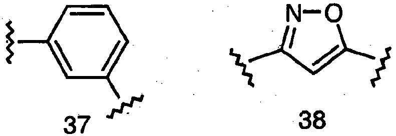 der in beliebiger Richtung an den gekennzeichneten Positionen im Grundgerüst gebunden und mit wenigstens einem weiteren Substituenten subsituiert sein kann,
ganz besonders bevorzugt für einen Rest ausgewählt aus der Gruppe bestehend aus den Resten 1 bis 38 steht, 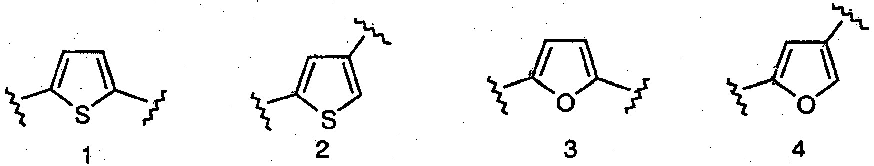 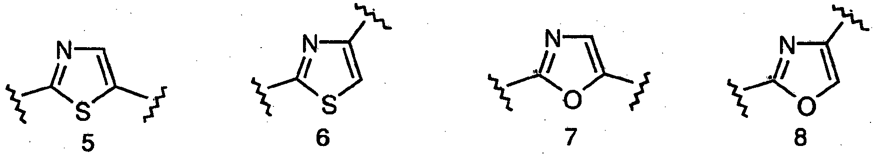 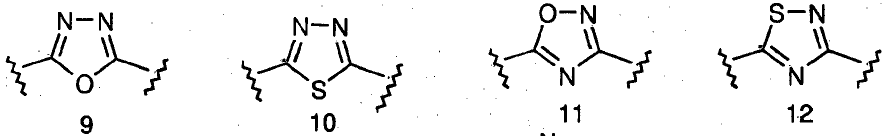 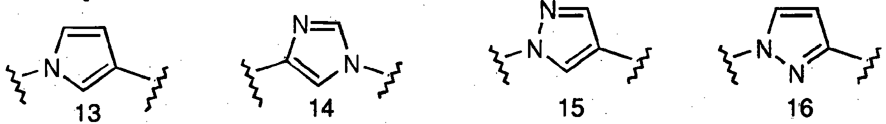 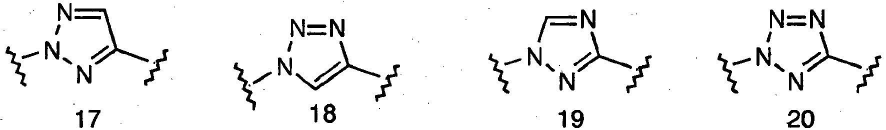 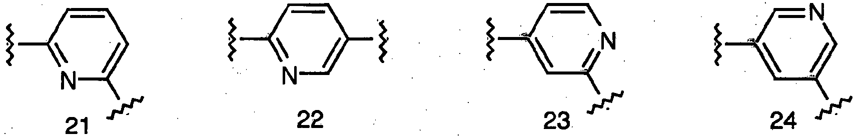 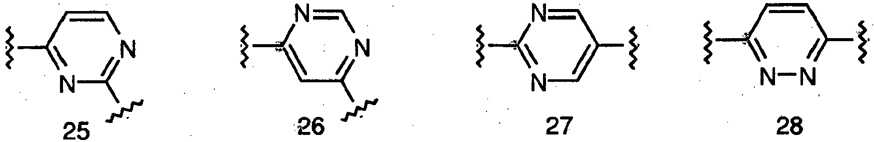 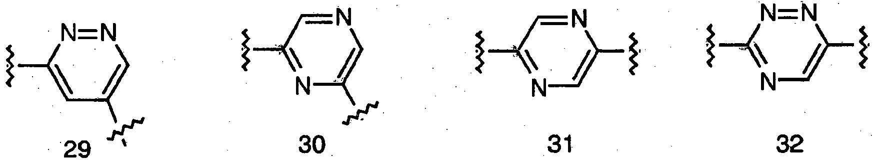 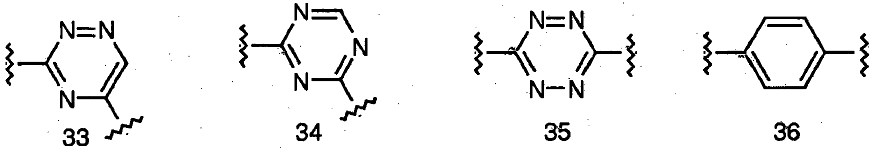 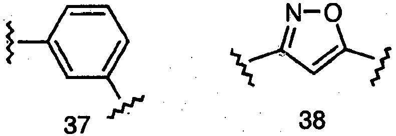 der in beliebiger Richtung an den gekennzeichneten Positionen im Grundgerüst gebunden sein kann und ggf. mit 1, 2, 3 oder 4 weiteren Substituenten substituiert ist, die unabhängig voneinander ausgewählt werden können aus der Gruppe bestehend aus F, Cl, Br, -CN, -CH₂-CN, -CF₃, -SF₅, Methyl, methyl, n-Propyl, iso-Propyl, n-Butyl, sec-Butyl, iso-Butyl, tert-Butyl, -OH, -O-CH₃, -O-C₂H₅, -O-C₃H₇, -NH₂, -N(CH₃)₂, -N(C₂H₅)₂, -NO₂, -O-CF₃, -S-CF₃, -SH, -C(=O)-CH₃ und -C(=O)-C₂H₅,
noch weiter bevorzugt für einen Rest ausgewählt aus der Gruppe bestehend aus den Resten 1 bis 9, 11, 21, 22 und 36 bis 38 steht, 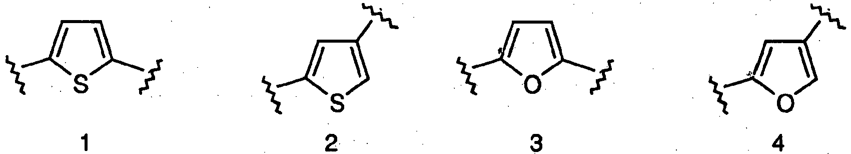 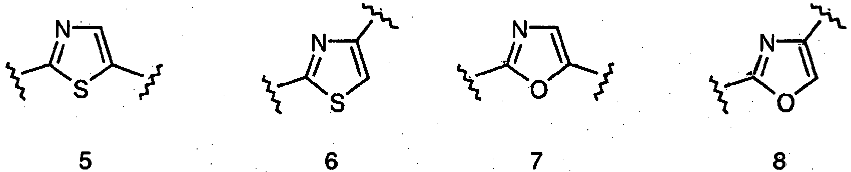 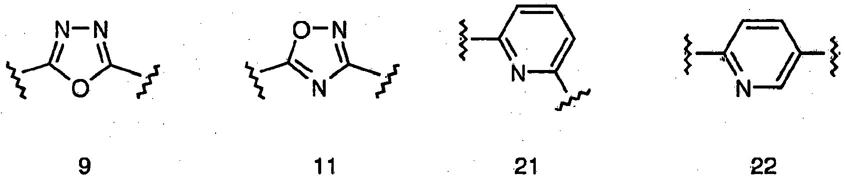 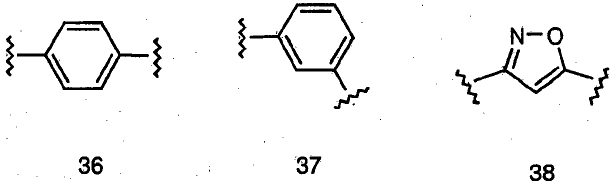 der in beliebiger Richtung an den gekennzeichneten Positionen im Grundgerüst gebunden sein kann und ggf. mit 1, 2, 3 oder 4 weiteren Substituenten substituiert ist, die unabhängig voneinander ausgewählt werden können aus der Gruppe bestehend aus F, Cl, Br, -CN, -CH₂-CN, -CF₃, -SF₅, Methyl, Ethyl, n-Prflpyl, iso-Propyl, n-Butyl, sec-Butyl, iso-Butyl, tert-Butyl, -OH, -O-CH₃, -O-C₂H₅, -O-C₃H₇, -NH₂, -N(CH₃)₂, -N(C₂H₅)₂, -NO₂, -O-CF₃, -S-CF₃, -SH, -C(=O)-CH₃ und -C(=O)-C₂H₅, am meisten bevorzugt für einen Rest ausgewählt aus der Gruppe bestehend aus den Resten 1 bis 9, 11, 21, 22 und 36 bis 38 steht, 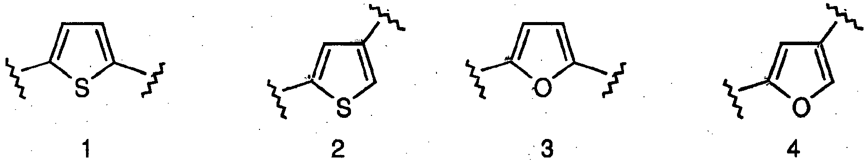 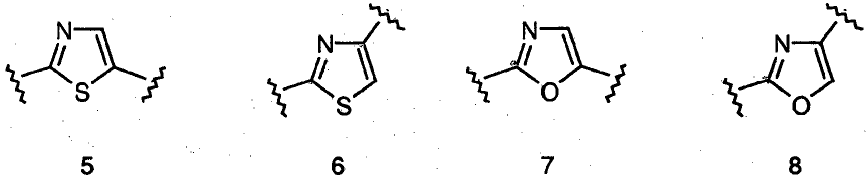 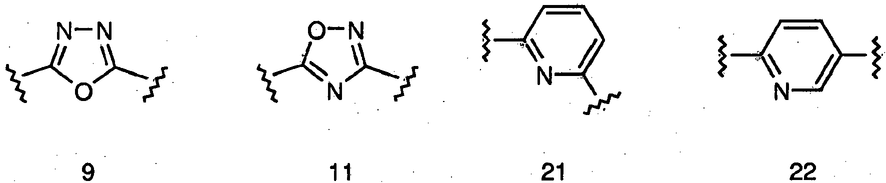 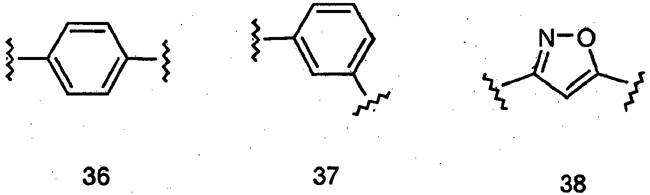 der in beliebiger Richtung an den gekennzeichneten Positionen im Grundgerüst gebunden sein kann und ggf. mit 1 oder 2 weiteren Substituenten substituiert ist, die unabhängig voneinander ausgewählt werden können aus der Gruppe bestehend aus F, Cl, Br, -CN, -CH₂-CN, -CF₃, -SF₅, Methyl, Ethyl, n-Propyl, iso-Propyl, tert-Butyl, -O-CH₃ und -O-CF₃,
und jeweils die übrigen Reste A¹, A², A³, A⁴, R^{1a}, R^{1b}, R^{1c}, R^{1d}, R² bis R²⁹ und M² die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ferner bevorzugt sind substituierte Imidazo-3-yl-amin-Verbindungen der vorstehend angegebenen allgemeinen Formel I, worin M² für einen unsubstituierten oder wenigstens einfach substituierten 5- oder 6-gliedrigen Aryl- oder Heteroaryl-Rest steht, wobei der Heteroaryl-Rest 1, 2, 3, 4 oder 5 Heteroatom(e) als Ringglied(er) aufweist, die unabhängig voneinander ausgewählt sind aus der Gruppe bestehend aus Stickstoff, Sauerstoff und Schwefel, und wobei der Aryl- oder Heteroaryl-Rest mit einem unsubsituierten oder wenigstens einfach substituierten mono- oder bizyklischem Ringsystem kondensiert sein kann, wobei die Ringe des Ringsystems jeweils 5- oder 6-gliedrig sind und jeweils 1, 2, 3 oder 4 Heteroatom(e) als Ringglied(er) aufweisen können, die unabhängig voneinander ausgewählt sind aus der Gruppe bestehend aus Stickstoff, Sauerstoff und Schwefel,
bevorzugt M² für einen Rest ausgewählt aus der Gruppe bestehend aus Phenyl, Furanyl, Thiophenyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Diazinyl, Triazinyl, Tetrazinyl, Tetrazolyl, Pentazolyl, Imidazolyl, Chinolinyl, Isochinolinyl, Naphthyl, Indolyl, Isoindolyl, Benzofuranyl, Isobenzofuranyl, Benzothiophenyl und Isobenzothiophenyl steht, wobei der Rest jeweils unsubstituiert oder wenigstens einfach substituiert sein kann,
besonders bevorzugt M² für einen Rest ausgewählt aus der Gruppe bestehend aus Phenyl, Furanyl, Thiophenyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Diazinyl, Triazinyl, Tetrazinyl, Tetrazolyl, Pentazolyl, Imidazolyl, Chinolinyl, Isochinolinyl, Naphthyl, Indolyl, Isoindolyl, Benzofuranyl, Isobenzofuranyl, Benzothiophenyl und Isobenzothiophenyl steht, wobei der jeweilige Rest unsubstituiert oder ggf. mit 1, 2, 3, 4 oder 5 Substituenten substituiert sein kann, die unabhängig voneinander ausgewählt sind aus der Gruppe bestehend aus F, Cl, Br, -CN, -CF₃, -SF₅, S-CH₃, - S-C₂H₅, Methyl, Ethyl, n-Propyl, iso-Propyl, n-Butyl, sec-Butyl, iso-Butyl, tert-Butyl, Ethenyl, Propenyl, -OH, -O-CH₃, -O-C₂H₅, -O-C₃H₇, -NH₂, -N(CH₃)₂, -NH-CH₃, -CH₂-NH₂, -N(C₂H₅)₂, -NO₂, -O-CF₃, -C(=O)-H, -S-CF₃, -SH, -C(=O)-O-CH₃, -C(=O)-O-C₂H₅, Phenyl, Pyrrolyl, (1,3)-Dioxolanyl, -Si(Phenyl)₂[C(CH₃)₃], -C(=O)-CH₃, -C(=O)-C₂H₅, NH-S(=O)₂-CH₃, -NH-S(=O)₂-C₂H₅, -S(=O)₂-NH₂, -S(=O)₂-NH-CH₃, -CH₂-OH, - C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂;
ganz besonders bevorzugt M² für einen Rest ausgewählt aus der Gruppe bestehend aus den Resten 1 bis 36 steht, 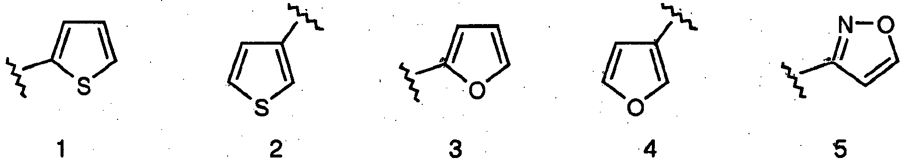 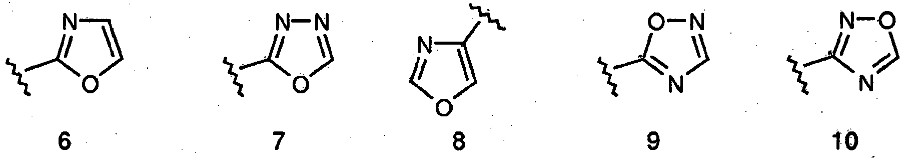 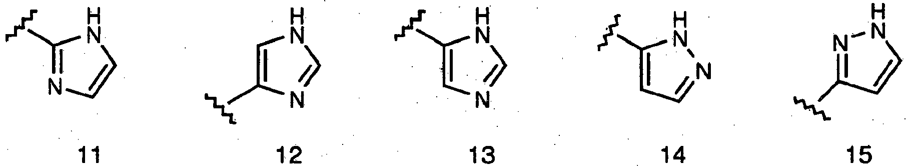 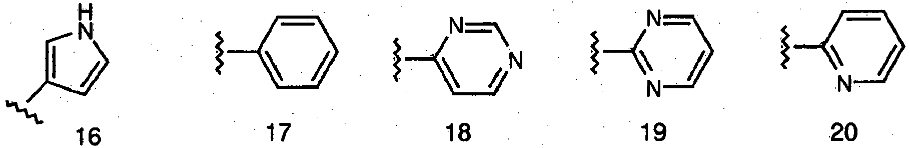 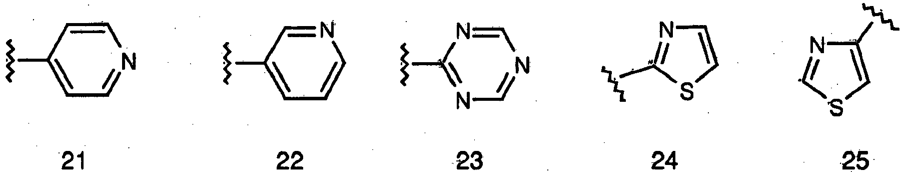 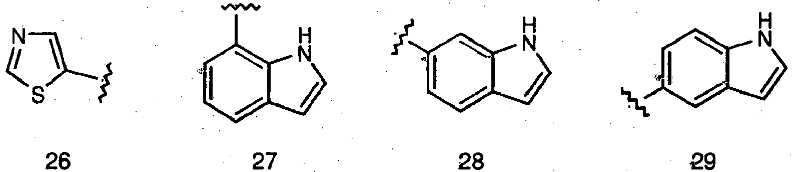 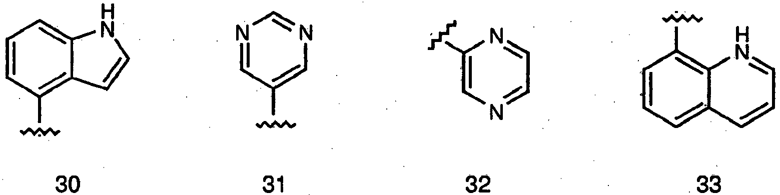 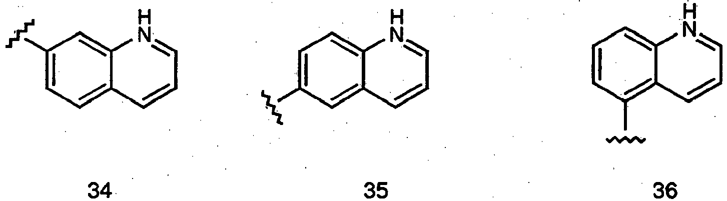 der an der gekennzeichneten Stelle im Grundgerüst gebunden ist, wobei der jeweilige Rest unsubstituiert oder ggf. mit 1, 2, 3, 4 oder 5 Substituenten substituiert sein kann, die unabhängig voneinander ausgewählt sind aus der-Gruppe bestehend aus F, Cl, Br, -CN, -CF₃, -SF₅, -S-CH₃, -S-C₂H₅, Methyl, Ethyl, n-Propyl, iso-Propyl, n-Butyl, sec-Butyl, iso-Butyl, tert-Butyl, Ethenyl, Propenyl, -OH, -O-CH₃, -O-C₂H₅, -O-C₃H₇, -NH₂, -N(CH₃)₂, -NH-CH₃, -CH₂-NH₂, -N(C₂H₅)₂, -NO₂, -O-CF₃, -S-CF₃, -SH, - C(=O)-H, -C(=O)-O-CH₃, -C(=O)-O-C₂H₅, Phenyl, Pyrrolyl, (1,3)-Dioxolanyl, Si(Phenyl)₂[C(CH₃)₃], -C(=O)-CH₃, -C(=O)-C₂H₅, NH-S(=O)₂-CH₃, -NH-S(=O)₂-C₂H₅, -S(=O)₂-NH₂, -S(=O)₂-NH-CH₃, -CH₂-OH, -C(=O)-OH, -CH₂-O-CH₃, -C(=O)-NH₂, - C(=O)-NH-CH₃, -NH-C(=O)-CH₃, -NH-C(=NH)-NH₂, -NH-S(=O)₂-OH und -S(=O)₂-N(CH₃)₂;
am meisten bevorzugt M² für einen Rest ausgewählt aus der Gruppe bestehend aus den Resten 1 bis 36 steht, 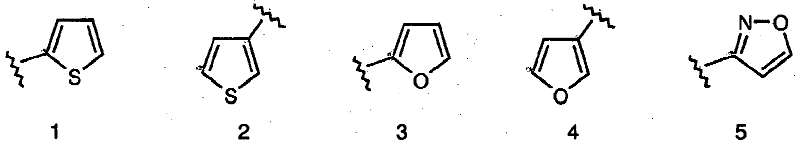 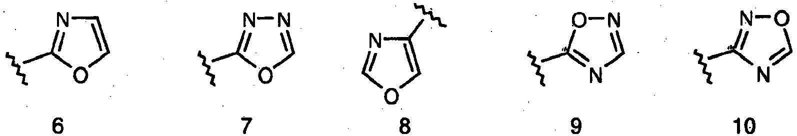 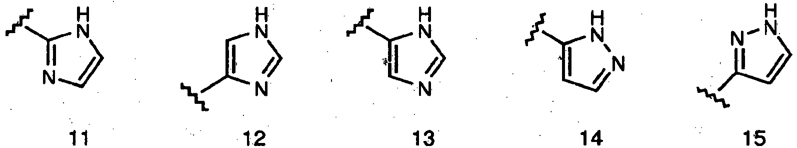 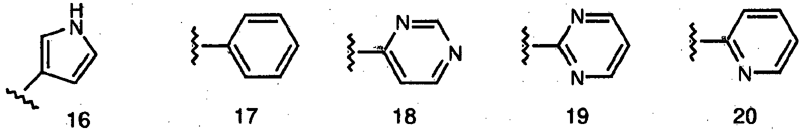 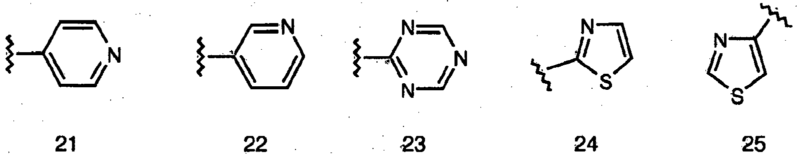 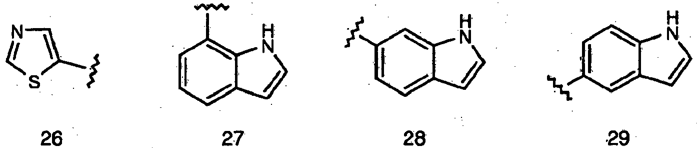 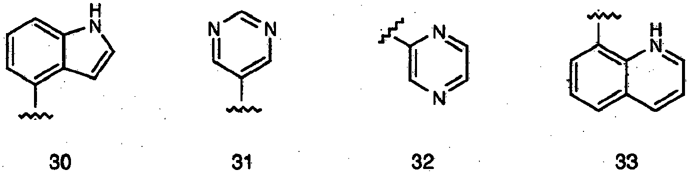 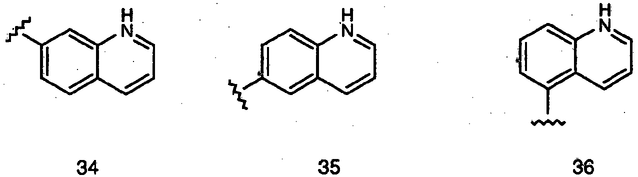 der an der gekennzeichneten Stelle im Grundgerüst gebunden ist, wobei der jeweilige Rest unsubstituiert oder mit 1, 2 oder 3 Substituenten substituiert sein kann, die unabhängig voneinander ausgewählt sind aus der Gruppe bestehend aus F, Cl, Br, -CN, -CF₃, -SF₅, -S-CH₃, -S-C₂H₅, Methyl, Ethyl, iso-Propyl, tert-Butyl, Ethenyl, Propenyl, -O-CH₃, -O-C₂H₅, -NH₂, -N(CH₃)₂, -NH-CH₃, -CH₂-NH₂, -O-CF₃, -C(=O)-H, - C(=O)-O-CH₃, -C(=O)-O-C₂H₅, Phenyl, Pyrrolyl, (1,3)-Dioxolanyl, - Si(Phenyl)₂(C(CH₃)₃)], -NO₂, NH-S(=O)₂-CH₃, -NH-S(=O)₂-C₂H₅, -S(=O)₂-NH₂, - S(=O)₂-NH-CH₃, -CH₂-OH, -C(=O)-CH₃, -C(=O)-OH, -CH₂-O-CH₃, -C(=O)-NH₂, - C(=O)-NH-CH₃, -NH-C(=O)-CH₃, -NH-C(=NH)-NH₂, -NH-S(=O)₂-OH und -S(=O)₂- N(CH₃)₂;
und jeweils die übrigen Reste A¹, A², A³, A⁴, R^{1a}, R^{1b}, R^{1c}, R^{1d}, R² bis R²⁹ und M¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind substituierte Imidazo-3-yl-amin-Verbindungen der allgemeinen Formel I,
A¹ für eine C-R^{1a}-Gruppe steht,
A² für eine C-R^{1b}-Gruppe steht,
A³ für eine C-R^{1c}-Gruppe steht und
A⁴ für eine C-R^{1d}-Gruppe steht;
oder
A¹ für ein Stickstoffatom steht,
A² für eine C-R^{1b}-Gruppe steht,
A³ für eine C-R^{1c}-Gruppe steht und
A⁴ für eine C-R^{1d}-Gruppe steht;
oder
A¹ für eine C-R^{1a}-Gruppe steht,
A² für ein Stickstoffatom steht,
A³ für eine C-R^{1c}-Gruppe steht und
A⁴ für eine C-R^{1d}-Gruppe steht:
   R^{1a} R^{1b} R^{1c} R^{1d} unabhängig voneinander, jeweils für -H; -F; -Cl, -Br; -I; -NO₂; -CN; - CF₃; -SF₅; -NH₂; -S(=O)-NH₂,-NHR⁴; -NR⁵R⁶; -C(=O)-OR¹²; -(CH₂)ₘ-C(=O)-OR¹³ mit m = 1, 2 oder 3; -O-C(=O)-R¹⁴; -OR¹⁶; -(CH₂)ₒ-O-R¹⁷ mit o = 1, 2 oder 3; für einen linearen oder verzweigten Alkyl-Rest ausgewählt aus der Gruppe bestehend aus Methyl, Ethyl, n-Propyl, iso-Propyl, n-Butyl, iso-Butyl, sec-Butyl und tert-Butyl, oder für einen Aryl- oder Heteroaryl-Rest ausgewählt aus der Gruppe bestehend aus Phenyl, Furanyl, Thiophenyl und Pyridinyl, der jeweils unsubstituiert oder mit 1, 2, 3, 4 oder 5 Substituenten unabhängig voneinander ausgewählt aus der Gruppe bestehend aus F, Cl, Br, -CN, Methyl, Ethyl, n-Propyl, iso-Propyl, n-Butyl, sec-Butyl, iso-Butyl, tert-Butyl, -OH, -O-CH₃, -O-C₂H₅ substituiert und ggf. über eine -(CH₂)-, - (CH₂)₂- oder -(CH₂)₃-Gruppe gebunden ist, stehen;
   oder ggf. R^{1a} und R^{1b} oder ggf. R^{1b} und R^{1c} oder ggf. R^{1c} und R^{1d} zusammen mit der sie verbindenden C-C-Brücke einen annelierten Phenyl-Rest bilden, der mit 1, 2, 3 oder 4 Substituenten unabhängig voneinander ausgewählt aus der Gruppe bestehend aus F, Cl, Br, -CN, Methyl, Ethyl, n-Propyl, iso-Propyl, n-Butyl, sec-Butyt, iso-Butyl, tert-Butyl, -OH, -O-CH₃, -O-C₂H₅ substituiert sein kann;
   R² und R³, unabhängig voneinander, jeweils für einen Wasserstoff-Rest; -C(=O)-R²⁰; -(CH₂)_{q}-C(=O)-R²¹ mit q = 1, 2 oder 3; -(CH₂)ᵣ-C(=O)-O-R²³ mit r = 1, 2 oder 3; für einen linearen oder verzweigten C₁₋₁₀ Alkyl-Rest; für einen C₄₋₈ Cycloalkyl-Rest, der jeweils unsubstituiert oder mit 1, 2, 3, 4 oder 5 Substituenten unabhängig voneinander ausgewählt aus der Gruppe bestehend aus F, Cl, Br, -CN, -CF₃, Methyl, Ethyl, n-Propyl, iso-Propyl, n-Butyl, sec-Butyl, iso-Butyl, tert-Butyl, -OH, -O-CH₃ und - O-C₂H₅ substituiert und ggf. über eine lineare oder verzweigte-C₁₋₃-Alkylen-Gruppe gebunden ist; oder für einen Aryl- oder Heteroaryl-Rest ausgewählt aus der Gruppe bestehend aus Phenyl, Thiophenyl, Furanyl und Pyridinyl, der jeweils unsubstituiert oder ggf. mit 1, 2, 3, 4 oder 5 Substituenten unabhängig voneinander ausgewählt aus der Gruppe bestehend aus F, Cl, Br, -CN, -CF₃, Methyl, Ethyl, n-Propyl, iso-Propyl, n-Butyl, sec-Butyl, iso-Butyl, tert-Butyl, -OH, -O-CH₃ und -O-C₂H₅ substituiert und ggf. über eine lineare oder verzweigte C₁₋₃-Alkylen-Gruppe gebunden ist, stehen;
   oder R² und R³ zusammen mit dem sie verbindenden Stickstoffatom als Ringglied einen Rest bilden ausgewählt aus der Gruppe bestehend aus Imidazolidinyl, Piperidinyl, Piperazinyl, Morpholinyl, Pyrrolidinyl, Thiomorpholinyl, Azepanyl, Diazepanyl und Azocanyl, der jeweils unsubstituiert oder ggf. mit 1, 2, 3, 4 oder 5 Substituenten unabhängig voneinander ausgewählt aus der Gruppe bestehend aus F, Cl, Br, I, -CN, Methyl, Ethyl, n-Propyl, iso-Propyl, n-Butyl, sec-Butyl, iso-Butyl, tert-Butyl, -OH, Oxo, Thioxo, -O-CH₃, -O-C₂H₅, -O-C₃H₇, -NH₂, -N(CH₃)₂, -N(C₂H₅)₂, -NH-CH₃, -NH-C₂H₅, -O-CF₃, -S-CF₃, -S(=O)-CH₃, -S(=O)-C₂H₅, -S(=O)₂-CH₃, -S(=O)₂-C₂H₅, -C(=O)-OH, -C(=O)-H, -C(=O)-CH₃, -C(=O)-C₂H₅, -C(=O)-N(CH₃)₂, -C(=O)-NH-CH₃, -C(=O)-NH₂, -C(=O)-O-CH₃, -C(=O)-O-C₂H₅ und -C(=O)-O-C(CH₃)₃ substituiert sein kann;
   R⁴, R⁵, R⁶ und R¹⁴, jeweils unabhängig voneinander, für einen Alkyl-Rest ausgewählt aus der Gruppe bestehend aus Methyl, Ethyl, n-Propyl und iso-Propyl, oder für einen Phenyl-Rest, der jeweils unsubstituiert oder mit 1, 2 oder 3 Substituenten unabhängig voneinander ausgewählt aus der Gruppe bestehend aus F, Cl, Br, -CN, - CF₃, -SF₅, Methyl, Ethyl, n-Propyl, iso-Propyl, tert-Butyl, =O-CH₃ und -O-C₂H₅ substituiert und ggf. über eine -(CH₂)-, -(CH₂)₂- oder -(CH₂)₃-Gruppe gebunden ist, stehen;
   R¹², R¹³, R¹⁶, R¹⁷, R²⁰, R²¹ und R²³, jeweils unabhängig voneinander, für einen Wasserstoff-Rest; für einen Alkyl-Rest ausgewählt aus der Gruppe bestehend aus Methyl, Ethyl, n-Propyl, iso-Propyl und tert-Butyl, oder für einen Phenyl-Rest, der jeweils unsubstituiert oder mit 1, 2 oder 3 Substituenten unabhängig voneinander ausgewählt aus der Gruppe bestehend aus F, Cl, Br, -CN, -CF₃, -SF₅, Methyl, Ethyl, n-Propyl, iso-Propyl, tert-Butyl, -O-CH₃ und -O-C₂H₅ substituiert und ggf. über eine - (CH₂)-, -(CH₂)₂- oder-(CH₂)₃-Gruppe gebunden ist, stehen;
   M¹ für einen Rest ausgewählt aus der Gruppe bestehend aus den Resten 1 bis 9, 11, 21, 22 und 36 bis 38 steht, 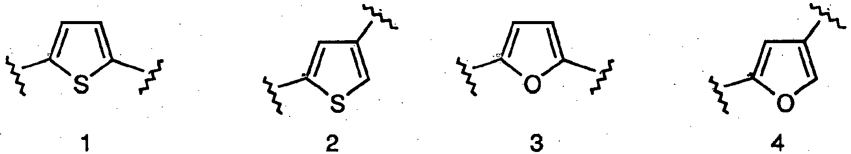 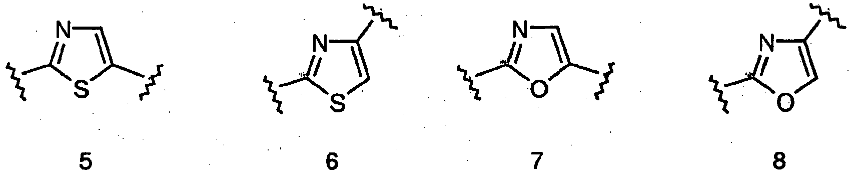 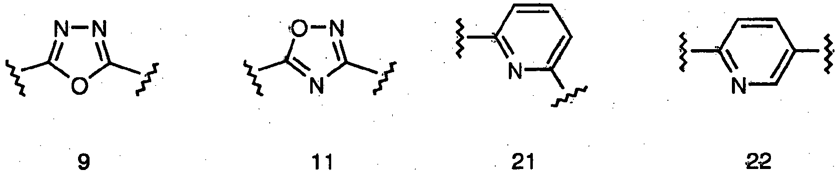 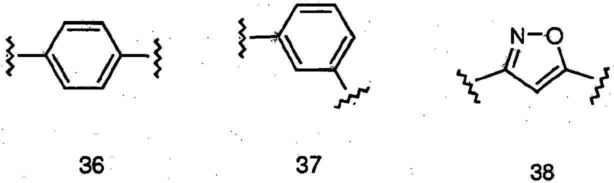
   der jeweils in beliebiger Richtung über die mit einer geschlängelten Linie gekennzeichneten Positionen mit dem Bizyklus und dem Kohlenstoffatom der Dreifachbindung verknüpft sein kann und ggf. mit 1 oder 2 weiteren Substituenten substituiert ist, die unabhängig voneinander ausgewählt sind aus der Gruppe bestehend aus F, Cl, Br, -CN, -CH₂-CH, -CF₃, -SF₅, Methyl, Ethyl, n-Propyl, iso-Propyl, n-Butyl, iso-Butyl, sec-Butyl, tert-Butyl, -O-CH₃ und -O-CF_{3;}
   und
   M² für einen Rest ausgewählt aus der Gruppe bestehend aus den Resten 1 bis 36 steht, 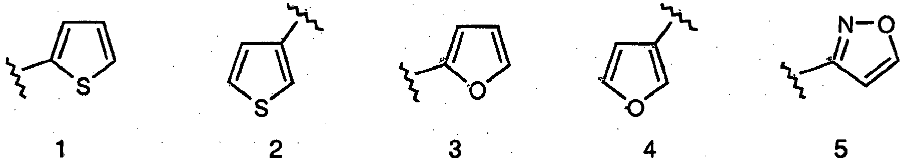 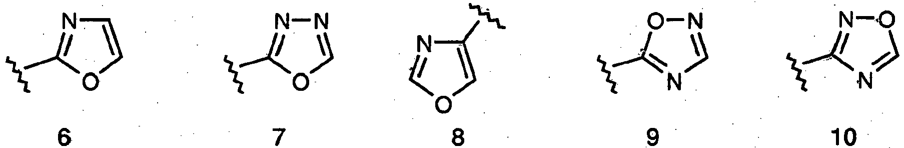 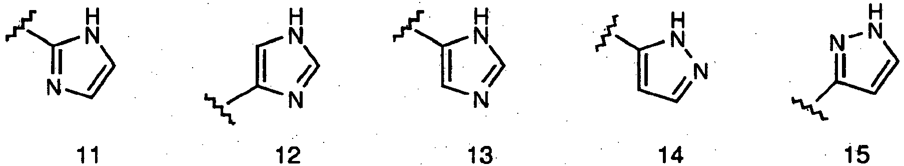 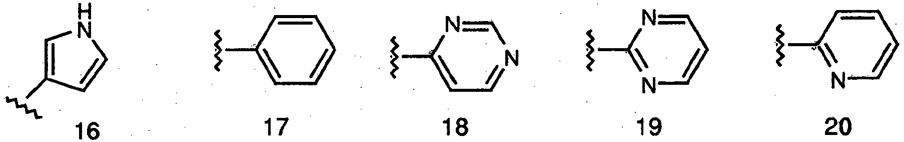 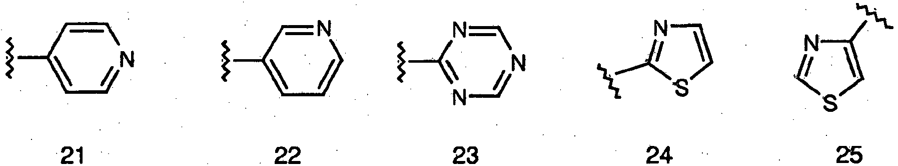 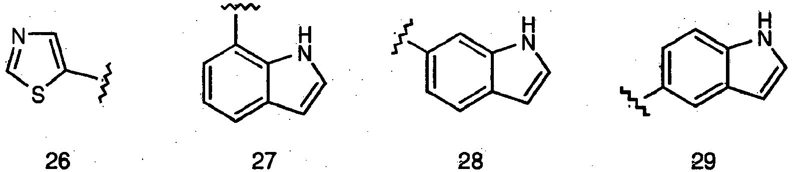 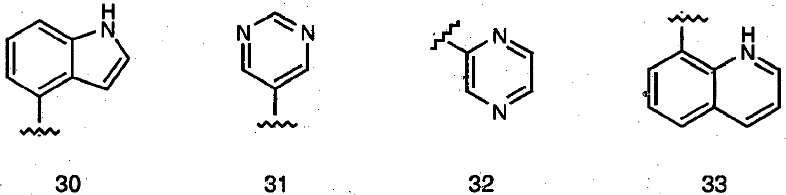 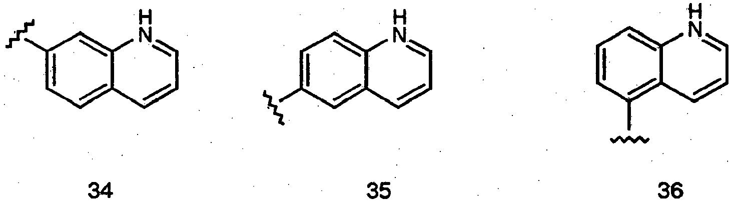
   der jeweils über die mit einer geschlängelten Linie gekennzeichnete Position mit dem Kohlenstoffatom der Dreifachbindung verknüpft und unsubstituiert oder ggf. mit 1, 2 oder 3 Substituenten substituiert ist, die unabhängig voneinander ausgewählt sind aus der Gruppe bestehend aus F, Cl, Br, -CN, -CF₃, -SF₅, -S-CH₃, -S-C₂H₅, Methyl, Ethyl, n-Propyl, iso-Propyl, n-Butyt, tert-Butyl, Ethenyl, Propenyl, -O-CH₃, -O-C₂H₅, - NH₂, -N(CH₃)₂, -NH-CH₃, -CH₂-NH₂, -O-CF₃, -C(=O)-H, -O(=O)-O-CH₃, -C(=O)-O-C₂H₅, Phenyl, Pyrrolyl, (1,3)-Dioxolanyl, -Si(Phenyl)₂[C(CH₃)₃], -NO₂, NH-S(=O)₂-CH₃, -NH-S(=O)₂-C₂H₅, -S(=O)₂-NH₂, -S(=O)₂-NH-CH₃, -CH₂-OH, -C(=O)-CH₃, - C(=O)-OH, -CH₂-O-CH₃, -C(=O)-NH₂, -C(=O)-NH-CH₃, -NH-C(=O)-CH₃, -S(=O)₂-N(CH₃)₂, -NH-S(=O)₂-OH und -NH₂(=NH)-NH₂;
   jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte Imidazo-3-yl-amin-Verbindungen der allgemeinen Formel I, 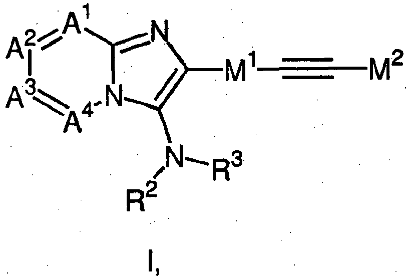 worin
A¹ für eine C-R^{1a}-Gruppe steht,
A² für eine C-R^{1b}-Gruppe steht,
A³ für eine C-R^{1c}-Gruppe steht und
A⁴ für eine C-R^{1d}-Gruppe steht,
oder
A¹ für ein Stickstoffatom steht,
A² für eine C-R^{1b}-Gruppe steht,
A³ für eine C-R^{1c}-Gruppe steht und
A⁴ für eine C-R^{1d}-Gruppe steht,
oder
A' für eine C-R¹⁸-Gruppe steht,
A² für ein Stickstoffatom steht,
A³ für eine C-R^{1c}-Gruppe steht und
A⁴ für eine C-R^{1d}-Gruppe steht,
R¹⁸, R^{1b}, R^{1c}, R^{1d}, jeweils unabhängig voneinander, für einen Wasserstoff-Rest; - OR¹⁶; -F; .Cl, -Br; -CF₃, -CN; -S(=O)₂-NH₂; -C(=O)-OR¹², einen Alkyl-Rest ausgewählt aus der Gruppe bestehend aus Methyl, Ethyl, n-Propyl, iso-Propyl, n-Butyl, sec-Butyl, iso-Butyl und tert-Butyl; für einen Phenyl-Rest; einen Benzyl-Rest; einen Phenethyl-Rest oder für einen (3-Phenyl)-prop-1-yl-Rest stehen,
oder ggf. R¹⁸ und R^{1b} zusammen mit der sie verbindenden C-C-Brücke einen unsubstituierten annelierten Phenyl-Rest bilden;
oder ggf. R^{1c} und R^{1d} zusammen mit der sie verbindenden C-C-Brücke einen unsubstituierten annelierten Phenyl-Rest bilden;
R² und R³, unabhängig voneinander, jeweils für einen Wasserstoff-Rest; -C(=O)-R²⁰; -(CH₂)_{q}-C(=O)-R²¹ mit q = 1; -(CH₂)ᵣ-C(=O)-O-R²³ mit r = 1; für einen Alkylrest ausgewählt aus der Gruppe bestehend aus Methyl; Ethyl; Propyl; iso-Propyl; n-Butyl; tert-Butyl; sec-Butyl; iso-Butyl; n-Pentyl; n-Hexyl; n-Heptyl; n-Octyl; (1,1,3,3)-Tetramethyl-butyl; (1,1)-Dimethyl-pentyl und (1,2)-Dimethylbutyl; für einen unsubstituierten Cyclopentyl- oder Cyclohexyl-Rest, der jeweils über eine -(CH₂)-, - (CH₂)₂- oder -(CH₂)₃-Gruppe gebunden sein kann; oder für einen Phenyl-, Pyridinyl-, Thiophenyl- oder Furanyl-Rest, der jeweils unsubstituiert oder ggf. mit 1, 2, 3, 4 oder 5 Substituenten unabhängig voneinander ausgewählt aus der Gruppe bestehend aus F, Cl, Br, -CN, -CF₃, Methyl, Ethyl, n-Propyl, iso-Propyl, n-Butyl, sec-Butyl, iso-Butyl, tert-Butyl, -O-CH₃ und -O-C₂H₅ substituiert und ggf. über eine -(CH₂)-, -(CH₂)₂-, - (CH(CH₃))- oder-(CH₂)₃-Gruppe gebunden ist, stehen,
oder R² und R³ zusammen mit dem sie verbindenden Stickstoffatom als Ringglied einen heterocycloaliphatischen Rest bilden ausgewählt aus der Gruppe bestehend aus Pyrrolidinyll, Piperidinyl, Piperazinyl und Morpholinyl,
R¹² für einen Wasserstoff-Rest oder für einen Alkyl-Rest ausgewählt aus der Gruppe bestehend aus Methyl, Ethyl, n-Propyl, iso-Propyl und tert-Butyl steht;
R¹⁶, R²⁰, R²¹ und R²³, jeweils unabhängig voneinander für einen Alkyl-Rest ausgewählt aus der Gruppe bestehend aus Methyl, Ethyl, n-Propyl, iso-Propyl und tert-Butyl, oder für einen Phenyl-Rest, der jeweils unsubstituiert oder mit 1 oder 2 Substituenten unabhängig voneinander ausgewählt aus der Gruppe bestehend aus F, Cl, Br, Methyl, Ethyl, -O-CH₃ und -O-C₂H₅ substituiert und ggf. über eine -(CH₂)-Gruppe gebunden ist, stehen,
M¹ für einen Rest ausgewählt aus der Gruppe bestehend aus den Resten 1 bis 6, 21, 22, 36 und 37 steht, 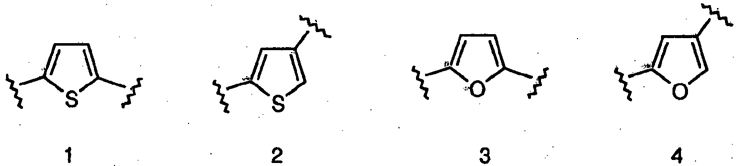 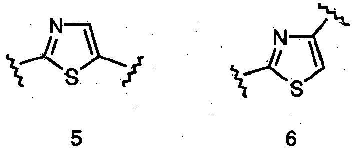 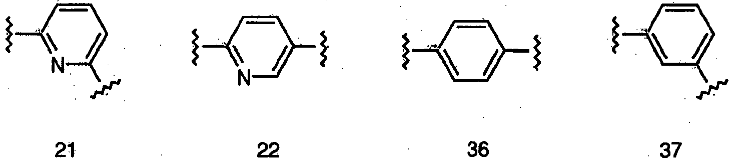 der in beliebiger Richtung an den gekennzeichneten Positionen im Grundgerüst gebunden sein kann und ggf. mit 1 oder 2 weiteren Substituenten substituiert ist, die unabhängig voneinander ausgewählt werden können aus der Gruppe bestehend aus F, Cl, Br, -CN, -CH₂-CN, -CF₃, -SF₅, Methyl, Ethyl, n-Propyl, iso-Propyl, tert-Butyl, -0-CH₃ und -O-CF₃,
und
M² für einen Rest ausgewählt aus der Gruppe bestehend aus Phenyl, 2-Pyridinyl, 3-Pyridinyl, 4-Pyridinyl, 2-Thiazolyl, 4-Thiazolyl, 5-Thiazolyl, 4-Indolyl, 5-Indolyl, 6-Indolyl, 7-Indolyl, 2-Thiophenyl, 3-Thiophenyl, 2-Imidazolyl, 4-Imidazolyl, 5-Imidazolyl, 2-Pyrimidinyl, 4-Pyrimidinyl, 5-Pyrimidinyl, 2-Pyrazinyl, 5-Chinolinyl, 6-Chinolinyl, 7-Chinolinyl und 8-Chinolinyl steht, wobei der jeweilige Rest unsubstituierte oder ggf. mit 1, 2, 3, 4 oder 5 Substituenten substituiert ist, die unabhängig voneinander ausgewählt sind aus der Gruppe bestehend aus F, Cl, Br, -CN, -CF₃, - SF₅. -S-CH₃, -S-C₂H₅, Methyl, Ethyl, iso-Propyl, tert-Butyl, Ethenyl, Propenyl, -OH, - O-CH₃, -O-C₂H₅, -NH₂, -N(CH₃)₂, -NH-CH₃, -CH₂-NH₂, -NO₂, -O-CF₃, -C(=O)-H; - C(=O)-OCH₃, -C(=O)-O-C₂H₅, Phenyl, Pyrrolyl, (1,3)-Dioxolanyl, NH-S(=O)₂-CH₃, - NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH; -S(=O)₂-N(CH₃)₂ und -Si(Phenyl)₂[C(CH₃)₃],
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind Verbindungen der allgemeinen Formel td, 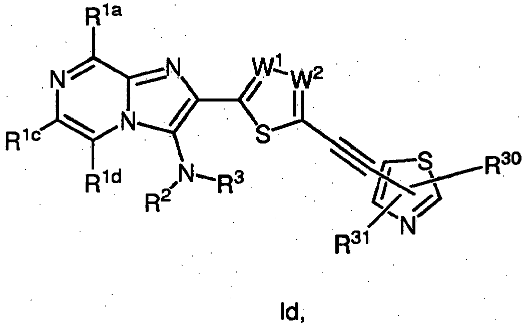 worin
R^{1a}, R^{1c}, R^{1d}, R² und R³ jeweils die vorstehend genannte Bedeutung haben;

W¹ für C steht und W² für C steht
   oder W¹ für C steht und W² für N steht
   oder W¹ für N steht und W² für C steht;
und
R²⁰ und R³¹, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃),₂, -NH-CH₃, -CH₂-NH₂, -NO₂, -O-CF₃, -C(=O)-H; -C(=O)-OCH₃, -C(=O)-O-C₂H₅, Phenyl, Pyrrolyl, (1,3)-Dioxolanyl, -Si(Phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, - C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

Ganz besonders bevorzugt sind Verbindungen der allgemeinen Formel le, 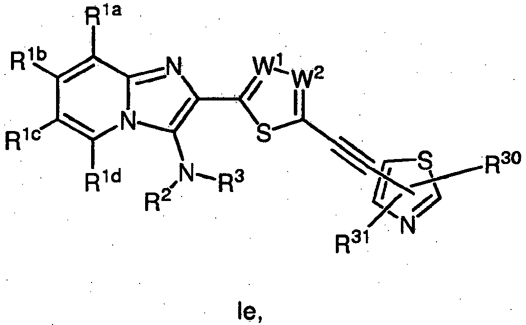 worin
R^{1a}, R^{1b}, R^{1c}, R^{1d}, R² und R³ jeweils die vorstehend genannte Bedeutung haben;

W¹ für C steht und W² für C steht
   oder W¹ für C steht und W² für N steht
   oder W¹ für N steht und W² für C steht;
und
R³⁰ und R³¹, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH, -N(CH₃)₂, -NH-CH3, -CH₂-NH₂, -NO₂, -O-CF₃, -C(=O)-H; -C(=O)-OCH₃, -C(=O)-O-C₂H₅, Phenyl, Pyrrolyl, (1,3)-Dioxolanyl, -Si(Phenyl)_{2[}C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, - C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

Ganz besonders bevorzugt sind Verbindungen der allgemeinen Formel If, 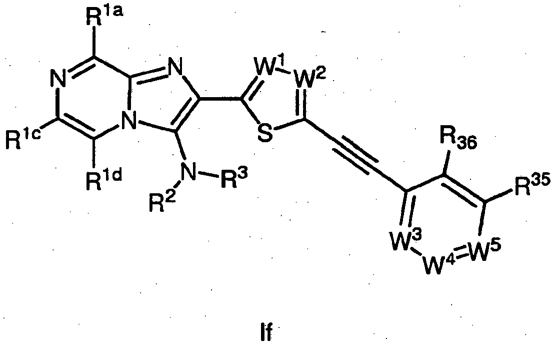 worin
R^{1a}, R^{1c}, R^{1d}, R² und R³ jeweils die vorstehend genannte Bedeutung haben;

W¹ für C steht und W² für C steht
   oder W¹ für C steht und W² für N steht
   oder W¹ für N steht und W² für C steht;
W³ für C-R³² steht; W⁴ für C-R³³ steht und W⁵ für C-R³⁴ steht;
oder einer der Reste W³, W⁴ und W⁵ für N steht und die anderen beiden Reste ausgewählt aus der Gruppe bestehend aus W³, W⁴ und W⁵ für C-R³² oder C-R³³ stehen;
und R³², R³³, R³⁴, R³⁵ und R³⁶, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, - NH₂, -N(CH₃)₂, -NH-CH₃, -CH₂-NH₂, -NO₂, -O-CF₃, -C(=O)-H; -C(=O)-OCH₃, -C(=O)-O-C₂H₅, Phenyl, Pyrrolyl, (1,3)-Dioxolanyl, -Si(Phenyl)₂[C(CH₃)₃], NH-S(=O)₂-CH₃, - NH-S(=O)₂-C₂H₅, -S(=O)₂-NH₂, -S(=O)₂-NH-CH₃, -CH₂-OH, -C(-O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechende Solvate.

Ganz besonders bevorzugt sind Verbindungen der allgemeinen Formel Ig, 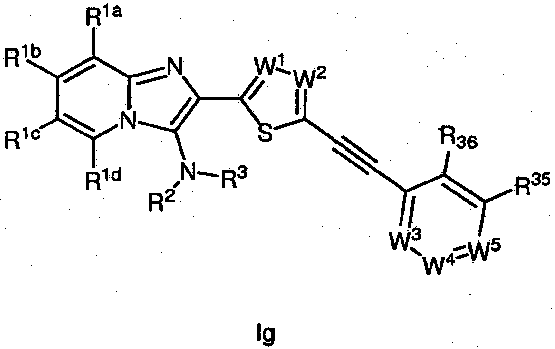 worin
R^{1a}, R^{1b}, R^{1c}, R^{1d}, R² und R³ jeweils die vorstehend genannte Bedeutung haben;
W¹ für C steht und W² für C steht.
   oder W¹ für C steht und W² für N steht
   oder W¹ für N steht und W² für C steht;
W³ für C-R³² steht; W⁴ für C-R³³ steht und W⁵ für C-R³⁴ steht;
oder einer der Reste W³, W⁴ und W⁵ für N steht und die anderen beiden Reste ausgewählt aus der Gruppe bestehend aus W³, W⁴ und W⁵ für C-R³² oder C-R³³ stehen;
und R³², R³³, R³⁴, R³⁵ und R³⁶, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, ₋OH, -O-CH₃, -O-C₂H₅, - NH₂, -N(CH₃)₂, -NH-CH₃, -CH₂-NH₂, -NO₂, -O-CF₃, -C(=O)-H; -C(=O)-OCH₃, -C(=O)-O-C₂H₅, Phenyl, Pyrrolyl, (1,3)-Dioxolanyl, -Si(Phenyl)₂[C(CH₃)₃), NH-S(=O)₂-CH₃, - NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-(=O)-CH₃, -NH-C₄=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

Ganz besonders bevorzugt sind Verbindungen der allgemeinen Formel Ih, 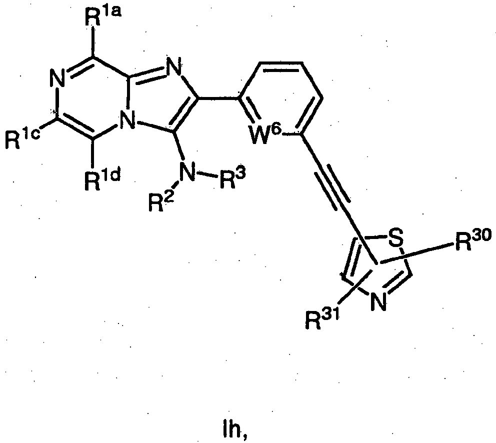 worin
A^{1a}, R^{1c}, R^{1d}, R² und R³ jeweils die vorstehend genannte Bedeutung haben;
W⁶ für C oder N steht;
und
A³⁰ und R³¹, unabhängig voneinander, jeweils für einen Rest ausgewählt aus der Gruppe bestehend aus H, F, Cl, Br, -CN, -CF₃, -SF₅, -S-CH₃, S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, Phenyl, Pyrrolyl, (1,3)-Dioxolanyl, -Si(Phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂O-CH₃, - C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

Ganz besonders bevorzugt sind Verbindungen der allgemeinen formel Ik, 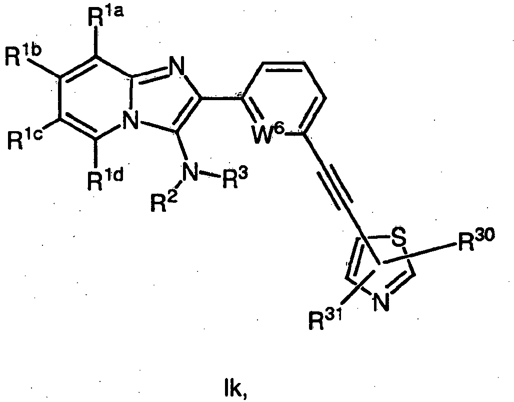 worin
R^{1a}, R^{1b}, R^{1c}, R^{1d}, R² und R³ jeweils die vorstehend genannte Bedeutung haben;
W⁶ für C oder N steht;
und
R³⁰ und R³¹, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, Phenyl, Pyrrolyl, (1,3)-Dioxolanyl, -Si(Phenyl)_{2[}C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, - C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

Ganz besonders bevorzugt sind Verbindungen der allgemeinen Formel Im, 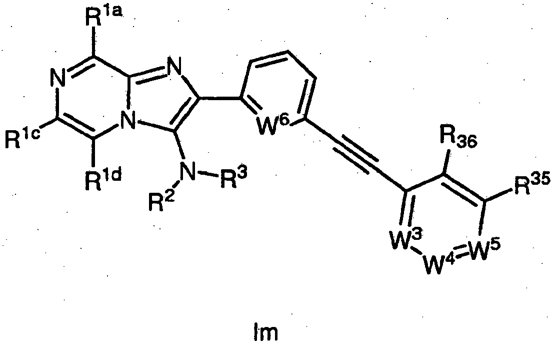 worin
R^{1a}, R^{1c}, R^{1d}, R² und R³ jeweils die Bedeutung gemäß Anspruch 14 haben;
W⁶ für C oder N steht;
W³ für C-R³² steht; W⁴ für C-R³³ steht und W⁵ für C-R³⁴ steht;
oder einer der Reste W³, W⁴ und W⁵ für N steht und die anderen beiden Reste ausgewählt aus der Gruppe bestehend aus W³, W⁴ und W⁵ für C-R³² oder C-R³³ stehen;
und R³², R³³, R³⁴, R³⁵ und R³⁶, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, **-O-C₂H₅,** - NH₂, -N(CH₃)₂, -NH-CH₃, -CH₂-NH₂, -NO₂, -O-CF₃, -C(=O)-H; -C(=O)-OCH₃, -C(=O)-O-C₂H₅, Phenyl, Pyrrolyl, (1,3)-Dioxolanyl, -Si(Phenyl)₂[C(CH₃)₃], NH-S(=O)₂-CH₃, - NH-S(=O)₂-C₂H₅, -S(=O)₂-NH₂, -S(=O)₂-NH-CH₃, -CH₂-OH, -C(=O)-CH₃, -C(=O)OH, -CH₂-O-CH₃, -C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

Ganz besonders bevorzugt sind Verbindungen der allgemeinen Formel In, 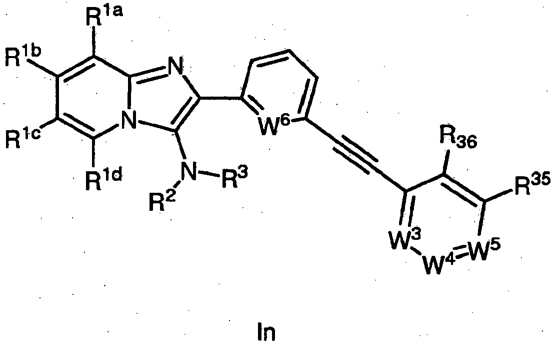 worin
R^{1a}, R^{1b}, R^{1c}, R^{1d}, R² und R³ jeweils die vorstehend genannte Bedeutung haben;
W⁶ für C oder N steht;
W³ für C-R³² steht; W⁴ für C-R³³ steht und W⁵ für C-R³⁴ steht;
oder einer der Reste W³, W⁴ und W⁵ für N steht und die anderen beiden Reste ausgewählt aus der Gruppe bestehend aus W³, W⁴ und W⁵ für C-R³² oder C-R³³ stehen;
und R³², R³³, R³⁴, R³⁵ und R³⁶, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, - NH₂, -N(CH₃)₂, -NH-CH₃, -CH₂-NH₂, -NO₂, -O-CF₃, -C(=O)-H; -C(=O)-OCH₃, -C(=O)-O-C₂H₅, Phenyl, Pyrrolyl, (1,3)-Dioxolanyl, -Si(Phonyl)₂[C(CH₃)₃], NH-S(=O)₂-CH₃, - NH-S(=O)₂-C₂H₅, -S(=O)₂-NH₂, -S(=O)₂-NH-CH₃, -CH₂=OH, -C(=O)-CH₃, -O(=O)-OH, -CH₂-O-CH₃, -C(=O)-NH₂, -C(=O)-NH-CH₃, -NH-C(=O)-CH₃, -NH-O(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

Noch weiter bevorzugt sind substituierte Imidazo-3-yl-amin-Verbindungen der allgemeinen Formel I ausgewählt aus der Gruppe bestehend aus
[1] Cyclopentyl-[2-(5-phenylethinyl-thiophen-2-yl)-imidazo[1,2-a]pyrazin-3-yl]-amin,
[2] Cyclohexylmethyl-[5-methyl-2-(5-phenylethinyl-thiophen-2-yl)-imidazo[1,2-a]pyridin-3-yl]-amin,
[3] Cyclohexylmethyl-[6-methyl-2-(5-phenylethinyl-thiophen-2-yl)-imidazo[1,2-a]pyridin-3-yl]-amin,
[4] Cyclohexylmethyl-[7-methyl-2-(5-phenylethinyl-thiophen-2-yl)-imidazo[1,2-a]pyridin-3-yl]-amin,
[5] Cyclohexylmethyl-[8-methyl-2-(5-phenylethinyl-thiophen-2-yl)-imidazo[1,2-a]pyridin-3-yl]-amin,
[6] Cyclohexylmethyl-[5,7-dimethyl-2-(5-phenylethinyl-thiophen-2-yl)-imidazo[1,2-a]pyridin-3-yl]-amin,
[7] [8-Benzyloxy-2-(5-phenylethinyl-thlophen-2-yl)-imidazo[1,2-a]pyridin-3-yl]-cyclohexylmethyl-amin,
[8] Cyclohexylmethyl-[2-(5-phenylethinyl-thiophen-2-yl)-imidazo[1,2-a]pyrazin-3-yl]-amin,
[9] Cyclohexylmethyl-[2-(5-phenylethinyl-thiophen-2-yl)-imidazo[1,2-a]pyrimidin-3-yl]-amin,
[10] (4-Methoxy-benzyl)-[5-methyl-2-(5-phenylethinyl-thiophen-2-yl)-imidazo[1,2-a]pyridin-3-yl]-amin,
[11] (4-Methoxy-benzyl)-[6-methyl-2-(5-phenylethinyl-thiophen-2-yl)-imidazo[1,2-a]pyridin-3-yl]-amin,
[12] (4-Methoxy-benzyl)-[7-methyl-2-(5-phenylethinyl-thiophen-2-yl)-imidazo[1,2-a]pyridin-3-yl]-amin,
[13] (4-Methoxy-benzyl)-[8-methyl-2-(5-phenylethinyl-thiophen-2-yl)-imidazo[1,2-a]pyridin-3-yl]-amin,
[14] [5,7-Dimethyl-2-(5-phenylethinyl-thiophen-2-yl)-imidazo[1,2-a]pyridin-3-yl]-{4-methoxy-benzyl)-amin,
[15] [8-Benzyloxy-2-(5-phenylethinyl-thiophen-2-yl)-imidazo[1,2-a]pyrrdin-3-yl]-(4-methoxy-benzyl)-amin,
[16] (4-Methoxy-benzyl)-[2-(5-phenylethinyl-thiophen-2-yl)-imidazo[1,2-a]pyridin-3-yl]-amin,
[17] (4-Methoxy-benzyl)-[2-(5-phenylethinyl-thiophen-2-yl)-imidazo[1,2-a]pyrazin-3-yl]-amin,
[18] tert-Butyl-[2-(5-phenylethinyl-thiophen-2-yl)-imidazo[1,2-a]pyrazin-3-yl]-amin,
[19] tert-Butyl-[6-methyl-2-(5-phenylethinyl-thiophen-2-yl)-imidazo[1,2-a]pyridin-3-yl]-amin,
[20] [8-Benzyloxy-2-(5-phenylethinyl-thiophen-2-yl)-imidazo[1,2-a]pyridin-3-yl]-(3-methoxy-phenyl)-amin,
[21] (3-Methoxy-benzyl)-[5-methyl-2-(5-phenylethinyl-thiophen-2-yl)-imidazo[1,2-a]pyridin-3-yl]-amin,
[22] (3-Methoxy-benzyl)-[8-methyl-2-(5-phenylethinyl-thiophen-2-yl)-imidazo[1,2-a]pyridin-3-yl]-amin,
[23] [5,7-Dimethyl-2-(5-phenylethinyl-thiophen-2-yl)-imidazo[1,2-a]pyridin-3-yl]-(3-methoxy-benzyl)-amin,
[24] [8-Benzyloxy-2-(5-phenylethinyl-thiophen-2-yl)-imidazo[1,2-a]pyridin-3-yl]-(3-methoxy-benzyl)-amin,
[25] (3-Methoxy-benzyl)-[2-(5-phenylethinyl-thiophen-2-yl)-imidazo[1,2-a]pyridin-3-yl]-amin,
[26] (3-Methoxy-benzyl)-[2-(5-phenylethinyl-thiophen-2-yl)-imidazo[1,2-a]pyrazin-3-yl]-amin,
[27] [6-Methyl-2-(5-phenylethinyl-thiophen-2-yl)-imidazo[1,2-a]pyridin-3-yl]-(1-phenyl-ethyl)-amin,
[28] [7-Methyl-2-(5-phenylethinyl-thiophen-2-yl)-imidazo[1,2-a]pyridin-yl]-(1-phenyl-ethyl)-amin,
[29] [5,7-Dimethyl-2-(5-phenylethinyl-thiophen-2-yl)-imidazo[1,2-a]pyridin-3-yl]-(1-phenyl-ethyl)-amin,
[30] [8-Benzyloxy-2-(5-phenylethinyl-thiophen-2-yl)-imidazo[1,2-a]pyridin-3-yl]-(1-phenyl-ethyl)-amin,
[31] (1-Phenyl-ethyl)-[2-(5-phenylethinyl-thiophen-2-yl)-imidazo[1,2-a]pyrazin-3-yl]-amin,
[32] (2-Chlor-benzyl)-[5,7-dimethyl-2-(5-phenylethinyl-thiophen-2-yl)-imidazo[1,2-a]pyridin-3-yl]-amin,
[33] (3-Chlor-4-fluor-phenyl)-[7-phenyl-2-(5-phenylethinyl-thiophen-2-yl)-imidazo[1,2-a]pyridin-3-yl]-amin,
[34] (4-Methoxy-benzyl)-[7-methyl-2-(5-phenylethinyl-thiophen-2-yl)-imidazo[1,2-a]pyrimidin-3-yl]-amin,
[35] [8-Methyl-2-(5-phenylethinyl-thiophen-2-yl)-imidazo[1,2-a]pyridin-3-yl]-(1-phenyl-ethyl)-amin,
[36] [7-Methyl-2-(5-phenylethinyl-thiophen-2-yl)-imidazo[1,2-a]pyrimidin-3-yl]-(1-phenyl-ethyl)-amin,
[37] (2-Methoxy-benzyl)-[7-methyl-2-(5-phenylethinyl-thiophen-2-yl)-imidazo[1,2-a]pyridin-3-yl]-amin,
[38] (2-Methoxy-benzyl)-[2-(5-phenylethinyl-thiophen-2-yl)-imidazo[1,2-a]pyridin-3-yl]-amin,
[39] (2-Chlor-benzyl)-[2-(5-phenylethinyl-thiophen-2-yl)-imidazo[1,2-a]pyridin-3-yl]-amin,
[40] (2-Methoxy-benzyl)-[7-methyl-2-(5-phenylethinyl-thiophen-2-yl)-imidazo[1,2-a]pyrimidin-3-yl]-amin,
[41] (3-Methoxy-phenyl)-[6-methyl-2-(5-phenylethinyl-thiophen-2-yl)-imidazo[1,2-a]pyridin-3-yl]-amin,
[42] [5,7-Dimethyl-2-(5-phenylethinyl-thiophen-2-yl)-imidazo[1,2-a]pyridin-3-yl]-(3-methoxy-phenyl)-amin,
[43] (3-Methoxy-phenyl)-[2-(5-phenylethinyl-thiophen-2-yl)-imidazo[1,2-a]pyrazin-3-yl]-amin,
[44] [7-Ethyl-2-(5-phenylethinyl-thiophen-2-yl)-imidazo[1,2-a]pyridin-3-yl]-(2-methoxy-benzyl)-amin,
[45] (2-Chlor-benzyl)-[7-ethyl-2-(5-phenylethinyl-thiophen-2-yl)-imdazo[1,2-a]pyridin-3-yl]-amin,
[46] [7-tert-Butyl-2-(5-phenylethinyl-thiophen-2-yl)-imidazo[1,2-a]pyridin-3-yl]-(3-chlor-4-fluor-phenyl)-amin,
[47] (3-Methoxy-benzyl)-[7-methyl-2-(5-phenylethinyl-thiophen-2-yl)-imidazo[1,2-a]pyrimidin-3-yl]-amin,
[48] [7-Ethyl-2-(5-phenylethinyl-thiophen-2-yl)-Imidazo[1,2-a]pyridin-3-yl]-(2-fluorphenyl)-amin,
[49] [7-tert-Butyl-2-(5-phenylethinyl-thiophen-2-yl)-imidazo[1,2-a]pyridin-3-yl]-(2-fluor-phenyl)-amin,
[50] (2,4-Difluor-phenyl)-[2-(5-phenylethinyl-thiophen-2-yl)-7-(3-phenyl-propyl)-imidazo[1,2-a]pyridin-3-yl]-amin,
[51] (4-Fluor-benzyl)-[7-methyl-2-(5-phenylethinyl-thiophen-2-yl)-imidazo[1,2-a]pyridin-3-yl]-amin,
[52] [5,7-Dimethyl-2-(5-phenylethinyl-thiophen-2-yl)-imidazo[1,2-a]pyridin-3-yl]-(4-fluor-benzyl)-amin,
[53] [7-Ethyl-2-(5-phenylethinyl-thiophen-2-yl)-imidazo[1,2-a]pyridin-3-yl]-(3-trifluormethyl-benzyl)-amin,
[54] [7-Isopropyl-2-(5-phenylethinyl-thiophen-2-yl)-imidazo[1,2-a]pyridin-3-yl]-(3-trifluormethyl-benzyl)-amin,
[55] tert-Butyl-[2-(4-phenylethinyl-thiophen-2-yl)-imidazo[1,2-a]pyrazin-3-yl]-amin,
[56] [2-(5-Pheny)ethinyl-thiophen-2-yl)-imidazo[1,2-a]pyrazin-3-yl]-(1,1,3,3-tetramethyl-butyl)-amin,
[57] Butyl-[2-(4-phenylethinyl-thiophen-2-yl)-imidazo[1,2-a]pyrazin-3-yl]-amin,
[59] [2-(5-Phenylethinyl-thiophen-2-yl)-imidazo[1,2-a]pyridin-3-yl]-(1,1,3,3-tetramethyl-butyl)-amin,
[60] [2-(5-Pyridinyl-thiophen-2-yl)-imidazo[1,2-a]pyrazin-3-yl]-(1,1,3,3-tetramethyl-butyl)-amin,
[61] [2-(5-Pyridin-4-ylethinyl-thiophen-2-yl)-imidazo[1,2-a]pyrazin-3-yl]-(1,1,3,3-tetramethyl-butyl)-amin,
[62] [6-Chlor-2-(5-phenylethinyl-thiophen-2-yl)-imidazo[1,2-a]pyridin-3-yl]-(1,1,3,3-tetramethyl-butyl)-amin,
[63] [6,8-Dichlor-2-(5-phenylethinyl-thiophen-2-yl)-imidazo[1,2-a]pyridin-3-yl]-(1,1,3,3-tetramethyl-butyl)-amin,
[64] [2-(5-Pyridin-2-ylethinyl-thiophen-2-yl)-imidazo[1,2-a]pyrazin-3-yl]-(1,1,3,3-tetramethyl-butyl)-amin,
[65] Dimethyl-[2-(5-phenylethinyl-thiophen-2-yl)-imidazo[1,-2-a]pyrazin-3-yl]-amin,
[66] Methyl-[2-(5-phenylethinyl-thiophen-2-yl)-imidazo[1,2-a]pyrazin-3-yl]-amin,
[67] N-[2-(5-Phenylethinyl-thiophen-2-yl)-imidazo[1,2-a]pyrazin-3-yl]-acetamid,
[68] Ethyl-[2-(5-phenylethinyl-thiophen-2-yl)-imidazo[1,2-a]pyrazin-3-yl]-amin,
[69] Propyl-[2-(5-phenylethinyl-thiophen-2-yl)-imidazo[1,2-a]pyrazin-3-yl]-amin,
[70] Butyl-[2-(5-phenylethinyl-thiopherl-2-yl)-Imidazo[1,2-a]pyrazin-3-yl]-amin,
[71] (2-Methylpropyl)-[2-(5-phenylethinyl-thiophen-2-yl)-imidazo[1,2-a]pyrazin-3-yl]-amin,
[72] Pentyl-[2-(5-phenylethinyl-thiophen-2-yl)-imidazo[1,2-a]pyrazin-3-yl]-amin,
[73] {(Methoxycarbonylmethyl)-[2-(5-phenylethynyl-thiophen-2-yl)-imidazo[1,2-a]pyrazin-3-yl]-amino}-essigsäuremethylester,
[74] Benzyl-[2-(5-phenylethynyl-thiophen-2-yl)-imidazo[1,2-a]pyrazin-3-yl]-amin,
[75] [2-(5-Phenylethynyl-thiophen-2-yl)-imidazo[1,2-a]pyrazin-3-ylamino]-essigsäuremethylester,
[76] tert-Butyl-[2-(5-pyridin-4-ylethinyl-thiophen-2-yl)-imidazo[1,2-a]pyrazin-3-yl]-amin,
[77] tert-Butyl-[2-(5-pyridin-2-ylethinyl-thiophen-2-yl)-imidazo[1,2-a]pyrazin-3-yl]-amin,
[78] N-[2-(5-Phenylethinyl-thiophen-2-yl)-imidazo[1,2-a]pyrazin-3-yl]-benzamid,
[79] [2-(5-Phenylethinyl-thiophen-2-yl)-imidazo[1,2-a]pyrazin-3-yl]-bis-pyridin-3-ylmethyl-amin,
[80] 2,2-Dimethyl-N-[2-(5-phenylethinyl-thiophen-2-yl)-imidazo[1,2-a]pyrazin-3-yl]-propionamid,
[81] 3-Methoxy-N-[2-(5-phenylethinyl-thiophen-2-yl)-imidazo[1,2-a]pyrazin-3-yl]-benzamid,
[82] tert-Butyl-[2-(5-pyridin-3-ylethinyl-thiophen-2-yl)-imidazo[1,2-a]pyrazin-3-yl]-amin
[83] 2-(5-Phenylethinyl-thiophen-2-yl)-imidazo[1,2-a]pyrazin-3-ylamin
[84] Methyl-2-(5-(phenylethinyl)thiophen-2-yl)-3-(2,4,4-trimethylpentan-2-ylamino)imidazo[1,2-a]pyrazin-8-carboxylat
[85] 2-(5-(Phenylethinyl)thiophen-2-yl)-N-(2,4,4-trimethylpentan-2-yl)imidazo[1,2-a]pyrimidin-3-amin
[86] 2-(5-((6-Methylpyridin.2-yl)ethinyl)thiophen.2-yl).N<2,4,4-trimethylpentan-2-yl)imidazo[1,2-a]pyrazin-3-amin
[87] N-Cyclohexyl-2-(5-(phenylethinyl)thiophen-2-yl)imidazo[1,2-a]pyra-zin-3-amin
[88] 2-(5-(Phenylethinyl)thiophen-2-yl)-3-(piperidin-1-yl)imidazo[1,2-a]pyrazin
[89] N-tert-Butyl-N-methyl-2-(5-(phenylethinyl)thiophen-2-yl)imidazo[1,2-a]pyrazin-3-amin
[90] Methyl-2-(5-(phenylethinyl)thiophen-2-yl)-3-(2,4,4-trimethylpentan-2-ylamino)imidazo[1,2-a]pyridin-6-carboxylat
[91] N-tert-Butyl-2-(5-(pyridin-2-ylethinyl)thiophen-2-yl)imidazo[1,2-a]pyrazin-3-amin Hydrochlorid
[92] 8-Brom-N-cyclopentyl-6-methyl-2-(5-(pyridin-2-ylethinyl)thiophen-2-yl)imidazo[1,2-a]pyridin-3-amin
[93] N,N-Diethyl-2-(5-(phenylethinyl)thiophen-2-yl)imidazo[1,2-a]pyrazin-3-amin
[94] N-tert-Butyl-2-(5-(pyridin-2-ylethinyl)thiophen-2-yl)imidazo[1,2-a]pyridin-3-amin
[95] N-tert-Butyl-2-(5-(phenylethinyl)furan-2-yl)imidazo[1,2-a]pyrazin-3-amin
[96] 8-Brom-N-tert-butyl-6-methyl-2-(3-(pyridin-2-ylethinyl)phenyl)imidazo[1,2-a]pyridin-3-amin
[97] N-tert-Butyl-8-methyl-2-(5-(phenylethinyl)thlophen-2-yl)imidazo[1,2-a]pyridin-3-amin
[98] N-Methyl-2-(5-(phenylethinyl)thiophen-2-yl)-N-(2,4,4-trimethylpentan-2-yl)imidazo[1,2-a]pyrazin-3-amin Hydrochlorid
[99] 2-(5-(Phenylethinyl)thiophen-2-yl)-3-(pyrrolidin-1-yl)imidazo[1,2-a]pyrazin Hydrochlorid
[100] N-tert-Butyl-2-(5-((4-fluorphenyl)ethinyl)thiaphen-2-yl)imidazo[1,2-a]pyrazin-3-amin Hydrochlorid
[101] N-tert-Butyl-7-methyl-2-(5-(phenylethinyl)thiophen-2-yl)imidazo[1,2-a]pyridin-3-amin
[102] N-tert-Butyl-5-methyl-2-(5-(phenylethinyl)thiophen-2-yl)imidazo[1,2-a]pyridin-3-amin
[103] 8-Chlor-2-(3-(pyridin-2-ylethinyl)phenyl)-6-(trifluormethyl)-N-(2,4,4-trimethylpentan-2-yl)imidazo[1,2-a]pyridin-3-amin
[104] N-tert-Butyl-2-(5-((3-fluorphenyl)ethinyl)thiophen-2-yl)imidazo[1,2-a]pyrazin-3-amin Hydrochlorid
[105] N-tert-Butyl-2-(5-((2-fluorphenyl)ethinyl)thiophen-2-yl)imidazo[1,2-a]pyrazin-3-amin Hydrochlorid
[106] Methyl-3-(tert-butylamino)-2-(5-(phenylethinyl)thiophen-2-yl)imidazo[1,2-a]pyrazin-8-carboxylat
[107] N-tert-Butyl-2-(5-(pyrazin-2-ylethinyl)thiophen-2-yl)imidazo[1,2-a]yrazin-3-amin
[108] 2-(5-((4-Aminophenyl)ethinyl)thiophen-2-yl)-N-tert-butylimidazo[1,2-a]pyrazin-3-amin
[109] N-Isopropyl-2-(5-(phenylethinyl)thiophen-2-yl)imidazo[1,2-a]pyrazin-3-amin
[110] N-tert-Butyl-2-(5-(thiophen-2-ylethinyl)thlophen-2-yl)imidazo[1,2-a]pyrazin-3-amin Hydrochlorid
[111] N-tert-Butyl-2-(3-(pyridin-2-ylethinyl)phenyl)imidazo[1,2-a]pyrazin-3-amin
[112] N-tert-Butyl-2-(5-((2-methoxyphenyl)ethinyl)thiophen-2-yl)imidazo[1,2-a]pyrazin-3-amin Hydrochlorid
[113] N-tert-Butyl-2-(5-((3-methoxyphenyl)ethinyl)thiophen-2-yl)imicazo[1,2-a]pyrazin-3-amin Hydrochlorid
[114] N-tert-Butyl-2-(5-((4-methoxyphenyl)ethinyl)thiophen-2-yl)imidazo[1,2-a]pyrazin-3-amin Hydrochlorid
[115] N-tert-Butyl-2-(5-(phenylethinyl)thiophen-2-yl)imidazo[1,2-a]chinolin-1-amin
[116] N-tert-Butyl-2-(4-(pyridin-2-ylethinyl)phenyl)imidazo[1,2-a]pyrazin-3-amin
[117] N-tert-Butyl-2-(3-((6-methylpyridin-2-yl)ethinyl)phenyl)imidazo[1,2-a]pyrazin-3-amin
[118] N-tert-Butyl-2-(2-methyl-6-(phenylethinyl)pyridin-3-yl)imidazo[1,2-a]pyrazin-3-amin
[119] N-tert-Butyl-2-(5-(pyridin-2-ylethinyl)furan-2-yl)imidazo[1,2-a]pyrazin-3-amin
[120] 3-(tert-Butylamino)-2-(5-(phenylethinyl)thiophen-2-yl)imidazo[1,2-a]pyrazin-8-carbonsäure
[121] 4-((5-(3-(tert-Butylamino)imidazo[1,2-a]pyrazin-2-yl)thiophen-2-yl)ethinyl)phenol Hydrochlorid
[122] 3-((5-(3-(tert-Butylamino)imidazo[1,2-a]pyrazin-2-yl)thiophen-2-yl)ethinyl)phenol
[123] 2-(5-((3-Aminophenyl)ethinyl)thiophen-2-yl)-N-tert-butylimidazo[1,2-a]pyrazin-3-amin Hydrochlorid
[124] 2-(5-((2-Aminophenyl)ethinyl)thiophen-2-yl)-N-tert-butylimidazo[1,2-a]pyrazin-3-amin Hydrochlorid
[125] N-tert-Butyl-2-(5-(phenylethinyl)thiophen-2-yl)imidazo[2,1-a]isochinolin-3-amin
[126] N-tert-Butyl-2-(6-(phenylethinyl)pyridin-3-yl)imidazo[1,2-a]pyrazin-3-amin
[127] N-tert-Butyl-2-(5-(pyridin-4-ylethinyl)thiophen-2-yl)imidazo[1,2-a]pyrazin-3-amin Hydrochlorid
[128] 2-(5-((6-Aminopyridin-3-yl)ethinyl)thiophen-2-yl)-N-tert-butylimidazo[1,2-a]pyrazin-3-amin Hydrochlorid
[129] N-tert-Butyl-2-(5-(pyrimidin-2-ylethinyl)thiophen-2-yl)imidazo[1,2-a]pyrazin-3-amin Hydrochlorid
[130] N-tert-Butyl-2-(5-((4-methylpyridin-2-yl)ethinyl)thiophen-2-yl)imidazo[1,2-a]pyrazin-3-amin Hydrochlorid
[131]N-tert-Butyl-2-(5-((5-methylpyridin-2-yl)ethinyl)thiophen-2-yl)imidazo[1,2-a]pyrazin-3-amin Hydrochlorid
[132] N-tert-Butyl-2-(5-(pyridin-4-ylethinyl)thiophen-2-yl)imidazo[1,2-a]pyridin-3-amin Hydrochlorid
[133] N-tert-Butyl-2-(5-(thiazol-2-ylethinyl)thiophen-2-yl)imidazo[1,2-a]pyrazin-3-amin
[134] 2-(5-(Pyridin-2-ylethinyl)thiophen-2-yl)imidazo[1,2-a]pyrazin-3-amin Hydrochlorid
[135] N-tert-Butyl-2-(5-((5-methylthiophen-2-yl)ethinyl)thiophen-2-yl)imidazo[1,2-a]pyrazin-3-amin Hydrochlorid
[136] 2-(5-((6-Aminopyridin-2-yl)ethinyl)thiophen-2-yl)-N-tert-butylimidazo[1,2-a]pyrazin-3-amin Hydrochlorid
[137] N-tert-Butyt-2-(5-((3-methylthiophen-2-yl)ethinyl)thiophen-2-yl)imidazo[1,2-a]pyrazin-3-amin
[138] N-tert-Butyl-2-(4-(phenylethinyl)thiazol-2-yl)imidazo[1,2-a]pyrazin-3-amin
[139] N-tert-Butyl-2-(5-(m-tolylethinyl)thiophen-2-yl)imidazo[1,2-a]pyrazin-3-amin
[140] 3-((5-(3-(tert-Butylamino)imidazo[1,2-a]pyrazin-2-yl)thiophen 2-yl)ethinyl)benzonitrit Hydrochlorid
[141] N-tert-Butyl-2-(5-(pyridin-2-ylethinyl)thiophen-2-yl)imidazo[1,2-a]pyrimidin-3-amin
[142] N-tert-Butyl-2-(6-(phenylethinyl)pyridin-2-yl)imidazo[1,2-a]pyrazin-3-amin Hydrochlorid
[143] N-tert-Butyl-N-methyl-2-(5-(pyridin-2-ylethinyl)thiophen-2-yl)imidazo[1,2-a]pyrazin-3-amin
[144] 4-((5-(3-(tert-Butylamino)imidazo[1,2-a]pyrazin-2-yl)thiophen-2-yl)ethinyl)benzonitril Hydrochlorid
[145] 2-(5-((1H-Indol-6-yl)ethinyl)thiophen-2-yl)-N-tert-butylimidazo[1,2-a]pyrazin-3-amin Hydrochlorid
[146] N-tert-Butyl-2-(2-(phenylethinyl)thiazol-5-yl)imidazo[1,2-a]pyrazin-3-amin
[147] N-tert-Butyl-2-(5-(chinolin-6-ylethinyl)thiophen-2-yl)imidazo[1,2-a]pyrazin-3-amin Hydrochlorid
[148] 2-(5-((3-(1H-Pyrrol-1-yl)phenyl)ethinyl)thiophen-2-yl)-N-tert-butylmidazo[1,2-a]pyrazin-3-amin Hydrochlorid
[149] 2-(5-((1H-Indol-4-yl)ethinyl)thiophen-2-yl)-N-ten-butylimidazo[1,2-a]pyrazin-3-amin Hydrochlorid
[150] N-tert-Butyl-2-(5-((3-nitrophenyl)ethinyl)thiophen-2-yl)imidazo[1,2-a]pyrazin-3-amin Hydrochlorid
[151] N-tert-Butyl-2-(5-((4-nitrophenyl)ethinyl)thiophen-2-yl)imidazo[1,2-a]pyrazin-3-amin Hydrochlorid
[152] N-tert-Butyl-2-(5-(thiazol-4-ylethinyl)thiophen-2-yl)imidazo[1,2-a]pyrazin-3-amin Hydrochlorid
[153] 2-((5-(3-(tert-Butylamino)imidazo[1,2-a]pyrazin-2-yl)thiophen-2-yl)ethinyl)phenol
[154] 2-(5-((3-(Aminomethyl)phenyl)ethinyl)thiophen-2-yl)-N-tert-butylimidazo[4,2-a]pyrazin-3-amin
[155] 2-(5-(Biphenyl-3-ylethinyl)thiophen-2-yl)-N-tert-butylimidazo[1,2-a]pyrazin-3-amin Hydrochlorid
[156] N-tert-Butyl-2-(5-(thiophen-3-ylethinyl)thiophen-2-yl)imidazo[1,2-a]pyrazin-3-amin Hydrochlorid
[157] N-tert-Butyt-2-(5-((3-(dimethylamino)phenyl)ethinyl)thiophen-2-yl)imidazo[1,2-a]pyrazin-3-amin Hydrochlorid
[158] N-tert-Butyl-2-(5-((6-methylpyridin-2-yl)ethinyl)thiophen-2-yl)imidazo[1,2-a]pyrazin-3-amin Hydrochlorid
[159] N-tert-Butyl-2-(5-((3-fluorpyridin-2-yl)ethinyl)thiophen-2-yl)imidazo[1,2-a]pyrazin-3-amin Hydrochlorid
[160] N-tert-Butyl-2-(5-((3-(methylamino)phenyl)ethinyl)thiophen-2-yl)imidazo[1,2-a]pyrazin-3-amin Hydrochlorid
[161] N-tert-Butyl-2-(5-(p-tolylethinyl)thiophen-2-yl)imidazo[1,2-a]pyrazin-3-amin
[162] N-tert-Butyl-2-(5-(o-tolylethinyl)thiophen-2-yl)imidazo[1,2-a]pyrazin-3-amin Hydrochlorid
[163] N-tert-Butyl-2-(4-methyl-5-(phenylethinyl)thiophen-2-yl)imidazo[1,2-a]pyrazin-3-amin Hydrochlorid
[164] N-tert-Butyl-2-(4-methyl-5-(pyridin-2-ylethinyl)thiophen-2-yl)imidazo[1,2-a]pyrazin-3-amin Hydrochlorid
[165] N-tert-Butyl-2-(5-((6-fluorpyridin-2-yl)ethinyl)thiophen-2-yl)imidazo[1,2-a]pyrazin-3-amin
[166] N-tert-Butyl-2-(5-((2-nitrophenyl)ethinyl)thiophen-2-yl)imidazo[1,2-a]pyrazin-3-amin
[167] N-tert-Butyl-8-chlor-2-(5-(pyridin-2-ylethinyl)thiophen-2-yl)imidazo[1,2-a]pyrazin-3-amin
[168] N-tert-Butyl-2-(5-((6-methoxypyridin-2-yl)ethinyl)thiophen-2-yl)imidazo[1,2-a]pyrazin-3-amin
[169] N-tert-Butyl-2-(5-((5-fluorpyridin-2-yl)ethinyl)thiophen-2-yl)imidazo[1,2-a]pyrazin-3-amin Hydrochlorid
[170] 2-(4-(3-(tert-Butylamino)imidazo[1,2-a]pyrazin-2-yl)-2-(phenylethinyl)phenyl)acetonitril
[171] N-tert-Butyl-2-(5-((5-methoxypyridin-3-yl)ethinyl)thiophen-2-yl)imidazo[1,2-a]pyrazin-3-amin Hydrochlorid
[172] 5-((5-(3-(tert-Butylamino)imidazo[1,2-a]pyrazin-2-yl)thiophen-2-yl)ethinyl)nicotinonitril Hydrochlorid
[173] N-tert-Butyl-2-(5-((3-(methylthio)phenyl)ethinyl)thiophen-2-yl)imidazo[1,2-a]pyrazin-3-amin
[174] Methyl-3-((5-(3-(tert-Butylamino)imidazo[1,2-a]pyrazin-2-yl)thiophen-2-yl)ethinyl)benzoate
[175] N-tert-Butyl-2-(5-((3,5-difluorpyridin-2-yl)ethinyl)thiophen-2-yl)imidazo[1,2-a]pyrazin-3-amin
[176] N-tert-Butyl-2-(5-(phenylethinyl)thiazol-2-yl)imidazo[1,2-a]pyrazin-3-amin
[177] N-tert-Butyl-2-(2-(pyridin-4-ylethinyl)thiazol-5-yl)imidazo[1,2-a]pyrazin-3-amin
[178] 3-((5-(3-(tert-Butylamino)imidazo[1,2-a]pyrazin-2-yl)thiophen-2-yl)ethinyl)benzaldehyd
[179] 3-((5-(3-(tert-Butylamino)iumidazo[1,2-a]pyrazin-2-yl)thiophen-2-yl)ethinyl)-4-fluorbenzonitril
[180] N-tert-Butyl-2-(5-((3-(trifluormethyl)phenyl)ethinyl)thiophen-2-yl)imidazo[1,2-a]pyrazin-3-amin Hydrochlorid
[181] N-tert-Butyl-2-(2-(pyridin-2-ylethinyl)thiazol-5-yl)imidazo[1,2-a]pyrazin-3-amin
[182] N-tert-Butyl-2-(3-methyl-5-(phenylethinyl)thiophen-2-yl)imidazo[1,2-a]pyrazin-3-amin Hydrochlorid
[183] N-tert-Butyl-2-(3-methyl-5-(pyridin-2-ylethinyl)thiophen-2-yl)imidazo[1,2-a]pyrazin-3-amin Hydrochlorid
[184] N-tert-Butyl-2-(5-((3-vinylphenyl)ethinyl)thiophen-2-yl)imidazo[1,2-a]pyrazin-3-amin
[185] 2-(5-((1H-imidazol-4-yl)ethinyl)thiophen-2-yl)-N-tert-butylimidazol[1,2-a]pyrazin-3-amin Hydrochlorid
[186] N-tert-Butyl-2-(5-((3-methylpyridin-2-yl)ethinyl)thiophen-2-yl)imidazo[1,2-a]pyrazin-3-amin
[187] N,N-Dimethyl-2-(5-(pyridin-2-ylethinyl)thiophen-2-yl)imidazo[1,2-a]pyrazin-3-amin
[188] N-tert-Butyl-2-(5-((2-(tert-butyldiphenylsilyl)thiazol-5-yl)ethinyl)thiophen-2-yl)imidazo[1,2-a]pyridin-3-amin
[189] 3-((5-(3-(tert-Butylamino)imidazo[1,2-a]pyridin-2-yl)thiophen-2-yl)ethinyl)benzonitril
[190] N-tert-Butyl-2-(5-(phenylethinyl)thiazol-2-yl)imidazo[1,2-a]pyridin-3-amin
[191] N-tert-Butyl-2-(5-(thiazol-5-ylethinyl)thiophen-2-yl)imidazo[1,2-a]pyridin-3-amin
[192] N-tert-Butyl-2-(5-(pyridin-2-ylethinyl)thiophen-2-yl)imidazo[1,2-a]pyridin-3-amin Hydrochlorid
[321] 6-Chlor-N-cyclohexyl-2-(3-((6-methylpyridin-2-yl)ethinyl)phenyl)imidazo[1,2-a]pyridin-3-amin
[194] 5,7-Dimethyl-N-phenethyl-2-(5-(phenylethinyl)thiophen-2-yl)imidazo[1,2-a]pyrimidin-3-amin
[195] N-(3-Methoxyphenethyl)-5,7-dimethyl-2-(5-(phenylethinyl)thiophen-2-yl)imidazo[1,2-a]pyrimidin-3-amin
[196] N-(3-Methoxyphenethyl)-5,7-dimethyl-2-(4-(phenylethinyl)thiophen-2-yl)imidazo[1,2-a]pyrimidin-3-amin
[197] N-(3-Methoxyphenethyl)-5,7-dimethyl-2-(5-(phenylethinyl)furan-2-yl)imidazo[1,2-a]pyrimidin-3-amin
[198] N-(4-Chlorbenzyl)-8-methyl-2-(5-(phenylethinyl)thiophen-2-yl)imidazo[1,2-a]pyridin-3-amin
[199] N-(3-Methoxyphenethyl)-5-methyl-2-(4-(phenylethinyl)thiophen-2-yl)imidazo[1,2-a]pyridin-3-amin
[200] N-(2-Methylhexan-2-yl)-2-(4-(phenylethinyl)thiophen-2-yl)imidazo[1,2-a]pyrazin-3-amin
[201] N-Phenethyl-2-(4-(phenylethinyl)thiophen-2-yl)imidazo[1,2-a]pyrazin-3-amin
[202] N-(3-Methoxyphenethyl)-2-(4-(phenylethinyl)thiophen-2-yl)imidazo[1,2-a]pyrazin-3-amin
[203] 2-(4-(Phenylethinyl)thiophen-2-yl)-N-(2-(thiophen-2-yl)ethyl)imidazo[1,2-a]pyrazin-3-amin
[204] N-(4-Chlorbenzyl)-2-(5-(phenylethinyl)thiophen-2-yl)imidazo[1,2-a]pyridin-3-amin
[205] N-(2-Methylpentan-2-yl)-2-(5-(phenylethinyl)thiophen-2-yl)imidazo[1,2-a]pyrazin-3-amin
[206] N-(Cyclohexylmethyl)-2-(4-(phenylethinyl)thiophen-2-yl)imidazo[1,2-a]pyrazin-3-amin
[207] N-(2-Methoxybenzyl)-2-(5-(phenylethinyl)furan-2-yl)imidazo[1,2-a]pyrazin-3-amin
[208] N-(Cyclohexylmethyl)-2-(4-(phenylethinyl)thiophen-2-yl)imidazo[1,2-a]pyridin-3-amin
[209] N-(2-Methylpentan-2-yl)-2-(4-(phenylethinyl)thiophen-2-yl)imidazo[1,2-a]pyridin-3-amin
[210] 8-Brom-6-methyl-2-(3-((6-methylpyridin-2-yl)ethinyl)phenyl)-N-(2,4,4-trimethylpentan-2-yl)imidazo[1,2-a]pyridin-3-amin
[211] 8-Brom-N-cyclopentyl-6-methyl-2-(3-(pyridin-2-ylethinyl)phenyl)imidazo[1,2-a]pyridin-3-amin
[212] N-Cyclopentyl-2-(4-(phenylethinyl)thiophen-2-yl)imidazo[1,2-a]pyrazin-3-amin
[213] N-(1-Phenylethyl)-2-(4-(phenylethinyl)thiophen-2-yl)imidazo[1,2-a]pyrazin-3-amin
[214] N-(2-Methylpentan-2-yl)-2-(4-(phenylethinyl)thiophen-2-yl)imidazo[1,2-a]pyrazin-3-amin
[215] 8-Brom-N-cyclohexyl-6-methyl-2-(5-(pyridin-2-ylethinyl)furan-2-yl)imidazo[1,2-a]pyridin-3-amin
[216] N-Cyclopentyl-2-(5-(phenylethinyl)furan-2-yl)imidazo[1,2-a]pyrazin-3-amin
[217] N-(3-Methoxyphenethyl)-7-methyl-2-(4-(phenylethinyl)thiophen-2-yl)imidazo[1,2-a]pyrimidin-3-amin
[218] 8-(Benzyloxy)-2-(5-(phenylethinyl)thiophen-2-yl)-N-(2,4,4-trimethylpentan-2-yl)imidazo[1,2-a]pyridin-3-amin
[219] 8-(Benzyloxy)-N-cyclopentyl-2-(4-(phenylethinyl)thiophen-2-yl)imidazo[1,2-a]pyridin-3-amin
[220] 8-(Benzyloxy)-N-(2-methylpentan-2-yl)-2-(4-(phenylethinyl)thiophen-2-yl)imidazo[1,2-a]pyridin-3-amin
[221] 6-Chlor-N-(4-fluorbenzyl)-2-(5-(phenylethinyl)furan-2-yl)imidazo[1,2-a]pyridin-3-amin
[222] 6-Brom-N-butyl-5-methyl-2-(4-(phenylethinyl)thiophen-2-yl)imidazo[1,2-a]pyridin-3-amin
[223] N-(Furan-2-yl)-8-methyl-2-(5-(phenylethinyl)furan-2-yl)imidazo[1,2-a]pyridin-3-amin
[224] N-(Furan-2-yl)-2-(5-(phenylethinyl)furan-2-yl)imidazo[1,2-a]pyridin-3-amin
[225] N-(Furan-2-yl)-2-(5-(phenylethinyl)furan-2-yl)-7-propylimidazo[1,2-a]pyridin-3-amin
[226] 5,7-Dimethyl-2-(4-(phenylethinyl)thiophen-2-yl)-N-(3-(trifluormethyl)phenyl)imidazo[1,2-a]pyrimidin-3-amin
[227] 6-Brom-N-(4-chlorphenethyl)-8-methyl-2-(4-(phenylethinyl)thiophen-2-yl)imidazo[1,2-a]pyridin-3-amin
[228] N-(4-Chlorphenethyl)-7-phenyl-2-(4-(phenylethinyl)thiophen-2-yl)imidazo[1,2-a]pyridin-3-amin
[229] N-Phenethyl-7-phenyl-2-(4-(phenylethinyl)thiophen-2-yl)imidazo[1,2-a]pyridin-3-amin
[230] N-(4-Chlorbenzyl)-5,7-dimethyl-2-(4-(phenylethinyl)thiophen-2-yl)imdazo[1,2-a]pyridin-3-amin
[231] 6-Brom-N-(4-chlorbenzyl)-5-methyl-2-(4-(phenylethinyl)thiophen-2-yl)imidazo[1,2-a]pyridin-3-amin
[232] 8-Brom-N-(4-chlorbenzyl)-6-methyl-2-(4-(phenylethinyl)thiophen-2-yl)imidazo[1,2-a]pyridin-3-amin
[233] N-(3-Methoxyphenethyl)-2-(4-(phenylethinyl)thiophen-2-yl)-5-propylimidazo[1,2-a]pyridin-3-amin
[234] 6-Brom-8-methyl-2-(4-(phenylethinyl)thiophen-2-yl)-N-(2-(thiophen-2-yl)ethyl)imidazo[1,2-a]pyridin-3-amin
[235] 7-Phenyl-2-(4-(phenylethinyl)thiophen-2-yl)-N-(2-(thiophen-2-yl)ethyl)imidazo[1,2-a]pyridin-3-amin
[236] 6,8-Dibrom-N-(2-methylpentan-2-yl)-2-(5-(phenylethinyl)thiophen-2-yl)imidazo[1,2-a]pyridin-3-amin
[237] 6-Brom-N-(2,6-dimethylphenyl)-8-methyl-2-(4-(phenylethinyl)thiophen-2-yl)imidazo[1,2-a]pyridin-3-amin
[238] N-Cyclohexyl-2-(3-(pyridin-2-ylethinyl)phenyl)imidazo[1,2-a]pyrazin-3-amin
[239] 2-(3-(Pyridin-2-ylethinyl)phenyl)-N-(2,4,4-trimethylpentan-2-yl)imidazo[1,2-a]pyrazin-3-amin
[240] N-Cyclopentyl-2-(4-(pyridin-2-ylethinyl)phenyl)imidazo[1,2-a]pyrazin-3-amin
[241] 8-Chlor-2-(4-(pyridin-2-ylethinyl)phenyl)-6-(trifluormethyl)-N-(2,4,4-trimethylpentan-2-yl)imidazo[1,2-a]pyridin-3-amin
[242] N-(4-Fluorphenyl)-2-(4-(pyridin-2-ylethinyl)phenyl)imidazo[1,2-a]pyrazin-3-amin
[243] N-Cyclopentyl-2-(2-methyl-6-(phenylethinyl)pyridin-3-yl)imidazo[1,2-a]-pyrazin-3-amin
[244] N-Cyclohexyl-2-(2-methyl-6-(phenylethinyl)pyridin-3-yl)imidazo[1,2-a]pyrazin-3-amin
[245] N-Cyclopentyl-2-(5-(pyridin-2-ylethinyl)furan-2-yl)imidazo[1,2-a]pyrazin-3-amin
[246] 8-Brom-N-cyclopentyl-6-methyl-2-(5-(pyridin-2-ylethinyl)furan-2-yl)imidazo-[1,2-a]pyridin-3-amin
[247] N-Cyclohexyl-2-(5-(pyridin-2-ylethinyl)furan-2-yl)imidazo[1,2-a]pyrazin-3-amin
[248] 2-(5-(Pyridin-2-ylethinyl)furan-2-yl)-N-(2,4,4-trimethylpentan-2-yl)imidazo[1,2-a]pyrazin-3-amin
[249] N-Cyclopentyl-2-(3-((6-methylpyridin-2-yl)ethinyl)phenyl)imidazo[1,2-a]pyrazin-3-amin
[250] 8-Brom-N-cyclopentyl-6-methyl-2-(4-(pyridin-2-ylethinyl)phenyl)imidazo[1,2-a]pyridin-3-amin
[251] N-Cyclohexyl-2-(3-((6-methylpyridin-2-yl)ethinyl)phenyl)imidazo[1,2-a]pyrazin-3-amin
[252] N-(4-Fluorphenyl)-2-(3-((6-methylpyridin-2-yl)ethinyl)phenyl)imidazo[1,2-a]pyrazin-3-amin
[253] 2-(5-(Pyridin-2-ylethinyl)thiophen-2-yl)-N-(2,4,4-trimethylpentan-2-yl)imidazo[1,2-a]pyrimidin-3-amin
[254] N-Cyclohexyl-2-(3-(pyridin-2-ylethinyl)phenyl)imidazo[1,2-a]pyrimidin-3-amin
[255] 2-(3-(Pyridin-2-ylethinyl)phenyl)-N-(2,4,4-trimethylpentan-2-yl)imidazo[1,2-a]pyrimidin-3-amin
[256] 2-(6-(Phenylethinyl)pyridin-3-yl)-N-(2,4,4-trimethylpentan-2-yl)imidazo[1,2-a]pyrimidin-3-amin
[257] N-Cyclopentyl-2-(5-(pyridin-2-ylethinyl)furan-2-yl)imidazo[1,2-a]pyrimidin-3-amin
[258] N-tert-Butyl-2-(5-(pyridin-2-ylethinyl)furan-2-yl)imidazo[1,2-a]pyrimidin-3-amin
[259] N-Cyclopentyl-2-(3-((6-methylpyridin-2-yl)ethinyl)phenyl)imidazo[1,2-a]pyrimidin-3-amin
[260] N-Cyclopentyl-2-(3-(pyridin-2-ylethinyl)phenyl)imidazo[1,2-a]pyridin-3-amin
[261] N-tert-Butyl-2-(3-(pyridin-2-ylethinyl)phenyl)imidazo[1,2-a]pyridin-3-amin
[262] 2-(3-(Pyridin-2-ylethinyl)phenyl)-N-(2,4,4-trimethylpentan-2-yl)imidazo[1,2-a]pyridin-3-amin
[263] N-(4-Fluorphenyl)-2-(3-(pyridin-2-ylethinyl)phenyl)imidazo[1,2-a]pyridin-3-amin
[264] N-tert-Butyl-2-(4-(pyridin-2-ylethinyl)phenyl)imidazo[1,2-a]pyridin-3-amin
[265] N-Cycopentyl-2-(2-methyl-6-(phenylethinyl)pyridin-3-yl)imidazo[1,2-a]pyridin-3-amin
[266] N-(4-Fluorphenyl)-2-(2-methyl-6-(phenylethinyl)pyridin-3-yl)imidazo[1,2-a]pyridin-3-amin
[267] N-Cyclopentyl-6-methyl-2-(5-(pyridin-2-ylethinyl)thiophen-2-yl)imidazo[1,2-a]pyridin-3-amin
[268] N-tert-Butyl-6-methyl-2-(5-(pyridin-2-ylethinyl)thiophen-2-yl)imidazo[1,2-a]pyridin-3-amin
[269] N-Cyclohexyl-6-methyl-2-(5-(pyridin-2-ylethinyl)thiophen-2-yl)imidazo(1,2-a]pyridin-3-amin
[270] 6-Methyl-2-(5-(pyridin-2-ylethinyl)thiophen-2-yl)-N-2,4,4-trimethylpentan-2-yl)imidazo[1,2-a]pyridin-3-amin
[271] 6-Methyl-2-(2-methyl-6-(phenylethinyl)pyridin-3-yl)-N-(2,4,4-trimethylpentan-2-yl)imidazo[1,2-a]pyridin-3-amin
[272] N-Cyclopentyl-6-methyl-2-(5-(pyridin-2-ylethinyl)furan-2-yl)imidazo[1,2-a]pyridin-3-amin
[273] N-Cyclohexyl-6-methyl-2-(3-((6-methylpyridin-2-yl)ethinyl)phenyl)imidazo[1,2-a]pyridin-3-amin
[274] N-Cyclohexyl-7-methyl-2-(5-(pyridin-2-ylethinyl)thiophen-2-yl)imidazo[1,2-a]pyridin-3-amin
[275] 7-Methyl-2-(5-(pyridin-2-ylethinyl)thiophen-2-yl)-N-(2,4,4-trimethylpentan-2-yl)imidazo[1,2-a]pyridin-3-amin
[276] N-tert-Butyl-7-methyl-2-(3-(pyridin-2-ylethinyl)phenyl)imidazo[1,2-a]pyridin-3-amin
[277] N-(4-Fluorphenyl)-7-methyl-2-(3-(pyridin-2-ylethinyl)phenyl)imidazo[1,2-a]pyridin-3-amin
[278] N-tert-Butyl-7-methyl-2-(4-(pyridin-2-ylethinyl)phenyl)imidazo[1,2-a]pyridin-3-amin
[279] N-(4-Fluorphenyl)-7-methyl-2-(5-(pyridin-2-ylethinyl)furan-2-yl)imidazo[1,2-a]pyridin-3-amin
[280] N-Cyclohexyl-8-methyl-2-(6-(phenylethinyl)pyridin-3-yl)imidazo[1,2-a]pyridin-3-amin
[281] N-Cyclopentyl-2-(6-(phenylethinyl)pyridin-3-yl)imidazo(1,2-a)pyridin-3-amin
[282] N-tert-Butyl-2-(6-(phenylethinyl)pyridin-3-yl)imidazo[1,2-a]pyridin-3-amin
[283] N-Cyclohexyl-2-(6-(phenylethinyl)pyridin-3-yl)imidazo[1,2-a]pyridin-3-amin
[284] 2-(6-(Phenylethinyl)pyridin-3-yl)-N-(2,4,4-trimethylpentan-2-yl)imidazo[1,2-a]pyridin-3-amin
[285] N-tert-Butyl-2-(5-(pyridin-2-ylethinyl)furan-2-yl)imidazo[1,2-a]pyridin-3-amin
[286] N-Cyclohexyl-2-(5-(pyridin-2-ylethinyl)furan-2-yl)imidazo[1,2-a]pyridin-3-amin
[287] N-(4-Fluorphenyl)-2-(5-(pyridin-2-ylethinyl)furan-2-yl)imidazo[1,2-a]pyridin-3-amin
[288] N-Cyclohexyl-2-(3-((6-methylpyridin-2-yl)ethinyl)phenyl)imidazo[1,2-a]pyridin-3-amin
[289] 2-(3-((6-Methylpyridin-2-yl)ethinyl)phenyl)-N-(2,4,4-trimethylpentan-2-yl)imidazo[1,2-a]pyridin-3-amin
[290] N-tert-Butyl-5-methyl-2-(3-((6-methylpyridin-2-yl)ethinyl)phenyl)imidazo[1,2-a]pyridin-3-amin
[291] 5-Methyl-2-(3-((6-methylpyridin-2-yl)ethinyl)phenyl)-N-(2,4,4-trimethylpentan-2-yl)imidazo[1,2-a]pyridin-3-amin
[292] N-Cyclohexyl-5,7-dimethyl-2-(3-(pyridin-2-ylethinyl)phenyl)imidazo[1,2-a]pyrimidin-3-amin
[293] N-Cyclohexyl-5,7-dimethyl-2-(2-methyl-6-(phenylethinyl)pyridin-3-yl)imidazo[1,2-a]pyrimidin-3-amin
[294] N-Cyclopentyl-5,7-dimethyl-2-(5-(pyridin-2-ylethinyl)furan-2-yl)imidazo[1,2-a]pyrimidin-3-amin
[295] N-tert-Butyl-5,7-dimethyl-2-(5-(pyridin-2-ylethinyl)furan-2-yl)imidazo[1,2-a]pyrimidin-3-amin
[296] N-(4-Fluorphenyl)-8-methyl-2-(3-(pyridin-2-ylethinyl)phenyl)imidazo[1,2-a]pyridin-3-amin
[297] N-Cyclohexyl-8-methyl-2-(4-(pyridin-2-ylethinyl)phenyl)imidazo[1,2-a]pyridin-3-amin
[298] N-Cyclohexyl-7-ethyl-2-(5-(pyridin-2-ylethinyl)thiophen-2-yl)imidazo[1,2-a]pyridin-3-amin
[299] 7-Ethyl-2-(5-(pyridin-2-ylethinyl)thiophen-2-yl)-N-(2,4,4-trimethylpentan-2-yl)imidazo[1,2-a]pyridin-3-amin
[300] N-Cyclohexyl-7-ethyl-2-(3-(pyridin-2-ylethinyl)phenyl)imidazo[1,2-a]pyridin-3-amin
[301] N-Cyclopentyl-7-ethyl-2-(4-(pyridin-2-ylethinyl)phenyl)imidazo[1,2-a]pyridin-3-amin
[302] N-Cyclopentyl-7-ethyl-2-(6-(phenylethinyl)pyridin-3-yl)imidazo[1,2-a]pyridin-3-amin
[303] N-tert-Butyl-7-ethyl-2-(6-(phenylethinyl)pyridin-3-yl)imidazo[1,2-a]pyridin-3-amin
[304] N-tert-Butyl-7-ethyl-2-(5-(pyridin-2-ylethinyl)furan-2-yl)imidazo[1,2-a]pyridin-3-amin
[305] 7-Isopropyl-2-(5-(pyridin-2-ylethinyl)thiophen-2-yl)-N-(2,4,4-trimethylpentan-2-yl)imidazo[1,2-a]pyridin-3-amin
[306] N-tert-Butyl-7-isopropyl-2-(3-(pyridin-2-ylethinyl)phenyl)imidazo[1,2-a]pyridin-3-amin
[307] N-tert-Butyl-7-isopropyl-2-(4-(pyridin-2-ylethinyl)phenyl)imidazo[1,2-a]pyridin-3-amin
[308] N-Cyclohexyl-7-isopropyl-2-(5-(pyridin-2-ylethinyl)furan-2-yl)imidazo[1,2-a]pyridin-3-amin
[309] N-tert-Butyl-7-isopropyl-2-(3-((6-methylpyridin-2-yl)ethinyl)phenyl)imidazo[1,2-a]pyridin-3-amin
[310] 6-Chlor-N-cyclopentyl-2-(5-(pyridin-2-ylethinyl)thiophen-2-yl)imidazo[1,2-a]pyridin-3-amin
[111] N-tert-Butyl-6-chlor-2-(5-(pyridin-2-ylethinyl)thiophen-2-yl)imidazo[1,2-a]pyridin-3-amin
[312] 6-Chlor-N-cyclohexyl-2-(3-(pyridin-2-ylethinyl)phenyl)imidazo[1,2-a]pyridin-3-amin
[313] 6-Chlor-2-(3-(pyridin-2-ylethinyl)phenyl)-N-(2,4,4-trimethylpentan-2-yl)imidazo[1,2-a]pyridin-3-amin
[314] N-tert-Butyl-6-chlor-2-(4-(pyridin-2-ylethinyl)phenyl)imidazo[1,2-a]pyridin-3-amin
[315] 6-Chlor-N-cyclohexyl-2-(4-(pyridin-2-ylethinyl)phenyl)imidazo[1,2-a]pyridin-3-amin
[316] N-tert-Butyl-6-chlor-2-(5-(pyridin-2-ylethinyl)furan-2-yl)imidazo[1,2-a]pyridin-3-amin
[317] 6-Chlor-N-cyclohexyl-2-(5-(pyridin-2-ylethinyl)furan-2-yl)imidazo[1,2-a]-pyridin-3-amin
[318] 6-Chlor-2-(5-(pyridin-2-ylethinyl)furan-2-yl)-N-(2,4,4-trimethylpentan-2-yl)imidazo[1,2-a]pyridin-3-amin
[319] 6-Chlor-N-cyclopentyl-2-(3-((6-methylpyridin-2-yl)ethinyl)phenyl)imidazo[1,2-a]pyridin-3-amin
[320] N-tert-Butyl-6-chlor-2-(3-((6-methylpyridin-2-yl)ethinyl)phenyl)imidazo[1,2-a]pyridin-3-amin
[321]N-tert-Butyl-2-(5-(pyridin-2-ylethinyl)thiophen-2-yl)imidazo[1,2-a]pyridin-3-amin Bis-Hydrochlorid
[322] N-Methyl-2-(5-(pyridin-2-ylethinyl)thiophen-2-yl)imidazo[1,2-a]pyrazin-3-amin
[323] N-tert-Butyl-2-(5-(pyridin-2-ylethinyl)thiophen-2-yl)imidazo[1,2-a]pyridin-3-amin Bis-Hydrochlorid
[324] [2-(5-Pyridin-2-ylethinyl-thiophen-2-yl)-imidazo[1,2-a]pyridin-3-yl]-(1,1,3,3-tetramethyl-butyl)-amin
[325] tert-Butyl-[2-(5-pyrimidin-2-ylethinyl-thiophen-2-yl)-imidazo[1,2-a]pyridin-3-yl]-amin Hydrochlorid
[326] {2-[5-(3-Amino-pyridin-2-ylethinyl)-thiophen-2-yl]-imidazo[1,2-a]pyridin-3-yl}-tert-butyl-amin
[327] tert-Butyl-[2-(5-pyridin-2-ylethinyl-thiazol-2-yl)-imidazo[1,2-a]pyridin-3-yl]-amin
[328] tert-Butyl-[2-(2-pyridin-2-ylethinyl-thiazol-5-yl)-imidazo[1,2-a]pyridin-3-yl]-amin
[329] tert-Butyl-{2-[5-(6-fluor-pyridin-2-ylethinyl)-thiophen-2-yl]-imidazo[1,2-a]pyridin-3-yl}-amin Hydrochlorid
[330] ten-Butyl-{2-[5-(3-chlor-5-fluor-phenylethinyl)-thiophen-2-yl]-imidazo[1,2-a]pyridin-3-yl}-amin Hydrochlorid
[331] tert-Butyl-[2-(5-pyridin-2-ylethinyl-thiophen-2-yl)-imidazo[1,2-b]pyridazin-3-yl]-amin Hydrochlorid
[332] tert-Butyl-[2-(5-thiazol-2-ylethinyl-thiophen-2-yl)-imidazo[1,2-a]pyridin-3-yl]-amin Hydrochlorid
[333] tert-Butyl-{2-[5-(3-trifluormethoxy-phenylethinyl)-thiophen-2-yl]-imidazo[1,2-a]pyrazin-3-yl}-amin
[334] tert-Butyl-{2-[5-(3-[1,3]dioxolan-2-yl-phenylethinyl)-thiophen-2-yl]-imidazo[1,2-a]pyridin-3-yl}-amin
[335] tert-Butyl-{2-[5-(3,5-dimethyl-phenylethinyl)-thiophen-2-yl]-imidazo[1,2-a]pyridin-3-yl}-amin Hydrochlorid
[336] tert-Butyl-{2-[5-(3-fluor-pyridin-2-ylethinyl)-thiophen-2-yl]-imidazo[1,2-a]pyridin-3-yl}-amin Hydrochlorid
[337] tert-Butyl-{2-[5-(3-methyl-3H-imidazol-4-ylethinyl)-thiophen-2-yl]-imidazo[1,2-a]pyridin-3-yl}-amin Hydrochlorid
[338] tert-Butyl-{2-[5-(5-chlor-thiophen-2-ylethinyl)-thiophen-2-yl]-imidazo[1,2-a]pyridin-3-yl}-amin Hydrochlorid
[339] tert-Butyl-{2-[5-(5-methyl-pyridin-2-ylethinyl)-thiophen-2-yl]-imidazo[1,2-a]pyridin-3-yl}-amin Hydrochlorid
[340] 1-{3-[5-(3-tert-Butylamino-imidazo[1,2-a]pyridin-2-yl)-thiophen-2-ylethinyl]-phenyl}-ethanon Hydrochlorid
[341] {3-[5-(3-tert-Butylamino-imidazo[1,2-a]pyridin-2-yl)-thiophen-2-ylethinyl]-phenyl}-methanol Hydrochlorid
[342] N-tert-Butyl-2-(5-((3-methoxypyridin-2-yl)ethinyl)thiophen-2-yl)imidazo[1,2-a]pyridin-3-amin
[343] N-tert-Butyl-2-(5-(thiophen-2-ylethinyl)thiophen-2-yl)imidazo[1,2-a]pyridin-3-amin Hydrochlorid
[344] 5-((5-(3-(tert-Butylamino)imidazo[1,2-a]pyridin-2-yl)thiophen-2-yl)ethinyl)-2-fluorbenzonitril Hydrochlorid
[345] N-tert-Butyl-2-(5-((3,4-difluorphenyl)ethinyl)thiophen-2-yl)imidazo[1,2-a]pyridin-3-amin
[346] N-tert-Butyl-2-(5-((3-(methoxymethyl)phenyl)ethinyl)thiophen-2-yl)imidazo[1,2-a]pyridin-3-amin
[347] 2-(5-((3-Aminophenyl)ethinyl)thiophen-2-yl)-N-tert-butylimidazo[1,2-a]pyridin-3-amin Hydrochlorid
[348] N-tert-Butyl-2-(5-((4-fluor-3-methylphenyl)ethinyl)thiophen-2-yl)imidazo[1,2-a]pyridin-3-amin Hydrochlorid
[349] N-tert-Butyl-2-(5-((3,5-difluorphenyl)ethinyl)thiophen-2-yl)imidazo[1,2-a]pyridin-3-amin
[350] N-tert-Butyl-2-(5-(thiophen-3-ylethinyl)thiophen-2-yl)imidazo[1,2-a]pyridin-3-amin Hydrochlorid
[351] N-tert-Butyl-2-(5-((3-methylpyridin-2-yl)ethinyl)thiophen-2-yl)imidazo[1,2-a]pyridin-3-amin Hydrochlorid
[352] 3-((5-(3-(tert-Butylamino)imidazo[1,2-a]pyridin-2-yl)thiophen-2-yl)ethinyl)benzolsulfonamid Hydrochlorid
[353] 3-((5-(3-(tert-Butylamino)imidazo[1,2-a]pyridin-2-yl)hiophen-2-yl)ethinyl)benzoesäure
[354] 3-((5-(3-(tert-Butylamino)imidazo[1,2-a]pyridin-2-yl)thiophen-2-yl)ethinyl)benzamid Hydrochlorid
[355] N-(3-((5-(3-(tert-Butylamino)imidazo[1,2-a]pyridin-2-yl)thiophen-2-yl)ethinyl)phenyl)acetamid
[356] N-tert-Butyl-N-methyl-2-(5-(pyridin-2-ylethinyl)thiophen-2-yl)imidazo[1,2-a]pyridin-3-amin
[357] N,N-Dimethyl-2-(5-(pyridin-2-ylethinyl)thiophen-2-yl)imidazo[1,2-a]pyridin-3-amin
[358] N-tert-Butyl-N-methyl-2-(5-(pyridin-2-ylethinyl)thiazol-2-yl)imidazo[1,2-a]pyridin-3-amin
[359] (6-((5-(3-(tert-Butylamino)imidazo[1,2-a]pyridin-2-yl)thiophen-2-yl)ethinyl)pyridin-2-yl)methanol
[360] N-(3-((5-(3-(tert-Butylamino)imidazo[1,2-a]pyridin-2-yl)thiophen-2-yl)ethinyl)phenyl)methansulfonamid
[361] N-tert-Butyl-8-methyl-2-(5-(pyridin-2-ylethinyl)thiophen-2-yl)imidazo[1,2-a]pyrazin-3-amin Hydrochlorid
[362] 2-(5-(Pyridin-2-ylethinyl)thiophen-2-yl)imidazo[1,2-a]pyridin-3-amin
[363] N-tert-Butyl-2-(5-((3-chlorphenyl)ethinyl)thiophen-2-yl)imidazo[1,2-a]pyridin-3-amin
[364] N-tert-Butyl-2-(5-((2,3-difluorphenyl)ethinyl)thiophen-2-yl)imidazo[1,2-a]pyridin-3-amin
   und
[365] N-tert-Butyl-7-chlor-2-(5-(pyridin-2-ylethinyl)thiophen-2-yl)imidazo[1,2-a]pyridin-3-amin und jeweils deren entsprechenden Salzen, insbesondere deren Hydrochloriden, und jeweils deren entsprechenden Solvaten.

Ein weiterer Gegenstand der vorliegenden Erfindung ist ein Verfahren zur Herstellung von Verbindungen der vorstehend angegebenen allgemeinen Formel I, gemäß dem wenigstens eine Verbindung der allgemeinen Formel II, 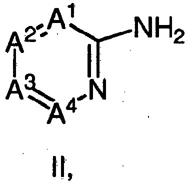 worin A¹, A², A³ und A⁴ die vorstehend angegebene Bedeutung haben, in einem Reaktionsmedium, ggf. in Gegenwart wenigstens einer organischen oder anorganischen Säure oder wenigstens eines Übergangsmetallsalzes mit wenigstens einem Isocyanid der allgemeinen Formel III,

R²-N≡C III,

worin R² die vorstehend angegebene Bedeutung hat, und wenigstens einem Aldehyd der allgemeinen Formel IV, 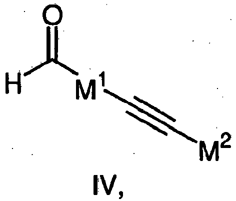 worin M¹ und M² die vorstehend angegebene Bedeutung haben, umgesetzt und die so erhaltene Verbindung der allgemeinen Formel V, 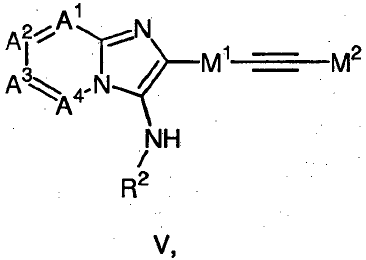 worin A¹, A², A³, A⁴, R², M¹ und M² die vorstehend genannte Bedeutung haben, ggf. gereinigt und/oder isoliert wird, und ggf. in ein entsprechendes Salz überführt und dieses ggf. gereinigt und/oder isoliert wird,
oder wenigstens eine Verbindung der allgemeinen Formel II, 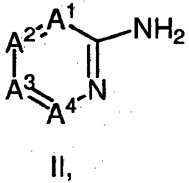 worin A¹, A², A³ und A⁴ die vorstehend angegebene Bedeutung haben, in einem Reaktionsmedium, ggf. in Gegenwart wenigstens einer organischen oder anorganischen Säure oder wenigstens eines Übergangsmetallsalzes mit wenigstens einem Isocyanid der allgemeinen Formel III,

R²-N≡C III,

worin R² die vorstehend angegebene Bedeutung hat, und wenigstens einem Aldehyd der allgemeinen Formel VI, 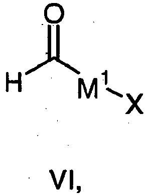 worin M¹ die vorstehend angegebene Bedeutung hat und X für eine Abgangsgruppe, vorzugsweise für einen Halogen-Rest oder für einen Sulfonsäureester, besonders bevorzugt für Chlor, Brom oder Trifluormethansulfonat, steht, umgesetzt und die so erhaltene Verbindung der allgemeinen Formel VII, 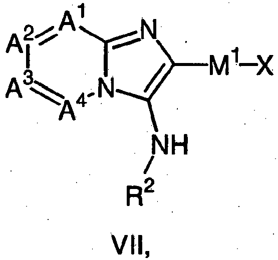 worin A¹, A², A³, A⁴, R², M¹ und X die vorstehend genannte Bedeutung haben, ggf. gereinigt und/oder isoliert wird, und ggf. in ein entsprechendes Salz überführt und dieses ggf. gereinigt und/oder isoliert wird, und
durch Umsetzung mit wenigstens einem Acetylen der allgemeinen Formel XI, 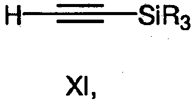 worin R, unabhängig voneinander, jeweils für einen linearen oder verzweigten Alkyl-Rest oder für einen unsubstituierten Phenyl-Rest steht, in einem Reaktionsmedium, ggf. in Gegenwart wenigstens eines geeigneten Katalysators, ggf. in Gegenwart wenigstens eines Kupfer(I)salzes, bevorzugt in Gegenwart von Kupfer-(I)-iodid, und ggf. in Gegenwart wenigstens einer anorganischen und/oder organischen Base in eine entsprechend substituierte Verbindung der allgemeinen Formel XII, 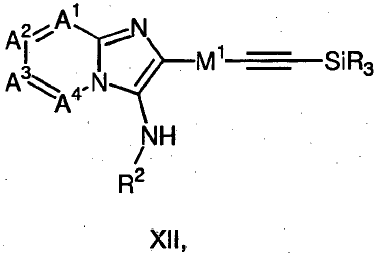 worin A¹, A², A³, A⁴, R² und M¹ die vorstehend genannte Bedeutung haben und R, unabhängig voneinander, jeweils für einen linearen oder verzweigten Alkyl-Rest oder für einen unsubstituierten Phenyl-Rest steht, überführt und ggf. gereinigt und/oder isoliert wird, und ggf. in ein entsprechendes Salz überführt und dieses ggf. gereinigt und/oder isoliert wird,
und wenigstens eine Verbindung der allgemeinen Formel XII in einem Reaktionsmedium, ggf. in Gegenwart wenigstens einer anorganischen und/oder organischen Base, ggf. in Gegenwart wenigstens eines anorganischen Salzes, und ggf. in Gegenwart wenigstens eines Ammoniumsalzes, in eine entsprechend substituierte Verbindung der allgemeinen Formel XIII, 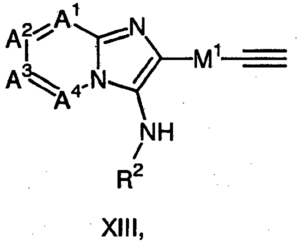 worin A¹, A², A³, A⁴, R² und M¹ die vorstehend genannte Bedeutung haben, überführt und ggf. gereinigt und/oder isoliert wird, und ggf. in ein entsprechendes Salz überführt und dieses ggf. gereinigt und/oder isoliert wird,
und wenigstens eine Verbindung der allgemeinen Formel XIII und/oder wenigstens eine Verbindung der allgemeinen Formel XII durch Umsetzung mit wenigstens einer Verbindung der allgemeinen Formel M²-X, worin M² die vorstehend genannte Bedeutung hat und X für eine Abgangsgruppe, vorzugsweise für einen Halogen-Rest oder für einen Sulfonsäureester, besonders bevorzugt für Chlor, Brom oder Trifluormethansulfonat steht, in einem Reaktionsmedium, ggf. in Gegenwart wenigstens eines geeigneten Katalysators, ggf. in Gegenwart wenigstens einer anorganischen und/oder organischen Base, ggf. in Gegenwart wenigstens eines anorganischen Salzes und ggf. in Gegenwart wenigstens eines Ammoniumsalzes in eine entsprechend substituierte Verbindung der allgemeinen Formel V, 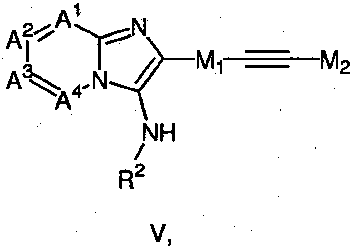 worin A¹, A², A³, A⁴, R², M¹ und M² die vorstehend genannte Bedeutung haben überführt und ggf. gereinigt und/oder isoliert wird, und ggf. in ein entsprechendes Salz überführt und dieses ggf. gereinigt und/oder isoliert wird,
oder eine Verbindung der allgemeinen Formel VII durch Umsetzung mit wenigstens einem Acetylen der allgemeinen Formel VIII, 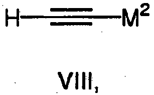 worin M² die vorstehend angegebene Bedeutung hat, in einem Reaktionsmedium, ggf. in Gegenwart wenigstens eines geeigneten Katalysators, ggf. in Gegenwart wenigstens eines Kupfer(1)salzes, bevorzugt in Gegenwart von Kupfer-(I)-iodid und ggf. in Gegenwart wenigstens einer anorganischen und/oder organischen Base in eine entsprechend substituierte Verbindung der allgemeinen Formel V, 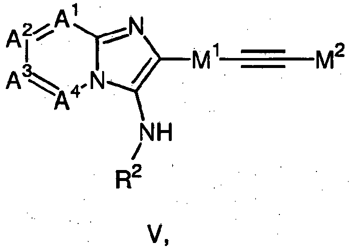 worin A¹, A², A³, A⁴, R², M¹ und M² die vorstehend genannte Bedeutung haben überführt und ggf. gereinigt und/oder isoliert wird, und ggf. in ein entsprechendes Salz überführt und dieses ggf. gereinigt und/oder isoliert wird,
und ggf. die Verbindung der allgemeinen Formel V durch Umsetzung mit wenigstens einer Verbindung der allgemeinen Formel R³-X, worin R³ die vorstehend angegebene Bedeutung hat und X für eine Abgangsgruppe, bevorzugt für einen Halogen-Rest oder für einen Sulfonsäureester, besonders bevorzugt für Chlor, steht, in einem Reaktionsmedium, in Gegenwart wenigstens einer organischen oder anorganischen Base, vorzugsweise in Gegenwart wenigstens eines Metallhydridsalzes,
oder durch Umsetzung mit wenigstens einer Verbindung der allgemeinen Formel R²⁰-C(=O)-OH, worin R²⁰ die vorstehend angegebene Bedeutung hat, in einem Reaktionsmedium, ggf. in Gegenwart wenigstens einer organischen oder anorganischen Base und/oder in Gegenwart wenigstens eines Kopplungsmittels, oder durch Umsetzung mit wenigstens einer Verbindung der allgemeinen Formel R²³-C(=O)-X, worin R²⁰ die vorstehend angegebene Bedeutung hat und X für eine Abgangsgruppe, bevorzugt für einen Halogen-Rest oder für einen Sulfonsäureester, besonders bevorzugt für Chlor, Brom oder Trifluormethansulfonat, steht, in einem Reaktionsmedium, ggf. in Gegenwart wenigstens einer organischen oder anorganischen Base,
oder durch Umsetzung mit wenigstens einer Verbindung der allgemeinen Formel R²⁰-C(=O)-H, worin R²⁰ die vorstehend angegebene Bedeutung hat, in einem Reaktionsmedium, ggf. in Gegenwart wenigstens eines Reduktionsmittels,
in eine Verbindung der allgemeinen Formel I, ggf. in Form eines entsprechenden Salzes, überführt wird, 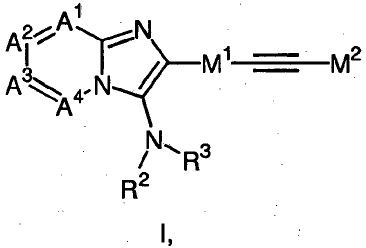 worin A¹, A², A³, A⁴, R², R³, M¹ und M² die vorstehend angegebene Bedeutung haben, und diese ggf. gereinigt und/oder isoliert wird.

Ebenfalls ein Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel V, 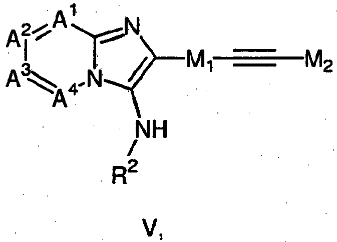 worin A¹, A², A³, A⁴, R², M¹ und M² die vorstehend genannte Bedeutung haben, ggf. in einem Reaktionsmedium in Gegenwart wenigstens einer organischen oder anorganischen Säure, umgesetzt, und die so erhaltene Verbindung der allgemeinen Formel IX, 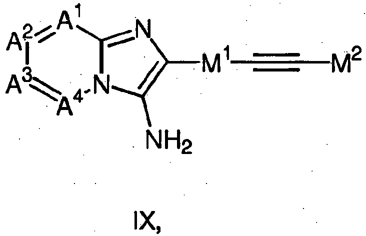 worin A¹, A², A³, A⁴, M¹ und M² die vorstehend genannte Bedeutung haben, ggf. gereinigt und/oder isoliert wird, und ggf. in ein entsprechendes Salz überführt und dieses ggf. gereinigt und/oder isoliert wird und in einem Reaktionsmedium, in Gegenwart wenigstens einer anorganischen oder organischen Base, vorzugsweise in Gegenwart wenigstens eines Metallhydridsalzes, mit wenigstens einer Verbindung der allgemeinen Formel R³-X, worin R³ die vorstehend angegebene Bedeutung hat und X für eine Abgangsgruppe, bevorzugt für einen Halogen-Rest oder für einen Sulfonsäureester, besonders bevorzugt für Chlor, steht, oder
in einem Reaktionsmedium, ggf. in Gegenwart wenigstens einer organischen oder anorganischen Base und/oder ggf. in Gegenwart wenigstens eines Kopplungsmittels mit wenigstens einer Verbindung der allgemeinen Formel R²⁰-C(=O)-OH, worin R²⁰ die vorstehend genannte Bedeutung hat,
oder in einem Reaktionsmedium, ggf. in Gegenwart wenigstens einer organischen oder anorganischen Base mit wenigstens einer Verbindung der allgemeinen Formeln R²⁰-C(=O)-X, worin R²⁰ die vorstehend genannte Bedeutung hat und X für eine Abgangsgruppe, vorzugsweise für einen Halogen-Rest oder für einen Sulfonsäureester, besonders bevorzugt für Chlor, Brom oder Trifluormethansulfonat, steht,
oder in einem Reaktionsmedium, ggf. in Gegenwart wenigstens eines Reduktionsmittels mit wenigstens einer Verbindung der allgemeinen Formel R²⁰-C(=O)-H, worin R²⁰ die vorstehend genannte Bedeutung hat,

in eine entsprechende Verbindung der allgemeinen Formel X, ggf. in Form eines entsprechenden Salzes, überführt wird, 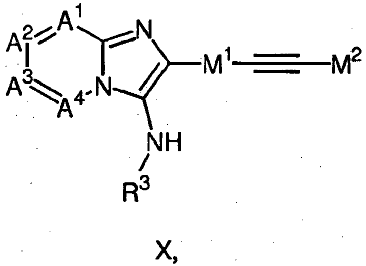 worin A¹, A², A³, A⁴, R³, M¹ und M² die vorstehend genannte Bedeutung haben, und diese ggf. gereinigt und/oder isoliert wird,
und ggf. die Verbindung der allgemeinen Formel X durch Umsetzung mit wenigstens einer Verbindung der allgemeinen Formel R²-X, worin R² die vorstehend genannte Bedeutung hat und X für eine Abgangsgruppe, bevorzugt für einen Halogen-Rest oder für einen Sulfonsäureester, besonders bevorzugt für Chlor, steht, in einem Reaktionsmedium, in Gegenwart wenigstens einer organischen oder anorganischen Base, vorzugsweise in Gegenwart wenigstens eines Metallhydridsalzes,
oder durch Umsetzung mit wenigstens einer Verbindung der allgemeinen Formel R²⁰-C(=O)-OH, worin R²⁰ vorstehend genannte Bedeutung hat, in einem Reaktionsmedium, ggf. in Gegenwart wenigstens einer organischen oder anorganischen Base und/oder in Gegenwart wenigstens eines Kopplungsmittels, oder durch Umsetzung mit wenigstens einer Verbindung der allgemeinen Formel R²⁰-C(=O)-X, worin R²⁰ die vorstehend genannte Bedeutung hat und X für eine Abgangsgruppe, bevorzugt für einen Halogen-Rest oder für einen Sulfonsäureester, besonders bevorzugt für Chlor, Brom oder Trifluormethansulfonat, steht, in einem Reaktionsmedium, ggf. in Gegenwart wenigstens einer organischen oder anorganischen Base,
oder durch Umsetzung mit wenigstens einer Verbindung der allgemeinen Formel R²⁰-C(=O)-H, worin R²⁰ die vorstehend genannte Bedeutung hat, in einem Reaktionsmedium, ggf. in Gegenwart wenigstens eines Reduktionsmittels,
in eine Verbindung der vorstehend angegebenen allgemeinen Formel I, ggf. in Form eines entsprechenden Salzes, überführt wird, 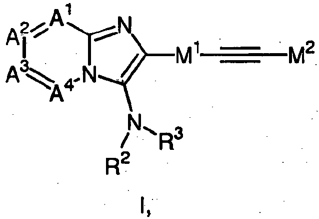 worin A¹, A², A³, A⁴, R², R³, M¹ und M² die vorstehend genannte Bedeutung haben, und diese ggf. gereinigt und/oder isoliert wird.

Die erfindungsgemäßen Verfahren zur Herstellung substituierter Imidazo-3-yl-amin-Verbindungen der vorstehend angegebenen allgemeinen Formel I sind auch in den nachfolgenden Schemata 1 bis 4 angegeben.

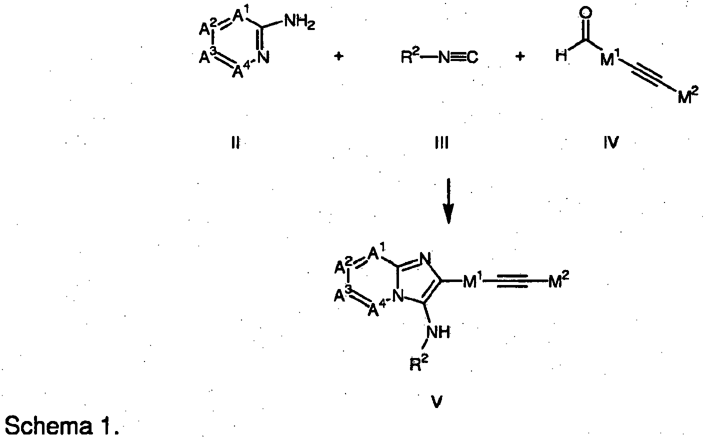

In einer Drei-Komponenten-Kopplungsreaktion werden Amine der allgemeinen Formel II mit Isocyaniden der allgemeinen Formel III und Aldehyden der allgemeinen Formel IV in einem Reaktionsmedium, vorzugsweise ausgewählt aus der Gruppe bestehend aus Chloroform, Dichlormethan, Acetonitril, Methanol und Ethanol, unter Zusatz wenigstens einer organischen oder anorganischen Säure, vorzugsweise Trifluoressigsäure oder Perchlorsäure, oder unter Zusatz wenigstens eines Übergangsmetallsalzes, vorzugsweise unter Zusatz wenigstens eines Übergangsmetalltriflats (Übergangsmetalltrifluormethansulfonats), besonders bevorzugt unter Zusatz wenigstens eines Übergangsmetalltriflats ausgewählt aus der Gruppe bestehend aus Scandium(III)trifluormethansulfonat, Ytterbiumtrifluormethansulfonat und Indium(III)trifluormethansulfonat, vorzugsweise bei Temperaturen von 0°C bis 150°C zu Verbindungen der allgemeinen Formel V umgesetzt.

Ein weiteres Verfahren zur Herstellung substituierter Imidazo-3-yl-amin-Verbindungen der vorstehend angegebenen allgemeinen Formel I ist in Schema 2 wiedergegeben.

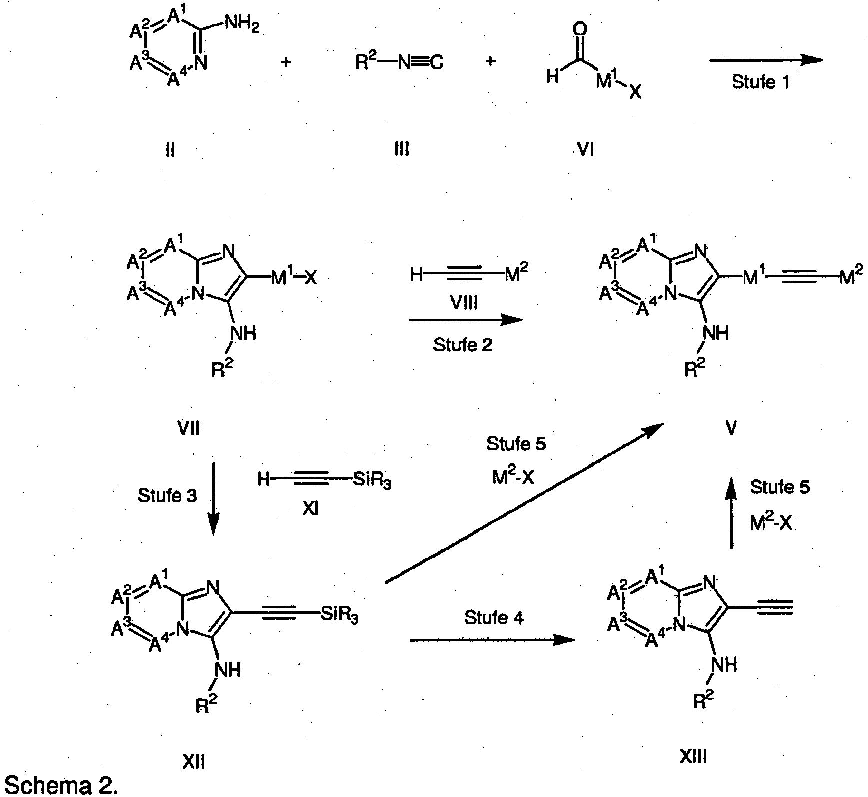

In Stufe 1 werden in einer Drei-Komponenten-Kopplungsreaktion Amine der allgemeinen Formel II mit Isocyaniden der allgemeinen Formel III und Aldehyden der allgemeinen Formel VI, worin X für eine Abgangsgruppe, vorzugsweise für einen Halogen-Rest oder für einen Sulfonsäureester, besonders bevorzugt für Chlor, Brom oder Trifluormethansulfonat, steht, in einem Reaktionsmedium, vorzugsweise ausgewählt aus der Gruppe bestehend aus Chloroform, Dichlormethan, Acetonitril, Methanol und Ethanol, unter Zusatz wenigstens einer organischen oder anorganischen Säure, vorzugsweise ausgewählt aus der Gruppe bestehend aus Trifluoressigsäure oder Perchlorsäure, oder unter Zusatz wenigstens eines Übergangsmetallsalzes, vorzugsweise unter Zusatz wenigstens eines Übergangsmetalltriflats (Übergangsmetalltrifluormethansulfonats), besonders bevorzugt unter Zusatz wenigstens eines Übergangsmetalltrifluormethansulfonats ausgewählt aus der Gruppe bestehend aus Scandium(III)trifluormethansulfonat, Ytterbiumtrifluormethansulfonat und Indium(III) trifluormethansulfonat, vorzugsweise bei Temperaturen von 0°C bis 150°C zu Verbindungen der allgemeinen Formel VII, worin X für eine Abgangsgruppe, vorzugsweise für einen Halogen-Rest oder für einen Sulfonsäureester, besonders bevorzugt für Chlor, Brom oder Trifluormethansulfonat, steht, umgesetzt.

In Stufe 2 werden Verbindungen der vorstehend angegebenen allgemeinen Formel VII, worin X für einen Halogen-Rest oder einen Sulfonsäureester, besonders bevorzugt für Chlor, Brom oder Trifluormethansulfonat, steht, mit Acetylenen der allgemeinen Formel VIII in einem Reaktionsmedium, vorzugsweise ausgewählt aus der Gruppe bestehend aus Methanol, Ethylacetat, Ethanol, Isopropanol, n-Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Dimethylformamid, Ethylacetat, Tetrahydrofuran, Wasser und entsprechenden Mischungen,vorzugsweise unter Zusatz wenigstens eines Palladiumkatalysators, vorzugsweise ausgewählt aus der-Gruppe bestehend aus Palladium(II)-dichlorid [PdCl₂], Bis(triphenylphosphin)-palladium(II)-acetat [Pd(PPh₃)₂(OAc)₂], Bis(triphenylphosphin)-palladium(II)-chlorid [PdCl₂(PPh₃)₂], Palladium(II)-acetat [Pd(OAc)₂; Ac = Acetat], Bis(acetonitril)-palladium(11)-chlorid [(CH₃CN)₂)PdCl₂], Bis(benzonitril)-palladium(II)-chlorid [(PhCN)₂PdCl₂] und Tetrakis(triphenylphosphin)palladium [(PPh₃)₄Pd], besonders bevorzugt ausgewählt aus der Gruppe bestehend aus Pd(PPh₃)₂(OAc)₂, (PPh₃)₄Pd und PdCl₂(PPh₃)₂, ggf. in Gegenwart wenigstens eines Kupfer(I)salzes, vorzugsweise in Gegenwart von Kupfer(1)-iodid, ggf. in Gegenwart wenigstens eines Phosphins, vorzugsweise eines Phosphins ausgewählt aus der Gruppe bestehend aus Triphenylphosphin, Tri-(tert-butyl)-phosphin, Triphenylarsin und Tri-(ortho-toluyl)-phosphin, besonders bevorzugt in Gegenwart von Triphenylphosphin, ggf. unter Zusatz wenigstens eines anorganischen Salzes, bevorzugt unter Zusatz von Lithium- und/oder Zinkchlorid, ggf. unter Zusatz wenigstens einer organischen Base, vorzugsweise einer organischen Base ausgewählt aus der Gruppe bestehend aus Triethylamin, Diisopropylamin, Diisopropylethylamin und [1,4]-Diazabicyclo-[2.2.2]octan und/oder Zusatz wenigstens einer anorganischen Base, vorzugsweise ausgewählt aus der Gruppe bestehend aus Kaliumcarbonat, Natriumhydrogencarbonat und Cäsiumcarbonat, wobei insbesondere die organische Base auch das Reaktionsmedium sein kann, bei Temperaturen von vorzugsweise -70 °C bis 300 °C, besonders bevorzugt von -70°C bis 150°C, zu Verbindungen der allgemeinen Formel V umgesetzt.

In Stufe 3 werden Verbindungen der vorstehend angegeben allgemeinen Formel VII mit Verbindungen der vorstehend angegebenen allgemeinen Formel XI unter den in Schema 2, Stufe 2, genannten Bedingungen zu Verbindungen der allgemeinen Formel XII umgesetzt.

In Stufe 4 werden Verbindungen der vorstehend angegebenen allgemeinen Formel XII in einem Reaktionsmedium, vorzugsweise ausgewählt aus der Gruppe bestehend aus Methanol, Ethylacetat, Ethanol, Isopropanol, n-Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Dimethylformamid, Ethylacetat, Tetrahydrofuran, Wasser und entsprechenden Mischungen, ggf. in Gegenwart wenigstens einer anorganischen Base, bevorzugt in Gegenwart wengistens einer anorganischen Base ausgewählt aus der Gruppe bestehend aus Kaliumcarbonat, Natriumhydroxid, Kaliumhydrogencarbonat, Natriumhydrogencarbonat, Kaliumhydroxid und Lithiumhydroxid, ggf. in Gegenwart wenigstens einer anorganischen Base, bevorzugt wenigstens einer anorganischen Base ausgewählt aus der Gruppe bestehend aus Triethylamin und Pyridin, ggf. in Gegenwart wenigstens eines anorganischen Salzes, bevorzugt in Gegenwart wenigstens eines Ammoniumsalzes oder in Gegenwart von Kalium- und/oder Natriumfluorid, besonders bevorzugt in Gegenwart wenigstens eines Ammoniumsalzes ausgewählt aus der Gruppe bestehend aus Tetra-n-butylammoniumfluorid, Tetra-n-butyl-ammoniumiodid und Tetrabutylammoniumbromid, bei Temperaturen von vorzugsweise -70 °C bis 300 °C, besonders bevorzugt von -70°C bis 150°C, zu Verbindungen der allgemeinen Formel XIII umgesetzt.

In Stufe 5 werden Verbindungen der vorstehend angegebenen allgemeinen Formeln XII und XIII mit Verbindungen der allgemeinen Formel M²-X, worin X für einen Halogen-Rest oder einen Sulfonsäureester, besonders bevorzugt für Chlor, Brom oder Trifluormethansulfonat, steht, in einem Reaktionsmedium, vorzugsweise ausgewählt aus der Gruppe bestehend aus Methanol, Ethylacetat, Ethanol, Isopropanol, n-Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Dimethylformamid, Ethylacetat, Tetrahydrofuran, Wasser und entsprechenden Mischungen,vorzugsweise unter Zusatz wenigstens eines Palladiumkatalysators, vorzugsweise ausgewählt aus der Gruppe bestehend aus Palladium(II)-dichlorid [PdCl₂], Bis(triphenylphosphin)-palladium(II)-acetat [Pd(PPh₃)₂(OAc)₂], Bis(triphenylphosphin)-palladium(II)-chlorid [PdCl2(PPh₃)₂], Palladium(II)-acetat [Pd(OAc)₂; Ac = Acetat], Bis(acetonitril)-palladium(II)-chlorid [(CH₃CN)₂)PdCl₂], Bis(benzonitril)-palladium(II)-chlorid [(PhCN)₂PdCl₂] und Tetrakis(triphenylphosphin)palladium [(PPh₃)₄Pd], besonders bevorzugt ausgewählt aus der Gruppe bestehend aus Pd(PPh₃)₂(OAc)₂, (PPh₃)₄Pd und PdCl₂(PPh₃)₂, ggf. in Gegenwart wenigstens eines Kupfer(I)salzes, vorzugsweise in Gegenwart von Kupfer(I)-iodid, ggf. in Gegenwart wenigstens eines Phosphins, vorzugsweise eines Phosphins ausgewählt aus der Gruppe bestehend aus Triphenylphosphin, Tri-(tert-butyl)-phosphin, Triphenylarsin und Tri-(ortho-toluyl)-phosphin, besonders bevorzugt in Gegenwart von Triphenylphosphin, ggf. unter Zusatz wenigstens eines anorganischen Salzes, bevorzugt unter Zusatz von Lithium-und/oder Zinkchlorid, ggf. in Gegenwart wenigstens eines Ammoniumsalzes oder in Gegenwart von Kalium- und/oder Natriumfluorid, bevorzugt in Gegenwart wenigstens eines Ammoniumsalzes ausgewählt aus der Gruppe bestehend aus Tetra-n-butylammoniumfluorid, Tetra-n-butyl-ammoniumiodid und Tetrabutylammoniumbromid, ggf. unter Zusatz wenigstens einer organischen Base, vorzugsweise einer organischen Base ausgewählt aus der Gruppe bestehend aus Triethylamin, Diisopropylamin, Diisopropylethylamin und [1,4]-Diazabicyclo-[2.2.2]octan und/oder Zusatz wenigstens einer anorganischen Base, vorzugsweise ausgewählt aus der Gruppe bestehend aus Kaliumcarbonat, Natriumhydrogencarbonat und Cäsiumcarbonat, wobei insbesondere die organische Base auch das Reaktionsmedium sein kann, bei Temperaturen von vorzugsweise - 70 °C bis 300 °C, besonders bevorzugt von -70°C bis 150°C, zu Verbindungen der allgemeinen Formel V umgesetzt.
Die Umsetzung von Verbindungen der allgemeinen Formel XII mit Verbindungen der allgemeinen Formel M²-X erfolgt vorzugsweise in Gegenwart wenigstens eines Ammoniumsalzes oder in Gegenwart von Kalium- und/oder Natriumfluorid.

Die Verbindungen der allgemeinen Formel V lassen sich wie in Schema 3 dargestellt zu Verbindungen der Formel X umsetzen.

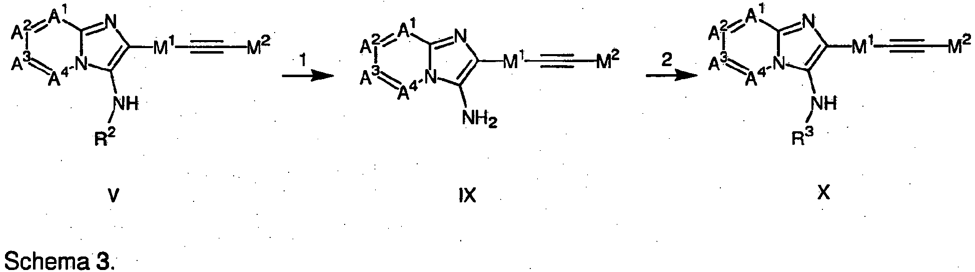

In Stufe 1 werden Verbindungen der vorstehend angegebenen allgemeinen Formel V in einem Reaktionsmedium; vorzugsweise ausgewählt aus der Gruppe bestehend aus Ethanol, Methanol und Aceton, unter Zusatz wenigstens einer organischen Säure, vorzugsweise Essigsäure oder Trifluoressigsäure und/oder unter Zusatz wenigstens einer anorganischen Säure, vorzugsweise Salzsäure oder Schwefelsäure, bei Temperaturen von vorzugsweise 0°C bis 80°C zu Verbindungen der allgemeinen Formel IX umgesetzt.

In Stufe 2 werden Verbindungen der vorstehend angegebenen allgemeinen Formel IX mit Carbonsäuren der allgemeinen Formel R²⁰-(C=O)OH, worin R²⁰ die vorstehend genannte Bedeutung hat, in einem Reaktionsmedium, vorzugsweise ausgewählt aus der Gruppe bestehend aus Diethylether, Tetrahydrofuran, Acetonitril, Methanol, Ethanol, Dimethylformamid und Dichlormethan, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dlcyclohexyicarbodiimid (DCC), N'-(3-Dimethylaminopropyl)-N-ethylcarbodiimid (EDCl), NI(Dimethylamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyl-uronium hexafluorophosphat (HBTU) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Pyridin, Dimethylaminopyridin und Diisopropylethylamin vorzugsweise bei Temperaturen von -70°C bis 100°C zu Verbindungen der allgemeinen Formel X umgesetzt.

Alternativ werden Verbindungen der allgemeinen Formel IX mit Carbonsäurederivaten bzw. Kohlensäurederivaten der allgemeinen Formel R²⁰-(C=O)-X, wobei X für einen Halogen-Rest, vorzugsweise Chlor oder Brom, steht, in einem Reaktionsmedium, vorzugsweise ausgewählt aus der Gruppe bestehend aus Diethylether, Tetrahydrofuran, Acetonitril, Methanol, Ethanol, Dimethylformamid und Dichlormethan, mit oder ohne Zusatz wenigstens einer organischen oder anorganischen Base, beispielsweise Triethylamin, Dimethylaminopyridin, Pyridin oder Diisopropylamin, ggf, in Gegenwart wenigstens einer organischen Base, vorzugsweise ausgewählt aus der Gruppe bestehend aus Triethylamin, Dimethylaminopyridin, Pyridin und Diisopropylamin, oder einer anorganischen Base bei Temperaturen von vorzugsweise -70°C bis 100°C zu Verbindungen der allgemeinen Formel X umgesetzt.

Als weitere Alternative werden Verbindungen der allgemeinen Formel IX mit Aldehyden der allgemeinen Formel R²⁰-C(=O)-H in einem Reaktionsmedium, vorzugsweise ausgewählt aus der Gruppe bestehend aus Diethylether, Tetrahydrofuran, Methanol, Ethanol, Dichlormethan und Toluol, unter Zusatz wenigstens eines Reduktionsmittels, vorzugsweise ausgewählt aus der Gruppe bestehend aus Natriumborhydrid, -Natriumacetoxyborhydrid oder Natriumcyanoborhydrid, bei Temperaturen von vorzugsweise -70°0 bis 100°C zu Verbindungen der allgemeinen Formel X umgesetzt.

Ebenfalls lassen sich Verbindungen der allgemeinen Formel IX mit Verbindungen der allgemeinen Formel R³-X, worin X für einen Halogen-Rest, bevorzugt Chlor, steht, in einem Reaktionsmedium, vorzugsweise ausgewählt aus der Gruppe bestehend aus Toluol, Tetrahydrofuran und Diethylether, unter Zusatz wenigstens eines Metallhydridsalzes, vorzugsweise unter Zusatz wenigstens eines Metallhydridsalzes ausgewählt aus der Gruppe bestehend aus Natriumhydrid, Kallumhydrid und Lithiumhydrid, bei Temperaturen von vorzugsweise 0 °C bis 40 °C zu Verbindungen der allgemeinen Formel X umsetzen.

Verbindungen der allgemeinen Formeln X und V lassen sich wie in Schema 4 angegeben weiter zu den Verbindungen der allgemeinen Formel I umsetzer, wobei die gleichen Methoden, wie unter Schema 3, Stufe 2, beschrieben, verwendet werden können.

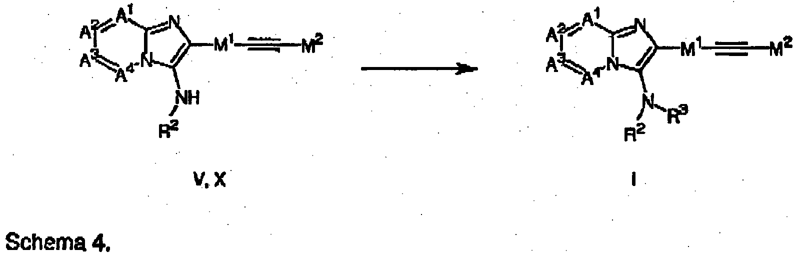

Die Verbindungen der vorstehend angegebenen Formeln II, III, IV, VI und VIII, sowie der vorstehend genannten allgemeinen Formeln R²-X, R³-X, R²⁰-C(=O)-OH, R²⁰-C(=O)-X und R²⁰-C(=O)-H sind jeweils am Markt käuflich erhältlich und/oder können nach den üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie Jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Die erfindungsgemäßen substituierten Imidazo-3-yl-amin-Verbindungen der vorstehend genannten allgemeinen Formeln I, Ia, Ib, Ic, Id, Ie, If, Ig, Ih, Ik, Im und In, im folgenden nur als Verbindungen der allgemeinen Formel I bezeichnet, sowie entsprechende Stereoisomere können sowohl in Form Ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten Imidazo-3-yl-amin-Verbindungen der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden Salze, vorzugsweise physiologisch verträglichen Salze, überführt werden.

Die freien Basen der jeweiligen substituierten Imidazo-3-yl-amin-Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten Imidazo-3-yl-amin-Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen substituierten Imidazo-3-yl-amin-Verbindungen der vorstehend genannten allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Imidazo-3-yl-amin-Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Imidazo-3-yl-amin-Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße Imidazo-3-yl-amin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Das erfindungsgemäße Arzneimittel eignet sich zur mGluR5-Rezeptor-Regulation, insbesondere zur Inhibierung des mGluR5-Rezeptors und somit auch zur Prophylaxe und/oder Behandlung von Störungen und/oder Krankheiten, die zumindest teilweise durch mGluR5-Rezeptoren vermittelt werden.

Bevorzugt eignet sich das erfindungsgemäße Arzneimittel daher zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; von Migräne; Depressionen; neurodegenerativen Erkrankungen, vorzugsweise ausgewählt aus der Gruppe bestehend aus Morbus Parkinson, Morbus Alzheimer, Morbus Huntington und Multipler Sklerose; kognitiven Erkrankungen, vorzugsweise kognitiven Mangelzuständen, besonders bevorzugt des Aufmerksamkeit-Defizit-Syndroms (ADS); Panikattacken; Angstsyndrom; Epilepsie; Husten; Harninkontinenz; Diarrhöe; Pruritus; Schizophrenie; cerebralen Ischämien; Muskelspasmen; Krämpfen; Reizdarmsyndrom; Störungen der Nahrungsaufnahme, vorzugsweise ausgewählt aus der Gruppe bestehend aus Bulimie, Kachexie, Anorexie und Fettleibigkeit; Alkohol- und/oder Drogen (insbesondere Nikotin- und/oder Kokain-) und/oder Medikamentenmißbrauch, Alkohol- und/oder Drogen (insbesondere Nikotin- und/oder Kokain-) und/oder Medikamentenabhängigkeit, vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; der Entwicklung von Toleranzerscheinungen gegenüber Drogen- und/oder Medikamenten, insbesondere gegenüber Opioiden; des Magen-Ösophagus-Reflux-Syndroms; des gastroösophagealen Rückflusses; zur Diurese; zur Antinatriurese; zur Beeinflussung des kardiovaskulären Systems; zur Anxiolyse; zur Vigilanzsteigerung; zur Libidosteigerung; zur Modulation der Bewegungsaktivität und zur Lokalanästhesie.

Besonders bevorzugt eignet sich das erfindungsgemäße Arzneimittel zur Behandlung und/oder Prophylaxe von Schmerz vorzugsweise ausgewählt aus der Gruppe bestehend aus akutem Schmerz, chronischem Schmerz, neuropathischem Schmerz und visceralem Schmerz; Depressionen; Epilepsie; neurodegenerativen Erkrankungen, vorzugsweise ausgewählt aus der Gruppe bestehend aus Morbus Parkinson, Morbus Alzheimer, Morbus Huntington und Multipler Sklerose; Alkohol- und/oder Drogen (insbesondere Nikotin- und/oder Kokain-) und/oder Medikamentenmißbrauch, Alkohol- und/oder Drogen (insbesondere Nikotin- und/oder Kokain-) und/oder Medikamentenabhängigkeit, vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; der Entwicklung von Toleranzerscheinungen gegenüber Drogen- und/oder Medikamenten, insbesondere gegenüber Opioiden; oder zur Anxiolyse.

Ganz besonders bevorzugt eignet sich das erfindungsgemäße Arzneimittel zur Behandlung und/oder Prophylaxe von Schmerz vorzugsweise von Schmerz ausgewählt aus der Gruppe bestehend aus akutem Schmerz, chronischem Schmerz, neuropathischem Schmerz und visceralem Schmerz.

Ein weiterer Gegenstand der vorliegenden Erfindung ist die Verwendung wenigstens einer erfindungsgemäßen Imidazo-3-yl-amin-Verbindung der vorstehend angegebenen allgemeinen Formel I, jeweils ggf. in Form eines ihrer reinen Stereoisomeren, insbesondere Enantiomeren oder Diastereomeren, Ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur mGluR5-Rezeptor-Regulation, vorzugsweise zur inhibierung des mGluR5-Rezeptors.

Bevorzugt ist die Verwendung wenigstens einer substituierten Imidazo-3-yl-amin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen und/oder Krankheiten, die zumindest teilweise durch mGluR5-Rezeptoren vermittelt werden.

Besonders bevorzugt ist die Verwendung wenigstens einer substituierten Imidazo-3-yl-amin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz vorzugsweise ausgewählt aus der Gruppe bestehend aus akutem Schmerz, chronischem Schmerz, neuropathischem Schmerz und visceralem Schmerz; von Migräne; Depressionen; neurodegenerativen Erkrankungen, vorzugsweise ausgewählt aus der Gruppe bestehend aus Morbus Parkinson, Morbus Alzheimer, Morbus Huntington und Multipler Sklerose; kognitiven Erkrankungen, vorzugsweise kognitiven Mangelzuständen, besonders bevorzugt des Aufmerksamkeit-Defizit-Syndroms (ADS); Panikattacken; Angstsyndrom; Epilepsie; Husten; Harninkontinenz; Diarrhöe; Pruritus; Schizophrenie; cerebralen Ischämien; Muskelspasmen; Krämpfen; Reizdarmsyndrom; Störungen der Nahrungsaufnahme, vorzugsweise ausgewählt aus der Gruppe bestehend aus Bulimie, Kachexie, Anorexie und Fettleibigkeit; Alkohol- und/oder Drogen (insbesondere Nikotin- und/oder Kokain-) und/oder Medikamentenmißbrauch, Alkohol- und/oder Drogen (insbesondere Nikotin- und/oder Kokain-) und/oder Medikamentenabhängigkeit, vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; der Entwicklung von Toleranzerscheinungen gegenüber Drogen- und/oder Medikamenten, insbesondere gegenüber Opioiden; des Magen-Ösophagus-Reflux-Syndroms; des gastroösophagealen Rückflusses; zur Diurese; zur Antinatriurese; zur Beeinflussung des kardiovaskulären Systems; zur Anxiolyse; zur Vigilanzsteigerung; zur Libidosteigerung; zur Modulation der Bewegungsaktivität und zur Lokalanästhesie.

Ganz besonders bevorzugt ist die Verwendung wenigstens einer substituierten Imidazo-3-yl-amin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz vorzugsweise ausgewählt aus der Gruppe bestehend aus akutem Schmerz, chronischem Schmerz, neuropathischem Schmerz und visceralem Schmerz.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer substituierten Imidazo-3-yl-amin-Verbindung der vorstehend angegebenen allgemeinen Formel I, ggf. in Form eines ihrer reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln. Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden erfindungsgemäßen substituierten Imidazo-3-yl-amin-Verbindungen in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweilige erfindungsgemäße substituierte Imidazo-3-yl-amin-Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen substituierten Imidazo-3-yl-amin-Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,005 bis 2000 mg/kg, vorzugsweise 0,05 bis 500 mg/kg, besonders bevorzugt 0,05 bis 100 mg/kg Körpergewicht des Patienten wenigstens einer solchen erfindungsgemäßen Verbindung pro Tag appliziert.

### Pharmakologische Methoden:

### 1. Methode zur Bestimmung der Affinität zum mGluR5-Rezeptor

Schweinehirnhomogenat wird durch Homogenisieren (Polytron PT 3000, Kinematica AG, 10.000 Umdrehungen pro Minute für 90 Sekunden) von Schweinehirnhälften ohne Medulla, Cerebellum und Pons in Puffer pH 8.0 (30mM Hepes, Sigma, Bestellnr. H3375 + 1 Tablette Complete auf 100ml, Roche Diagnostics, Bestellnr. 1836145) im Verhältnis 1:20 (Hirngewicht/Volumen) und Differentialzentrifugation bei 900 x g und 40.000 x g hergestellt. In 250 µl Inkubationsansätzen in 96 Well Mikrotiterplatten werden jeweils 450 µg Protein aus Hirnhomogenat mit 5nM³[H]-MPEP (Tocris, Bestellnr. R1212) (MPEP = 2-Methyl-6-(3-methoxyphenyl)-ethinylpyridin) und die zu untersuchenden Verbindungen (10 µM im Test) in Puffer (wie oben) bei Raumtemperatur für 60 min inkubiert.

Danach werden die Ansätze mit Hilfe eines Brandel Cell Harvesters (Brandel, TYP Robotic 9600) auf Unifilterplates mit Glasfaserfiltermatten (Perkin Elmer, Bestellnr. 6005177) filtriert und anschließend mit Puffer (wie oben) 3 mal mit je 250 µl pro Probe gewaschen. Die Filterplatten werden anschließend für 60 min bei 55 °C getrocknet. Anschließend werden pro Well 30 µL Ultima Gold^{™} Szintillator (Packard BioScience, Bestellnr.6013159) zugegeben und nach 3 Stunden werden die Proben am ß-Counter (Mikrobeta, Perkin Elmer) gemessen. Die unspezifische Bindung wird durch Zugabe von 10 µM MPEP (Tocris, Bestellnr.1212) bestimmt.

### 2. Neuropathischer Schmerz an der Ratte

Die Untersuchung auf Wirksamkeit im neuropathischen Schmerz wurde im Bennett-Modell (chronic constriction injury; Bennett und Xie, 1988, Pain 33:87-107) durchgeführt. Die entsprechenden Teile der Literatur gelten hiermit als Teil der vorliegenden Offenbarung.

Sprague-Dawley Ratten mit einem Gewicht von 140-160 g werden unter Nembutal-Narkose mit vier losen Ligaturen des rechten nervus ischiaticus versehen. Die Tiere entwickeln an der vom geschädigten Nerv innervierten Pfote eine Überempfindlichkeit, die nach einer Erholungsphase von einer Woche über etwa vier Wochen mittels einer 4°C kalten Metallplatte quantifiziert wird (Kälte-Allodynie). Die Tiere werden für einen Zeitraum von 2 min. auf dieser Platte beobachtet und die Anzahl der Wegziehreaktionen der geschädigten Pfote wird gemessen. Bezogen auf den Vorwert vor Substanzapplikation wird die Substanzwirkung über einen Zeitraum von einer Stunde an vier Zeitpunkten (15, 30, 45, 60 min. nach Applikation) bestimmt und die resultierende Fläche unter der Kurve (AUC) sowie die Hemmung der Kälte-Allodynie zu den einzelnen Meßpunkten in Prozent Wirkung zur Vehikelkontrolle (AUC) bzw. zum Ausgangswert (Einzelmeßpunkte) ausgedrückt. Die Gruppengröße beträgt n = 10. Die Signifikanz einer anti-allodynischen Wirkung wird anhand der AUC-Werte über einen gepaarten T-Test (* 0.05 ≥ p >0.01; ** 0.01 ≥ p > 0.001; *** p ≤ 0.001; Armitage und Berry, 1987, Stat. Methods in Medical Research, London: Blackwell Scientific Publications) bestimmt.

### 3. "Elevated Plus Maze" Modell

Im "elevated plus maze" (EPM) Modell werden Verbindungen auf mögliche anxiolytische Effekte getestet. Die Tests werden bei männlichen Sprague-Dawley Ratten (200-250 g) durchgeführt und 2 "elevated plus mazes" (Med Associates) mit elektronisch gesteuerten Infrarot-Lichtschranken zur Bestimmung des Aufenthaltsortes der Tiere im Labyrinth werde verwendet. Jedes Labyrinth besitzt 2 offene und 2 geschlossene Arme und eine zentrale Plattform. Die Ränder der offenen Arme werden von schmalen Leisten begrenzt. Das gesamte Labyrinth ist auf einem Metallständer montiert.

Zu Beginn eines 5-min Tests wird jedes Tier einzeln mit dem Kopf in Richtung eines geschlossenen Armes auf die zentrale Plattform gesetzt.

Die folgenden Parameter werden bestimmt bzw. berechnet und ausgewertet: Anzahl und Prozent Eintritte in die offenen und geschlossenen Arme, sowie Prozent Zeit in den offenen und geschlossenen Armen und auf der zentralen Plattform.

Die Daten werden mittels einer 1-faktoriellen ANOVA (Vergleich Behandlungsgruppen versus Vehikel-Gruppe) analysiert. Das Signifikanz-Niveau wird bei p < 0.05 festgelegt. Alle Gruppen haben eine Größe von N = 10.

Der Test ist ebenfalls in Hogg, S. (1996) A review of the validity and variability of the elevated plus-maze as an animal model of anxiety. Pharmacol. Biochem. Behav. 54, 21-30 und Rodgers, R.J., Cole, J.C. (1994) The elevated plus-maze: pharmacology, methodology and ethology. In: Cooper, S.J., Hendrie, C.A. (eds.) Ethology and Psychopharmacology. Wiley & Sons; pp. 9-44 beschrieben. Die entsprechenden Stellen der Literatur gelten hiermit als Teil der Offenbarung.

### 4. Formalin-Test

### a. Formalin-Test an der Ratte

Der Formalin Test wie in Dubuisson, D. and Dennis, S.G., 1977, Pain, 4, 161 - 174 beschrieben stellt ein Modell für den akuten sowie den chronischen Schmerz dar. Die entsprechenden Stellen der Literatur gelten hiermit als Teil der Offenbarung. Durch eine einmalige Formalin-Injektion in die dorsale Seite einer Hinterpfote wird bei frei beweglichen Versuchstieren eine biphasische nozizeptive Reaktion induziert, die durch Beobachtung von drei deutlich voneinander unterscheidbaren Verhaltensmustern erfaßt wird. Die Reaktion ist zweiphasisch: Phase 1 = Sofortreaktion (Dauer bis 10 min; Pfotenschütteln, Lecken), Phase 2 = Spätreaktion (nach einer Ruhephase; ebenfalls Pfotenschütteln, Lecken; Dauer bis 60 min). Die 1. Phase reflektiert eine direkte Stimulation der peripheren Nozisensoren mit hohem spinalen nozizeptiven Input bzw. Glutamatfreisetzung (akute Schmerzphase); die 2. Phase reflektiert eine spinale und periphere Hypersensibilisierung (chronische Schmerzphase). In den hier vorgestellten Untersuchungen wurde die chronische Schmerzkomponente (Phase 2) ausgewertet.

Formalin wird mit dem Volumen von 50 µl und einer Konzentration von 5 % subkutan in die dorsale Seite der rechten Hinterpfote jedes Tieres appliziert. Die zu testenden Substanzen werden 15 min vor der Formalin-Injektion intraperitoneal (i.p.), oder 5 min vor der Formalin-Injektion intravenös (i.v.) appliziert. Die spezifischen Verhaltensänderungen, wie Anheben und Schütteln der Pfote, Gewichtsverlagerungen des Tieres sowie Beiß- und Leckreaktionen werden im Beobachtungszeitraum von 21 bis 27 min nach Formalin-Injektion beobachtet und registriert. Die Zusammenfassung der verschiedenen Verhaltensweisen erfolgt in der sogenannten Pain-Rate (PR), die auf die Teilintervalle von 3 min bezogen die Berechnung einer mittleren Nozizeptionsreaktion darstellt. Die Berechnung der PR erfolgt aufgrund einer numerischen Gewichtung (= jeweils Faktor 1, 2, 3) der beobachteten Verhaltensweisen (entsprechend Verhaltensscore 1, 2, 3) und wird mit folgender Formel berechnet: PR = [(T₀ x 0) + (T₁ x 1) + (T₂ x 2) + (T₃ x 3)] / 180 wobei T₀, T₁, T₂, und T₃ jeweils der Zeit in Sekunden entspricht, in der das Tier die Verhaltensweisen 0, 1, 2 oder 3 zeigt. Die Gruppengröße beträgt 10 Tiere (n=10).

### b. Formalin-Test an der Maus

Formalin wird mit dem Volumen von 20 µl und einer Konzentration von 1 % subkutan in die dorsale Seite der rechten Hinterpfote jedes Tieres appliziert. Die zu testenden Substanzen werden 15 min vor der Formalin-Injektion intraperitoneal (i.p.) appliziert. Die spezifischen Verhaltensänderungen, wie Anheben und Schütteln der Pfote (score 3, Dubuisson & Dennis, 1977), werden im Beobachtungszeitraum von 21 bis 24 min nach Formalin-Injektion beobachtet und registriert. Die Gruppengröße beträgt 10 Tiere (n=10).

Im folgenden wird die Erfindung anhand von Beispielen erläutert. Diese Erläuterungen sind lediglich beispielhaft und schränken den allgemeinen Erfindungsgedanken nicht ein.

### Beispiele:

Die Ausbeuten der hergestellten Verbindungen sind nicht optimiert.

Alle Temperaturen sind unkorrigiert.

### Abkürzungen:

- Äqulvalent: Stoffmengenäqulvalent
- aq.: wäßrig
- d: Tage
- DCC: Dicyclohexylcarbodiimid
- DCM: Dichlormethan
- DMAP: 4-Dimethylaminopyridin
- DMF: N,N-Dimethylformamid
- DIPE: Diisopropylether
- EE: Essigsäureethylester
- EDCI: N'-(3-Dimethylaminopropyl)-N-ethylcarbodiimid
- Ether: Diethylether
- EtOH: Ethanol
- ges.: gesättigt
- H₂O: Wasser
- HATU: N-[(Dimethyamino)-1H-1,2,3-triazolo[4,5-b]pyridino-1-ylmethylen]-N-methylmethanaminium hexafluorophosphat N-oxid
- HBTU: O-(Benzotriazol-1-yl)-N,N,N',N'-tetramethyluronium hexafluorophosphat
- MeCN: Acetonitril
- MeOH: Methanol
- NEt₃: Triethylamin
- RT: Raumtemperatur
- SC: Säulenchromatographie
- THF: Tetrahydrofuran

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch Massenspektroskopie und NMR.

### Allgemeine Vorschriften für die Herstellung beispielgemäßer substituierter bizyklischer Imidazo-3-yl-amine

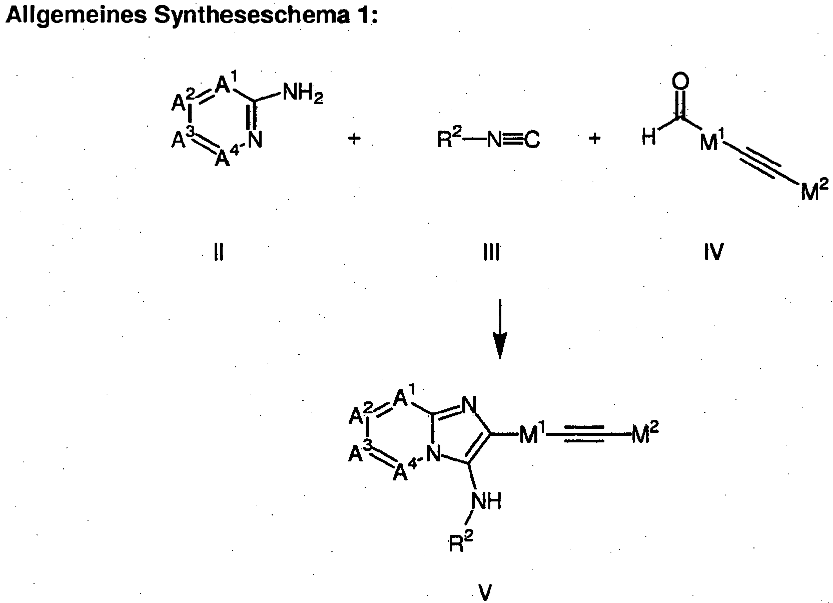

Die Umsetzung von Aminen (1 Äquivalent) der allgemeinen Formel II(1 Äquivalent) mit Isocyaniden der allgemeinen Formel III und Aldehyden (1 Äquivalent) der allgemeinen Formel IV zu Verbindungen der allgemeinen Formel V wurde in organischen Lösungsmitteln oder Lösungsmittelgemischen, beispielsweise von Chloroform, DCM, MeCN, MeOH oder EtOH, unter Zusatz einer organischen oder anorganischen Säure, beispielsweise Trifluoressigsäure oder Perchlorsäure, oder unter Zusatz eines Übergangsmetalltriflats, beispielsweise Scandium(III)triflat, Ytterbiumtriflat oder Indium(III)triflat, bei Temperaturen von 0°C bis 150°C durchgeführt.

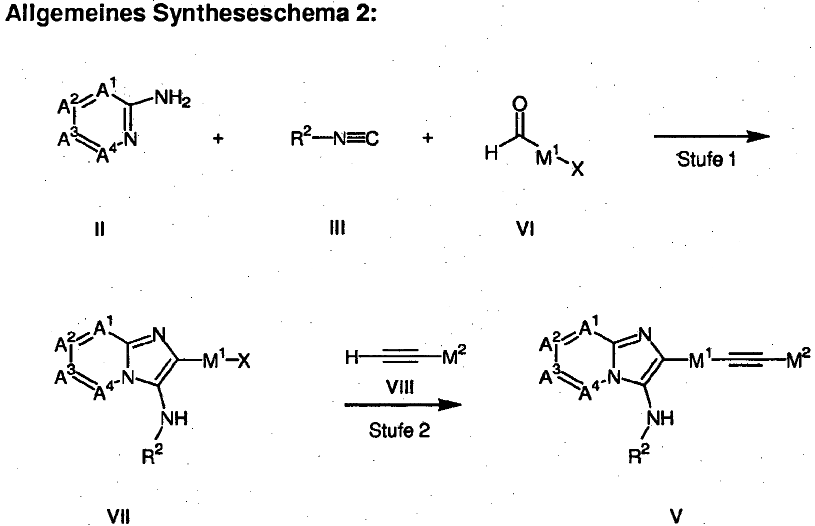

In Stufe 1 wurde die Umsetzung von Aminen (1 Äquivalent) der allgemeinen formel II mit Isocyaniden (1 Äquivalent) der allgemeinen Formel III und Aldehyden (1 Äquivalent) der allgemeinen Formel VI, worin X für einen Halogen-Rest steht, zu Verbindungen der allgemeinen Formel VII in organischen Lösungsmitteln oder Lösungsmittelgemischen, beispielsweise von Chloroform, DCM, MeCN, MeOH oder EtOH, unter Zusatz einer organischen oder anorganischen Säure, beispielsweise Trifluoressigsäure oder Perchlorsäure, oder unter Zusatz eines Übergangsmetalltriflats; beispielsweise Scandium(III)triflat, Ytterbiumtriflat oder Indium(III)triflat, bei Temperaturen von 0°C bis 150°C durchgeführt.

In Stufe 2 erfolgte die Umsetzung von Verbindungen der allgemeinen Formel VII (1 Äquivalent), worin X für einen Halogen-Rest steht, mit Acetylenen (2.5 Äquivalenten) der allgemeinen Formel VIII zu Verbindungen der allgemeinen Formel V in einem Lösungsmittel oder Lösungsmittelgemisch, beispielsweise von Toluol, THF, DMF, MeCN, Ether, NEt₃ oder Diisopropylamin, unter Zusatz eines Palladiumkatalysators, beispielsweise Bis(triphenylphosphin)-palladium(II)-chlorid, von Kupfer(I)-iodid und einer organischen Base, beispielsweise NEt₃ oder Diisopropylamin, und/oder anorganischen Base, beispielsweise Kaliumcarbonat oder Cäsiumcarbonat, bei Temperaturen von -70°C bis 150°C.

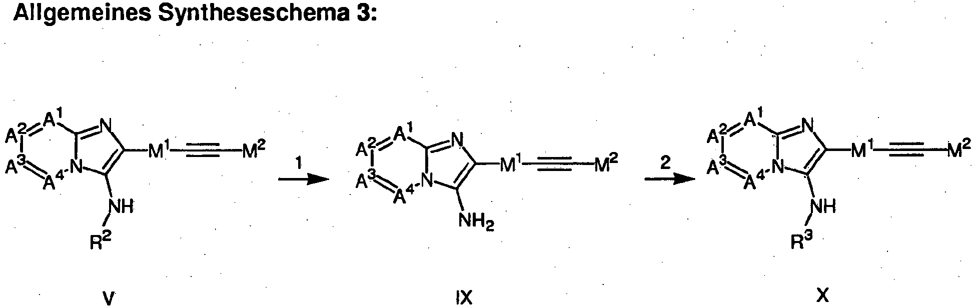

In Stufe 1 wurden Verbindungen der allgemeinen Formel V zu Aminen der allgemeinen Formel IX in einem Lösungsmittel oder Lösungsmittelgemisch, beispielsweise von EtOH, MeOH oder Aceton, unter Zusatz einer organischen oder anorganischen Säure, beispielsweise Essigsäure, Trifluoressigsäure, Salzsäure oder Schwefelsäure, bei Temperaturen von 0°C bis 80°C umgesetzt.

In Stufe 2 wurden Verbindungen der allgemeinen Formel IX (1 Äquivalent) mit Carbonsäuren (1 Äquivalent) der allgemeinen Formel R²⁰-(C=O)-OH in einem Lösungsmittel oder Lösungsmittelgemisch, beispielsweise von Ether, THF, MeCN, MeOH, EtOH, DMF oder DCM, mit oder ohne Zusatz eines Kupplungsreagenz (1 Äquivalent), beispielsweise DCC, BOP, HATU oder EDCl und ggf. in Gegenwart wenigstens einer anorganischen oder organischen Base, beispielsweise NEt₃ oder Diisopropylethylamin, bei Temperaturen von -70°C bis 100°C zu Verbindungen der allgemeinen Formel X umgesetzt.

Alternativ wurden Verbindungen der allgemeinen Formel IX (1 Äquivalent) mit Carbonsäurehalogeniden (1 Äquivalent) bzw. Kohlensäurederivaten der allgemeinen Formel R²⁰-(C=O)-X, wobei X für einen Halogen-Rest steht, in einem Lösungsmitteln oder Lösungsmittelgemisch, beispielsweise von Ether, THF, MeCN, MeOH, EtOH, DMF oder DCM, mit oder ohne Zusatz einer organischen oder anorganischen Base, beispielsweise NEt₃, DMAP, Pyridin oder Diisopropylamin, bei Temperaturen von-70°C bis 100°C zu Verbindungen der allgemeinen Formel X umgesetzt.

Als weitere Alternative wurden Verbindungen der allgemeinen Formel IX (1 Äquivalent) mit Aldehyden (1 Äquivalent) der allgemeinen Formel R²⁰-C(=O)-H in einem Lösungsmittel oder Lösungsmittelgemisch, beispielsweise von Ether, THF, MeOH, EtOH, DCM oder Toluol, und nachfolgender Zugabe eines Reduktionsmittels, beispielsweise Natriumborhydrid, Natriumacetoxyborhydrid oder Natriumcyanoborhydrid, bei Temperaturen von -70°C bis 100°C zu Verbindungen der allgemeinen Formel X umgesetzt.

Ebenfalls wurden Verbindungen der allgemeinen Formel IX (1 Äquivalent) mit Verbindungen der allgemeinen Formel R³-X (1.1 Äquivalente), worin X für einen Halogen-Rest, bevorzugt Chlor, steht, in einem Lösungsmittel oder Lösungsmittelgemisch, beispielsweise von Toluol, THF, oder Ether, unter Zusatz eines Metallhydridsalzes (1.1 Äquivalente), vorzugsweise unter Zusatz von Natriumhydrid, zu Verbindungen der allgemeinen Formel X bei Temperaturen von 0 °C bis 40 °C umgesetzt.

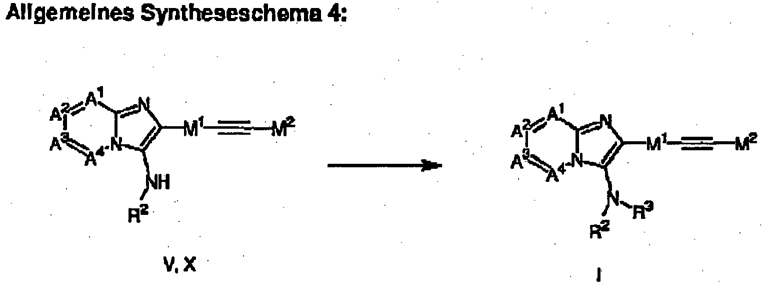

Die Verbindungen der allgemeinen Formel V oder X lassen sich mit den gleichen Methoden, wie im Allgemeinen Syntheseschema 3, Stufe 2, beschrieben, zu Verbindungen der allgemeinen Formel I umsetzen.

Im folgenden werden die vorstehend beschriebenen Vorschriften für die Herstellung substituierter bizyklischer Imidazo-3-yl-amine anhand einiger Beispielverbindungen detailliert erläutert:

### Beispiel 1: Synthese von Cyclopentyl-[2-(5-phenylethinyl-thiophen-2-yl)-imlidazo[1,2-a]pyrazin-3-yl]-amin

Zu einer Lösung von 9.5 mg (0.1 mmol) 2-Aminopyrazin in MeOH (1 ml) wurden nacheinander 10 µl (0.022 mmol) 20%-ige aq. Perchlorsäure, eine Lösung von 31:8 mg (0.15 mmol) 5-Phenylethinyl-thiophen-2-carbaldehyd in einem Gemisch aus MeOH und DCM (0.5 ml, 1:1 v/v) und eine Lösung von 11.1 mg (0.115 mmol) Cyclopentyl-isocyanid in MeOH (0.5 ml) gegeben. Die Reaktionslösung wurde 36 h bei RT gerührt und anschließend mit ges. aq. NaCl-Lösung (3 ml) versetzt und weitere 45 min bei RT gerührt. Die Phasen wurden getrennt und die aq. Phase wurde mit DCM (2 x 3 ml) extrahiert. Die gesammelten organischen Phasen wurden über MgSO₄ getrocknet, filtriert und im Vakuum eingeengt. Der Rückstand wurde mittels präparativer HPLC gereinigt, wobei 18 mg (0.047 mmol, 47%) Cyclopentyl-[2-(5-phenylethinyl-thiophen-2-yl)-imidazo[1,2-a]pyrazin-3-yl]-amin erhalten wurden.

### Beispiel 18:

### tert-Butyl-[2-(5-phenylethinyl-thiophen-2-yl)-imidazo[1,2-a]pyrazin-3-yl]-amin

### a) Synthese von [2-(5-Bromthiophen-2-yl)-imidazo[1,2-a]pyrazin-3-yl]-tert-butylamin

237 mg (2.50 mmol) 2-Aminopyrazin wurden zusammen mit 208 mg (2.50 mmot) *tert-*Butylisonitril und 0.27 ml (2.50 mmol) 5-Bromthiophen-2-carbaldehyd in DCM (5 ml) gelöst. Nach Zugabe von Perchlorsäure (0.25 ml) wurde 5 d bei RT gerührt. Anschließend wurde mit einer ges. aq. Natriumcarbonatlösung und einer ges. aq. Kochsalzlösung gewaschen. Die organische Phase wurde über Magnesiumsulfat getrocknet, filtriert und im Vakuum eingeengt. Nach Durchführung einer Säulenchromatographie (EE/DIPE/DCM 2:2:1) mit dem Rückstand wurden 471 mg (1.3 mmol, 54% der Theorie) [2-(5-Bromthiophen-2-yl)-imidazo[1,2-a]pyrazin-3-yl]-tert-butyl-amin erhalten.

### b) Synthese von tert-Butyl-[2-(5-phenylethinyl-thiophen-2-yl)-imidazo[1,2-a]pyrazin-3-yl]-amin

420 mg (1.2 mmol) [2-(5-Bromthiophen-2-yl)-imidazo[1,2-a]pyrazin-3-yl]-tert-butylamin wurden zusammen mit 0.33 ml (3.0 mmol) Phenylacetylen, 42 mg (0.06 mmol) Bis(triphenylphosphin)-palladium(II)-chlorid und 22 mg (0.12 mmol) Kupfer(I)-iodid in DMF (9 ml) gelöst. Nach Zugabe von 1.6 ml (12.0 mmol) Triethylamin wurde 16 h bei 50°C gerührt. Anschließend wurde die Reaktionslösung mit EE verdünnt und mit einer ges. aq. Natriumcarbonatlösung versetzt. Die Phasen wurden getrennt und die wäßrige Phase mit EE extrahiert. Die vereinigten organischen Phasen wurden 2x mit einer ges. aq. Natriumcarbonatlösung und 2x mit einer ges. aq. Kochsalzlösung gewaschen und über Magnesiumsulfat getrocknet. Nach Filtrieren und Entfernen der Lösungsmittel im Vakuum wurde mit dem Rückstand eine Säulenchromatographie (EE/DCM 1:4) durchgeführt, nach der 326 mg (0.88 mmol, 73% der Theorie) tert-Butyl-[2-(5-phenylethinyl-thiophen-2-yl)-imidazo[1,2-a]pyrazin-3-yl]-amin erhalten wurden.

### Beispiel 31:(1-Phenyl-ethyl)-[2-(5-phenylethinyl-thiophen-2-yl)-imidazo[1,2-alpyrazin-3-yl]-amin

### a) Synthese von [2-(5-Bromthiophen-2-yl)-imidazo[1,2-a]pyrazin-3-yl]-(1-phenyl-ethyl)-amin

951 mg (10.0 mmol) 2-Aminopyrazin wurden zusammen mit 1.31 g (10.0 mmol) 1-Phenyl-ethylisonitril und 1.08 ml (10.0 mmol) 5-Bromthiophen-2-carbaldehyd in Chloroform (24 ml) gelöst. Nach Zugabe von Perchlorsäure (1.00 ml) wurde 5 d bei RT gerührt. Anschließend wurde mit einer ges. aq. Natriumcarbonatlösung versetzt und die Phasen getrennt. Die wäßrige Phase wurde mit DCM extrahiert. Die vereinigten organischen Phasen wurden mit einer ges. aq. Natriumcarbonatlösung gewaschen und über Magnesiumsulfat getrocknet, filtriert und im Vakuum eingeengt. Nach Durchführung einer Säulenchromatographie (EE/DCM 1:1) mit dem Rückstand wurden 831 mg (2.1 mmol, 21% der Theorie) [2-(5-Bromthiophen-2-yl)-imidazo[1,2-a]pyrazin-3-yl]-(1-phenyl-ethyl)-amin erhalten.

### b) Synthese von (1-Phenyl-ethyl)-[2-(5-phenylethinyl-thiophen-2-yl)-imidazo[1,2-a]pyrazin-3-yl]-amin

497 mg (1.25 mmol) [2-(5-Bromthiophen-2-yl)-imidazo[1,2-a]pyrazin-3-yl]-(1-phenyl-ethyl)-amin wurden zusammen mit 0.34 ml (3.13 mmol) Phenylacetylen, 43 mg (0.06 mmol) Bis(triphenylphosphin)-palladium(II)-chlorid und 24 mg (0.13 mmol) Kupfer(I)-iodid in DMF (10 ml) gelöst. Nach Zugabe von 1.7 ml (12.5 mmol) Triethylamin wurde 16 h bei 50°C gerührt. Anschließend wurde die Reaktionslösung mit EE verdünnt und mit einer ges. aq. Natriumcarbonatlösung versetzt. Die Phasen wurden getrennt und die wäßrige Phase mit EE extrahiert. Die vereinigten organischen Phasen wurden 2x mit einer ges. aq. Natriumcarbonatlösung und 2x mit einer ges. aq. Kochsalzlösung gewaschen und Ober Magnesiumsulfat getrocknet. Nach Filtrieren und Entfernen der Lösungsmittel im Vakuum wurde mit dem Rückstand eine Säulenchromatographie (EE/DCM 1:3) durchgeführt, nach der 158 mg (0.38 mmol. 30% der Theorie (1-Phenyl-ethyl)-[2-(5-phenylethinyl-thiophen-2-yl-imidazo[1,2-a]pyrazin-3-yl]-amin erhalten wurden.

### Beispiel 56:

### [2-(5-Phenylethinyl-thiophen-2-yl)-imidazo[1,2-a]pyrazin-3-yl]-(1,1,3,3-tetramethyl-butyl)-amin

### a) Synthese von [2-(5-Bromthiophen-2-yl)-imidazo[1,2-a]Pyrazin-3-yl]-(1,1,3,3-tetramethyl-butyl)-amin

4.76 g (50.0 mmol) 2-Aminopyrazin wurden zusammen mit 8.76 ml (50.0 mmol) 1,1,3,3-Tetramethyl-butylisonitril und 5.42 ml (50.0 mmol) 5-Bromthiophen-2-carbaldehyd in Chloroform (40 ml) gelöst. Nach Zugabe von Perchlorsäure (5 ml) wurde 16 h bei 50°C gerührt. Nach Abkühlen auf RT wurde 2x mit einer ges. aq. Natriumcarbonatlösung gewaschen. Die organische Phase wurde über Magnesiumsulfat getrocknet, filtriert und im Vakuum eingeengt. Nach Durchführung einer Säulenchromatographie (EE/DCM 1:3) mit dem Rückstand wurden 11.39 g (28.0 mmol, 56% der Theorie) des [2-(5-Bromthiophen-2-yl)-imidazo[1,2-a]pyrazin-3-yl]-(1,1,3,3-tetramethyl-butyl)-amin erhalten.

### b) Synthese von [2-(5-Phenylethinyl-thiophen-2-yl)-imidazo[1,2-a]pyrazin-3-yl]-(1,1,3,3-tetramethyl-butyl)-amin

3.65 g (9.0 mmol) [2-(5-Bromthiophen-2-yl)-imidazo[1,2-a]pyrarin-3-yl]-(1,1,3,3-tetra-methyl-butyl)-amin wurden zusammen mit 1.18 ml (10.8 mmol) Phenylacetylen, 0.16 g (0.23 mmol) Bis(triphenylphosphin)-palladium(II)-chlorid und 0.08 g (0.45 mmol) Kupfer(I)-iodid in DMF (30 ml) gelöst. Nach Zugabe von 2.50 ml (18.00 mmol) Triethylamin wurde 16 h bei 50°C gerührt. Anschließend wurde die Reaktionslösung mit EE verdünnt und mit einer ges. aq. Natriumcarbonatlösung versetzt. Die Phasen wurden getrennt und die wäßrige Phase mit EE extrahiert. Die vereinigten organischen Phasen wurden 2x mit einer ges. aq. Natriumcarbonatlösung gewaschen und über Magnesiumsulfat getrocknet. Nach Filtrieren und Entfernen der Lösungsmittel im Vakuum wurde mit dem Rückstand eine Säulenchromatographie (EE/DIPE/DCM 3:3:4) durchgeführt, nach der 3.38 g (7.9 mmol, 88% der Theorie) [2-(5-Phenylethinyl-thiophen-2-yl)-imidazo[1,2-a]pyrazin-3-yl]-(1,1,3,3-tetramethyl-butyl)-amin erhalten wurden.

### Beispie 59:

### [2-(5-Phenylethinyl-thiophen-2-yl)-imidazo[1,2-a]pyridin-3-yl]-(1,1,3,3-tetramethyl-butyl)-amin

### a) Synthese von [2-(5-Bromthiophen-2-yl)-imidazo[1,2-a]pyridin-3-yl]-(1,1,3,3-tetramethyl-butyl)-amin

235 mg (2.5 mmol) 2-Aminopyridin wurden zusammen mit 0.42 ml (2.5 mmol) 1,1,3,3-Tetramethyl-butylisonitril und 0.27 ml (2.5 mmol) 5-Bromthiophen-2-carbaldehyd in DCM (5 ml) gelöst. Nach Zugabe von Perchlorsäure (0.25 ml) wurde 5 d bei RT gerührt. Anschließend wurde mit einer ges. aq. Natriumcarbonatlösung und einer ges. aq. Kochsalzlösung gewaschen. Die organische Phase wurde über Magnesiumsulfat getrocknet, filtriert und im Vakuum eingeengt. Nach Durchführung einer Säulenchromatographie (EE/DIPE/DCM 2:2:1) mit dem Rückstand wurden 724 mg (1.7 mmol, 71 % der Theorie) [2-(5-Bromthiophen-2-yl)-imidazo[1,2-a]pyridin-3-yl]-(1,1,3,3-tetramethyl-butyl)-amin erhalten.

### b) Synthese von [2-(5-Phenylethinyl-thiophen-2-yl)-imidazo[1,2-a]pyridin-3-yl]-(1,1,3,3-tetramethyl-butyl)-amin

324 mg (0.80 mmol) [2-(5-Bromthiophen-2-yl)-imidazo[1,2-a]pyridin-3-yl]-(1,1,3,3-tetramethyl-butyl)-amin wurden zusammen mit 0.11 ml (0.96 mmol) Phenylacetylen, 14 mg (0.02 mmol) Bis(triphenylphosphin)-palladium(II)-chlorid und 7 mg (0.04 mmol) Kupfer(I)-iodid in DMF (6 ml) gelöst. Nach Zugabe von 1.1 ml (8.0 mmol) Triethylamin wurde 2 d bei 50 °C gerührt. Anschließend wurde die Reaktionslösung mit EE verdünnt und mit einer ges. aq. Natriumcarbonatlösung versetzt. Die Phasen wurden getrennt und die wäßrige Phase mit EE extrahiert. Die vereinigten organischen Phasen wurden 2x mit einer ges. aq. Natriumcarbonatlösung und 2x mit einer ges. aq. Kochsalzlösung gewaschen und über Magnesiumsulfat getrocknet. Nach Filtrieren und Entfernen der Lösungsmittel im Vakuum wurde mit dem Rückstand eine Säulenchromatographie (EE/DIPE/DCM 3:3:4) durchgeführt, nach der 54 mg (0.13 mmol, 16% der Theorie) [2-(5-Phenylethinyl-thiophen-2-yl)-imidazo[1,2-a]pyridin-3-yl]-(1,1,3,3-tetramethyl-butyl)-amin erhalten wurden.

### Beispiel 60:

### [2-(5-Pyridin-3-ylethinyl-thiophen-2-yl)amtdazo[1,2-a]Pyrazin-3-yl]-(1,1,3,3-tetramethyl-butyl)-amin

### Synthese von [2-(5-Pyridin-3-ylethinyl-thiophen-2-yl)-imidazo[1,2-a]pyrazin-3-yl]-(1,1,3,3-tetramethyl-butyl)-amin

600 mg (1.48 mmol) [2-(5-Bromthiophen-2-yl)-imidazo[1,2-a]pyrazin-3-yl]-(1,1,3,3-tetra-methyl-butyl)-amin wurden zusämmen mit 183 mg (1.77 mmol) 3-Ethinylpyridin, 104 mg (0.15 mmol) Bis(triphenylphosphin)-palladium(II)-chlorid und 28 mg (0.15 mmol) Kupfer(I)-iodid in DMF (10 ml) gelöst. Nach Zugabe von 2.00 ml (14.78 mmol) Triethylamin wurde 3 d bei 50°C gerührt. Anschließend wurde die Reatkionslösung mit EE verdünnt und mit einer ges. aq. Natriumcarbonatlösung versetzt. Die Phasen wurden getrennt und die wäßrige Phase mit EE extrahiert Die vereinigten organischen Phasen wurden 2x mit einer ges. aq. Natriumcarbonatlösung gewaschen und über Natriumsultat getrocknet. Nach Filtrieren und Entfernen der Lösungsmittel im Vakuum wurde mit dem Rückstand eine Säulenchromatographie (EE) durchgeführt. Nach Auskristallisieren des erhaltenen Rohprodukts wurden 310 mg (0.72 mmol, 49% der Theorie) [2-(5-Pyridin-3-ylethinyl-thiophen-2-yl)-imidazo[1,2-a]pyrazin-3-yl]-(1,1,3,3-tetramethyl-butyl)-amin erhalten.

### Beispiel 83:

### 2-(5-Phenylethinyl-thiophen-2-yl)-imidazo[1,2-a]pyrazin-3-ylamin Hydrochlorid

2.32 g (5.44 mmol) [2-(5-Phenylethinyl-thiophen-2-yl)-imidazo[1,2-a]pyrazin-3-yl]-(1,1,3,3-tetramethylbutyl)-amin (Beispiel 60) wurden Methanol (90 ml) gelöst und mit 5,3 ml (54.4 mmol) einer 32%-igen HCl-Lösung versetzt. Die Reaktionlösung wurde 16 h bei RT gerührt. Der entstandene Niederschlag wurde abfiltriert und es wurde mit Methanol nachgewaschen. Dabei wurden 1,39 g (3,95 mmol, 73% der Theorie) 2-(5-Phenylethinyl-thiophen-2-yl)-imidazo[1,2-a]pyrazin-3-ylamin Hydrochlorid als Feststoff erhalten.

### Beispiel 65:

### Dimethyl-[2(5-phenylethinyl-thiophen-2-yl)-imidazo[1,2-a]pyrazin-3-yl]-amin

790 mg (2.5 mmol) 2-(5-Phenylethinyl-thiophen-2-yl)-imidazo[1,2-a]pyrazin-3-ylamin Hydrochlorid (Beispiel 83) wurden in DMF (8 ml) gelöst. Nach portionsweiser Zugabe von 300 mg (7.5 mmol) Natriumhydrid (60%-ige Dispersion in Mineralöl) wurde 30 min bei RT gerührt. Anschließend wurden 0.3 ml (5.0 mmol) Methyliodid gelöst in DMF (2 ml) zugetropft. Die Reaktionlösung wurde weitere 4 h bei RT gerührt und dann mit Wasser (20 ml) gequencht und mit EE (2 x 50 ml) extrahiert. Die vereinigten organischen Phasen wurde über MgSO₄ getrocknet, filtriert und im Vakuum eingeengt. Aus dem Rückstand wurden 351 mg (1.0 mmol, 40% der Theorie) Dimethyl-[2-(5-phenylethinyl-thiophen-2-yl)-imidazo[1,2-a]pyrazin-3-yl]-amin durch Kristallisation (EE) erhalten.

### Beispiel 68:

### Ethyl-[2-(5-phenylethinyl-thiophen-2-yl)-imidazo[1,2a]pyrazin-3-yl]-amin

395 mg (1.25 mmol) 2-(5-Phenylethinyl-thiophen-2-yl)-imidazo[1,2-a]pyrazin-3-ylamin Hydrochlorid (Beispiel 83) wurden in DMF (8 ml) gelöst. Nach Zugabe von 100 mg (2.5 mmol) Natriumhydrid (60%-ige Dispersion in Mineralöl) wurde 1 h bei RT gerührt. Anschließend wurde eine Lösung von 74 µl (0.8 mmol) Ethylbromid in DMF (2 ml) zugetropft. Die Reaktionlösung wurde 16 h bei RT gerührt, mit Wasser (10 ml) gequencht und mit EE (2 x 50 ml) extrahiert. Die vereinigten organischen Phasen wurde über MgSO₄ getrocknet, filtriert und im Vakuum eingeengt. Mittels Säulenchromatographie (Kieselgel, EE/DCM 4:1) wurden 253 mg (0,74 mmol, 58% der Theorie) Ethyl-[2-(5-phenylethinyl-thiophen-2-yl)-imidazo[1,2-a]pyrazin-3-yl]-amin erhalten.

### Beispiel 94: Synthese von N-tert-Butyl-2-(5-(pyridin-2-ylethinyl)thiophen-2-yl)imidazo[1,2-a]pyridin-3-amin

1.32 g (14.1 mmol) 2-Aminopyrazin wurden zusammen mit 3.00 g (14.1 mmol) 5-Pyridin-2-ylethinyl-thiophen-2-carbaldehyd, 1.59 ml (14.1 mmol) tert.-Butyl-isonitril und 2.07 g (4.2 mmol) Scandium(III)-trifluormethansulfonat in Chloroform (40 ml) gelöst. Die Reaktionslösung wurde 60 min bei 80 °C in der Mikrowelle (800 Watt, MLS-Ethos1600) erhitzt und anschließend mit DCM verdünnt. Danach wurde mit einer 1 M Na₂CO₃-Lsg und einer ges. aq. NaCl-Lsg gewaschen. Anschließend wurde über Na₂SO₄ getrocknet, filtriert und im Vakuum eingeengt. Durch Kristallisation des Rückstandes aus EE wurden 3.43 g (9.2 mmol, 65%) N-tert-Butyl-2-(5-(pyridin-2-ylethinyl)thiophen-2-yl)imidazo[1,2-a]pyridin-3-amin erhalten.

### Beispiel 116: Synthese von N-tert-Butyl-2-(4-(pyridin-2-ylethinyl)phenyl)-imidazo[1,2-a]pyrazin-3-amin

Zu einer Lösung von 229 mg (2.4 mmol) 2-Aminopyrazin in Chloroform (20 ml) wurden nacheinander 500 mg (2.4 mmol) 4-Pyridin-2-ylethinyl-benzaldehyd, 200 mg (2.4 mmol) tert.-Butyl-isonitril und 215 µl (0.48 mmol) 20%-ige aq. Perchlorsäure gegeben. Die Reaktionslösung wurde 16 h bei 45 °C gerührt und anschließend mit DCM verdünnt. Danach wurde mit einer 1 M Na₂CO₃-Lsg und einer ges. aq. NaCl-Lsg gewaschen. Anschließend wurde über MgSO₄ getrocknet, filtriert und im Vakuum eingeengt. Mit dem Rückstand wurde eine SC (EE) durchgeführt, wobei 565 mg (1.5 mmol, 64%) N-tert-Butyl-2-(4-(pyridin-2-ylethinyl)phenyl)-imidazo[1,2-a]pyrazin-3-amin erhalten wurden.

### Beispiel 120: Synthese von 3-(tert-Butylamino)-2-(5-(phenylethinyl)thiophen-2-yl)imidazo[1,2-a]pyrazin-8-carbonsäure

Eine Lösung von 200 mg (0.47 mmol) 3-(tert-Butylamino)-2-(5-(phenylethinyl)-thiophen-2-yl)imidazo[1,2-a]pyrazin-8-carbonsäuremethylester (Beispiel 106) in einem Gemisch aus MeOH (5 ml) und MeCN (5.5 ml) wurde mit 5 m) (0.51 mmol) einer 0.1 M aq. NaOH-Lsg versetzt und 1 h bei RT gerührt. Anschließend wurde dier Reaktionsmischung mit 26 µl einer 5N aq. Essigsäure-Lösung versetzt. Danach wurden die Lösungmittel im Vakuum weitgehend abdestilliert. Die zurückgebliebene aq. Phase wurde mit EE mehrmals extrahiert. Die vereinigten organischen Phasen wurde über MgSO₄ getrocknet, filtriert und im Vakuum eingeengt. Durch Kristallisation des Rückstands aus einem DCM/Hexan (1:2) wurden 87 mg (0.21 mmol, 44%) 3-(tert-Butylamino)-2-(5-(phenylethinyl)thiophen-2-yl)imidazo[1,2-a]pyrazin-8-carbonsäure erhalten.

### Beispiel 141: Synthese von N-tert-Butyl-2-(5-(pyridin-2-ylethinyl)thiophen-2-yl)imidazo[1,2-a]pyrimidin-3-amin

Eine Lösung von 222 mg (2.34 mmol) 2-Aminopyrimidin, 499 mg (2.34 mmol) 5-Pyridin-2-ylethinyl-thiophen-2-carbaldehyd, 195 mg (2.34 mmol) tert.-Butyl-isonitril und 234 µl einer 20%-igen aq. Perchlorsäure in Chloroform (20 ml) wurde 3 d bei 47 °C gerührt. Anschließend wurde mit DCM verdünnt und mit einer 1 M aq. Na₂CO₃-Lsg versetzt. Die Phasen wurden getrennt und die organische Phase wurde mit einer ges. NaCl-Lsg gewaschen und über MgSO₄ getrocknet. Nach Filtrieren und Entfernen der Lösungsmittel im Vakuum wurde mit dem Rückstand eine SC (EE) durchgeführt, wobei 80 mg (0.21 mmol, 9%) N-tert-Butyl-2-(5-(pyridin-2-ylethinyl)thiophen-2-yl)imidazo[1,2-a]pyrimidin-3-amin erhalten wurden.

### Beispiel 143: Synthese von N-tert-Butyl-N-methyl-2-(5-(pyridin-2-ylethinyl)thiophen-2-yl)imidazo[1,2-a]pyrazin-3-amin

Zu einer Lösung von 135 mg (0.36 mmol) tert-Butyl-[2-(5-pyridin-2-ylethinyl-thiophen-2-yl)-imidazo[1,2-a]pyrazin-3-yl]-amin (Beispiel 77) in DMF (3 ml) wurden 43 mg (1.08 mmol, 60%-ig in Mineralöl) Natriumhydrid gegeben und es wurde 1 h bei RT gerührt. Anschließend wurde eine Lösung von 67 µl (1.08 mmol) Methyliodid in DMF (500 µl) hinzu getropft und weitere 16 h bei RT gerührt. Danach wurden erneut 43 mg (1.08 mmol, 60%-ig in Mineralöl) Natriumhydrid und eine Lösung von 67 µl (1.08 mmol) Methyliodid in DMF (500 µl) zugegeben und es wurde weitere 16 h bei RT gerührt. Die Reaktionslösung wurde nacheinander mit Wasser, einer 1 M aq. Na₂CO₃-Lsg. und einem Gemisch aus EE/THF (3:1) versetzt. Die organische Phase wurde abgetrennt und die aq. Phase wurde mit EE extrahiert. Die vereinigten organischen Phasen wurden über MgSO₄ getrocknet, filtriert und im Vakuum eingeengt. Durch SC (Aceton/MeCN 1:1) wurden 33 mg (0.09 mmol, 24%) N-tert-Butyl-N-methyl-2-(5-(pyridin-2-ylethinyl)thiophen-2-yl)imidazol[1,2-a]pyrazin-3-amin erhalten.

### Beispiel 156: Synthese von N-tert-Butyl-2-(5-(thiophen-3-ylethinyl)thiophen-2-yl)imidazo[1,2-a]pyrazin-3-amin Hydrochlorid

Zu einer Lösung von 699 mg (1.9 mmol) tert-Butyl-[2-(5-trimethylsilanylethinylthiophen-2-yl)-imidazo[1 ,2-a]pyrazin-3-yl]-amin (Synthese beschrieben unter Beispiel 158 Teil a) und 402 mg (2.5 mmol) 3-Brom-thiophen in DMF (20 ml) wurden 94 mg ( 0.5 mmol) Kupfer(I)-iodid, 129 mg (0.5 mmol) Triphenylphosphin, 1.32 ml (9.5 mmol) NEt₃, 701 mg (1.9 mmol) Tetra-n-butyl-ammonium-iodid und 173 mg (0.25 mmol) Bis(triphenylphosphin)-palladium(II)-chlorid gegeben. Zur Reaktionslösung wurde über einen Zeitraum von 1 h 2.5 ml (2.5 mmol) einer 1 M-Lsg Tetra-n-butylammoniumfluorid in THF getropft. Anschließend wurde 18 h bei 70 °C gerührt. Nach Abkühlen auf RT wurde mit EE verdünnt und mit einer 0.5M aq. Na₂CO₃-Lsg versetzt. Die Phasen wurden getrennt und die aq. Phase wurde mit EE extrahiert. Die vereinigten organischen Phasen wurden über MgSO₄ getrocknet, filtriert und im Vakuum eingeengt. Mit dem Rückstand wurde eine SC (Aceton/EE 1:3) durchgeführt, wobei 912 mg leicht verunreinigtes Produkt isoliert wurden. Durch Hydrochloridfällung nach dem bei Beispiel 192 beschriebenen Verfahren wurden 304 mg (0.73 mmol, 38%) N-tert-Butyl-2-(5-(thiophen-3-ylethinyl)thiophen-2-yl)imidazo[1 ,2-a]pyrazin-3-amin Hydrochlorid erhalten.

### Beispiel 158: N-tert-Butyl-2-(5-((6-methylpyridin-2-yl)ethinyl)thiophen-2-yl)imidazo[1,2-a]pyrazin-3-amin Hydrochlorid

### a) Synthese von tert-Butyl-[2-(5-trimethylsilanylethinyl-thiophen-2-yl)-imidazo[1,2-a]pyrazin-3-yl]-amin

Zu einer Lösung von 45.3 g (129 mmol) [2-(5-Brom-thiophen-2-yl)-imidazo[1,2-a]pyrazin-3-yl]-tert-butyl-amin (Synthese beschrieben unter Beispiel 18, Teil a) in DMF (760 ml) und NEt₃ (90 ml) wurden 21.4 ml (155 mmol) Trimethylsilylacetylen, 2.4 g (12.9 mmol) Kupfer(I)-iodid und 4.5 g (6.5 mmol) Bis(triphenylphosphin)-palladium(II)-chlorid gegeben. Die Reaktionslösung wurde 4 h auf 60 °C erhitzt und 16 h bei RT gerührt. Anschließend wurde mit einer 1 M aq. Na₂-CO₃-Lsg versetzt und mit EE verdünnt. Die Phasen wurden getrennt und die aq. Phase wurde mit EE extrahiert. Die vereinigten organischen Phasen wurden mit Wasser gewaschen und über MgSO₄ getrocknet. Nach filtrieren und Entfernen der Lösungsmittel im Vakuum wurde der erhaltenen Rückstand mit einem EE/DCM (1:1)-Gemisch aufgenommen und durch Kieselgur filtriert. Das Filtrat wurde im Vakuum eingeengt. Aus dem Rückstand wurden durch Kristallisation aus tert.-Butyl-methylether 30.8 g (84 mmol, 65%) tert-Butyl-[2-(5-trimethylsilanylethinyl-thiophen-2-yl)-imidazo[1,2-a]pyrazin-3-yl]-amin erhalten.

### b) Synthese von tert-Butyl-[2-(5-ethinyl-thiophen-2-yl)-imidazo[1,2-a]pyrazin-3-yl]-amin

Eine Lösung von 14.0 g (38.0 mmol) tert-Butyl-[2-(5-trimethylsilanylethinyl-thiophen-2-yl)-imidazo[1,2-a]pyrazin-3-yl]-amin in einem Gemisch aus MeOH (385 ml) und MeCN (440 ml) wurde mit 417 ml (41.8 mmol) einer 0.1 M aq. NaOH-Lsg versetzt. Die Reaktionslösung wurde 1 h bei RT gerührt. Anschließend wurden die Lösungsmittel im Vakuum weitgehend entfernt. Die so erhaltene aq. Lösung wurde mehrmals mit EE extrahiert und die vereinigten organischen Phasen wurde über MgSO₄ getrocknet, filtriert und im Vakuum eingeengt. Dabei wurden 11.1 g (37.4 mmol, 99%) tert-Butyl-[2-(5-ethinyl-thiophen-2-yl)-imidazo[1,2-a]pyrazin-3-yl]-amin erhalten.

### c) Synthese von N-tert-Butyl-2-(5-((6-methylpyridin-2-yl)ethinyl)thiophen-2-yl)imidazo[1,2-a]pyrazin-3-amin

300 mg (1.0 mmol) tert-Butyl-[2-(5-ethinyl-thiophen-2-yl)-imidazo[1,2-a]pyrazin-3-yl]-amin wurden zusammen mit 138 µl (1.2 mmol) 2-Brom-6-methyl-pyridin in DMF (7 ml) gelöst. Zu dieser Lösung wurden 1.4 ml (10.0 mmol) NEt₃, 19 mg (0.1 mmol) Kupfer(I)-iodid und 71 mg (0.1 mmol) Bis(triphenylphosphin)-palladium(II)-chlorid gegeben. Die Reaktionsmischung wurde 6 h auf 50 °C erhitzt. Anschließend wurde mit EE verdünnt und mit einer 1 M aq. Na₂CO₃-Lsg. versetzt. Die Phasen wurden getrennt und die aq. Phase wurde mit EE extrahiert. Die vereinigten organischen Phasen wurden über MgSO₄ getrocknet, filtriert und im Vakuum eingeengt. Durch SC (Aceton/MeCN 1:1) mit dem Rückstand wurden 166 mg (0.42 mmol, 42%) N-tert-Butyl-2-(5-((6-methylpyridin-2-yl)ethinyl)thiophen-2-yl)imidazo[1,2-a]pyrazin-3-amin erhalten.

### d) Synthese von N-tert-Butyl-2-(5-((6-methylpyridin-2-yl)ethinyl)thiophen-2-yl)imidazo[1,2-a]pyrazin-3-amin Hydrochlorid

Aus 161 mg (0.42 mmol) N-tert-Butyl-2-(5-((6-methylpyridin-2-yl)ethinyl)thiophen-2-yl)imidazo[1,2-a]pyrazin-3-amin wurden nach dem bei Beispiel 192 beschriebenen Verfahren 115 mg (0.27 mmol, 65%) N-tert-Butyl-2-(5-((6-methylpyridin-2-yl)ethinyl)thiophen-2-yl)imidazo[1,2-a]pyrazin-3-amin Hydrochlorid erhalten.

### Beispiel 165: Synthese von N-tert-Butyl-2-(5-((6-fluorpyridin-2-yl)ethinyl)thiophen-2-yl)imidazo[1,2-a]pyrazin-3-amin

500 mg (1.69 mmol) tert-Butyl-[2-(5-ethinyl-thiophen-2-yl)-imidazo[1,2-a]pyrazin-3-yl]-amin (Synthese beschrieben unter Beispiel 158 Teil b) wurden zusammen mit 355 mg (2.02 mmol) 2-Brom-6-fluor-pyridin in DMF (20 ml) gelöst. Zu dieser Lösung wurden 2.4 ml (16.9 mmol) NEt₃, 32 mg (0.17 mmol) Kupfer(I)-iodid und 119 mg (0.17 mmol) Bis(triphenylphosphin)-palladium(II)-chlorid gegeben. Die Reaktionsmischung wurde 6 h auf 50 °C erhitzt. Anschließend wurde mit EE verdünnt und mit einer 1 M aq. Na₂CO₃-Lsg versetzt. Die Phasen wurden getrennt und die aq. Phase wurde mit EE extrahiert. Die vereinigten organischen Phasen wurden mit einer 1 M aq. Na₂CO₃-Lsg gewaschen, über MgSO₄ getrocknet, filtriert und im Vakuum eingeengt. Durch SC (EE) mit dem Rückstand wurden 200 mg (0.51 mmol, 30%) N-tert-Butyl-2-(5-((6-fluorpyridin-2-yl)ethinyl)thiophen-2-yl)imidazo[1,2-a]pyrazin-3-amin erhalten.

### Beispiel 176: Synthese von N-tert-Butyl-2-(5-(phenylethinyl)thiazol-2-yl)imidazo[1,2-a]pyrazin-3-amin

Eine Lösung von 290 mg (3.0 mmol) 2-Aminopyrazin, 650 mg (3.0 mmol) 5-Pyridin-2-ylethinyl-thiazol-2-carbaldehyd, 344 µl (3.0 mmol) tert.-Butyl-isonitril und 305 µl einer 20%-igen aq. Perchlorsäure in DCM (6 ml) wurde 6 d bei RT gerührt. Anschließend wurde mit Ether verdünnt und die Phasen wurden getrennt. Die organische Phase wurde mit Wasser gewaschen und über MgSO₄ getrocknet. Nach Filtrieren und Entfernen der Lösungsmittel im Vakuum wurde mit dem Rückstand eine SC (Hexan/tert.-Butyl-methylether 3:2) durchgeführt, wobei 170 mg (0.46 mmol, 15%) N-tert-Butyl-2-(5-(phenylethinyl)thiazol-2-yl)imidazo[1,2-a]pyrazin-3-amin erhalten wurden.

### Beispiel 192: Synthese von N-tert-Butyl-2-(5-(pyridin-2-ylethinyl)thiophen-2-yl)imidazo[1,2-a]pyridin-3-amin Hydrochlorid

Eine Lösung von 6.93 g (18.6 mmol) N-tert-Butyl-2-(5-(pyridin-2-ylethinyl)thiophen-2-yl)imidazo[1,2-a]pyridin-3-amin (Beispiel 94) in DCM (180 ml) wurde mit 335 µl (18.6 mmol) Wasser und 2.35 ml (18.6 mmol) Chlortrimethylsilan versetzt und 16 h bei RT gerührt. Der entstandene Niederschlag wurde abfiltriert und im Vakuum bei 40 °C getrocknet. Dabei wurden 6.09 g (14.8 mmol, 80%) N-tert-butyl-2-(5-(pyridin-2-ylethinyl)thiophen-2-yl)imidazo[1,2-a]pyridin-3-amin Hydrochlorid erhalten.

### Beispiel 331: tert-Butyl-[2-(5-pyridin-2-ylethinyl-thiophen-2-yl)-imidazo(1,2-b]pyridazin-3-yl]-amin Hydrochlorid

### a) Synthese von [2-(5-Brom-thiophen-2-yl)-imidazo[1,2-b]pyridazin-3-yl]-tert-butyl-amin

Zu einer Lösung von 15.0 g (158 mmol) 3-Amino-pyridazin in DCM (150 ml) wurden 13.1 g (158 mmol) tert.-Butyl-isonitril, 30.2 g (158 mmol) 5-Brom-thiophen-2-carboxaldehyd und 5.2 g einer 20%-igen aq. Perchlorsäure-Lsg gegeben. Die Reaktionslösung wurde 5 d bei 40 °C gerührt. Anschließend wurde mit Wasser (200 ml) versetzt und die Phasen wurden getrennt. Die aq. Phase wurde mit DCM extrahiert. Die vereinigten organischen Phasen wurden nacheinander mit einer ges. NaHCO₃-Lsg und einer ges. aq. NaCl-Lsg. gewaschen, über Na₂SO₄ getrocknet, filtriert und im Vakuum eingeengt. Mit dem Rückstand wurde eine SC (Alumina, EE/Hexan 2:98) durchgeführt, wobei 1.7 g (4.8 mmol, 3%) [2-(5-Brom-thiophen-2-yl)-imidazo[1,2-b]pyridazin-3-yl]-tert-butyl-amin erhalten wurden.

### b) Synthese von tert-Butyl-[2-(5-pyridin-2-ylethinyl-thiophen-2-yl)-imidazo[1,2-b]pyridazin-3-yl]-amin Hydrochlorid

Zu einer Lösung von 2.0 g (5.7 mmol) [2-(5-Brom-thiophen-2-yl)-imidazo[1,2-b]pyridazin-3-yl]-tert-butyl-amin in DMF (50 ml) wurden 0.42 ml (7.4 mmol) 2-Ethinylpyridin, 7.9 ml (57.0 mmol) NEt₃, 280 mg (0.26 mmol) Kupfer(I)-iodid und 519 mg (0.74 mmol) Bis(triphenylphosphin)-palladium(II)-chlorid gegeben. Die Reaktionslösung wurde 6 h auf 50 °C erhitzt. Anschließend wurde mit EE verdünnt und mit einer 1 M aq. Na₂CO₃-Lsg versetzt. Die Phasen wurden getrennt und die aq. Phase wurde mit EE extrahiert. Die vereinigten organischen Phasen wurden über MgSO₄ getrocknet, filtriert und im Vakuum eingeengt. Durch SC (EE) mit dem Rückstand wurden 546 mg Amin erhalten. Daraus wurden nach dem bei Beispiel 192 beschriebenen Verfahren 440 mg (1.1 mmol, 19%) tert-Butyl-[2-(5-pyridin-2-ylethinyl-thiophen-2-yl)-imidazo[1,2-b]pyridazin-3-yl]-amin Hydrochlorid hergestellt.

### Beispiel 332: tert-Butyl-[2-(5-thiazol-2-ylethinyl-thiophen-2-yl)-imidazo[1,2-a]pyridin-3-yl]-amin Hydrochlorid

### a) Synthese von [2-(5-Brom-thiophen-2-yl)-imidazo[1,2-a]pyridin-3-yl]-tert-butyl-amin

Eine Lösung von 32.3 g (343.4 mmol) 2-Aminopyridin, 37.2 ml (343.4 mmol) 5-Pyridin-2-ylethinyl-thiophen-2-carbaldehyd, 28.6 g (343.4 mmol) tert.-Butyl-isonitril und 34.3 ml einer 20%-igen aq. Perchlorsäure in DCM (660 ml) wurde 10 d bei RT gerührt. Anschließend wurde die Reaktionslösung mit DCM verdünnt und nacheinander mit einer 1 M aq. Na₂CO₃-Lsg und einer ges. aq. NaCl-Lsg gewaschen. Die organische Phase wurde über MgSO₄ getrocknet, filtriert und in Vakuum eingeengt. Aus dem Rückstand wurden durch Kristallisation aus EE 56.3 g (160.7 mmol, 47%) [2-(5-Brom-thiophen-2-yl)-imidazo[1,2-a]pyridin-3-yl]-tert-butyl-amin erhalten.

### b) Synthese von tert-Butyl-[2-(5-trimethylsilanylethinyl-thiophen-2-yl)-imidazo[1,2-a]pyridin-3-yl]-amin

Zu einer Lösung von 10.5 g (30.0 mmol) [2-(5-Brom-thiophen-2-yl)-imidazo[1,2-a]pyridin-3-yl]-tert-butyl-amin in DMF (180 ml) und NEt₃ (21 ml) wurden 5.0 ml (36.0 mmol) Trimethylsilylacetylen, 0.57 g (3.0 mmol) Kupfer(I)-iodid und 2.1 g (3.0 mmol) Bis(triphenylphosphin)-palladium(II)-chlorid gegeben. Die Reaktionslösung wurde 16 h bei 50 °C gerührt. Anschließend wurde mit einer 1 M aq. Na₂CO₃-Lsg versetzt und mit EE verdünnt. Diese Mischung wurde über Kieselgur filtriert. Danach wurden die Phasen getrennt und die aq. Phase wurde mit EE extrahiert. Die vereinigten organischen Phasen wurden über MgSO₄ getrocknet, filtriert und in Vakuum eingeengt. Mit dem Rückstand wurde eine SC (EE/DCM 1:1) durchgeführt, wobei 8.0 g (21.8 mmol, 73%) tert-Butyl-[2-(5-trimethylsilanylethinyl-thiophen-2-yl)-imidazo[1,2-a]pyridin-3-yl]-amin erhalten wurden.

### c) Synthese von tert-Butyl-[2-(5-ethinyl-thiophen-2-yl)-imidazo[1,2-a]pyridin-3-yl]-amin

11.5 g (31.3 mmol) tert-Butyl-[2-(5-trimethylsilanylethinyl-thiophen-2-yl)-imidazo[1,2-a]pyridin-3-yl]-amin wurden in einem Gemisch aus MeOH (230 ml) und DCM (50 ml) gelöst und mit 431 mg (3.13 mmol) Kaliumcarbonat versetzt. Die Reaktionslösung wurde 30 min bei RT gerührt und anschließend mit Wasser (200 ml) versetzt. Danach wurde mit DCM extrahiert und die organische Phase wurde mit Wasser gewaschen und über MgSO₄ getrocknet. Nach Filtrieren und Einengen im Vakuum wurden 9.0 g (30.5 mmol, 97%) tert-Butyl-[2-(5-ethinyl-thiophen-2-yl)-imidazo[1,2-a]pyridin-3-yl]-amin erhalten.

### d) Synthese von tert-Butyl-[2-(5-thiazol-2-ylethinyl-thiophen-2-yl)-imidazo[1,2-a]pyridin-3-yl]-amin Hydrochlorid

Zu einer Lösung von 1.0 g (3.4 mmol) tert-Butyl-[2-(5-ethinyl-thiophen-2-yl)-imidazo[1,2-a]pyridin-3-yl]-amin in EE (20 ml) wurden 383 µl (4.3 mmol) 2-Brom-thiazol, 882 µl (6.36 mmol) Triethylamin, 43 mg (0.23 mmol) Kupfer(I)-iodid und 87 mg (0.13 mmol) Bis(triphenyl-phosphin)-palladium(II)-chlorid gegeben. Diese Reaktionslösung wurde 16 h bei 50 °C gerührt. Anschließend wurde durch Kieselgur filtriert und im Vakuum eingeengt. Mit dem Rückstand wurde eine SC (EE/DCM 3:7) durchgeführt, wobei 902 mg (2.4 mmol, 70%) tert-Butyl-[2-(5-thiazol-2-ylethinyl-thiophen-2-yl)-imidazo[1,2-a]pyridin-3-yl]-amin erhalten wurden. Daraus wurde nach dem bei Beispiel 192 beschriebenen Verfahren tert-Butyl-[2-(5-thiazol-2-ylethinyl-thiophen-2-yl)-imidazo[1,2-a]pyridin-3-yl]-amin Hydrochlorid hergestellt.

Die zuvor nicht detailliert beschriebene Herstellung der übrigen Verbindungen gemäß den nachstehend angegebenen Beispielen erfolgte ebenfalls analog den vorstehend angegebenen Herstellungsvorschriften, wobei dem Fachmann aufgrund dieser Vorschriften die jeweils eingesetzten Edukte bekannt sind.

| **Beispiel** | **Name** |
|---|---|
| 1 | Cyclopentyl-[2-(5-phenylethinyl-thiophen-2-yl)-imidazo[1,2-a]pyrazin-3-yl]-amin |
| 2 | Cyclohexylmethyl-[5-methyl-2-(5-phenylethinyl-thiophen-2-yl)-imidazo[1,2-a]pyridin-3-yl]-amin |
| 3 | Cyclohexylmethyl-[6-methyl-2-(5-phenylethinyl-thiophen-2-yl)-imidazo[1,2-a]pyridin-3-yl]-amin |
| 4 | Cyclohexylmethyl-7-methyl-2-(5-phenylethinyl-thiophen-2-yl)-imidazo[1,2-a]pyridin-3-yl]-amin |
| 5 | Cyclohexylmethyl-[8-methyl-2-(5-phenylethinyl-thiophen-2-yl)-imidazo[1,2-a]pyridin-3-yl]-amin |
| 6 | Cyclohexylmethyl-[5,7-dimethyl-2-(5-phenylethinyl-thiophen-2-yl)-imidazo[1,2-a]pyridin-3-yl]-amin |
| 7 | [8-Benzyloxy-2-(5-phenylethinyl-thiophen-2-yl)-imidazo[1,2-a]pyridin-3-yl]-cyclohexylmethyl-amin |
| 8 | Cyclohexylmethyl-[2-(5-phenylethinyl-thiophen-2-yl)-imidazo[1,2-a]pyrazin-3-yl]-amin |
| 9 | Cyclohexylmethyl-[2-(5-phenylethinyl-thiophen-2-yl)-imidazo[1,2-a]pyrimidin-3-yl]-amin |
| 10 | (4-Methoxy-benzyl)-[5-methyl-2-(5-phenylethinyl-thiophen-2-yl)-imidazo[1,2-a]pyridin-3-yl]-amin |
| 11 | (4-Methoxy-benzyl)-[6-methyl-2-(5-phenylethinyl-thiophen-2-yl)-imidazo[1,2-a]pyridin-3-yl]-amin |
| 12 | (4-Methoxy-benzyl)-[7-methyl-2-(5-phenylethinyl-thiophen-2-yl)-imidazo[1,2-a]pyridin-3-yl]-amin |
| 13 | (4-Methoxy-benzyl)-[8-methyl-2-(5-phenylethinyl-thiophen-2-yl)-imidazo[1,2-a]pyridin-3-yl]-amin |
| 14 | [5,7-Dimethyl-2-(5-phenylethinyl-thiophen-2-yl)-imidazo[1,2-a]pyridin-3-yl]-(4-methoxy-benzyl)-amin |
| 15 | [8-Benzyloxy-2-(5-phenylethinyl-thiophen-2-yl)-imidazo[1,2-a]pyridin-3-yl]-(4-methoxy-benzyl)-amin |
| 16 | (4-Methoxy-benzyl)-[2-(5-phenylethinyl-thiophen-2-yl)-imidazo[1,2-a]pyridin-3-yl]-amin |
| 17 | (4-Methoxy-benzyl)-[2-(5-phenylethinyl-thiophen-2-yl)-imidazo[1,2-a]pyrazin-3-yl]-amin |
| 18 | tert-Butyl-[2-(5-phenylethinyl-thiophen-2-yl)-imidazo[1,2-a]pyrazin-3-yl]-amin |
| 19 | tert-Butyl-[6-methyl-2-(5-phenylethinyl-thiophen-2-yl)-imidazo[1,2-a]pyridin-3-yl]-amin |
| 20 | [8-Benzyloxy-2-(5-phenylethinyl-thiophen-2-yl)-imidazo[1,2-a]pyridin-3-yl]-(3-methoxy-phenyl)-amin |
| 21 | (3-Methoxy-benzyl)-[5-methyl-2-(5-phenylethinyl-thiophen-2-yl)-imidazo[1,2-a]pyridin-3-yl]-amin |
| 22 | (3-Methoxy-benzyl)-[8-methyl-2-(5-phenylethinyl-thiophen-2-yl)-imidazo[1,2-a]pyridin-3-yl]-amin |
| 23 | [5,7-Dimethyl-2-(5-phenylethinyl-thiophen-2-yl)-imidazo[1,2-a]pyridin-3-yl]-(3-methoxy-benzyl)-amin |
| 24 | [8-Benzyloxy-2-(5-phenylethinyl-thiophen-2-yl)-imidazo[1,2-a]pyridin-3-yl]-(3-methoxy-benzyl)-amin |
| 25 | (3-Methoxy-benzyl)-[2-(5-phenylethinyl-thiophen-2-yl)-imidazo[1,2-a]pyridin-3-yl]-amin |
| 26 | (3-Methoxy-benzyl)-[2-(5-phenylethinyl-thiophen-2-yl)-imidazo[1,2-a]pyrazin-3-yl]-amin |
| 27 | [6-Methyl-2-(5-phenylethinyl-thiophen-2-yl)-imidazo[1,2-a]pyridin-3-yl]-(1-phenyl-ethyl)-amin |
| 28 | [7-Methyl-2-(5-phenylethinyl-thiophen-2-yl)-imidazo[1,2-a]pyridin-3-yl]-(1-phenyl-ethyl)-amin |
| 29 | [5,7-Dimethyl-2-(5-phenylethinyl-thiophen-2-yl)-imidazo[1,2-a]pyridin-3-yl]-(1-phenyl-ethyl)-amin |
| 30 | [8-Benzyloxy-2-(5-phenylethinyl-thiophen-2-yl)-imidazo[1,2-a]pyridin-3-yl]-(1-phenyl-ethyl)-amin |
| 31 | (1-Phenyl-ethyl)-[2-(5-phenylethinyl-thiophen-2-yl)-imidazo[1,2-a]pyrazin-3-yl]-amin |
| 32 | (2-Ch lor-benzyl)-[5,7-dimethyl-2-(5-phenylethinyl-thiophen-2-yl)-imidazo[1,2-a]pyridin-3-yl]-amin |
| 33 | (3-Chlor-4-fluor-phenyl)-[7-phenyl-2-(5-phenylethinyl-thiophen-2-yl)-imidazo[1,2-a]pyridin-3-yl]-amin |
| 34 | (4-Methoxy-benzyl)-[7-methyl-2-(5-phenylethinyl-thiophen-2-yl)-imidazo[1,2-a]pyrimidin-3-yl]-amin |
| 35 | [8-Methyl-2-(5-phenylethinyl-thiophen-2-yl)-imidazo[1,2-a]pyridin-3-yl]-(1-phenyl-ethyl)-amin |
| 36 | [7-Methyl-2-(5-phenylethinyl-thiophen-2-yl)-imidazo[1,2-a]pyrimidin-3-yl]-(1-phenyl-ethyl)-amin |
| 37 | (2-Methoxy-benzyl)-[7-methyl-2-(5-phenylethinyl-thiophen-2-yl)-imidazo[1,2-a]pyridin-3-yl]-amin |
| 38 | (2-Methoxy-benzyl)-[2-(5-phenylethinyl-thiophen-2-yl)-imidazo[1,2-a]pyridin-3-yl]-amin |
| 39 | (2-Chlor-benzyl)-[2-(5-phenylethinyl-thiophen-2-yl)-imidazo[1,2-a]pyridin-3-yl]-amin |
| 40 | (2-Methoxy-benzyl)-[7-methyl-2-(5-phenylethinyl-thiophen-2-yl)-imidazo[1,2-a]pyrimidin-3-yl]-amin |
| 41 | (3-Methoxy-phenyl)-(6-methyl-2-(5-phenylethinyl-thiophen-2-yl)-imidazo[1,2-a]pyridin-3-yl]-amin |
| 42 | [5,7-Dimethyl-2-(5-phenylethinyl-thiophen-2-yl)-imidazo[1,2-a]pyridin-3-yl]-(3-methoxy-phenyl)-amin |
| 43 | (3-Methoxy-phenyl)-[2-(5-phenylethinyl-thiophen-2-yl)-imidazo[1,2-a]pyrazin-3-yl]-amin |
| 44 | [7-Ethyl-2-(5-phenylethinyl-thiophen-2-yl)-imidazo[1,2-a]pyridin-3-yl]-(2-methoxy-benzyl)-amin |
| 45 | (2-Chlor-benzyl)-[7-ethyl-2-(5-phenylethinyl-thiophen-2-yl)-imidazo[1,2-a]pyridin-3-yl]-amin |
| 46 | [7-tert-Butyl-2-(5-phenylethinyl-thiophen-2-yl)-imidazo(1,2-a]pyridin-3-yl]-(3-chlor-4-fluor-phenyl)-amin |
| 47 | (3-Methoxy-benzyl)-[7-methyl-2-(5-phenylethinyl-thiophen-2-yl)-imidazo[1,2-a]pyrimidin-3-yl]-amin |
| 48 | [7-Ethyl-2-(5-phenylethinyl-thiophen-2-yl)-imidazo[1,2-a]pyridin-3-yl]-(2-fluor-phenyl)-amin |
| 49 | [7-tert-Butyl-2-(5-phenylethinyl-thiophen-2-yl)-imidazo[1,2-a]pyridin-3-yl]-(2-fluor-phenyl)-amin |
| 50 | (2,4-Difluor-phenyl)-[2-(5-phenylethinyl-thiophen-2-yl)-7-(3-phenyl-propyl)-imidazo[1,2-a]pyridin-3-yl]-amin |
| 51 | (4-Fluor-benzyl)-[7-methyl-2-(5-phenylethinyl-thiophen-2-yl)-imidazo[1,2-a]pyridin-3-yl]-amin |
| 52 | [5,7-Dimethyl-2-(5-phenylethinyl-thiophen-2-yl)-imidazo[1,2-a]pyridin-3-yl]-(4-fluor-benzyl)-amin |
| 53 | [7-Ethyl-2-(5-phenylethinyl-thiophen-2-yl)-imidazo[1,2-a]pyridin-3-yl]-(3-trifluormethyl-benzyl)-amin |
| 54 | [7-Isopropyl-2-(5-phenylethinyl-thiophen-2-yl)-imidazo[1,2-a]pyridin-3-yl]-(3-trifluormethyl-benzyl)-amin |
| 55 | tert-Butyl-[2-(4-phenylethinyl-thiophen-2-yl)-imidazo[1,2-a]pyrazin-3-yl]-amin |
| 56 | [2-(5-Phenylethinyl-thiophen-2-yl)-imidazo[1,2-a]pyrazin-3-yl]-(1,1,3,3-tetramethyl-butyl)-amin |
| 57 | Butyl-[2-(4-phenylethinyl-thiophen-2-yl)-imidazo[1,2-a]pyrazin-3-yl]-amin |
| 59 | [2-(5-Phenylethinyl-thiophen-2-yl)-imidazo[1,2-a]pyridin-3-yl]-(1,1,3,3-tetramethyl-butyl)-amin |
| 60 | [2-(5-Pyridin-3-ylethinyl-thiophen-2-yl)-imidazo[1,2-a]pyrazin3-yl]-(1,1,3,3-tetramethyl-butyl)-amin |
| 61 | [2-(5-Pyridin-4-ylethinyl-thiophen-2-yl)-imidazo[1,2-a]pyrazin-3-yl]-(1,1,3,3-tetramethyl-butyl)-amin |
| 62 | [6-Chlor-2-(5-phenylethinyl-thiophen-2-yl)-imidazo[1,2-a]pyridin-3-yl]-(1,1,3,3-tetramethyl-butyl)-amin |
| 63 | [6,8-Dichlor-2-(5-phenylethinyl-thiophen-2-yl)-imidazo[1,2-a]pyridin-3-yl]-(1,1,3,3-tetramethyl-butyl)-amin |
| 64 | [2-(5-Pyridin-2-ylethinyl-thiophen-2-yl)-imidazo[1,2-a]pyrazin-3-yl]-(1,1,3,3-tetramethyl-butyl)-amin |
| 65 | Dimethyl-[2-(5-phenylethinyl-thiophen-2-yl)-imidazo[1,2-a]pyrazin-3-yl]-amin |
| 66 | Methyl-[2-(5-phenylethinyl-thiophen-2-yl)-imidazo[1,2-a]pyrazin-3-yl]-amin |
| 67 | N-[2-(5-Phenylethinyl-thiophen-2-yl)-imidazo[1,2-a]pyrazin-3-yl]-acetamid |
| 68 | Ethyl-(2-(5-phenylethinyl-thiophen-2-yl)-imidazo[1,2-a]pyrazin-3-yl]-amin |
| 69 | Propyl-[2-(5-phenylethinyl-thiophen-2-yl)-imidazo[1,2-a]pyrazin-3-yl]-amin |
| 70 | Butyl-[2-(5-phenylethinyl-thiophen-2-yl)-imidazo[1,2-a]pyrazin-3-yl]-amin |
| 71 | (2-Methylpropyl)-[2-(5-phenylethinyl-thiophen-2-yl)-imidazo[1,2-a]pyrazin-3-yl]-amin |
| 72 | Pentyl-[2-(5-phenylethinyl-thiaphen-2-yl)-imidazo[1,2-a]pyrazin-3-yl]-amin |
| 73 | {(Methoxycarbonylmethyl)-[2-(5-phenylethynyl-thiophen-2-yl)-imidazo[1,2-a]pyrazin-3-yl(-amino}-essigsäuremethylester |
| 74 | Benzyl-[2-(5-phenylethynyl-thiophen-2-yl)-imidazo[1,2-a]pyrazin-3-yl]-amin |
| 75 | [2-(5-Phenylethynyl-thiophen-2-yl)-imidazo[1,2-a]pyrazin-3-ylamino]-essigsäuremethylester |
| 76 | tert-Butyl-[2-(5-pyridin-4-ylethinyl-thiophen-2-yl)-imidazo[1,2-a]pyrazin-3-yl]-amin |
| 77 | tert-Butyl-[2-(5-pyridin-2-ylethinyl-thiophen-2-yl)-imidazo[1,2-a]pyrazin-3-yl]-amin |
| 78 | N-[2-(5-Phenylethinyl-thiophen-2-yl)-imidazo[1,2-a]pyrazin-3-yl]-benzamid |
| 79 | [2-(5-Phenylethinyl-thiophen-2-yl)-imidazo[1,2-a]pyrazin-3-yl]-bis-pyridin -3-ylmethyl-amin |
| 80 | 2,2-Dimeihyl-N-[2-(5-phenylethinyl-thiophen-2-yl)-imidazo[1,2-a]pyrazin-3-yl]-propionamid |
| 81 | 3-Methoxy-N-[2-(5-phenylethinyl-thiophen-2-yl)-imidazo[1,2-a]pyrazin-3-yl]-benzamid |
| 82 | tert-Butyl-[2-(5-pyridin-3-ylethinyl-thiophen-2-yl)-imidazo[1,2-a]pyrazin-3-yl]-amin |
| 83 | 2-(5-Phenylethinyl-thiophen-2-yl)-imidazo[1,2-a]pyrazin-3-ylamin |
| 84 | Methyl-2-(5-(phenylethinyl)thiophen-2-yl)-3-(2,4,4-trimethylpentan-2-ylamino)imidazo[1,2-a]pyrazin-8-carboxylat |
| 85 | 2-(5-(Phenylethinyl)thiophen-2-yl)-N-(2,4,4-trimethylpentan-2-yl)imidazo[1,2-a]pyrimidin-3-amin |
| 86 | 2-(5-((6-Methylpyridin-2-yl)ethinyl)thiophen-2-yl)-N-(2,4,4-trimethylpentan-2-yl)imidazo[1,2-a]pyrazin-3-amin |
| 87 | N-Cyclohexyl-2-(5-(phenylethinyl)thiophen-2-yl)imidazo[1,2-a]pyrazin-3-amin |
| 88 | 2-(5-(Phenylethinyl)thiophen-2-yl)-3-(piperidin-1-yl)imidazo[1,2-a]pyrazin |
| 89 | N-tert-Butyl-N-methyl-2-(5-(phenylethinyl)thiophen-2-yl)imidazo[1,2-a]pyrazin-3-amin |
| 90 | Methyl-2-(5-(phenylethinyl)thiophen-2-yl)-3-(2,4,4-trimethylpentan-2-ylamino)imidazo[1,2-a]pyridin-6-carboxylat |
| 91 | N-tert-Butyl-2-(5-(pyridin-2-ylethinyl)thiophen-2-yl)imidazo[1,2-a]pyrazin-3-amin Hydrochlorid |
| 92 | 8-Brom-N-cyclopentyl-6-methyl-2-(5-(pyridin-2-ylethinyl)thiophen-2-yl)imidazo[1,2-a]pyridin-3-amin |
| 93 | N,N-Diethyl-2-(5-(phenylethinyl)thiophen-2-yl)imidazo[1,2-a]pyrazin-3-amin |
| 94 | N-tert-Butyl-2-(5-(pyridin-2-ylethinyl)thiophen-2-yl)imadazo[1,2-a]pyridin-3-amin |
| 95 | N-tert-Butyl-2-(5-(phenylethinyl)furan-2-yl)imidazo[1,2-a]pyrazin-3-amin |
| 96 | 8-Brom-N-tert-butyl-6-methyl-2-(3-(pyridin-2-ylethinyl)phenyl)imidazo[1,2-a]pyridin-3-amin |
| 97 | N-tert-Butyl-8-methyl-2-(5-(phenylethinyl)thiophen-2-yl)imidazo[1,2-a]pyridin-3-amin |
| 98 | N-Methyl-2-(5-(phenylethinyl)thiophen-2-yl)-N-(2,4,4-trimethylpentan-2-yl)imidazo[1,2-a]pyrazin-3-amin Hydrochlorid |
| 99 | 2-(5-(Phenylethinyl)thiophen-2-yl)-3-(pyrrolidin-1-yl)imidazo[1,2-a]pyrazin Hydrochlorid |
| 100 | N-tert-Butyl-2-(5-((4-fluorphenyl)ethinyl)thiophen-2-yl)imidazo[1,2-a]pyrazin-3-amin Hydrochlorid |
| 101 | N-tert-Butyl-7-methyl-2-(5-(phenylethinyl)thiophen-2-yl)imidazo[1,2-a]pyridin-3-amin |
| 102 | N-tert-Butyl-5-methyl-2-(5-(phenylethinyl)thiophen-2-yl)imidazo[1,2-a]pyridin-3-amin |
| 103 | 8-Chlor-2-(3-(pyridin-2-ylethinyl)phenyl)-6-(trifluormethyl)-N-(2,4,4-trimethylpentan-2-yl)imidazo[1,2-a]pyridin-3-amin |
| 104 | N-tert-Butyl-2-(5-((3-fluorphenyl)ethinyl)thiophen-2-yl)imidazo[1,2-a]pyrazin-3-amin Hydrochlorid |
| 105 | N-tert-Butyl-2-(5-((2-fluorphenyl)ethinyl)thiophen-2-yl)imidazo[1,2-a]pyrazin-3-amin Hydrochlorid |
| 106 | Methyl-3-(tert-butylamino)-2-(5-(phenylethinyl)thiophen-2-yl)imidazo[1,2-a]pyrazin-8-carboxylat |
| 107 | N-tert-Butyl-2-(5-(pyrazin-2-ylethinyl)thiophen-2-yl)imidazo[1,2-a]pyrazin-3-amin |
| 108 | 2-(5-((4-Aminophenyl)ethinyl)thiophen-2-yl)-N-tert-butylimidazo[1,2-a]pyrazin-3-amin |
| 109 | N-Isopropyl-2-(5-(phenylethinyl)thiophen-2-yl)imidazo[1,2-a]pyrazin-3-amin |
| 110 | N-tert-Butyl-2-(5-(thiophen-2-ylethinyl)thiophen-2-yl)imidazo[1,2-a]pyrazin-3-amin Hydrochlorid |
| 111 | N-tert-Butyl-2-(3-(pyridin-2-ylethinyl)phenyl)imidazo[1,2-a]pyrazin-3-amin |
| 112 | N-tert-Butyl-2-(5-((2-methoxyphenyl)ethinyl)thiophen-2-yl)imidazo[1,2-a]pyrazin-3-amin Hydrochlorid |
| 113 | N-tert-Butyl-2-(5-((3-methoxyphenyl)ethinyl)thiophen-2-yl)imidazo[1,2-a]pyrazin-3-amin Hydrochlorid |
| 114 | N-tert-Butyl-2-(5-((4-methoxyphenyl)ethinyl)thiophen-2-yl)imidazo[1,2-a]pyrazin-3-amin Hydrochlorid |
| 115 | N-tert-Butyl-2-(5-(phenylethinyl)thiophen-2-yl)imidazo[1,2-a]chinolin-1-amin |
| 116 | N-tert-Butyl-2-(4-(pyridin-2-ylethinyl)phenyl)imidazo[1,2-a]pyrazin-3-amin |
| 117 | N-tert-Butyl-2-(3-((6-methylpyridin-2-yl)ethinyl)phenyl)imidazo[1,2-a]pyrazin-3-amin |
| 118 | N-tert-Butyl-2-(2-methyl-6-(phonylethinyl)pyridin-3-yl)imidazo[1,2-a]pyrazin-3-amin |
| 119 | N-tert-Butyl-2-(5-(pyridin-2-ylethinyl)furan-2-yl)imidazo[1,2-a]pyrazin-3-amin |
| 120 | 3-(tert-Butylamino)-2-(5-(phenylethinyl)thiophen-2-yl)imidazo[1,2-a]pyrazin-8-carbonsäure |
| 121 | 4-((5-(3-(tert-Butylamino)imidazo[1,2-a]pyrazin-2-yl)thiophen-2-yl)ethinyl)phenol Hydrochlorid |
| 122 | 3-((5-(3-(tert-Butylamino)imidazo[1,2-a]pyrazin-2-yl)thiophen-2-yl)ethinyl)phenol |
| 123 | 2-(5-((3-Aminophenyl)ethinyl)thiophen-2-yl)-N-tert-butylimidazo[1,2-a]pyrazin-3-amin Hydrochlorid |
| 124 | 2-(5-((2-Aminophenyl)ethinyl)thiophen-2-yl)-N-tert-butylimidazo[1,2-a]pyrazin-3-amin Hydrochlorid |
| 125 | N-tert-Butyl-2-(5-(phenylethinyl)thiophen-2-yl)imidazo[2,1-a]isochinolin-3-amin |
| 126 | N-tert-Butyl-2-(6-(phenylethinyl)pyridin-3-yl)imidazo[1,2-a]pyrazin-3-amin |
| 127 | N-tert-Butyl-2-(5-(pyridin-4-ylethinyl)thiophen-2-yl)imidazo[1,2-a]pyrazin-3-amin Hydrochlorid |
| 128 | 2-(5-((6-Aminopyridin-3-yl)ethinyl)thiophen-2-yl)-N-tert-butylimidazo[1,2-a]pyrazin-3-amin Hydrochlorid |
| 129 | N-tert-Butyl-2-(5-(pyrimidin-2-ylethinyl)thiophen-2-yl)imidazo[1,2-a]pyrazin-3-amin Hydrochlorid |
| 130 | N-tert-Butyl-2-(5-((4-methylpyridin-2-yl)ethinyl)thiophen-2-yl)imidazo[1,2-a]pyrazin-3-amin Hydrochlorid |
| 131 | N-tert-Butyl-2-(5-((5-methylpyridin-2-yl)ethinyl)thiophen-2-yl)imidazo[1,2-a]pyrazin-3-amin Hydrochlorid |
| 132 | N-tert-Butyl-2-(5-(pyridin-4-ylethinyl)thiophen-2-yl)imidazo[1,2-a]pyridin-3-amin Hydrochlorid |
| 133 | N-tert-Butyl-2-(5-(thiazol-2-ylethinyl)thiophen-2-yl)imidazo[1,2-a]pyrazin-3-amin |
| 134 | 2-(5-(Pyridin-2-ylethinyl)thiophen-2-yl)imidazo[1,2-a]pyrazin-3-amin Hydrochlorid |
| 135 | N-tert-Butyl-2-(5-((5-methylthiophen-2-yl)ethinyl)thiophen-2-yl)imidazo[1,2-a]pyrazin-3-amin Hydrochlorid |
| 136 | 2-(5-((6-Aminopyridin-2-yl)ethinyl)thiophen-2-yl)-N-tert-butylimidazo[1,2-a]pyrazin-3-amin Hydrochlorid |
| 137 | N-tert-Butyl-2-(5-((3-methylthiophen-2-yl)ethinyl)thiophen-2-yl)imidazo[1,2-a]pyrazin-3-amin |
| 138 | N-tert-Butyl-2-(4-(phenylethinyl)thiazol-2-yl)imidazo[1,2-a]pyrazin-3-amin |
| 139 | N-tert-Butyl-2-(5-(m-tolylethinyl)thiophen-2-yl)imidazo[1,2-a]pyrazin-3-amin |
| 140 | 3-((5-(3-(tert-Butylamino)imidazo[1,2-a]pyrazin-2-yl)thiophen-2-yl)ethinyl)benzonitril Hydrochlorid |
| 141 | N-tert-Butyl-2-(5-(pyridin-2-ylethinyl)thiophen-2-yl)imidazo[1,2-a]pyrimidin-3-amin |
| 142 | N-tert-Butyl-2-(6-(phenylethinyl)pyridin-2-yl)imidazo[1,2-a]pyrazin-3-amin Hydrochlorid |
| 143 | N-tert-Butyl-N-methyl-2-(5-(pyridin-2-ylethinyl)thiophen-2-yl)imidazo[1,2-a]pyrazin-3-amin |
| 144 | 4-((5-(3-(tert-Butylamino)imidazo[1,2-a]pyrazin-2-yl)thiophen-2-yl)ethinyl)benzonitril Hydrochlorid |
| 145 | 2-(5-((1H-Indol-6-yl)ethinyl)thiophen-2-yl)-N-tert-butylimidazo[1,2-a]pyrazin-3-amin Hydrochlorid |
| 146 | N-tert-Butyl-2-(2-(phenylethinyl)thiazol-5-yl)imidazo[1,2-a]pyrazin-3-amin |
| 147 | N-tert-Butyl-2-(5-(chinolin-6-ylethinyl)thiophen-2-yl)imidazo[1,2-a]pyrazin-3-amin Hydrochlorid |
| 148 | 2-(5-((3-(1H-Pyrrol-1-yl)phenyl)ethinyl)thiophen-2-yl)-N-tert-butylimidazo[1,2-a]pyrazin-3-amin Hydrochlorid |
| 149 | 2-(5-((1H-Indol-4-yl)ethinyl)thiophen-2-yl)-N-tert-butylimidazo[1,2-a]pyrazin-3-amin Hydrochlorid |
| 150 | N-tert-Butyl-2-(5-((3-nitrophenyl)ethinyl)thiophen-2-yl)imidazo[1,2-a]pyrazin-3-amin Hydrochlorid |
| 151 | N-tert-Butyl-2-(5-((4-nitrophenyl)ethinyl)thiophen-2-yl)imidazo[1,2-a]pyrazin-3-amin Hydrochlorid |
| 152 | N-tert-Butyl-2-(5-(thiazol-4-ylethinyl)thiophen-2-yl)imidazo[1,2-a]pyrazin-3-amin Hydrochlorid |
| 153 | 2-((5-(3-(tert-Butylamino)imidazo[1,2-a]pyrazin-2-yl)thiophen-2-yl)ethinyl)phenol |
| 154 | 2-(5-((3-(Aminomethyl)phenyl)ethinyl)thiophen-2-yl)-N-tert-butylimidazo[1,2-a]pyrazin-3-amin |
| 155 | 2-(5-(Biphenyl-3-ylethinyl)thiophen-2-yl)-N-tert-butylimidazo[1,2-a]pyrazin-3-amin Hydrochlorid |
| 156 | N-tert-Butyl-2-(5-(thiophen-3-ylethinyl)thiophen-2-yl)imidazo[1,2-a]pyrazin-3-amin Hydrochlorid |
| 157 | N-tert-Butyl-2-(5-((3-(dimethylamino)phenyl)ethinyl)thiophen-2-yl)imidazo[1,2-a]pyrazin-3-amin Hydrochlorid |
| 158 | N-tert-Butyl-2-(5-((6-methylpyridin-2-yl)ethinyl)thiophen-2-yl)imidazo[1,2-a]pyrazin-3-amin Hydrochlorid |
| 159 | N-tert-Butyl-2-(5-((3-fluorpyridin-2-yl)ethinyl)thiophen-2-yl)imidazo[1,2-a]pyrazin-3-amin Hydrochlorid |
| 160 | N-tert-Butyl-2-(5-((3-(methylamino)phenyl)ethinyl)thiophen-2-yl)imidazo[1,2-a]pyrazin-3-amin Hydrochlorid |
| 161 | N-tert-Butyl-2-(5-(p-tolylethinyl)thiophen-2-yl)imidazo[1,2-a]pyrazin-3-amin |
| 162 | N-tert-Butyl-2-(5-(o-tolylethinyl)thiophen-2-yl)imidazo[1,2-a]pyrazin-3-amin Hydrochlorid |
| 163 | N-tert-Butyl-2-(4-methyl-5-(phenylethinyl)thiophen-2-yl)imidazo[1,2-a]pyrazin-3-amin Hydrochlorid |
| 164 | N-tert-Butyl-2-(4-methyl-5-(pyridin-2-ylethinyl)thiophen-2-yl)imidazo[1,2-a]pyrazin-3-amin Hydrochlorid |
| 165 | N-tert-Butyl-2-(5-((6-fluorpyridin-2-yl)ethinyl)thiophen-2-yl)imidazo[1,2-a]pyrazin-3-amin |
| 166 | N-tert-Butyl-2-(5-((2-nitrophenyl)ethinyl)thiophen-2-yl)imidazo[1,2-a]pyrazin-3-amin |
| 167 | N-tert-Butyl-8-chlor-2-(5-(pyridin-2-ylethinyl)thiophen-2-yl)imidazo[1,2-a]pyrazin-3-amin |
| 168 | N-tert-Butyl-2-(5-((6-methoxypyridin-2-yl)ethinyl)thiophen-2-yl)imidazo[1,2-a]pyrazin-3-amin |
| 169 | N-tert-Butyl-2-(5-((5-fluorpyridin-2-yl)ethinyl)thiophen-2-yl)imidazo[1,2-a]pyrazin-3-amin Hydrochlorid |
| 170 | 2-(4-(3-(tert-Butylamino)imidazo[1,2-a]pyrazin-2-yl)-2-(phenylethinyl)phenyl)acetonitril |
| 171 | N-tert-Butyl-2-(5-((5-methoxypyridin-3-yl)ethinyl)thiophen-2-yl)imidazo[1,2-a]pyrazin-3-amin Hydrochlorid |
| 172 | 5-((5-(3-(tert-Butylamino)imidazo[1,2-a]pyrazin-2-yl)thiophen-2-yl)ethinyl)nicotinonitril Hydrochlorid |
| 173 | N-tert-Butyl-2-(5-((3-(methylthio)phenyl)ethinyl)thiophen-2-yl)imidazo[1,2-a]pyrazin-3-amin |
| 174 | Methyl-3-((5-(3-(tert-Butylamino)imidazo[1,2-a]pyrazin-2-yl)thiophen-2-yl)ethinyl)benzoate |
| 175 | N-tert-Butyl-2-(5-((3,5-difluorpyridin-2-yl)ethinyl)thiophen-2-yl)imidazo[1,2-a]pyrazin-3-amin |
| 176 | N-tert-Butyl-2-(5-(phenylethinyl)thiazol-2-yl)imidazo[1,2-a]pyrazin-3-amin |
| 177 | N-tert-Butyl-2-(2-(pyridin-4-ylethinyl)thiazol-5-yl)imidazo[1,2-a]pyrazin-3-amin |
| 178 | 3-((5-(3-(tert-Butylamino)imidazo[1,2-a]pyrazin-2-yl)thiophen-2-yl)ethinyl)benzaldehyd |
| 179 | 3-((5-(3-(tert-Butylamino)imidazo[1,2-a]pyrazin-2-yl)thiophen-2-yl)ethinyl)-4-fluorbenzonitril |
| 180 | N-tert-Butyl-2-(5-((3-(trifluormethyl)phenyl)ethinyl)thiophen-2-yl)imidazo[1,2-a]pyrazin-3-amin Hydrochlorid |
| 181 | N-tert-Butyl-2-(2-(pyridin-2-ylethinyl)thiazol-5-yl)imidazo[1,2-a]pyrazin-3-amin |
| 182 | N-tert-Butyl-2-(3-methyl-5-(phenylethinyl)thiophen-2-yl)imidazo[1,2-alpyrazin-3-amin Hydrochlorid |
| 183 | N-tert-Butyl-2-(3-methyl-5-(pyridin-2-ylethinyl)thiophen-2-yl)imidazo[1,2-a]pyrazin-3-amin Hydrochlorid |
| 184 | N-tert-Butyl-2-(5-((3-vinylphenyl)ethinyl)thiophen-2-yl)imidazo[1,2-a]pyrazin-3-amin |
| 185 | 2-(5-((1H-Imidazol-4-yl)ethinyl)thiophen-2-yl)-N-tert-butylimidazo[1,2-a]pyrazin-3-amin Hydrochlorid |
| 186 | N-tert-Butyl-2-(5-((3-methylpyridin-2-yl)ethinyl)thiophen-2-yl)imidazo[1,2-a]pyrazin-3-amin |
| 187 | N,N-Dimethyl-2-(5-(pyridin-2-ylethinyl)thiophen-2-yl)imidazo[1,2-a]pyrazin-3-amin |
| 188 | N-tert-Butyl-2-(5-((2-(tert-butyldiphenylsilyl)thiazol-5-yl)ethinyl)thiophen-2-yl)imidazo[1,2-a]pyridin-3-amin |
| 189 | 3-((5-(3-(tert-Butylamino)imidazo[1,2-a]pyridin-2-yl)thiophen-2-yl)ethinyl)benzonitril |
| 190 | N-tert-Butyl-2-(5-(phenylethinyl)thiazol-2-yl)imidazo[1,2-a]pyridin-3-amin |
| 191 | N-tert-Butyl-2-(5-(thiazol-5-ylethinyl)thiophen-2-yl)imidazo[1,2-a]pyridin-3-amin |
| 192 | N-tert-Butyl-2-(5-(pyridin-2-ylethinyl)thiophen-2-yl)imidazo[1,2-a]pyridin-3-amin Hydrochlorid |
| 193 | 6-Chlor-N-cyclohexyl-2-(3-((6-methylpyridin-2-yl)ethinyl)phenyl)imidazo[1,2-a]pyridin-3-amin |
| 194 | 5,7-Dimethyl-N-phenethyl-2-(5-(phenylethinyl)thiophen-2-yl)imidazo[1,2-a]pyrimidin-3-amin |
| 195 | N-(3-Methoxyphenethyl)-5,7-dimethyl-2-(5-(phenylethinyl)thiophen-2-yl)imidazo[1,2-a]pyrimidin-3-amin |
| 196 | N-(3-Methoxyphenethyl)-5,7-dimethyl-2-(4-(phenylethinyl)thiophen-2-yl)imidazo[1,2-a]pyrimidin-3-amin |
| 197 | N-(3-Methoxyphenethyl)-5,7-dimethyl-2-(5-(phenylethinyl)furan-2-yl)imidazo[1,2-a]pyrimidin-3-amin |
| 198 | N-(4-Chlorbenzyl)-8-methyl-2-(5-(phenylethinyl)thiophen-2-yl)imidazo[1,2-a]pyridin-3-amin |
| 199 | N-(3-Methoxyphenethyl)-5-methyl-2-(4-(phenylethinyl)thiophen-2-yl)imidazo[1,2-a]pyridin-3-amin |
| 200 | N-(2-Methylhexan-2-yl)-2-(4-(phenylethinyl)thiophen-2-yl)imidazo[1,2-a]pyrazin-3-amin |
| 201 | N-Phenethyl-2-(4-(phenylethinyl)thiophen-2-yl)imidazo[1,2-a]pyrazin-3-amin |
| 202 | N-(3-Methoxyphenethyl)-2-(4-(phenylethinyl)thiophen-2-yl)imidazo[1,2-a]pyrazin-3-amin |
| 203 | 2-(4-(Phenylethinyl)thiophen-2-yl)-N-(2-(thiophen-2-yl)ethyl)imidazo[1,2-a]pyrazin-3-amin |
| 204 | N-(4-Chlorbenzyl)-2-(5-(phenylethinyl)thiophen-2-yl)imidazo[1,2-a]pyridin-3-amin |
| 205 | N-(2-Methylpentan-2-yl)-2-(5-(phenylethinyl)thiophen-2-yl)imidazo[1,2-a]pyrazin-3-amin |
| 206 | N-(Cyclohexylmethyl)-2-(4-(phenylethinyl)thiophen-2-yl)imidazo[1,2-a]pyrazin-3-amin |
| 207 | N-(2-Methoxybenzyl)-2-(5-(phenylethinyl)furan-2-yl)imidazo[1,2-a]pyrazin-3-amin |
| 208 | N-(Cyclohexylmethyl)-2-(4-(phenylethinyl)thiophen-2-yl)imidazo[1,2-a]pyridin-3-amin |
| 209 | N-(2-Methylpentan-2-yl)-2-(4-(phenylethinyl)thiophen-2-yl)imidazo[1,2-a]pyridin-3-amin |
| 210 | 8-Brom-6-methyl-2-(3-((6-methylpyridin-2-yl)ethinyl)phenyl)-N-(2,4,4-trimethylpentan-2-yl)imidazo[1,2-a]pyridin-3-amin |
| 211 | 8-Brom-N-cyclopentyl-6-methyl-2-(3-(pyridin-2-ylethinyl)phenyl)imidazo[1,2-a]pyridin-3-amin |
| 212 | N-Cyclopentyl-2-(4-(phenylethinyl)thiophen-2-yl)imidazo[1,2-a]pyrazin-3-amin |
| 213 | N-(1 -Phenylethyl)-2-(4-(phenylethinyl)thiophen-2-yl)imidazo[1,2-a]pyrazin-3-amin |
| 214 | N-(2-Methylpentan-2-yl)-2-(4-(phenylethinyl)thiophen-2-yl)imidazo[1,2-a]pyrazin-3-amin |
| 215 | 8-Brom-N-cyclohexyl-6-methyl-2-(5-(pyridin-2-ylethinyl)furan-2-yl)imidazo[1,2-a]pyridin-3-amin |
| 216 | N-Cyclopentyl-2-(5-(phenylethinyl)furan-2-yl)imidazo[1,2-a]pyrazin-3-amin |
| 217 | N-(3-Methoxyphenethyl)-7-methyl-2-(4-(phenylethinyl)thiophen-2-yl)imidazo[1,2-a]pyrimidin-3-amin |
| 218 | 8-(Benzyloxy)-2-(5-(phenylethinyl)thiophen-2-yl)-N-(2,4,4-trimethylpentan-2-yl)imidazo[1,2-a]pyridin-3-amin |
| 219 | 8-(Benzyloxy)-N-cyclopentyl-2-(4-(phenylethinyl)thiophen-2-yl)imidazo[1,2-a]pyridin-3-amin |
| 220 | 8-(Benzyloxy)-N-(2-methylpentan-2-yl)-2-(4-(phenylethinyl)thiophen-2-yl)imidazo[1,2-a]pyridin-3-amin |
| 221 | 6-Chlor-N-(4-fluorbenzyl)-2-(5-(phenylethinyl)furan-2-yl)imidazo[1,2-a]pyridin-3-amin |
| 222 | 6-Brom-N-butyl-5-methyl-2-(4-(phenylethinyl)thiophen-2-yl)imidazo[1,2-a]pyridin-3-amin |
| 223 | N-(Furan-2-yl)-8-methyl-2-(5-(phenylethinyl)furan-2-yl)imidazo[1,2-a]pyridin-3-amin |
| 224 | N-(Furan-2-yl)-2-(5-(phenylethinyl)furan-2-yl)imidazo[1,2-a]pyridin-3-amin |
| 225 | N-(Furan-2-yl)-2-(5-(phenylethinyl)furan-2-yl)-7-propylimidazo[1,2-a]pyridin-3-amin |
| 226 | 5,7-Dimethyl-2-(4-(phenylethinyl)thiophen-2-yl)-N-(3-(trifluormethyl)phenyl)imidazo[1,2-a]pyrimidin-3-amin |
| 227 | 6-Brom-N-(4-chlorphenethyl)-8-methyl-2-(4-(phenylethinyl)thiophen-2-yl)imidazo[1,2-a]pyridin-3-amin |
| 228 | N-(4-Chlorphenethyl)-7-phenyl-2-(4-(phenylethinyl)thiophen-2-yl)imidazo[1,2-a]pyridin-3-amin |
| 229 | N-Phenethyl-7-phenyl-2-(4-(phenylethinyl)thiophen-2-yl)imidazo[1,2-a]pyridin-3-amin |
| 230 | N-(4-Chlorbenzyl)-5,7-dimethyl-2-(4-(phenylethinyl)thiophen-2-yl)imidazo[1,2-a]pyridin-3-amin |
| 231 | 6-Brom-N-(4-chlorbenzyl)-5-methyl-2-(4-(phenylethinyl)thiophen-2-yl)imidazo[1,2-a]pyridin-3-amin |
| 232 | 8-Brom-N-(4-chlorbenzyl)-6-methyl-2-(4-(phenylethinyl)thiophen-2-yl)imidazo[1,2-a]pyridin-3-amin |
| 233 | N-(3-Methoxyphenethyl)-2-(4-(phenylethinyl)thiophen-2-yl)-5-propylimidazo[1,2-a]pyridin-3-amin |
| 234 | 6-Brom-8-methyl-2-(4-(phenylethinyl)thiophen-2-yl)-N-(2-(thiophen-2-yl)ethyl)imidazo[1,2-a]pyridin-3-amin |
| 235 | 7-Phenyl-2-(4-(phenylethinyl)thiophen-2-yl)-N-(2-(thiophen-2-yl)ethyl)imidazo[1,2-a]pyridin-3-amin |
| 236 | 6,8-Dibrom-N-(2-methylpentan-2-yl)-2-(5-(phenylethinyl)thiophen-2-yl)imidazo[1,2-a]pyridin-3-amin |
| 237 | 6-Brom-N-(2,6-dimethylphenyl)-8-methyl-2-(4-(phenylethinyl)thiophen-2-yl)imidazo[1,2-a]pyridin-3-amin |
| 238 | N-Cyclohexyl-2-(3-(pyridin-2-ylethinyl)phenyl)imidazo[1,2-a]pyrazin-3-amin |
| 239 | 2-(3-(Pyridin-2-ylethinyl)phenyl)-N-(2,4,4-trimethylpentan-2-yl)imidazo[1,2-a]pyrazin-3-amin |
| 240 | N-Cyclopentyl-2-(4-(pyridin-2-ylethinyl)phenyl)imidazo[1,2-a]pyrazin-3-amin |
| 241 | 8-Ch lor-2-(4-(pyridin-2-ylethinyl)phenyl)-6-(trifluormethyl)-N-(2,4,4-trimethylpentan-2-yl)imidazo[1,2-a]pyridin-3-amin |
| 242 | N-(4-Fluorphenyl)-2-(4-(pyridin-2-ylethinyl)phenyl)imidazo[1,2-a]pyrazin-3-amin |
| 243 | N-Cyclopentyl-2-(2-methyl-6-(phenylethinyl)pyridin-3-yl)imidazo[1,2-a]pyrazin-3-amin |
| 244 | N-Cyclohexyl-2-(2-methy)-6-(phenylethinyl)pyridin-3-yl)imidazo[1,2-a]pyrazin-3-amin |
| 245 | N-Cyclopentyl-2-(5-(pyridin-2-ylethinyl)furan-2-yl)imidazo[1,2-a]pyrazin-3-amin |
| 246 | 8-Brom-N-cyclopentyl-6-methyl-2-(5-(pyridin-2-ylethinyl)furan-2-yl)imidazo[1,2-a]pyridin-3-amin |
| 247 | N-Cyclohexyl-2-(5-(pyridin-2-ylethinyl)furan-2-yl)imidazo[1,2-a]pyrazin-3-amin |
| 248 | 2-(5-(Pyridin-2-ylethinyl)furan-2-yl)-N-(2,4,4-trimethylpentan-2-yl)imidazo[1,2-a]pyrazin-3-amin |
| 249 | N-Cyclopentyl-2-(3-((6-methylpyridin-2-yl)ethinyl)phenyl)imidazo[1,2-a]pyrazin-3-amin |
| 250 | 8-Brom-N-cyclopentyl-6-methyl-2-(4-(pyridin-2-ylethinyl)phenyl)imidazo[1,2-a]pyridin-3-amin |
| 251 | N-Cyclohexyl-2-(3-((6-methylpyridin-2-yl)ethinyl)phenyl)imidazo[1,2-a]pyrazin-3-amin |
| 252 | N-(4-Fluorphenyl)-2-(3-((6-methylpyridin-2-yl)ethinyl)phenyl)imidazo[1,2-a]pyrazin-3-amin |
| 253 | 2-(5-(Pyridin-2-ylethinyl)thiophen-2-yl)-N-(2,4,4-trimethylpentan-2-yl)imidazol[1,2-a]pyrimidin-3-amin |
| 254 | N-Cyclohexyl-2-(3-(pyridin-2-ylethinyl)phenyl)imidazo[1,2-a]pyrimidin-3-amin |
| 255 | 2-(3-(Pyridin-2-ylethinyl)phenyl)-N-(2,4,4-trimethylpentan-2-yl)imidazo[1,2-a]pyrimidin-3-amin |
| 256 | 2-(6-(Phenylethinyl)pyridin-3-yl)-N-(2,4,4-trimethylpentan-2-yl)imidazo[1,2-a]pyrimidin-3-amin |
| 257 | N-Cyclopentyl-2-(5-(pyridin-2-ylethinyl)furan-2-yl)imidazo[1,2-a]pyrimidin-3-amin |
| 258 | N-tert-Butyl-2-(5-(pyridin-2-ylethinyl)furan-2-yl)imidazo[1,2-a]pyrimidin-3-amin |
| 259 | N-Cyclopentyl-2-(3-((6-methylpyridin-2-yl)ethinyl)phenyl)imidazo[1,2-a]pyrimidin-3-amin |
| 260 | N-Cyclopentyl-2-(3-(pyridin-2-ylethinyl)phenyl)imidazo[1,2-a]pyridin-3-amin |
| 261 | N-tert-Butyl-2-(3-(pyridin-2-ylethinyl)phenyl)imidazo[1,2-a]pyridin-3-amin |
| 262 | 2-(3-(Pyridin-2-ylethinyl)phenyl)-N-(2,4,4-trimethylpentan-2-yl)imidazo[1,2-a]pyridin-3-amin |
| 263 | N-(4-Fluorphenyl)-2-(3-(pyridin-2-ylethinyl)phenyl)imidazo[1,2-a]pyridin-3-amin |
| 264 | N-tert-Butyl-2-(4-(pyridin-2-ylethinyl)phenyl)imidazo[1,2-a]pyridin-3-amin |
| 265 | N-Cyclopentyl-2-(2-methyl-6-(phenylethinyl)pyridin-3-yl)imidazo[1,2-a]pyridin-3-amin |
| 266 | N-(4-Fluorphenyl)-2-(2-methyl-6-(phenylethinyl)pyridin-3-yl)imidazo[1,2-a]pyridin-3-amin |
| 267 | N-Cyclopentyl-6-methyl-2-(5-(pyridin-2-ylethinyl)thiophen-2-yl)imidazo[1,2-a]pyridin-3-amin |
| 268 | N-tert-Butyl-6-methyl-2-(5-(pyridin-2-ylethinyl)thiophen-2-yl)imidazo[1,2-a]pyridin-3-amin |
| 269 | N-Cyclohexyl-6-methyl-2-(5-(pyridin-2-ylethinyl)thiophen-2-yl)imidazo[1,2-a]pyridin-3-amin |
| 270 | 6-Methyl-2-(5-(pyridin-2-ylethinyl)thiophen-2-yl)-N-(2,4,4-trimethylpentan-2-yl)imidazo[1,2-a]pyridin-3-amin |
| 271 | 6-Methyl-2-(2-methyl-6-(phenylethinyl)pyridin-3-yl)-N-(2,4,4-trimethylpentan-2-yl)imidazo[1,2-a]pyridin-3-amin |
| 272 | N-Cyclopentyl-6-methyl-2-(5-(pyridin-2-ylethinyl)furan-2-yl)imidazo[1,2-a]pyridin-3-amin |
| 273 | N-Cyclohexyl-6-methyl-2-(3-((6-methylpyridin-2-yl)ethinyl)phenyl)imidazo[1,2-a]pyridin-3-amin |
| 274 | N-Cyclohexyl-7-methyl-2-(5-(pyridin-2-ylethinyl)thiophen-2-yl)imidazo[1,2-a]pyridin-3-amin |
| 275 | 7-Methyl-2-(5-(pyridin-2-ylethinyl)thiophen-2-yl)-N-(2,4,4-trimethylpentan-2-yl)imidazo[1,2-a]pyridin-3-amin |
| 276 | N-tert-Butyl-7-methyl-2-(3-(pyridin-2-ylethinyl)phenyl)imidazo[1,2-a]pyridin-3-amin |
| 277 | N-(4-Fluorphenyl)-7-methyl-2-(3-(pyridin-2-ylethinyl)phenyl)imidazo[1,2-a]pyridin-3-amin |
| 278 | N-tert-Butyl-7-methyl-2-(4-(pyridin-2-ylethinyl)phenyl)imidazo[1,2-a]pyridin-3-amin |
| 279 | N-(4-Fluorphenyl)-7-methyl-2-(5-(pyridin-2-ylethinyl)furan-2-yl)imidazo[1,2-a]pyridin-3-amin |
| 280 | N-Cyclohexyl-8-methyl-2-(6-(phenylethinyl)pyridin-3-yl)imidazo[1,2-a]pyridin-3-amin |
| 281 | N-Cyclopentyl-2-(6-(phenylethinyl)pyridin-3-yl)imidazo[1,2-a]pyridin-3-amin |
| 282 | N-tert-Butyl-2-(6-(phenylethinyl)pyridin-3-yl)imidazo[1,2-a]pyridin-3-amin |
| 283 | N-Cyclohexyl-2-(6-(phenylethinyl)pyridin-3-yl)imidazo[1,2-a]pyridin-3-amin |
| 284 | 2-(6-(Phenylethinyl)pyridin-3-yl)-N-(2,4,4-trimethylpentan-2-yl)imidazo[1,2-a]pyridin-3-amin |
| 285 | N-tert-Butyl-2-(5-(pyridin-2-ylethinyl)furan-2-yl)imidazo[1,2-a]pyridin-3-amin |
| 286 | N-Cyclohexyl-2-(5-(pyridin-2-ylethinyl)furan-2-yl)imidazo[1,2-a]pyridin-3-amin |
| 287 | N-(4-Fluorphenyl)-2-(5-(pyridin-2-ylethinyl)furan-2-yl)imidazo[1,2-a]pyridin-3-amin |
| 288 | N-Cyclohexyl-2-(3-((6-methylpyridin-2-yl)ethinyl)phenyl)imidazo[1,2-a]pyridin-3-amin |
| 289 | 2-(3-((6-Methylpyridin-2-yl)ethinyl)phenyl)-N-(2,4,4-trimethylpentan-2-yl)imidazo[1,2-a]pyridin-3-amin |
| 290 | N-tert-Butyl-5-methyl-2-(3-((6-methylpyridin-2-yl)ethinyl)phenyl)imidazo[1,2-a]pyridin-3-amin |
| 291 | 5-Methyl-2-(3-((6-methylpyridin-2-yl)ethinyl)phenyl)-N-(2,4,4-trimethylpentan-2-yl)imidazo[1,2-a]pyridin-3-amin |
| 292 | N-Cyclohexyl-5,7-dimethyl-2-(3-(pyridin-2-ylethinyl)phenyl)imidazo[1,2-a]pyrimidin-3-amin |
| 293 | N-Cyclohexyl-5,7-dimethyl-2-(2-methyl-6-(phenylethinyl)pyridin-3-yl)imidazo[1,2-a]pyrimidin-3-amin |
| 294 | N-Cyclopentyl-5,7-dimethyl-2-(5-(pyridin-2-ylethinyl)furan-2-yl)imidazo[1,2-a]pyrimidin-3-amin |
| 295 | N-tert-Butyl-5,7-dimethyl-2-(5-(pyridin-2-ylethinyl)furan-2-yl)imidazo[1,2-a]pyrimidin-3-amin |
| 296 | N-(4-Fluorphenyl)-8-methyl-2-(3-(pyridin-2-ylethinyl)phenyl)imidazo[1,2-a]pyridin-3-amin |
| 297 | N-Cyclohexyl-8-methyl-2-(4-(pyridin-2-ylethinyl)phenyl)imidazo[1,2-a]pyridin-3-amin |
| 298 | N-Cyclohexyl-7-ethyl-2-(5-(pyridin-2-ylethinyl)thiophen-2-yl)imidazo[1,2-a]pyridin-3-amin |
| 299 | 7-Ethyl-2-(5-(pyridin-2-ylethinyl)thiophen-2-yl)-N-(2,4,4-trimethylpentan-2-yl)imidazo[1,2-a]pyridin-3-amin |
| 300 | N-Cyclohexyl-7-ethyl-2-(3-(pyridin-2-ylethinyl)phenyl)imidazo[1,2-a]pyridin-3-amin |
| 301 | N-Cyclopentyl-7-ethyl-2-(4-(pyridin-2-ylethinyl)phenyl)imidazo[1,2-a]pyridin-3-amin |
| 302 | N-Cyclopentyl-7-ethyl-2-(6-(phenylethinyl)pyridin-3-yl)imidazo[1,2-a]pyridin-3-amin |
| 303 | N-tert-Butyl-7-ethyl-2-(6-(phenylethinyl)pyridin-3-yl)imidazo[1,2-a]pyridin-3-amin |
| 304 | N-tert-Butyl-7-ethyl-2-(5-(pyridin-2-ylethinyl)furan-2-yl)imidazo[1,2-a]pyridin-3-amin |
| 305 | 7-Isopropyl-2-(5-(pyridin-2-ylethinyl)thiophen-2-yl)-N-(2,4,4-trimethylpentan-2-yl)imidazo[1,2-a]pyridin-3-amin |
| 306 | N-tert-Butyl-7-isopropyl-2-(3-(pyridin-2-ylethinyl)phenyl)imidazo[1,2-a]pyridin-3-amin |
| 307 | N-tert-Butyl-7-isopropyl-2-(4-(pyridin-2-ylethinyl)phenyl)imidazo[1,2-a]pyridin-3-amin |
| 308 | N-Cyclohexyl-7-isopropyl-2-(5-(pyridin-2-ylethinyl)furan-2-yl)imidazo[1,2-a]pyridin-3-amin |
| 309 | N-tert-Butyl-7-isopropyl-2-(3-((6-methylpyridin-2-yl)ethinyl)phenyl)imidazo[1,2-a]pyridin-3-amin |
| 310 | 6-Chlor-N-cyclopentyl-2-(5-(pyridin-2-ylethinyl)thiophen-2-yl)imidazo[1,2-a]pyridin-3-amin |
| 311 | N-tert-Butyl-6-chlor-2-(5-(pyridin-2-ylethinyl)thiophen-2-yl)imidazo[1,2-a]pyridin-3-amin |
| 312 | 6-Chlor-N-cyclohexyl-2-(3-(pyridin-2-ylethinyl)phenyl)imidazo[1,2-a]pyridin-3-amin |
| 313 | 6-Chlor-2-(3-(pyridin-2-ylethinyl)phenyl)-N-(2,4,4-trimethylpentan-2-y)imidazo[1,2-a]pyridin-3-amin |
| 314 | N-tert-Butyl-6-chlor-2-(4-(pyridin-2-ylethinyl)phenyl)imidazo[1,2-a]pyridin-3-amin |
| 315 | 6-Chlor-N-cyclohexyl-2-(4-(pyridin-2-ylethinyl)phenyl)imidazo[1,2-a]pyridin-3-amin |
| 316 | N-tert-Butyl-6-chlor-2-(5-(pyridin-2-ylethinyl)furan-2-yl)imidazo[1,2-a]pyridin-3-amin |
| 317 | 6-Chlor-N-cyclohexyl-2-(5-(pyridin-2-ylethinyl)furan-2-yl)imidazo[1,2-a]pyridin-3-amin |
| 318 | 6-Chlor-2-(5-(pyridin-2-ylethinyl)furan-2-yl)-N-(2,4,4-trimethylpentan-2-yl)imidazo[1,2-a]pyridin-3-amin |
| 319 | 6-Chlor-N-cyclopentyl-2-(3-((6-methylpyridin-2-yl)ethinyl)phenyl)imidazo[1,2-a]pyridin-3-amin |
| 320 | N-tert-Butyl-6-chlor-2-(3-((6-methylpyridin-2-yl)ethinyl)phenyl)imidazo[1,2-a]pyridin-3-amin |
| 321 | N-tert-Butyl-2-(5-(pyridin-2-ylethinyl)thiophen-2-yl)imidazo[1,2-a]pyridin-3-amin Bis-Hydrochlorid |
| 322 | N-Methyl-2-(5-(pyridin-2-ylethinyl)thiophen-2-yl)imidazo[1,2-a]pyrazin-3-amin |
| 323 | N-tert-Butyl-2-(5-(pyridin-2-ylethinyl)thiophen-2-yl)imidazo[1,2-a]pyridin-3-amin Bis-Hydrochlorid |
| 324 | [2-(5-Pyridin-2-ylethinyl-thiophen-2-yl)-imidazo[1,2-a]pyridin-3-yl]-(1,1,3,3-tetramethyl-butyl)-amin |
| 325 | tert-Butyl-[2-(5-pyrimidin-2-ylethinyl-thiophen-2-yl)-imidazo[1,2-a]pyridin-3-yl]-amin Hydrochlorid |
| 326 | {2-[5-(3-Amino-pyridin-2-ylethinyl)-thiophen-2-yl]-imidazo[1,2-a]pyridin-3-yl}-tert-butyl-amin |
| 327 | tert-Butyl-[2-(5-pyridin-2-ylethinyl-thiazol-2-yl)-imidazo[1,2-a]pyridin-3-yl]-amin |
| 328 | tert-Butyl-[2-(2-pyridin-2-ylethinyl-thiazol-5-yl)-imidazo[1,2-a]pyridin-3-yl]-amin |
| 329 | tert-Butyl-{2-[5-(6-fluor-pyridin-2-ylethinyl)-thiophen-2-yl]-imidazo[1,2-a]pyridin-3-yl}-amin Hydrochlorid |
| 330 | tert-Butyl-{2-[5-(3-chlor-5-fluor-phenylethinyl)-thiophen-2-yl]-imidazo[1,2-a]pyridin-3-yl}-amin Hydrochlorid |
| 331 | tert-Butyl-[2-(5-pyridin-2-ylethinyl-thiophen-2-yl)-imidazo[1,2-b]pyridazin-3-yl]-amin Hydrochlorid |
| 332 | tert-Butyl-[2-(5-thiazol-2-ylethinyl-thiophen-2-yl)-imidazo[1,2-a]pyridin-3-yl]-amin Hydrochlorid |
| 333 | tert-Butyl-{2-[5-(3-trifluormethoxy-phenylethinyl)-thiophen-2-yl]-imidazo[1,2-a]pyrazin-3-yl}-amin |
| 334 | tert-Butyl-{2-[5-(3-[1,3]dioxolan-2-yl-phenylethinyl)-thiophen-2-yl]-imidazol[1,2-a]pyridin-3-yl}-amin |
| 335 | tert-Butyl-{2-[5-(3,5-dimethyl-phenylethinyl)-thiophen-2-yl]-imidazo[1,2-a]pyridin-3-yl}-amin Hydrochlorid |
| 336 | tert-Butyl-{2-[5-(3-fluor-pyridin-2-ylethinyl)-thiophen-2-yl]-imidazo[1,2-a]pyridin-3-yl}-amin Hydrochlorid |
| 337 | tert-Butyl-{2-[5-(3-methyl-3H-imidazol-4-ylethinyl)-thiophen-2-yl]-imidazo[1,2-a]pyridin-3-yl}-amin Hydrochlorid |
| 338 | tert-Butyl-{2-[5-(5-chlor-thiophen-2-ylethinyl)-thiophen-2-yl]-imidazo[1,2-a]pyridin-3-yl}-amin Hydrochlorid |
| 339 | tert-Butyl-{2-[5-(5-methyl-pyridin-2-ylethinyl)-thiophen-2-yl]-imidazo[1,2-a]pyridin-3-yl}-amin Hydrochlorid |
| 340 | 1-{3-[5-(3-tert-Butylamino-imidazo[1,2-a]pyridin-2-yl)-thiophen-2-ylethinyl]-phenyl}-ethanon Hydrochlorid |
| 341 | {3-[5-(3-tert-Butylamino-imidazo[1,2-a]pyridin-2-yl)-thiophen-2-ylethinyl]-phenyl}-methanol Hydrochlorid |
| 342 | N-tert-Butyl-2-(5-((3-methoxypyridin-2-yl)ethinyl)thiophen-2-yl)imidazo[1,2-a]pyridin-3-amin |
| 343 | N-tert-Butyl-2-(5-(thiophen-2-ylethinyl)thiophen-2-yl)imidazo[1,2-a]pyridin-3-amin Hydrochlorid |
| 344 | 5-((5-(3-(tert-Butylamino)imidazo[1,2-a]pyridin-2-yl)thiophen-2-yl)ethinyl)-2-fluorbenzonitril Hydrochlorid |
| 345 | N-tert-Butyl-2-(5-((3,4-difluorphenyl)ethinyl)thiophen-2-yl)imidazo[1,2-a]pyridin-3-amin |
| 346 | N-tert-Butyl-2-(5-((3-(methoxymethyl)phenyl)ethinyl)thiophen-2-yl)imidazo[1,2-a]pyridin-3-amin |
| 347 | 2-(5-((3-Aminophenyl)ethinyl)thiophen-2-yl)-N-tert-butylimidazo[1,2-a]pyridin-3-amin Hydrochlorid |
| 348 | N-tert-Butyl-2-(5-((4-fluor-3-methylphenyl)ethinyl)thiophen-2-yl)imidazo[1,2-a]pyridin-3-amin Hydrochlorid |
| 349 | N-tert-Butyl-2-(5-((3,5-difluorphenyl)ethinyl)thiophen-2-yl)imidazo[1,2-a]pyridin-3-amin |
| 350 | N-tert-Butyl-2-(5-(thiophen-3-ylethinyl)thiophen-2-yl)imidazo[1,2-a]pyridin-3-amin Hydrochlorid |
| 351 | N-tert-Butyl-2-(5-((3-methylpyridin-2-yl)ethinyl)thiophen-2-yl)imidazo[1,2-a]pyridin-3-amin Hydrochlorid |
| 352 | 3-((5-(3-(tert-Butylamino)imidazo[1,2-a]pyridin-2-yl)thiophen-2-yl)ethinyl)benzolsulfonamid Hydrochlorid |
| 353 | 3-((5-(3-(tert-Butylamino)imidazo[1,2-a]pyridin-2-yl)hiophen-2-yl)ethinyl)benzoesäure |
| 354 | 3-((5-(3-(tert-Butylamino)imidazo[1,2-a]pyridin-2-yl)thiophen-2-yl)ethinyl)benzamid Hydrochlorid |
| 355 | N-(3-((5-(3-(tert-Butylamino)imidazo[1,2-a]pyridin-2-yl)thiophen-2-yl)ethinyl)phenyl)acetamid |
| 356 | N-tert-Butyl-N-methyl-2-(5-(pyridin-2-ylethinyl)thiophen-2-yl)imidazo[1,2-a]pyridin-3-amin |
| 357 | N,N-Dimethyl-2-(5-(pyridin-2-ylethinyl)thiophen-2-yl)imidazo[1,2-a]pyridin-3-amin |
| 358 | N-tert-Butyl-N-methyl-2-(5-(pyridin-2-ylethinyl)thiazol-2-yl)imidazo[1,2-a]pyridin-3-amin |
| 359 | (6-((5-(3-(tert-Butylamino)imidazo[1,2-a]pyridin-2-yl)thiophen-2-yl)ethinyl)pyridin-2-yl)methanol |
| 360 | N-(3-((5-(3-(tert-Butylamino)imidazo(1,2-a]pyridin-2-yl)thiophen-2-yl)ethinyl)phenyl)methansulfonamid |
| 361 | N-tert-Butyl-8-methyl-2-(5-(pyridin-2-ylethinyl)thiophen-2-yl)imidazo[1,2-a]pyrazin-3-amin Hydrochlorid |
| 362 | 2-(5-(Pyridin-2-ylethinyl)thiophen-2-yl)imidazo[1,2-a]pyridin-3-amin |
| 363 | N-tert-Butyl-2-(5-((3-chlorphenyl)ethinyl)thiophen-2-yl)imidazo[1,2-a]pyridin-3-amin |
| 364 | N-tert-Butyl-2-(5-((2,3-difluorphenyl)ethinyl)thiophen-2-yl)imidazo[1,2-a]pyridin-3-amin |
| 365 | N-tert-Butyl-7-chlor-2-(5-(pyridin-2-ylethinyl)thiophen-2-yl)imidazo[1,2-a]pyridin-3-amin |

### Pharmakologische Daten:

1. Die Affinität der erfindungsgemäßen substituierten Imidazo-3-yl-amin-Verbindungen der allgemeinen Formel I für den mGluR5-Rezeptor wurde wie vorstehend beschrieben bestimmt.
Die erfindungsgemäßen substituierten Imidazo-3-yl-amin-Verbindungen zeigen eine ausgezeichnete Affinität für den mGluR5-Rezeptor.
In der nachfolgenden Tabelle 1 sind die pharmakologischen Daten für die substituierten Imidazo-3-yl-amin-Vebindungen gemäß den Beispielen 1 bis 332 wiedergegeben:
**Tabelle 1.**

| **Verbindung gemäß Beispiel** | **mGluR5-Rezeptor Hemmung der³ [H]-MPEP-Bindung [10µM] in % bei einer Konzentration der Beispielverbindungen von 10 µM** | **IC₅₀** |
|---|---|---|
| **1** | 52 | |
| **2** | 95 | |
| **3** | 95 | |
| **4** | 91 | |
| **5** | 100 | |
| **6** | 92 | |
| **7** | 89 | |
| **8** | 100 | |
| **9** | 98 | |
| **10** | 95 | |
| **11** | 82 | |
| **12** | 96 | |
| **13** | 96 | |
| **14** | 100 | |
| **15** | 98 | |
| **16** | 93 | |
| **17** | 96 | |
| **18** | | 0,0640 |
| **19** | 78 | |
| **20** | 79 | |
| **21** | 91 | |
| **22** | 84 | |
| **23** | 86 | |
| **24** | 94 | |
| **25** | 81 | |
| **26** | 97 | |
| **27** | 82 | |
| **28** | 98 | |
| **29** | 87 | |
| **30** | 73 | |
| **31** | | 0,5300 |
| **32** | 98 | |
| **33** | 98 | |
| **34** | 98 | |
| **35** | 95 | |
| **36** | 100 | |
| **37** | 85 | |
| **38** | 87 | |
| **39** | 72 | |
| **40** | 87 | |
| **41** | 73 | |
| **42** | 93 | |
| **43** | 83 | |
| **44** | 75 | |
| **45** | 93 | |
| **46** | 76 | |
| **47** | 79 | |
| **48** | 82 | |
| **49** | 85 | |
| **50** | 90 | |
| **51** | 89 | |
| **52** | 75 | |
| **53** | 80 | |
| **54** | 69 | |
| **55** | 77 | |
| **56** | 100 | |
| **57** | 94 | |
| **59** | | 2,0700 |
| **60** | | 1,2000 |
| **61** | | 0,0425 |
| **62** | 26 | |
| **63** | 65 | |
| **64** | | 0,0330 |
| **65** | | 3,8400 |
| **66** | | 3,1750 |
| **67** | | 3,8400 |
| **68** | | 2,3200 |
| **69** | | 2,9300 |
| **70** | | 1,0600 |
| **71** | | 1,2700 |
| **72** | | 5,3400 |
| **73** | | 2,2100 |
| **74** | | 1,1000 |
| **75** | | 1,2900 |
| **76** | | 0,0037 |
| **77** | | 0,0057 |
| **79** | 35 | |
| **82** | | 0,2000 |
| **83** | 35 | |
| **84** | | 1,3200 |
| **85** | | 2,0100 |
| **86** | | 0,3200 |
| **87** | 35 | |
| **88** | | 1,4000 |
| **89** | | 0,0450 |
| **91** | | 0,0054 |
| **92** | 82 | |
| **93** | | 0,2500 |
| **94** | | 0,0033 |
| **95** | | 1,7400 |
| **96** | 79 | |
| **97** | | 0,5900 |
| **98** | | 0,2500 |
| **99** | | 0,5400 |
| **100** | | 0,3400 |
| **101** | | 2,4700 |
| **102** | | 8,8500 |
| **103** | 66 | |
| **104** | | 0,0920 |
| **105** | | 0,1500 |
| **106** | | 0,2100 |
| **107** | | 0,0520 |
| **108** | | 1,2400 |
| **109** | | 0,3000 |
| **110** | | 0,0490 |
| **111** | | 1,3700 |
| **112** | | 7,5200 |
| **113** | | 0,0750 |
| **114** | | 2,1100 |
| **115** | | 7,7400 |
| **116** | | 0,0760 |
| **117** | | 0,8800 |
| **118** | | 6,3800 |
| **119** | | 0,0360 |
| **120** | | 3,6300 |
| **121** | | 2,5400 |
| **122** | | 0,1300 |
| **123** | | 0,0140 |
| **124** | | 0,1700 |
| **125** | | 6,5200 |
| **126** | | 3,2500 |
| **127** | | 0,0084 |
| **128** | | 15,5500 |
| **129** | | 0,0120 |
| **130** | | 0,0370 |
| **131** | | 0,1400 |
| **132** | | 0,0160 |
| **133** | | 0,0150 |
| **134** | | 0,5700 |
| **135** | | 1,2000 |
| **136** | | 13,7700 |
| **137** | | 0,8600 |
| **138** | | 15,1100 |
| **139** | | 0,0710 |
| **140** | | 0,0260 |
| **141** | | 0,0028 |
| **143** | | 0,0032 |
| **144** | | 3,6800 |
| **146** | | 0,0350 |
| **148** | | 0,3300 |
| **150** | | 0,1100 |
| **152** | | 0,0230 |
| **153** | | 2,9900 |
| **154** | | 0,5900 |
| **156** | | 0,0270 |
| **157** | | 1,5500 |
| **158** | | 0,0330 |
| **159** | | 0,0100 |
| **160** | | 0,0940 |
| **161** | | 7,7400 |
| **162** | | 4,2400 |
| **164** | | 0,0540 |
| **165** | | 0,0180 |
| **166** | | 11,3300 |
| **167** | | 0,0120 |
| **168** | | 0,6400 |
| **169** | | 0,0110 |
| **171** | | 0,3600 |
| **172** | | 0,0360 |
| **173** | | 0,8000 |
| **174** | | 0,8400 |
| **175** | | 0,0510 |
| **176** | | 0,0190 |
| **177** | | 0,0160 |
| **178** | | 0,0240 |
| **179** | | 0,0260 |
| **180** | | 0,5800 |
| **181** | | 0,0180 |
| **182** | | 8,8500 |
| **183** | | 0,2300 |
| **184** | | 0,2700 |
| **185** | | 0,1900 |
| **192** | | 0,0053 |
| **193** | 79 | |
| **194** | 30 | |
| **195** | 53 | |
| **196** | 68 | |
| **197** | 31 | |
| **198** | 67 | |
| **199** | 57 | |
| **200** | 62 | |
| **201** | 55 | |
| **202** | 70 | |
| **203** | 60 | |
| **204** | 30 | |
| **205** | 92 | |
| **206** | 63 | |
| **207** | 35 | |
| **208** | 57 | |
| **209** | 55 | |
| **210** | 68 | |
| **211** | 82 | |
| **212** | 90 | |
| **213** | 84 | |
| **214** | 77 | |
| **215** | 51 | |
| **216** | 49 | |
| **217** | 62 | |
| **218** | 38 | |
| **219** | 33 | |
| **220** | 31 | |
| **221** | 40 | |
| **222** | 33 | |
| **223** | 80 | |
| **224** | 69 | |
| **225** | 43 | |
| **226** | 41 | |
| **227** | 61 | |
| **228** | 43 | |
| **229** | 30 | |
| **230** | 60 | |
| **231** | 62 | |
| **232** | 75 | |
| **233** | 75 | |
| **234** | 64 | |
| **235** | 32 | |
| **236** | 66 | |
| **237** | 36 | |
| **238** | 92 | |
| **239** | 60 | |
| **240** | 95 | |
| **241** | 65 | |
| **242** | 91 | |
| **243** | 80 | |
| **244** | 54 | |
| **245** | 92 | |
| **246** | 74 | |
| **247** | 83 | |
| **248** | 68 | |
| **249** | 80 | |
| **250** | 69 | |
| **251** | 80 | |
| **252** | 86 | |
| **253** | 73 | |
| **254** | 77 | |
| **255** | 81 | |
| **256** | 43 | |
| **257** | 77 | |
| **258** | 86 | |
| **259** | 82 | |
| **260** | 64 | |
| **261** | 79 | |
| **262** | 72 | |
| **263** | 85 | |
| **264** | 83 | |
| **265** | 68 | |
| **266** | 63 | |
| **267** | 85 | |
| **268** | 75 | |
| **269** | 94 | |
| **270** | 93 | |
| **271** | 31 | |
| **272** | 72 | |
| **273** | 84 | |
| **274** | 85 | |
| **275** | 85 | |
| **276** | 93 | |
| **277** | 95 | |
| **278** | 93 | |
| **279** | 67 | |
| **280** | 31 | |
| **281** | 83 | |
| **282** | 72 | |
| **283** | 82 | |
| **284** | 68 | |
| **285** | 88 | |
| **286** | 72 | |
| **287** | 30 | |
| **288** | 89 | |
| **289** | 51 | |
| **290** | 88 | |
| **291** | 92 | |
| **292** | 88 | |
| **293** | 70 | |
| **294** | 79 | |
| **295** | 84 | |
| **296** | 85 | |
| **297** | 80 | |
| **298** | 95 | |
| **299** | 86 | |
| **300** | 50 | |
| **301** | 78 | |
| **302** | 55 | |
| **303** | 67 | |
| **304** | 94 | |
| **305** | 75 | |
| **306** | 76 | |
| **307** | 88 | |
| **308** | 70 | |
| **309** | 82 | |
| **310** | 86 | |
| **311** | 86 | |
| **312** | 64 | |
| **313** | 33 | |
| **314** | 79 | |
| **315** | 75 | |
| **316** | 55 | |
| **317** | 58 | |
| **318** | 43 | |
| **319** | 81 | |
| **320** | 77 | |
| **321** | | 0,0093 |
| **322** | | 0,1200 |
| **323** | | 0,1100 |
| **324** | | 0,0590 |
| **325** | | 0,0130 |
| **326** | | 0,0390 |
| **332** | | 0,0140 |
| **333** | | 0,4200 |
| **334** | | 10,0400 |
| **335** | | |
| **336** | | 0,0061 |
| **337** | | 6,2600 |
| **338** | 52 | |
| **339** | | 0,3200 |
| **340** | | 0,6200 |
| **341** | | 0,2400 |
| **342** | | 1,3600 |
| **343** | | 0,0930 |
| **344** | | 0,1700 |
| **345** | | |
| **346** | | 0,6700 |
| **347** | | 0,9200 |
| **348** | 44 | |
| **349** | 39 | |
| **350** | | 0,5700 |
| **351** | | 0,2300 |

2. Die untersuchte erfindungsgemäße Verbindung Beispiel 192 zeigte eine ausgeprägte, langanhaltende anti-allodynische Wirkung. Die Ergebnisse sind in der nachfolgenden Tabelle 2 zusammengefaßt.

**Tabelle 2: Prüfung der Hemmung im neuropathischen Schmerz an der Ratte, [% MPE, maximal possible effect, maximaler möglicher Effekt] Hemmung zu den einzelnen Meßpunkten und über die gesamte Meßzeit (AUC, area under the curve, Fläche unter der Kurve)**

| | | % MPE zum Zeitpunkt nach Gabe der Verbindungen | | | | | |
|---|---|---|---|---|---|---|---|
| Dosis (mg/kg) p.o. | | 15 Min | 30 Min | 45 Min | 60 Min | AUC (0-60min) | t-test |
| 0 | Mittelwert | 0.4 | 3.3 | 2.4 | 3.4 | n.a. | |
| | Standardfehler des Mittelwertes | 1.5 | 1.0 | 0.7 | 0.8 | n.a. | |
| 0.215 | Mittelwert | 27.1 | 19.9 | 9.3 | 5.4 | 15.0 | ** |
| | Standardfehler des Mittelwertes | 5.4 | 4.6 | 3.1 | 2.2 | 3.5 | |
| 0.464 | Mittelwert | 30.2 | 27.7 | 20.3 | 12.4 | 22.3 | *** |
| | Standardfehler des Mittelwertes | 4.8 | 4.2 | 4.2 | 4.5 | 3.0 | |
| 1 | Mittelwert | 48.7 | 50.9 | 39.4 | 31.2 | 42.9 | *** |
| | Standardfehler des Mittelwertes | 5.9 | 3.6 | 5.8 | 5.7 | 4.2 | |
| 2.15 | Mittelwert | 42.9 | 54.7 | 34.3 | 13.7 | 38.2 | *** |
| | Standardfehler des Mittelwertes | 7.8 | 7.0 | 6.3 | 4.7 | 3.9 | |
| 4.64 | Mittelwert | 46.2 | 72.1 | 39.9 | 23.9 | 47.4 | *** |
| | Standardfehler des Mittelwertes | 6.5 | 5.8 | 7.5 | 8.0 | 5.3 | |
| | | n.a. nicht anwendbar | | | | | |

3. Die erfindungsgemäße Verbindung Beispiel 192 wurde 60 Min nach p.o. Applikation getestet und produzierte einen dosisabhängigen anxiolytischen Effekt gekennzeichnet durch einen signifikanter Anstieg der Zeit in den offenen Armen. Die niedrigste aktive Dosis war 2.15 mg/kg, und es wurde ein ED₅₀ Wert von 2.75 (93% Konfidenz-Intervall 0.65-5.58) mg/kg bestimmt.
4. Die erfindungsgemäßen substituierten Imidazo-3-yl-amin-Verbindungen zeigen ebenfalls eine ausgezeichnete Wirkung im Formalin-Test an der Maus oder an der Ratte wie in der nachfolgenden Tabelle 3 wieder gegeben ist.

**Tabelle 3.**

| **ED₅₀ Wert [mg/kg] bzw. % Hemmung** | **Ratte** | | | **Maus** | |
|---|---|---|---|---|---|
| **Beispiel** | **i.v.** | **p.o** | **i.p** | **p.o.** | **i.p.** |
| 91 | - | 7.3 | 4.7 | 2.3 | 1.2 |
| 110 | - | 61 % bei 21.5mg/kg | - | - | - |
| 116 | - | - | - | - | 15.2 |
| 127 | - | 5.9 | 2.2 | - | 7.7 |
| 192 | 0.8 | 7.6 | 41% bei 4.64mg/kg | - | - |

## Patentansprüche

1. Substituierte bizyklische Imidazo-3-yl-amin-Verbindungen der allgemeinen Formel I, 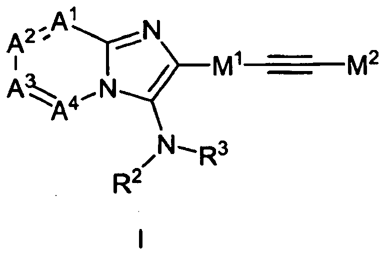 worin
A¹ für ein Stickstoffatom oder für eine C-R^{1a}-Gruppe steht,
A² für ein Stickstoffatom oder für eine C-R^{1b}-Gruppe steht,
A³ für ein Stickstoffatom oder für eine C-R^{1c}-Gruppe steht,
A⁴ für ein Stickstoffatom oder für eine C-R^{1d}-Gruppe steht,
R¹⁸, R^{1b}, R^{1c}, R^{1d}, unabhängig voneinander, jeweils für einen Wasserstoff-Rest; für einen Halogen-Rest; -NO₂; -CN; -NH₂; -NHR⁴; -NR⁵R⁶; -NH-C(=O)-R⁷; -C(=O)-R⁸, -C(=O)-NH₂; -C(=O)-NHR⁹; C(=O)-NR¹⁰R¹¹; -C(=O)-OR¹²; -(CH₂)ₘ-C(=O)OR¹³ mit m = 1, 2, 3, 4 oder 5; -O-C(=O)-R¹⁴; -(CH₂)ₙ-O-C(=O)-R¹⁵ mit n = 1, 2, 3, 4 oder 5; -OR¹⁶; -(CH₂)ₒ-O-R¹⁷ mit o = 1, 2, 3; 4 oder 5; -SR¹⁸; -(CH₂)ₚ-S(=O)ₜ-R¹⁹ mit p = 1, 2, 3, 4 oder 5 und t = 0, 1 oder 2; -NH-S(=O)₂-R²⁶R²⁷; -S(=O)₂-NR²⁸R²⁹,-SF₅; für einen linearen oder verzweigten, gesättigten oder ungesättigten, unsubstituierten oder wenigstens einfach substituierten aliphatischen Rest; für einen gesättigten oder ungesättigten, unsubstituierten oder wenigstens einfach substituierten, ggf. wenigstens ein Heteroatom als Ringglied aufweisenden cycloaliphatischen Rest, der über eine lineare oder verzweigte Alkylen-Gruppe gebunden und/oder mit einem unsubstituierten oder wenigstens einfach substituierten mono- oder polyzyklischen Ringsystem kondensiert sein kann; oder für einen unsubstituierten oder wenigstens einfach substituierten Aryl- oder Heteroaryl-Rest, der über eine lineare oder verzweigte Alkylen-Gruppe gebunden und/oder mit einem unsubstituierten oder wenigstens einfach substituierten mono- oder polyzyklischen Ringsystem kondensiert sein kann, stehen,
oder ggf. R^{1a} und R^{1b} mit der sie verbindenden C-C-Brücke einen unsubstituierten oder wenigstens einfach substituierten annelierten Phenyl-Rest bilden,
oder ggf. R^{1b} und R^{1c} mit der sie verbindenden C-C-Brücke einen unsubstituierten oder wenigstens einfach substituierten annelierten Phenyl-Rest bilden,
oder ggf. R^{1c} und R^{1d} mit der sie verbindenden C-C-Brücke einen unsubstituierten oder wenigstens einfach substituierten annelierten Phenyl-Rest bilden,
R² und R³, unabhängig voneinander, jeweils für einen Wasserstoff-Rest; -C(=O)-R²⁰; -(CH₂)_{q}-C(=O)-R²¹ mit q = 1, 2, 3, 4 oder 5; -C(=O)-O-R²²; - (CH₂)ᵣ-C(=O)-O-R²³ mit r = 1, 2, 3, 4 oder 5; -C(=O)-NHR²⁴; -(CH₂)ₛ-C(=O)-NHR²⁵ mit s = 1, 2, 3, 4 oder 5; für einen linearen oder verzweigten, gesättigten oder ungesättigten, unsubstituierten oder wenigstens einfach substituierten aliphatischen Rest; für einen gesättigten oder ungesättigten, unsubstituierten oder wenigstens einfach substituierten, ggf. wenigstens ein Heteroatom als Ringglied aufweisenden cycloaliphatischen Rest, der über eine lineare oder verzweigte Alkylen-Gruppe gebunden und/oder mit einem unsubstituierten oder wenigstens einfach substituierten mono- oder polyzyklischen Ringsystem kondensiert sein kann; oder für einen unsubstituierten oder wenigstens einfach substituierten Aryl- oder Heteroaryl-Rest, der über eine lineare oder verzweigte Alkylen-Gruppe gebunden und/oder mit einem unsubstituierten oder wenigstens einfach substituierten mono- oder polyzyklischen Ringsystem kondensiert sein kann, stehen,
oder R² und R³ zusammen mit dem sie verbindenden Stickstoffatom als Ringglied einen gesättigten oder ungesättigten, ggf. wenigstens ein weiteres Heteroatom als Ringglied aufweisenden heterocycloaliphatischen Rest bilden, der mit einem unsubstituierten oder wenigstens einfach substituierten mono- oder polyzyklischen Ringsystem kondensiert sein kann,
R⁴, R⁵, R⁶, R⁷, R⁹, R¹⁰, R¹¹, R¹⁴ und R¹⁵, jeweils unabhängig voneinander, für einen linearen oder verzweigten, gesättigten oder ungesättigten aliphatischen Rest oder für einen unsubstituierten oder wenigstens einfach substituierten Aryl- oder Heteroaryl-Rest, der über eine lineare oder verzweigte Alkylen-Gruppe gebunden und/oder mit einem unsubstituierten oder wenigstens einfach substituierten mono- oder polyzyklischen Ringsystem kondensiert sein kann, stehen,
R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ und R²⁹, jeweils unabhängig voneinander, für einen Wasserstoff-Rest; für einen linearen oder verzweigten, gesättigten oder ungesättigten aliphatischen Rest oder für einen unsubstituierten oder wenigstens einfach substituierten Aryl- oder Heteroaryl-Rest, der über eine lineare oder verzweigte Alkylen-Gruppe gebunden und/oder mit einem unsubstituierten oder wenigstens einfach substituierten mono- oder polyzyklischen Ringsystem kondensiert sein kann, stehen,
M¹ für einen Aryl- oder Heteroaryl-Rest steht, der mit wenigstens einem weiteren Substituenten substituiert und/oder mit einem unsubstituierten oder wenigstens einfach substituierten mono- oder polyzyklischen Ringsystem kondensiert sein kann,
M² für einen Aryl- oder Heteroaryl-Rest steht, der unsubstituiert oder wenigstens einfach substituiert und ggf. mit einem unsubstituierten oder wenigstens einfach substituierten mono- oder polyzyklischen Ringsystem kondensiert ist,
wobei
sofern einer oder mehrere der Substituenten R^{1a}, R^{1b}, R^{1c}, R^{1d} und R² bis R²⁹ für einen gesättigten oder ungesättigten aliphatischen Rest stehen, der einfach oder mehrfach substituiert ist, dieser mit 1, 2, 3, 4 oder 5 Substituenten unabhängig voneinander ausgewählt aus der Gruppe bestehend aus F, Cl, Br, -NO₂, -CN, -OH, -SH und -NH₂ substituiert sein kann,
sofern einer oder mehrere der Substituenten R^{1a}, R^{1b}, R^{1c}, R^{1d}, R² und R³ für einen cycloaliphatischen Rest stehen oder einen cycloaliphatischen Rest aufweisen, der einfach oder mehrfach substituiert ist, dieser ggf. mit 1, 2, 3, 4 oder 5 Substituenten unabhängig voneinander ausgewählt aus der Gruppe bestehend aus F, Cl, Br, I, -CN, -CF₃, -SF₅, -OH, -NH₂, -O-CF₃, -SH, -O-C₁₋₅-Alkyl, -(CH₂)-O-C₁₋₅-Alkyl, -S-C₁₋₅-Alkyl, -C₁₋₅-Alkyl, -C₂₋₅-Alkenyl, -C₂₋₅-Alkinyl, -C(=O)-O-C₁₋₅-Alkyl, -C(=O)-CF₃, -S(=O)₂-C₁₋₅-Alkyl, -S(=O)-C₁₋₅-Alkyl, - S(=O)₂-Phenyl, Oxo (=O), Thioxo (=S), -N(C₁₋₅-Alkyl)₂, -N(H)(C₁₋₅-Alkyl), -NO₂, -S-CF₃, -C(=O)-OH, -NH-S(=O)₂-C₁₋₅-Alkyl, -NH-C(=O)-C₁₋₅-Alky), -C(=O)-H; - C(=O)-C₁₋₅-Alkyl, -C(=O)-NH₂, -C(=O)-N(C₁₋₅-Alkyl)₂, -C(=O)-N(H)(C₁₋₅-Alkyl) und Phenyl substituiert sein kann, wobei die vorstehend genannten C₁₋₅-Alkyl-Reste jeweils linear oder verzweigt sein können und die Phenyl-Reste jeweils unsubstituiert oder mit 1, 2, 3 oder 4 Substituenten unabhängig voneinander ausgewählt aus der Gruppe bestehend aus F, Cl, Br, I, Methyl, Ethyl, Methoxy und Ethoxy substituiert sein können,
sofern die cycloaliphatischen Reste eines oder mehrere Heteroatome als Ringglieder aufweisen, diese 1,2,3,4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,
sofern die Substituenten R² und R³ zusammen mit dem sie verbindenden Stickstoffatom als Ringglied einen gesättigten oder ungesättigten heterocycloaliphatischen Rest bilden, der einfach oder mehrfach substituiert ist, dieser ggf. mit 1,2,3,4 oder 5 Substituenten unabhängig voneinander ausgewählt aus der Gruppe bestehend aus F, Cl, Br, I, -CN, -CF₃, -SF₅, -OH, - NH₂, -O-CF₃, -SH, -O-C₁₋₅-Alkyl, -(CH₂)-O-C₁₋₅-Alkyl, -S-C₁₋₅-Alkyl, -C₁₋₅-Alkyl, - C₂₋₅-Alkenyl, -C₂₋₅-Alkinyl, -C(=O)-O-C₁₋₅-Alkyl, -C(=O)-CF₃, -S(=O)₂-C₁₋₅-Alkyl, -S(=O)-C₁₋₅-Alky), -S(=O)₂-Phenyl, Oxo (=O), Thioxo (=S), -N(C₁₋₅-Alkyl)₂, - N(H)(C₁₋₅-Alkyl), -NO₂, -S-CF₃, -C(=O)-OH, -NH-S(=O)₂-C₁₋₅-Alkyl, -NH-C(=O)-C₁₋₅-Alkyl, -C(=O)-H; -C(=O)-C₁₋₅-Alkyl, -C(=O)-NH₂, -C(=O)N(C₁₋₅-Alkyl)₂, - C(=O)-N(H)(C₁₋₅-Alkyl) und Phenyl substituiert sein kann, wobei die vorstehend genannten C₁₋₅-Alkyl-Reste jeweils linear oder verzweigt sein können und die Phenyl-Reste jeweils unsubstituiert oder mit 1, 2, 3 oder 4 Substituenten unabhängig voneinander ausgewählt aus der Gruppe bestehend aus F, Cl, Br, I, Methyl, Ethyl, Methoxy und Ethoxy substituiert sein können,
sofern die heterocycloaliphatischen Reste eines oder mehrere weitere Heteroatome als Ringglieder aufweisen, diese 1, 2, 3, 4 oder 5, Heteroatom(e) als Ringglied(er) aufweisen, die jeweils unabhängig voneinander, ausgewählt werden können aus der Gruppe bestehend aus Stickstoff, Sauerstoff und Schwefel,
sofern einer oder mehrere der Substituenten R^{1a}, R^{1b}, R^{1c}, R^{1d}, R² bis R²⁹ und M¹ und M² für einen Aryl- oder Heteroaryl-Rest stehen oder einen Aryl- oder Heteroaryl-Rest aufweisen, der einfach oder mehrfach substituiert ist, dieser ggf. mit 1, 2, 3, 4 oder 5 Substituenten unabhängig voneinander ausgewählt aus der Gruppe bestehend aus Halogen, -CN, -CH₂-CN, -NO₂, -OH, -SH, - NH₂, -CH₂-NH₂, -C(=O)-OH, C₁₋₅-Alkyl, -(CH₂)-OH, -(CH₂)-O-C₁₋₅-Alkyl, -C₂₋₅-Alkenyl, -C₂₋₅-Alkinyl, -S-C₁₋₅-Alkyl, -O-C₁₋₅-Alkyl, -CF₃, -SF₅, -CHF₂, -CH₂F, - O-CF₃, -O-CHF₂, -O-CH₂F, -C(=O)-CF₃, -S-CF₃, -S-CHF₂, -S-CH₂F, -S(=O)₂-Phenyl, -S(=O)₂-C₁₋₅-Alkyl, -S(=O)-C₁₋₅-Alkyl, -NH-C₁₋₅-Alkyl, -N-(C₁₋₅Alkyl)₂, - CH₂-NH-C₁₋₅-Alkyl, -CH₂-N-(C₁₋₅Alkyl)₂, -C(=O)-O-C₁₋₅-Alkyl, -C(=O)-H, - C(=O)-C₁₋₅-Alkyl, -CH₂-O-C(=O)-Phenyl, -O-C(=O)-Phenyl, -NH-S(=O)₂-C₁₋₅-Alkyl, -NH-C(=O)-C₁₋₅-Alkyl, -S(=O)₂-NH₂, -S(=O)₂-NH-C₁₋₅-Alkyl, -S(=O)₂-N(C₁₋₅-Alkyl)₂, -NH-C(=NH)-NH₂, -NH-S(=O)₂-OH, -C(=O)-NH-C₁₋₅-Alkyl, - C(=O)-NH₂, -C(=O)-N(C₁₋₅-Alkyl)₂, -Si(Phenyl)₂[C₁₋₅-Alkyl], Pyrazolyl, Pyrrolyl, (1,3)-Dioxolanyl, Phenyl, Furyl (Furanyl), Thiazolyl, Thiadiazolyl, Thiophenyl (Thienyl), Phenoxy, Benzyl und Phenethyl substituiert sein kann, wobei die zyklischen Substituenten selbst jeweils ggf. mit 1, 2, 3 oder 4 Substituenten unabhängig voneinander ausgewählt aus der Gruppe bestehend aus F, Cl, Br, I, -CF₃, Methyl, Ethyl, Methoxy und Ethoxy substituiert sein können,
sofern einer oder mehrere der Substituenten R^{1a}, R^{1b}, R^{1c}, R^{1d}, R² bis R²⁹ und M¹ und M² für einen Heteroaryl-Rest stehen oder einen Heteroaryl-Rest aufweisen, dessen Heteroatom(e), jeweils unabhängig voneinander, ausgewählt werden können aus der Gruppe bestehend aus Sauerstoff, Schwefel und Stickstoff,
sofern die Substituenten R^{1a} und R^{1b} oder R^{1b} und R^{1c} oder R^{1c} und R^{1d} zusammen mit der sie verbindenden C-C-Brücke einen annelierten Phenyl-Rest bilden, der einfach oder mehrfach substituiert ist, dieser mit 1, 2, 3 oder 4 Substituenten unabhängig voneinander ausgewählt aus der Gruppe bestehend aus Halogen, -CN, -CH₂-CN, -NO₂, -OH, -SH, -NH₂, -CH₂-NH₂, - C(=O)-OH, C₁₋₅-Alkyl, -(CH₂)-O-C₁₋₅-Alkyl, -C₂₋₅-Alkenyl, -C₂₋₅-Alkinyl, -S-C₁₋₅-Alkyl, -O-C₁₋₅-Alkyl, -CF₃, -SF₅, -CHF₂, -CH₂F, -O-CF₃, -O-CHF₂, -O-CH₂F, - C(=O)-CF₃, -S-CF₃, -S-CHF₂, -S-CH₂F, -S(=O)₂-Phenyl, -S(=O)₂-C₁₋₅-Alkyl, - S(=O)-C₁₋₅-Alkyl, -NH-C₁₋₅-Alkyl, -N-(C₁₋₅Alkyl)₂, -CH₂-NH-C₁₋₅-Alkyl, -CH₂-N-(C₁₋₅Alkyl)₂, -C(=O)-O-C₁₋₅-Alkyl, -C(=O)-H, -C(=O)-C₁₋₅-Alkyl, -CH₂-O-C(=O)-Phenyl, -O-C(=O)-Phenyl, -NH-S(=O)₂-C₁₋₅-Alkyl, -NH-C(=O)-C₁₋₅-Alkyl, - C(=O)-NH-C₁₋₅-Alkyl, -C(=O)-NH₂, -C(=O)-N(C₁₋₅-Alkyl)₂, -Si(Phenyl)₂[C₁₋₅-Alkyl], Pyrazolyl, Pyrrolyl, (1,3)-Dioxolanyl, Phenyl, Furyl (Furanyl), Thiazolyl, Thiadiazolyl, Thiophenyl (Thienyl), Phenoxy, Benzyl und Phenethyl substituiert sein kann, wobei die zyklischen Substituenten selbst jeweils ggf. mit 1, 2, 3 oder 4 Substituenten unabhängig voneinander ausgewählt aus der Gruppe bestehend aus F, Cl, Br, I, -CF₃, Methyl, Ethyl, Methoxy und Ethoxy substituiert sein können,
sofern ein polyzyklisches Ringsystem vorliegt, die verschiedenen Ringe, jeweils unabhängig voneinander, gesättigt, ungesättigt oder aromatisch sein können und die Heteroatome jedes Ringes, jeweils unabhängig voneinander, ausgewählt werden können aus der Gruppe bestehend aus Sauerstoff, Stickstoff und Schwefel,
sofern einer oder mehrere der Substituenten R^{1a}, R^{1b}, R^{1c}, R^{1d}, R² bis R²⁹ und M¹ und M² ein monozyklisches oder polyzyklisches Ringsystem aufweisen, das einfach oder mehrfach substituiert ist, dieses ggf. mit 1, 2, 3, 4 oder 5 Substituenten substituiert sein kann, die unabhängig voneinander ausgewählt werden können aus der Gruppe bestehend aus Halogen, -CN, -NO₂, -OH, - SH, -NH₂, Oxo (=O), Thioxo (=S), -C(=O)-OH, -C₁₋₅-Alkyl, -C₂₋₅-Alkenyl, -C₂₋₅-Alkinyl, -(CH₂)-O-C₁₋₅-Alkyl, -S-C₁₋₅-Alkyl, -O-C₁₋₅-Alkyl, -CF₃,-SF₅, -CHF₂, - CH₂F, -O-CF₃, -O-CHF₂, -O-CH₂F, -C(=O)-CF₃, -S-CF₃, -S-CHF₂, -S-CH₂F, - S(=O)₂-Phenyl, -S(=O)₂-C₁₋₅-Alkyl, -S(=O)-C₁₋₅-Alkyl, -NH-C₁₋₅-Alkyl, -N(C₁₋₅Alkyl)(C₁₋₅-Alkyl), -C(=O)-O-C₁₋₅-Alkyl, -C(=O)-H, -C(=O)-C₁₋₅-Alkyl, -CH₂-O- C(=O)-Phenyl, -O-C(=O)-Phenyl, -NH-S(=O)₂-C₁₋₅-Alkyl, -NH-C(=O)-C₁₋₅-Alkyl, -C(=O)-NH₂, -C(=O)-NH-C₁₋₅-Alkyl, -C(=O)-N(C₁₋₅-Alkyl)₂, Pyrazolyl, Phenyl, Furyl (Furanyl), Thiadiazolyl, Thiophenyl (Thienyl), Phenoxy und Benzyl, wobei die zyklischen Substituenten selbst jeweils ggf. mit 1, 2, 3 oder 4 Substituenten unabhängig voneinander ausgewählt aus der Gruppe bestehend aus F, Cl, Br, I, Methyl, Ethyl, Methoxy und Ethoxy substituiert sein können,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, daß**
A¹ für ein Stickstoffatom oder für eine C-R^{1a}-Gruppe steht,
A² für ein Stickstoffatom oder für eine C-R^{1b}-Gruppe steht,
A³ für ein Stickstoffatom oder für eine C-R^{1c}-Gruppe steht,
A⁴ für ein Stickstoffatom oder für eine C-R^{1d}-Gruppe steht,
R¹⁸, R^{1b}, R^{1c}, R^{1d}, unabhängig voneinander, jeweils für einen Wasserstoff-Rest; für einen Halogen-Rest; -NO₂; -CN; -NH₂; -NHR⁴; -NR⁵R⁶; -NH-C(=O)-R⁷; -C(=O)-R⁸, -C(=O)-NH₂; -C(=O)-NHR⁹; -C(=O)-NR¹⁰R¹¹; -C(=O)-OR¹²; -(CH₂)ₘ-C(=O)OR¹³ mit m = 1, 2, 3, 4 oder 5; -O-C(=O)-R¹⁴; -(CH₂)ₙ-O-C(=O)-R¹⁵ mit n = 1, 2, 3, 4 oder 5; -OR¹⁶; -(CH₂)ₒ-O-R¹⁷ mit o = 1, 2, 3; 4 oder 5; -SR¹⁸; -(CH₂)ₚ-S(=O)ₜR¹⁹ mit p = 1,2,3,4 oder 5 und t = 0, 1 oder 2; -NH-S(=O)₂-R²⁶R²⁷; -S(=O)-NR²⁸R²⁹,-SF₅; für einen linearen oder verzweigten, gesättigten oder ungesättigten, unsubstituierten oder wenigstens einfach substituierten aliphatischen C₁₋₁₀-Rest; für einen gesättigten oder ungesättigten, unsubstituierten oder wenigstens einfach substituierten, ggf. wenigstens ein Heteroatom als Ringglied aufweisenden cycloaliphatischen C₃₈-Rest, der über eine lineare oder verzweigte C₁₋₅-Alkylen-Gruppe gebunden sein kann; oder für einen unsubstituierten oder wenigstens einfach substituierten 5- bis 14-gliedrigen Aryl- oder Heteroaryl-Rest, der über eine lineare oder verzweigte C₁₋₅-Alkylen-Gruppe gebunden sein kann, stehen,
oder ggf. R^{1a} und R^{1b} mit der sie verbindenden C-C-Brücke einen unsubstituierten oder wenigstens einfach substituierten annelierten Phenyl-Rest bilden,
oder ggf. R^{1b} und R^{1c} mit der sie verbindenden C-C-Brücke einen unsubstituierten oder wenigstens einfach substituierten annelierten Phenyl-Rest bilden,
oder ggf. R^{1c} und R^{1d} mit der sie verbindenden C-C-Brücke einen unsubstituierten oder wenigstens einfach substituierten annelierten Phenyl-Rest bilden,
R² und R³, unabhängig voneinander, jeweils für einen Wasserstoff-Rest; -C(=O)-R²⁰; -(CH₂)_{q}-C(=O)-R²¹ mit q = 1,2,3,4 oder 5; -C(=O)-O-R²²; - (CH₂)ᵣ-C(=O)-O-R²³ mit r =1,2, 3, 4 oder 5; -C(=O)-NHR²⁴; -(CH₂)ₛ-C(=O)-NHR²⁵ mit s = 1, 2, 3, 4 oder 5; für einen linearen oder verzweigten, gesättigten oder ungesättigten, unsubstituierten oder wenigstens einfach substituierten aliphatischen C₁₋₁₀-Rest; für einen gesättigten oder ungesättigten, unsubstituierten oder wenigstens einfach substituierten, ggf. wenigstens ein Heteroatom als Ringglied aufweisenden cycloaliphatischen C₄₋₈-Rest, der über eine lineare oder verzweigte C₁₋₅-Alkylen-Gruppe gebunden sein kann; oder für einen unsubstituierten oder wenigstens einfach substituierten 5- bis 14-gliedrigen Aryl- oder Heteroaryl-Rest, der über eine lineare oder verzweigte C₁₋₅-Alkyen-Gruppe gebunden sein kann, stehen,
oder R² und R³ zusammen mit dem sie verbindenden Stickstoffatom als Ringglied einen gesättigten oder ungesättigten, ggf. wenigstens ein weiteres Heteroatom als Ringglied aufweisenden heterocycloaliphatischen C₄₋₁₀-Rest bilden, der ggf. mit einem unsubstituierten oder wenigstens einfach substituierten mono- oder polyzyklischen Ringsystem kondensiert ist, wobei die Ringe des Ringsystems jeweils 5-, 6- oder 7-gliedrig sind;
R⁴, R⁵, R⁶, R⁷, R⁹, R¹⁰, R¹¹, R¹⁴ und R¹⁵, jeweils unabhängig voneinander, für einen linearen oder verzweigten, gesättigten oder ungesättigten aliphatischen C₁₋₄-Rest oder für einen unsubstituierten oder wenigstens einfach substituierten 5- bis 14-gliedrigen Aryl- oder Heteroaryl-Rest, der über eine lineare oder verzweigte C₁₋₅-Alkylen-Gruppe gebunden sein kann, stehen,
R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ und R²⁹, jeweils unabhängig voneinander, für einen Wasserstoff-Rest; für einen linearen oder verzweigten, gesättigten oder ungesättigten aliphatischen C₁₋₄-Rest oder für einen unsubstituierten oder wenigstens einfach substituierten 5-bis 14-gliedrigen Aryl- oder Heteroaryl-Rest, der über eine lineare oder verzweigte C₁₋₅-Alkylen-Gruppe gebunden sein kann, stehen,
M¹ für einen 5- oder 6-gliedrigen Aryl- oder Heteroaryl-Rest steht, der mit wenigstens einem weiteren Substituenten substituiert sein kann und ggf. mit einem unsubsituierten oder wenigstens einfach substituierten mono- oder bizyklischem Ringsystem kondensiert ist, wobei die Ringe des Ringsystems jeweils 5-, 6- oder 7-gliedrig sind, und
M² für einen 5- oder 6-gliedrigen Aryl- oder Heteroaryl-Rest steht, der unsubstituiert oder wenigstens einfach substituiert und mit einem unsubstituierten oder wenigstens einfach substituierten mono- oder bizyklischem Ringsystem kondensiert sein kann, wobei die Ringe des Ringsystems jeweils 5-, 6- oder 7-gliedrig sind;
wobei
die vorstehend genannten cycloaliphatischen Reste ggf. 1, 2, 3, 4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,
die vorstehend genannten heterocycloaliphatischen Reste ggf. weitere 1, 2, 3, 4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,
die Ringe des mono- oder polyzyklischen Ringsystems jeweils ggf. 0,1,2 oder 3 Heteroatom(e) als Ringglied(er) aufweisen, die unabhängig voneinander aus der Gruppe bestehend aus Sauerstoff, Stickstoff und Schwefel ausgewählt sind;
und
die vorstehend genannten Heteroaryl-Reste ggf. 1, 2, 3, 4 oder 5 Heteroatom(e) als Ringglied(er) aufweisen können, die jeweils unabhängig voneinander, ausgewählt werden können aus der Gruppe bestehend aus Sauerstoff, Schwefel und Stickstoff.

3. Verbindungen gemäß Anspruch 1 oder 2, **dadurch gekennzeichnet, dass**
A¹ für eine C-R^{1a}-Gruppe steht,
A² für eine C-R^{1b}-Gruppe steht,
A³ für eine C-R^{1c}-Gruppe steht und
A⁴ für eine C-R^{1d}-Gruppe steht,
oder
A¹ für ein Stickstoffatom steht,
A² für eine C-R^{1b}-Gruppe steht,
A³ für eine C-R^{1c}-Gruppe steht und
A⁴ für eine C-R^{1d}-Gruppe steht,
oder
A¹ für eine C-R^{1a}-Gruppe steht,
A² für ein Stickstoffatom steht,
A³ für eine C-R^{1c}-Gruppe steht und
A⁴ für eine C-R^{1d}-Gruppe steht,
oder
A¹ für eine C-R^{1a}-Gruppe steht,
A² für eine C-R^{1b}-Gruppe steht,
A³ für ein Stickstoffatom steht und
A⁴ für eine C-R^{1d}-Gruppe steht,
oder
A¹ für eine C-R^{1a}-Gruppe steht,
A² für eine C-R^{1b}-Gruppe steht,
A³ für eine C-R^{1c}-Gruppe steht und
A⁴ für ein Stickstoffatom steht,
oder
A¹ und A³ jeweils für ein Stickstoffatom stehen,
A² für eine C-R^{1b}-Gruppe steht und
A⁴ für eine C-R^{1d}-Gruppe steht.

4. Verbindungen gemäß Anspruch 3, **dadurch gekennzeichnet, dass**
A¹ für eine C-R^{1a}-Gruppe steht,
A² für eine C-R^{1b}-Gruppe steht,
A³ für eine C-R^{1c}-Gruppe steht und
A⁴ für eine C-R^{1d}-Gruppe steht,
oder
A¹ für ein Stickstoffatom steht,
A² für eine C-R^{1b}-Gruppe steht,
A³ für eine C-R^{1c}-Gruppe steht und
A⁴ für eine C-R^{1d}-Gruppe steht,
oder
A¹ für eine C-R^{1a}-Gruppe steht,
A² für ein Stickstoffatom steht,
A³ für eine C-R^{1c}-Gruppe steht und
A⁴ für eine C-R^{1d}-Gruppe steht,
oder
A¹ für eine C-R^{1a}-Gruppe steht,
A² für eine C-R^{1b}-Gruppe steht,
A³ für eine C-R^{1c}-Gruppe steht und
A⁴ für ein Stickstoffatom steht.

5. Verbindungen gemäß einem oder mehreren der Ansprüche 1 bis 4, **dadurch gekennzeichnet, dass** R^{1a}, R^{1b}, R^{1c}, R^{1d}, unabhängig voneinander, jeweils für - H; -F; -Cl, -Br, -I; NO₂; -CN; -CF₃; -SF₅; -NH₂; -S(=O)-NH₂, -NHR⁴; -NR⁵R⁶; - C(=O)-OR¹²; -(CH₂)ₘ-C(=O)-OR¹³ mit m = 1, 2 oder 3; -O-C(=O)-R¹⁴; -OR¹⁶; - (CH₂)ₒ-O-R¹⁷ mit o = 1, 2 oder 3; für einen linearen oder verzweigten C₁₋₁₀-Alkyl-Rest; oder für einen unsubstituierten oder wenigstens einfach substituierten 5- oder 6-gliedrigen Aryl- oder Heteroaryl-Rest, der über eine lineare oder verzweigte C₁₋₄-Alkylen-Gruppe gebunden sein kann, stehen.

6. Verbindungen gemäß einem oder mehreren der Ansprüche 1 bis 5, dadurchgekennzeichnet, dass R^{1a} und R^{1b} oder R^{1b} und R^{1c} oder R^{1c} und R^{1d} zusammen mit der sie verbindenden C-C-Brücke einen annelierten Phenyl-Rest bilden, der mit 1, 2, 3 oder 4 Substituenten unabhängig voneinander ausgewählt aus der Gruppe bestehend aus F, Cl, Br, -CN, Methyl, Ethyl, n-Propyl, iso-Propyl, n-Butyl, sec-Butyl, iso-Butyl, tert-Butyl, -OH, -O-CH₃, -O-C₂H₅ substituiert sein kann.

7. Verbindungen gemäß einem oder mehreren der Ansprüche 1 bis 6, **dadurch gekennzeichnet, dass** R² und R³, unabhängig voneinander, jeweils für einen Wasserstoff-Rest; -C(=O)-R²⁰; -(CH₂)_{q}-C(=O)-R²¹ mit q = 1, 2, 3, 4 oder 5; - (CH₂)ᵣC(=O)-O-R²³ mit r = 1,2,3,4 oder 5; -C(=O)-NHR²⁴; für einen linearen oder verzweigten C₁₋₁₆ Alkyl-Rest; für einen unsubstituierten oder wenigstens einfach substituierten C₄₋₈ Cycloalkyl-Rest, der über eine lineare oder verzweigte C₁₋₃-Alkylen-Gruppe gebunden sein kann; oder für einen unsubstituierten oder wenigstens einfach substituierten, 5- oder 6-gliedrigen Aryl- oder Heteroaryl-Rest, der über eine lineare oder verzweigte C₁₋₃-Alkylen-Gruppe gebunden sein kann, stehen.

8. Verbindungen gemäß einem oder mehreren der Ansprüche 1 bis 7, **dadurch gekennzeichnet, dass** R² und R³ zusammen mit dem sie verbindenden Stickstoffatom als Ringglied einen Rest bilden ausgewählt aus der Gruppe bestehend aus Imidazolidinyl, Piperidinyl, Piperazinyl, Morpholinyl, Pyrrolidinyl, Thiomorpholinyl, Azepanyl, Diazepanyl und Azocanyl, der jeweils unsubstituiert oder ggf. mit 1, 2, 3, 4 oder 5 Substituenten unabhängig voneinander ausgewählt aus der Gruppe bestehend aus F, Cl, Br, I, -CN, Methyl, Ethyl, n-Propyl, iso-Propyl, n-Butyl, sec-Butyl, iso-Butyl, tert-Butyl, - OH, Oxo, Thioxo, -O-CH₃, -O-C₂H₅, -O-C₃H₇, -NH₂, -N(CH₃)₂, -N(C₂H₅)₂, -NH-CH₃, -NH-C₂H₅, -O-CF₃, -S-CF₃, -S(=O)-CH₃, -S(=O)-C₂H₅, -S(=O)₂-CH₃,-S(=O)₂-C₂H₅, -C(=O)-OH, -C(=O)-H, -C(=O)-CH₃, -C(=O)-C₂H₅, -C(=O)-N(CH₃)₂, -C(=O)-NH-CH₃, -C(=O)-NH₂, -C(=O)-O-CH₃, -C(=O)-O-C₂H₅ und - C(=O)-O-C(CH₃)₃ substituiert sein kann.

9. Verbindungen gemäß einem oder mehreren der Ansprüche 1 bis 8, **dadurch gekennzeichnet, dass** R⁴, R⁵, R⁶, R⁷, R⁹, R¹⁰, R¹¹, R¹⁴ und R¹⁵, jeweils unabhängig voneinander, für einen linearen oder verzweigten C₁₋₄ Alkyl-Rest oder für einen unsubstituierten oder wenigstens einfach substituierten 5- bis 14-gliedrigen Aryl- oder Heteroaryl-Rest, der über eine lineare oder verzweigte C₁₋₅-Alkylen-Gruppe gebunden sein kann, stehen.

10. Verbindungen gemäß einem oder mehreren der Ansprüche 1 bis 9, **dadurch gekennzeichnet, dass** R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ und R²⁹, jeweils unabhängig voneinander, für einen Wasserstoff-Rest; für einen linearen oder verzweigten C₁₋₈-Alkyl-Rest, oder für einen unsubstituierten oder wenigstens einfach substituierten 5- bis 14-gliedrigen Aryl- oder Heteroaryl-Rest, der über eine lineare oder verzweigte C₁₋₅-Alkylen-Gruppe gebunden sein kann, stehen.

11. Verbindungen gemäß einem oder mehreren der Ansprüche 1 bis 10, **dadurch gekennzeichnet, dass** M¹ für einen 5- oder 6-gliedrigen Aryl- oder Heteroaryl-Rest steht, der ggf. mit wenigstens einem weiteren Substituenten substituiert sein kann, wobei der Heteroaryl-Rest ggf. 1, 2, 3, 4 oder 5 Heteroatom(e) als Ringglied(er) aufweist, die unabhängig voneinander ausgewählt sind aus der Gruppe bestehend aus Stickstoff, Sauerstoff und Schwefel.

12. Verbindungen gemäß Anspruch 11, **dadurch gekennzeichnet, daß** M¹ für einen Rest ausgewählt aus der Gruppe bestehend aus den Resten 1 bis 38 steht, 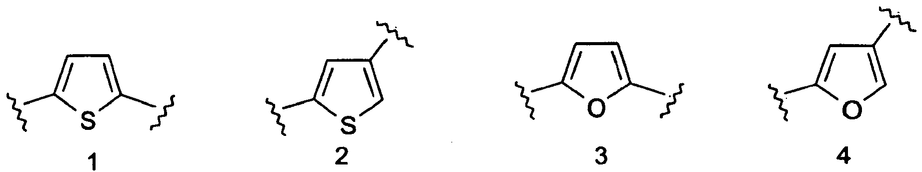 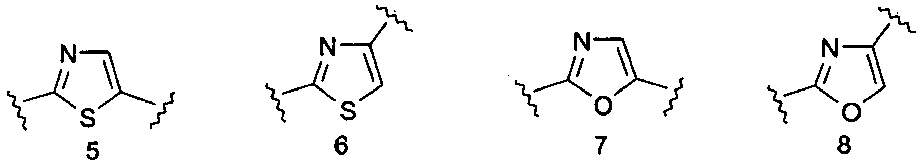 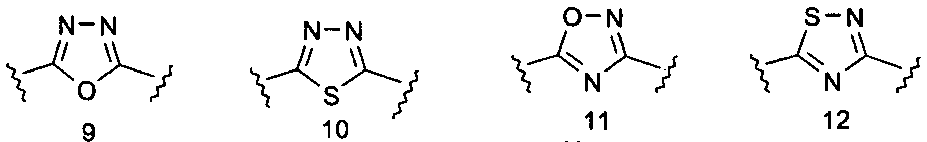 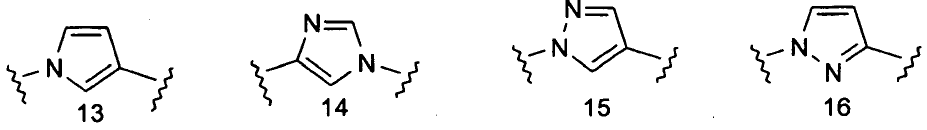 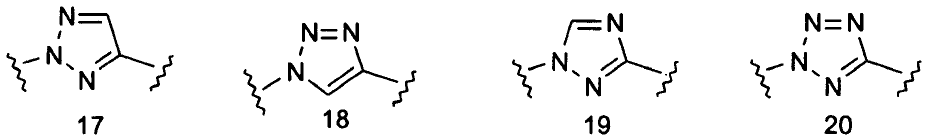 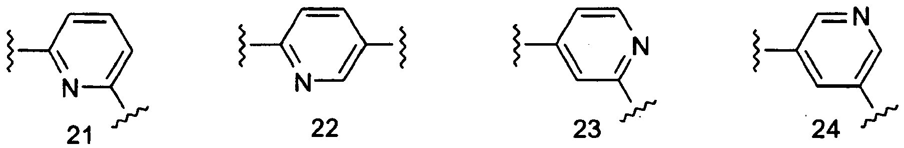 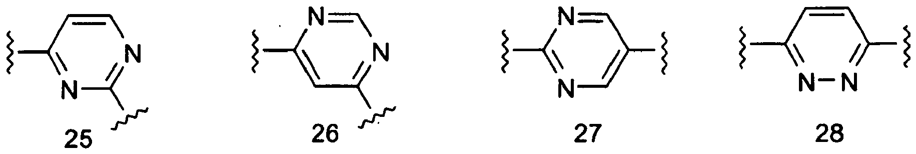 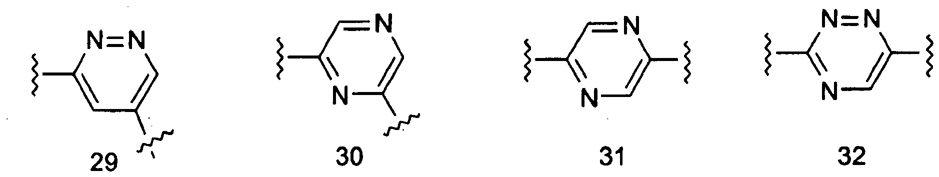 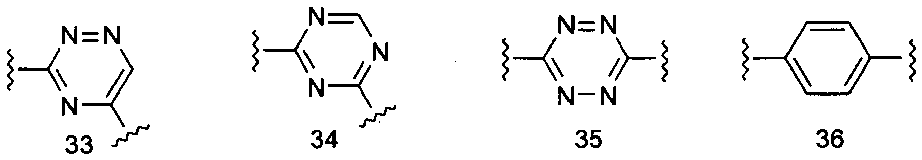 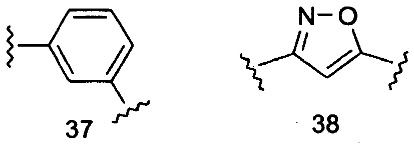 der jeweils in beliebiger Richtung über die mit einer geschlängelten Linie gekennzeichneten Positionen mit dem Bizyklus und dem Kohlenstoffatom der Dreifachbindung verknüpft sein kann und der ggf. mit 1, 2, 3 oder 4 weiteren Substituenten substituiert ist, die unabhängig voneinander ausgewählt sind aus der Gruppe bestehend aus F, Cl, Br, -CN, -CH₂-CN, -CF₃, -SF₅, Methyl, Ethyl, n-Propyl, iso-Propyl, n-Butyl, sec-Butyl, iso-Butyl, tert-Butyl, -OH, -O-CH₃, -O-C₂H₅, -O-C₃H₇, -NH₂, -N(CH₃)₂, -N(C₂H₅)₂, -NO₂, -O-CF₃, -S-CF₃, -SH, -C(=O)-CH₃ und -C(=O)-C₂H₅.

13. Verbindungen gemäß einem oder mehreren der Ansprüche 1 bis 12, **dadurch gekennzeichnet, dass** M² für einen unsubstituierten oder wenigstens einfach substituierten, 5- oder 6-gliedrigen Aryl- oder Heteroaryl-Rest steht, wobei der Heteroaryl-Rest 1, 2, 3, 4 oder 5 Heteroatom(e) als Ringglied(er) aufweist, die unabhängig voneinander ausgewählt sind aus der Gruppe bestehend aus Stickstoff, Sauerstoff und Schwefel, und wobei der Aryl- oder Heteroaryl-Rest mit einem unsubsituierten oder wenigstens einfach substituierten mono- oder bizyklischem Ringsystem kondensiert sein kann, wobei die Ringe des Ringsystems jeweils 5- oder 6-gliedrig sind und jeweils 1, 2, 3 oder 4 Heteroatom(e) als Ringglied(er) aufweisen können, die unabhängig voneinander ausgewählt sind aus der Gruppe bestehend aus Stickstoff, Sauerstoff und Schwefel.

14. Verbindungen gemäß einem oder mehreren der Ansprüche 1 bis 13, **dadurch gekennzeichnet, dass**
A¹ für eine C-R^{1a}-Gruppe steht,
A² für eine C-R^{1b}-Gruppe steht,
A³ für eine C-R^{1c}-Gruppe steht und
A⁴ für eine C-R^{1d}-Gruppe steht;
oder
A¹ für ein Stickstoffatom steht,
A² für eine C-R^{1b}-Gruppe steht,
A³ für eine C-R^{1c}-Gruppe steht und
A⁴ für eine C-R^{1d}-Gruppe steht;
oder
A¹ für eine C-R^{1a}-Gruppe steht,
A² für ein Stickstoffatom steht,
A³ für eine C-R^{1c}-Gruppe steht und
A⁴ für eine C-R^{1d}-Gruppe steht:
R^{1a}, R^{1b}, R^{1c}, R^{1d}, unabhängig voneinander, jeweils für -H; -F; -Cl, -Br, -I; -NO₂; -CN; -CF₃; -SF₅; -NH₂; -S(=O)-NH₂,-NHR⁴; -NR⁵R⁶; -C(=O)-OR¹²; -(CH₂)ₘ-C(=O)-OR¹³ mit m = 1, 2 oder 3; -O-C(=O)-R¹⁴; -OR¹⁶; -(CH₂)ₒ-O-R¹⁷ mit o = 1, 2 oder 3; für einen linearen oder verzweigten Alkyl-Rest ausgewählt aus der Gruppe bestehend aus Methyl, Ethyl, n-Propyl, iso-Propyl, n-Butyl, iso-Butyl, sec-Butyl und tert-Butyl, oder für einen Aryl- oder Heteroaryl-Rest ausgewählt aus der Gruppe bestehend aus Phenyl, Furanyl, Thiophenyl und Pyridinyl, der jeweils unsubstituiert oder mit 1, 2, 3, 4 oder 5 Substituenten unabhängig voneinander ausgewählt aus der Gruppe bestehend aus F, Cl, Br, -CN, Methyl, Ethyl, n-Propyl, iso-Propyl, n-Butyl, sec-Butyl, iso-Butyl, tert-Butyl, - OH, -O-CH₃, -O-C₂H₅ substituiert und ggf. über eine -(CH₂)-, -(CH₂)₂- oder - (CH₂)₃-Gruppe gebunden ist, stehen;
oder ggf. R^{1a} und R^{1b} oder ggf. R^{1b} und R^{1c} oder ggf. R^{1c} und R^{1d} zusammen mit der sie verbindenden C-C-Brücke einen annelierten Phenyl-Rest bilden, der mit 1, 2, 3 oder 4 Substituenten unabhängig voneinander ausgewählt aus der Gruppe bestehend aus F, Cl, Br, -CN, Methyl, Ethyl, n-Propyl, iso-Propyl, n-Butyl, sec-Butyl, iso-Butyl, tert-Butyl, -OH, -O-CH₃, -O-C₂H₅ substituiert sein kann;
R² und R³, unabhängig voneinander, jeweils für einen Wasserstoff-Rest; - C(=O)-R²⁰; -(CH₂)_{q}-C(=O)-R²¹ mit q = 1, 2 oder 3; -(CH₂)ᵣC(=O)-O-R²³ mit r = 1, 2 oder 3; für einen linearen oder verzweigten C₁₋₁₀ Alkyl-Rest; für einen C₄₋₈ Cycloalkyl-Rest, der jeweils unsubstituiert oder mit 1, 2, 3, 4 oder 5 Substituenten unabhängig voneinander ausgewählt aus der Gruppe bestehend aus F, Cl, Br, -CN, -CF₃, Methyl, Ethyl, n-Propyl, iso-Propyl, n-Butyl, sec-Butyl, iso-Butyl, tert-Butyl, -OH, -O-CH₃ und -O-C₂H₅ substituiert und ggf. über eine lineare oder verzweigte C₁₋₃-Alkylen-Gruppe gebunden ist;
oder für einen Aryl- oder Heteroaryl-Rest ausgewählt aus der Gruppe bestehend aus Phenyl, Thiophenyl, Furanyl und Pyridinyl, der jeweils unsubstituiert oder ggf. mit 1, 2, 3, 4 oder 5 Substituenten unabhängig voneinander ausgewählt aus der Gruppe bestehend aus F, Cl, Br, -CN, -CF₃, Methyl, Ethyl, n-Propyl, iso-Propyl, n-Butyl, sec-Butyl, iso-Butyl, tert-Butyl, - OH, -O-CH₃ und -O-C₂H₅ substituiert und ggf. über eine lineare oder verzweigte C₁₋₃-Alkylen-Gruppe gebunden ist, stehen;
oder R² und R³ zusammen mit dem sie verbindenden Stickstoffatom als Ringglied einen Rest bilden ausgewählt aus der Gruppe bestehend aus Imidazolidinyl, Piperidinyl, Piperazinyl, Morpholinyl, Pyrrolidinyl, Thiomorpholinyl, Azepanyl, Diazepanyl und Azocanyl, der jeweils unsubstituiert oder ggf. mit 1, 2, 3, 4 oder 5 Substituenten unabhängig voneinander ausgewählt aus der Gruppe bestehend aus F, Cl, Br, I, -CN, Methyl, Ethyl, n-Propyl, iso-Propyl, n-Butyl, sec-Butyl, iso-Butyl, tert-Butyl, - OH, Oxo, Thioxo, -O-CH₃, -O-C₂H₅, -O-C₃H₇, -NH₂, -N(CH₃)₂, -N(C₂H₅)₂, -NH-CH₃, -NH-C₂H₅, -O-CF₃, -S-CF₃, -S(=O)-CH₃, -S(=O)C₂H₅, -S(=O)₂-CH₃, - S(=O)₂-C₂H₅, -C(=O)-OH, -C(=O)-H, -C(=O)-CH₃, -C(=O)-C₂H₅, -C(=O)-N(CH₃)₂, -C(=O)-NH-CH₃, -C(=O)-NH₂, -C(=O)-O-CH₃, -C(=O)-O-C₂H₅ und - C(=O)-O-C(CH₃)₃ substituiert sein kann;
R⁴, R⁵, R⁶ und R¹⁴, jeweils unabhängig voneinander, für einen Alkyl-Rest ausgewählt aus der Gruppe bestehend aus Methyl, Ethyl, n-Propyl und isoPropyl, oder für einen Phenyl-Rest, der jeweils unsubstituiert oder mit 1, 2 oder 3 Substituenten unabhängig voneinander ausgewählt aus der Gruppe bestehend aus F, Cl, Br, -CN, -CF₃, -SF₅, Methyl, Ethyl, n-Propyl, iso-Propyl, tert-Butyl, -O-CH₃ und -O-C₂H₅ substituiert und ggf. über eine -(CH₂)-, -(CH₂)₂-oder -(CH₂)₃-Gruppe gebunden ist, stehen;
R¹², R¹³, R¹⁶, R¹⁷, R²⁰, R²¹ und R²³, jeweils unabhängig voneinander, für einen Wasserstoff-Rest; für einen Alkyl-Rest ausgewählt aus der Gruppe bestehend aus Methyl, Ethyl, n-Propyl, iso-Propyl und tert-Butyl, oder für einen Phenyl-Rest, der jeweils unsubstituiert oder mit 1, 2 oder 3 Substituenten unabhängig voneinander ausgewählt aus der Gruppe bestehend aus F, Cl, Br, -CN, -CF₃, - SF₅, Methyl, Ethyl, n-Propyl, iso-Propyl, tert-Butyl, -O-CH₃ und -O-C₂H₅ substituiert und ggf. über eine -(CH₂)-, -(CH₂)₂- oder -(CH₂)₃-Gruppe gebunden ist, stehen;
M¹ für einen Rest ausgewählt aus der Gruppe bestehend aus den Resten 1
bis 9, 11, 21, 22 und 36 bis 38 steht, 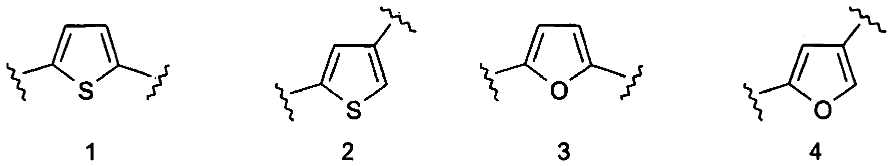 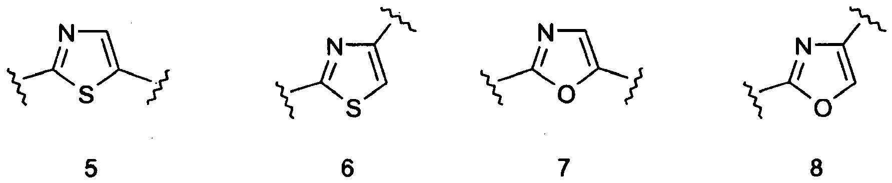 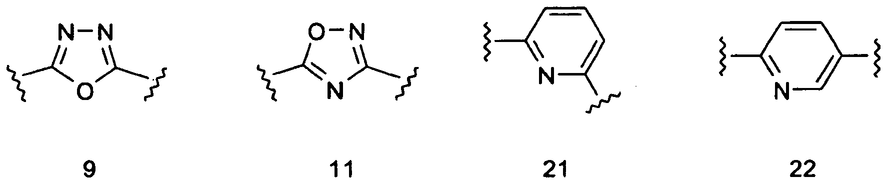 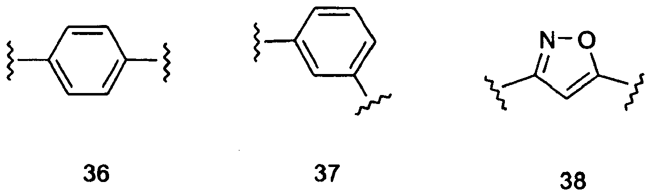
der jeweils in beliebiger Richtung über die mit einer geschlängelten Linie gekennzeichneten Positionen mit dem Bizyklus und dem Kohlenstoffatom der Dreifachbindung verknüpft sein kann und ggf. mit 1 oder 2 weiteren Substituenten substituiert ist, die unabhängig voneinander ausgewählt sind aus der Gruppe bestehend aus F, Cl, Br, -CN, -CH₂-CH, -CF₃, -SF₅, Methyl, Ethyl, n-Propyl, iso-Propyl, n-Butyl, iso-Butyl, sec-Butyl, tert-Butyl, -O-CH₃ und -O-CF₃;
und
M² für einen Rest ausgewählt aus der Gruppe bestehend aus den Resten 1 bis 36 steht, 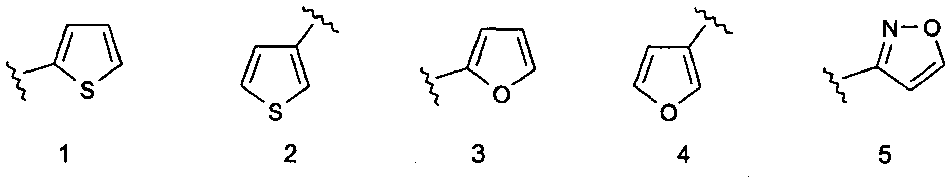 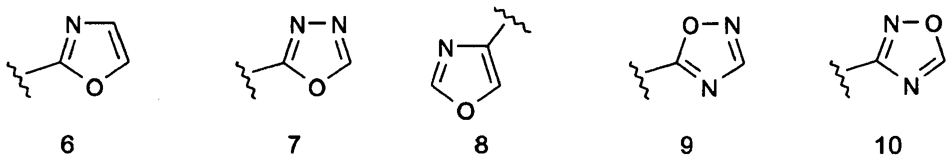 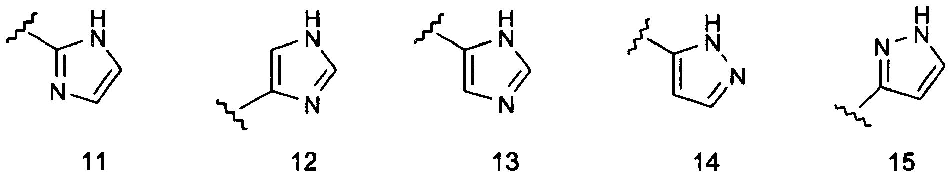 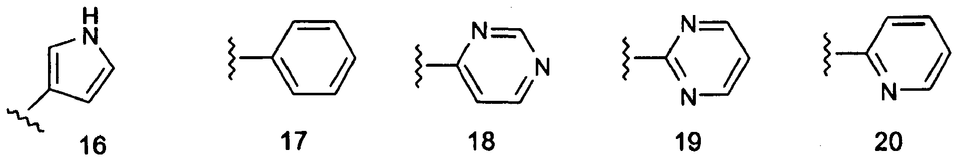 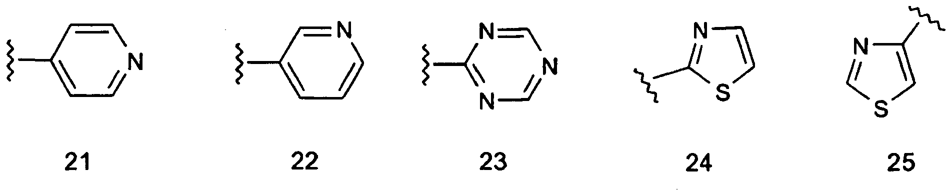 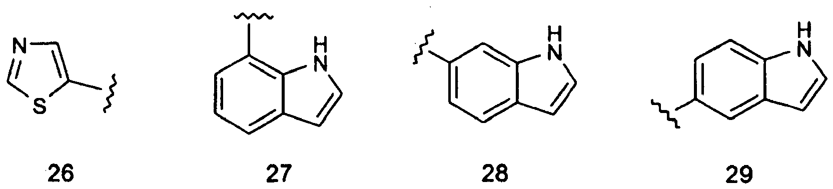 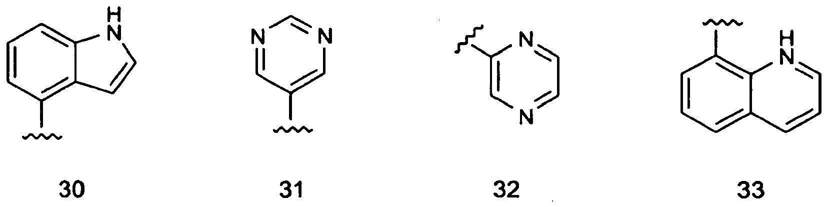 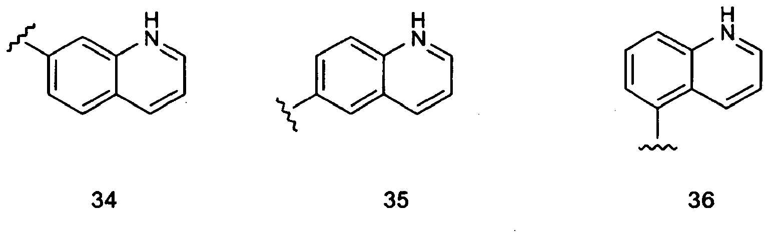
der jeweils über die mit einer geschlängelten Linie gekennzeichnete Position mit dem Kohlenstoffatom der Dreifachbindung verknüpft und unsubstituiert oder ggf. mit 1, 2 oder 3 Substituenten substituiert ist, die unabhängig voneinander ausgewählt sind aus der Gruppe bestehend aus F, Cl, Br, -CN, - CF₃, -SF₅, -S-CH₃, -S-C₂H₅, Methyl, Ethyl, n-Propyl, iso-Propyl, n-Butyl, tert-Butyl, Ethenyl, Propenyl, -O-CH₃, -O-C₂H₅, -NH₂, -N(CH₃)₂, -NH-CH₃, -CH₂-NH₂, -O-CF₃, -C(=O)-H, -C(=O)-O-CH₃, -C(=O)-O-C₂H₅, Phenyl, Pyrrolyl, (1,3)-Dioxolanyl, -Si(Phenyl)₂[C(CH₃)₃], -NO₂, NH-S(=O)₂-CH₃, -NH-S(=O)₂-C₂H₅, - S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -S(=O)₂-N(CH₃)₂, -NH-S(=O)₂-OH und -NH-C(=NH)-NH₂;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

15. Verbindungen gemäß einem oder mehreren der Ansprüche 1 bis 14, **dadurch gekennzeichnet, dass**
A¹ für eine C-R^{1a}-Gruppe steht,
A² für eine C-R^{1b}-Gruppe steht,
A³ für eine C-R^{1c}-Gruppe steht und
A⁴ für eine C-R^{1d}-Gruppe steht,
oder
A¹ für ein Stickstoffatom steht,
A² für eine C-R^{1b}-Gruppe steht,
A³ für eine C-R^{1c}-Gruppe steht und
A⁴ für eine C-R^{1d}-Gruppe steht,
oder
A¹ für eine C-R^{1a}-Gruppe steht,
A² für ein Stickstoffatom steht,
A³ für eine C-R^{1c}-Gruppe steht und
A⁴ für eine C-R^{1d}-Gruppe steht,
R^{1a}, R^{1b}, R^{1c}, R^{1d}, jeweils unabhängig voneinander, für einen Wasserstoff-Rest; -OR¹⁶; -F; -Cl, -Br; -CN; -S(=O)₂-NH₂; -CF₃; -C(=O)-OR¹²; einen Alkyl-Rest ausgewählt aus der Gruppe bestehend aus Methyl, Ethyl, n-Propyl, iso-Propyl, n-Butyl, sec-Butyl, iso-Butyl und tert-Butyl; für einen Phenyl-Rest; einen Benzyl-Rest; einen Phenethyl-Rest oder für einen (3-Phenyl)-prop-1-yl-Rest stehen,
oder ggf. R^{1a} und R^{1b} zusammen mit der sie verbindenden C-C-Brücke einen unsubstituierten annelierten Phenyl-Rest bilden;
oder ggf. R^{1c} und R^{1d} zusammen mit der sie verbindenden C-C-Brücke einen unsubstituierten annelierten Phenyl-Rest bilden;
R² und R³, unabhängig voneinander, jeweils für einen Wasserstoff-Rest; - C(=O)-R²⁰; -(CH₂)_{q}-C(=O)-R²¹ mit q = 1; -(CH₂)ᵣ-C(=O)-O-R²³ mit r = 1; für einen Alkyl-Rest ausgewählt aus der Gruppe bestehend aus Methyl; Ethyl; n-Propyl; iso-Propyl; n-Butyl; tert-Butyl; sec-Butyl; iso-Butyl; n-Pentyl; n-Hexyl; n-Heptyl; n-Octyl; (1,1,3,3)-Tetramethyl-butyl; (1,1)-Dimethyl-pentyl und (1,1)-Dimethylbutyl; für einen unsubstituierten Cyclopentyl- oder Cyclohexyl-Rest, der jeweils über eine -(CH₂)-, -(CH₂)₂- oder -(CH₂)₃-Gruppe gebunden sein kann; oder für einen Phenyl-, Pyridinyl-, Thiophenyl- oder Furanyl-Rest, der jeweils unsubstituiert oder ggf. mit 1, 2, 3, 4 oder 5 Substituenten unabhängig voneinander ausgewählt aus der Gruppe bestehend aus F, Cl, Br, -CN, -CF₃, Methyl, Ethyl, n-Propyl, iso-Propyl, tert-Butyl, -O-CH₃ und -O-C₂H₅ substituiert und ggf. über eine -(CH₂)-, -(CH₂)₂-, -(CH(CH₃))- oder -(CH₂)₃-Gruppe gebunden ist, stehen,
oder R² und R³ zusammen mit dem sie verbindenden Stickstoffatom als Ringglied einen heterocycloaliphatischen Rest bilden ausgewählt aus der Gruppe bestehend aus Pyrrolidinyl, Piperidinyl, Piperazinyl und Morpholinyl,
R¹² für einen Wasserstoff-Rest oder für einen Alkyl-Rest ausgewählt aus der Gruppe bestehend aus Methyl, Ethyl, n-Propyl, iso-Propyl und tert-Butyl steht;
R¹⁶, R²⁰, R²¹ und R²³, jeweils unabhängig voneinander für einen Alkyl-Rest ausgewählt aus der Gruppe bestehend aus Methyl, Ethyl, n-Propyl, iso-Propyl und tert-Butyl, oder für einen Phenyl-Rest, der jeweils unsubstituiert oder mit 1 oder 2 Substituenten unabhängig voneinander ausgewählt aus der Gruppe bestehend aus F, Cl, Br, Methyl, Ethyl, -O-CH₃ und -O-C₂H₅ substituiert und ggf. über eine -(CH₂)-Gruppe gebunden ist, stehen,
M¹ für einen Rest ausgewählt aus der Gruppe bestehend aus den Resten 1 bis 6, 21, 22, 36 und 37 steht, 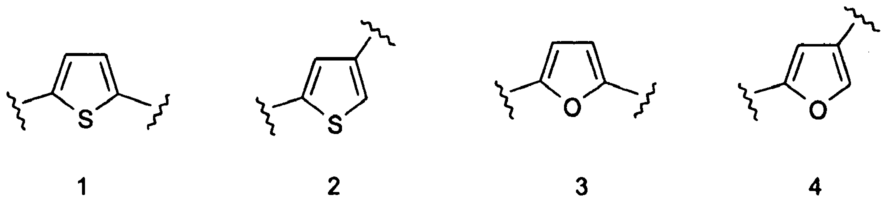 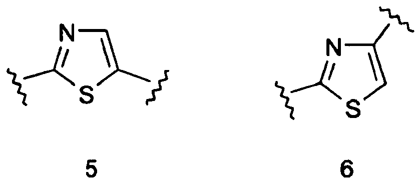 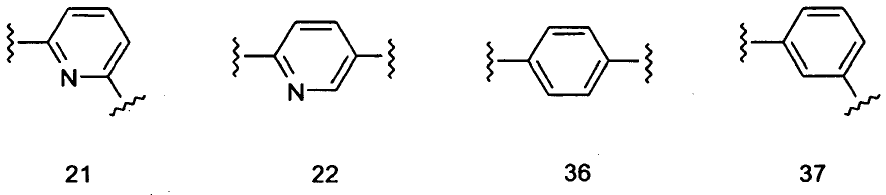
der jeweils in beliebiger Richtung über die mit einer geschlängelten Linie gekennzeichneten Positionen mit dem Bizyklus und dem Kohlenstoffatom der Dreifachbindung verknüpft und ggf. mit 1 oder 2 weiteren Substituenten substituiert ist, die unabhängig voneinander ausgewählt sind aus der Gruppe bestehend aus F, Cl, Br, -CN, -CH₂-CN, -CF₃, -SF₅, Methyl, Ethyl, n-Propyl, iso-Propyl, tert-Butyl, -O-CH₃ und -O-CF₃,
und
M² für einen Rest ausgewählt aus der Gruppe bestehend aus Phenyl, 2-Pyridinyl, 3-Pyridinyl, 4-Pyridinyl, 2-Thiazolyl, 4-Thiazolyl, 5-Thiazolyl, 4-Indolyl, 5-Indolyl, 6-Indolyl, 7-Indolyl, 2-Thiophenyl, 3-Thiophenyl, 2-Imidazolyl, 4-Imidazolyl, 5-Imidazolyl, 2-Pyrimidinyl, 4-Pyrimidinyl, 5-Pyrimidinyl, 2-Pyrazinyl, 5-Chinolinyl, 6-Chinolinyl, 7-Chinolinyl und 8-Chinolinyl steht, wobei der jeweilige Rest unsubstituiert oder ggf. mit 1, 2, 3, 4 oder 5 Substituenten substituiert ist, die unabhängig voneinander ausgewählt sind aus der Gruppe bestehend aus F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, - NH-CH₃, -CH₂-NH₂, -NO₂, -O-CF₃, -C(=O)-H; -C(=O)-OCH₃, -C(=O)-O-C₂H₅, Phenyl, Pyrrolyl, (1,3)-Dioxolanyl, -Si(Phenyl)₂[C(CH₃)₃], NH-S(=O)₂-CH₃, - NH-S(=O)₂-C₂H₅, -S(=O)₂-NH₂, -S(=O)₂-NH-CH₃, -CH₂-OH, -C(=O)-CH₃, - C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

16. Verbindungen der allgemeinen Formel Id gemäß Anspruch 15, 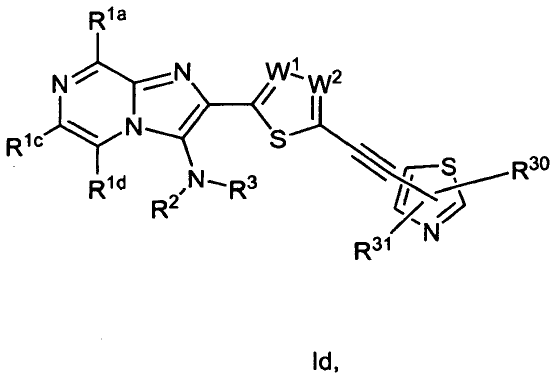 worin
R^{1a}, R^{1c}, R^{1d}, R² und R³ jeweils die Bedeutung gemäß Anspruch 15 haben;
W¹ für C steht und W² für C steht
oder W¹ für C steht und W² für N steht
oder W¹ für N steht und W² für C steht;
und
R³⁰ und R³¹, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, - C(=O)-O-C₂H₅, Phenyl, Pyrrolyl, (1,3)-Dioxolanyl, -Si(Phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, - C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

17. Verbindungen der allgemeinen Formel Ie gemäß Anspruch 15, 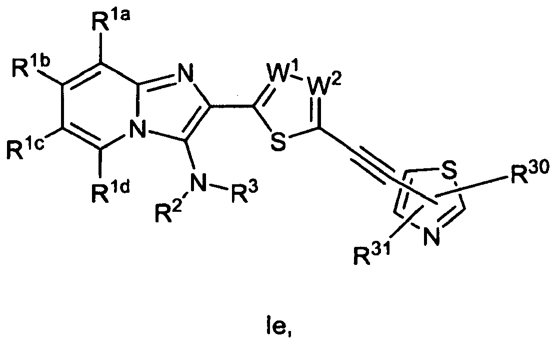 worin
R^{1a}, R^{1b}, R^{1c}, R^{1d}, R² und R³ jeweils die Bedeutung gemäß Anspruch 15 haben;
W¹ für C steht und W² für C steht
oder W¹ für C steht und W² für N steht
oder W¹ für N steht und W² für C steht;
und
R³⁰ und R³¹, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, - C(=O)-O-C₂H₅, Phenyl, Pyrrolyl, (1,3)-Dioxolanyl, -Si(Phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, - C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

18. Verbindungen der allgemeinen Formel If gemäß Anspruch 15, 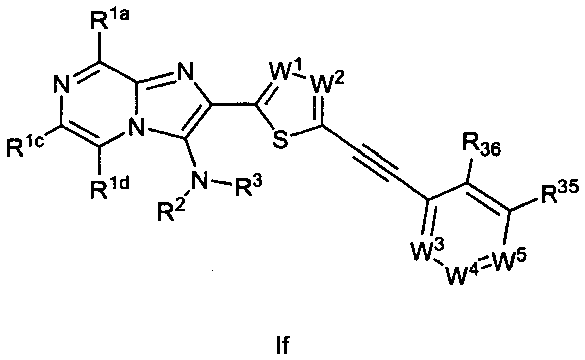 worin
R^{1a}, R^{1c}, R^{1d}, R² und R³ jeweils die Bedeutung gemäß Anspruch 15 haben;
W¹ für C steht und W² für C steht
oder W¹ für C steht und W² für N steht
oder W¹ für N steht und W² für C steht;
W³ für C-R³² steht; W⁴ für C-R³³ steht und W⁵ für C-R³⁴ steht;
oder einer der Reste W³, W⁴ und W⁵ für N steht und die anderen beiden Reste ausgewählt aus der Gruppe bestehend aus W³, W⁴ und W⁵ für C-R³² oder C-R³³ stehen;
und R³², R³³, R³⁴, R³⁵ und R³⁶, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; - C(=O)-OCH₃, -C(=O)-O-C₂H₅, Phenyl, Pyrrolyl, (1,3)-Dioxolanyl, - Si(Phenyl)₂[C(CH₃)₃], NH-S(=O)₂-CH₃, -NH-S(=O)₂-C₂H₅, -S(=O)₂-NH₂, - S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und - S(=O)₂-N(CH₃)₂ stehen;
jeweils ggf. in Form entsprechender Salze, oder jeweils ggf. in Form entsprechender Solvate.

19. Verbindungen der allgemeinen Formel Ig gemäß Anspruch 15, 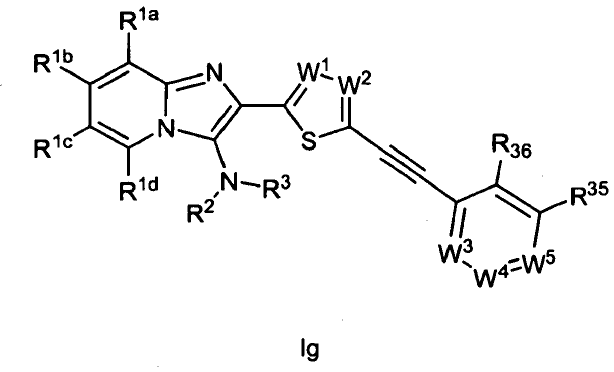 worin
R^{1a}, R^{1b}, R^{1c}, R^{1d}, R² und R³ jeweils die Bedeutung gemäß Anspruch 15 haben;
W¹ für C steht und W² für C steht
oder W¹ für C steht und W² für N steht
oder W¹ für N steht und W² für C steht;
W³ für C-R³² steht; W⁴ für C-R³³ steht und W⁵ für C-R³⁴ steht;
oder einer der Reste W³, W⁴ und W⁵ für N steht und die anderen beiden Reste ausgewählt aus der Gruppe bestehend aus W³, W⁴ und W⁵ für C-R³² oder C-R³³ stehen;
und R³², R³³, R³⁴, R³⁵ und R³⁶, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; - C(=O)-OCH₃, -C(=O)-O-C₂H₅, Phenyl, Pyrrolyl, (1,3)-Dioxolanyl, - Si(Phenyl)₂[C(CH₃)₃], NH-S(=O)₂-CH₃, -NH-S(=O)₂-C₂H₅, -S(=O)₂-NH₂, - S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und - S(=O)₂-N(CH₃)₂ stehen;
jeweils ggf. in Form entsprechender Salze, oder jeweils ggf. in Form entsprechender Solvate.

20. Verbindungen der allgemeinen Formel Ih gemäß Anspruch 15, 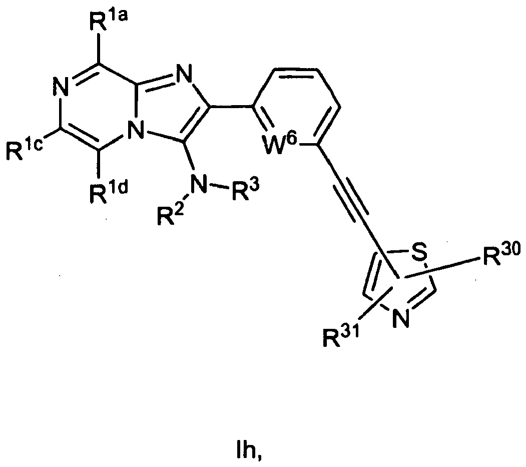 worin
R^{1a}, R^{1c}, R^{1d}, R² und R³ jeweils die Bedeutung gemäß Anspruch 15 haben;
W⁶ für C oder N steht;
und
R³⁰ und R³¹, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, - C(=O)-O-C₂H₅, Phenyl, Pyrrolyl, (1,3)-Dioxolanyl, -Si(Phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, - C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

21. Verbindungen der allgemeinen Formel Ik gemäß Anspruch 15, 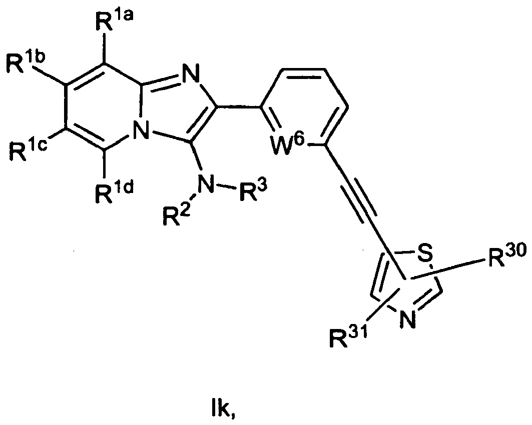 worin
R^{1a}, R^{1b}, R^{1c}, R^{1d}, R² und R³ jeweils die Bedeutung gemäß Anspruch 15 haben;
W⁶ für C oder N steht;
und
R³⁰ und R³¹, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, - C(=O)-O-C₂H₅, Phenyl, Pyrrolyl, (1,3)-Dioxolanyl, -Si(Phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, - C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und -S(=O)₂-N(CH₃)₂ stehen;
jeweils ggf. in Form entsprechender Salze, oder jeweils ggf. in Form entsprechender Solvate.

22. Verbindungen der allgemeinen Formel Im gemäß Anspruch 15, 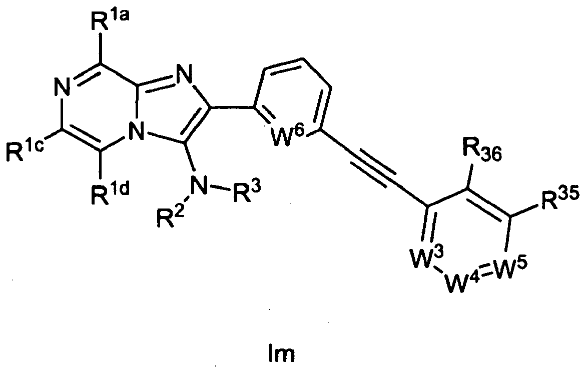 worin
R^{1a}, R^{1c}, R^{1d}, R² und R³ jeweils die Bedeutung gemäß Anspruch 15 haben;
W⁶ für C oder N steht;
W³ für C-R³² steht; W⁴ für C-R³³ steht und W⁵ für C-R³⁴ steht;
oder einer der Reste W³, W⁴ und W⁵ für N steht und die anderen beiden Reste ausgewählt aus der Gruppe bestehend aus W³, W⁴ und W⁵ für C-R³² oder C-R³³ stehen;
und R³², R³³, R³⁴, R³⁵ und R³⁶, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; - C(=O)-OCH₃, -C(=O)-O-C₂H₅, Phenyl, Pyrrolyl, (1,3)-Dioxolanyl, - Si(Phenyl)₂₁C(CH₃)₃], NH-S(=O)₂-CH₃, -NH-S(=O)₂-C₂H₅, -S(=O)₂-NH₂, - S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und - S(=O)₂-N(CH₃)₂ stehen;
jeweils ggf. in Form entsprechender Salze, oder jeweils ggf. in Form entsprechender Solvate.

23. Verbindungen der allgemeinen Formel In gemäß Anspruch 15, 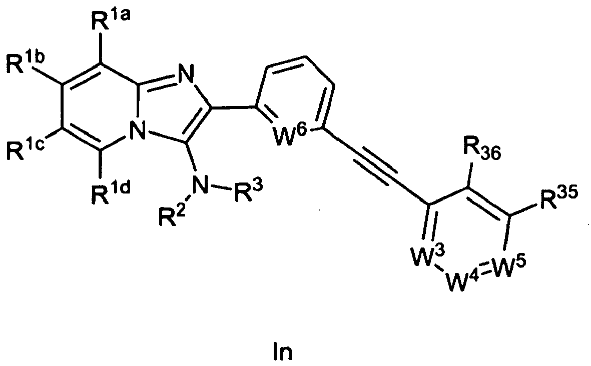 worin
R^{1a}, R^{1b}, R^{1c}, R^{1d}, R² und R³ jeweils die Bedeutung gemäß Anspruch 15 haben;
W⁶ für C oder N steht;
W³ für C-R³² steht; W⁴ für C-R³³ steht und W⁵ für C-R³⁴ steht;
oder einer der Reste W³, W⁴ und W⁵ für N steht und die anderen beiden Reste ausgewählt aus der Gruppe bestehend aus W³, W⁴ und W⁵ für C-R³² oder C-R³³ stehen;
und R³², R³³, R³⁴, R³⁵ und R³⁶, unabhängig voneinander, jeweils für einen Rest ausgewählt aus der Gruppe bestehend aus H, F, Cl, Br, -CN, -CF₃, -SF₅, -S-CH₃, -S-C₂H₅, Methyl, Ethyl, iso-Propyl, tert-Butyl, Ethenyl, Propenyl, -OH, -O-CH₃, -O-C₂H₅, -NH₂, -N(CH₃)₂, -NH-CH₃, -CH₂-NH₂, -NO₂, -O-CF₃, -C(=O)-H; - C(=O)-OCH₃, -C(=O)-O-C₂H₅, Phenyl, Pyrrolyl, (1,3)-Dioxolanyl, - Si(Phenyl)₂[C(CH₃)₃], NH-S(=O)₂-CH₃, -NH-S(=O)₂-C₂H₅, -S(=O)₂-NH₂, - S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH und - S(=O)₂-N(CH₃)₂ stehen;
jeweils ggf. in Form entsprechender Salze, oder jeweils ggf. in Form entsprechender Solvate.

24. Verbindungen gemäß einem oder mehreren der Ansprüche 1 bis 23 ausgewählt aus der Gruppe bestehend aus
[1] Cyclopentyl-[2-(5-phenylethinyl-thiophen-2-yl)-imidazo[1,2-a]pyrazin-3-yl]-amin,
[2] Cyclohexylmethyl-[5-methyl-2-(5-phenylethinyl-thiophen-2-yl)-imidazo[1,2-a]pyridin-3-yl]-amin,
[3] Cyclohexylmethyl-[6-methyl-2-(5-phenylethinyl-thiophen-2-yl)-imidazo[1,2-a]pyridin-3-yl]-amin,
[4] Cyclohexylmethyl-[7-methyl-2-(5-phenylethinyl-thiophen-2-yl)-imidazo[1,2-a]pyridin-3-yl]-amin,
[5] Cyclohexylmethyl-[8-methyl-2-(5-phenylethinyl-thiophen-2-yl)-imidazo[1,2-a]pyridin-3-yl]-amin,
[6] Cyclohexylmethyl-[5,7-dimethyl-2-(5-phenylethinyl-thiophen-2-yl)-imidazo[1,2-a]pyridin-3-yl]-amin,
[7] [8-Benzyloxy-2-(5-phenylethinyl-thiophen-2-yl)-imidazo[1,2-a]pyridin-3-yl]-cyclohexylmethyl-amin,
[8] Cyclohexylmethyl-[2-(5-phenylethinyl-thiophen-2-yl)-imidazo[1,2-a]pyrazin-3-yl]-amin,
[9] Cyclohexylmethyl-[2-(5-phenylethinyl-thiophen-2-yl)-imidazo[1,2-a]pyrimidin-3-yl]-amin,
[10] (4-Methoxy-benzyl)-[5-methyl-2-(5-phenylethinyl-thiophen-2-yl)-imidazo[1,2-a]pyridin-3-yl]-amin,
[11] (4-Methoxy-benzyl)-[6-methyl-2-(5-phenylethinyl-thiophen-2-yl)-imidazo[1,2-a]pyridin-3-yl]-amin,
[12] (4-Methoxy-benzyl)-[7-methyl-2-(5-phenylethinyl-thiophen-2-yl)-imidazo[1,2-a]pyridin-3-yl]-amin,
[13] (4-Methoxy-benzyl)-[8-methyl-2-(5-phenylethinyl-thiophen-2-yl)-imidazo[1,2-a]pyridin-3-yl]-amin,
[14] [5,7-Dimethyl-2-(5-phenylethinyl-thiophen-2-yl)-imidazo[1,2-a]pyridin-3-yl]-(4-methoxy-benzyl)-amin,
[15] [8-Benzyloxy-2-(5-phenylethinyl-thiophen-2-yl)-imidazo[1,2-a]pyridin-3-yl]-(4-methoxy-benzyl)-amin,
[16] (4-Methoxy-benzyl)-[2-(5-phenylethinyl-thiophen-2-yl)-imidazo[1,2-a]pyridin-3-yl]-amin,
[17] (4-Methoxy-benzyl)-[2-(5-phenylethinyl-thiophen-2-yl-imidazo[1,2-a]pyrazin-3-yl]-amin,
[18] tert-Butyl-[2-(5-phenylethinyl-thiophen-2-yl)-imidazo[1,2-a]pyrazin-3-yl]-amin,
[19] tert-Butyl-[6-methyl-2-(5-phenylethinyl-thiophen-2-yl)-imidazo[1,2-a]pyridin-3-yl]-amin,
[20] [8-Benzyloxy-2-(5-phenylethinyl-thiophen-2-yl)-imidazo[1,2-a]pyridin-3-yl]-(3-methoxy-phenyl)-amin,
[21] (3-Methoxy-benzyl)-[5-methyl-2-(5-phenylethinyl-thiophen-2-yl)-imidazo[1,2-a]pyridin-3-yl]-amin,
[22] (3-Methoxy-benzyl)-[8-methyl-2-(5-phenylethinyl-thiophen-2-yl)-imidazo[1,2-a]pyridin-3-yl]-amin,
[23] [5,7-Dimethyl-2-(5-phenylethinyl-thiophen-2-yl)-imidazo[1,2-a]pyridin-3-yl]-(3-methoxy-benzyl)-am in,
[24] [8-Benzyloxy-2-(5-phenylethinyl-thiophen-2-yl)-imidazo[1,2-a]pyridin-3-yl]-(3-methoxy-benzyl)-amin,
[25] (3-Methoxy-benzyl)-[2-(5-phenylethinyl-thiophen-2-yl)-imidazo[1,2-a]pyridin-3-yl]-amin,
[26] (3-Methoxy-benzyl)-[2-(5-phenylethinyl-thiophen-2-yl)-imidazo[1,2-a]pyrazin-3-yl]-amin,
[27] [6-Methyl-2-(5-phenylethinyl-thiophen-2-yl)-imidazo[1,2-a]pyridin-3-yl]-(1-phenyl-ethyl)-amin,
[28] [7-Methyl-2-(5-phenylethinyl-thiophen-2-yl)-imidazo[1,2-a]pyridin-3-yl]-(1-phenyl-ethyl)-amin,
[29] [5,7-Dimethyl-2-(5-phenylethinyl-thiophen-2-yl)-imidazo[1,2-a]pyridin-3-yl]-(1-phenyl-ethyl)-amin,
[30] [8-Benzyloxy-2-(5-phenylethinyl-thiophen-2-yl)-imidazo[1,2-a]pyridin-3-yl]-(1-phenyl-ethyl)-amin,
[31] (1-Phenyl-ethyl)-[2-(5-phenylethinyl-thiophen-2-yl)-imidazo[1,2-a]pyrazin-3-yl]-amin,
[32] (2-Chlor-benzyl)-[5,7-dimethyl-2-(5-phenylethinyl-thiophen-2-yl)-imidazo[1,2-a]pyridin-3-yl]-amin,
[33] (3-Chlor-4-fluor-phenyl)-[7-phenyl-2-(5-phenylethinyl-thiophen-2-yl)-imidazo[1,2-a]pyridin-3-yl]-amin,
[34] (4-Methoxy-benzyl)-[7-methyl-2-(5-phenylethinyl-thiophen-2-yl)-imidazo[1,2-a]pyrimidin-3-yl]-amin,
[35] [8-Methyl-2-(5-phenylethinyl-thiophen-2-yl)-imidazo[1,2-a]pyridin-3-yl]-(1-phenyl-ethyl)-amin,
[36] [7-Methyl-2-(5-phenylethinyl-thiophen-2-yl)-imidazo[1,2-a]pyrimidin-3-yl]-(1-phenyl-ethyl)-amin,
[37] (2-Methoxy-benzyl)-[7-methyl-2-(5-phenylethinyl-thiophen-2-yl)-imidazo[1,2-a]pyridin-3-yl]-amin,
[38] (2-Methoxy-benzyl)-[2-(5-phenylethinyl-thiophen-2-yl)-imidazo[1,2-a]pyridin-3-yl]-amin,
[39] (2-Chlor-benzyl)-[2-(5-phenylethinyl-thiophen-2-yl)-imidazo[1,2-a]pyridin-3-yl]-amin,
[40] (2-Methoxy-benzyl)-[7-methyl-2-(5-phenylethinyl-thiophen-2-yl)-imidazo[1,2-a]pyrimidin-3-yl]-amin,
[41] (3-Methoxy-phenyl)-[6-methyl-2-(5-phenylethinyl-thiophen-2-yl)-imidazo[1,2-a]pyridin-3-yl]-amin,
[42] [5,7-Dimethyl-2-(5-phenylethinyl-thiophen-2-yl)-imidazo[1,2-a]pyridin-3-yl]-(3-methoxy-phenyl)-amin,
[43] (3-Methoxy-phenyl)-[2-(5-phenylethinyl-thiophen-2-yl)-imidazo[1,2-a]pyrazin-3-yl]-amin,
[44] [7-Ethyl-2-(5-phenylethinyl-thiophen-2-yl)-imidazo[1,2-a]pyridin-3-yl]-(2-methoxy-benzyl)-amin,
[45] (2-Chlor-benzyl)-[7-ethyl-2-(5-phenylethinyl-thiophen-2-yl)-imidazo[1,2-a]pyridin-3-yl]-amin,
[46] [7-tert-Butyl-2-(5-phenylethinyl-thiophen-2-yl)-imidazo[1,2-a]pyridin-3-yl]-(3-chlor-4-fluor-phenyl)-amin,
[47] (3-Methoxy-benzyl)-[7-methyl-2-(5-phenylethinyl-thiophen-2-yl)-imidazo[1,2-a]pyrimidin-3-yl]-amin,
[48] [7-Ethyl-2-(5-phenylethinyl-thiophen-2-yl)-imidazo[1,2-a]pyridin-3-yl]-(2-fluor-phenyl)-amin,
[49] [7-tert-Butyl-2-(5-phenylethinyl-thiophen-2-yl)-imidazo[1,2-a]pyridin-3-yl]-(2-fluor-phenyl)-amin,
[50] (2,4-Difluor-phenyl)-[2-(5-phenylethinyl-thiophen-2-yl)-7-(3-phenyl-propyl)-imidazo[1,2-a]pyridin-3-yl]-amin,
[51] (4-Fluor-benzyl)-[7-methyl-2-(5-phenylethinyl-thiophen-2-yl)-imidazo[1,2-a]pyridin-3-yl]-amin,
[52] [5,7-Dimethyl-2-(5-phenylethinyl-thiophen-2-yl)-imidazo[1,2-a]pyridin-3-yl]-(4-fluor-benzyl)-amin,
[53] [7-Ethyl-2-(5-phenylethinyl-thiophen-2-yl)-imidazo[1,2-a]pyridin-3-yl]-(3-trifluormethyl-benzyl)-amin,
[54] [7-Isopropyl-2-(5-phenylethinyl-thiophen-2-yl)-imidazo[1,2-a]pyridin-3-yl]-(3-trifluormethyl-benzyl)-amin,
[55] tert-Butyl-[2-(4-phenylethinyl-thiophen-2-yl)-imidazo[1,2-a]pyrazin-3-yl]-amin,
[56] [2-(5-Phenylethinyl-thiophen-2-yl)-imidazo[1,2-a]pyrazin-3-yl]-(1,1,3,3-tetramethyl-butyl)-amin,
[57] Butyl-[2-(4-phenylethinyl-thiophen-2-yl)-imidazo[1,2-a]pyrazin-3-yl]-amin,
[59] [2-(5-Phenylethinyl-thiophen-2-yl)-imidazo[1,2-a]pyridin-3-yl]-(1,1,3,3-tetramethyl-butyl)-amin,
[60] [2-(5-Pyridinyl-thiophen-2-yl)-imidazo[1,2-a]pyrazin-3-yl]-(1,1,3,3-tetramethyl-butyl)-amin,
[61] [2-(5-Pyridin-4-ylethinyl-thiophen-2-yl)-imidazo[1,2-a]pyrazin-3-yl]-(1,1,3,3-tetramethyl-butyl)-amin,
[62] [6-Chlor-2-(5-phenylethinyl-thiophen-2-yl)-imidazo[1,2-a]pyridin-3-yl]-(1,1,3,3-tetramethyl-butyl)-amin,
[63] [6,8-Dichlor-2-(5-phenylethinyl-thiophen-2-yl)-imidazo[1,2-a]pyridin-3-yl]-(1,1,3,3-tetramethyl-butyl)-amin,
[64] [2-(5-Pyridin-2-ylethinyl-thiophen-2-yl)-imidazo[1,2-a]pyrazin-3-yl]-(1,1,3,3-tetramethyl-butyl)-amin,
[65] Dimethyl-[2-(5-phenylethinyl-thiophen-2-yl)-imidazo[1,2-a]pyrazin-3-yl]-amin,
[66] Methyl-[2-(5-phenylethinyl-thiophen-2-yl)-imidazo[1,2-a]pyrazin-3-yl]-amin,
[67] N-[2-(5-Phenylethinyl-thiophen-2-yl)-imidazo[1,2-a]pyrazin-3-yl]-acetamid,
[68] Ethyl-[2-(5-phenylethinyl-thiophen-2-yl)-imidazo[1,2-a]pyrazin-3-yl]-amin,
[69] Propyl-[2-(5-phenylethinyl-thiophen-2-yl)-imidazo[1,2-a]pyrazin-3-yl]-amin,
[70] Butyl-[2-(5-phenylethinyl-thiophen-2-imidazo[1,2-a]pyrazin-3-yl]-amin,
[71] (2-Methylpropyl)-[2-(5-phenylethinyl-thiophen-2-yl)-imidazo[1,2-a]pyrazin-3-yl]-amin,
[72] Pentyl-[2-(5-phenylethinyl-thiophen-2-yl)-imidazo[1,2-a]pyrazin-3-yl]-amin,
[73] {(Methoxycarbonylmethyl)-[2-(5-phenylethynyl-thiophen-2-yl)-imidazo[1,2-a]pyrazin-3-yl]-amino}-essigsäuremethylester,
[74] Benzyl-[2-(5-phenylethynyl-thiophen-2-yl)-imidazo[1,2-a]pyrazin-3-yl]-amin,
[75] [2-(5-Phenylethynyl-thiophen-2-yl)-imidazo[1,2-a]pyrazin-3-ylamino]-essigsäuremethylester,
[76] tert-Butyl-[2-(5-pyridin-4-ylethinyl-thiophen-2-yl)-imidazo[1,2-a]pyrazin-3-yl]-amin,
[77] tert-Butyl-[2-(5-pyridin-2-ylethinyl-thiophen-2-yl)-imidazo[1,2-a]pyrazin-3-yl]-amin,
[78] N-[2-(5-Phenylethinyl-thiophen-2-yl)-imidazo[1,2-a]pyrazin-3-yl]-benzamid,
[79] [2-(5-Phenylethinyl-thiophen-2-yl)-imidazo[1,2-a]pyrazin-3-yl]-bis-pyridin-3-ylmethyl-amin,
[80] 2,2-Dimethyl-N-[2-(5-phenylethinyl-thiophen-2-yl)-imidazo[1,2-a]pyrazin-3-yl]-propionamid,
[81] 3-Methoxy-N-[2-(5-phenylethinyl-thiophen-2-yl)-imidazo[1,2-a]pyrazin-3-yl]-benzamid,
[82] tert-Butyl-[2-(5-pyridin-3-ylethinyl-thiophen-2-yl)-imidazo[1,2-a]pyrazin-3-yl]-amin [83] 2-(5-Phenylethinyl-thiophen-2-yl)-imidazo[1,2-a]pyrazin-3-ylamin
[84] Methyl-2-(5-(phenylethinyl)thiophen-2-yl)-3-(2,4,4-trimethylpentan-2-ylamino)imidazo[1,2-a]pyrazin-8-carboxylat
[85] 2-(5-(Phenylethinyl)thiophen-2-yl)-N-(2,4,4-trimethylpentan-2-yl)imidazo[1,2-a]pyrimidin-3-amin
[86] 2-(5-((6-Methylpyridin-2-yl)ethinyl)thiophen-2-yl)-N-(2,4,4-trimethylpentan-2-yl)imidazo[1,2-a]pyrazin-3-amin
[87] N-Cyclohexyl-2-(5-(phenylethinyl)thiophen-2-yl)imidazo[1,2-a]pyrazin-3-amin
[88] 2-(5-(Phenylethinyl)thiophen-2-yl)-3-(piperidin-1-yl)imidazo[1,2-a]pyrazin
[89] N-tert-Butyl-N-methyl-2-(5-(phenylethinyl)thiophen-2-yl)imidazo[1,2-a]pyrazin-3-amin
[90] Methyl-2-(5-(phenylethinyl)thiophen-2-yl)-3-(2,4,4-trimethylpentan-2-ylamino)imidazo[1,2-a]pyridin-6-carboxylat
[91] N-tert-Butyl-2-(5-(pyridin-2-ylethinyl)thiophen-2-yl)imidazo[1,2-a]pyrazin-3-amin Hydrochlorid
[92] 8-Brom-N-cyclopentyl-6-methyl-2-(5-(pyridin-2-ylethinyl)thiophen-2-yl)imidazo[1,2-a]pyridin-3-amin
[93] N,N-Diethyl-2-(5-(phenylethinyl)thiophen-2-yl)imidazo[1,2-a]pyrazin-3-amin
[94] N-tert-Butyl-2-(5-(pyridin-2-ylethinyl)thiophen-2-yl)imidazo[1,2-a]pyridin-3-amin
[95] N-tert-Butyl-2-(5-(phenylethinyl)furan-2-yl)imidazo[1,2-a]pyrazin-3-amin
[96] 8-Brom-N-tert-butyl-6-methyl-2-(3-(pyridin-2-ylethinyl)phenyl)imidazo[1,2-a]pyridin-3-amin
[97] N-tert-Butyl-8-methyl-2-(5-(phenylethinyl)thiophen-2-yl)imidazo[1,2-a]pyridin-3-amin
[98] N-Methyl-2-(5-(phenylethinyl)thiophen-2-yl)-N-(2,4,4-trimethylpentan-2-yl)imidazo[1,2-a]pyrazin-3-amin Hydrochlorid
[99] 2-(5-(Phenylethinyl)thiophen-2-yl)-3-(pyrrolidin-1-yl)imidazo[1,2-a]pyrazin Hydrochlorid
[100] N-tert-Butyl-2-(5-((4-fluorphenyl)ethinyl)thiophen-2-yl)imidazo[1,2-a]pyrazin-3-amin Hydrochlorid
[101] N-tert-Butyl-7-methyl-2-(5-(phenylethinyl)thiophen-2-yl)imidazo[1,2-a]pyridin-3-amin
[102] N-tert-Butyl-5-methyl-2-(5-(phenylethinyl)thiophen-2-yl)imidazo[1,2-a]pyridin-3-amin
[103] 8-Chlor-2-(3-(pyridin-2-ylethinyl)phenyl)-6-(trifluormethyl)-N-(2,4,4-trimethylpentan-2-yl)imidazo[1,2-a]pyridin-3-amin
[104] N-tert-Butyl-2-(5-((3-fluorphenyl)ethinyl)thiophen-2-yl)imidazo[1,2-a]pyrazin-3-amin Hydrochlorid
[105] N-tert-Butyl-2-(5-((2-fluorphenyl)ethinyl)thiophen-2-yl)imidazo[1,2-a]pyrazin-3-amin Hydrochlorid
[106] Methyl-3-(tert-butylamino)-2-(5-(phenylethinyl)thiophen-2-yl)imidazo[1,2-a]pyrazin-8-carboxylat
[107] N-tert-Butyl-2-(5-(pyrazin-2-ylethinyl)thiophen-2-yl)imidazo[1,2-a]pyrazin-3-amin
[108] 2-(5-((4-Aminophenyl)ethinyl)thiophen-2-yl-N-tert-butylimidazo[1,2-a]pyrazin-3-amin
[109] N-Isopropyl-2-(5-(phenylethinyl)thiophen-2-yl)imidazo[1,2-a]pyrazin-3-amin
[110] N-tert-Butyl-2-(5-(thiophen-2-ylethinyl)thiophen-2-yl)imidazo[1,2-a]pyrazin-3-amin Hydrochlorid
[111] N-tert-Butyl-2-(3-(pyridin-2-ylethinyl)phenyl)imidazo[1,2-a]pyrazin-3-amin
[112] N-tert-Butyl-2-(5-((2-methoxyphenyl)ethinyl)thiophen-2-yl)imidazo[1,2-a]pyrazin-3-amin Hydrochlorid
[113] N-tert-Butyl-2-(5-((3-methoxyphenyl)ethinyl)thiophen-2-yl)imidazo[1,2-a]pyrazin-3-amin Hydrochlorid
[114] N-tert-Butyl-2-(5-((4-methoxyphenyl)ethinyl)thiophen-2-yl)imidazo[1,2-a]pyrazin-3-amin Hydrochlorid
[115] N-tert-Butyl-2-(5-(phenylethinyl)thiophen-2-yl)imidazo[1,2-a]chinolin-1-amin
[116] N-tert-Butyl-2-(4-(pyridin-2-ylethinyl)phenyl)imidazo[1,2-a]pyrazin-3-amin
[117] N-tert-Butyl-2-(3-((6-methylpyridin-2-yl)ethinyl)phenyl)imidazo[1,2-a]pyrazin-3-amin
[118] N-tert-Butyl-2-(2-methyl-6-(phenylethinyl)pyridin-3-yl)imidazo[1,2-a]pyrazin-3-amin
[119] N-tert-Butyl-2-(5-(pyridin-2-ylethinyl)furan-2-yl)imidazo[1,2-a]pyrazin-3-amin
[120] 3-(tert-Butylamino)-2-(5-(phenylethinyl)thiophen-2-yl)imidazo[1,2-a]pyrazin-8-carbosäure
[121] 4-((5-(3-(tert-Butylamino)imidazo[1,2-a]pyrazin-2-yl)thiophen-2-yl)ethinyl)phenol Hydrochlorid
[122] 3-((5-(3-(tert-Butylamino)imidazo[1,2-a]pyrazin-2-yl)thiophen-2-yl)ethinyl)phenol
[123] 2-(5-((3-Aminophenyl)ethinyl)thiophen-2-yl)-N-tert-butylimidazo[1,2-a]pyrazin-3-amin Hydrochlorid
[124] 2-(5-((2-Aminophenyl)ethinyl)thiophen-2-yl)-N-tert-butylimidazo[1,2-a]pyrazin-3-amin Hydrochlorid
[125] N-tert-Butyl-2-(5-(phenylethinyl)thiophen-2-yl)imidazo[2,1-a]isochinolin-3-amin
[126] N-tert-Butyl-2-(6-(phenylethinyl)pyridin-3-yl)imidazo[1,2-a]pyrazin-3-amin
[127] N-tert-Butyl-2-(5-(pyridin-4-ylethinyl)thiophen-2-yl)imidazo[1,2-a]pyrazin-3-amin Hydrochlorid
[128] 2-(5-((6-Aminopyridin-3-yl)ethinyl)thiophen-2-yl)-N-tert-butylimidazo[1,2-a]pyrazin-3-amin Hydrochlorid
[129] N-tert-Butyl-2-(5-(pyrimidin-2-ylethinyl)thiophen-2-yl)imidazo[1,2-a]pyrazin-3-amin Hydrochlorid
[130] N-tert-Butyl-2-(5-((4-methylpyridin-2-yl)ethinyl)thiophen-2-yl)imidazo[1,2-a]pyrazin-3-amin Hydrochlorid
[131] N-tert-Butyl-2-(5-((5-methylpyridin-2-yl)ethinyl)thiophen-2-yl)imidazo[1,2-a]pyrazin-3-amin Hydrochlorid
[132] N-tert-Butyl-2-(5-(pyridin-4-ylethinyl)thiophen-2-yl)imidazo[1,2-a]pyridin-3-amin Hydrochlorid
[133] N-tert-Butyl-2-(5-(thiazol-2-ylethinyl)thiophen-2-yl)imidazo[1,2-a]pyrazin-3-amin
[134] 2-(5-(Pyridin-2-ylethinyl)thiophen-2-yl)imidazo[1,2-a]pyrazin-3-amin Hydrochlorid
[135] N-tert-Butyl-2-(5-((5-methylthiophen-2-yl)ethinyl)thiophen-2-yl)imidazo[1,2-a]pyrazin-3-amin Hydrochlorid
[136] 2-(5-((6-Aminopyridin-2-yl)ethinyl)thiophen-2-yl)-N-tert-butylimidazo[1,2-a]pyrazin-3-amin Hydrochlorid
[137] N-tert-Butyl-2-(5-((3-methylthiophen-2-yl)ethinyl)thiophen-2-yl)imidazo[1,2-a]pyrazin-3-amin
[138] N-tert-Butyl-2-(4-(phenylethinyl)thiazol-2-yl)imidazo[1,2-a]pyrazin-3-amin
[139] N-tert-Butyl-2-(5-(m-tolylethinyl)thiophen-2-yl)imidazo[1,2-a]pyrazin-3-amin
[140] 3-((5-(3-(tert-Butylamino)imidazo[1,2-a]pyrazin-2-yl)thiophen-2-yl)ethinyl)benzonitril Hydrochlorid
[141] N-tert-Butyl-2-(5-(pyridin-2-ylethinyl)thiophen-2-yl)imidazo[1,2-a]pyrimidin-3-amin
[142] N-tert-Butyl-2-(6-(phenylethinyl)pyridin-2-yl)imidazo[1,2-a]pyrazin-3-amin Hydrochlorid
[143] N-tert-Butyl-N-methyl-2-(5-(pyridin-2-ylethinyl)thiophen-2-yl)imidazo[1,2-a]pyrazin-3-amin
[144] 4-((5-(3-(tert-Butylamino)imidazo[1,2-a]pyrazin-2-yl)thiophen-2-yl)ethinyl)benzonitril Hydrochlorid
[145] 2-(5-((1H-Indol-6-yl)ethinyl)thiophen-2-yl)-N-tert-butylimidazo[1,2-a]pyrazin-3-amin Hydrochlorid
[146] N-tert-Butyl-2-(2-(phenylethinyl)thiazol-5-yl)imidazo[1,2-a]pyrazin-3-amin
[147] N-tert-Butyl-2-(5-(chinolin-6-ylethinyl)thiophen-2-yl)imidazo[1,2-a]pyrazin-3-amin Hydrochlorid
[148] 2-(5-((3-(1H-Pyrrol-1-yl)phenyl)ethinyl)thiophen-2-yl)-N-tert-butylimidazo[1,2-a]pyrazin-3-amin Hydrochlorid
[149] 2-(5-((1H-Indol-4-yl)ethinyl)thiophen-2-yl)-N-tert-butylimidazo[1,2-a]pyrazin-3-amin Hydrochlorid
[150] N-tert-Butyl-2-(5-((3-nitrophenyl)ethinyl)thiophen-2-yl)imidazo[1,2-a]pyrazin-3-amin Hydrochlorid
[151] N-tert-Butyl-2-(5-((4-nitrophenyl)ethinyl)thiophen-2-yl)imidazo[1,2-a]pyrazin-3-amin Hydrochlorid
[152] N-tert-Butyl-2-(5-(thiazol-4-ylethinyl)thiophen-2-yl)imidazo[1,2-a]pyrazin-3-amin Hydrochlorid
[153] 2-((5-(3-(tert-Butylamino)imidazo[1,2-a]pyrazin-2-yl)thiophen-2-yl)ethinyl)phenol
[154] 2-(5-((3-(Aminomethyl)phenyl)ethinyl)thiophen-2-yl)-N-tert-butylimidazo[1,2-a]pyrazin-3-amin
[155] 2-(5-(Biphenyl-3-ylethinyl)thiophen-2-yl)-N-tert-butylimidazo[1,2-a]pyrazin-3-amin Hydrochlorid
[156] N-tert-Butyl-2-(5-(thiophen-3-ylethinyl)thiophen-2-yl)imidazo[1,2-a]pyrazin-3-amin Hydrochlorid
[157] N-tert-Butyl-2-(5-((3-(dimethylamino)phenyl)ethinyl)thiophen-2-yl)imidazo[1,2-a]pyrazin-3-amin Hydrochlorid
[158] N-tert-Butyl-2-(5-((6-methylpyridin-2-yl)ethinyl)thiophen-2-yl)imidazo[1,2-a]pyrazin-3-amin Hydrochlorid
[159] N-tert-Butyl-2-(5-((3-fluorpyridin-2-yl)ethinyl)thiophen-2-yl)imidazo[1,2-a]pyrazin-3-amin Hydrochlorid
[160] N-tert-Butyl-2-(5-((3-(methylamino)phenyl)ethinyl)thiophen-2-yl)imidazo[1,2-a]pyrazin-3-amin Hydrochlorid
[161] N-tert-Butyl-2-(5-(p-tolylethinyl)thiophen-2-yl)imidazo[1,2-a]pyrazin-3-amin
[162] N-tert-Butyl-2-(5-(o-tolylethinyl)thiophen-2-yl)imidazo[1,2-a]pyrazin-3-amin Hydrochlorid
[163] N-tert-Butyl-2-(4-methyl-5-(phenylethinyl)thiophen-2-yl)imidazo[1,2-a]pyrazin-3-amin Hydrochlorid
[164] N-tert-Butyl-2-(4-methyl-5-(pyridin-2-ylethinyl)thiophen-2-yl)imidazo[1,2-a]pyrazin-3-amin Hydrochlorid
[165] N-tert-Butyl-2-(5-((6-fluorpyridin-2-yl)ethinyl)thiophen-2-yl)imidazo[1,2-a]pyrazin-3-amin
[166] N-tert-Butyl-2-(5-((2-nitrophenyl)ethinyl)thiophen-2-yl)imidazo[1,2-a]pyrazin-3-amin
[167] N-tert-Butyl-8-chlor-2-(5-(pyridin-2-ylethinyl)thiophen-2-yl)imidazo[1,2-a]pyrazin-3-amin
[168] N-tert-Butyl-2-(5-((6-methoxypyridin-2-yl)ethinyl)thiophen-2-yl)imidazo[1,2-a]pyrazin-3-amin
[169] N-tert-Butyl-2-(5-((5-fluorpyridin-2-yl)ethinyl)thiophen-2-yl)imidazo[1,2-a]pyrazin-3-amin Hydrochlorid
[170] 2-(4-(3-(tert-Butylamino)imidazo[1,2-a]pyrazin-2-yl)-2-(phenylethinyl)phenyl)acetonitril
[171] N-tert-Butyl-2-(5-((5-methoxypyridin-3-yl)ethinyl)thiophen-2-yl)imidazo[1,2-a]pyrazin-3-amin Hydrochlorid
[172] 5-((5-(3-(tert-Butylamino)imidazo[1,2-a]pyrazin-2-yl)thiophen-2-yl)ethinyl)nicotinonitril Hydrochlorid
[173] N-tert-Butyl-2-(5-((3-(methylthio)phenyl)ethinyl)thiophen-2-yl)imidazo[1,2-a]pyrazin-3-amin
[174] Methyl-3-((5-(3-(tert-Butylamino)imidazo[1,2-a]pyrazin-2-yl)thiophen-2-yl)ethinyl)benzoate
[175] N-tert-Butyl-2-(5-((3,5-difluorpyridin-2-yl)ethinyl)thiophen-2-yl)imidazo[1,2-a]pyrazin-3-amin
[176] N-tert-Butyl-2-(5-(phenylethinyl)thiazol-2-yl)imidazo[1,2-a]pyrazin-3-amin
[177] N-tert-Butyl-2-(2-(pyridin-4-ylethinyl)thiazol-5-yl)imidazo[1,2-a]pyrazin-3-amin
[178] 3-((5-(3-(tert-Butylamino)imidazo[1,2-a]pyrazin-2-yl)thiophen-2-yl)ethinyl)benzaldehyd
[179] 3-((5-(3-(tert-Butylamino)imidazo[1,2-a]pyrazin-2-yl)thiophen-2-yl)ethinyl)-4-fluorbenzonitril
[180] N-tert-Butyl-2-(5-((3-(trifluormethyl)phenyl)ethinyl)thiophen-2-yl)imidazo[1,2-a]pyrazin-3-amin Hydrochlorid
[181] N-tert-Butyl-2-(2-(pyridin-2-ylethinyl)thiazol-5-yl)imidazo[1,2-a]pyrazin-3-amin
[182] N-tert-Butyl-2-(3-methyl-5-(phenylethinyl)thiophen-2-yl)imidazo[1,2-a]pyrazin-3-amin Hydrochlorid
[183] N-tert-Butyl-2-(3-methyl-5-(pyridin-2-ylethinyl)thiophen-2-yl)imidazo[1,2-a]pyrazin-3-amin Hydrochlorid
[184] N-tert-Butyl-2-(5-((3-vinylphenyl)ethinyl)thiophen-2-yl)imidazo[1,2-a]pyrazin-3-amin
[185] 2-(5-((1H-Imidazol-4-yl)ethinyl)thiophen-2-yl)-N-tert-butylimidazo[1,2-a]pyrazin-3-amin Hydrochlorid
[186] N-tert-Butyl-2-(5-((3-methylpyridin-2-yl)ethinyl)thiophen-2-yl)imidazo[1,2-a]pyrazin-3-amin
[187] N,N-Dimethyl-2-(5-(pyridin-2-ylethinyl)thiophen-2-yl)imidazo[1,2-a]pyrazin-3-amin
[188] N-tert-Butyl-2-(5-((2-(tert-butyldiphenylsilyl)thiazol-5-yl)ethinyl)thiophen-2-yl)imidazo[1,2-a]pyridin-3-amin
[189] 3-((5-(3-(tert-Butylamino)imidazo[1,2-a]pyridin-2-yl)thiophen-2-yl)ethinyl)benzonitril
[190] N-tert-Butyl-2-(5-(phenylethinyl)thiazol-2-yl)imidazo[1,2-a]pyridin-3-amin
[191] N-tert-Butyl-2-(5-(thiazol-5-ylethinyl)thiophen-2-yl)imidazo[1,2-a]pyridin-3-amin
[192] N-tert-Butyl-2-(5-(pyridin-2-ylethinyl)thiophen-2-yl)imidazo[1,2-a]pyridin-3-amin Hydrochlorid
[193] 6-Chlor-N-cyclohexyl-2-(3-((6-methylpyridin-2-yl)ethinyl)phenyl)imidazo[1,2-a]pyridin-3-amin
[194] 5,7-Dimethyl-N-phenethyl-2-(5-(phenylethinyl)thiophen-2-yl)imidazo[1,2-a]pyrimidin-3-amin
[195] N-(3-Methoxyphenethyl)-5,7-dimethyl-2-(5-(phenylethinyl)thiophen-2-yl)imidazo[1,2-a]pyrimidin-3-amin
[196] N-(3-Methoxyphenethyl)-5,7-dimethyl-2-(4-(phenylethinyl)thiophen-2-yl)imidazo[1,2-a]pyrimidin-3-amin
[197] N-(3-Methoxyphenethyl)-5,7-dimethyl-2-(5-(phenylethinyl)furan-2-yl)imidazo[1,2-a]pyrimidin-3-amin
[198] N-(4-Chlorbenzyl)-8-methyl-2-(5-(phenylethinyl)thiophen-2-yl)imidazo[1,2-a]pyridin-3-amin
[199] N-(3-Methoxyphenethyl)-5-methyl-2-(4-(phenylethinyl)thiophen-2-yl)imidazo[1,2-a]pyridin-3-amin
[200] N-(2-Methylhexan-2-yl)-2-(4-(phenylethinyl)thiophen-2-yl)imidazo[1,2-a]pyrazin-3-amin
[201] N-Phenethyl-2-(4-(phenylethinyl)thiophen-2-yl)imidazo[1,2-a]pyrazin-3-amin
[202] N-(3-Methoxyphenethyl)-2-(4-(phenylethinyl)thiophen-2-yl)imidazo[1,2-a]pyrazin-3-amin
[203] 2-(4-(Phenylethinyl)thiophen-2-yl)-N-(2-(thiophen-2-yl)ethyl)imidazo[1,2-a]pyrazin-3-amin
[204] N-(4-Chlorbenzyl)-2-(5-(phenylethinyl)thiophen-2-yl)imidazo[1,2-a]pyridin-3-amin
[205] N-(2-Methylpentan-2-yl)-2-(5-(phenylethinyl)thiophen-2-yl)imidazo[1,2-a]pyrazin-3-amin
[206] N-(Cyclohexylmethyl)-2-(4-(phenylethinyl)thiophen-2-yl)imidazo[1,2-a]pyrazin-3-amin
[207] N-(2-Methoxybenzyl)-2-(5-(phenylethinyl)furan-2-yl)imidazo[1,2-a]pyrazin-3-amin
[208] N-(Cyclohexylmethyl)-2-(4-(phenylethinyl)thiophen-2-yl)imidazo[1,2-a]pyridin-3-amin
[209] N-(2-Methylpentan-2-yf)-2-(4-(phenylethinyl)thiophen-2-yl)imidazo[1,2-a]pyridin-3-amin
[210] 8-Brom-6-methyl-2-(3-((6-methylpyridin-2-yl)ethinyl)phenyl)-N-(2,4,4-trimethylpentan-2-yl)imidazo[1,2-a]pyridin-3-amin
[211] 8-Brom-N-cyclopentyl-6-methyl-2-(3-(pyridin-2-ylethinyl)phenyl)imidazo[1,2-a]pyridin-3-amin
[212] N-Cyclopentyl-2-(4-(phenylethinyl)thiophen-2-yl)imidazo[1,2-a]pyrazin-3-amin
[213] N-(1-Phenylethyl)-2-(4-(phenylethinyl)thiophen-2-yl)imidazo[1,2-a]pyrazin-3-amin
[214] N-(2-Methylpentan-2-yl)-2-(4-(phenylethinyl)thiophen-2-yl)imidazo[1,2-a]pyrazin-3-amin
[215] 8-Brom-N-cyclohexyl-6-methyl-2-(5-(pyridin-2-ylethinyl)furan-2-yl)imidazo[1,2-a]pyridin-3-amin
[216] N-Cyclopentyl-2-(5-(phenylethinyl)furan-2-yl)imidazo[1,2-a]pyrazin-3-amin
[217] N-(3-Methoxyphenethyl)-7-methyl-2-(4-(phenylethinyl)thiophen-2-yl)imidazo[1,2-a]pyrimidin-3-amin
[218] 8-(Benzyloxy)-2-(5-(phenylethinyl)thiophen-2-yl)-N-(2,4,4-trimethylpentan-2-yl)imidazo[1,2-a]pyridin-3-amin
[219] 8-(Benzyloxy)-N-cyclopentyl-2-(4-(phenylethinyl)thiophen-2-yl)imidazo[1,2-a]pyridin-3-amin
[220] 8-(Benzyloxy)-N-(2-methylpentan-2-yl)-2-(4-(phenylethinyl)thiophen-2-yl)imidazo[1,2-a]pyridin-3-amin
[221] 6-Chlor-N-(4-fluorbenzyl)-2-(5-(phenylethinyl)furan-2-yl)imidazo[1,2-a]pyridin-3-amin
[222] 6-Brom-N-butyl-5-methyl-2-(4-(phenylethinyl)thiophen-2-yl)imidazo[1,2-a]pyridin-3-amin
[223] N-(Furan-2-yl)-8-methyl-2-(5-(phenylethinyl)furan-2-yl)imidazo[1,2-a]pyridin-3-amin
[224] N-(Furan-2-yl)-2-(5-(phenylethinyl)furan-2-yl)imidazo[1,2-a]pyridin-3-amin
[225] N-(Furan-2-yl)-2-(5-(phenylethinyl)furan-2-yl)-7-propylimidazo[1,2-a]pyridin-3-amin
[226] 5,7-Dimethyl-2-(4-(phenylethinyl)thiophen-2-yl)-N-(3-(trifluormethyl)phenyl)imidazo[1,2-a]pyrimidin-3-amin
[227] 6-Brom-N-(4-chlorphenethyl)-8-methyl-2-(4-(phenylethinyl)thiophen-2-yl)imidazo[1,2-a]pyridin-3-amin
[228] N-(4-Chlorphenethyl)-7-phenyl-2-(4-(phenylethinyl)thiophen-2-yl)imidazo[1,2-a]pyridin-3-amin
[229] N-Phenethyl-7-phenyl-2-(4-(phenylethinyl)thiophen-2-yl)imidazo[1,2-a]pyridin-3-amin
[230] N-(4-Chlorbenzyl)-5,7-dimethyl-2-(4-(phenylethinyl)thiophen-2-yl)imidazo[1,2-a]pyridin-3-amin
[231] 6-Brom-N-(4-chlorbenzyl)-5-methyl-2-(4-(phenylethinyl)thiophen-2-yl)imidazo[1,2-a]pyridin-3-amin
[232] 8-Brom-N-(4-chlorbenzyl)-6-methyl-2-(4-(phenylethinyl)thiophen-2-yl)imidazo[1,2-a]pyridin-3-amin
[233] N-(3-Methoxyphenethyl)-2-(4-(phenylethinyl)thiophen-2-yl)-5-propylimidazo[1,2-a]pyridin-3-amin
[234] 6-Brom-8-methyl-2-(4-(phenylethinyl)thiophen-2-yl)-N-(2-(thiophen-2-yl)ethyl)imidazo[1,2-a]pyridin-3-amin
[235] 7-Phenyl-2-(4-(phenylethinyl)thiophen-2-yl)-N-(2-(thiophen-2-yl)ethyl)imidazo[1,2-a]pyridin-3-amin
[236] 6,8-Dibrom-N-(2-methylpentan-2-yl)-2-(5-(phenylethinyl)thiophen-2-yl)imidazo[1,2-a]pyridin-3-amin
[237] 6-Brom-N-(2,6-dimethylphenyl)-8-methyl-2-(4-(phenylethinyl)thiophen-2-yl)imidazo[1,2-a]pyridin-3-amin
[238] N-Cyclohexyl-2-(3-(pyridin-2-ylethinyl)phenyl)imidazo[1,2-a]pyrazin-3-amin
[239] 2-(3-(Pyridin-2-ylethinyl)phenyl)-N-(2,4,4-trimethylpentan-2-yl)imidazo[1,2-a]pyrazin-3-amin
[240] N-Cyclopentyl-2-(4-(pyridin-2-ylethinyl)phenyl)imidazo[1,2-a]pyrazin-3-amin
[241] 8-Chlor-2-(4-(pyridin-2-ylethinyl)phenyl)-6-(trifluormethyl)-N-(2,4,4-trimethylpentan-2-yl)imidazo[1,2-a]pyridin-3-amin
[242] N-(4-Fluorphenyl)-2-(4-(pyridin-2-ylethinyl)phenyl)imidazo[1,2-a]pyrazin-3-amin
[243] N-Cyclopentyl-2-(2-methyl-6-(phenylethinyl)pyridin-3-yl)imidazo[1,2-a]pyrazin-3-amin
[244] N-Cyclohexyl-2-(2-methyl-6-(phenylethinyl)pyridin-3-yl)imidazo[1,2-a]pyrazin-3-amin
[245] N-Cyclopentyl-2-(5-(pyridin-2-ylethinyl)furan-2-yl)imidazo[1,2-a]pyrazin-3-amin
[246] 8-Brom-N-cyclopentyl-6-methyl-2-(5-(pyridin-2-ylethinyl)furan-2-yl)imidazo[1,2-a]pyridin-3-amin
[247] N-Cyclohexyl-2-(5-(pyridin-2-ylethinyl)furan-2-yl)imidazo[1,2-a]pyrazin-3-amin
[248] 2-(5-(Pyridin-2-ylethinyl)furan-2-yl)-N-(2,4,4-trimethylpentan-2-yl)imidazo[1,2-a]pyrazin-3-amin
[249] N-Cyclopentyl-2-(3-((6-methylpyridin-2-yl)ethinyl)phenyl)imidazo[1,2-a]pyrazin-3-amin
[250] 8-Brom-N-cyclopentyl-6-methyl-2-(4-(pyridin-2-ylethinyl)phenyl)imidazo[1,2-a]pyridin-3-amin
[251] N-Cyclohexyl-2-(3-((6-methylpyridin-2-yl)ethinyl)phenyl)imidazo[1,2-a]pyrazin-3-amin
[252] N-(4-Fluorphenyl)-2-(3-((6-methylpyridin-2-yl)ethinyl)phenyl)imidazo[1,2-a]pyrazin-3-amin
[253] 2-(5-(Pyridin-2-ylethinyl)thiophen-2-yl)-N-(2,4,4-trimethylpentan-2-yl)imidazo[1,2-a]pyrimidin-3-amin
[254] N-Cyclohexyl-2-(3-(pyridin-2-ylethinyl)phenyl)imidazo[1,2-a]pyrimidin-3-amin
[255] 2-(3-(Pyridin-2-ylethinyl)phenyl)-N-(2,4,4-trimethylpentan-2-yl)imidazo[1,2-a]pyrimidin-3-amin
[256] 2-(6-(Phenylethinyl)pyridin-3-yl)-N-(2,4,4-trimethylpentan-2-yl)imidazo[1,2-a]pyrimidin-3-amin
[257] N-Cyclopentyl-2-(5-(pyridin-2-ylethinyl)furan-2-yl)imidazo[1,2-a]pyrimidin-3-amin
[258] N-tert-Butyl-2-(5-(pyridin-2-ylethinyl)furan-2-yl)imidazo[1,2-a]pyrimidin-3-amin
[259] N-Cyclopentyl-2-(3-((6-methylpyridin-2-yl)ethinyl)phenyl)imidazo[1,2-a]pyrimidin-3-amin
[260] N-Cyclopentyl-2-(3-(pyridin-2-ylethinyl)phenyl)imidazo[1,2-a]pyridin-3-amin
[261] N-tert-Butyl-2-(3-(pyridin-2-ylethinyl)phenyl)imidazo[1,2-a]pyridin-3-amin
[262] 2-(3-(Pyridin-2-ylethinyl)phenyl)-N-(2,4,4-trimethylpentan-2-yl)imidazo[1,2-a]pyridin-3-amin
[263] N-(4-Fluorphenyl)-2-(3-(pyridin-2-ylethinyl)phenyl)imidazo[1,2-a]pyridin-3-amin
[264] N-tert-Butyl-2-(4-(pyridin-2-ylethinyl)phenyl)imidazo[1,2-a]pyridin-3-amin
[265] N-Cyclopentyl-2-(2-methyl-6-(phenylethinyl)pyridin-3-yl)imidazo[1,2-a]pyridin-3-amin
[266] N-(4-Fluorphenyl)-2-(2-methyl-6-(phenylethinyl)pyridin-3-yl)imidazo[1,2-a]pyridin-3-amin
[267] N-Cyclopentyl-6-methyl-2-(5-(pyridin-2-ylethinyl)thiophen-2-yl)imidazo[1,2-a]pyridin-3-amin
[268] N-tert-Butyl-6-methyl-2-(5-(pyridin-2-ylethinyl)thiophen-2-yl)imidazo[1,2-a]pyridin-3-amin
[269] N-Cyclohexyl-6-methyl-2-(5-(pyridin-2-ylethinyl)thiophen-2-yl)imidazo[1,2-a]pyridin-3-amin
[270] 6-Methyl-2-(5-(pyridin-2-ylethinyl)thiophen-2-yl)-N-(2,4,4-trimethylpentan-2-yl)imidazo[1,2-a]pyridin-3-amin
[271] 6-Methyl-2-(2-methyl-6-(phenylethinyl)pyridin-3-yl)-N-(2,4,4-trimethylpentan-2-yl)imidazo[1,2-a]pyridin-3-amin
[272] N-Cyclopentyl-6-methyl-2-(5-(pyridin-2-ylethinyl)furan-2-yl)imidazo[1,2-a]pyridin-3-amin
[273] N-Cyclohexyl-6-methyl-2-(3-((6-methylpyridin-2-yl)ethinyl)phenyl)imidazo[1,2-a]pyridin-3-amin
[274] N-Cyclohexyl-7-methyl-2-(5-(pyridin-2-ylethinyl)thiophen-2-yl)imidazo[1,2-a]pyridin-3-amin
[275] 7-Methyl-2-(5-(pyridin-2-ylethinyl)thiophen-2-yl)-N-(2,4,4-trimethylpentan-2-yl)imidazo[1,2-a]pyridin-3-amin
[276] N-tert-Butyl-7-methyl-2-(3-(pyridin-2-ylethinyl)phenyl)imidazo[1,2-a]pyridin-3-amin
[277] N-(4-Fluorphenyl)-7-methyl-2-(3-(pyridin-2-ylethinyl)phenyl)imidazo[1,2-a]pyridin-3-amin
[278] N-tert-Butyl-7-methyl-2-(4-(pyridin-2-ylethinyl)phenyl)imidazo[1,2-a]pyridin-3-amin
[279] N-(4-Fluorphenyl)-7-methyl-2-(5-(pyridin-2-ylethinyl)furan-2-yl)imidazo[1,2-a]pyridin-3-amin
[280] N-Cyclohexyl-8-methyl-2-(6-(phenylethinyl)pyridin-3-yl)imidazo[1,2-a]pyridin-3-amin
[281] N-Cyclopentyl-2-(6-(phenylethinyl)pyridin-3-yl)imidazo[1,2-a]pyridin-3-amin
[282] N-tert-Butyl-2-(6-(phenylethinyl)pyridin-3-yl)imidazo[1,2-a]pyridin-3-amin
[283] N-Cyclohexyl-2-(6-(phenylethinyl)pyridin-3-yl)imidazo[1,2-a]pyridin-3-amin
[284] 2-(6-(Phenylethinyl)pyridin-3-yl)-N-(2,4,4-trimethylpentan-2-yl)imidazo[1,2-a]pyridin-3-amin
[285] N-tert-Butyl-2-(5-(pyridin-2-ylethinyl)furan-2-yl)imidazo[1,2-a]pyridin-3-amin
[286] N-Cyclohexyl-2-(5-(pyridin-2-ylethinyl)furan-2-yl)imidazo[1,2-a]pyridin-3-amin
[287] N-(4-Fluorphenyl)-2-(5-(pyridin-2-ylethinyl)furan-2-yl)imidazo[1,2-a]pyridin-3-amin
[288] N-Cyclohexyl-2-(3-((6-methylpyridin-2-yl)ethinyl)phenyl)imidazo[1,2-a]pyridin-3-amin
[289] 2-(3-((6-Methylpyridin-2-yl)ethinyl)phenyl)-N-(2,4,4-trimethylpentan-2-yl)imidazo[1,2-a]pyridin-3-amin
[290] N-tert-Butyl-5-methyl-2-(3-((6-methylpyridin-2-yl)ethinyl)phenyl)imidazo[1,2-a]pyridin-3-amin
[291] 5-Methyl-2-(3-((6-methylpyridin-2-yl)ethinyl)phenyl)-N-(2,4,4-trimethylpentan-2-yl)imidazo[1,2-a]pyridin-3-amin
[292] N-Cyclohexyl-5,7-dimethyl-2-(3-(pyridin-2-ylethinyl)phenyl)imidazo[1,2-a]pyrimidin-3-amin
[293] N-Cyclohexyl-5,7-dimethyl-2-(2-methyl-6-(phenylethinyl)pyridin-3-yl)imidazo[1,2-a]pyrimidin-3-amin
[294] N-Cyclopentyl-5,7-dimethyl-2-(5-(pyridin-2-ylethinyl)furan-2-yl)imidazo[1,2-a]pyrimidin-3-amin
[295] N-tert-Butyl-5,7-dimethyl-2-(5-(pyridin-2-ylethinyl)furan-2-yl)imidazo[1,2-a]pyrimidin-3-amin
[296] N-(4-Fluorphenyl)-8-methyl-2-(3-(pyridin-2-ylethinyl)phenyl)imidazo[1,2-a]pyridin-3-amin
[297] N-Cyclohexyl-8-methyl-2-(4-(pyridin-2-ylethinyl)phenyl)imidazo[1,2-a]pyridin-3-amin
[298] N-Cyclohexyl-7-ethyl-2-(5-(pyridin-2-ylethinyl)thiophen-2-yl)imidazo[1,2-a]pyridin-3-amin
[299] 7-Ethyl-2-(5-(pyridin-2-ylethinyl)thiophen-2-yl)-N-(2,4,4-trimethylpentan-2-yl)imidazo[1,2-a]pyridin-3-amin
[300] N-Cyclohexyl-7-ethyl-2-(3-(pyridin-2-ylethinyl)phenyl)imidazo[1,2-a]pyridin-3-amin
[301] N-Cyclopentyl-7-ethyl-2-(4-(pyridin-2-ylethinyl)phenyl)imidazo[1,2-a]pyridin-3-amin
[302] N-Cyclopentyl-7-ethyl-2-(6-(phenylethinyl)pyridin-3-yl)imidazo[1,2-a]pyridin-3-amin
[303] N-tert-Butyl-7-ethyl-2-(6-(phenylethinyl)pyridin-3-yl)imidazo[1,2-a]pyridin-3-amin
[304] N-tert-Butyl-7-ethyl-2-(5-(pyridin-2-ylethinyl)furan-2-yl)imidazo[1,2-a]pyridin-3-amin
[305] 7-Isopropyl-2-(5-(pyridin-2-ylethinyl)thiophen-2-yl)-N-(2,4,4-trimethylpentan-2-yl)imidazo[1,2-a]pyridin-3-amin
[306] N-tert-Butyl-7-isopropyl-2-(3-(pyridin-2-ylethinyl)phenyl)imidazo[1,2-a]pyridin-3-amin
[307] N-tert-Butyl-7-isopropyl-2-(4-(pyridin-2-ylethinyl)phenyl)imidazo[1,2-a]pyridin-3-amin
[308] N-Cyclohexyl-7-isopropyl-2-(5-(pyridin-2-ylethinyl)furan-2-yl)imidazo[1,2-a]pyridin-3-amin
[309] N-tert-Butyl-7-isopropyl-2-(3-((6-methylpyridin-2-yl)ethinyl)phenyl)imidazo[1,2-a]pyridin-3-amin
[310] 6-Chlor-N-cyclopentyl-2-(5-(pyridin-2-ylethinyl)thiophen-2-yl)imidazo[1,2-a]pyridin-3-amin
[311] N-tert-Butyl-6-chlor-2-(5-(pyridin-2-ylethinyl)thiophen-2-yl)imidazo[1,2-a]pyridin-3-amin
[312] 6-Chlor-N-cyclohexyl-2-(3-(pyridin-2-ylethinyl)phenyl)imidazo[1,2-a]pyridin-3-amin
[313] 6-Chlor-2-(3-(pyridin-2-ylethinyl)phenyl)-N-(2,4,4-trimethylpentan-2-yl)imidazo[1,2-a]pyridin-3-amin
[314] N-tert-Butyl-6-chlor-2-(4-(pyridin-2-ylethinyl)phenyl)imidazo[1,2-a]pyridin-3-amin
[315] 6-Chlor-N-cyclohexyl-2-(4-(pyridin-2-ylethinyl)phenyl)imidazo[1,2-a]pyridin-3-amin
[316] N-tert-Butyl-6-chlor-2-(5-(pyridin-2-ylethinyl)furan-2-yl)imidazo[1,2-a]pyridin-3-amin
[317] 6-Chlor-N-cyclohexyl-2-(5-(pyridin-2-ylethinyl)furan-2-yl)imidazo[1,2-a]pyridin-3-amin
[318] 6-Chlor-2-(5-(pyridin-2-ylethinyl)furan-2-yl)-N-(2,4,4-trimethylpentan-2-yl)imidazo[1,2-a]pyridin-3-amin
[319] 6-Chlor-N-cyclopentyl-2-(3-((6-methylpyridin-2-yl)ethinyl)phenyl)imidazo[1,2-a]pyridin-3-amin
[320] N-tert-Butyl-6-chlor 2-(3-((6-methylpyridin-2-yl)ethinyl)phenyl)imidazo[1,2-a]pyridin-3-amin
[321] N-tert-Butyl-2-(5-(pyridin-2-ylethinyl)thiophen-2-yl)imidazo[1,2-a]pyridin-3-amin Bis-Hydrochlorid
[322] N-Methyl-2-(5-(pyridin-2-ylethinyl)thiophen-2-yl)imidazo[1,2-a]pyrazin-3-amin
[323] N-tert-Butyl-2-(5-(pyridin-2-ylethinyl)thiophen-2-yl)imidazo[1,2-a]pyridin-3-amin Bis-Hydrochlorid
[324] [2-(5-Pyridin-2-ylethinyl-thiophen-2-yl)-imidazo[1,2-a]pyridin-3-yl]-(1,1,3,3-tetramethyl-butyl)-amin
[325] tert-Butyl-[2-(5-pyrimidin-2-ylethinyl-thiophen-2-yl)-imidazo[1,2-a]pyridin-3-yl]-amin Hydrochlorid
[326] {2-[5-(3-Amino-pyridin-2-ylethinyl)-thiophen-2-yl]-imidazo[1,2-a]pyridin-3-yl}-tert-butyl-amin
[327] tert-Butyl-[2-(5-pyridin-2-ylethinyl-thiazol-2-yl)-imidazo[1,2-a]pyridin-3-yl]-amin
[328] tert-Butyl-[2-(2-pyridin-2-ylethinyl-thiazol-5-yl)-imidazo[1,2-a]pyridin-3-yl]-amin
[329] tert-Butyl-{2-[5-(6-fluor-pyridin-2-ylethinyl)-thiophen-2-yl]-imidazo[1,2-a]pyridin-3-yl}-amin Hydrochlorid
[330] tert-Butyl-{2-[5-(3-chlor-5-fluor-phenylethinyl)-thiophen-2-yl]-imidazo[1,2-a]pyridin-3-yl}-amin Hydrochlorid
[331] tert-Butyl-[2-(5-pyridin-2-ylethinyl-thiophen-2-yl)-imidazo[1,2-b]pyridazin-3-yl]-amin Hydrochlorid
[332] tert-Butyl-[2-(5-thiazol-2-ylethinyl-thiophen-2-yl)-imidazo[1,2-a]pyridin-3-yl]-amin Hydrochlorid
[333] tert-Butyl-{2-[5-(3-trifluormethoxy-phenylethinyl)-thiophen-2-yl]-imidazo[1,2-a]pyrazin-3-yl}-amin
[334] tert-Butyl-{2-[5-(3-[1,3]dioxolan-2-yl-phenylethinyl)-thiophen-2-yl]-imidazo[1,2-a]pyridin-3-yl}-amin
[335] tert-Butyl-{2-[5-(3,5-dimethyl-phenylethinyl)-thiophen-2-yl]-imidazo[1,2-a]pyridin-3-yl}-amin Hydrochlorid
[336] tert-Butyl-{2-[5-(3-fluor-pyridin-2-ylethinyl)-thiophen-2-yl]-imidazo[1,2-a]pyridin-3-yl}-amin Hydrochlorid
[337] tert-Butyl-{2-[5-(3-methyl-3H-imidazol-4-ylethinyl)-thiophen-2-yl]-imidazo[1,2-a]pyridin-3-yl}-amin Hydrochlorid und
[338] tert-Butyl-{2-[5-(5-chlor-thiophen-2-ylethinyl)-thiophen-2-yl]-imidazo[1,2-a]pyridin-3-yl}-amin Hydrochlorid
[339] tert-Butyl-{2-[5-(5-methyl-pyridin-2-ylethinyl)-thiophen-2-yl]-imidazo[1,2-a]pyridin-3-yl}-amin Hydrochlorid
[340] 1-{3-[5-(3-tert-Butylamino-imidazo[1,2-a]pyridin-2-yl)-thiophen-2-ylethinyl]-phenyl}-ethanon Hydrochlorid
[341] {3-[5-(3-tert-Butylamino-imidazo[1,2-a]pyridin-2-yl)-thiophen-2-ylethinyl]-phenyl}-methanol Hydrochlorid
[342] N-tert-Butyl-2-(5-((3-methoxypyridin-2-yl)ethinyl)thiophen-2-yl)imidazo[1,2-a]pyridin-3-amin
[343] N-tert-Butyl-2-(5-(thiophen-2-ylethinyl)thiophen-2-yl)imidazo[1,2-a]pyridin-3-amin Hydrochlorid
[344] 5-((5-(3-(tert-Butylamino)imidazo[1,2-a]pyridin-2-yl)thiophen-2-yl)ethinyl)-2-fluorbenzonitril Hydrochlorid
[345] N-tert-Butyl-2-(5-((3,4-difluorphenyl)ethinyl)thiophen-2-yl)imidazo[1,2-a]pyridin-3-amin
[346] N-tert-Butyl-2-(5-((3-(methoxymethyl)phenyl)ethinyl)thiophen-2-yl)imidazo[1,2-a]pyridin-3-amin
[347] 2-(5-((3-Aminophenyl)ethinyl)thiophen-2-yl)-N-tert-butylimidazo[1,2-a]pyridin-3-amin Hydrochlorid
[348] N-tert-Butyl-2-(5-((4-fluor-3-methylphenyl)ethinyl)thiophen-2-yl)imidazo[1,2-a]pyridin-3-amin Hydrochlorid
[349] N-tert-Butyl-2-(5-((3,5-difluorphenyl)ethinyl)thiophen-2-yl)imidazo[1,2-a]pyridin-3-amin
[350] N-tert-Butyl-2-(5-(thiophen-3-ylethinyl)thiophen-2-yl)imidazo[1,2-a]pyridin-3-amin Hydrochlorid
[351] N-tert-Butyl-2-(5-((3-methylpyridin-2-yl)ethinyl)thiophen-2-yl)imidazo[1,2-a]pyridin-3-amin Hydrochlorid
[352] 3-((5-(3-(tert-Butylamino)imidazo[1,2-a]pyridin-2-yl)thiophen-2-yl)ethinyl)benzolsulfonamid Hydrochlorid
[353] 3-((5-(3-(tert-Butylamino)imidazo[1,2-a]pyridin-2-yl)hiophen-2-yl)ethinyl)benzoesäure
[354] 3-((5-(3-(tert-Butylamino)imidazo[1,2-a]pyridin-2-yl)-thiophen-2-yl)ethinyl)benzamid Hydrochlorid
[355] N-(3-((5-(3-(tert-Butylamino)imidazo[1,2-a]pyridin-2-yl)thiophen-2-yl)ethinyl)phenyl)acetamid
[356] N-tert-Butyl-N-methyl-2-(5-(pyridin-2-ylethinyl)thiophen-2-yl)imidazo[1,2-a]pyridin-3-amin
[357] N,N-Dimethyl-2-(5-(pyridin-2-ylethinyl)thiophen-2-yl)imidazo[1,2-a]pyridin-3-amin
[358] N-tert-Butyl-N-methyl-2-(5-(pyridin-2-ylethinyl)thiazol-2-yl)imidazo[1,2-a]pyridin-3-amin
[359] (6-((5-(3-(tert-Butylamino)imidazo[1,2-a]pyridin-2-yl)thiophen-2-yl)ethinyl)pyridin-2-yl)methanol
[360] N-(3-((5-(3-(tert-Butylamino)imidazo[1,2-a]pyridin-2-yl)thiophen-2-yl)ethinyl)phenyl)methansulfonamid
[361] N-tert-Butyl-8-methyl-2-(5-(pyridin-2-ylethinyl)thiophen-2-yl)imidazo[1,2-a]pyrazin-3-amin Hydrochlorid
[362] 2-(5-(Pyridin-2-ylethinyl)thiophen-2-yl)imidazo[1,2-a]pyridin-3-amin
[363] N-tert-Butyl-2-(5-((3-chlorphenyl)ethinyl)thiophen-2-yl)imidazo[1,2-a]pyridin-3-amin
[364] N-tert-Butyl-2-(5-((2,3-difluorphenyl)ethinyl)thiophen-2-yl)imidazo[1,2-a]pyridin-3-amin
und
[365] N-tert-Butyl-7-chlor-2-(5-(pyridin-2-ylethinyl)thiophen-2-yl)imidazo[1,2-a]pyridin-3-amin
sowie jeweils ggf. in Form entsprechender Salze, insbesondere Hydrochloride, oder jeweils ggf. in Form entsprechender Solvate.

25. Verfahren zur Herstellung substituierter bizyklischer Imidazo-3-yl-amin-Verbindungen der allgemeinen Formel I gemäß einem oder mehreren der Ansprüche 1 bis 24, **dadurch gekennzeichnet, dass** wenigstens eine Verbindung der allgemeinen Formel II, 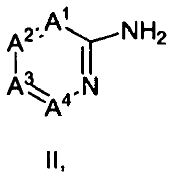 worin A¹, A², A³ und A⁴ die Bedeutung gemäß einem oder mehreren der Ansprüche 1 bis 24 haben, in einem Reaktionsmedium, ggf. in Gegenwart wenigstens einer organischen oder anorganischen Säure oder wenigstens eines Übergangsmetallsalzes mit wenigstens einem Isocyanid der allgemeinen Formel III,
R²-N≡C III,
worin R² die Bedeutung gemäß einem oder mehreren der Ansprüche 1 bis 24 hat, und wenigstens einem Aldehyd der allgemeinen Formel IV, 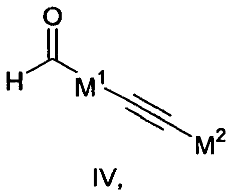 worin M¹ und M² die Bedeutung gemäß einem oder mehreren der Ansprüche 1 bis 24 haben, umgesetzt und die so erhaltene Verbindung der allgemeinen Formel V, 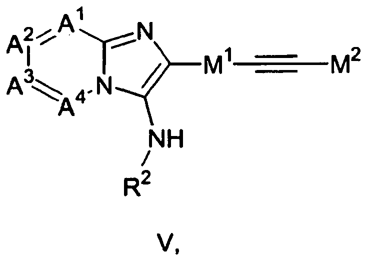 worin A¹, A², A³, A⁴, R², M¹ und M² die vorstehend genannte Bedeutung haben, ggf. gereinigt und/oder isoliert wird, und ggf. in ein entsprechendes Salz überführt und dieses ggf. gereinigt und/oder isoliert wird,
oder wenigstens eine Verbindung der allgemeinen Formel II, 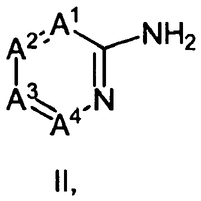 worin A¹, A², A³ und A⁴ die Bedeutung gemäß einem oder mehreren der Ansprüche 1 bis 24 haben, in einem Reaktionsmedium, ggf. in Gegenwart wenigstens einer organischen oder anorganischen Säure oder wenigstens eines Übergangsmetallsalzes mit wenigstens einem Isocyanid der allgemeinen Formel III,
R²-N≡C III,
worin R² die Bedeutung gemäß einem oder mehreren der Ansprüche 1 bis 24 hat, und wenigstens einem Aldehyd der allgemeinen Formel VI, 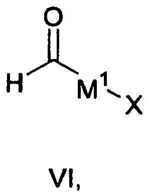 worin M¹ die Bedeutung gemäß einem oder mehreren der Ansprüche 1 bis 24 hat und X für eine Abgangsgruppe steht, umgesetzt und die so erhaltene Verbindung der allgemeinen Formel VII, 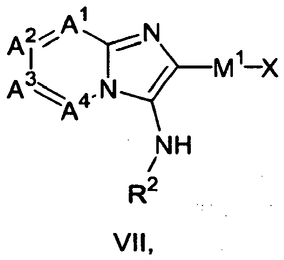 worin A¹, A², A³, A⁴, R², M¹ und X die vorstehend genannte Bedeutung haben, ggf. gereinigt und/oder isoliert wird, und ggf. in ein entsprechendes Salz überführt und dieses ggf. gereinigt und/oder isoliert wird, und durch Umsetzung mit wenigstens einem Acetylen der allgemeinen Formel XI, 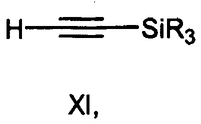 worin R, unabhängig voneinander, jeweils für einen linearen oder verzweigten Alkyl-Rest oder für einen unsubstituierten Phenyl-Rest steht, in einem Reaktionsmedium, ggf. in Gegenwart wenigstens eines geeigneten Katalysators und ggf. in Gegenwart wenigstens einer anorganischen und/oder organischen Base in eine entsprechend substituierte Verbindung der allgemeinen Formel XII, 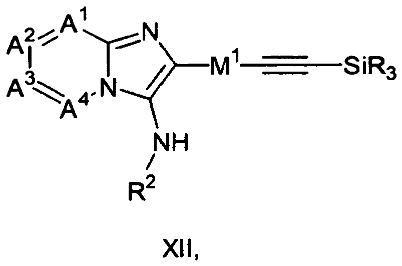 worin A¹, A², A³, A⁴, R² und M¹ die vorstehend genannte Bedeutung haben und R, unabhängig voneinander, jeweils für einen linearen oder verzweigten Alkyl-Rest oder für einen unsubstituierten Phenyl-Rest steht, überführt und ggf. gereinigt und/oder isoliert wird, und ggf. in ein entsprechendes Salz überführt und dieses ggf. gereinigt und/oder isoliert wird,
und wenigstens eine Verbindung der allgemeinen Formel XII in einem Reaktionsmedium, ggf. in Gegenwart wenigstens einer anorganischen und/oder organischen Base, ggf. in Gegenwart wenigstens eines anorganischen Salzes, und ggf. in Gegenwart wenigstens eines Ammoniumsalzes, in eine entsprechend substituierte Verbindung der allgemeinen Formel XIII, 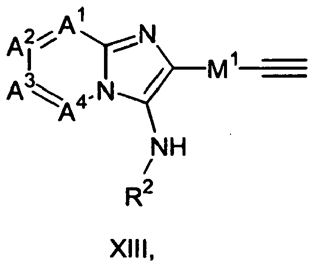 worin A¹, A², A³, A⁴, R² und M¹ die vorstehend genannte Bedeutung haben, überführt und ggf. gereinigt und/oder isoliert wird, und ggf. in ein entsprechendes Salz überführt und dieses ggf. gereinigt und/oder isoliert wird, und wenigstens eine Verbindung der allgemeinen Formel XIII und/oder wenigstens eine Verbindung der allgemeinen Formel XII durch Umsetzung mit wenigstens einer Verbindung der allgemeinen Formel M²-X, worin M² die Bedeutung gemäß einem oder mehreren der Ansprüche 1 bis 24 hat und X für eine Abgangsgruppe steht, in einem Reaktionsmedium, ggf. in Gegenwart wenigstens eines geeigneten Katalysators, ggf. in Gegenwart wenigstens einer anorganischen und/oder organischen Base, ggf. in Gegenwart wenigstens eines anorganischen Salzes und ggf. in Gegenwart wenigstens eines Ammoniumsalzes in eine entsprechend substituierte Verbindung der allgemeinen Formel V, 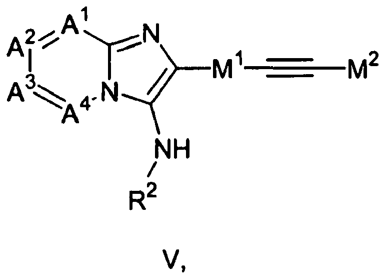 worin A¹, A², A³, A⁴, R², M¹ und M² die vorstehend genannte Bedeutung haben überführt und ggf. gereinigt und/oder isoliert wird, und ggf. in ein entsprechendes Salz überführt und dieses ggf. gereinigt und/oder isoliert wird,
oder eine Verbindung der allgemeinen Formel VII durch Umsetzung mit wenigstens einem Acetylen der allgemeinen Formel VIII, 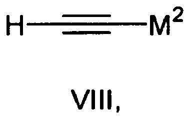 worin M² die Bedeutung gemäß einem oder mehreren der Ansprüche 1 bis 24 hat, in einem Reaktionsmedium, ggf. in Gegenwart wenigstens eines geeigneten Katalysators und ggf. in Gegenwart wenigstens einer anorganischen und/oder organischen Base in eine entsprechend substituierte Verbindung der allgemeinen Formel V, 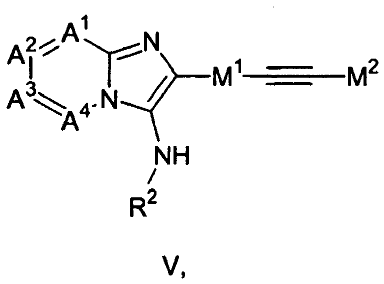 worin A¹, A², A³, A⁴, R², M¹ und M² die vorstehend genannte Bedeutung haben überführt und ggf. gereinigt und/oder isoliert wird, und ggf. in ein entsprechendes Salz überführt und dieses ggf. gereinigt und/oder isoliert wird,
und ggf. die Verbindung der allgemeinen Formel V durch Umsetzung mit wenigstens einer Verbindung der allgemeinen Formel R³-X, worin R³ die Bedeutung gemäß einem oder mehreren der Ansprüche 1 bis 24 hat und X für eine Abgangsgruppe steht, in einem Reaktionsmedium, in Gegenwart wenigstens einer organischen oder anorganischen Base
oder durch Umsetzung mit wenigstens einer Verbindung der allgemeinen Formel R²⁰-C(=O)-OH, worin R²⁰ die Bedeutung gemäß einem oder mehreren der Ansprüche 1 bis 24 hat, in einem Reaktionsmedium, ggf. in Gegenwart wenigstens einer organischen oder anorganischen Base und/oder in Gegenwart wenigstens eines Kopplungsmittels,
oder durch Umsetzung mit wenigstens einer Verbindung der allgemeinen Formel R²⁰-C(=O)-X, worin R²⁰ die Bedeutung gemäß einem oder mehreren der Ansprüche 1 bis 24 hat und X für eine Abgangsgruppe in einem Reaktionsmedium, ggf. in Gegenwart wenigstens einer organischen oder anorganischen Base,
oder durch Umsetzung mit wenigstens einer Verbindung der allgemeinen Formel R²⁰-C(=O)-H, worin R²⁰ die Bedeutung gemäß einem oder mehreren der Ansprüche 1 bis 24 hat, in einem Reaktionsmedium, ggf. in Gegenwart wenigstens eines Reduktionsmittels,
in eine Verbindung der allgemeinen Formel I, ggf. in Form eines entsprechenden Salzes, überführt wird, 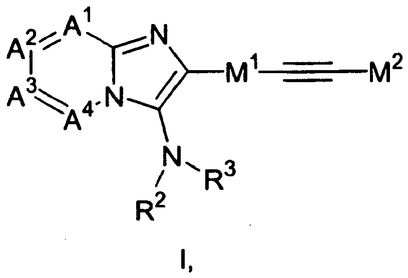 worin A¹, A², A³, A⁴, R², R³, M¹ und M² die Bedeutung gemäß einem oder mehreren der Ansprüche 1 bis 24 haben, und diese ggf. gereinigt und/oder isoliert wird.

26. Verfahren zur Herstellung substituierter bizyklischer Imidazo-3-yl-amin-Verbindungen der allgemeinen Formel I gemäß einem oder mehreren der Ansprüche 1 bis 24, **dadurch gekennzeichnet, dass** wenigstens eine Verbindung der allgemeinen Formel V, 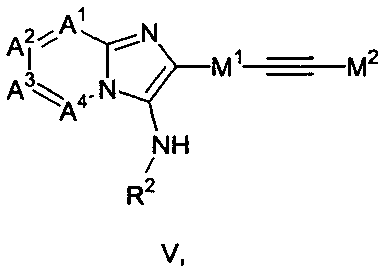 worin A¹, A², A³, A⁴, R², M¹ und M² die Bedeutung gemäß einem oder mehreren der Ansprüche 1 bis 24 haben, ggf. in einem Reaktionsmedium in Gegenwart wenigstens einer organischen oder anorganischen Säure, umgesetzt, und die so erhaltene Verbindung der allgemeinen Formel IX, 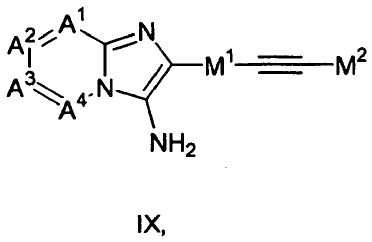 worin A¹, A², A³, A⁴, M¹ und M² die Bedeutung gemäß einem oder mehreren der Ansprüche 1 bis 24 haben, ggf. gereinigt und/oder isoliert wird, und ggf. in ein entsprechendes Salz überführt und dieses ggf. gereinigt und/oder isoliert wird und diese Verbindung /dieses Salz ggf. durch Umsetzung
in einem Reaktionsmedium, in Gegenwart wenigstens einer anorganischen oder organischen Base mit wenigstens einer Verbindung der allgemeinen Formel R³-X, worin R³ die Bedeutung gemäß einem oder mehreren der Ansprüche 1 bis 24 hat und X für eine Abgangsgruppe steht, oder
in einem Reaktionsmedium, ggf. in Gegenwart wenigstens einer organischen oder anorganischen Base und/oder ggf. in Gegenwart wenigstens eines Kopplungsmittels mit wenigstens einer Verbindung der allgemeinen Formel R²⁰-C(=O)-OH, worin R²⁰ die Bedeutung gemäß einem oder mehreren der Ansprüche 1 bis 24hat,
oder in einem Reaktionsmedium, ggf. in Gegenwart wenigstens einer organischen oder anorganischen Base mit wenigstens einer Verbindung der allgemeinen Formel R²⁰-C(=O)-X, worin R²⁰ die Bedeutung gemäß einem oder mehreren der Ansprüche 1 bis 24 hat und X für eine Abgangsgruppe steht,
oder in einem Reaktionsmedium, ggf. in Gegenwart wenigstens eines Reduktionsmittels mit wenigstens einer Verbindung der allgemeinen Formel R²⁰-C(=O)-H, worin R²⁰ die Bedeutung gemäß einem oder mehreren der Ansprüche 1 bis 24 hat,
in eine entsprechende Verbindung der allgemeinen Formel X, ggf. in Form eines entsprechenden Salzes, überführt wird, 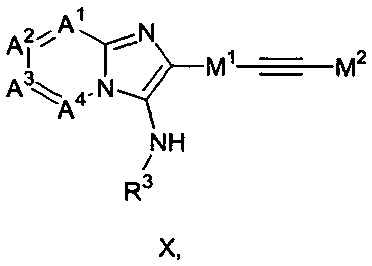 worin A¹, A², A³, A⁴, R³, M¹ und M² die Bedeutung gemäß einem oder mehreren der Ansprüche 1 bis 24 haben, und diese ggf. gereinigt und/oder isoliert wird,
und ggf. die Verbindung der allgemeinen Formel X durch Umsetzung mit wenigstens einer Verbindung der allgemeinen Formel R²-X, worin R² die Bedeutung gemäß einem oder mehreren der Ansprüche 1 bis 24 hat und X für eine Abgangsgruppe steht, in einem Reaktionsmedium, in Gegenwart wenigstens einer organischen oder anorganischen Base
oder durch Umsetzung mit wenigstens einer Verbindung der allgemeinen Formel R²⁰-C(=O)-OH, worin R²⁰ die Bedeutung gemäß einem oder mehreren der Ansprüche 1 bis 24 hat, in einem Reaktionsmedium, ggf. in Gegenwart wenigstens einer organischen oder anorganischen Base und/oder in Gegenwart wenigstens eines Kopplungsmittels,
oder durch Umsetzung mit wenigstens einer Verbindung der allgemeinen Formel R²⁰-C(=O)-X, worin R²⁰ die Bedeutung gemäß einem oder mehreren der Ansprüche 1 bis 24 hat und X für eine Abgangsgruppe steht, in einem Reaktionsmedium, ggf. in Gegenwart wenigstens einer organischen oder anorganischen Base,
oder durch Umsetzung mit wenigstens einer Verbindung der allgemeinen Formel R²⁰-C(=O)-H, worin R²⁰ die Bedeutung gemäß einem oder mehreren der Ansprüche 1 bis 24 hat, in einem Reaktionsmedium, ggf. in Gegenwart wenigstens eines Reduktionsmittels,
in eine Verbindung der allgemeinen Formel I, ggf. in Form eines entsprechenden Salzes, überführt wird, 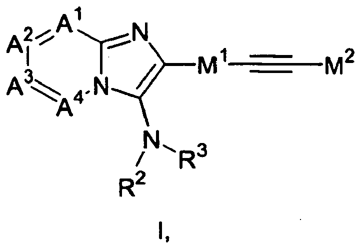 worin A¹, A², A³, A⁴, R², R³, M¹ und M² die Bedeutung gemäß einem oder mehreren der Ansprüche 1 bis 24 haben, und diese ggf. gereinigt und/oder isoliert wird.

27. Arzneimittel enthaltend wenigstens eine Verbindung gemäß einem oder mehreren der Ansprüche 1 bis 24 und ggf. einen oder mehrere physiologisch verträgliche Hilfsstoffe.

28. Arzneimittel gemäß Anspruch 27 zur Prophylaxe und/oder Behandlung von Störungen und/oder Krankheiten, die zumindest teilweise durch mGluR5-Rezeptoren vermittelt werden.

29. Arzneimittel gemäß Anspruch 27 oder 28 zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz.

30. Arzneimittel gemäß Anspruch 27 oder 28 zur Behandlung und/oder Prophylaxe von Migräne; Depressionen; neurodegenerativen Erkrankungen, vorzugsweise ausgewählt aus der Gruppe bestehend aus Morbus Parkinson, Morbus Alzheimer, Morbus Huntington und Multipler Sklerose; kognitiven Erkrankungen, vorzugsweise kognitiven Mangelzuständen, besonders bevorzugt des Aufmerksamkeit-Defizit-Syndroms (ADS); Panikattacken; Angstsyndrom; Epilepsie; Husten; Harninkontinenz; Diarrhöe; Pruritus; Schizophrenie; cerebralen Ischämien; Muskelspasmen; Krämpfen; Reizdarmsyndrom; Störungen der Nahrungsaufnahme, vorzugsweise ausgewählt aus der Gruppe bestehend aus Bulimie, Kachexie, Anorexie und Fettleibigkeit; Alkohol- und/oder Drogen (insbesondere Nikotin- und/oder Kokain-) und/oder Medikamentenmißbrauch; Alkohol- und/oder Drogen (insbesondere Nikotin- und/oder Kokain-) und/oder Medikamentenabhängigkeit; vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; zur Prophylaxe und/oder Verminderung von Toleranzerscheinungen gegenüber Drogen- und/oder Medikamenten, insbesondere gegenüber Opioiden; des Magen-Ösophagus-Reflux-Syndroms; des gastroösophagealen Rückflusses; zur Diurese; zur Antinatriurese; zur Beeinflussung des kardiovaskulären Systems; zur Anxiolyse; zur Vigilanzsteigerung; zur Libidosteigerung; zur Modulation der Bewegungsaktivität und zur Lokalanästhesie.

31. Verwendung wenigstens einer Verbindung gemäß einem oder mehreren der Ansprüche 1 bis 24 zur Herstellung eines Arzneimittels zur Prophylaxe und/oder Behandlung von Störungen und/oder Krankheiten, die zumindest teilweise durch mGluR5-Rezeptoren vermittelt werden.

32. Verwendung wenigstens einer Verbindung gemäß einem oder mehreren der Ansprüche 1 bis 24 zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz.

33. Verwendung wenigstens einer Verbindung gemäß einem oder mehreren der Ansprüche 1 bis 24 zur Herstellung eines Arzneimittels zur Behandlung und/oder Prophylaxe von Migräne; Depressionen; neurodegenerativen Erkrankungen, vorzugsweise ausgewählt aus der Gruppe bestehend aus Morbus Parkinson, Morbus Alzheimer, Morbus Huntington und Multipler Sklerose; kognitiven Erkrankungen, vorzugsweise kognitiven Mangelzuständen, besonders bevorzugt des Aufmerksamkeit-Defizit-Syndroms (ADS); Panikattacken; Angstsyndrom; Epilepsie; Husten; Haminkontinenz; Diarrhöe; Pruritus; Schizophrenie; cerebralen Ischämien; Muskelspasmen; Krämpfen; Reizdarmsyndrom; Störungen der Nahrungsaufnahme, vorzugsweise ausgewählt aus der Gruppe bestehend aus Bulimie, Kachexie, Anorexie und Fettleibigkeit; Alkohol- und/oder Drogen (insbesondere Nikotin- und/oder Kokain-) und/oder Medikamentenmißbrauch, Alkohol- und/oder Drogen (insbesondere Nikotin- und/oder Kokain-) und/oder Medikamentenabhängigkeit, vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; der Entwicklung von Toleranzerscheinungen gegenüber Drogen- und/oder Medikamenten, insbesondere gegenüber Opioiden; des Magen-Ösophagus-Reflux-Syndroms; des gastroösophagealen Rückflusses; zur Diurese; zur Antinatriurese; zur Beeinflussung des kardiovaskulären Systems; zur Anxiolyse; zur Vigilanzsteigerung; zur Libidosteigerung; zur Modulation der Bewegungsaktivität und zur Lokalanästhesie.

## Claims

1. Substituted bicyclic imidazo-3-yl-amine compounds of the general formula I, 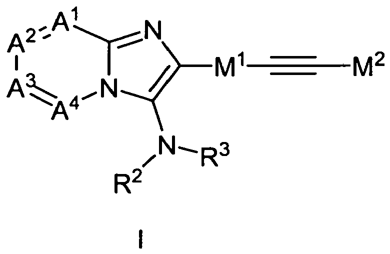 in which
A¹ denotes a nitrogen atom or a C-R^{1a} group,
A² denotes a nitrogen atom or a C-R^{1b} group,
A³ denotes a nitrogen atom or a C-R^{1c} group,
A⁴ denotes a nitrogen atom or a C-R^{1d} group,
R^{1a}, R^{1b}, R^{1c}, R^{1d}, mutually independently, in each case denote a hydrogen residue; a halogen residue; -NO₂; -CN; -NH₂; -NHR⁴; -NR⁵R⁶; -NH-C(=O)-R⁷; -C(=O)-R⁸, -C(=O)-NH₂; -C(=O)-NHR⁹; -C(=O)-NR¹⁰R¹¹; -C(=O)-OR¹²; -(CH₂)ₘ-C(=O)-OR¹³ with m = 1, 2, 3, 4 or 5; -O-C(=O)-R¹⁴; -(CH₂)ₙ-O-C(=O)-R¹⁵ with n = 1, 2, 3, 4 or 5; -OR¹⁶; -(CH₂)ₒ-O-R¹⁷ with o = 1, 2, 3; 4 or 5; -SR¹⁸; -(CH₂)ₚ-S(=O)ₜ-R¹⁹ with p = 1, 2, 3, 4 or 5 and t = 0, 1 or 2; -NH-S(=O)₂-R²⁶R²⁷; -S(=O)₂-NR²⁸R²⁹, -SF₅; a linear or branched, saturated or unsaturated, unsubstituted or at least monosubstituted aliphatic residue; a saturated or unsaturated, unsubstituted or at least monosubstituted cycloaliphatic residue optionally comprising at least one heteroatom as a ring member, which cycloaliphatic residue may be attached via a linear or branched alkylene group and/or may be fused with an unsubstituted or at least monosubstituted mono- or polycyclic ring system; or denote an unsubstituted or at least monosubstituted aryl or heteroaryl residue, which may be attached via a linear or branched alkylene group and/or may be fused with an unsubstituted or at least monosubstituted mono- or polycyclic ring system,
or optionally R^{1a} and R^{1b} form an unsubstituted or at least monosubstituted anellated phenyl residue with the C-C bridge joining them together,
or optionally R^{1b} and R^{1c} form an unsubstituted or at least monosubstituted anellated phenyl residue with the C-C bridge joining them together,
or optionally R^{1c} and R^{1d} form an unsubstituted or at least monosubstituted anellated phenyl residue with the C-C bridge joining them together,
R² and R³, mutually independently, in each case denote a hydrogen residue; -C(=O)-R²⁰; -(CH₂)_{q}-C(=O)-R²¹ with q = 1, 2, 3, 4 or 5; -C(=O)-O-R²²; -(CH₂)ᵣ-C(=O)-O-R²³ with r = 1, 2, 3, 4 or 5; -C(=O)-NHR²⁴; -(CH₂)s-C(=O)-NHR²⁵ with s = 1, 2, 3, 4 or 5; a linear or branched, saturated or unsaturated, unsubstituted or at least monosubstituted aliphatic residue; a saturated or unsaturated, unsubstituted or at least monosubstituted cycloaliphatic residue optionally comprising at least one heteroatom as a ring member, which cycloaliphatic residue may be attached via a linear or branched alkylene group and/or may be fused with an unsubstituted or at least monosubstituted mono- or polycyclic ring system; or denote an unsubstituted or at least monosubstituted aryl or heteroaryl residue, which may be attached via a linear or branched alkylene group and/or may be fused with an unsubstituted or at least monosubstituted mono- or polycyclic ring system,
or R² and R³, together with the nitrogen atom joining them together as a ring member, form a saturated or unsaturated heterocycloaliphatic residue optionally comprising at least one further heteroatom as a ring member, which heterocycloaliphatic residue may be fused with an unsubstituted or at least monosubstituted mono- or polycyclic ring system,
R⁴, R⁵, R⁶, R⁷, R⁹, R¹⁰, R¹¹, R¹⁴ and R¹⁵, in each case mutually independently, denote a linear or branched, saturated or unsaturated aliphatic residue or an unsubstituted or at least monosubstituted aryl or heteroaryl residue, which may be attached via a linear or branched alkylene group and/or may be fused with an unsubstituted or at least monosubstituted mono- or polycyclic ring system,
R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ and R²⁹, in each case mutually independently, denote a hydrogen residue; a linear or branched, saturated or unsaturated aliphatic residue or an unsubstituted or at least monosubstituted aryl or heteroaryl residue, which may be attached via a linear or branched alkylene group and/or may be fused with an unsubstituted or at least monosubstituted mono- or polycyclic ring system,
M¹ denotes an aryl or heteroaryl residue, which may be substituted with at least one further substituent and/or fused with an unsubstituted or at least monosubstituted mono- or polycyclic ring system,
M² denotes an aryl or heteroaryl residue, which is unsubstituted or at least monosubstituted and optionally fused with an unsubstituted or at least monosubstituted mono- or polycyclic ring system,
wherein
if one or more of the substituents R^{1a}, R^{1b}, R^{1c}, R^{1d} and R² to R²⁹ denote a saturated or unsaturated aliphatic residue, this may be substituted with 1, 2, 3, 4 or 5 substituents mutually independently selected from the group consisting of F, Cl, Br, -NO₂, -CN, -OH, -SH and -NH₂,
if one or more of the substituents R^{1a}, R^{1b}, R^{1c}, R^{1d}, R² and R³ denote a cycloaliphatic residue or comprise a cycloaliphatic residue, which is mono- or polysubstituted, this may optionally be substituted with 1, 2, 3, 4 or 5 substituents mutually independently selected from the group consisting of F, Cl, Br, I, -CN, -CF₃, -SF₅, -OH, -NH₂, -O-CF₃, -SH, -O-C₁₋₅-alkyl, -(CH₂)-O-C₁₋₅-alkyl, -S-C₁₋₅-alkyl, -C₁₋₅ alkyl, -C₂₋₅ alkenyl, -C₂₋₅ alkynyl, -C(=O)-O-C₁₋₅-alkyl, -C(=O)-CF₃, -S(=O)₂-C₁₋₅-alkyl, -S(=O)-C₁₋₅-alkyl, -S(=O)₂-phenyl, oxo (=O), thioxo (=S), -N(C₁₋₅-alkyl)₂, -N(H)(C₁₋₅-alkyl), -NO₂, -S-CF₃, -C(=O)-OH, -NH-S(=O)₂-C₁₋₅-alkyl, -NH-C(=O)-C₁₋₅-alkyl, -C(=O)-H; -C(=O)-C₁₋₅-alkyl, -C(=O)-NH₂, -C(=O)-N(C₁₋₅-alkyl)₂, -C(=O)-N(H)(C₁₋₅-alkyl) and phenyl, wherein the above-stated C₁₋₅ alkyl residues may in each case be linear or branched and the phenyl residues may in each case be unsubstituted or substituted with 1, 2, 3 or 4 substituents mutually independently selected from the group consisting of F, Cl, Br, I, methyl, ethyl, methoxy and ethoxy,
if the cycloaliphatic residues comprise one or more heteroatoms as ring members, these may comprise 1, 2, 3, 4 or 5 heteroatoms as ring member(s), which may be selected in each case mutually independently from the group consisting of nitrogen, oxygen and sulfur,
if the substituents R² and R³ together with the nitrogen atom joining them together as a ring member, form a saturated or unsaturated heterocycloaliphatic residue, which is mono- or polysubstituted, this may optionally be substituted with 1, 2, 3, 4, or 5 substituents mutually independently selected from the group consisting of F, Cl, Br, I, -CN, -CF₃, -SF₅, -OH, -NH₂, -O-CF₃, -SH, -O-C₁₋₅-alkyl, -(CH₂)-O-C₁₋₅-alkyl, -S-C₁₋₅-alkyl, -C₁₋₅ alkyl, -C₂₋₅ alkenyl, -C₂₋₅ alkynyl, -C(=O)-O-C₁₋₅-alkyl, -C(=O)-CF₃, -S(=O)₂-C₁₋₅-alkyl, -S(=O)-C₁₋₅-alkyl, -S(=O)₂-phenyl, oxo (=O), thioxo (=S), -N(C₁₋₅-alkyl)₂, -N(H)(C₁₋₅-alkyl), -NO₂, -S-CF₃, -C(=O)-OH, -NH-S(=O)₂-C₁₋₅-alkyl, -NH-C(=O)-C₁₋₅-alkyl, -C(=O)-H; -C(=O)-C₁₋₅-alkyl, -C(=O)-NH₂, -C(=O)-N(C₁₋₅-alkyl)₂, -C(=O)-N(H)(C₁₋₅-alkyl) and phenyl, wherein the above-stated C₁₋₅ alkyl residues may in each case be linear or branched and the phenyl residues may in each case be unsubstituted or substituted with 1, 2, 3 or 4 substituents mutually independently selected from the group consisting of F, Cl, Br, I, methyl, ethyl, methoxy and ethoxy,
if the heterocycloaliphatic residues comprise one or more heteroatoms as ring members, these comprise 1, 2, 3, 4 or 5, heteroatoms as ring member(s), which may, in each case mutually independently, be selected from the group consisting of nitrogen, oxygen and sulfur,
if one or more of the substituents R^{1a}, R^{1b}, R^{1c}, R^{1d}, R² to R²⁹ and M¹ and M² denote an aryl or heteroaryl residue or comprise an aryl or heteroaryl residue, which is mono- or polysubstituted, this may optionally be substituted with 1, 2, 3, 4 or 5 substituents mutually independently selected from the group consisting of halogen, -CN, -CH₂-CN, -NO₂, -OH, -SH, -NH₂, -CH₂-NH₂, -C(=O)-OH, C₁₋₅ alkyl, -(CH₂)-OH, -(CH₂)O-C₁₋₅-alkyl, -C₂₋₅ alkenyl, -C₂₋₅ alkynyl, -S-C₁₋₅-alkyl, -O-C₁₋₅-alkyl, -CF₃, -SF₅, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, -S(=O)₂-C₁₋₅-alkyl, -S(=O)-C₁₋₅-alkyl, -NH-C₁₋₅-alkyl, -N-(C₁₋₅-alkyl)₂, -CH₂-NH-C₁₋₅-alkyl, -CH₂-N-(C₁₋₅-alkyl)₂, -C(=O)-O-C₁₋₅-alkyl, -C(=O)-H, -C(=O)-C₁₋₅-alkyl, -CH₂-O-C(=O)-phenyl, -O-C(=O)-phenyl, -NH-S(=O)₂-C₁₋₅-alkyl, -NH-C(=O)-C₁₋₅-alkyl, -S(=O)₂-NH₂, -S(=O)₂-NH-C₁₋₅-alkyl, -S(=O)₂-N(C₁₋₅-alkyl)₂, -NH-C(=NH)-NH₂, -NH-S(=O)₂-OH, -C(=O)-NH-C₁₋₅-alkyl, -C(=O)-NH₂, -C(=O)-N(C₁₋₅-alkyl)₂, -Si(phenyl)₂[C₁₋₅-alkyl], pyrazolyl, pyrrolyl, (1,3)-dioxolanyl, phenyl, furyl (furanyl), thiazolyl, thiadiazolyl, thiophenyl (thienyl), phenoxy, benzyl and phenethyl, wherein the cyclic substituents may themselves in each case optionally be substituted with 1, 2, 3 or 4 substituents mutually independently selected from the group consisting of F, Cl, Br, I, -CF₃, methyl, ethyl, methoxy and ethoxy,
if one or more of the substituents R^{1a}, R^{1b}, R^{1c}, R^{1d}, R² to R²⁹ and M¹ and M² denote a heteroaryl residue or comprise a heteroaryl residue, the heteroatom(s) thereof may be selected, mutually independently, from the group consisting of oxygen, sulfur and nitrogen,
if the substituents R^{1a} and R^{1b} or R^{1b} and R^{1c} or R^{1c} and R^{1d}, together with the C-C bridge joining them together, form an anellated phenyl residue, which is mono- or polysubstituted, this may be substituted with 1, 2, 3 or 4 substituents mutually independently selected from the group consisting of halogen, -CN, -CH₂-CN, -NO₂, -OH, -SH, -NH₂, -CH₂-NH₂, -C(=O)-OH, C₁₋₅ alkyl, -(CH₂)-O-C₁₋₅ alkyl, -C₂₋₅ alkenyl, -C₂₋₅ alkynyl, -S-C₁₋₅-alkyl, -O-C₁₋₅-alkyl, -CF₃, -SF₅, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, -S(=O)₂-C₁₋₅-alkyl, -S(=O)-C₁₋₅-alkyl, -NH-C₁₋₅-alkyl, -N-(C₁₋₅-alkyl)₂, -CH₂-NH-C₁₋₅-alkyl, -CH₂-N-(C₁₋₅-alkyl)₂, -C(=O)-O-C₁₋₅-alkyl, -C(=O)-H, -C(=O)-C₁₋₅-alkyl, -CH₂-O-C(=O)-phenyl, -O-C(=O)-phenyl, -NH-S(=O)₂-C₁₋₅-alkyl, -NH-C(=O)-C₁₋₅-alkyl, -C(=O)-NH-C₁₋₅-alkyl, -C(=O)-NH₂, -C(=O)-N(C₁₋₅-alkyl)₂, -Si(phenyl)₂[C₁₋₅-alkyl], pyrazolyl, pyrrolyl, (1,3)-dioxolanyl, phenyl, furyl (furanyl), thiazolyl, thiadiazolyl, thiophenyl (thienyl), phenoxy, benzyl and phenethyl, wherein the cyclic substituents themselves may in each case optionally be substituted with 1, 2, 3 or 4 substituents mutually independently selected from the group consisting of F, Cl, Br, I, -CF₃, methyl, ethyl, methoxy and ethoxy,
if a polycyclic ring system is present, the various rings may in each case mutually independently be saturated, unsaturated or aromatic and the heteroatoms of each ring may, in each case mutually independently, be selected from the group consisting of oxygen, nitrogen and sulfur,
if one or more of the substituents R^{1a,} R^{1b,} R^{1c,} R^{1d,} R² to R²⁹ and M¹ and M² comprise a monocyclic or polycyclic ring system, which is mono- or polysubstituted, this may optionally be substituted with 1, 2, 3, 4 or 5 substituents, which may mutually independently be selected from the group consisting of halogen, -CN, -NO₂, -OH, -SH, -NH₂, oxo (=O), thioxo (=S), -C(=O)-OH, -C₁₋₅ alkyl, -C₂₋₅ alkenyl, -C₂₋₅ alkynyl, -(CH₂)-O-C₁₋₅-alkyl, -S-C₁₋₅-alkyl, -O-C₁₋₅-alkyl, -CF₃, -SF₅, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, -S(=O)₂-C₁₋₅-alkyl, -S(=O)-C₁₋₅-alkyl, -NH-C₁₋₅-alkyl, -N(C₁₋₅-alkyl)(C₁₋₅-alkyl), -C(=O)-O-C₁₋₅-alkyl, -C(=O)-H, -C(=O)-C₁₋₅-alkyl, -CH₂-O-C(=O)-phenyl, -O-C(=O)-phenyl, -NH-S(=O)₂-C₁₋₅-alkyl, -NH-C(=O)-C₁₋₅-alkyl, -C(=O)-NH₂, -C(=O)-NH-C₁₋₅-alkyl, -C(=O)-N(C₁₋₅-alkyl)₂, pyrazolyl, phenyl, furyl (furanyl), thiadiazolyl, thiophenyl (thienyl), phenoxy and benzyl, wherein the cyclic substituents may themselves in each case optionally be substituted with 1, 2, 3 or 4 substituents mutually independently selected from the group consisting of F, Cl, Br, I, methyl, ethyl, methoxy and ethoxy,
in each case in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

2. Compounds according to claim 1, **characterised in that**
A¹ denotes a nitrogen atom or a C-R^{1a} group,
A² denotes a nitrogen atom or a C-R^{1b} group,
A³ denotes a nitrogen atom or a C-R^{1c} group,
A⁴ denotes a nitrogen atom or a C-R^{1d} group,
R^{1a}, R^{1b}, R^{1c}, R^{1d}, mutually independently, in each case denote a hydrogen residue; a halogen residue; -NO₂; -CN; -NH₂; -NHR⁴; -NR⁵R⁶; -NH-C(=O)-R⁷; -C(=O)-R⁸, -C(=O)-NH₂; -C(=O)-NHR⁹; -C(=O)-NR¹⁰R¹¹; -C(=O)-OR¹²; -(CH₂)ₘ-C(=O)-OR¹³ with m = 1, 2, 3, 4 or 5; -O-C(=O)-R¹⁴; -(CH₂)_{N}-O-C(=O)-R¹⁵ with N = 1, 2, 3, 4 or 5; -OR¹⁶; -(CH₂)ₒ-O-R¹⁷ with o = 1, 2, 3; 4 or 5; -SR¹⁸; -(CH₂)ₚ-S(=O)ₜ-R¹⁹ with p = 1, 2, 3, 4 or 5 and t = 0, 1 or 2; -NH-S(=O)₂-R²⁶R²⁷; -S(=O)₂-NR²⁸R²⁹,-SF₅; a linear or branched, saturated or unsaturated, unsubstituted or at least monosubstituted aliphatic C₁₋₁₀ residue; a saturated or unsaturated, unsubstituted or at least monosubstituted cycloaliphatic C₃₋₈ residue optionally comprising at least one heteroatom as a ring member, which cycloaliphatic C₃₋₈ residue may be attached via a linear or branched C₁₋₅ alkylene group; or denote an unsubstituted or at least monosubstituted 5- to 14-membered aryl or heteroaryl residue which may be attached via a linear or branched C₁₋₅ alkylene group,
or optionally R^{1a} and R^{1b} form an unsubstituted or at least monosubstituted anellated phenyl residue with the C-C bridge joining them together,
or optionally R^{1b} and R^{1c} form an unsubstituted or at least monosubstituted anellated phenyl residue with the C-C bridge joining them together,
or optionally R^{1c} and R^{1d} form an unsubstituted or at least monosubstituted anellated phenyl residue with the C-C bridge joining them together,
R² and R³, mutually independently, in each case denote a hydrogen residue; -C(=O)-R²⁰; -(CH₂)_{q}-C(=O)-R²¹ with q = 1, 2, 3, 4 or 5; -C(=O)-O-R²²; -(CH₂)ᵣ-C(=O)-O-R²³ with r = 1, 2, 3, 4 or 5; -C(=O)-NHR²⁴; -(CH₂)ₛ-C(=O)-NHR²⁵ with s = 1, 2, 3, 4 or 5; a linear or branched, saturated or unsaturated, unsubstituted or at least monosubstituted aliphatic C₁₋₁₆ residue; a saturated or unsaturated, unsubstituted or at least monosubstituted cycloaliphatic C₄₋₈ residue, optionally comprising at least one heteroatom as a ring member, which cycloaliphatic C₄₋₈ residue may be attached via a linear or branched C₁₋₅ alkylene group; or denote an unsubstituted or at least monosubstituted 5- to 14-membered aryl or heteroaryl residue, which may be attached via a linear or branched C₁₋₅ alkylene group,
or R² and R³, together with the nitrogen atom joining them together as a ring member, form a saturated or unsaturated heterocycloaliphatic C₄₋₁₀ residue optionally comprising at least one further heteroatom as a ring member, which heterocycloaliphatic C₄₋₁₀ residue is optionally fused with an unsubstituted or at least monosubstituted mono- or polycyclic ring system, wherein the rings of the ring system are in each case 5-, 6- or 7-membered;
R⁴, R⁵, R⁶, R⁷, R⁹, R¹⁰, R¹¹, R¹⁴ and R¹⁵, in each case mutually independently, denote a linear or branched, saturated or unsaturated aliphatic C₁₋₄ residue or denote an unsubstituted or at least monosubstituted 5- to 14-membered aryl or heteroaryl residue, which may be attached via a linear or branched C₁₋₅ alkylene group,
R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ and R²⁹, in each case mutually independently, denote a hydrogen residue; a linear or branched, saturated or unsaturated aliphatic C₁₋₄ residue or an unsubstituted or at least monosubstituted 5- to 14-membered aryl or heteroaryl residue, which may be attached via a linear or branched C₁₋₅ alkylene group,
M¹ denotes a 5- or 6-membered aryl or heteroaryl residue, which may be substituted with at least one further substituent and is optionally fused with an unsubstituted or at least monosubstituted mono- or bicyclic ring system, wherein the rings of the ring system are in each case 5-, 6- or 7-membered, and
M² denotes a 5- or 6-membered aryl or heteroaryl residue, which may be unsubstituted or at least monosubstituted and may be fused with an unsubstituted or at least monosubstituted mono- or bicyclic ring system, wherein the rings of the ring system are in each case 5-, 6- or 7-membered;
wherein
the above-stated cycloaliphatic residues may optionally comprise 1, 2, 3, 4 or 5 heteroatom(s) as ring member(s), which may be selected, in each case mutually independently, from the group consisting of nitrogen, oxygen and sulfur,
the above-stated heterocycloaliphatic residues may optionally comprise a further 1, 2, 3, 4 or 5 heteroatom(s) as ring member(s), which may be selected, in each case mutually independently, from the group consisting of nitrogen, oxygen and sulfur,
the rings of the mono- or polycyclic ring system in each case optionally comprise 0, 1, 2 or 3 heteroatom(s) as ring member(s), which are selected mutually independently from the group consisting of oxygen, nitrogen and sulfur;
and
the above-stated heteroaryl residues may optionally comprise 1, 2, 3, 4 or 5 heteroatom(s) as ring member(s), which may be selected, in each case mutually independently, from the group consisting of nitrogen, oxygen and sulfur.

3. Compounds according to claim 1 or claim 2, **characterised in that**
A¹ denotes a C-R^{1a} group,
A² denotes a C-R^{1b} group,
A³ denotes a C-R^{1c} group and
A⁴ denotes a C-R^{1d} group,
or
A¹ denotes a nitrogen atom,
A² denotes a C-R^{1b} group,
A³ denotes a C-R^{1c} group and
A⁴ denotes a C-R^{1d} group,
or
A¹ denotes a C-R^{1a} group,
A² denotes a nitrogen atom,
A³ denotes a C-R^{1c} group and
A⁴ denotes a C-R^{1d} group,
or
A¹ denotes a C-R^{1a} group,
A² denotes a C-R^{1b} group,
A³ denotes a nitrogen atom and
A⁴ denotes a C-R^{1d} group,
or
A¹ denotes a C-R^{1a} group,
A² denotes a C-R^{1b} group,
A³ denotes a C-R^{1c} group and
A⁴ denotes a nitrogen atom,
or
A¹ and A³ in each case denote a nitrogen atom,
A² denotes a C-R^{1b} group, and
A⁴ denotes a C-R^{1d} group.

4. Compounds according to claim 3, **characterised in that**
A¹ denotes a C-R^{1a} group,
A² denotes a C-R^{1b} group,
A³ denotes a C-R^{1c} group and
A⁴ denotes a C-R^{1d} group,
or
A¹ denotes a nitrogen atom,
A² denotes a C-R^{1b} group,
A³ denotes a C-R^{1c} group and
A⁴ denotes a C-R^{1d} group,
or
A¹ denotes a C-R^{1a} group,
A² denotes a nitrogen atom,
A³ denotes a C-R^{1c} group and
A⁴ denotes a C-R^{1d} group,
or
A¹ denotes a C-R^{1a} group,
A² denotes a C-R^{1b} group,
A³ denotes a C-R^{1c} group and
A⁴ denotes a nitrogen atom.

5. Compounds according to one or more of claims 1 to 4, **characterised in that** R^{1a}, R^{1b}, R^{1c}, R^{1d}, mutually independently, in each case denote -H; -F; -Cl, -Br; -I; -NO₂; -CN; -CF₃; -SF₅; -NH₂; -S(=O)-NH₂, -NHR⁴; -NR⁵R⁶; -C(=O)-OR¹²; -(CH₂)ₘ-C(=O)-OR¹³ with m = 1, 2 or 3; -O-C(=O)-R¹⁴; -OR¹⁶; -(CH₂)ₒ-O-R¹⁷ with o = 1, 2 or 3; a linear or branched C₁₋₁₀ alkyl residue; or denote an unsubstituted or at least monosubstituted 5- or 6-membered aryl or heteroaryl residue, which may be attached via a linear or branched C₁₋₄ alkylene group.

6. Compounds according to one or more of claims 1 to 5, **characterised in that** R^{1a} and R^{1b} or R^{1b} and R^{1c} or R^{1c} and R^{1d}, together with the C-C bridge joining them together, form an anellated phenyl residue which may be substituted with 1, 2, 3 or 4 substituents mutually independently selected from the group consisting of F, Cl, Br, -CN, methyl, ethyl, n-propyl, iso-propyl, n-butyl, sec-butyl, iso-butyl, tert-butyl, -OH, -O-CH₃, -O-C₂H₅.

7. Compounds according to one or more of claims 1 to 6, **characterised in that** R² and R³, mutually independently, in each case denote a hydrogen residue; -C(=O)-R²⁰; -(CH₂)_{q}-C(=O)-R²¹ with q = 1, 2, 3, 4 or 5; -(CH₂)ᵣ-C(=O)-O-R²³ with r = 1, 2, 3, 4 or 5; -C(=O)-NHR²⁴; a linear or branched C₁₋₁₆ alkyl residue; an unsubstituted or at least monosubstituted C₄₋₈ cycloalkyl residue, which may be attached via a linear or branched C₁₋₃ alkylene group; or denote an unsubstituted or at least monosubstituted, 5- or 6-membered aryl or heteroaryl residue, which may be attached via a linear or branched C₁₋₃ alkylene.

8. Compounds according to one or more of claims 1 to 7, **characterised in that** R² and R³, together with the nitrogen atom joining them together as a ring member, form a residue selected from the group consisting of imidazolidinyl, piperidinyl, piperazinyl, morpholinyl, pyrrolidinyl, thiomorpholinyl, azepanyl, diazepanyl and azocanyl, which may in each case be unsubstituted or optionally substituted with 1, 2, 3, 4 or 5 substituents mutually independently selected from the group consisting of F, Cl, Br, I, -CN, methyl, ethyl, n-propyl, iso-propyl, n-butyl, sec-butyl, iso-butyl, tert-butyl, -OH, oxo, thioxo, -O-CH₃, -O-C₂H₅, -O-C₃H₇, -NH₂, -N(CH₃)₂, -N(C₂H₅)₂, -NH-CH₃, -NH-C₂H₅, -O-CF₃, -S-CF₃, -S(=O)-CH₃, -S(=O)-C₂H₅, -S(=O)₂-CH₃, -S(=O)₂-C₂H₅, -C(=O)-OH, -C(=O)-H, -C(=O)-CH₃, -C(=O)-C₂H₅, -C(=O)-N(CH₃)₂, -C(=O)-NH-CH₃, -C(=O)-NH₂, -C(=O)-O-CH₃, -C(=O)-O-C₂H₅ and -C(=O)-O-C(CH₃)₃.

9. Compounds according to one or more of claims 1 to 8, **characterised in that** R⁴, R⁵, R⁶, R⁷, R⁹, R¹⁰, R¹¹, R¹⁴ and R¹⁵, in each case mutually independently, denote a linear or branched C₁₋₄ alkyl residue or an unsubstituted or at least monosubstituted 5- to 14-membered aryl or heteroaryl residue, which may be attached via a linear or branched C₁₋₅ alkylene group.

10. Compounds according to one or more of claims 1 to 9, **characterised in that** R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ and R²⁹, in each case mutually independently denote a hydrogen residue; a linear or branched C₁₋₈ alkyl residue, or denote an unsubstituted or at least monosubstituted 5- to 14-membered aryl or heteroaryl residue, which may be attached via a linear or branched C₁₋₅ alkylene group.

11. Compounds according to one or more of claims 1 to 10, **characterised in that** M¹ denotes a 5- or 6-membered aryl or heteroaryl residue, which may optionally be substituted with at least one further substituent, wherein the heteroaryl residue optionally comprises 1, 2, 3, 4 or 5 heteroatom(s) as ring member(s), which are mutually independently selected from the group consisting of nitrogen, oxygen and sulfur.

12. Compounds according to claim 11, **characterised in that** M¹ denotes a residue selected from the group consisting of residues 1 to 38, 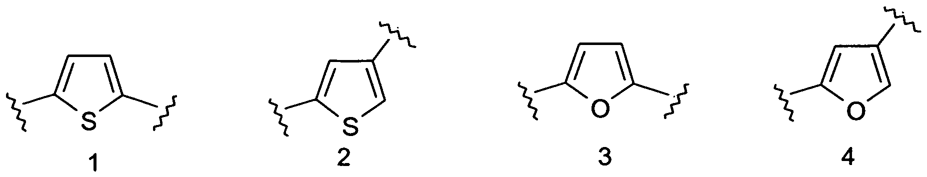 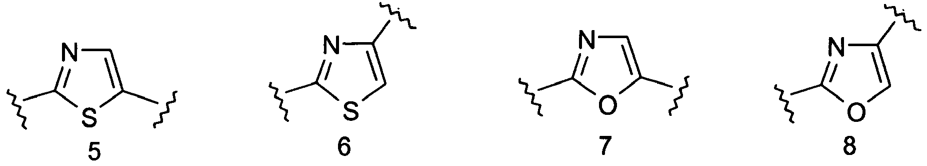 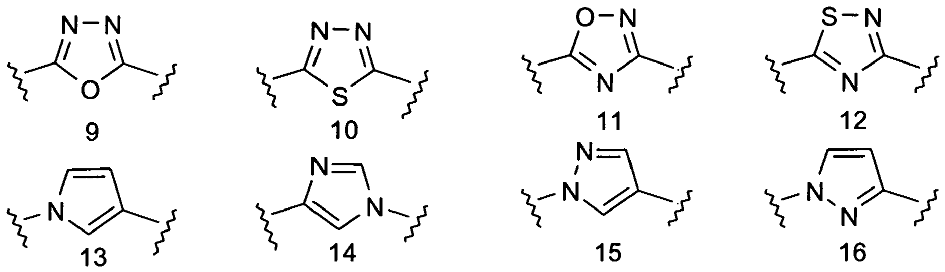 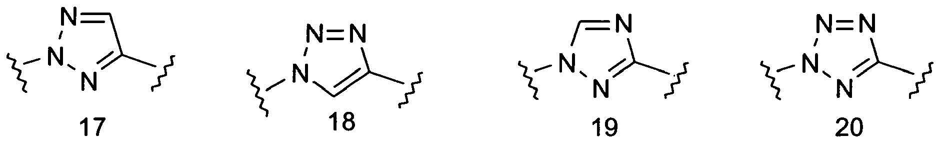 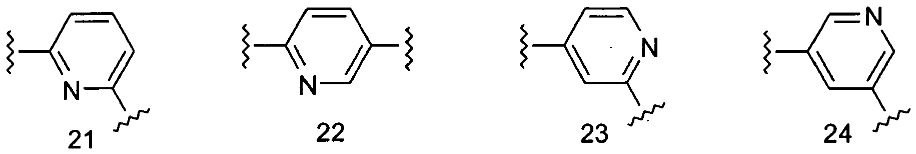 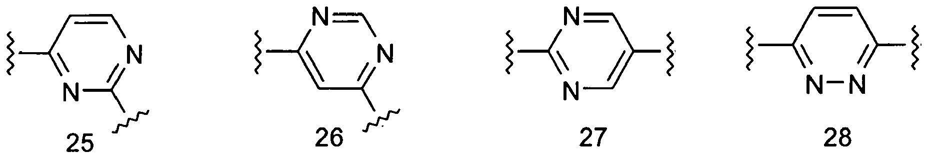 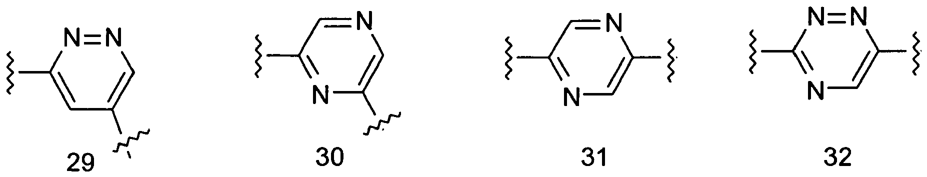 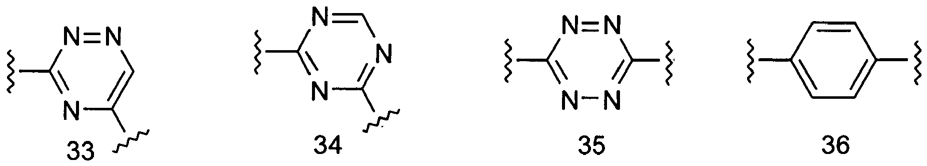 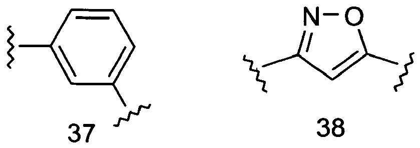 which may in each case be linked in any desired direction via the positions indicated with a wavy line to the bicyclic ring system and to the carbon atom of the triple bond and which is optionally substituted with 1, 2, 3 or 4 further substituents, which are mutually independently selected from the group consisting of F, Cl, Br, -CN, -CH₂-CN, -CF₃, -SF₅, methyl, ethyl, n-propyl, iso-propyl, n-butyl, sec-butyl, iso-butyl, tert-butyl, -OH, -O-CH₃, -O-C₂H₅, -O-C₃H₇, -NH₂, -N(CH₃)₂, -N(C₂H₅)₂, -NO₂, -O-CF₃, -S-CF₃, -SH, -C(=O)-CH₃ and -C(=O)-C₂H₅.

13. Compounds according to one or more of claims 1 to 12, **characterised in that** M² denotes an unsubstituted or at least monosubstituted, 5- or 6-membered aryl or heteroaryl residue, wherein the heteroaryl residue comprises 1, 2, 3, 4 or 5 heteroatom(s) as ring member(s), which are mutually independently selected from the group consisting of nitrogen, oxygen and sulfur, and wherein the aryl or heteroaryl residue may be fused with an unsubstituted or at least monosubstituted mono- or bicyclic ring system, wherein the rings of the ring system are in each case 5- or 6-membered and may in each case comprise 1, 2, 3 or 4 heteroatom(s) as ring member(s), which are mutually independently selected from the group consisting of nitrogen, oxygen and sulfur.

14. Compounds according to one or more of claims 1 to 13, **characterised in that**
A¹ denotes a C-R^{1a} group,
A² denotes a C-R^{1b} group,
A³ denotes a C-R^{1c} group and
A⁴ denotes a C-R^{1d} group;
or
A¹ denotes a nitrogen atom,
A² denotes a C-R^{1b} group,
A³ denotes a C-R^{1c} group and
A⁴ denotes a C-R^{1d} group;
or
A¹ denotes a C-R^{1a} group,
A² denotes a nitrogen atom,
A³ denotes a C-R^{1c} group and
A⁴ denotes a C-R^{1d} group:
R^{1a}, R^{1b}, R^{1c}, R^{1d}, mutually independently, in each case denote -H; -F; -Cl, -Br; -I; -NO₂; -CN; -CF₃; -SF₅; -NH₂; -S(=O)-NH₂,-NHR⁴; -NR⁵R⁶; -C(=O)-OR¹²; -(CH₂)ₘ-C(=O)-OR¹³ with m = 1, 2 or 3; -O-C(=O)-R¹⁴; -OR¹⁶; -(CH₂)ₒ-O-R¹⁷ with o = 1, 2 or 3; a linear or branched alkyl residue selected from the group consisting of methyl, ethyl, n-propyl, iso-propyl, n-butyl, iso-butyl, sec-butyl and tert-butyl, or denote an aryl or heteroaryl residue selected from the group consisting of phenyl, furanyl, thiophenyl and pyridinyl, which is in each case unsubstituted or substituted with 1, 2, 3, 4 or 5 substituents mutually independently selected from the group consisting of F, Cl, Br, -CN, methyl, ethyl, n-propyl, iso-propyl, n-butyl, sec-butyl, iso-butyl, tert-butyl, -OH, -O-CH₃, -O-C₂H₅ and is optionally attached via a -(CH₂)-, -(CH₂)₂- or -(CH₂)₃ group;
or optionally R^{1a} and R^{1b} or optionally R^{1b} and R^{1c} or optionally R^{1c} and R^{1d}, together with the C-C bridge joining them together, form an anellated phenyl residue, which may be substituted with 1, 2, 3 or 4 substituents mutually independently selected from the group consisting of F, Cl, Br, -CN, methyl, ethyl, n-propyl, iso-propyl, n-butyl, sec-butyl, iso-butyl, tert-butyl, -OH, -O-CH₃, -O-C₂H₅;
R² and R³, mutually independently, in each case denote a hydrogen residue; -C(=O)-R²⁰; -(CH₂)_{q}-C(=O)-R²¹ with q = 1, 2 or 3; -(CH₂)ᵣ-C(=O)-O-R²³ with r = 1, 2 or 3; a linear or branched C₁₋₁₀ alkyl residue; a C₄₋₈ cycloalkyl residue, which is in each case unsubstituted or substituted with 1, 2, 3, 4 or 5 substituents mutually independently selected from the group consisting of F, Cl, Br, -CN, -CF₃, methyl, ethyl, n-propyl, iso-propyl, n-butyl, sec-butyl, iso-butyl, tert-butyl, -OH, -O-CH₃ and -O-C₂H₅ and is optionally attached via a linear or branched C₁₋₃ alkylene group; or denote an aryl or heteroaryl residue selected from the group consisting of phenyl, thiophenyl, furanyl and pyridinyl, which is in each case unsubstituted or substituted with 1, 2, 3, 4 or 5 substituents mutually independently selected from the group consisting of F, Cl, Br, -CN, -CF₃, methyl, ethyl, n-propyl, iso-propyl, n-butyl, sec-butyl, iso-butyl, tert-butyl, -OH, -O-CH₃ and -O-C₂H₅ and is optionally attached via a linear or branched C₁₋₃ alkylene group;
or R² and R³, together with the nitrogen atom joining them together as a ring member, form a residue selected from the group consisting of imidazolidinyl, piperidinyl, piperazinyl, morpholinyl, pyrrolidinyl, thiomorpholinyl, azepanyl, diazepanyl and azocanyl, which may in each case be unsubstituted or optionally substituted with 1, 2, 3, 4 or 5 substituents mutually independently selected from the group consisting of F, Cl, Br, I, -CN, methyl, ethyl, n-propyl, iso-propyl, n-butyl, sec-butyl, iso-butyl, tert-butyl, -OH, oxo, thioxo, -O-CH₃, -O-C₂H₅, -O-C₃H₇, -NH₂, -N(CH₃)₂, -N(C₂H₅)₂, -NH-CH₃, -NH-C₂H₅, -O-CF₃, -S-CF₃, -S(=O)-CH₃, -S(=O)-C₂H₅, -S(=O)₂-CH₃, -S(=O)₂-C₂H₅, -C(=O)-OH, -C(=O)-H, -C(=O)-CH₃, -C(=O)-C₂H₅, -C(=O)-N(CH₃)₂, -C(=O)-NH-CH₃, -C(=O)-NH₂, -C(=O)-O-CH₃, -C(=O)-O-C₂H₅ and -C(=O)-O-C(CH₃)₃;
R⁴, R⁵, R⁶ and R¹⁴, in each case mutually independently, denote an alkyl residue selected from the group consisting of methyl, ethyl, n-propyl and iso-propyl, or denote a phenyl residue, which is in each case unsubstituted or substituted with 1, 2 or 3 substituents mutually independently selected from the group consisting of F, Cl, Br, -CN, -CF₃, -SF₅, methyl, ethyl, n-propyl, iso-propyl, tert-butyl, -O-CH₃ and -O-C₂H₅ and is optionally attached via a -(CH₂), - (CH₂)₂ or -(CH₂)₃ group;
R¹², R¹³, R¹⁶, R¹⁷, R²⁰, R²¹ and R²³, in each case mutually independently, denote a hydrogen residue; an alkyl residue selected from the group consisting of methyl, ethyl, n-propyl, iso-propyl and tert-butyl, or a phenyl residue, which is in each case unsubstituted or substituted with 1, 2 or 3 substituents mutually independently selected from the group consisting of F, Cl, Br, -CN, -CF₃, -SF₅, methyl, ethyl, n-propyl, iso-propyl, tert-butyl, -O-CH₃ and -O-C₂H₅ and is optionally attached via a -(CH₂), -(CH₂)₂ or -(CH₂)₃ group;
M¹ denotes a residue selected from the group consisting of residues 1 to 9, 11, 21, 22 and 36 to 38, 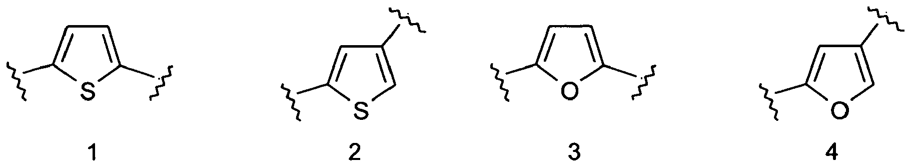 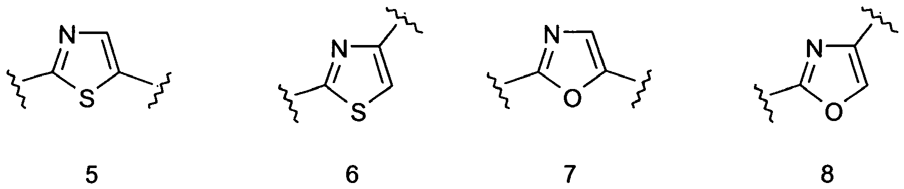 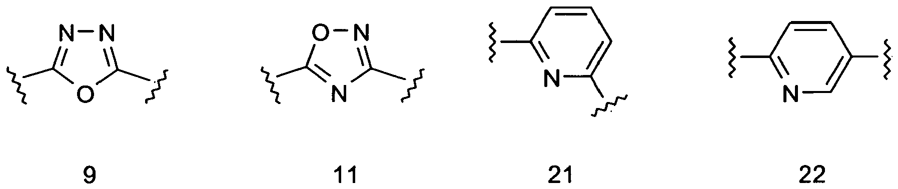 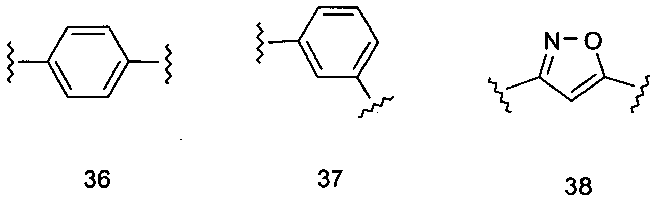
which may in each case be linked in any desired direction via the positions indicated with a wavy line to the bicyclic ring system and to the carbon atom of the triple bond and is optionally substituted with 1 or 2 further substituents, which are mutually independently selected from the group consisting of F, Cl, Br, -CN, -CH₂-CH, -CF₃, -SF₅, methyl, ethyl, n-propyl, iso-propyl, n-butyl, isobutyl, sec-butyl, tert-butyl, -O-CH₃ and -O-CF₃;
and
M² denotes a residue selected from the group consisting of residues 1 to 36, 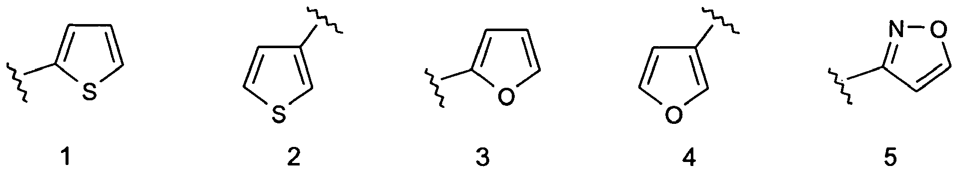 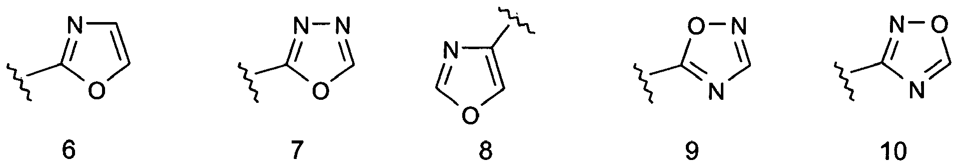 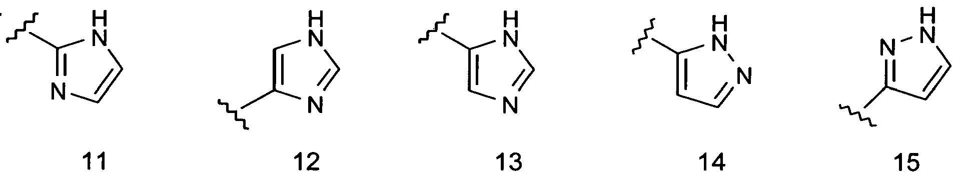 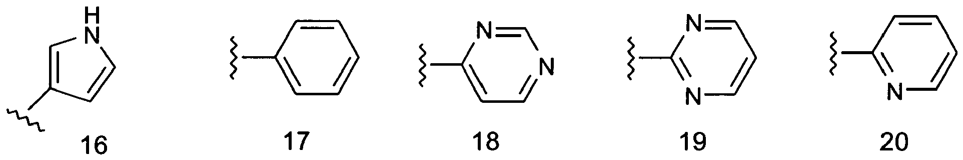 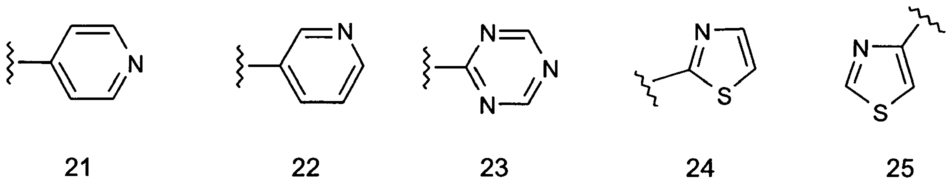 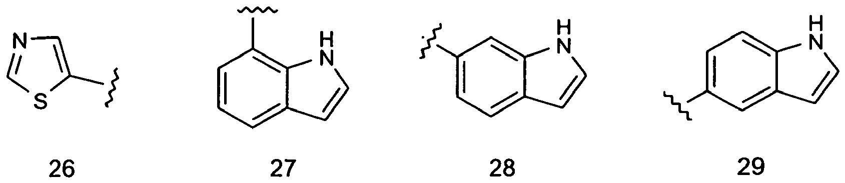 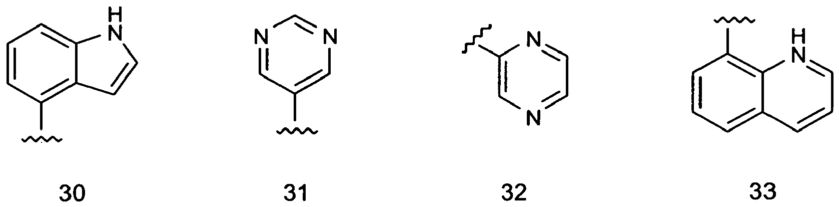 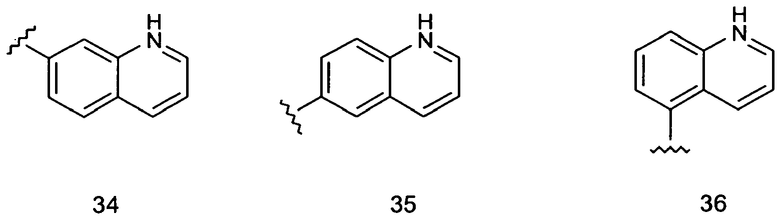
which is in each case linked via the position indicated with a wavy line with the carbon atom of triple bond and is unsubstituted or optionally substituted with 1, 2 or 3 substituents, which are mutually independently selected from the group consisting of F, Cl, Br, -CN, -CF₃, -SF₅, -S-CH₃, -S-C₂H₅, methyl, ethyl, n-propyl, iso-propyl, n-butyl, tert-butyl, ethenyl, propenyl, -O-CH₃, -O-C₂H₅, -NH₂, -N(CH₃)₂, -NH-CH₃, -CH₂-NH₂, -O-CF₃, -C(=O)-H, -C(=O)-O-CH₃, -C(=O)-O-C₂H₅, phenyl, pyrrolyl, (1,3)-dioxolanyl, -Si(phenyl)₂[C(CH₃)₃], -NO₂, NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -S(=O)₂-N(CH₃)₂, -NH-S(=O)₂-OH and -NH-C(=NH)-NH₂;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

15. Compounds according to one or more of claims 1 to 14, **characterised in that**
A¹ denotes a C-R^{1a} group,
A² denotes a C-R^{1b} group,
A³ denotes a C-R^{1c} group and
A⁴ denotes a C-R^{1d} group,
or
A¹ denotes a nitrogen atom,
A² denotes a C-R^{1b} group,
A³ denotes a C-R^{1c} group and
A⁴ denotes a C-R^{1d} group,
or
A¹ denotes a C-R^{1a} group,
A² denotes a nitrogen atom,
A³ denotes a C-R^{1c} group and
A⁴ denotes a C-R^{1d} group,
R^{1a}, R^{1b}, R^{1c}, R^{1d}, in each case mutually independently denote a hydrogen residue; -OR¹⁶; -F; -Cl, -Br; -CN; -S(=O)₂-NH₂; -CF₃; -C(=O)-OR¹²; an alkyl residue selected from the group consisting of methyl, ethyl, n-propyl, iso-propyl, n-butyl, sec-butyl, iso-butyl and tert-butyl; a phenyl residue; a benzyl residue; a phenethyl residue or denote a (3-phenyl)-prop-1-yl residue,
or optionally R^{1a} and R^{1b}, together with the C-C bridge joining them together, form an unsubstituted anellated phenyl residue;
or optionally R^{1c} and R^{1d}, together with the C-C bridge joining them together, form an unsubstituted anellated phenyl residue;
R² and R³, mutually independently, in each case denote a hydrogen residue; -C(=O)-R²⁰; -(CH₂)_{q}-C(=O)-R²¹ with q = 1; -(CH₂)ᵣ-C(=O)-O-R²³ with r = 1; an alkyl residue selected from the group consisting of methyl; ethyl; n-propyl; isopropyl; n-butyl; tert-butyl; sec-butyl; iso-butyl; n-pentyl; n-hexyl; n-heptyl; n-octyl; (1,1,3,3)-tetramethyl-butyl; (1,1)-dimethyl-pentyl and (1,1)-dimethylbutyl; an unsubstituted cyclopentyl or cyclohexyl residue, which may in each case be attached via a -(CH₂)-, -(CH₂)₂ - or -(CH₂)₃ group; or denote a phenyl, pyridinyl, thiophenyl or furanyl residue, which is in each case unsubstituted or optionally substituted with 1, 2, 3, 4 or 5 substituents mutually independently selected from the group consisting of F, Cl, Br, -CN, -CF₃, methyl, ethyl, n-propyl, iso-propyl, tert-butyl, -O-CH₃ and -O-C₂H₅ and is optionally attached via a -(CH₂)-, -(CH₂)₂-, -(CH(CH₃)- or -(CH₂)₃ group,
or R² and R³, together with the nitrogen atom joining them together as a ring member, form a heterocycloaliphatic residue selected from the group consisting of pyrrolidinyl, piperidinyl, piperazinyl and morpholinyl,
R¹² denotes a hydrogen residue or an alkyl residue selected from the group consisting of methyl, ethyl, n-propyl, iso-propyl and tert-butyl;
R¹⁶, R²⁰, R²¹ and R²³, in each case mutually independently denote an alkyl residue selected from the group consisting of methyl, ethyl, n-propyl, iso-propyl and tert-butyl, or a phenyl residue, which is in each case unsubstituted or substituted with 1 or 2 substituents mutually independently selected from the group consisting of F, Cl, Br, methyl, ethyl, -O-CH₃ and -O-C₂H₅ and is optionally attached via a -(CH₂) group,
M¹ denotes a residue selected from the group consisting of residues 1 to 6, 21, 22, 36 and 37, 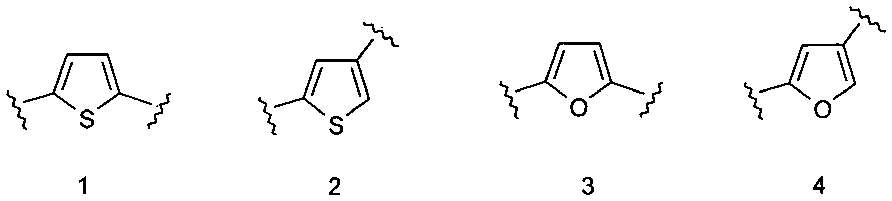 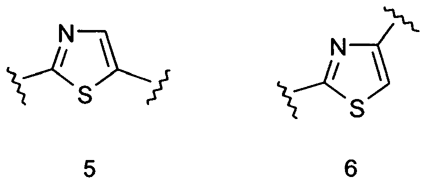 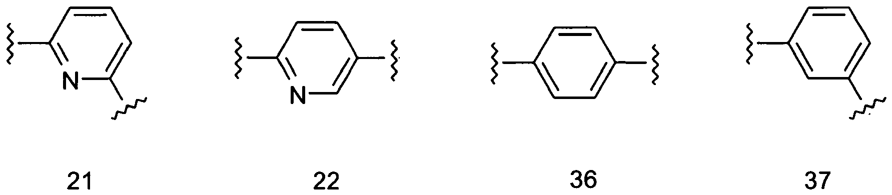
which may in each case be linked in any desired direction via the positions indicated with a wavy line to the bicyclic ring system and to the carbon atom of the triple bond and is optionally substituted with 1 or 2 further substituents, which are mutually independently selected from the group consisting of F, Cl, Br, -CN, -CH₂-CH, -CF₃, -SF₅, methyl, ethyl, n-propyl, iso-propyl, n-butyl, iso-butyl, sec-butyl, tert-butyl, -O-CH₃ and -O-CF₃,
and
M² denotes a residue selected from the group consisting of phenyl, 2-pyridinyl, 3-pyridinyl, 4-pyridinyl, 2-thiazolyl, 4-thiazolyl, 5-thiazolyl, 4-indolyl, 5-indolyl, 6-indolyl, 7-indolyl, 2-thiophenyl, 3-thiophenyl, 2-imidazolyl, 4-imidazolyl, 5-imidazolyl, 2-pyrimidinyl, 4-pyrimidinyl, 5-pyrimidinyl, 2-pyrazinyl, 5-quinolinyl, 6-quinolinyl, 7-quinolinyl and 8-quinolinyl, wherein the particular residue is unsubstituted or optionally substituted with 1, 2, 3, 4 or 5 substituents, which are mutually independently selected from the group consisting of F, Cl, Br, -CN, -CF₃, -SF₅, -S-CH₃, -S-C₂H₅, methyl, ethyl, isopropyl, tert-butyl, ethenyl, propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, =C(=O)-O-C₂H₅, phenyl, pyrrolyl, (1,3)-dioxolanyl, -Si(phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH and -S(=O)₂-N(CH₃)₂;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

16. Compounds of the general formula Id according to claim 15, 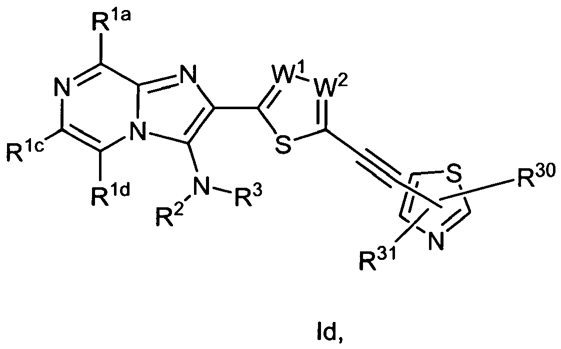 in which
R^{1a}, R^{1c}, R^{1d}, R² and R³ in each case have the meaning according to claim 15;
W¹ denotes C and W² denotes C
or W¹ denotes C and W² denotes N
or W¹ denotes N and W² denotes C;
and
R³⁰ and R³¹, mutually independently, in each case denote a residue selected from the group consisting of H, F, Cl, Br, -CN, -CF₃, -SF₅, -S-CH₃, -S-C₂H₅, methyl, ethyl, iso-propyl, tert-butyl, ethenyl, propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, phenyl, pyrrolyl, (1,3)-dioxolanyl, -Si(phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH and -S(=O)₂-N(CH₃)₂;
in each case optionally in the form of corresponding salts, or in each case optionally in the form of corresponding solvates.

17. Compounds of the general formula le according to claim 15, 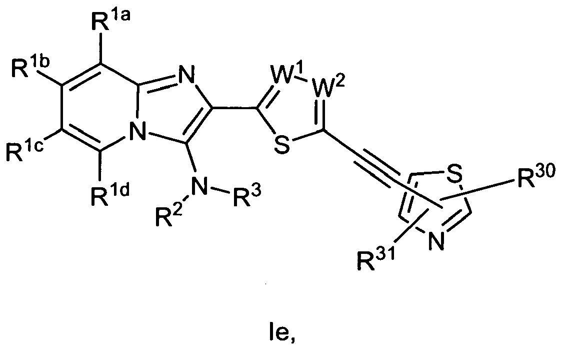 in which
R^{1a}, R^{1b}, R^{1c}, R^{1d}, R² and R³ in each case have the meaning according to claim 15;
W¹ denotes C and W² denotes C
or W¹ denotes C and W² denotes N
or W¹ denotes N and W² denotes C;
and
R³⁰ and R³¹, mutually independently, in each case denote a residue selected from the group consisting of H, F, Cl, Br, -CN, -CF₃, -SF₅, -S-CH₃, -S-C₂H₅, methyl, ethyl, iso-propyl, tert-butyl, ethenyl, propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, phenyl, pyrrolyl, (1,3)-dioxolanyl, -Si(phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH and -S(=O)₂-N(CH₃)₂;
in each case optionally in the form of corresponding salts, or in each case optionally in the form of corresponding solvates.

18. Compounds of the general formula If according to claim 15, 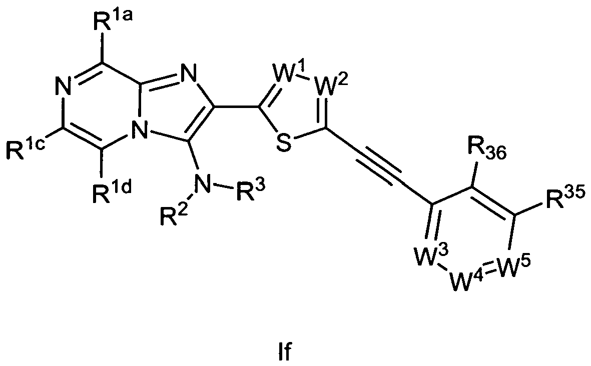 in which
R^{1a}, R^{1c}, R^{1d}, R² and R³ in each case have the meaning according to claim 15;
W¹ denotes C and W² denotes C
or W¹ denotes C and W² denotes N
or W¹ denotes N and W² denotes C;
W³ denotes C-R³²; W⁴ denotes C-R³³ and W⁵ denotes C-R³⁴;
or one of the residues W³, W⁴ and W⁵ denotes N and the other two residues selected from the group consisting of W³, W⁴ and W⁵ denote C-R³² or C-R³³;
and R³², R³³, R³⁴, R³⁵ and R³⁶, mutually independently, in each case denote a residue selected from the group consisting of H, F, Cl, Br, -CN, -CF₃, -SF₅, -S-CH₃, -S-C₂H₅, methyl, ethyl, iso-propyl, tert-butyl, ethenyl, propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, phenyl, pyrrolyl, (1,3)-dioxolanyl, -Si(phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH and -S(=O)₂-N(CH₃)₂;
in each case optionally in the form of corresponding salts, or in each case optionally in the form of corresponding solvates.

19. Compounds of the general formula Ig according to claim 15, 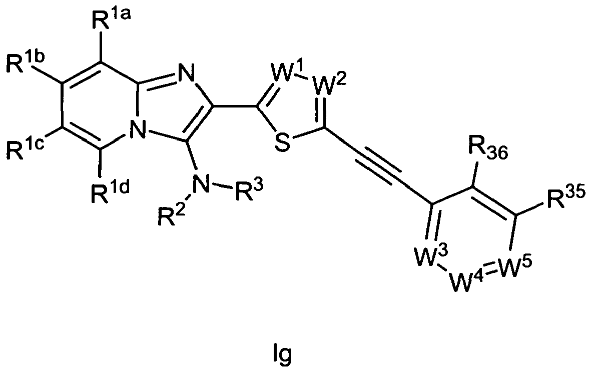 in which
R^{1a}, R^{1b}, R^{1c}, R^{1d}, R² and R³ in each case have the meaning according to claim 15;
W¹ denotes C and W² denotes C
or W¹ denotes C and W² denotes N
or W¹ denotes N and W² denotes C;
W³ denotes C-R³²; W⁴ denotes C-R³³ and W⁵ denotes C-R³⁴;
or one of the residues W³, W⁴ and W⁵ denote N and the other two residues selected from the group consisting of W³, W⁴ and W⁵ denote C-R³² or C-R³³; and R³², R³³, R³⁴, R³⁵ and R³⁶, mutually independently, in each case denote a residue selected from the group consisting of H, F, Cl, Br, -CN, -CF₃, -SF₅, -S-CH₃, -S-C₂H₅, methyl, ethyl, iso-propyl, tert-butyl, ethenyl, propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, phenyl, pyrrolyl, (1,3)-dioxolanyl, -Si(phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH and -S(=O)₂-N(CH₃)₂;
in each case optionally in the form of corresponding salts, or in each case optionally in the form of corresponding solvates.

20. Compounds of the general formula Ih according to claim 15, 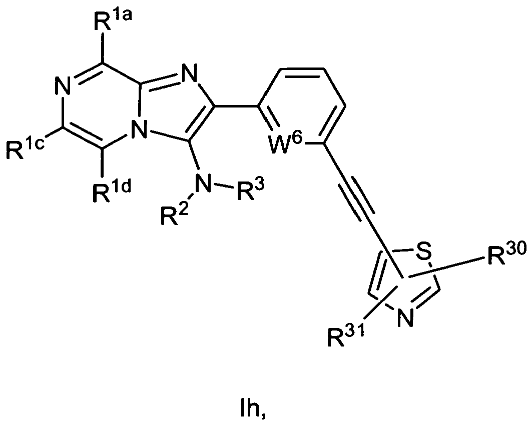 in which
R^{1a}, R^{1c}, R^{1d}, R² and R³ in each case have the meaning according to claim 15;
W⁶ denotes C or N;
and
R³⁰ and R³¹, mutually independently, in each case denote a residue selected from the group consisting of H, F, Cl, Br, -CN, -CF₃, -SF₅, -S-CH₃, -S-C₂H₅, methyl, ethyl, iso-propyl, tert-butyl, ethenyl, propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, phenyl, pyrrolyl, (1,3)-dioxolanyl, -Si(phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH and -S(=O)₂-N(CH₃)₂;
in each case optionally in the form of corresponding salts, or in each case optionally in the form of corresponding solvates.

21. Compounds of the general formula Ik according to claim 15, 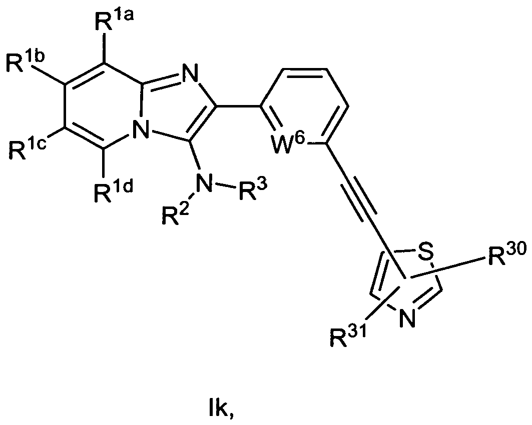 in which
R^{1a}, R^{1b}, R^{1c}, R^{1d}, R² and R³ in each case have the meaning according to claim 15;
W⁶ denotes C or N;
and
R³⁰ and R³¹, mutually independently, in each case denote a residue selected from the group consisting of H, F, Cl, Br, -CN, -CF₃, -SF₅, -S-CH₃, -S-C₂H₅, methyl, ethyl, iso-propyl, tert-butyl, ethenyl, propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, phenyl, pyrrolyl, (1,3)-dioxolanyl, -Si(phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH and -S(=O)₂-N(CH₃)₂;
in each case optionally in the form of corresponding salts, or in each case optionally in the form of corresponding solvates.

22. Compounds of the general formula Im according to claim 15, 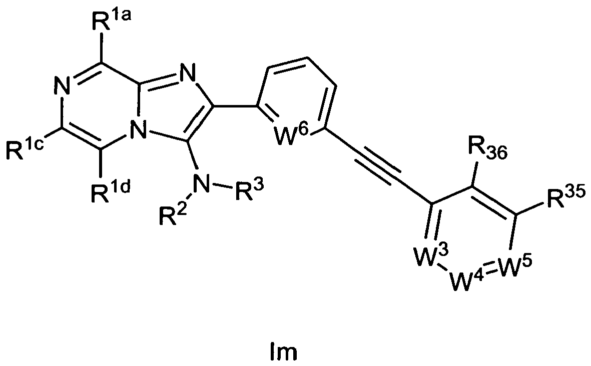 in which
R^{1a}, R^{1c}, R^{1d}, R² and R³ in each case have the meaning according to claim 15;
W⁶ denotes C or N;
W³ denotes C-R³²; W⁴ denotes C-R³³ and W⁵ denotes C-R³⁴;
or one of the residues W³, W⁴ and W⁵ denote N and the other two residues selected from the group consisting of W³, W⁴ and W⁵ denote C-R³² or C-R³³;
and R³², R³³, R³⁴, R³⁵ and R³⁶, mutually independently, in each case denote a residue selected from the group consisting of H, F, Cl, Br, -CN, -CF₃, -SF₅, -S-CH₃, -S-C₂H₅, methyl, ethyl, iso-propyl, tert-butyl, ethenyl, propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, phenyl, pyrrolyl, (1,3)-dioxolanyl, -Si(phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH and -S(=O)₂-N(CH₃)₂;
in each case optionally in the form of corresponding salts, or in each case optionally in the form of corresponding solvates.

23. Compounds of the general formula In according to claim 15, 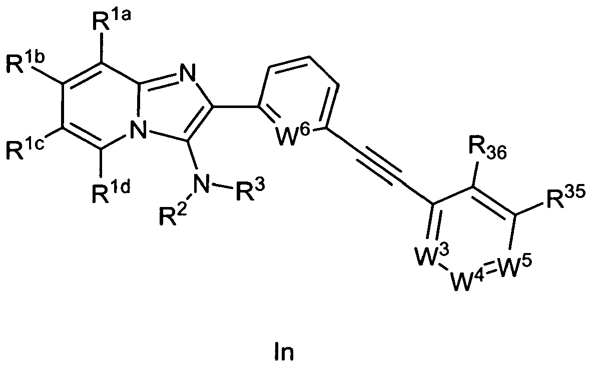 in which
R^{1a}, R^{1b}, R^{1c}, R^{1d}, R² and R³ in each case have the meaning according to claim 15;
W⁶ denotes C or N;
W³ denotes C-R³²; W⁴ denotes C-R³³ and W⁵ denotes C-R³⁴;
or one of the residues W³, W⁴ and W⁵ denote N and the other two residues selected from the group consisting of W³, W⁴ and W⁵ denote C-R³² or C-R³³; and R³², R³³, R³⁴, R³⁵ and R³⁶, mutually independently, in each case denote a residue selected from the group consisting of H, F, Cl, Br, -CN, -CF₃, -SF₅, -S-CH₃, -S-C₂H₅, methyl, ethyl, iso-propyl, tert-butyl, ethenyl, propenyl, -OH, -O-CH₃, -O-C₂H₅, -NH₂, -N(CH₃)₂, -NH-CH₃, -CH₂-NH₂, -NO₂, -O-CF₃, -C(=O)-H; -C(=O)-OCH₃, -C(=O)-O-C₂H₅, phenyl, pyrrolyl, (1,3)-dioxolanyl, -Si(phenyl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH and -S(=O)₂-N(CH₃)₂;
in each case optionally in the form of corresponding salts, or in each case optionally in the form of corresponding solvates.

24. Compounds according to one or more of claims 1 to 23 selected from the group consisting of
[1] cyclopentyl-[2-(5-phenylethynyl-thiophen-2-yl)-imidazo[1,2-a]pyrazin-3-yl]-amine,
[2] cyclohexylmethyl-[5-methyl-2-(5-phenylethynyl-thiophen-2-yl)-imidazo[1,2-a]pyridin-3-yl]-amine,
[3] cyclohexylmethyl-[6-methyl-2-(5-phenylethynyl-thiophen-2-yl)-imidazo[1,2-a]pyridin-3-yl]-amine,
[4] cyclohexylmethyl-[7-methyl-2-(5-phenylethynyl-thiophen-2-yl)-imidazo[1,2-a]pyridin-3-yl]-amine,
[5] cyclohexylmethyl-[8-methyl-2-(5-phenylethynyl-thiophen-2-yl)-imidazo[1,2-a]pyridin-3-yl]-amine,
[6] cyclohexylmethyl-[5,7-dimethyl-2-(5-phenylethynyl-thiophen-2-yl)-imidazo[1,2-a]pyridin-3-yl]-amine,
[7] [8-benzyloxy-2-(5-phenylethynyl-thiophen-2-yl)-imidazo[1,2-a]pyridin-3-yl]-cyclohexylmethyl-amine,
[8] cyclohexylmethyl-[2-(5-phenylethynyl-thiophen-2-yl)-imidazo[1,2-a]pyrazin-3-yl]-amine,
[9] cyclohexylmethyl-[2-(5-phenylethynyl-thiophen-2-yl)-imidazo[1,2-a]pyrimidin-3-yl]-amine,
[10] (4-methoxy-benzyl)-[5-methyl-2-(5-phenylethynyl-thiophen-2-yl)-imidazo[1,2-a]pyridin-3-yl]-amine,
[11] (4-methoxy-benzyl)-[6-methyl-2-(5-phenylethynyl-thiophen-2-yl)-imidazo[1,2-a]pyridin-3-yl]-amine,
[12] (4-methoxy-benzyl)-[7-methyl-2-(5-phenylethynyl-thiophen-2-yl)-imidazo[1,2-a]pyridin-3-yl]-amine,
[13] (4-methoxy-benzyl)-[8-methyl-2-(5-phenylethynyl-thiophen-2-yl)-imidazo[1,2-a]pyridin-3-yl]-amine,
[14] [5,7-dimethyl-2-(5-phenylethynyl-thiophen-2-yl)-imidazo[1,2-a]pyridin-3-yl]-(4-methoxy-benzyl)-amine,
[15] [8-benzyloxy-2-(5-phenylethynyl-thiophen-2-yl)-imidazo[1,2-a]pyridin-3-yl]-(4-methoxy-benzyl)-amine,
[16] (4-methoxy-benzyl)-[2-(5-phenylethynyl-thiophen-2-yl)-imidazo[1,2-a]pyridin-3-yl]-amine,
[17] (4-methoxy-benzyl)-[2-(5-phenylethynyl-thiophen-2-yl)-imidazo[1,2-a]pyrazin-3-yl]-amine,
[18] tert-butyl-[2-(5-phenylethynyl-thiophen-2-yl)-imidazo[1,2-a]pyrazin-3-yl]-amine,
[19] tert-butyl-[6-methyl-2-(5-phenylethynyl-thiophen-2-yl)-imidazo[1,2-a]pyridin-3-yl]-amine,
[20] [8-benzyloxy-2-(5-phenylethynyl-thiophen-2-yl)-imidazo[1,2-a]pyridin-3-yl]-(3-methoxy-phenyl)-amine,
[21] (3-methoxy-benzyl)-[5-methyl-2-(5-phenylethynyl-thiophen-2-yl)-imidazo[1,2-a]pyridin-3-yl]-amine,
[22] (3-methoxy-benzyl)-[8-methyl-2-(5-phenylethynyl-thiophen-2-yl)-imidazo[1,2-a]pyridin-3-yl]-amine,
[23] [5,7-dimethyl-2-(5-phenylethynyl-thiophen-2-yl)-imidazo[1,2-a]pyridin-3-yl]-(3-methoxy-benzyl)-amine,
[24] [8-benzyloxy-2-(5-phenylethynyl-thiophen-2-yl)-imidazo[1,2-a]pyridin-3-yl]-(3-methoxy-benzyl)-amine,
[25] (3-methoxy-benzyl)-[2-(5-phenylethynyl-thiophen-2-yl)-imidazo[1,2-a]pyridin-3-yl]-amine,
[26] (3-methoxy-benzylf[2-(5-phenylethynyl-thiophen-2-yl)-imidazo[1,2-a]pyrazin-3-yl]-amine,
[27] [6-methyl-2-(5-phenylethynyl-thiophen-2-yl)-imidazo[1,2-a]pyridin-3-yl]-(1-phenyl-ethyl)-amine,
[28] [7-methyl-2-(5-phenylethynyl-thiophen-2-yl)-imidazo[1,2-a]pyridin-3-yl]-(1-phenyl-ethyl)-amine,
[29] [5,7-dimethyl-2-(5-phenylethynyl-thiophen-2-yl)-imidazo[1,2-a]pyridin-3-y)]-(1-phenyl-ethyl)-amine,
[30] [8-benzyloxy-2-(5-phenylethynyl-thiophen-2-yl)-imidazo[1,2-a]pyridin-3-yl]-(1-phenyl-ethyl)-amine,
[31] (1-phenyl-ethyl)-[2-(5-phenylethynyl-thiophen-2-yl)-imidazo[1,2-a]pyrazin-3-yl]-amine,
[32] (2-chloro-benzyl)-[5,7-dimethyl-2-(5-phenylethynyl-thiophen-2-yl)-imidazo[1,2-a]pyridin-3-yl]-amine,
[33] (3-chloro-4-fluoro-phenyl)-[7-phenyl-2-(5-phenylethynyl-thiophen-2-yl)-imidazo[1,2-alpyridin-3-yl]-amine,
[34] (4-methoxy-benzyl)-[7-methyl-2-(5-phenylethynyl-thiophen-2-yl)-imidazo[1,2-a]pyrimidin-3-yl]-amine,
[35] [8-methyl-2-(5-phenylethynyl-thiophen-2-yl)-imidazo[1,2-a]pyridin-3-yl]-(1-phenyl-ethyl)-amine,
[36] [7-methyl-2-(5-phenylethynyl-thiophen-2-yl)-imidazo[1,2-a]pyrimidin-3-yl]-(1-phenyl-ethyl)-amine,
[37] (2-methoxy-benzyl)-[7-methyl-2-(5-phenylethynyl-thiophen-2-yl)-imidazo[1,2-a]pyridin-3-yl]-amine,
[38] (2-methoxy-benzyl)-[2-(5-phenylethynyl-thiophen-2-yl)-imidazo[1,2-a]pyridin-3-yl]-amine,
[39] (2-chloro-benzyl)-[2-(5-phenylethynyl-thiophen-2-yl)-imidazo[1,2-a]pyridin-3-yl]-amine,
[40] (2-methoxy-benzyl)-[7-methyl-2-(5-phenylethynyl-thiophen-2-yl)-imidazo[1,2-a]pyrimidin-3-yl]-amine,
[41] (3-methoxy-phenyl)-[6-methyl-2-(5-phenylethynyl-thiophen-2-yl)-imidazo[1,2-a]pyridin-3-yl]-amine,
[42] [5,7-dimethyl-2-(5-phenylethynyl-thiophen-2-yl)-imidazo[1,2-a]pyridin-3-yl]-(3-methoxy-phenyl)-amine,
[43] (3-methoxy-phenyl)-[2-(5-phenylethynyl-thiophen-2-yl)-imidazo[1,2-a]pyrazin-3-yl]-amine,
[44] [7-ethyl-2-(5-phenylethynyl-thiophen-2-yl)-imidazo[1,2-a]pyridin-3-yl]-(2-methoxy-benzyl)-amine,
[45] (2-chloro-benzyl)-[7-ethyl-2-(5-phenylethynyl-thiophen-2-yl)-imidazo[1,2-a]pyridin-3-yl]-amine,
[46] [7-tert-butyl-2-(5-phenylethynyl-thiophen-2-yl)-imidazo[1,2-a]pyridin-3-yl]-(3-chloro-4-fluoro-phenylyamine,
[47] (3-methoxy-benzyl)-[7-methyl-2-(5-phenylethynyl-thiophen-2-yl)-imidazo[1,2-a]pyrimidin-3-yl]-amine,
[48] [7-ethyl-2-(5-phenylethynyl-thiophen-2-yl)-imidazo[1,2-a]pyridin-3-yl]-(2-fluoro-phenyl)-amine,
[49] [7-tert-butyl-2-(5-phenylethynyl-thiophen-2-yl)-imidazo[1,2-a]pyridin-3-yl]-(2-fluoro-phenyl)-amine,
[50] (2,4-difluoro-phenyl)-[2-(5-phenylethynyl-thiophen-2-yl)-7-(3-phenyl-propyl)-imidazo[1,2-a]pyridin-3-yl]-amine,
[51] (4-fluoro-benzyl)-[7-methyl-2-(5-phenylethynyl-thiophen-2-yl)-imidazo[1,2-a]pyridin-3-yl]-amine,
[52] [5,7-dimethyl-2-(5-phenylethynyl-thiophen-2-yl)-imidazo[1,2-a]pyridin-3-yl]-(4-fluoro-benzyl)-amine,
[53] [7-ethyl-2-(5-phenylethynyl-thiophen-2-yl)-imidazo[1,2-a]pyridin-3-yl]-(3-trifluoromethyl-benzyl)-amine,
[54] [7-isopropyl-2-(5-phenylethynyl-thiophen-2-yl)-imidazo[1,2-a]pyridin-3-yl]-(3-trifluoromethyl-benzyl)-amine,
[55] tert-butyl-[2-(4-phenylethynyl-thiophen-2-yl)-imidazo[1,2-a]pyrazin-3-yl]-amine,
[56] [2-(5-phenylethynyl-thiophen-2-yl)-imidazo[1,2-a]pyrazin-3-yl]-(1,1,3,3-tetramethyl-butyl)-amine,
[57] butyl-[2-(4-phenylethynyl-thiophen-2-yl)-imidazo[1,2-a]pyrazin-3-yl]-amine,
[59] [2-(5-phenylethynyl-thiophen-2-yl)-imidazo[1,2-a]pyridin-3-yl]-(1,1,3,3-tetramethyl-butyl)-amine,
[60] [2-(5-pyridinyl-thiophen-2-yl)-imidazo[1,2-a]pyrazin-3-yl]-(1,1,3,3-tetramethyl-butyl)-amine,
[61] [2-(5-pyridin-4-ylethynyl-thiophen-2-yl)-imidazo[1,2-a]pyrazin-3-yl]-(1,1,3,3-tetramethyl-butyl)-amine,
[62] [6-chloro-2-(5-phenylethynyl-thiophen-2-yl)-imidazo[1,2-a]pyridin-3-yl]-(1,1,3,3-tetramethyl-butyl)-amine,
[63] [6,8-dichloro-2-(5-phenylethynyl-thiophen-2-yl)-imidazo[1,2-a]pyridin-3-yl]-(1,1,3,3-tetramethyl-butyl)-amine,
[64] [2-(5-pyridin-2-ylethynyl-thiophen-2-yl)-imidazo[1,2-a]pyrazin-3-yl]-(1,1,3,3-tetramethyl-butyl)-amine,
[65] dimethyl-[2-(5-phenylethynyl-thiophen-2-yl)-imidazo[1,2-a]pyrazin-3-yl]-amine,
[66] methyl-[2-(5-phenylethynyl-thiophen-2-yl)-imidazo[1,2-a]pyrazin-3-yl]-amine,
[67] N-[2-(5-phenylethynyl-thiophen-2-yl)-imidazo[1,2-a]pyrazin-3-yl]-acetamide,
[68] ethyl-[2-(5-phenylethynyl-thiophen-2-yl)-imidazo[1,2-a]pyrazin-3-yl]-amine,
[69] propyl-[2-(5-phenylethynyl-thiophen-2-yl)-imidazo[1,2-a]pyrazin-3-yl]-amine,
[70] butyl-[2-(5-phenylethynyl-thiophen-2-yl)-imidazo[1,2-a]pyrazin-3-yl]-amine,
[71] (2-methylpropyl)-[2-(5-phenylethynyl-thiophen-2-yl)-imidazo[1,2-a]pyrazin-3-yl]-amine,
[72] pentyl-[2-(5-phenylethynyl-thiophen-2-yl)-imidazo[1,2-a]pyrazin-3-yl]-amine,
[73] {(methoxycarbonylmethyl)-[2-(5-phenylethynyl-thiophen-2-yl)-imidazo[1,2-a]pyrazin-3-yl]-amino}-acetic acid methyl ester,
[74] benzyl-[2-(5-phenylethynyl-thiophen-2-yl)-imidazo[1,2-a]pyrazin-3-yl]-amine,
[75] [2-(5-phenylethynyl-thiophen-2-yl)-imidazo[1,2-a]pyrazin-3-ylamino]-acetic acid methyl ester,
[76] tert-butyl-[2-(5-pyridin-4-ylethynyl-thiophen-2-yl)-imidazo[1,2-a]pyrazin-3-yl]-amine,
[77] tert-butyl-[2-(5-pyridin-2-ylethynyl-thiophen-2-yl)-imidazo[1,2-a]pyrazin-3-yl]-amine,
[78] N-[2-(5-phenylethynyl-thiophen-2-yl)-imidazo[1,2-a]pyrazin-3-yl]-benzamide,
[79] [2-(5-phenylethynyl-thiophen-2-yl)-imidazo[1,2-a]pyrazin-3-yl]-bis-pyridin-3-ylmethyl-amine,
[80] 2,2-dimethyl-N-[2-(5-phenylethynyl-thiophen-2-yl)-imidazo[1,2-a]pyrazin-3-yl]-propionamide,
[81] 3-methoxy-N-[2-(5-phenylethynyl-thiophen-2-yl)-imidazo[1,2-a]pyrazin-3-yl]-benzamide,
[82] tert-butyl-[2-(5-pyridin-3-ylethynyl-thiophen-2-yl)-imidazo[1,2-a]pyrazin-3-yl]-amine
[83] 2-(5-phenylethynyl-thiophen-2-yl)-imidazo[1,2-a]pyrazin-3-ylamine
[84] methyl-2-(5-(phenylethynyl)thiophen-2-yl)-3-(2,4,4-trimethylpentan-2-ylamino)imidazo[1,2-a]pyrazine-8-carboxylate
[85] 2-(5-(phenylethynyl)thiophen-2-yl)-N-(2,4,4-trimethylpentan-2-yl)imidazo[1,2-a]pyrimidin-3-amine
[86] 2-(5-((6-methylpyridin-2-yl)ethynyl)thiophen-2-yl)-N-(2,4,4-trimethylpentan-2-yl)imidazo[1,2-a]pyrazin-3-amine
[87] N--cyclohexyl-2-(5-(phenylethynyl)thiophen-2-yl)imidazo[1,2-a]pyrazin-3-amine
[88] 2-(5-(phenylethynyl)thiophen-2-yl)-3-(piperidin-1-yl)imidazo[1,2-a]pyrazine
[89] N-tert-butyl-N-methyl-2-(5-(phenylethynyl)thiophen-2-yl)imidazo[1,2-a]pyrazin-3-amine
[90] methyl-2-(5-(phenylethynyl)thiophen-2-yl)-3-(2,4,4-trimethylpentan-2-ylamino)imidazo[1,2-a]pyridine-6-carboxylate
[91] N-tert-butyl-2-(5-(pyridin-2-ylethynyl)thiophen-2-yl)imidazo[1,2-a]pyrazin-3-amine hydrochloride
[92] 8-bromo-N-cyclopentyl-6-methyl-2-(5-(pyridin-2-ylethynyl)thiophen-2-yl)imidazo[1,2-a]pyridin-3-amine
[93] N,N-diethyl-2-(5-(phenylethynyl)thiophen-2-yl)imidazo[1,2-a]pyrazin-3-amine
[94] N-tert-butyl-2-(5-(pyridin-2-ylethynyl)thiophen-2-yl)imidazo[1,2-a]pyridin-3-amine
[95] N-tert-butyl-2-(5-(phenylethynyl)furan-2-yl)imidazo[1,2-a]pyrazin-3-amine
[96] 8-bromo-N-tert-butyl-6-methyl-2-(3-(pyridin-2-ylethynyl)phenyl)imidazo[1,2-a]pyridin-3-amine
[97] N-tert-butyl-8-methyl-2-(5-(phenylethynyl)thiophen-2-yl)imidazo[1,2-a]pyridin-3-amine
[98] N-methyl-2-(5-(phenylethynyl)thiophen-2-yl)-N-(2,4,4-trimethylpentan-2-yl)imidazo[1,2-a]pyrazin-3-amine hydrochloride
[99] 2-(5-(phenylethynyl)thiophen-2-yl)-3-(pyrrolidin-1-yl)imidazo[1,2-a]pyrazine hydrochloride
[100] N-tert-butyl-2-(5-((4-fluorophenyl)ethynyl)thiophen-2-yl)imidazo[1,2-a]pyrazin-3-amine hydrochloride
[101] N-tert-butyl-7-methyl-2-(5-(phenylethynyl)thiophen-2-yl)imidazo[1,2-a]pyridin-3-amine
[102] N-tert-butyl-5-methyl-2-(5-(phenylethynyl)thiophen-2-yl)imidazo[1,2-a]pyridin-3-amine
[103] 8-chloro-2-(3-(pyridin-2-ylethynyl)phenyl)-6-(trifluoromethyl)-N-(2,4,4-trimethylpentan-2-yl)imidazo[1,2-a]pyridin-3-amine
[104] N-tert-butyl-2-(5-((3-fluorophenyl)ethynyl)thiophen-2-yl)imidazo[1,2-a]pyrazin-3-amine hydrochloride
[105] N-tert-butyl-2-(5-((2-fluorophenyl)ethynyl)thiophen-2-yl)imidazo[1,2-a]pyrazin-3-amine hydrochloride
[106] methyl-3-(tert-butylamino)-2-(5-(phenylethynyl)thiophen-2-yl)imidazo[1,2-a]pyrazine-8-carboxylate
[107] N-tert-butyl-2-(5-(pyrazin-2-ylethynyl)thiophen-2-yl)imidazo[1,2-a]pyrazin-3-amine
[108] 2-(5-((4-aminophenyl)ethynyl)thiophen-2-yl)-N-tert-butylimidazo[1,2-a]pyrazin-3-amine
[109] N-isopropyl-2-(5-(phenylethynyl)thiophen-2-yl)imidazo[1,2-a]pyrazin-3-amine
[110] N-tert-butyl-2-(5-(thiophen-2-ylethynyl)thiophen-2-yl)imidazo[1,2-a]pyrazin-3-amine hydrochloride
[111] N-tert-butyl-2-(3-(pyridin-2-ylethynyl)phenyl)imidazo[1,2-a]pyrazin-3-amine
[112] N-tert-butyl-2-(5-((2-methoxyphenyl)ethynyl)thiophen-2-yl)imidazo[1,2-a]pyrazin-3-amine hydrochloride
[113] N-tert-butyl-2-(5-((3-methoxyphenyl)ethynyl)thiophen-2-yl)imidazo[1,2-a]pyrazin-3-amine hydrochloride
[114] N-tert-butyl-2-(5-((4-methoxyphenyl)ethynyl)thiophen-2-yl)imidazo[1,2-a]pyrazin-3-amine hydrochloride
[115] N-tert-butyl-2-(5-(phenylethynyl)thiophen-2-yl)imidazo[1,2-a]quinolin-1-amine
[116] N-tert-butyl-2-(4-(pyridin-2-ylethynyl)phenyl)imidazo[1,2-a]pyrazin-3-amine
[117] N-tert-butyl-2-(3-((6-methylpyridin-2-yl)ethynyl)phenyl)imidazo[1,2-a]pyrazin-3-amine
[118] N-tert-butyl-2-(2-methyl-6-(phenylethynyl)pyridin-3-yl)imidazo[1,2-a]pyrazin-3-amine
[119] N-tert-butyl-2-(5-(pyridin-2-ylethynyl)furan-2-yl)imidazo[1,2-a]pyrazin-3-amine
[120] 3-(tert-butylamino)-2-(5-(phenylethynyl)thiophen-2-yl)imidazo[1,2-a]pyrazine-8-carboxylic acid
[121] 4-((5-(3-(tert-butylamino)imidazo[1,2-a]pyrazin-2-yl)thiophen-2-yl)ethynyl)phenol hydrochloride
[122] 3-((5-(3-(tert-butylamino)imidazo[1,2-a]pyrazin-2-yl)thiophen-2-yl)ethynyl)phenol
[123] 2-(5-((3-aminophenyl)ethynyl)thiophen-2-yl)-N-tert-butylimidazo[1,2-a]pyrazin-3-amine hydrochloride
[124] 2-(5-((2-aminophenyl)ethynyl)thiophen-2-yl)-N-tert-butylimidazo[1,2-a]pyrazin-3-amine hydrochloride
[125] N-tert-butyl-2-(5-(phenylethynyl)thiophen-2-yl)imidazo[2,1-a]isoquinolin-3-amine
[126] N-tert-butyl-2-(6-(phenylethynyl)pyridin-3-yl)imidazo[1,2-a]pyrazin-3-amine
[127] N-tert-butyl-2-(5-(pyridin-4-ylethynyl)thiophen-2-yl)imidazo[1,2-a]pyrazin-3-amine hydrochloride
[128] 2-(5-((6-aminopyridin-3-yl)ethynyl)thiophen-2-yl)-N-tert-butylimidazo[1,2-a]pyrazin-3-amine hydrochloride
[129] N-tert-butyl-2-(5-(pyrimidin-2-ylethynyl)thiophen-2-yl)imidazo[1,2-a]pyrazin-3-amine hydrochloride
[130] N-tert-butyl-2-(5-((4-methylpyridin-2-yl)ethynyl)thiophen-2-yl)imidazo[1,2-a]pyrazin-3-amine hydrochloride
[131] N-tert-butyl-2-(5-((5-methylpyridin-2-yl)ethynyl)thiophen-2-yl)imidazo[1,2-a]pyrazin-3-amine hydrochloride
[132] N-tert-butyl-2-(5-(pyridin-4-ylethynyl)thiophen-2-yl)imidazo[1,2-a]pyridin-3-amine hydrochloride
[133] N-tert-butyl-2-(5-(thiazol-2-ylethynyl)thiophen-2-yl)imidazo[1,2-a]pyrazin-3-amine
[134] 2-(5-(pyridin-2-ylethynyl)thiophen-2-yl)imidazo[1,2-a]pyrazin-3-amine hydrochloride
[135] N-tert-butyl-2-(5-((5-methylthiophen-2-yl)ethynyl)thiophen-2-yl)imidazo[1,2-a]pyrazin-3-amine hydrochloride
[136] 2-(5-((6-aminopyridin-2-yl)ethynyl)thiophen-2-yl)-N-tert-butylimidazo[1,2-a]pyrazin-3-amine hydrochloride
[137] N-tert-butyl-2-(5-((3-methylthiophen-2-yl)ethynyl)thiophen-2-yl)imidazo[1,2-a]pyrazin-3-amine
[138] N-tert-butyl-2-(4-(phenylethynyl)thiazol-2-yl)imidazo[1,2-a]pyrazin-3-amine
[139] N-tert-butyl-2-(5-(m-tolylethynyl)thiophen-2-yl)imidazo[1,2-a]pyrazin-3-amine
[140] 3-((5-(3-(tert-butylamino)imidazo[1,2-a]pyrazin-2-yl)thiophen-2-yl)ethynyl)benzonitrile hydrochloride
[141] N-tert-butyl-2-(5-(pyridin-2-ylethynyl)thiophen-2-yl)imidazo[1,2-a]pyrimidin-3-amine
[142] N-tert-butyl-2-(6-(phenylethynyl)pyridin-2-yl)imidazo[1,2-a]pyrazin-3-amine hydrochloride
[143] N-tert-butyl-N-methyl-2-(5-(pyridin-2-ylethynyl)thiophen-2-yl)imidazo[1,2-a]pyrazin-3-amine
[144] 4-((5-(3-(tert-butylamino)imidazo[1,2-a]pyrazin-2-yl)thiophen-2-yl)ethynyl)benzonitrile hydrochloride
[145] 2-(5-((1H-indol-6-yl)ethynyl)thiophen-2-yl)-N-tert-butylimidazo[1,2-a]pyrazin-3-amine hydrochloride
[146] N-tert-butyl-2-(2-(phenylethynyl)thiazol-5-yl)imidazo[1,2-a]pyrazin-3-amine
[147] N-tert-butyl-2-(5-(quinolin-6-ylethynyl)thiophen-2-yl)imidazo[1,2-a]pyrazin-3-amine hydrochloride
[148] 2-(5-((3-(1H-pyrrol-1-yl)phenyl)ethynyl)thiophen-2-yl)-N-tert-butylimidazo[1,2-a]pyrazin-3-amine hydrochloride
[149] 2-(5-((1H-indol-4-yl)ethynyl)thiophen-2-yl)-N-tert-butylimidazo[1,2-a]pyrazin-3-amine hydrochloride
[150] N-tert-butyl-2-(5-((3-nitrophenyl)ethynyl)thiophen-2-yl)imidazo[1,2-a]pyrazin-3-amine hydrochloride
[151] N-tert-butyl-2-(5-((4-nitrophenyl)ethynyl)thiophen-2-yl)imidazo[1,2-a]pyrazin-3-amine hydrochloride
[152] N-tert-butyl-2-(5-(thiazol-4-ylethynyl)thiophen-2-yl)imidazo[1,2-a]pyrazin-3-amine hydrochloride
[153] 2-((5-(3-(tert-butylamino)imidazo[1,2-a]pyrazin-2-yl)thiophen-2-yl)ethynyl)phenol
[154] 2-(5-((3-(aminomethyl)phenyl)ethynyl)thiophen-2-yl)-N-tert-butylimidazo[1,2-a]pyrazin-3-amine
[155] 2-(5-(biphenyl-3-ylethynyl)thiophen-2-yl)-N-tert-butylimidazo[1,2-a]pyrazin-3-amine hydrochloride
[156] N-tert-butyl-2-(5-(thiophen-3-ylethynyl)thiophen-2-yl)imidazo[1,2-a]pyrazin-3-amine hydrochloride
[157] N-tert-butyl-2-(5-((3-(dimethylamino)phenyl)ethynyl)thiophen-2-yl)imidazo[1,2-a]pyrazin-3-amine hydrochloride
[158] N-tert-butyl-2-(5-((6-methylpyridin-2-yl)ethynyl)thiophen-2-yl)imidazo[1,2-a]pyrazin-3-amine hydrochloride
[159] N-tert-butyl-2-(5-((3-fluoropyridin-2-yl)ethynyl)thiophen-2-yl)imidazo[1,2-a]pyrazin-3-amine hydrochloride
[160] N-tert-butyl-2-(5-((3-(methylamino)phenyl)ethynyl)thiophen-2-yl)imidazo[1,2-a]pyrazin-3-amine hydrochloride
[161] N-tert-butyl-2-(5-(p-tolylethynyl)thiophen-2-yl)imidazo[1,2-a]pyrazin-3-amine
[162] N-tert-butyl-2-(5-(o-tolylethynyl)thiophen-2-yl)imidazo[1,2-a]pyrazin-3-amine hydrochloride
[163] N-tert-butyl-2-(4-methyl-5-(phenylethynyl)thiophen-2-yl)imidazo[1,2-a]pyrazin-3-amine hydrochloride
[164] N-tert-butyl-2-(4-methyl-5-(pyridin-2-ylethynyl)thiophen-2-yl)imidazo[1,2-a]pyrazin-3-amine hydrochloride
[165] N-tert-butyl-2-(5-((6-fluoropyridin-2-yl)ethynyl)thiophen-2-yl)imidazo[1,2-a]pyrazin-3-amine
[166] N-tert-butyl-2-(5-((2-nitrophenyl)ethynyl)thiophen-2-yl)imidazo[1,2-a]pyrazin-3-amine
[167] N-tert-butyl-8-chloro-2-(5-(pyridin-2-ylethynyl)thiophen-2-yl)imidazo[1,2-a]pyrazin-3-amine
[168] N-tert-butyl-2-(5-((6-methoxypyridin-2-yl)ethynyl)thiophen-2-yl)imidazo[1,2-a]pyrazin-3-amine
[169] N-tert-butyl-2-(5-((5-fluoropyridin-2-yl)ethynyl)thiophen-2-yl)imidazo[1,2-a]pyrazin-3-amine hydrochloride
[170] 2-(4-(3-(tert-butylamino)imidazo[1,2-a]pyrazin-2-yl)-2-(phenylethynyl)phenyl)acetonitrile
[171] N-tert-butyl-2-(5-((5-methoxypyridin-3-yl)ethynyl)thiophen-2-yl)imidazo[1,2-a]pyrazin-3-amine hydrochloride
[172] 5-((5-(3-(tert-butylamino)imidazo[1,2-a]pyrazin-2-yl)thiophen-2-yl)ethynyl)nicotinonitrile hydrochloride
[173] N-tert-butyl-2-(5-((3-(methylthio)phenyl)ethynyl)thiophen-2-yl)imidazo[1,2-a]pyrazin-3-amine
[174] methyl-3-((5-(3-(tert-butylamino)imidazo[1,2-a]pyrazin-2-yl)thiophen-2-yl)ethynyl)benzoate
[175] N-tert-butyl-2-(5-((3,5-difluoropyridin-2-yl)ethynyl)thiophen-2-yl)imidazo[1,2-a]pyrazin-3-amine
[176] N-tert-butyl-2-(5-(phenylethynyl)thiazol-2-yl)imidazo[1,2-a]pyrazin-3-amine
[177] N-tert-butyl-2-(2-(pyridin-4-ylethynyl)thiazol-5-yl)imidazo[1,2-a]pyrazin-3-amine
[178] 3-((5-(3-(tert-butylamino)imidazo[1,2-a]pyrazin-2-yl)thiophen-2-yl)ethynyl)benzaldehyde
[179] 3-((5-(3-(tert-butylamino)imidazo[1,2-a]pyrazin-2-yl)thiophen-2-yl)ethynyl)-4-fluorobenzonitrile
[180] N-tert-butyl-2-(5-((3-(trifluoromethyl)phenyl)ethynyl)thiophen-2-yl)imidazo[1,2-a]pyrazin-3-amine hydrochloride
[181] N-tert-butyl-2-(2-(pyridin-2-ylethynyl)thiazol-5-yl)imidazo[1,2-a]pyrazin-3-amine
[182] N-tert-butyl-2-(3-methyl-5-(phenylethynyl)thiophen-2-yl)imidazo[1 ,2-a]pyrazin-3-amine hydrochloride
[183] N-tert-butyl-2-(3-methyl-5-(pyridin-2-ylethynyl)thiophen-2-yl)imidazo[1,2-a]pyrazin-3-amine hydrochloride
[184] N-tert-butyl-2-(5-((3-vinylphenyl)ethynyl)thiophen-2-yl)imidazo[1,2-a]pyrazin-3-amine
[185] 2-(5-((1H-imidazol-4-yl)ethynyl)thiophen-2-yl)-N-tert-butylimidazo[1,2-a]pyrazin-3-amine hydrochloride
[186] N-tert-butyl-2-(5-((3-methylpyridin-2-yl)ethynyl)thiophen-2-yl)imidazo[1,2-a]pyrazin-3-amine
[187] N,N-dimethyl-2-(5-(pyridin-2-ylethynyl)thiophen-2-yl)imidazo[1,2-a]pyrazin-3-amine
[188] N-tert-butyl-2-(5-((2-(tert-butyldiphenylsilyl)thiazol-5-yl)ethynyl)thiophen-2-yl)imidazo[1,2-a]pyridin-3-amine
[189] 3-((5-(3-(tert-butylamino)imidazo[1,2-a]pyridin-2-yl)thiophen-2-yl)ethynyl)benzonitrile
[190] N-tert-butyl-2-(5-(phenylethynyl)thiazol-2-yl)imidazo[1,2-a]pyridin-3-amine
[191] N-tert-butyl-2-(5-(thiazol-5-ylethynyl)thiophen-2-yl)imidazo[1,2-a]pyridin-3-amine
[192] N-tert-butyl-2-(5-(pyridin-2-ylethynyl)thiophen-2-yl)imidazo[1,2-a]pyridin-3-amine hydrochloride
[193] 6-chloro-N-cyclohexyl-2-(3-((6-methylpyridin-2-yl)ethynyl)phenyl)imidazo[1,2-a]pyridin-3-amine
[194] 5,7-dimethyl-N-phenethyl-2-(5-(phenylethynyl)thiophen-2-yl)imidazo[1,2-a]pyrimidin-3-amine
[195] N-(3-methoxyphenethyl)-5,7-dimethyl-2-(5-(phenylethynyl)thiophen-2-yl)imidazo[1,2-a]pyrimidin-3-amine
[196] N-(3-methoxyphenethyl)-5,7-dimethyl-2-(4-(phenylethynyl)thiophen-2-yl)imidazo[1,2-a]pyrimidin-3-amine
[197] N-(3-methoxyphenethyl)-5,7-dimethyl-2-(5-(phenylethynyl)furan-2-yl)imidazo[1,2-a]pyrimidin-3-amine
[198] N-(4-chlorobenzyl)-8-methyl-2-(5-(phenylethynyl)thiophen-2-yl)imidazo[1,2-a]pyridin-3-amine
[199] N-(3-methoxyphenethyl)-5-methyl-2-(4-(phenylethynyl)thiophen-2-yl)imidazo[1,2-a]pyridin-3-amine
[200] N-(2-methylhexan-2-yl)-2-(4-(phenylethynyl)thiophen-2-yl)imidazo[1,2-a]pyrazin-3-amine
[201] N-phenethyl-2-(4-(phenylethynyl)thiophen-2-yl)imidazo[1,2-a]pyrazin-3-amine
[202] N-(3-methoxyphenethyl)-2-(4-(phenylethynyl)thiophen-2-yl)imidazo[1,2-a]pyrazin-3-amine
[203] 2-(4-(phenylethynyl)thiophen-2-yl)-N-(2-(thiophen-2-yl)ethyl)imidazo[1,2-a]pyrazin-3-amine
[204] N-(4-chlorobenzyl)-2-(5-(phenylethynyl)thiophen-2-yl)imidazo[1,2-a]pyridin-3-amine
[205] N-(2-methylpentan-2-yl)-2-(5-(phenylethynyl)thiophen-2-yl)imidazo[1,2-a]pyrazin-3-amine
[206] N-(cyclohexylmethyl)-2-(4-(phenylethynyl)thiophen-2-yl)imidazo[1,2-a]pyrazin-3-amine
[207] N-(2-methoxybenzyl)-2-(5-(phenylethynyl)furan-2-yl)imidazo[1,2-a]pyrazin-3-amine
[208] N-(cyclohexylmethyl)-2-(4-(phenylethynyl)thiophen-2-yl)imidazo[1,2-a]pyridin-3-amine
[209] N-(2-methylpentan-2-yl)-2-(4-(phenylethynyl)thiophen-2-yl)imidazo[1,2-a]pyridin-3-amine
[210] 8-bromo-6-methyl-2-(3-((6-methylpyridin-2-yl)ethynyl)phenyl)-N-(2,4,4-trimethylpentan-2-yl)imidazo[1,2-a]pyridin-3-amine
[211] 8-bromo-N-cyclopentyl-6-methyl-2-(3-(pyridin-2-ylethynyl)phenyl)imidazo[1,2-a]pyridin-3-amine
[212] N-cyclopentyl-2-(4-(phenylethynyl)thiophen-2-yl)imidazo[1,2-a]pyrazin-3-amine
[213] N-(1-phenylethyl)-2-(4-(phenylethynyl)thiophen-2-yl)imidazo[1,2-a]pyrazin-3-amine
[214] N-(2-methylpentan-2-yl)-2-(4-(phenylethynyl)thiophen-2-yl)imidazo[1,2-a]pyrazin-3-amine
[215] 8-bromo-N-cyclohexyl-6-methyl-2-(5-(pyridin-2-ylethynyl)furan-2-yl)imidazo[1,2-a]pyridin-3-amine
[216] N-cyclopentyl-2-(5-(phenylethynyl)furan-2-yl)imidazo[1,2-a]pyrazin-3-amine
[217] N-(3-methoxyphenethyl)-7-methyl-2-(4-(phenylethynyl)thiophen-2-yl)imidazo[1,2-a]pyrimidin-3-amine
[218] 8-(benzyloxy)-2-(5-(phenylethynyl)thiophen-2-yl)-N-(2,4,4-trimethylpentan-2-yl)imidazo[1,2-a]pyridin-3-amine
[219] 8-(benzyloxy)-N-cyclopentyl-2-(4-(phenylethynyl)thiophen-2-yl)imidazo[1,2-a]pyridin-3-amine
[220] 8-(benzyloxy)-N-(2-methylpentan-2-yl)-2-(4-(phenylethynyl)thiophen-2-yl)imidazo[1,2-a]pyridin-3-amine
[221] 6-chloro-N-(4-fluorobenzyl)-2-(5-(phenylethynyl)furan-2-yl)imidazo[1,2-a]pyridin-3-amine
[222] 6-bromo-N-butyl-5-methyl-2-(4-(phenylethynyl)thiophen-2-yl)imidazo[1,2-a]pyridin-3-amine
[223] N-(furan-2-yl)-8-methyl-2-(5-(phenylethynyl)furan-2-yl)imidazo[1,2-a]pyridin-3-amine
[224] N-(furan-2-yl)-2-(5-(phenylethynyl)furan-2-yl)imidazo[1,2-a]pyridin-3-amine
[225] N-(furan-2-yl)-2-(5-(phenylethynyl)furan-2-yl)-7-propylimidazo[1,2-a]pyridin-3-amine
[226] 5,7-dimethyl-2-(4-(phenylethynyl)thiophen-2-yl)-N-(3-(trifluoromethyl)phenyl)imidazo[1,2-a]pyrimidin-3-amine
[227] 6-bromo-N-(4-chlorophenethyl)-8-methyl-2-(4-(phenylethynyl)thiophen-2-yl)imidazo[1,2-a]pyridin-3-amine
[228] N-(4-chlorophenethyl)-7-phenyl-2-(4-(phenylethynyl)thiophen-2-yl)imidazo[1,2-a]pyridin-3-amine
[229] N-phenethyl-7-phenyl-2-(4-(phenylethynyl)thiophen-2-yl)imidazo[1,2-a]pyridin-3-amine
[230] N-(4-chlorobenzyl)-5,7-dimethyl-2-(4-(phenylethynyl)thiophen-2-yl)imidazo[1,2-a]pyridin-3-amine
[231] 6-bromo-N-(4-chlorobenzyl)-5-methyl-2-(4-(phenylethynyl)thiophen-2-yl)imidazo[1,2-a]pyridin-3-amine
[232] 8-bromo-N-(4-chlorobenzyl)-6-methyl-2-(4-(phenylethynyl)thiophen-2-yl)imidazo[1,2-a]pyridin-3-amine
[233] N-(3-methoxyphenethyl)-2-(4-(phenylethynyl)thiophen-2-yl)-5-propylimidazo[1,2-a]pyridin-3-amine
[234] 6-bromo-8-methyl-2-(4-(phenylethynyl)thiophen-2-yl)-N-(2-(thiophen-2-yl)ethyl)imidazo[1,2-a]pyridin-3-amine
[235] 7-phenyl-2-(4-(phenylethynyl)thiophen-2-yl)-N-(2-(thiophen-2-yl)ethyl)imidazo[1,2-a]pyridin-3-amine
[236] 6,8-dibromo-N-(2-methylpentan-2-yl)-2-(5-(phenylethynyl)thiophen-2-yl)imidazo[1,2-a]pyridin-3-amine
[237] 6-bromo-N-(2,6-dimethylphenyl)-8-methyl-2-(4-(phenylethynyl)thiophen-2-yl)imidazo[1,2-a]pyridin-3-amine
[238] N-cyclohexyl-2-(3-(pyridin-2-ylethynyl)phenyl)imidazo[1,2-a]pyrazin-3-amine
[239] 2-(3-(pyridin-2-ylethynyl)phenyl)-N-(2,4,4-trimethylpentan-2-yl)imidazo[1,2-a]pyrazin-3-amine
[240] N-cyclopentyl-2-(4-(pyridin-2-ylethynyl)phenyl)imidazo[1,2-a]pyrazin-3-amine
[241] 8-chloro-2-(4-(pyridin-2-ylethynyl)phenyl)-6-(trifluoromethyl)-N-(2,4,4-trimethylpentan-2-yl)imidazo[1,2-a]pyridin-3-amine
[242] N-(4-fluorophenyl)-2-(4-(pyridin-2-ylethynyl)phenyl)imidazo[1,2-a]pyrazin-3-amine
[243] N-cyclopentyl-2-(2-methyl-6-(phenylethynyl)pyridin-3-yl)imidazo[1,2-a]pyrazin-3-amine
[244] N-cyclohexyl-2-(2-methyl-6-(phenylethynyl)pyridin-3-yl)imidazo[1,2-a]pyrazin-3-amine
[245] N-cyclopentyl-2-(5-(pyridin-2-ylethynyl)furan-2-yl)imidazo[1,2-a]pyrazin-3-amine
[246] 8-bromo-N-cyclopentyl-6-methyl-2-(5-(pyridin-2-ylethynyl)furan-2-yl)imidazo[1,2-a]pyridin-3-amine
[247] N-cyclohexyl-2-(5-(pyridin-2-ylethynyl)furan-2-yl)imidazo[1,2-a]pyrazin-3-amine
[248] 2-(5-(pyridin-2-ylethynyl)furan-2-yl)-N-(2,4,4-trimethylpentan-2-yl)imidazo[1 ,2-a]pyrazin-3-amine
[249] N-cyclopentyl-2-(3-((6-methylpyridin-2-yl)ethynyl)phenyl)imidazo[1,2-a]pyrazin-3-amine
[250] 8-bromo-N-cyclopentyl-6-methyl-2-(4-(pyridin-2-ylethynyl)phenyl)imidazo[1,2-a]pyridin-3-amine
[251] N-cyclohexyl-2-(3-((6-methylpyridin-2-yl)ethynyl)phenyl)imidazo[1,2-a]pyrazin-3-amine
[252] N-(4-fluorophenyl)-2-(3-((6-methylpyridin-2-yl)ethynyl)phenyl)imidazo[1,2-a]pyrazin-3-amine
[253] 2-(5-(pyridin-2-ylethynyl)thiophen-2-yl)-N-(2,4,4-trimethylpentan-2-yl)imidazo[1,2-a]pyrimidin-3-amine
[254] N-cyclohexyl-2-(3-(pyridin-2-ylethynyl)phenyl)imidazo[1,2-a]pyrimidin-3-amine
[255] 2-(3-(pyridin-2-ylethynyl)phenyl)-N-(2,4,4-trimethylpentan-2-yl)imidazo[1,2-a]pyrimidin-3-amine
[256] 2-(6-(phenylethynyl)pyridin-3-yl)-N-(2,4,4-trimethylpentan-2-yl)imidazo[1,2-a]pyrimidin-3-amine
[257] N-cyclopentyl-2-(5-(pyridin-2-ylethynyl)furan-2-yl)imidazo[1,2-a]pyrimidin-3-amine
[258] N-tert-butyl-2-(5-(pyridin-2-ylethynyl)furan-2-yl)imidazo[1,2-a]pyrimidin-3-amine
[259] N-cyclopentyl-2-(3-((6-methylpyridin-2-yl)ethynyl)phenyl)imidazo[1,2-a]pyrimidin-3-amine
[260] N-cyclopentyl-2-(3-(pyridin-2-ylethynyl)phenyl)imidazo[1,2-a]pyridin-3-amine
[261] N-tert-butyl-2-(3-(pyridin-2-ylethynyl)phenyl)imidazo[1,2-a]pyridin-3-amine
[262] 2-(3-(pyridin-2-ylethynyl)phenyl)-N-(2,4,4-trimethylpentan-2-yl)imidazo[1,2-a]pyridin-3-amine
[263] N-(4-fluorophenyl)-2-(3-(pyridin-2-ylethynyl)phenyl)imidazo[1,2-a]pyridin-3-amine
[264] N-tert-butyl-2-(4-(pyridin-2-ylethynyl)phenyl)imidazo[1,2-a]pyridin-3-amine
[265] N-cyclopentyl-2-(2-methyl-6-(phenylethynyl)pyridin-3-yl)imidazo[1,2-a]pyridin-3-amine
[266] N-(4-fluorophenyl)-2-(2-methyl-6-(phenylethynyl)pyridin-3-yl)imidazo[1,2-a]pyridin-3-amine
[267] N-cyclopentyl-6-methyl-2-(5-(pyridin-2-ylethynyl)thiophen-2-yl)imidazo[1,2-a]pyridin-3-amine
[268] N-tert-butyl-6-methyl-2-(5-(pyridin-2-ylethynyl)thiophen-2-yl)imidazo[1,2-a]pyridin-3-amine
[269] N-cyclohexyl-6-methyl-2-(5-(pyridin-2-ylethynyl)thiophen-2-yl)imidazo[1,2-a]pyridin-3-amine
[270] 6-methyl-2-(5-(pyridin-2-ylethynyl)thiophen-2-yl)-N-(2,4,4-trimethylpentan-2-yl)imidazo[1,2-a]pyridin-3-amine
[271] 6-methyl-2-(2-methyl-6-(phenylethynyl)pyridin-3-yl)-N-(2,4,4-trimethylpentan-2-yl)imidazo[1,2-a]pyridin-3-amine
[272] N-cyclopentyl-6-methyl-2-(5-(pyridin-2-ylethynyl)furan-2-yl)imidazo[1,2-a]pyridin-3-amine
[273] N-cyclohexyl-6-methyl-2-(3-((6-methylpyridin-2-yl)ethynyl)phenyl)imidazo[1,2-a]pyridin-3-amine
[274] N-cyclohexyl-7-methyl-2-(5-(pyridin-2-ylethynyl)thiophen-2-yl)imidazo[1,2-a]pyridin-3-amine
[275] 7-methyl-2-(5-(pyridin-2-ylethynyl)thiophen-2-yl)-N-(2,4,4-trimethylpentan-2-yl)imidazo[1,2-a]pyridin-3-amine
[276] N-tert-butyl-7-methyl-2-(3-(pyridin-2-ylethynyl)phenyl)imidazo[1,2-a]pyridin-3-amine
[277] N-(4-fluorophenyl)-7-methyl-2-(3-(pyridin-2-ylethynyl)phenyl)imidazo[1,2-a]pyridin-3-amine
[278] N-tert-butyl-7-methyl-2-(4-(pyridin-2-ylethynyl)phenyl)imidazo[1,2-a]pyridin-3-amine [279] N-(4-fluorophenyl)-7-methyl-2-(5-(pyridin-2-ylethynyl)furan-2-yl)imidazo[1,2-a]pyridin-3-amine
[280] N-cyclohexyl-8-methyl-2-(6-(phenylethynyl)pyridin-3-yl)imidazo[1,2-a]pyridin-3-amine
[281] N-cyclopentyl-2-(6-(phenylethynyl)pyridin-3-yl)imidazo[1,2-a]pyridin-3-amine
[282] N-tert-butyl-2-(6-(phenylethynyl)pyridin-3-yl)imidazo[1,2-a]pyridin-3-amine
[283] N-cyclohexyl-2-(6-(phenylethynyl)pyridin-3-yl)imidazo[1,2-a]pyridin-3-amine
[284] 2-(6-(phenylethynyl)pyridin-3-yl)-N-(2,4,4-trimethylpentan-2-yl)imidazo[1,2-a]pyridin-3-amine
[285] N-tert-butyl-2-(5-(pyridin-2-ylethynyl)furan-2-yl)imidazo[1,2-a]pyridin-3-amine
[286] N-cyclohexyl-2-(5-(pyridin-2-ylethynyl)furan-2-yl)imidazo[1,2-a]pyridin-3-amine
[287] N-(4-fluorophenyl)-2-(5-(pyridin-2-ylethynyl)furan-2-yl)imidazo[1,2-a]pyridin-3-amine
[288] N-cyclohexyl-2-(3-((6-methylpyridin-2-yl)ethynyl)phenyl)imidazo[1,2-a]pyridin-3-amine
[289] 2-(3-((6-methylpyridin-2-yl)ethynyl)phenyl)-N-(2,4,4-trimethylpentan-2-yl)imidazo[1,2-a]pyridin-3-amine
[290] N-tert-butyl-5-methyl-2-(3-((6-methylpyridin-2-yl)ethynyl)phenyl)imidazo[1,2-a]pyridin-3-amine
[291] 5-methyl-2-(3-((6-methylpyridin-2-yl)ethynyl)phenyl)-N-(2,4,4-trimethylpentan-2-yl)imidazo[1,2-a]pyridin-3-amine
[292] N-cyclohexyl-5,7-dimethyl-2-(3-(pyridin-2-ylethynyl)phenyl)imidazo[1,2-a]pyrimidin-3-amine
[293] N-cyclohexyl-5,7-dimethyl-2-(2-methyl-6-(phenylethynyl)pyridin-3-yl)imidazo[1,2-a]pyrimidin-3-amine
[294] N-cyclopentyl-5,7-dimethyl-2-(5-(pyridin-2-ylethynyl)furan-2-yl)imidazo[1,2-a]pyrimidin-3-amine
[295] N-tert-butyl-5,7-dimethyl-2-(5-(pyridin-2-ylethynyl)furan-2-yl)imidazo[1,2-a]pyrimidin-3-amine
[296] N-(4-fluorophenyl)-8-methyl-2-(3-(pyridin-2-ylethynyl)phenyl)imidazo[1,2-a]pyridin-3-amine
[297] N-cyclohexyl-8-methyl-2-(4-(pyridin-2-ylethynyl)phenyl)imidazo[1,2-a]pyridin-3-amine
[298] N-cyclohexyl-7-ethyl-2-(5-(pyridin-2-ylethynyl)thiophen-2-yl)imidazo[1,2-a]pyridin-3-amine
[299] 7-ethyl-2-(5-(pyridin-2-ylethynyl)thiophen-2-yl)-N-(2,4,4-trimethylpentan-2-yl)imidazo[1,2-a]pyridin-3-amine
[300] N-cyclohexyl-7-ethyl-2-(3-(pyridin-2-ylethynyl)phenyl)imidazo[1,2-a]pyridin-3-amine
[301] N-cyclopentyl-7-ethyl-2-(4-(pyridin-2-ylethynyl)phenyl)imidazo[1,2-a]pyridin-3-amine
[302] N-cyclopentyl-7-ethyl-2-(6-(phenylethynyl)pyridin-3-yl)imidazo[1,2-a]pyridin-3-amine
[303] N-tert-butyl-7-ethyl-2-(6-(phenylethynyl)pyridin-3-yl)imidazo[1,2-a]pyridin-3-amine
[304] N-tert-butyl-7-ethyl-2-(5-(pyridin-2-ylethynyl)furan-2-yl)imidazo[1,2-a]pyridin-3-amine
[305] 7-isopropyl-2-(5-(pyridin-2-ylethynyl)thiophen-2-yl)-N-(2,4,4-trimethylpentan-2-yl)imidazo[1,2-a]pyridin-3-amine
[306] N-tert-butyl-7-isopropyl-2-(3-(pyridin-2-ylethynyl)phenyl)imidazo[1,2-a]pyridin-3-amine
[307] N-tert-butyl-7-isopropyl-2-(4-(pyridin-2-ylethynyl)phenyl)imidazo[1,2-a]pyridin-3-amine
[308] N-cyclohexyl-7-isopropyl-2-(5-(pyridin-2-ylethynyl)furan-2-yl)imidazo[1,2-a]pyridin-3-amine
[309] N-tert-butyl-7-isopropyl-2-(3-((6-methylpyridin-2-yl)ethynyl)phenyl)imidazo[1,2-a]pyridin-3-amine
[310] 6-chloro-N-cyclopentyl-2-(5-(pyridin-2-ylethynyl)thiophen-2-yl)imidazo[1,2-a]pyridin-3-amine
[311] N-tert-butyl-6-chloro-2-(5-(pyridin-2-ylethynyl)thiophen-2-yl)imidazo[1,2-a]pyridin-3-amine
[312] 6-chloro-N-cyclohexyl-2-(3-(pyridin-2-ylethynyl)phenyl)imidazo[1,2-a]pyridin-3-amine
[313] 6-chloro-2-(3-(pyridin-2-ylethynyl)phenyl)-N-(2,4,4-trimethylpentan-2-yl)imidazo[1,2-a]pyridin-3-amine
[314] N-tert-butyl-6-chloro-2-(4-(pyridin-2-ylethynyl)phenyl)imidazo[1,2-a]pyridin-3-amine
[315] 6-chloro-N-cyclohexyl-2-(4-(pyridin-2-ylethynyl)phenyl)imidazo[1,2-a]pyridin-3-amine
[316] N-tert-butyl-6-chloro-2-(5-(pyridin-2-ylethynyl)furan-2-yl)imidazo[1,2-a]pyridin-3-amine
[317] 6-chloro-N-cyclohexyl-2-(5-(pyridin-2-ylethynyl)furan-2-yl)imidazo[1,2-a]pyridin-3-amine
[318] 6-chloro-2-(5-(pyridin-2-ylethynyl)furan-2-yl)-N-(2,4,4-trimethylpentan-2-yl)imidazo[1,2-a]pyridin-3-amine
[319] 6-chloro-N-cyclopentyl-2-(3-((6-methylpyridin-2-yl)ethynyl)phenyl)imidazo[1,2-a]pyridin-3-amine
[320] N-tert-butyl-6-chloro-2-(3-((6-methylpyridin-2-yl)ethynyl)phenyl)imidazo[1,2-a]pyridin-3-amine
[321] N-tert-butyl-2-(5-(pyridin-2-ylethynyl)thiophen-2-yl)imidazo[1,2-a]pyridin-3-amine bis-hydrochloride
[322] N-methyl-2-(5-(pyridin-2-ylethynyl)thiophen-2-yl)imidazo[1,2-a]pyrazin-3-amine
[323] N-tert-butyl-2-(5-(pyridin-2-ylethynyl)thiophen-2-yl)imidazo[1,2-a]pyridin-3-amine bis-hydrochloride
[324] [2-(5-pyridin-2-ylethynyl-thiophen-2-yl)-imidazo[1,2-a]pyridin-3-yl]-(1,1,3,3-tetramethyl-butyl)-amine
[325] tert-butyl-[2-(5-pyrimidin-2-ylethynyl-thiophen-2-yl)-imidazo[1,2-a]pyridin-3-yl]-amine hydrochloride
[326] {2-[5-(3-amino-pyridin-2-ylethynyl)-thiophen-2-yl]-imidazo[1,2-a]pyridin-3-yl}-tert-butyl-amine
[327] tert-butyl-[2-(5-pyridin-2-ylethynyl-thiazol-2-yl)-imidazo[1,2-a]pyridin-3-yl]-amine
[328] tert-butyl-[2-(2-pyridin-2-ylethynyl-thiazol-5-yl)-imidazo[1,2-a]pyridin-3-yl]-amine
[329] tert-butyl-{2-[5-(6-fluoro-pyridin-2-ylethynyl)-thiophen-2-yl]-imidazo[1,2-a]pyridin-3-yl}-amine hydrochloride
[330] tert-butyl-{2-[5-(3-chloro-5-fluoro-phenylethynyl)-thiophen-2-yl]-imidazo[1,2-a]pyidin-3-yl}-amine hydrochloride
[331] tert-butyl-[2-(5-pyridin-2-ylethynyl-thiophen-2-yl)-imidazo[1,2-b]pyridazin-3-yl]-amine hydrochloride
[332] tert-butyl-[2-(5-thiazol-2-ylethynyl-thiophen-2-yl)-imidazo[1,2-a]pyridin-3-yl]-amine hydrochloride
[333] tert-butyl-{2-[5-(3-trifluoromethoxy-phenylethynyl)-thiophen-2-yl]-imidazo[1,2-a]pyrazin-3-yl}-amine
[334] tert-butyl-{2-[5-(3-[1,3]dioxolan-2-yl-phenylethynyl)-thiophen-2-yl]-imidazo[1,2-a]pyridin-3-yl}-amine
[335] tert-butyl-{2-[5-(3,5-dimethyl-phenylethynyl)-thiophen-2-yl]-imidazo[1,2-a]pyridin-3-yl}-amine hydrochloride
[336] tert-butyl-{2-[5-(3-fluoro-pyridin-2-ylethynyl)-thiophen-2-yl]-imidazo[1,2-a]pyridin-3-yl}-amine hydrochloride
[337] tert-butyl-{2-[5-(3-methyl-3H-imidazol-4-ylethynyl)-thiophen-2-yl]-imidazo[1,2-a]pyridin-3-yl}-amine hydrochloride and
[338] tert-butyl-{2-[5-(5-chloro-thiophen-2-ylethynyl)-thiophen-2-yl]-imidazo[1,2-a]pyridin-3-yl}-amine hydrochloride
[339] tert-butyl-{2-[5-(5-methyl-pyridin-2-ylethynyl)-thiophen-2-yl]-imidazo[1,2-a]pyridin-3-yl}-amine hydrochloride
[340] 1-{3-[5-(3-tert-butylamino-imidazo[1,2-a]pyridin-2-yl)-thiophen-2-ylethynyl]-phenyl}-ethanone hydrochloride
[341] {3-[5-(3-tert-butylamino-imidazo[1,2-a]pyridin-2-yl)-thiophen-2-ylethynyl]-phenyl}-methanol hydrochloride
[342] N-tert-butyl-2-(5-((3-methoxypyridin-2-yl)ethynyl)thiophen-2-yl)imidazo[1,2-a]pyridin-3-amine
[343] N-tert-butyl-2-(5-(thiophen-2-ylethynyl)thiophen-2-yl)imidazo[1,2-a]pyridin-3-amine hydrochloride
[344] 5-((5-(3-(tert-butylamino)imidazo[1,2-a]pyridin-2-yl)thiophen-2-yl)ethynyl)-2-fluorobenzonitrile hydrochloride
[345] N-tert-butyl-2-(5-((3,4-difluorophenyl)ethynyl)thiophen-2-yl)imidazo[1,2-a]pyridin-3-amine
[346] N-tert-butyl-2-(5-((3-(methoxymethyl)phenyl)ethynyl)thiophen-2-yl)imidazo[1,2-a]pyridin-3-amine
[347] 2-(5-((3-aminophenyl)ethynyl)thiophen-2-yl)-N-tert-butylimidazo[1,2-a]pyridin-3-amine hydrochloride
[348] N-tert-butyl-2-(5-((4-fluoro-3-methylphenyl)ethynyl)thiophen-2-yl)imidazo[1,2-a]pyridin-3-amine hydrochloride
[349] N-tert-butyl-2-(5-((3,5-difluorophenyl)ethynyl)thiophen-2-yl)imidazo[1,2-a]pyridin-3-amine
[350] N-tert-butyl-2-(5-(thiophen-3-ylethynyl)thiophen-2-yl)imidazo[1,2-a]pyridin-3-amine hydrochloride
[351] N-tert-butyl-2-(5-((3-methylpyridin-2-yl)ethynyl)thiophen-2-yl)imidazo[1,2-a]pyridin-3-amine hydrochloride
[352] 3-((5-(3-(tert-butylamino)imidazo[1,2-a]pyridin-2-yl)thiophen-2-yl)ethynyl)benzenesulfonamide hydrochloride
[353] 3-((5-(3-(tert-butylamino)imidazo[1,2-a]pyridin-2-yl)hiophen-2-yl)ethynyl)benzoic acid
[354] 3-((5-(3-(tert-butylamino)imidazo[1,2-a]pyridin-2-yl)thiophen-2-yl)ethynyl)benzamide hydrochloride
[355] N-(3-((5-(3-(tert-butylamino)imidazo[1,2-a]pyridin-2-yl)thiophen-2-yl)ethynyl)phenyl)acetamide
[356] N-tert-butyl-N-methyl-2-(5-(pyridin-2-ylethynyl)thiophen-2-yl)imidazo[1,2-a]pyridin-3-amine
[357] N,N-dimethyl-2-(5-(pyridin-2-ylethynyl)thiophen-2-yl)imidazo[1,2-a]pyridin-3-amine
[358] N-tert-butyl-N-methyl-2-(5-(pyridin-2-ylethynyl)thiazol-2-yl)imidazo[1,2-a]pyridin-3-amine
[359] (6-((5-(3-(tert-butylamino)imidazo[1,2-a]pyridin-2-yl)thiophen-2-yl)ethynyl)pyridin-2-yl)methanol
[360] N-(3-((5-(3-(tert-butylamino)imidazo[1,2-a]pyridin-2-yl)thiophen-2-yl)ethynyl)phenyl)methanesulfonamide
[361] N-tert-butyl-8-methyl-2-(5-(pyridin-2-ylethynyl)thiophen-2-yl)imidazo[1,2-a]pyrazin-3-amine hydrochloride
[362] 2-(5-(pyridin-2-ylethynyl)thiophen-2-yl)imidazo[1,2-a]pyridin-3-amine
[363] N-tert-butyl-2-(5-((3-chlorophenyl)ethynyl)thiophen-2-yl)imidazo[1,2-a]pyridin-3-amine
[364] N-tert-butyl-2-(5-((2,3-difluorophenyl)ethynyl)thiophen-2-yl)imidazo[1,2-a]pyridin-3-amine
and
[365] N-tert-butyl-7-chloro-2-(5-(pyridin-2-ylethynyl)thiophen-2-yl)imidazo[1,2-a]pyridin-3-amine
and in each case optionally in the form of corresponding salts, in particular hydrochlorides, or in each case optionally in the form of corresponding solvates.

25. A process for the production of substituted bicyclic imidazo-3-yl-amine compounds of the general formula I according to one or more of claims 1 to 24, **characterised in that** at least one compound of the general formula II, 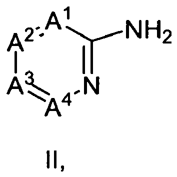 in which A¹, A², A³ and A⁴ have the meaning according to one or more of claims 1 to 24, is reacted in a reaction medium, optionally in the presence of at least one organic or inorganic acid or at least one transition metal salt with at least one isocyanide of the general formula III,
R²-N≡C III,
in which R² has the meaning according to one or more of claims 1 to 24, and at least one aldehyde of the general formula IV, 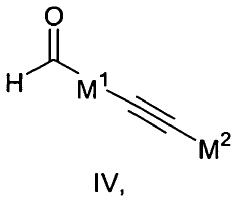 in which M¹ and M² have the meaning according to one or more of claims 1 to 24, and the resultant compound of the general formula V, 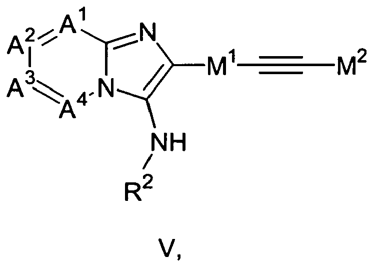 in which A¹, A², A³, A⁴, R², M¹ and M² have the above-stated meaning, is optionally purified and/or isolated, and optionally converted into a corresponding salt and this is optionally purified and/or isolated,
or at least one compound of the general formula II, 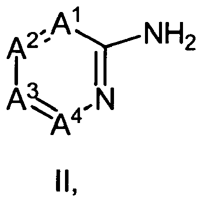 in which A¹, A², A³ and A⁴ have the meaning according to one or more of claims 1 to 24, is reacted in a reaction medium, optionally in the presence of at least one organic or inorganic acid or at least one transition metal salt with at least one isocyanide of the general formula III,
R²-N≡C III,
in which R² has the meaning according to one or more of claims 1 to 24, and at least one aldehyde of the general formula VI, 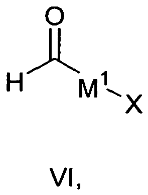 in which M¹ has the meaning according to one or more of claims 1 to 24 and X denotes a leaving group and the resultant compound of the general
formula VII, 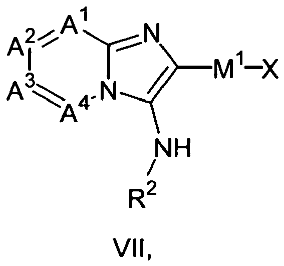 in which A¹, A², A³, A⁴, R², M¹ and X have the above-stated meaning, is optionally purified and/or isolated, and optionally converted into a corresponding salt and this is optionally purified and/or isolated, and by reaction with at least one acetylene of the general formula XI, 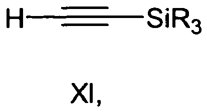 in which R, mutually independently, in each case denotes a linear or branched alkyl residue or denotes an unsubstituted phenyl residue, is converted in a reaction medium, optionally in the presence of at least one suitable catalyst and optionally in the presence of at least one inorganic and/or organic base, into a correspondingly substituted compound of the general formula XII, 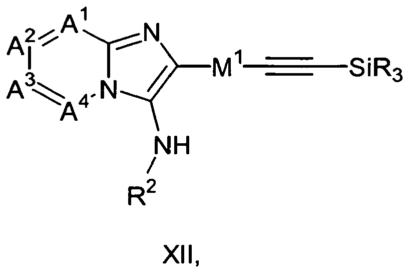 in which A¹, A², A³, A⁴, R² and M¹ have the above-stated meaning and R, mutually independently, in each case denotes a linear or branched alkyl residue or denotes an unsubstituted phenyl residue, and is optionally purified and/or isolated, and optionally converted into a corresponding salt and this is optionally purified and/or isolated,
and at least one compound of the general formula XII is converted in a reaction medium, optionally in the presence of at least one inorganic and/or organic base, optionally in the presence of at least one inorganic salt, and optionally in the presence of at least one ammonium salt, into a correspondingly substituted compound of the general formula XIII, 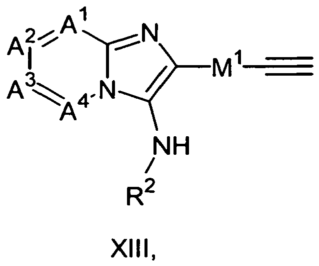 in which A¹, A², A³, A⁴, R² and M¹ have the above-stated meaning, and is optionally purified and/or isolated, and optionally converted into a corresponding salt and this is optionally purified and/or isolated,
and at least one compound of the general formula XIII and/or at least one compound of the general formula XII is converted by reaction with at least one compound of the general formula M²-X, in which M² has the meaning according to one or more of claims 1 to 24 and X denotes a leaving group, in a reaction medium, optionally in the presence of at least one suitable catalyst, optionally in the presence of at least one inorganic and/or organic base, optionally in the presence of at least one inorganic salt and optionally in the presence of at least one ammonium salt into a correspondingly substituted compound of the general formula V, 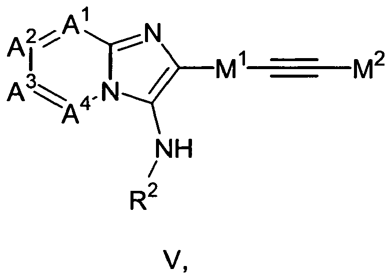 in which A¹, A², A³, A⁴, R², M¹ and M² have the above-stated meaning and is optionally purified and/or isolated, and optionally converted into a corresponding salt and this is optionally purified and/or isolated,
or a compound of the general formula VII is converted by reaction with at least one acetylene of the general formula VIII, 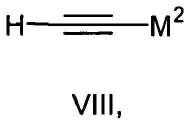 in which M² has the meaning according to one or more of claims 1 to 24 , in a reaction medium, optionally in the presence of at least one suitable catalyst and optionally in the presence of at least one inorganic and/or organic base, into a correspondingly substituted compound of the general formula V, 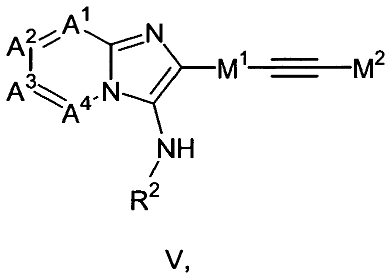 in which A¹, A², A³, A⁴, R², M¹ and M² have the above-stated meaning, and is optionally purified and/or isolated, and optionally converted into a corresponding salt and this is optionally purified and/or isolated,
and optionally the compound of the general formula V is converted by reaction with at least one compound of the general formula R³-X, in which R³ has the meaning according to one or more of claims 1 to 24 and X denotes a leaving group, in a reaction medium, in the presence of at least one organic or inorganic base,
or by reaction with at least one compound of the general formula R²⁰-C(=O)-OH, in which R²⁰ has the meaning according to one or more of claims 1 to 24, in a reaction medium, optionally in the presence of at least one organic or inorganic base and/or in the presence of at least one coupling agent,
or by reaction with at least one compound of the general formula R²⁰-C(=O)-X, in which R²⁰ has the meaning according to one or more of claims 1 to 24 and X denotes a leaving group, in a reaction medium, optionally in the presence of at least one organic or inorganic base,
or by reaction with at least one compound of the general formula R²⁰-C(=O)-H, in which R²⁰ has the meaning according to one or more of claims 1 to 24, in a reaction medium, optionally in the presence of at least one reducing agent,
into a compound of the general formula I, optionally in the form of a corresponding salt, 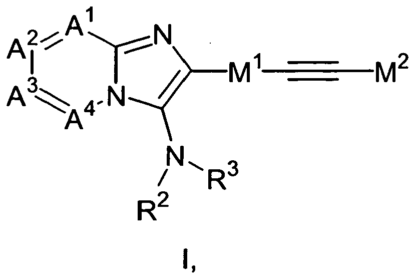 in which A¹, A², A³, A⁴, R², R³, M¹ and M² have the meaning according to one or more of claims 1 to 24, and this is optionally purified and/or isolated.

26. A process for the production of substituted bicyclic imidazo-3-yl-amine compounds of the general formula I according to one or more of claims 1 to 24, **characterised in that** at least one compound of the general formula V, 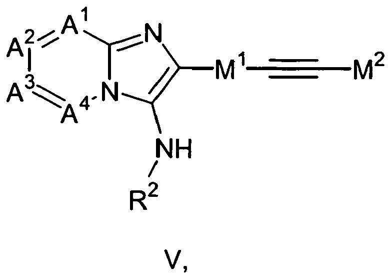 in which A¹, A², A³, A⁴, R², M¹ and M² have the meaning according to one or more of claims 1 to 24, is reacted, optionally in a reaction medium in the presence of at least one organic or inorganic acid, and the resultant compound of the general formula IX, 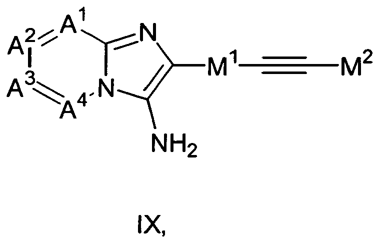 in which A¹, A², A³, A⁴, M¹ and M² have the meaning according to one or more of claims 1 to 24, is optionally purified and/or isolated, and optionally converted into a corresponding salt and this is optionally purified and/or isolated and this compound/this salt is optionally converted by reaction
in a reaction medium, in the presence of at least one inorganic or organic base, with at least one compound of the general formula R³-X, in which R³ has the meaning according to one or more of claims 1 to 24 and X denotes a leaving group, or
in a reaction medium, optionally in the presence of at least one organic or inorganic base and/or optionally in the presence of at least one coupling agent with at least one compound of the general formula R²⁰-C(=O)-OH, in which R²⁰ has the meaning according to one or more of claims 1 to 24,
or in a reaction medium, optionally in the presence of at least one organic or inorganic base with at least one compound of the general formula R²⁰-C(=O)-X, in which R²⁰ has the meaning according to one or more of claims 1 to 24 and X denotes a leaving group,
or in a reaction medium, optionally in the presence of at least one reducing agent with at least one compound of the general formula R²⁰-C(=O)-H, in which R²⁰ has the meaning according to one or more of claims 1 to 24,
into a corresponding compound of the general formula X, optionally in the form of a corresponding salt, 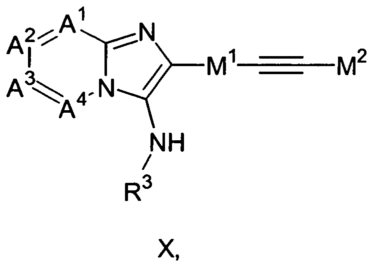 in which A¹, A², A³, A⁴, R³, M¹ and M² have the meaning according to one or more of claims 1 to 24, and this is optionally purified and/or isolated,
and optionally the compound of the general formula X is converted by reaction with at least one compound of the general formula R²-X, in which R² has the meaning according to one or more of claims 1 to 24 and X denotes a leaving group, in a reaction medium, in the presence of at least one organic or inorganic base,
or by reaction with at least one compound of the general formula R²⁰-C(=O)-OH, in which R²⁰ has the meaning according to one or more of claims 1 to 24, in a reaction medium, optionally in the presence of at least one organic or inorganic base and/or in the presence of at least one coupling agent,
or by reaction with at least one compound of the general formula R²⁰-C(=O)-X, in which R²⁰ has the meaning according to one or more of claims 1 to 24 and X denotes a leaving group, in a reaction medium, optionally in the presence of at least one organic or inorganic base,
or by reaction with at least one compound of the general formula R²⁰-C(=O)-H, in which R²⁰ has the meaning according to one or more of claims 1 to 24, in a reaction medium, optionally in the presence of at least one reducing agent,
into a compound of the general formula I, optionally in the form of a corresponding salt, 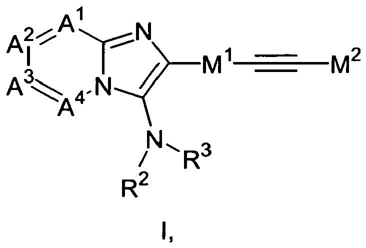 in which A¹, A², A³, A⁴, R², R³, M¹ and M² have the meaning according to one or more of claims 1 to 24, and this is optionally purified and/or isolated.

27. A medicament containing at least one compound according to one or more of claims 1 to 24 and optionally one or more physiologically acceptable auxiliary substances.

28. A medicament according to claim 27 for the prevention and/or treatment of disorders and/or diseases which are mediated at least in part by mGluR5 receptors.

29. A medicament according to claim 27 or claim 28 for the treatment and/or prevention of pain, preferably of pain selected from the group consisting of acute pain, chronic pain, neuropathic pain and visceral pain.

30. A medicament according to claim 27 or claim 28 for the treatment and/or prevention of migraine; depression; neurodegenerative diseases, preferably selected from the group consisting of Parkinson's disease, Alzheimer's disease, Huntington's chorea and multiple sclerosis; cognitive disorders, preferably cognitive deficiency states, particularly preferably of attention deficit syndrome (ADS); panic attacks; anxiety disorder; epilepsy; coughing; urinary incontinence; diarrhoea; pruritus; schizophrenia; cerebral ischaemia; muscle spasms; cramps; irritable bowel syndrome; disorders of food intake, preferably selected from the group consisting of bulimia, cachexia, anorexia and obesity; abuse of alcohol and/or drugs (in particular nicotine and/or cocaine) and/or abuse of medicines; dependency on alcohol and/or drugs (in particular nicotine and/or cocaine) and/or dependency on medicines; preferably for the prevention and/or reduction of withdrawal symptoms associated with dependency on alcohol and/or drugs (in particular nicotine and/or cocaine) and/or dependency on medicines; for the prevention and/or reduction of tolerance phenomena with regard to drugs and/or medicines, in particular with regard to opioids; of gastro-oesophageal reflux syndrome; of gastro-oesophageal reflux; for diuresis; for antinatriuresis; for influencing the cardiovascular system; for anxiolysis; for increasing vigilance; for increasing libido; for modulating locomotor activity, and for local anaesthesia.

31. Use of at least one compound according to one or more of claims 1 to 24 for the production of a medicament for the prevention and/or treatment of disorders and/or diseases which are mediated at least in part by mGluR5 receptors.

32. Use of at least one compound according to one or more of claims 1 to 24 for the production of a medicament for the treatment and/or prevention of pain, preferably of pain selected from the group consisting of acute pain, chronic pain, neuropathic pain and visceral pain.

33. Use of at least one compound according to one or more the claims 1 to 24 for the production of a medicament for the treatment and/or prevention of migraine; depression; neurodegenerative diseases, preferably selected from the group consisting of Parkinson's disease, Alzheimer's disease, Huntington's chorea and multiple sclerosis; cognitive disorders, preferably cognitive deficiency states, particularly preferably of attention deficit syndrome (ADS); panic attacks; anxiety disorder; epilepsy; coughing; urinary incontinence; diarrhoea; pruritus; schizophrenia; cerebral ischaemia; muscle spasms; cramps; irritable bowel syndrome; disorders of food intake, preferably selected from the group consisting of bulimia, cachexia, anorexia and obesity; abuse of alcohol and/or drugs (in particular nicotine and/or cocaine) and/or abuse of medicines, dependency on alcohol and/or drugs (in particular nicotine and/or cocaine) and/or dependency on medicines, preferably for the prevention and/or reduction of withdrawal symptoms associated with dependency on alcohol and/or drugs (in particular nicotine and/or cocaine) and/or dependency on medicines; the development of tolerance phenomena with regard to drugs and/or medicines, in particular with regard to opioids; of gastro-oesophageal reflux-syndrome; of gastro-oesophageal reflux; for diuresis; for antinatriuresis; for influencing the cardiovascular system; for anxiolysis; for increasing vigilance; for increasing libido; for modulating locomotor activity, and for local anaesthesia.

## Revendications

1. Composés imidazo-3-yl-amine bicycliques substitués de formule générale I, 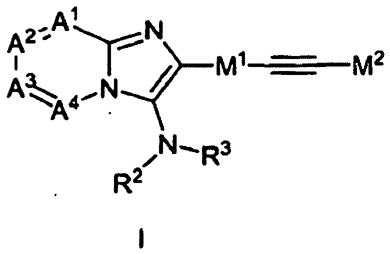 dans laquelle
A¹ représente un atome d'azote ou un groupe C-R^{1a},
A² représente un atome d'azote ou un groupe C-R^{1b},
A³ représente un atome d'azote ou un groupe C-R^{1c},
A⁴ représente un atome d'azote ou un groupe C-R^{1d},
R^{1a}, R^{1b}, R^{1c}, R^{1d} représentent chacun indépendamment les uns des autres un atome d'hydrogène ; un radical halogène ; -NO₂ ; -CN ; -NH₂ ; -NHR⁴ ; -NR⁵R⁶ ; -NH-C(=O)-R⁷ ; -C(=O)-R⁸, -C(=O)-NH₂ ; -C(=O)-NHR⁹ ; -C(=O)-NR¹⁰R¹¹ ; -C(=O)-OR¹² ; -(CH₂)ₘ-C(=O)-OR¹³ avec m = 1, 2, 3, 4 ou 5 ; -O-C(=O)-R¹⁴ ; -(CH₂)ₙ-O-C(=O)-R¹⁵ avec n = 1, 2, 3, 4 ou 5 ; -OR¹⁶ ; -(CH₂)ₒ-O-R¹⁷ avec o = 1, 2, 3, 4 ou 5 ; -SR¹⁸ ; -(CH₂)ₚ-S(=O)ₜ-R¹⁹ avec p = 1, 2, 3, 4 ou 5 et t = 0, 1 ou 2 ; -NH-S(=O)₂-R²⁶R²⁷ ; -S(=O)₂-NR²⁸R²⁹, - SF₅ ; un radical aliphatique linéaire ou ramifié, saturé ou insaturé, non substitué ou substitué au moins une fois ; un radical cycloaliphatique saturé ou insaturé, non substitué ou substitué au moins une fois, comprenant éventuellement au moins un hétéroatome en tant qu'élément de cycle, qui peut être relié par un groupe alkylène linéaire ou ramifié et/ou condensé avec un système cyclique mono- ou polycyclique non substitué ou substitué au moins une fois ; ou un radical aryle ou hétéroaryle non substitué ou substitué au moins une fois, qui peut être relié par un groupe alkylène linéaire ou ramifié et/ou condensé avec un système cyclique mono- ou polycyclique non substitué ou substitué au moins une fois,
ou R^{1a} et R^{1b} forment éventuellement avec le pont C-C qui les relie un radical phényle annelé non substitué ou substitué au moins une fois,
ou R^{1b} et R^{1c} forment éventuellement avec le pont C-C qui les relie un radical phényle annelé non substitué ou substitué au moins une fois,
ou R^{1c} et R^{1d} forment éventuellement avec le pont C-C qui les relie un radical phényle annelé non substitué ou substitué au moins une fois,
R² et R³ représentent chacun indépendamment l'un de l'autre un radical hydrogène ; -C(=O)-R²⁰ ; -(CH₂)_{q}-C(=O)-R²¹ avec q = 1, 2, 3, 4 ou 5 ; -C(=O)-O-R²² ; - (CH₂)ᵣ-C(=O)-O-R²³ avec r = 1, 2, 3, 4 ou 5 ; -C(=O)-NHR²⁴ ; -(CH2)ₛ-C(=O)-NHR²⁵ avec s = 1, 2, 3, 4 ou 5 ; un radical aliphatique linéaire ou ramifié, saturé ou insaturé, non substitué ou substitué au moins une fois ; un radical cycloaliphatique saturé ou insaturé, non substitué ou substitué au moins une fois, comprenant éventuellement au moins un hétéroatome en tant qu'élément de cycle, qui peut être relié par un groupe alkylène linéaire ou ramifié et/ou condensé avec un système cyclique mono- ou polycyclique non substitué ou substitué au moins une fois ; ou un radical aryle ou hétéroaryle non substitué ou substitué au moins une fois, qui peut être relié par un groupe alkylène linéaire ou ramifié et/ou condensé avec un système cyclique mono- ou polycyclique non substitué ou substitué au moins une fois,
ou R² et R³ forment ensemble avec l'atome d'azote qui les relie en tant qu'élément de cycle un radical hétérocycloaliphatique saturé ou insaturé, comprenant éventuellement au moins un autre hétéroatome supplémentaire en tant qu'élément de cycle, qui peut être condensé avec un système cyclique mono- ou polycyclique non substitué ou substitué au moins une fois,
R⁴, R⁵, R⁶, R⁷, R⁹, R¹⁰, R¹¹, R¹⁴ et R¹⁵ représentent chacun indépendamment les uns des autres un radical aliphatique linéaire ou ramifié, saturé ou insaturé, ou un radical aryle ou hétéroaryle non substitué ou substitué au moins une fois, qui peut être relié par un groupe alkylène linéaire ou ramifié et/ou condensé avec un système cyclique mono- ou polycyclique non substitué ou substitué au moins une fois,
R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ et R²⁹ représentent chacun indépendamment les uns des autres un radical hydrogène ; un radical aliphatique linéaire ou ramifié, saturé ou insaturé, ou un radical aryle ou hétéroaryle non substitué ou substitué au moins une fois, qui peut être relié par un groupe alkylène linéaire ou ramifié et/ou condensé avec un système cyclique mono- ou polycyclique non substitué ou substitué au moins une fois,
M¹ représente un radical aryle ou hétéroaryle, qui peut être substitué avec au moins un substituant supplémentaire et/ou condensé avec un système cyclique mono- ou polycyclique non substitué ou substitué au moins une fois,
M² représente un radical aryle ou hétéroaryle, qui est non substitué ou substitué au moins un fois et éventuellement condensé avec un système cyclique mono-ou polycyclique non substitué ou substitué au moins une fois,
dans lesquels
si un ou plusieurs des substituants R^{1a}, R^{1b}, R^{1c}, R^{1d} et R² à R²⁹ représentent un radical aliphatique saturé ou insaturé, qui est substitué une ou plusieurs fois, celui-ci peut être substitué avec 1, 2, 3, 4 ou 5 substituants choisis indépendamment les uns des autres dans le groupe constitué par F, Cl, Br, -NO₂, - CN, -OH, -SH et -NH₂,
si un ou plusieurs des substituants R^{1a}, R^{1b}, R^{1c}, R^{1d}, R² et R³ représentent un radical cycloaliphatique ou comprennent un radical cycloaliphatique, qui est substitué une ou plusieurs fois, celui-ci peut éventuellement être substitué avec 1, 2, 3, 4 ou 5 substituants choisis indépendamment les uns des autres dans le groupe constitué par F, Cl, Br, I, -CN, -CF₃, -SF₅, -OH, -NH₂, -O-CF₃, -SH, -O-alkyle en C₁₋₅, - (CH₂)-O-alkyle en C₁₋₅, -S-alkyle en C₁₋₅, -alkyle en C₁₋₅, -alcényle en C₂₋₅, -alcynyle en C₂₋₅, -C(=O)-O-alkyle en C₁₋₅, -C(=O)-CF₃, -S(=O)₂-alkyle en C₁₋₅, -S(=O)-alkyle en C₁₋₅, -S(=O)₂-phényle, oxo (=O), thioxo (=S), -N(alkyle en C₁₋₅)₂, -N(H)(alkyle en C₁₋₅), -NO₂, -S-CF₃, -C(=O)-OH, -NH-S(=O)₂-alkyle en C₁₋₅, -NH-C(=O)-alkyle en C₁₋₅, - C(=O)-H ; -C(=O)-alkyle en C₁₋₅, -C(=O)-NH₂, -C(=O)-N (alkyle en C₁₋₅)₂, -C (=O) -N (H) (alkyle en C₁₋₅) et phényle, les radicaux alkyle en C₁₋₅ susmentionnés pouvant chacun être linéaires ou ramifiés et les radicaux phényle pouvant chacun être non substitués ou substitués avec 1, 2, 3 ou 4 substituants choisis indépendamment les uns des autres dans le groupe constitué par F, Cl, Br, I, méthyle, éthyle, méthoxy et éthoxy,
si les radicaux cycloaliphatiques comprennent un ou plusieurs hétéroatomes en tant qu'éléments de cycle, ceux-ci peuvent comprendre 1, 2, 3, 4 ou 5 hétéroatomes en tant qu'éléments de cycle, qui peuvent chacun être choisis indépendamment les uns des autres dans le groupe constitué par l'azote, l'oxygène et le soufre,
si les substituants R² et R³ forment ensemble avec l'atome d'azote qui les relie en tant qu'élément de cycle un radical hétérocycloaliphatique saturé ou insaturé, qui est substitué une ou plusieurs fois, celui-ci est éventuellement substitué avec 1, 2, 3, 4 ou 5 substituants choisis indépendamment les uns des autres dans le groupe constitué par F, Cl, Br, I, -CN, -CF₃, -SF₅, -OH, -NH₂, -O-CF₃, -SH, -O-alkyle en C₁₋₅, - (CH₂)-O-alkyle en C₁₋₅, -S-alkyle en C₁₋₅, -alkyle en C₁₋₅, -alcényle en C₂₋₅, -alcynyle en C₂₋₅, -C(=O)-O-alkyle en C₁₋₅, -C(=O)-CF₃, -S(=O)₂-alkyle en C₁₋₅, -S(=O)-alkyle en C₁₋₅, -S(=O)₂-phényle, oxo (=O), thioxo (=S), -N (alkyle en C₁₋₅)₂, -N(H)(alkyle en C₁₋₅), -NO₂, -S-CF₃, -C(=O)-OH, -NH-S(=O)₂-alkyle en C₁₋₅, -NH-C(=O)-alkyle en C₁₋₅, - C(=O)-H ; -C(=O)-alkyle en C₁₋₅, -C(=O)-NH₂, -C(=O)-N (alkyle en C₁₋₅)₂, -C(=O)-N(H)(alkyle en C₁₋₅) et phényle, les radicaux alkyle en C₁₋₅ susmentionnés pouvant chacun être linéaires ou ramifiés et les radicaux phényle pouvant chacun être non substitués ou substitué avec 1, 2, 3 ou 4 substituants choisis indépendamment les uns des autres dans le groupe constitué par F, Cl, Br, I, méthyle, éthyle, méthoxy et éthoxy,
si les radicaux hétérocycloaliphatiques comprennent un ou plusieurs hétéroatomes supplémentaires en tant qu'éléments de cycle, ceux-ci peuvent comprendre 1, 2, 3, 4 ou 5 hétéroatomes en tant qu'éléments de cycle, qui peuvent chacun être choisis indépendamment les uns des autres dans le groupe constitué par l'azote, l'oxygène et le soufre,
si un ou plusieurs des substituants R^{1a}, R^{1b}, R^{1c}, R^{1d}, R² à R²⁹ et M¹ et M² représentent un radical aryle ou hétéroaryle ou comprennent un radical aryle ou hétéroaryle, qui est substitué une ou plusieurs fois, celui-ci peut éventuellement être substitué avec 1, 2, 3, 4 ou 5 substituants choisis indépendamment les uns des autres dans le groupe constitué par halogène, -CN, -CH₂-CN, -NO₂, -OH, -SH, -NH₂, -CH₂-NH₂, -C(=O)-OH, alkyle en C₁₋₅, -(CH₂)-OH, -(CH₂)-O-alkyle en C₁₋₅, - alcényle en C₂₋₅, -alcynyle en C₂₋₅, -S-alkyle en C₁₋₅, - O-alkyle en C₁₋₅, -CF₃, -SF₅, -CHF₂, -CH₂F, -O-CF₃, -O-CHF₂, -O-CH₂F, -C(=O)-CF₃, -S-CF₃, -S-CHF₂, -S-CH₂F, - S(=O)₂-phényle, -S(=O)₂-alkyle en C₁₋₅, -S(=O)-alkyle en C₁₋₅, -NH-alkyle en C₁₋₅, -N-(alkyle en C₁₋₅)₂, -CH₂-NH-alkyle en C₁₋₅, -CH₂-N-(alkyle en C₁₋₅)₂, -C(=O)-O-alkyle en C₁₋₅, -C(=O)-H, -C(=O)-alkyle en C₁₋₅, -CH₂-O-C(=O)-phényle, -O-C(=O)-phényle, -NH-S(=O)₂-alkyle en C₁₋₅, - NH-C(=O)-alkyle en C₁₋₅, -S(=O)₂-NH₂, -S(=O)₂-NH-alkyle en C₁₋₅, -S(=O)₂-N(alkyle en C₁₋₅)₂, -NH-C(=NH)-NH₂, -NH-S(=O)₂-OH, -C(=O)-NH-alkyle en C₁₋₅, -C(=O)-NH₂, -C(=O)-N (alkyle en C₁₋₅)₂, -Si(phényle)₂[alkyle en C₁₋₅], pyrazolyle, pyrrolyle, (1,3)-dioxolanyle, phényle, furyle (furanyle), thiazolyle, thiadiazolyle, thiophényle (thiényle), phénoxy, benzyle et phénéthyle, les substituants cycliques pouvant chacun eux-mêmes éventuellement être substitués avec 1, 2, 3 ou 4 substituants choisis indépendamment les uns des autres dans le groupe constitué par F, Cl, Br, I, -CF₃, méthyle, éthyle, méthoxy et éthoxy,
si un ou plusieurs des substituants R^{1a}, R^{1b}, R^{1c}, R^{1d}, R² à R²⁹ et M¹ et M² représentent un radical hétéroaryle ou comprennent un radical hétéroaryle, son ou ses hétéroatomes peuvent chacun être choisis indépendamment les uns des autres dans le groupe constitué par l'oxygène, le soufre et l'azote,
si les substituants R^{1a} et R^{1b} ou R^{1b} et R^{1c} ou R^{1c} et R^{1d} forment ensemble avec le pont C-C qui les relie un radical phényle annelé, qui est substitué une ou plusieurs fois, celui-ci peut être substitué avec 1, 2, 3 ou 4 substituants choisis indépendamment les uns des autres dans le groupe constitué par halogène, -CN, -CH₂-CN, -NO₂, -OH, -SH, -NH₂, -CH₂-NH₂, -C(=O)-OH, alkyle en C₁₋₅, -(CH₂)-O-alkyle en C₁₋₅, -alcényle en C₂₋₅, -alcynyle en C₂₋₅, -S-alkyle en C₁₋₅, -O-alkyle en C₁₋₅, -CF₃, -SF₅, -CHF₂, -CH₂F, -O-CF₃, -O-CHF₂, -O-CH₂F, -C(=O)-CF₃, -S-CF₃, -S-CHF₂, -S-CH₂F, -S(=O)₂-phényle, -S(=O)₂-alkyle en C₁₋₅, -S(=O)-alkyle en C₁₋₅, -NH-alkyle en C₁₋₅, -N-(alkyle en C₁₋₅)₂, -CH₂-NH-alkyle en C₁₋₅, -CH₂-N-(alkyle en C₁₋₅)₂, -C(=O)-O-alkyle en C₁₋₅, -C(=O)-H, -C(=O)-alkyle en C₁₋₅, -CH₂-O-C(=)-phényle, -O-C(=O)-phényle, - NH-S(=O)₂-alkyle en C₁₋₅, -NH-C(=O)-alkyle en C₁₋₅, - C(=O)-NH-alkyle en C₁₋₅, -C(=O)-NH₂, -C(=O)-N(alkyle en C₁₋₅)₂, -Si(phényle)₂[alkyle en C₁₋₅], pyrazolyle, pyrrolyle, (1,3)-dioxolanyle, phényle, furyle (furanyle), thiazolyle, thiadiazolyle, thiophényle (thiényle), phénoxy, benzyle et phénéthyle, les substituants cycliques pouvant chacun eux-mêmes éventuellement être substitués avec 1, 2, 3 ou 4 substituants choisis indépendamment les uns des autres dans le groupe constitué par F, Cl, Br, I, -CF₃, méthyle, éthyle, méthoxy et éthoxy,
si un système cyclique polycyclique est présent, les différents cycles peuvent chacun indépendamment les uns des autres être saturés, insaturés ou aromatiques, et les hétéroatomes de chaque cycle peuvent chacun être choisis indépendamment les uns des autres dans le groupe constitué par l'oxygène, l'azote et le soufre,
si un ou plusieurs des substituants R^{1a}, R^{1b}, R^{1c}, R^{1d}, R² à R²⁹ et M¹ et M² comprennent un système cyclique monocyclique ou polycyclique, qui est substitué une ou plusieurs fois, celui-ci peut éventuellement être substitué avec 1, 2, 3, 4 ou 5 substituants choisis indépendamment les uns des autres dans le groupe constitué par halogène, -CN, -NO₂, -OH, -SH, -NH₂, oxo (=O), thioxo (=S), -C(=O)-OH, -alkyle en C₁₋₅, -alcényle en C₂₋₅, -alcynyle en C₂₋₅, -(CH₂)-O-alkyle en C₁₋₅, -S-alkyle en C₁₋₅, -O-alkyle en C₁₋₅, -CF₃, -SF₅, -CHF₂, - CH_{2F}, -O-CF₃, -O-CHF₂, -O-CH₂F, -C(=O)-CF₃, -S-CF₃, -S-CHF₂, -S-CH₂F, -S(=O)₂-phényle, -S(=O)₂-alkyle en C₁₋₅, - S(=O)-alkyle en C₁₋₅, -NH-alkyle en C₁₋₅, -N(alkyle en C₁₅) (alkyle en C₁₋₅), -C(=O)-O-alkyle en C₁₋₅, -C(=O)-H, - C(=O)-alkyle en C₁₋₅, -CH₂-O-C(=O)-phényle, -O-C(=O)-phényle, -NH-S(=O)₂-alkyle en C₁₋₅, -NH-C(=O)-alkyle en C₁₋₅, -C(=O)-NH₂, -C(=O)-NH-alkyle en C₁₋₅, -C(=O)-N (alkyle en C₁₋₅)₂, pyrazolyle, phényle, furyle (furanyle), thiadiazolyle, thiophényle (thiényle), phénoxy et benzyle, les substituants cycliques pouvant chacun eux-mêmes éventuellement être substitués avec 1, 2, 3 ou 4 substituants choisis indépendamment les uns des autres dans le groupe constitué par F, Cl, Br, I, méthyle, éthyle, méthoxy et éthoxy,
à chaque fois sous la forme d'un de leurs stéréoisomères purs, notamment énantiomères ou diastéréomères, de leurs racémats ou sous la forme d'un mélange de stéréoisomères, notamment les énantiomères et/ou diastéréomères, en un rapport de mélange quelconque, ou à chaque fois sous la forme des sels correspondants, ou à chaque fois sous la forme des solvates correspondants.

2. Composés selon la revendication 1, **caractérisés en ce que**
A¹ représente un atome d'azote ou un groupe C-R^{1a},
A² représente un atome d'azote ou un groupe C-R^{1b},
A³ représente un atome d'azote ou un groupe C-R^{1c},
A⁴ représente un atome d'azote ou un groupe C-R^{1d},
R^{1a}, R^{1b}, R^{1c}, R^{1d} représentent chacun indépendamment les uns des autres un radical hydrogène ; un radical halogène ; -NO₂ ; -CN ; -NH₂ ; -NHR⁴ ; -NR⁵R⁶ ; -NH-C(=O)-R⁷ ; -C(=O)-R⁸, -C(=O)-NH₂ ; -C(=O)-NHR⁹ ; -C(=O)-NR¹⁰R¹¹ ; -C(=O)-OR¹² ; -(CH₂)ₘ-C(=O)-OR¹³ avec m = 1, 2, 3, 4 ou 5 ; -O-C(=O)-R¹⁴ ; -(CH₂)ₙ-O-C(=O)-R¹⁵ avec n = 1, 2, 3, 4 ou 5 ; -OR¹⁶ ; -(CH₂)ₒ-O-R¹⁷ avec o = 1, 2, 3, 4 ou 5 ; -SR¹⁸ ; -(CH₂)ₚ-S(=O)ₜ-R¹⁹ avec p = 1, 2, 3, 4 ou 5 et t = 0, 1 ou 2 ; -NH-S(=O)₂-R²⁶R²⁷ ; -S(=O)₂-NR²⁸R²⁹, - SF₅ ; un radical aliphatique en C₁₋₁₀ linéaire ou ramifié, saturé ou insaturé, non substitué ou substitué au moins une fois ; un radical cycloaliphatique en C₃₋₈ saturé ou insaturé, non substitué ou substitué au moins une fois, comprenant éventuellement au moins un hétéroatome en tant qu'élément de cycle, qui peut être relié par un groupe alkylène en C₁₋₅ linéaire ou ramifié ; ou un radical aryle ou hétéroaryle de 5 à 14 éléments non substitué ou substitué au moins une fois, qui peut être relié par un groupe alkylène en C₁₋₅ linéaire ou ramifié,
ou R^{1a} et R^{1b} forment éventuellement avec le pont C-C qui les relie un radical phényle annelé non substitué ou substitué au moins une fois,
ou R^{1b} et R^{1c} forment éventuellement avec le pont C-C qui les relie un radical phényle annelé non substitué ou substitué au moins une fois,
ou R^{1c} et R^{1d} forment éventuellement avec le pont C-C qui les relie un radical phényle annelé non substitué ou substitué au moins une fois,
R² et R³ représentent chacun indépendamment l'un de l'autre un radical hydrogène ; -C(=O)-R²⁰ ; -(CH₂)_{q}-C(=O)-R²¹ avec q = 1, 2, 3, 4 ou 5 ; -C(=O)-O-R²² ; - (CH₂)ᵣ-C(=O)-O-R²³ avec r = 1, 2, 3, 4 ou 5 ; -C(=O)-NHR²⁹ ; -(CH₂)S-C(=O)-NHR²⁵ avec s = 1, 2, 3, 4 ou 5 ; un radical aliphatique en C₁₋₁₆ linéaire ou ramifié, saturé ou insaturé, non substitué ou substitué au moins une fois ; un radical cycloaliphatique en C₄₋₈ saturé ou insaturé, non substitué ou substitué au moins une fois, comprenant éventuellement au moins un hétéroatome en tant qu'élément de cycle, qui peut être relié par un groupe alkylène en C₁₋₅ linéaire ou ramifié ; ou un radical aryle ou hétéroaryle de 5 à 14 éléments non substitué ou substitué au moins une fois, qui peut être relié par un groupe alkylène en C₁₋₅ linéaire ou ramifié,
ou R² et R³ forment ensemble avec l'atome d'azote qui les relie en tant qu'élément de cycle un radical hétérocycloaliphatique en C₄₋₁₀ saturé ou insaturé, comprenant éventuellement au moins un hétéroatome supplémentaire en tant qu'élément de cycle, qui est éventuellement condensé avec un système cyclique mono-ou polycyclique non substitué ou substitué au moins une fois, les cycles du système cyclique étant chacun à 5, 6 ou 7 éléments ;
R⁴, R⁵, R⁶, R⁷, R⁹, R¹⁰, R¹¹, R¹⁴ et R¹⁵ représentent chacun indépendamment les uns des autres un radical aliphatique en C₁₋₄ linéaire ou ramifié, saturé ou insaturé, ou un radical aryle ou hétéroaryle de 5 à 14 éléments non substitué ou substitué au moins une fois, qui peut être relié par un groupe alkylène en C₁₋₅ linéaire ou ramifié,
R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ et R²⁹ représentent chacun indépendamment les uns des autres un radical hydrogène ; un radical aliphatique en C₁₋₄ linéaire ou ramifié, saturé ou insaturé, ou un radical aryle ou hétéroaryle de 5 à 14 éléments non substitué ou substitué au moins une fois, qui peut être relié par un groupe alkylène en C₁₋₅ linéaire ou ramifié,
M¹ représente un radical aryle ou hétéroaryle à 5 ou 6 éléments, qui peut être substitué avec au moins un substituant supplémentaire et est éventuellement condensé avec un système cyclique mono- ou bicyclique non substitué ou substitué au moins une fois, les cycles du système cyclique étant chacun à 5, 6 ou 7 éléments, et
M² représente un radical aryle ou hétéroaryle à 5 ou 6 éléments, qui peut être non substitué ou substitué au moins une fois et condensé avec un système cyclique mono- ou bicyclique non substitué ou substitué au moins une fois, les cycles du système cyclique étant chacun à 5, 6 ou 7 éléments,
dans lesquels
les radicaux cycloaliphatiques susmentionnés peuvent éventuellement comprendre 1, 2, 3, 4 ou 5 hétéroatomes en tant qu'éléments de cycle, qui peuvent chacun être choisis indépendamment les uns des autres dans le groupe constitué par l'azote, l'oxygène et le soufre, les radicaux hétérocycloaliphatiques susmentionnés peuvent éventuellement comprendre 1, 2, 3, 4 ou 5 hétéroatomes supplémentaires en tant qu'éléments de cycle, qui peuvent chacun être choisis indépendamment les uns des autres dans le groupe constitué par l'azote, l'oxygène et le soufre,
les cycles du système cyclique mono- ou polycyclique comprennent chacun éventuellement 0, 1, 2 ou 3 hétéroatomes en tant qu'éléments de cycle, qui sont choisis indépendamment les uns des autres dans le groupe constitué par l'oxygène, l'azote et le soufre ; et
les radicaux hétéroaryle susmentionnés peuvent éventuellement comprendre 1, 2, 3, 4 ou 5 hétéroatomes en tant qu'éléments de cycle, qui peuvent chacun être choisis indépendamment les uns des autres dans le groupe constitué par l'oxygène, le soufre et l'azote.

3. Composés selon la revendication 1 ou 2, **caractérisés en ce que**
A¹ représente un groupe C-R^{1a},
A² représente un groupe C-R^{1b},
A³ représente un groupe C-R^{1c} et
A⁴ représente un groupe C-R^{1d},
ou
A¹ représente un atome d'azote,
A² représente un groupe C-R^{1b},
A³ représente un groupe C-R^{1c} et
A⁴ représente un groupe C-R^{1d},
ou
A¹ représente un groupe C-R^{1a},
A² représente un atome d'azote,
A³ représente un groupe C-R^{1c} et
A⁴ représente un groupe C-R^{1d},
ou
A¹ représente un groupe C-R^{1a},
A² représente un groupe C-R^{1b},
A³ représente un atome d'azote et
A⁴ représente un groupe C-R^{1d},
ou
A¹ représente un groupe C-R^{1a},
A² représente un groupe C-R^{1b},
A³ représente un groupe C-R^{1c} et
A⁴ représente un atome d'azote,
ou
A¹ et A³ représentent chacun un atome d'azote,
A² représente un groupe C-R^{1b} et
A⁴ représente un groupe C-R^{1d}.

4. Composés selon la revendication 3, **caractérisés en ce que**
A¹ représente un groupe C-R^{1a},
A² représente un groupe C-R^{1b},
A³ représente un groupe C-R^{1c} et
A⁴ représente un groupe C-R^{1d},
ou
A¹ représente un atome d'azote,
A² représente un groupe C-R^{1b},
A³ représente un groupe C-R^{1c} et
A⁴ représente un groupe C-R^{1d},
ou
A¹ représente un groupe C-R^{1a},
A² représente un atome d'azote,
A³ représente un groupe C-R^{1c} et
A⁴ représente un groupe C-R^{1d},
ou
A¹ représente un groupe C-R^{1a},
A² représente un groupe C-R^{1b},
A³ représente un groupe C-R^{1c} et
A⁴ représente un atome d'azote.

5. Composés selon une ou plusieurs des revendications 1 à 4, **caractérisés en ce que** R^{1a}, R^{1b}, R^{1c}, R^{1d} représentent chacun indépendamment les uns des autres -H ; -F ; -Cl, -Br ; -I ; -NO₂ ; S -CN ; -CF₃ ; - SF₅ ; -NH₂ ; -S(=O)-NH₂, -NHR⁴ ; -NR⁵R⁶ ; -C=O-OR¹² ; - (CH₂)ₘ-C(=O)-OR¹³ avec m = 1, 2 ou 3 ; -O-C(=O)-R¹⁴ ; - OR¹⁶ ; -(CH₂)ₒ-O-R¹⁷ avec o = 1, 2 ou 3 ; un radical alkyle en C₁₋₁₀ linéaire ou ramifié ; ou un radical aryle ou hétéroaryle à 5 ou 6 éléments non substitué ou substitué au moins une fois, qui peut être relié par un groupe alkylène en C₁₋₄ linéaire ou ramifié.

6. Composés selon une ou plusieurs des revendications 1 à 5, **caractérisé en ce que** R^{1a} et R^{1b} ou R^{1b} et R^{1c} ou R^{1c} et R^{1d} forment ensemble avec le pont C-C qui les relie un radical phényle annelé, qui peut être substitué avec 1, 2, 3 ou 4 substituants choisis indépendamment les uns des autres dans le groupe constitué par F, Cl, Br, -CN, méthyle, éthyle, n-propyle, iso-propyle, n-butyle, sec-butyle, iso-butyle, tert-butyle, -OH, -O-CH₃, -O-C₂H₅.

7. Composés selon une ou plusieurs des revendications 1 à 6, **caractérisé en ce que** R² et R³ représentent chacun indépendamment l'un de l'autre un radical hydrogène ; -C(=O)-R²⁰ ; -(CH₂)_{q}-C(=O)-R²¹ avec q - 1, 2, 3, 4 ou 5 ; -(CH₂)ᵣ-C(=O)-O-R²³ avec r = 1, 2, 3, 4 ou 5 ; -C(=O)-NHR²⁴ ; un radical alkyle en C₁₋₁₆ linéaire ou ramifié ; un radical cycloalkyle en C₄₋₈ non substitué ou substitué au moins une fois, qui peut être relié par un groupe alkylène en C₁₋₃ linéaire ou ramifié ; ou un radical aryle ou hétéroaryle à 5 ou 6 éléments non substitué ou substitué au moins une fois, qui peut être relié par un groupe alkylène en C₁₋₃ linéaire ou ramifié.

8. Composés selon une ou plusieurs des revendications 1 à 7, **caractérisé en ce que** R² et R³ forment ensemble avec l'atome d'azote qui les relie en tant qu'élément de cycle un radical choisi dans le groupe constitué par imidazolidinyle, pipéridinyle, pipérazinyle, morpholinyle, pyrrolidinyle, thiomorpholinyle, azépanyle, diazépanyle et azocanyle, qui peut à chaque fois être non substitué ou éventuellement substitué avec 1, 2, 3, 4 ou 5 substituants choisis indépendamment les uns des autres dans le groupe constitué par F, Cl, Br, I, -CN, méthyle, éthyle, n-propyle, iso-propyle, n-butyle, sec-butyle, iso-butyle, tert-butyle, -OH, oxo, thioxo, -O-CH₃, -O-C₂H₅, -O-C₃H₇, -NH₂, -N(CH₃)₂, -N(C₂H₅)₂, -NH-CH₃, -NH-C₂H₅, - O-CF₃, -S-CF₃, -S(=O)-CH₃, -S(=O) -C₂H₅, -S(=O)₂-CH₃, - S(=O)₂-C₂H₅, -C(=O)-OH, -C(=O)-H, -C(=O)-CH₃, -C(=O)-C₂H₅, -C (=O) -N (CH₃)₂, -C (=O) -NH-CH₃, -C (=O) -NH₂, -C (=O) -O-CH₃, -C(=O)-O-C₂H₅ et -C(=O)-O-C(CH₃)₃.

9. Composés selon une ou plusieurs des revendications 1 à 8, **caractérisé en ce que** R⁴, R⁵, R⁶, R⁷, R⁹, R¹⁰, R¹¹, R¹⁴ et R¹⁵ représentent chacun indépendamment les uns des autres un radical alkyle en C₁₋₄ linéaire ou ramifié ou un radical aryle ou hétéroaryle de 5 à 14 éléments non substitué ou substitué au moins une fois, qui peut être relié par un groupe alkylène en C₁₋₅ linéaire ou ramifié.

10. Composés selon une ou plusieurs des revendications 1 à 9, **caractérisé en ce que** R⁸, R¹², R¹³, R¹⁶, R¹⁷, R¹⁸, R¹⁹, R²⁰, R²¹, R²², R²³, R²⁴, R²⁵, R²⁶, R²⁷, R²⁸ et R²⁹ représentent chacun indépendamment les uns des autres un radical hydrogène ; un radical alkyle en C₁₋₈ linéaire ou ramifié ou un radical aryle ou hétéroaryle de 5 à 14 éléments non substitué ou substitué au moins une fois, qui peut être relié par un groupe alkylène en C₁₋₅ linéaire ou ramifié.

11. Composés selon une ou plusieurs des revendications 1 à 10, **caractérisés en ce que** M¹ représente un radical aryle ou hétéroaryle à 5 ou 6 éléments, qui peut éventuellement être substitué avec au moins un substituant supplémentaire, le radical hétéroaryle comprenant éventuellement 1, 2, 3, 4 ou 5 hétéroatomes en tant qu'éléments de cycle, qui sont choisis indépendamment les uns des autres dans le groupe constitué par l'azote, l'oxygène et le soufre.

12. Composés selon la revendication 11, **caractérisés en ce que** M¹ représente un radical choisi dans le groupe constitué par les radicaux 1 à 38 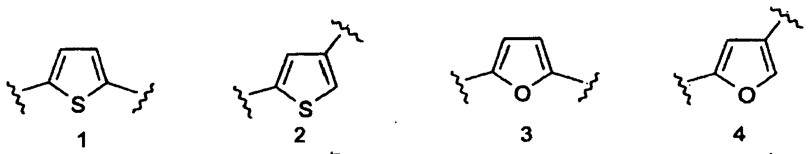 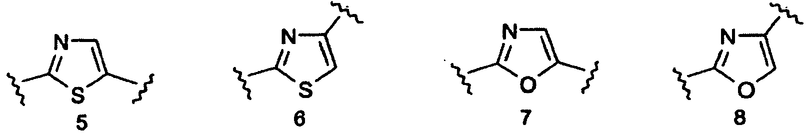 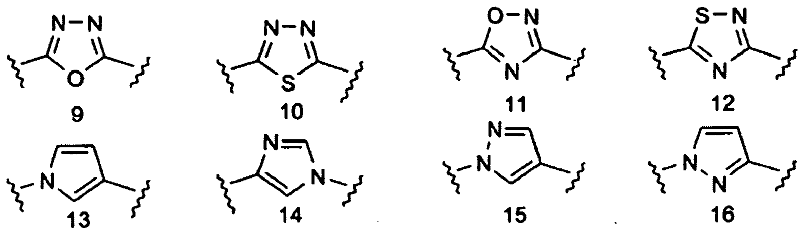 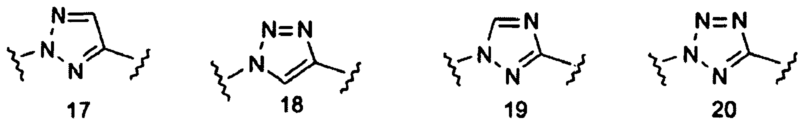 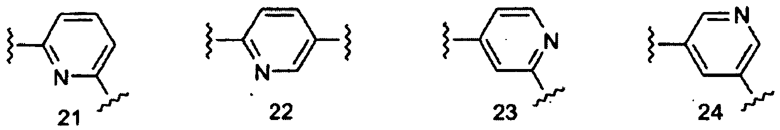 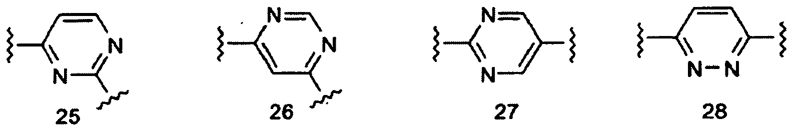 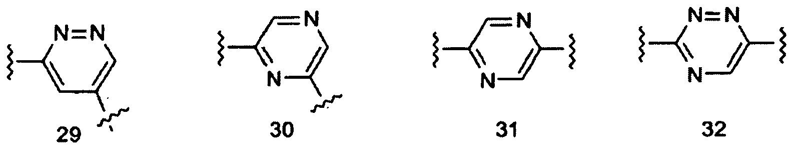 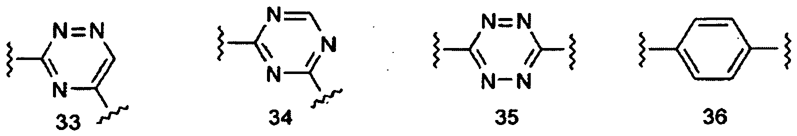 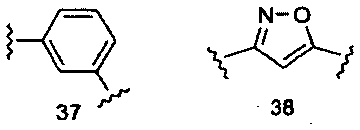 qui peut à chaque fois être relié dans un sens quelconque aux positions marquées par une ligne ondulée avec le bicycle et l'atome de carbone de la triple liaison et qui est éventuellement substitué avec 1, 2, 3 ou 4 substituants supplémentaires choisis indépendamment les uns des autres dans le groupe constitué par F, Cl, Br, -CN, -CH₂-CN, - CF₃, -SF₅, méthyle, éthyle, n-propyle, iso-propyle, n-butyle, sec-butyle, iso-butyle, tert-butyle, -OH, -O-CH₃, -O-C₂H₅, -O-C₃H₇, -NH₂, -N(CH₃)₂, -N(C₂H₅)₂, -NO₂, -O-CF₃, -S-CF₃, -SH, -C(=O)-CH₃ et -C(=O)-C₂H₅.

13. Composés selon une ou plusieurs des revendications 1 à 12, **caractérisés en ce que** M² représente un radical aryle ou hétéroaryle à 5 ou 6 substituants non substitué ou substitué au moins une fois, le radical hétéroaryle comprenant 1, 2, 3, 4 ou 5 hétéroatomes en tant qu'éléments de cycle, qui sont choisis indépendamment les uns des autres dans le groupe constitué par l'azote, l'oxygène et le soufre, et le radical aryle ou hétéroaryle pouvant être condensé avec un système cyclique mono- ou bicyclique non substitué ou substitué au moins une fois, les cycles du système cyclique étant chacun à 5 ou 6 éléments et pouvant chacun comprendre 1, 2, 3 ou 4 hétéroatomes en tant qu'éléments de cycle, qui sont choisis indépendamment les uns des autres dans le groupe constitué par l'azote, l'oxygène et le soufre.

14. Composés selon une ou plusieurs des revendications 1 à 13, **caractérisés en ce que**
A¹ représente un groupe C-R^{1a},
A² représente un groupe C-R^{1b},
A³ représente un groupe C-R^{1c} et
A⁴ représente un groupe C-R^{1d},
ou
A¹ représente un atome d'azote,
A² représente un groupe C-R^{1b},
A³ représente un groupe C-R^{1c} et
A⁴ représente un groupe C-R^{1d},
ou
A¹ représente un groupe C-R^{1a},
A² représente un atome d'azote,
A³ représente un groupe C-R^{1c} et
A⁴ représente un groupe C-R^{1d} ;
R^{1a}, R^{1b}, R^{1c}, R^{1d} représentent chacun indépendamment les uns des autres -H ; -F ; -Cl, -Br ; -I ; -NO₂ ; -CN ; - CF₃ ; -SF₅ ; -NH₂ ; -S(=O)-NH₂, -NHR⁴ ; -NR⁵R⁶ ; -C(=O)-OR¹² ; - (CH₂)ₘ-C(=O)-OR¹³ avec m = 1, 2 ou 3 ; -O-C(=O)-R¹⁴ ; - OR¹⁶ ; -(CH₂)ₒ-O-R¹⁷ avec o = 1, 2 ou 3 ; un radical alkyle linéaire ou ramifié choisi dans le groupe constitué par méthyle, éthyle, n-propyle, iso-propyle, n-butyle, iso-butyle, sec-butyle et tert-butyle, ou un radical aryle ou hétéroaryle choisi dans le groupe constitué par phényle, furanyle, thiophényle et pyridinyle, qui est à chaque fois non substitué ou substitué avec 1, 2, 3, 4 ou 5 substituants choisis indépendamment les uns des autres dans le groupe constitué par F, Cl, Br, -CN, méthyle, éthyle, n-propyle, iso-propyle, n-butyle, sec-butyle, iso-butyle, tert-butyle, -OH, -O-CH₃, -O-C₂H₅ et éventuellement relié par un groupe -(CH₂)-, -(CH₂)₂- ou - (CH₂)₃- ;
ou R^{1a} et R^{1b} ou R^{1b} et R^{1c} ou R^{1c} et R^{1d} forment éventuellement ensemble avec le pont C-C qui les relie un radical phényle annelé, qui peut être substitué avec 1, 2, 3 ou 4 substituants choisis indépendamment les uns des autres dans le groupe constitué par F, Cl, Br, -CN, méthyle, éthyle, n-propyle, iso-propyle, n-butyle, sec-butyle, iso-butyle, tert-butyle, -OH, -O-CH₃, -O-C₂H₅ ;
R² et R³ représentent chacun indépendamment l'un de l'autre un radical hydrogène ; -C(=O)-R²⁰ ; -(CH₂)_{q}-C(=O)-R²¹ avec q = 1, 2 ou 3 ; -(CH₂)ᵣC(=O)-O-R²³ avec r = 1, 2 ou 3 ; un radical alkyle en C₁₋₁₀ linéaire ou ramifié ; un radical cycloalkyle en C₄₋₈ qui est à chaque fois non substitué ou substitué avec 1, 2, 3, 4 ou 5 substituants choisis indépendamment les uns des autres dans le groupe constitué par F, Cl, Br, -CN, -CF₃, méthyle, éthyle, n-propyle, iso-propyle, n-butyle, sec-butyle, iso-butyle, tert-butyle, -OH, -O-CH₃ et -O-C₂H₅ et éventuellement relié par un groupe alkylène en C₁₋₃ linéaire ou ramifié ;
ou un radical aryle ou hétéroaryle choisi dans le groupe constitué par phényle, thiophényle, furanyle et pyridinyle, qui est à chaque fois non substitué ou substitué avec 1, 2, 3, 4 ou 5 substituants choisis indépendamment les uns des autres dans le groupe constitué par F, Cl, Br, -CN, -CF₃, méthyle, éthyle, n-propyle, iso-propyle, n-butyle, sec-butyle, iso-butyle, tert-butyle, -OH, -O-CH₃ et -O-C₂H₅ et éventuellement relié par un groupe alkylène en C₁₋₃ linéaire ou ramifié ;
ou R² et R³ forment ensemble avec l'atome d'azote qui les relie en tant qu'élément de cycle un radical choisi dans le groupe constitué par imidazolidinyle, pipéridinyle, pipérazinyle, morpholinyle, pyrrolidinyle, thiomorpholinyle, azépanyle, diazépanyle et azocanyle, qui peut à chaque fois être non substitué ou substitué avec 1, 2, 3, 4 ou 5 substituants choisis indépendamment les uns des autres dans le groupe constitué par F, Cl, Br, I, -CN, méthyle, éthyle, n-propyle, iso-propyle, n-butyle, sec-butyle, iso-butyle, tert-butyle, -OH, oxo, thioxo, -O-CH₃, -O-C₂H₅, -O-C₃H₇, -NH₂, -N(CH₃)₂, -N(C₂H₅)₂, - NH-CH₃, -NH-C₂H₅, -O-CF₃, -S-CF₃, -S(=O)-CH₃, -S(=O)-C₂H₅, - S(=O)₂-CH₃, -S(=O)₂-C₂H₅, -C(=O)-OH, -C(=O)-H, -C(=O)-CH₃, -C(=O)-C₂H₅, -C (=O) -N (CH₃)₂, -C (=O) -NH-CH₃, -C (=O) -NH₂, - C(=O)-O-CH₃, -C(=O)-O-C₂H₅ et -C(=O)-O-C(CH₃)₃ ;
R⁴, R⁵, R⁶ et R¹⁴ représentent chacun indépendamment les uns des autres un radical alkyle choisi dans le groupe constitué par méthyle, éthyle, n-propyle et isopropyle, ou un radical phényle, qui est à chaque fois non substitué ou substitué avec 1, 2 ou 3 substituants choisis indépendamment les uns des autres dans le groupe constitué par F, Cl, Br, -CN, -CF₃, -SF₅, méthyle, éthyle, n-propyle, iso-propyle, tert-butyle, - O-CH₃ et -O-C₂H₅ et éventuellement relié par un groupe - (CH₂)-, -(CH₂)₂- ou -(CH₂)₃- ;
R¹², R¹³, R¹⁶, R¹⁷, R²⁰, R²¹ et R²³ représentent chacun indépendamment les uns des autres un radical hydrogène ; un radical alkyle choisi dans le groupe constitué par méthyle, éthyle, n-propyle, iso-propyle et tert-butyle, ou un radical phényle, qui est à chaque fois non substitué ou substitué avec 1, 2 ou 3 substituants choisis indépendamment les uns des autres dans le groupe constitué par F, Cl, Br, -CN, -CF₃, -SF₅, méthyle, éthyle, n-propyle, iso-propyle, tert-butyle, - O-CH₃ et -O-C₂H₅ et éventuellement relié par un groupe - (CH₂)-, -(CH₂)₂- ou -(CH₂)₃- ;
M¹ représente un radical choisi dans le groupe constitué par les radicaux 1 à 9, 11, 21, 22 et 36 à 38 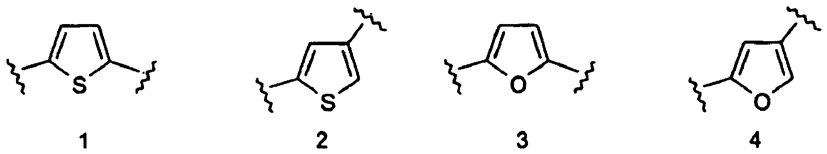 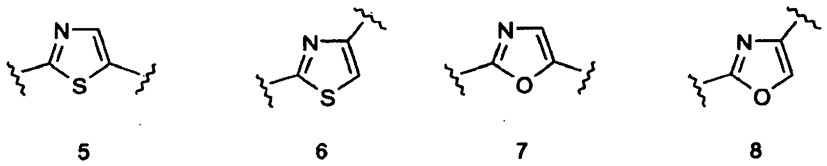 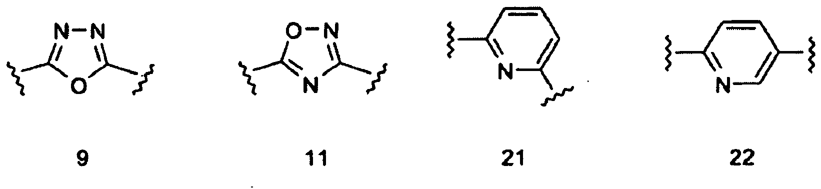 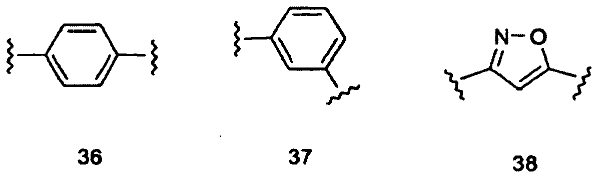 qui peut à chaque fois être relié dans un sens quelconque aux positions marquées par une ligne ondulée avec le bicycle et l'atome de carbone de la triple liaison et qui est éventuellement substitué avec 1 ou 2 substituants supplémentaires choisis indépendamment les uns des autres dans le groupe constitué par F, Cl, Br, -CN, -CH₂-CH, - CF₃, -SF₅, méthyle, éthyle, n-propyle, iso-propyle, n-butyle, iso-butyle, sec-butyle, tert-butyle, -O-CH₃ et - O-CF₃ ;
et
M² représente un radical choisi dans le groupe constitué par les radicaux 1 à 36 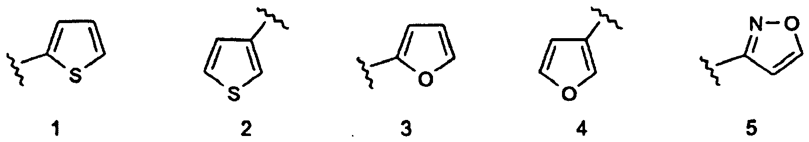 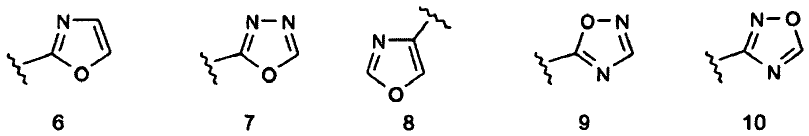 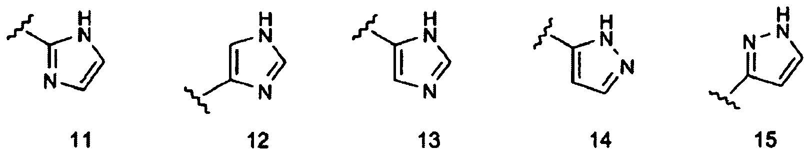 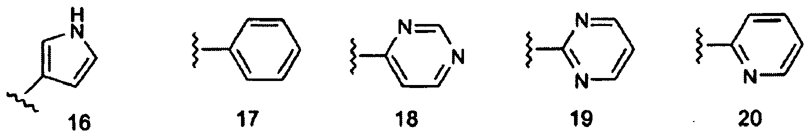 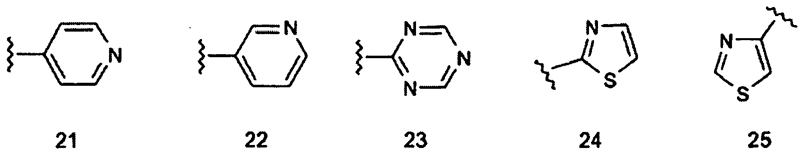 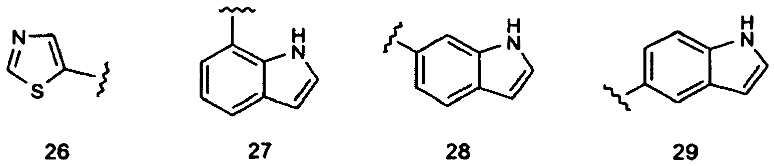 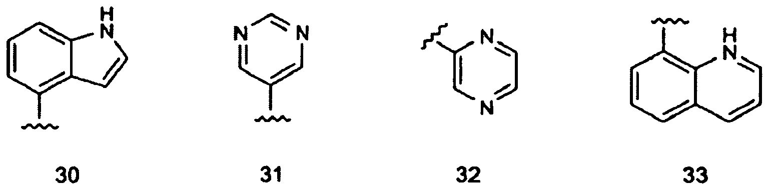 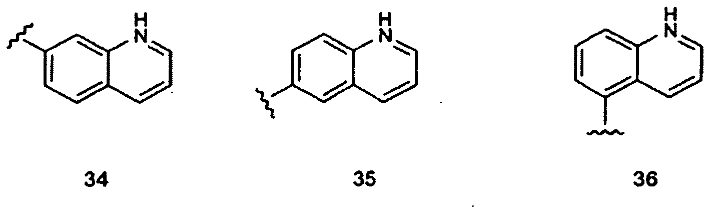 qui peut à chaque fois être relié à la position marquée par une ligne ondulée avec l'atome de carbone de la triple liaison et qui est non substitué ou éventuellement substitué avec 1, 2 ou 3 substituants choisis indépendamment les uns des autres dans le groupe constitué par F, Cl, Br, -CN, CF₃, -SF₅, -S-CH₃, -S-C₂H₅, méthyle, éthyle, n-propyle, iso-propyle, n-butyle, tert-butyle, éthényle, propényle, -O-CH₃, -O-C₂H₅, -NH₂, -N(CH₃)₂, -NH-CH₃, -CH₂-NH₂, -O-CF₃, -C(=O)-H, -C(=O)-O-CH₃, -C(=O)-O-C₂H₅, phényle, pyrrolyle, (1,3)-dioxolanyle, - Si (phényl)₂[C(CH₃)₃], -NO₂, NH-S(=O)₂-CH₃, -NH-S(=O)₂-C₂H₅, -S (=O)₂-NH₂, -S(=O)₂-NH-CH₃, -CH₂-OH, -C (=O) -CH₃, -C (=O)-OH, -CH₂-O-CH₃, -C(=O)-NH₂, -C(=O)-NH-CH₃, -NH-C(=O)-CH₃, -S(=O)₂-N(CH₃)₂, -NH-S(=O)₂-OH et -NH-C(=NH)-NH₂ ;
à chaque fois sous la forme d'un de leurs stéréoisomères purs, notamment énantiomères ou diastéréomères, de leurs racémats ou sous la forme d'un mélange de stéréoisomères, notamment les énantiomères et/ou diastéréomères, en un rapport de mélange quelconque, ou à chaque fois sous la forme des sels correspondants, ou à chaque fois sous la forme des solvates correspondants.

15. Composés selon une ou plusieurs des revendications 1 à 14, **caractérisés en ce que**
A¹ représente un groupe C-R^{1a},
A² représente un groupe C-R^{1b},
A³ représente un groupe C-R^{1c} et
A⁴ représente un groupe C-R^{1d},
ou
A¹ représente un atome d'azote,
A² représente un groupe C-R^{1b},
A³ représente un groupe C-R^{1c} et
A⁴ représente un groupe C-R^{1d},
ou
A¹ représente un groupe C-R^{1a},
A² représente un atome d'azote,
A³ représente un groupe C-R^{1c} et
A⁴ représente un groupe C-R^{1d} ;
R^{1a}, R^{1b}, R^{1c}, R^{1d} représentent chacun indépendamment les uns des autres un radical hydrogène ; -OR¹⁶ ; -F ; -Cl, - Br ; -CN ; -S(=O)₂-NH₂ ; -CF₃ ; -C(=O)-OR¹² ; un radical alkyle choisi dans le groupe constitué par méthyle, éthyle, n-propyle, iso-propyle, n-butyle, sec-butyle, iso-butyle et tert-butyle ; un radical phényle ; un radical benzyle ; un radical phénétyle ou un radical (3-phényl)-prop-1-yle,
ou R^{1a} et R^{1b} forment éventuellement ensemble avec le pont C-C qui les relie un radical phényle annelé non substitué ;
ou R^{1c} et R^{1d} forment éventuellement ensemble avec le pont C-C qui les relie un radical phényle annelé non substitué ;
R² et R³ représentent chacun indépendamment l'un de l'autre un radical hydrogène ; -C(=O)-R²⁰ ; -(CH₂)_{q}-C(=O)-R²¹ avec q = 1 ; -(CH₂)ᵣC(=O)-O-R²³ avec r = 1 ; un radical alkyle choisi dans le groupe constitué par méthyle ; éthyle ; n-propyle ; iso-propyle ; n-butyle ; tert-butyle ; sec-butyle ; iso-butyle ; n-pentyle ; n-hexyle ; n-heptyle ; n-octyle ; (1,1,3,3)-tétraméthylbutyle ; (1,1)-diméthyl-pentyle et (1,1)-diméthylbutyle ; un radical cyclopentyle ou cyclohexyle non substitué, qui peut à chaque fois être relié par un groupe -(CH₂)-, -(CH₂)₂- ou -(CH₂)₃- ; ou un radical phényle, pyridinyle, thiophényle ou furanyle, qui est à chaque fois non substitué ou éventuellement substitué avec 1, 2, 3, 4 ou 5 substituants choisis indépendamment les uns des autres dans le groupe constitué par F, Cl, Br, -CN, -CF₃, méthyle, éthyle, n-propyle, iso-propyle, tert-butyle, -O-CH₃ et -O-C₂H₅ et éventuellement relié par un groupe -(CH₂)-, -(CH₂)₂-, -(CH(CH₃))- ou -(CH₂)₃-, ou R² et R³ forment ensemble avec l'atome d'azote qui les relie en tant qu'élément de cycle un radical hétérocycloaliphatique choisi dans le groupe constitué par pyrrolidinyle, pipéridinyle, pipérazinyle et morpholinyle,
R¹² représente un radical hydrogène ou un radical alkyle choisi dans le groupe constitué par méthyle, éthyle, n-propyle, iso-propyle et tert-butyle ;
R¹⁶, R²⁰, R²¹ et R²³ représentent chacun indépendamment les uns des autres un radical alkyle choisi dans le groupe constitué par méthyle, éthyle, n-propyle, iso-propyle et tert-butyle, ou un radical phényle, qui est à chaque fois non substitué ou substitué avec 1 ou 2 substituants choisis indépendamment les uns des autres dans le groupe constitué par F, Cl, Br, méthyle, éthyle, -O-CH₃ et -O-C₂H₅ et éventuellement relié par un groupe -(CH₂)-,
M¹ représente un radical choisi dans le groupe constitué par les radicaux 1 à 6, 21, 22, 36 et 37 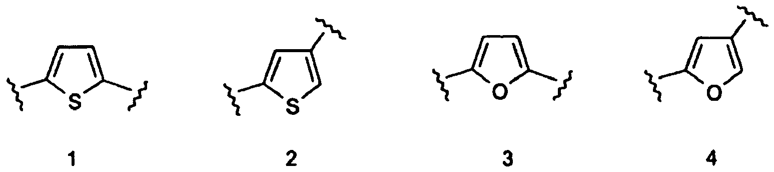 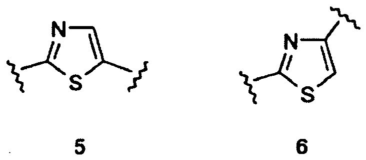 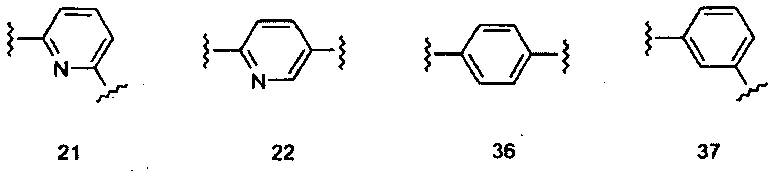 qui peut à chaque fois être relié dans un sens quelconque aux positions marquées par une ligne ondulée avec le bicycle et l'atome de carbone de la triple liaison et qui est éventuellement substitué avec 1 ou 2 substituants supplémentaires choisis indépendamment les uns des autres dans le groupe constitué par F, Cl, Br, -CN, -CH₂-CN, - CF₃, -SF₅, méthyle, éthyle, n-propyle, iso-propyle, tert-butyle, -O-CH₃ et -O-CF₃,
et
M² représente un radical choisi dans le groupe constitué par phényle, 2-pyridinyle, 3-pyridinyle, 4-pyridinyle, 2-thiazolyle, 4-thiazolyle, 5-thiazolyle, 4-indolyle, 5-indolyle, 6-indolyle, 7-indolyle, 2-thiophényle, 3-thiophényle, 2-imidazolyle, 4-imidazolyle, 5-imidazolyle, 2-pyrimidinyle, 4-pyrimidinyle, 5-pyrimidinyle, 2-pyrazinyle, 5-quinolinyle, 6-quinolinyle, 7-quinolinyle et 8-quinolinyle, chaque radical étant non substitué ou éventuellement substitué avec 1, 2, 3, 4 ou 5 substituants choisis indépendamment les uns des autres dans le groupe constitué par F, Cl, Br, -CN, -CF₃, -SF₅, -S-CH₃, -S-C₂H₅, méthyle, éthyle, iso-propyle, tert-butyle, éthényle, propényle, -OH, -O-CH₃, - O-C₂H₅, -NH₂, -N(CH₃)₂, -NH-CH₃, -CH₂-NH₂, -NO₂, -O-CF₃, - C(=O)-H ; -C(=O)-OCH₃, -C(=O)-O-C₂H₅, phényle, pyrrolyle, (1,3)-dioxolanyle, -Si(phényl)₂[C(CH₃)₃], NH-S(=O)₂-CH₃, - NH-S(=O)₂-C₂H₅, -S(=O)₂-NH₂, -S(=O)₂-NH-CH₃, -CH₂-OH, - C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH et - S(=O)₂-N(CH₃)₂ ;
à chaque fois sous la forme d'un de leurs stéréoisomères purs, notamment énantiomères ou diastéréomères, de leurs racémats ou sous la forme d'un mélange de stéréoisomères, notamment les énantiomères et/ou diastéréomères, en un rapport de mélange quelconque, ou à chaque fois sous la forme des sels correspondants, ou à chaque fois sous la forme des solvates correspondants.

16. Composés de formule générale Id selon la revendication 15 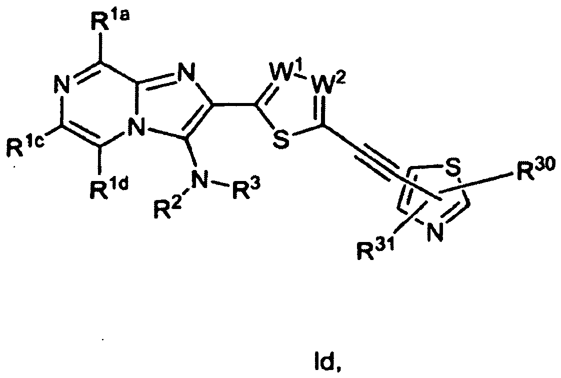 dans laquelle
R^{1a}, R^{1c}, R^{1d}, R² et R³ ont chacun la signification selon la revendication 15 ;
W¹ représente C et W² représente C
ou W¹ représente C et W² représente N
ou W¹ représente N et W² représente C ;
et
R³⁰ et R³¹ représentent chacun indépendamment l'un de l'autre un radical choisi dans le groupe constitué par H, F, Cl, Br, -CN, -CF₃, -SF₅, -S-CH₃, -S-C₂H₅, méthyle, éthyle, iso-propyle, tert-butyle, éthényle, propényle, - OH, -O-CH₃, -O-C₂H₅, -NH₂, -N(CH₃)₂, -NH-CH₃, -CH₂-NH₂,-NO₂, -O-CF₃, -C(=O)-H ; -C(=O)-OCH₃, -C (=O) -O-C₂H₅, phényle, pyrrolyle, (1,3)-dioxolanyle, - Si(phényl)₂[C(CH₃)₃], NH-S(=O)₂-CH₃, -NH-S (=O)₂-C₂H₅, - S=(O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH et -S(=O)₂-N(CH₃)₂ ;
à chaque fois éventuellement sous la forme des sels correspondants ou à chaque fois éventuellement sous la forme des solvates correspondants.

17. Composés de formule générale le selon la revendication 15 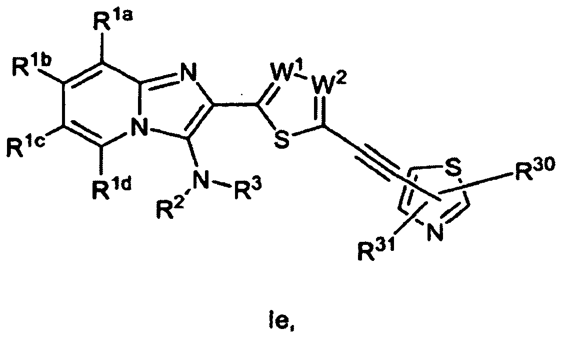 dans laquelle
R^{1a}, R^{1b}, R^{1c}, R^{1d}, R² et R³ ont chacun la signification selon la revendication 15 ;
W¹ représente C et W² représente C
ou W¹ représente C et W² représente N
ou W¹ représente N et W² représente C ;
et
R³⁰ et R³¹ représentent chacun indépendamment l'un de l'autre un radical choisi dans le groupe constitué par H, F, Cl, Br, -CN, -CF₃, -SF₅, -S-CH₃, -S-C₂H₅, méthyle, éthyle, iso-propyle, tert-butyle, éthényle, propényle, - OH, -O-CH₃, -O-C₂H₅, -NH₂, -N(CH₃)₂, -NH-CH₃, -CH₂-NH₂, - NO₂, -O-CF₃, -C(=O)-H ; -C(=O)-OCH₃, -C(=O)-O-C₂H₅, phényle, pyrrolyle, (1,3)-dioxolanyle, - Si (phényl)₂[C(CH₃)₃], NH-S(=O)₂-CH₃, -NH-S(=O)₂-C₂H₅, - S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, -NH-C(=O)-CH₃, -NH-C(=NH)-NH₂, -NH-S(=O)₂-OH et -S(=O)₂-N(CH₃)₂ ;
à chaque fois éventuellement sous la forme des sels correspondants ou à chaque fois éventuellement sous la forme des solvates correspondants.

18. Composés de formule générale If selon la revendication 15 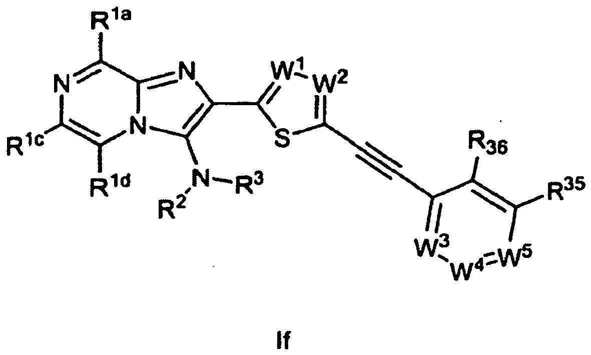 dans laquelle
R^{1a}, R^{1c}, R^{1d}, R² et R³ ont chacun la signification selon la revendication 15 ;
W¹ représente C et W² représente C
ou W¹ représente C et W² représente N
ou W¹ représente N et W² représente C ;
W³ représente C-R³² ; W⁴ représente C-R³³ et W⁵ représente C-R³⁴ ;
ou un des radicaux W³, W⁴ et W⁵ représente N et les deux autres radicaux choisis dans le groupe constitué par W³, W⁴ et W⁵ représentent C-R³² ou C-R³³ ;
et R³², R³³, R³⁴, R³⁵ et R³⁶ représentent chacun indépendamment les uns des autres un radical choisi dans le groupe constitué par H, F, Cl, Br, -CN, -CF₃, -SF₅, - S-CH₃, -S-C₂H₅, méthyle, éthyle, iso-propyle, tert-butyle, éthényle, propényle, -OH, -O-CH₃, -O-C₂H₅, -NH₂, -N(CH₃)₂, -NH-CH₃, -CH₂-NH₂, -NO₂, -O-CF₃, -C(=O)-H ; - C(=O)-OCH₃, -C(=O)-O-C₂H₅, phényle, pyrrolyle, (1,3)-dioxolanyle, -Si (phényl)₂[C(CH₃)₃] NH-S(=O)₂-CH₃, -NH-S(=O)2-C₂H₅, -S(=O)₂-NH₂, -S (=O)₂-NH-CH₃, -CH₂-OH, -C (=O)-CH₃, -C(=O)-OH, -CH₂-O-CH₃, -C(=O)-NH₂, -C(=O)-NH-CH₃, - NH-C(=O)-CH₃, -NH-C (=NH) -NH₂, -NH-S(=O)₂-OH et -S(=O)₂-N(CH₃)₂ ;
à chaque fois éventuellement sous la forme des sels correspondants ou à chaque fois éventuellement sous la forme des solvates correspondants.

19. Composés de formule générale Ig selon la revendication 15 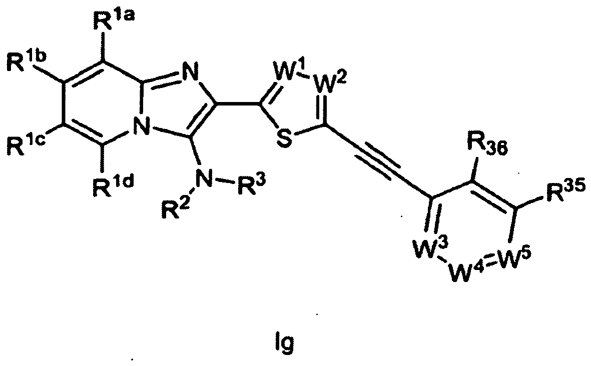 dans laquelle
R^{1a}, R^{1b}, R^{1c}, R^{1d}, R² et R³ ont chacun la signification selon la revendication 15 ;
W¹ représente C et W² représente C
ou W¹ représente C et W² représente N
ou W¹ représente N et W² représente C ;
W³ représente C-R³² ; W⁴ représente C-R³³ et W⁵ représente C-R³⁴ ;
ou un des radicaux W³, W⁴ et W⁵ représente N et les deux autres radicaux choisis dans le groupe constitué par W³, W⁴ et W⁵ représentent C-R³² ou C-R³³ ;
et R³², R³³, R³⁴, R³⁵ et R³⁶ représentent chacun indépendamment les uns des autres un radical choisi dans le groupe constitué par H, F, Cl, Br, -CN, -CF₃, -SF₅, - S-CH₃, -S-C₂H₅, méthyle, éthyle, iso-propyle, tert-butyle, éthényle, propényle, -OH, -O-CH₃, -O-C₂H₅, -NH₂, -N(CH₃)₂, -NH-CH₃, -CH₂-NH₂, -NO₂, -O-CHF₃, -C(=O)-H ; - C(=O)-OCH₃, -C(=O)-O-C₂H₅, phényle, pyrrolyle, (1,3)-dioxolanyle, -Si(phényl)₂[C(CH₃)₃], NH-S(=O)₂-CH₃, -NH-S(=O)₂-C₂H₅, -S(=O)₂-NH₂, -S(=O)₂-NH-CH₃, -CH₂-OH, -C(=O)-CH₃, -C(=O)-OH, -CH₂-O-CH₃, -C(=O)-NH₂, -C(=O)-NH-CH₃, - NH-C(=O)-CH₃, -NH-C (=NH) -NH₂, -NH-S (=O)₂-OH et -S (=O) ₂-N(CH3)₂ ;
à chaque fois éventuellement sous la forme des sels correspondants ou à chaque fois éventuellement sous la forme des solvates correspondants.

20. Composés de formule générale Ih selon la revendication 15 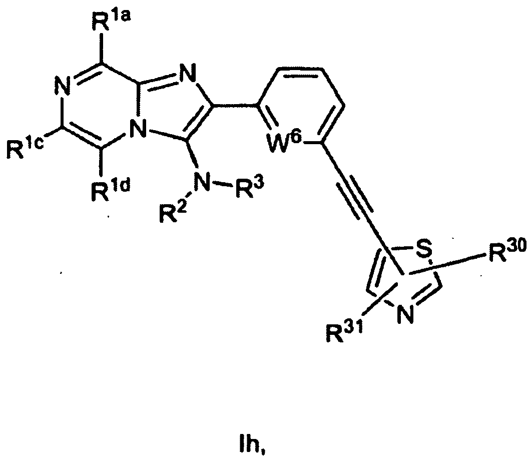 dans laquelle
R^{1a}, R^{1c}, R^{1a}, R² et R³ ont chacun la signification selon la revendication 15 ;
W⁶ représente C ou N ;
et
R³⁰ et R³¹ représentent chacun indépendamment l'un de l'autre un radical choisi dans le groupe constitué par H, F, Cl, Br, -CN, -CF₃, -SF₅, -S-CH₃, -S-C₂H₅, méthyle, éthyle, iso-propyle, tert-butyle, éthényle, propényle, - OH, -O-CH₃, -O-C₂H₅, -NH₂, -N(CH₃)₂, -NH-CH₃, -CH₂-NH₂, - NO₂, -O-CF₃, -C(=O)-H; -C(=O)-OCH₃, -C(=O)-O-C₂H₅, phényle, pyrrolyle, (1,3)-dioxolanyle, - Si(phényl)₂[C(CH₃)₃], NH-S(=O)₂-CH₃, -NH-S(=O)₂-C₂H₅, - S(=O)₂-NH₂, -S(=O)₂-NH-CH₃, -CH₂-OH, -C(=O)-CH₃, -C(=O) - OH, -CH₂-O-CH₃, -C(=O)-NH₂, -C(=O)-NH-CH₃, -NH-C(=O)-CH₃, -NH-C(=NH)-NH₂, -NH-S(=O)₂-OH et -S(=O)₂-N(CH₃)₂ ;
à chaque fois éventuellement sous la forme des sels correspondants ou à chaque fois éventuellement sous la forme des solvates correspondants.

21. Composés de formule générale Ik selon la revendication 15 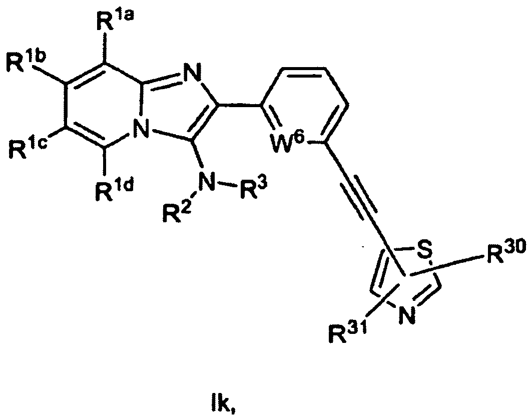 dans laquelle
R^{1a}, R^{1b}, R^{1c}, R^{1d}, R² et R³ ont chacun la signification selon la revendication 15 ;
W⁶ représente C ou N ;
et
R³⁰ et R³¹ représentent chacun indépendamment l'un de l'autre un radical choisi dans le groupe constitué par H, F, Cl, Br, -CN, -CF₃, -SF₅, -S-CH₃, -S-C₂H₅, méthyle, éthyle, iso-propyle, tert-butyle, éthényle, propényle, - OH, -O-CH₃, -O-C₂H₅, -NH₂, -N(CH₃)₂, -NH-CH₃, -CH₂-NH₂, - NO₂, -O-CF₃, -C(=O)-H; -C(=O)-OCH₃, -C(=O)-O-C₂H₅, phényle, pyrrolyle, (1,3)-dioxolanyle, - Si (phényl)₂[C(CH₃) ], NH-S(=O)2-CH₃, -NH-S(=O)₂-C₂H₅, - S(=O)₂-NH₂, -S(=O)₂-NH-CH₃, -CH₂-OH, -C(=O)-CH₃, -C(=O) - OH, -CH₂-O-CH₃, -C(=O)-NH2, -C(=O)-NH-CH₃, -NH-C (=O) -CH₃, -NH-C(=NH)-NH₂, -NH-S(=O)₂-OH et -S(=O)₂-N(CH₃)₂;
à chaque fois éventuellement sous la forme des sels correspondants ou à chaque fois éventuellement sous la forme des solvates correspondants.

22. Composés de formule générale Im selon la revendication 15 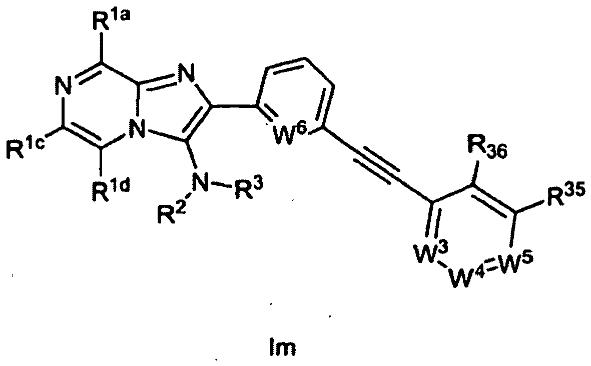 dans laquelle
R^{1a}, R^{1c}, R^{1d}, R² et R³ ont chacun la signification selon la revendication 15 ;
W⁶ représente C ou N ;
W³ représente C-R³² ; W⁴ représente C-R³³ et W⁵ représente C-R³⁴ ;
ou un des radicaux W³, W⁴ et W⁵ représente N et les deux autres radicaux choisis dans le groupe constitué par W³, W⁴ et W⁵ représentent C-R³² ou C-R³³ ;
et R³², R³³, R³⁴, R³⁵ et R³⁶ représentent chacun indépendamment l'un de l'autre un radical choisi dans le groupe constitué par H, F, Cl, Br, -CN, -CF₃, -SF₅, -S-CH₃, -S-C₂H₅, méthyle, éthyle, iso-propyle, tert-butyle, éthényle, propényle, -OH, -O-CH₃, -O-C₂H₅, -NH₂, -N(CH₃)₂, -NH-CH₃, -CH₂-NH₂, -NO₂, -O-CF₃, -C(=O)-H ; -C(=O)-OCH₃, - C(=O)-O-C₂H₅, phényle, pyrrolyle, (1,3)-dioxolanyle, - Si(phényl)₂[C(CH₃)₃], NH-S(=O)2-CH₃, -NH-S (=O)₂-C₂H₅, - S(=O)₂-NH₂, -S(=O)₂-NH-CH₃, -CH₂-OH, -C(=O)-CH₃, -C(=O) - OH, -CH₂-O-CH₃, -C(=O)-NH₂, -C(=O)-NH-CH₃, -NH-C(=O)-CH₃, -NH-C(=NH)-NH₂, -NH-S(=O)₂-OH et -S(=O)₂-N(CH₃)₂ ;
à chaque fois éventuellement sous la forme des sels correspondants ou à chaque fois éventuellement sous la forme des solvates correspondants.

23. Composés de formule générale In selon la revendication 15 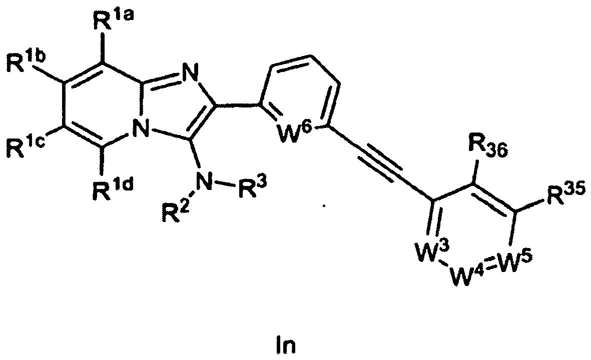 dans laquelle
R^{1a}, R^{1b}, R^{1c}, R^{1d}, R² et R³ ont chacun la signification selon la revendication 15 ;
W⁶ représente C ou N ;
W³ représente C-R³² ; W⁴ représente C-R³³ et W⁵ représente C-R³⁴ ;
ou un des radicaux W³, W⁴ et W⁵ représente N et les deux autres radicaux choisis dans le groupe constitué par W³, W⁴ et W⁵ représentent C-R³² ou C-R³³ ;
et R³², R³³, R³⁴, R³⁵ et R³⁶ représentent chacun indépendamment l'un de l'autre un radical choisi dans le groupe constitué par H, F, Cl, Br, -CN, -CF₃, -SF₅, -S-CH₃, -S-C₂H₅, méthyle, éthyle, iso-propyle, tert-butyle, éthényle, propényle, -OH, -O-CH₃, -O-C₂H₅, -NH₂, -N(CH₃)₂, -NH-CH₃, -CH₂-NH₂, -NO₂, -O-CF₃, -C(=O)-H ; -C(=O)-OCH₃, - C(=O)-O-C₂H₅, phényle, pyrrolyle, (1,3)-dioxolanyle, - Si(phényl)₂[C(CH₃)₃], NH-S (=O) ₂-CH₃, -NH-S (=O)₂-C₂H₅, - S(=O)₂-NH₂, -S(=O)₂-NH-CH₃, -CH₂-OH, -C(=O)-CH₃, -C(=O) - OH, -CH₂-O-CH₃, -C(=O)-NH₂, -C(=O)-NH-CH₃, -NH-C(=O)-CH₃, -NH-C(=NH)-NH₂, -NH-S(=O)₂-OH et -S(=O)₂-N(CH₃)₂ ;
à chaque fois éventuellement sous la forme des sels correspondants ou à chaque fois éventuellement sous la forme des solvates correspondants.

24. Composés selon une ou plusieurs des revendications 1 à 23, choisis dans le groupe constitué par
[1] cyclopentyl-[2-(5-phényléthynyl-thiophén-2-yl)-imidazo[1,2-a]pyrazin-3-yl]-amine,
[2] cyclohexylméthyl-[5-méthyl-2-(5-phényléthynyl-thiophén-2-yl)-imidazo[1,2-a]pyridin-3-yl]-amine,
[3] cyclohexylméthyl-[6-méthyl-2-(5-phényléthynyl-thiophén-2-yl)-imidazo[1,2-a]pyridin-3-yl]-amine,
[4] cyclohexylméthyl-[7-méthyl-2-(5-phényléthynyl-thiophén-2-yl)-imidazo[1,2-a]pyridin-3-yl]-amine,
[5] cyclohexylméthyl-[8-méthyl-2-(5-phényléthynyl-thiophén-2-yl)-imidazo[1,2-a]pyridin-3-yl]-amine,
[6] cyclohexylméthyl-[5,7-diméthyl-2-(5-phényléthynyl-thiophén-2-yl)-imidazo[1,2-a]pyridin-3-yl]-amine,
[7] [8-benzyloxy-2-(5-phényléthynyl-thiophén-2-yl)-imidazo[1,2-a]pyridin-3-yl]-cyclohexylméthyl-amine,
[8] cyclohexylméthyl-[2-(5-phényléthynyl-thiophén-2-yl)-imidazo[1,2-a]pyrazin-3-yl]-amine,
[9] cyclohexylméthyl-[2-(5-phényléthynyl-thiophén-2-yl)-imidazo[1,2-a]pyrimidin-3-yl]-amine,
[10] (4-méthoxy-benzyl)-[5-méthyl-2-(5-phényléthynyl-thiophén-2-yl)-imidazo[1,2-a]pyridin-3-yl]-amine,
[11] (4-méthoxy-benzyl)-[6-méthyl-2-(5-phényléthynyl-thiophén-2-yl)-imidazo[1,2-a]pyridin-3-yl]-amine,
[12] (4-méthoxy-benzyl)-[7-méthyl-2-(5-phényléthynyl-thiophén-2-yl)-imidazo[1,2-a]pyridin-3-yl]-amine,
[13] (4-méthoxy-benzyl)-[8-méthyl-2-(5-phényléthynyl-thiophén-2-yl)-imidazo[1,2-a]pyridin-3-yl]-amine,
[14] [5,7-diméthyl-2-(5-phényléthynyl-thiophén-2-yl)-imidazo[1,2-a]pyridin-3-yl]-(4-méthoxy-benzyl)-amine,
[15] [8-benzyloxy-2-(5-phényléthynyl-thiophén-2-yl)-imidazo[1,2-a]pyridin-3-yl]-(4-méthoxy-benzyl)-amine,
[16] (4-méthoxy-benzyl)-[2-(5-phényléthynyl-thiophén-2-yl)-imidazo[1,2-a]pyridin-3-yl]-amine,
[17] (4-méthoxy-benzyl)-[2-(5-phényléthynyl-thiophén-2-yl)-imidazo[1,2-a]pyrazin-3-yl]-amine,
[18] tert-butyl-[2-(5-phényléthynyl-thiophén-2-yl)-imidazo[1,2-a]pyrazin-3-yl]-amine,
[19] tert-butyl-[6-méthyl-2-(5-phényléthynyl-thiophén-2-yl)-imidazo[1,2-a]pyridin-3-yl]-amine,
[20] [8-benzyloxy-2-(5-phényléthynyl-thiophén-2-yl)-imidazo[1,2-a]pyridin-3-yl]-(3-méthoxy-phényl)-amine,
[21] (3-méthoxy-benzyl)-[5-méthyl-2-(5-phényléthynyl-thiophén-2-yl)-imidazo[1,2-a]pyridin-3-yl]-amine,
[22] (3-méthoxy-benzyl)-[8-méthyl-2-(5-phényléthynyl-thiophén-2-yl)-imidazo[1,2-a]pyridin-3-yl]-amine,
[23] [5,7-diméthyl-2-(5-phényléthynyl-thiophén-2-yl)-imidazo[1,2-a]pyridin-3-yl]-(3-méthoxy-benzyl)-amine,
[24] [8-benzyloxy-2-(5-phényléthynyl-thiophén-2-yl)-imidazo[1,2-a]pyridin-3-yl]-(3-méthoxy-benzyl)-amine,
[25] (3-méthoxy-benzyl)-[2-(5-phényléthynyl-thiophén-2-yl)-imidazo[1,2-a]pyridin-3-yl]-amine,
[26] (3-méthoxy-benzyl)-[2-(5-phényléthynyl-thiophén-2-yl)-imidazo[1,2-a]pyrazin-3-yl]-amine,
[27] [6-méthyl-2-(5-phényléthynyl-thiophén-2-yl)-imidazo[1,2-a]pyridin-3-yl]-(1-phényl-éthyl)-amine,
[28] [7-méthyl-2-(5-phényléthynyl-thiophén-2-yl)-imidazo[1,2-a]pyridin-3-yl]-(1-phényl-éthyl)-amine,
[29] [5,7-diméthyl-2-(5-phényléthynyl-thiophén-2-yl)-imidazo[1,2-a]pyridin-3-yl]-(1-phényl-éthyl)-amine,
[30] [8-benzyloxy-2-(5-phényléthynyl-thiophén-2-yl)-imidazo[1,2-a]pyridin-3-yl]-(1-phényl-éthyl)-amine,
[31] (1-phényl-éthyl)-[2-(5-phényléthynyl-thiophén-2-yl)-imidazo[1,2-a]pyrazin-3-yl]-amine,
[32] (2-chloro-benzyl)-[5,7-diméthyl-2-(5-phényléthynyl-thiophén-2-yl)-imidazo[1,2-a]pyridin-3-yl]-amine,
[33] (3-chloro-4-fluoro-phényl)-[7-phényl-2-(5-phényléthynyl-thiophén-2-yl)-imidazo[1,2-a]pyridin-3-yl]-amine,
[34] (4-méthoxy-benzyl)-[7-méthyl-2-(5-phényléthynyl-thiophén-2-yl)-imidazo[1,2-a]pyrimidin-3-yl]-amine,
[35] [8-méthyl-2-(5-phényléthynyl-thiophén-2-yl)-imidazo[1,2-a]pyridin-3-yl]-(1-phényl-éthyl)-amine,
[36] [7-méthyl-2-(5-phényléthynyl-thiophén-2-yl)-imidazo[1,2-a]pyrimidin-3-yl]-(1-phényl-éthyl)-amine,
[37] (2-méthoxy-benzyl)-[7-méthyl-2-(5-phényléthynyl-thiophén-2-yl)-imidazo[1,2-a]pyridin-3-yl]-amine,
[38] (2-méthoxy-benzyl)-[2-(5-phényléthynyl-thiophén-2-yl)-imidazo[1,2-a]pyridin-3-yl]-amine,
[39] (2-chloro-benzyl)-[2-(5-phényléthynyl-thiophén-2-yl)-imidazo[1,2-a]pyridin-3-yl]-amine,
[40] (2-méthoxy-benzyl)-[7-méthyl-2-(5-phényléthynyl-thiophén-2-yl)-imidazo[1,2-a]pyrimidin-3-yl]-amine,
[41] (3-méthoxy-phényl)-[6-méthyl-2-(5-phényléthynyl-thiophén-2-yl)-imidazo[1,2-a]pyridin-3-yl]-amine,
[42] [5,7-diméthyl-2-(5-phényléthynyl-thiophén-2-yl)-imidazo[1,2-a]pyridin-3-yl]-(3-méthoxy-phényl)-amine,
[43] (3-méthoxy-phényl)-[2-(5-phényléthynyl-thiophén-2-yl)-imidazo[1,2-a]pyrazin-3-yl]-amine,
[44] [7-éthyl-2-(5-phényléthynyl-thiophén-2-yl)-imidazo[1,2-a]pyridin-3-yl]-(2-méthoxy-benzyl)-amine,
[45] (2-chloro-benzyl)-[7-éthyl-2-(5-phényléthynyl-thiophén-2-yl)-imidazo[1,2-a]pyridin-3-yl]-amine,
[46] [7-tert-butyl-2-(5-phényléthynyl-thiophén-2-yl)-imidazo[1,2-a]pyridin-3-yl]-(3-chloro-4-fluor-phényl)-amine,
[47] (3-méthoxy-benzyl)-[7-méthyl-2-(5-phényléthynyl-thiophén-2-yl)-imidazo[1,2-a]pyrimidin-3-yl]-amine,
[48] [7-éthyl-2-(5-phényléthynyl-thiophén-2-yl)-imidazo[1,2-a]pyridin-3-yl]-(2-fluoro-phényl)-amine,
[49] [7-tert-butyl-2-(5-phényléthynyl-thiophén-2-yl)-imidazo[1,2-a]pyridin-3-yl]-(2-fluoro-phényl)-amine,
[50] (2,4-difluoro-phényl)-[2-(5-phényléthynyl-thiophén-2-yl)-7-(3-phényl-propyl)-imidazo[1,2-a]pyridin-3-yl]-amine,
[51] (4-fluoro-benzyl)-[7-méthyl-2-(5-phényléthynyl-thiophén-2-yl)-imidazo[1,2-a]pyridin-3-yl]-amine,
[52] [5,7-diméthyl-2-(5-phényléthynyl-thiophén-2-yl)-imidazo[1,2-a]pyridin-3-yl]-(4-fluoro-benzyl)-amine,
[53] [7-éthyl-2-(5-phényléthynyl-thiophén-2-yl)-imidazo[1,2-a]pyridin-3-yl]-(3-trifluorométhyl-benzyl)-amine,
[54] [7-isopropyl-2-(5-phényléthynyl-thiophén-2-yl)-imidazo[1,2-a]pyridin-3-yl]-(3-trifluorométhyl-benzyl)-amine,
[55] tert-butyl-[2-(4-phényléthynyl-thiophén-2-yl)-imidazo[1,2-a]pyrazin-3-yl]-amine,
[56] [2-(5-phényléthynyl-thiophén-2-yl)-imidazo[1,2-a]pyrazin-3-yl]-(1,1,3,3-tétraméthyl-butyl)-amine,
[57] butyl-[2-(4-phényléthynyl-thiophén-2-yl)-imidazo[1,2-a]pyrazin-3-yl]-amine,
[59] [2-(5-phényléthynyl-thiophén-2-yl)-imidazo[1,2-a]pyridin-3-yl]-(1,1,3,3-tétraméthyl-butyl)-amine,
[60] [2-(5-pyridinyl-thiophén-2-yl)-imidazo[1,2-a]pyrazin-3-yl]-(1,1,3,3-tétraméthyl-butyl)-amine,
[61] [2-(5-pyridin-4-yléthynyl-thiophén-2-yl)-imidazo[1,2-a]pyrazin-3-yl]-(1,1,3,3-tétraméthyl-butyl)-amine,
[62] [6-chloro-2-(5-phényléthynyl-thiophén-2-yl)-imidazo[1,2-a]pyridin-3-yl]-(1,1,3,3-tétraméthyl-butyl)-amine,
[63] [6,8-dichloro-2-(5-phényléthynyl-thiophén-2-yl)-imidazo[1,2-a]pyridin-3-yl]-(1,1,3,3-tétraméthyl-butyl)-amine,
[64] [2-(5-pyridin-2-yléthynyl-thiophén-2-yl)-imidazo[1,2-a]pyrazin-3-yl]-(1,1,3,3-tétraméthyl-butyl)-amine,
[65] diméthyl-[2-(5-phényléthynyl-thiophén-2-yl)-imidazo[1,2-a]pyrazin-3-yl]-amine,
[66] méthyl-[2-(5-phényléthynyl-thiophén-2-yl)-imidazo[1,2-a]pyrazin-3-yl]-amine,
[67] N-[2-(5-phényléthynyl-thiophén-2-yl)-imidazo[1,2-a]pyrazin-3-yl]-acétamide,
[68] éthyl-[2-(5-phényléthynyl-thiophén-2-yl)-imidazo[1,2-a]pyrazin-3-yl]-amine,
[69] propyl-[2-(5-phényléthynyl-thiophén-2-yl)-imidazo[1,2-a]pyrazin-3-yl]-amine,
[70] butyl-[2-(5-phényléthynyl-thiophén-2-yl)-imidazo[1,2-a]pyrazin-3-yl]-amine,
[71] (2-méthylpropyl)-[2-(5-phényléthynyl-thiophén-2-yl)-imidazo[1,2-a]pyrazin-3-yl]-amine,
[72] pentyl-[2-(5-phényléthynyl-thiophén-2-yl)-imidazo[1,2-a]pyrazin-3-yl]-amine,
[73] ester méthylique de l'acide {(méthoxycarbonylméthyl)-[2-(5-phényléthynyl-thiophén-2-yl)-imidazo[1,2-a]pyrazin-3-yl]-amino}-acétique,
[74] benzyl-[2-(5-phényléthynyl-thiophén-2-yl)-imidazo[1,2-a]pyrazin-3-yl]-amine,
[75] ester méthylique de l'acide [2-(5-phényléthynyl-thiophén-2-yl)-imidazo[1,2-a]pyrazin-3-ylamino]-acétique,
[76] tert-butyl-[2-(5-pyridin-4-yléthynyl-thiophén-2-yl)-imidazo[1,2-a]pyrazin-3-yl]-amine,
[77] tert-butyl-[2-(5-pyridin-2-yléthynyl-thiophén-2-yl)-imidazo[1,2-a]pyrazin-3-yl]-amine,
[78] N-[2-(5-phényléthynyl-thiophén-2-yl)-imidazo[1,2-a]pyrazin-3-yl]-benzamide,
[79] [2-(5-phényléthynyl-thiophén-2-yl)-imidazo[1,2-a]pyrazin-3-yl]-bis-pyridin-3-ylméthyl-amine,
[80] 2,2-diméthyl-N-[2-(5-phényléthynyl-thiophén-2-yl)-imidazo[1,2-a]pyrazin-3-yl]-propionamide,
[81] 3-méthoxy-N-[2-(5-phényléthynyl-thiophén-2-yl)-imidazo[1,2-a]pyrazin-3-yl]-benzamide,
[82] tert-butyl-[2-(5-pyridin-3-yléthynyl-thiophén-2-yl)-imidazo[1,2-a]pyrazin-3-yl]-amine
[83] 2-(5-phényléthynyl-thiophén-2-yl)-imidazo[1,2-a]pyrazin-3-ylamine
[84] méthyl-2-(5-(phényléthynyl)thiophén-2-yl)-3-(2,4,4-triméthylpentan-2-ylamino)imidazo[1,2-a]pyrazine-8-carboxylate
[85] 2-(5-(phényléthynyl)thiophén-2-yl)-N-(2,4,4-triméthylpentan-2-yl)imidazo[1,2-a]pyrimidine-3-amine
[86] 2-(5-((6-méthylpyridin-2-yl)éthynyl)thiophén-2-yl)-N-(2,4,4-triméthylpentan-2-yl)imidazo[1,2-a]pyrazine-3-amine
[87] N-cyclohexyl-2-(5-(phényléthynyl)thiophén-2-yl)imidazo[1,2-a]pyrazine-3-amine
[88] 2-(5-(phényléthynyl)thiophén-2-yl)-3-(pipéridin-1-yl)imidazo[1,2-a]pyrazine
[89] N-tert-butyl-N-méthyl-2-(5-(phényléthynyl)thiophén-2-yl)imidazo[1,2-a]pyrazine-3-amine
[90] méthyl-2-(5-(phényléthynyl)thiophén-2-yl)-3-(2,4,4-triméthylpentan-2-ylamino)imidazo[1,2-a]pyridine-6-carboxylate
[91] chlorhydrate de N-tert-butyl-2-(5-(pyridin-2-yléthynyl)thiophén-2-yl)imidazo[1,2-a]pyrazine-3-amine
[92] 8-bromo-N-cyclopentyl-6-méthyl-2-(5-(pyridin-2-yléthynyl)thiophén-2-yl)imidazo[1,2-a]pyridine-3-amine
[93] N,N-diéthyl-2-(5-(phényléthynyl)thiophén-2-yl)imidazo[1,2-a]pyrazine-3-amine
[94] N-tert-butyl-2-(5-(pyridin-2-yléthynyl)thiophén-2-yl)imidazo[1,2-a]pyridine-3-amine
[95] N-tert-butyl-2-(5-(phényléthynyl)furan-2-yl)imidazo[1,2-a]pyrazine-3-amine
[96] 8-bromo-N-tert-butyl-6-méthyl-2-(3-(pyridin-2-yléthynyl)phényl)imidazo[1,2-a]pyridine-3-amine
[97] N-tert-butyl-8-méthyl-2-(5-(phényléthynyl)thiophén-2-yl)imidazo[1,2-a]pyridine-3-amine
[98] chlorhydrate de N-méthyl-2-(5-(phényléthynyl)thiophén-2-yl)-N-(2,4,4-triméthylpentan-2-yl)imidazo[1,2-a]pyrazine-3-amine
[99] chlorhydrate de 2-(5-(phényléthynyl)thiophén-2-yl)-3-(pyrrolidin-1-yl)imidazo[1,2-a]pyrazine
[100] chlorhydrate de N-tert-butyl-2-(5-((4-fluorophényl)éthynyl)thiophén-2-yl)imidazo[1,2-a]pyrazine-3-amine
[101] N-tert-butyl-7-méthyl-2-(5-(phényléthynyl)thiophén-2-yl)imidazo[1,2-a]pyridine-3-amine
[102] N-tert-butyl-5-méthyl-2-(5-(phényléthynyl)thiophén-2-yl)imidazo[1,2-a]pyridine-3-amine
[103] 8-chloro-2-(3-(pyridin-2-yléthynyl)phényl)-6-(trifluorométhyl)-N-(2,4,4-triméthylpentan-2-yl)imidazo[1,2-a]pyridine-3-amine
[104] chlorhydrate de N-tert-butyl-2-(5-((3-fluorophényl)éthynyl)thiophén-2-yl)imidazo[1,2-a]pyrazine-3-amine
[105] chlorhydrate de N-tert-butyl-2-(5-((2-fluorophényl)éthynyl)thiophén-2-yl)imidazo[1,2-a]pyrazine-3-amine
[106] méthyl-3-(tert-butylamino)-2-(5-(phényléthynyl)thiophén-2-yl)imidazo[1,2-a]pyrazine-8-carboxylate
[107] N-tert-butyl-2-(5-(pyrazin-2-yléthynyl)thiophén-2-yl)imidazo[1,2-a]pyrazine-3-amine
[108] 2-(5-((4-aminophényl)éthynyl)thiophén-2-yl)-N-tert-butylimidazo[1,2-a]pyrazine-3-amine
[109] N-isopropyl-2-(5-(phényléthynyl)thiophén-2-yl)imidazo[1,2-a]pyrazine-3-amine
[110] chlorhydrate de N-tert-butyl-2-(5-(thiophén-2-yléthynyl)thiophén-2-yl)imidazo[1,2-a]pyrazine-3-amine
[111] N-tert-butyl-2-(3-(pyridin-2-yléthynyl)phényl)imidazo[1,2-a]pyrazine-3-amine [112] chlorhydrate de N-tert-butyl-2-(5-((2-méthoxyphényl)éthynyl)thiophén-2-yl)imidazo[1,2-a]pyrazine-3-amine
[113] chlorhydrate de N-tert-butyl-2-(5-((3-méthoxyphényl)éthynyl)thiophén-2-yl)imidazo[1,2-a]pyrazine-3-amine
[114] chlorhydrate de N-tert-butyl-2-(5-((4-méthoxyphényl)éthynyl)thiophén-2-yl)imidazo[1,2-a]pyrazine-3-amine
[115] N-tert-butyl-2-(5-(phényléthynyl)thiophén-2-yl)imidazo[1,2-a]quinoline-1-amine
[116] N-tert-butyl-2-(4-(pyridin-2-yléthynyl)phényl)imidazo[1,2-a]pyrazine-3-amine [117] N-tert-butyl-2-(3-((6-méthylpyridin-2-yl)éthynyl)phényl)imidazo[1,2-a]pyrazine-3-amine [118] N-tert-butyl-2-(2-méthyl-6-(phényléthynyl)pyridin-3-yl)imidazo[1,2-a]pyrazine-3-amine
[119] N-tert-butyl-2-(5-(pyridin-2-yléthynyl)furan-2-yl)imidazo[1,2-a]pyrazine-3-amine
[120] acide 3-(tert-butylamino)-2-(5-(phényléthynyl)thiophén-2-yl)imidazo[1,2-a]pyrazine-8-carboxylique
[121] chlorhydrate de 4-((5-(3-(tert-butylamino)imidazo[1,2-a]pyrazin-2-yl)thiophén-2-yl)éthynyl)phénol
[122] 3-((5-(3-(tert-butylamino)imidazo[1,2-a]pyrazin-2-yl)thiophén-2-yl)éthynyl)phénol
[123] chlorhydrate de 2-(5-((3-aminophényl)éthynyl)thiophén-2-yl)-N-tert-butylimidazo[1,2-a]pyrazine-3-amine
[124] chlorhydrate de 2-(5-((2-aminophényl)éthynyl)thiophén-2-yl)-N-tert-butylimidazo[1,2-a]pyrazine-3-amine
[125] N-tert-butyl-2-(5-(phényléthynyl)thiophén-2-yl)imidazo[2,1-a]isoquinoline-3-amine
[126] N-tert-butyl-2-(6-(phényléthynyl)pyridin-3-yl)imidazo[1,2-a]pyrazine-3-amine
[127] chlorhydrate de N-tert-butyl-2-(5-(pyridin-4-yléthynyl)thiophén-2-yl)imidazo[1,2-a]pyrazine-3-amine
[128] chlorhydrate de 2-(5-((6-aminopyridin-3-yl)éthynyl)thiophén-2-yl)-N-tert-butylimidazo[1,2-a]pyrazine-3-amine
[129] chlorhydrate de N-tert-butyl-2-(5-(pyrimidin-2-yléthynyl)thiophén-2-yl)imidazo[1,2-a]pyrazine-3-amine
[130] chlorhydrate de N-tert-butyl-2-(5-((4-méthylpyridin-2-yl)éthynyl)thiophén-2-yl)imidazo[1,2-a]pyrazine-3-amine
[131] chlorhydrate de N-tert-butyl-2-(5-((5-méthylpyridin-2-yl)éthynyl)thiophén-2-yl)imidazo[1,2-a]pyrazine-3-amine
[132] chlorhydrate de N-tert-butyl-2-(5-(pyridin-4-yléthynyl)thiophén-2-yl)imidazo[1,2-a]pyridine-3-amine
[133] N-tert-butyl-2-(5-(thiazol-2-yléthynyl)thiophén-2-yl)imidazo[1,2-a]pyrazine-3-amine
[134] chlorhydrate de 2-(5-(pyridin-2-yléthynyl)thiophén-2-yl)imidazo[1,2-a]pyrazine-3-amine
[135] chlorhydrate de N-tert-butyl-2-(5-((5-méthylthiophén-2-yl)éthynyl)thiophén-2-yl)imidazo[1,2-a]pyrazine-3-amine
[136] chlorhydrate de 2-(5-((6-aminopyridin-2-yl)éthynyl)thiophén-2-yl)-N-tert-butylimidazo[1,2-a]pyrazine-3-amine
[137] N-tert-butyl-2-(5-((3-méthylthiophén-2-yl)éthynyl)thiophén-2-yl)imidazo[1,2-a]pyrazine-3-amine
[138] N-tert-butyl-2-(4-(phényléthynyl)thiazol-2-yl)imidazo[1,2-a]pyrazine-3-amine
[139] N-tert-butyl-2-(5-(m-tolyléthynyl)thiophén-2-yl)imidazo[1,2-a]pyrazine-3-amine
[140] chlorhydrate de 3-((5-(3-(tert-butylamino)imidazo[1,2-a]pyrazin-2-yl)thiophén-2-yl)éthynyl)benzonitrile
[141] N-tert-butyl-2-(5-(pyridin-2-yléthynyl)thiophén-2-yl)imidazo[1,2-a]pyrimidine-3-amine
[142] chlorhydrate de N-tert-butyl-2-(6-(phényléthynyl)pyridin-2-yl)imidazo[1,2-a]pyrazine-3-amine
[143] N-tert-butyl-N-méthyl-2-(5-(pyridin-2-yléthynyl)thiophén-2-yl)imidazo[1,2-a]pyrazine-3-amine
[144] chlorhydrate de 4-((5-(3-(tert-butylamino)imidazo[1,2-a]pyrazin-2-yl)thiophén-2-yl)éthynyl)benzonitrile
[145] chlorhydrate de 2-(5-((1H-indol-6-yl)éthynyl)thiophén-2-yl)-N-tert-butylimidazo[1,2-a]pyrazine-3-amine
[146] N-tert-butyl-2-(2-(phényléthynyl)thiazol-5-yl)imidazo[1,2-a]pyrazine-3-amine
[147] chlorhydrate de N-tert-butyl-2-(5-(quinolin-6-yléthynyl)thiophén-2-yl)imidazo[1,2-a]pyrazine-3-amine
[148] chlorhydrate de 2-(5-((3-(1H-pyrrol-1-yl)phényl)éthynyl)thiophén-2-yl)-N-tert-butylimidazo[1,2-a]pyrazine-3-amine
[149] chlorhydrate de 2-(5-((1H-indol-4-yl)éthynyl)thiophén-2-yl)-N-tert-butylimidazo[1,2-a]pyrazine-3-amine
[150] chlorhydrate de N-tert-butyl-2-(5-((3-nitrophényl)éthynyl)thiophén-2-yl)imidazo[1,2-a]pyrazine-3-amine
[151] chlorhydrate de N-tert-butyl-2-(5-((4-nitrophényl)éthynyl)thiophén-2-yl)imidazo[1,2-a]pyrazine-3-amine
[152] chlorhydrate de N-tert-butyl-2-(5-(thiazol-4-yléthynyl)thiophén-2-yl)imidazo[1,2-a]pyrazine-3-amine
[153] 2-((5-(3-(tert-butylamino)imidazo[1,2-a]pyrazin-2-yl)thiophén-2-yl)éthynyl)phénol
[154] 2-(5-((3-(aminométhyl)phényl)éthynyl)thiophén-2-yl)-N-tert-butylimidazo[1,2-a]pyrazine-3-amine
[155] chlorhydrate de 2-(5-(biphényl-3-yléthynyl)thiophén-2-yl)-N-tert-butylimidazo[1,2-a]pyrazine-3-amine
[156] chlorhydrate de N-tert-butyl-2-(5-(thiophén-3-yléthynyl)thiophén-2-yl)imidazo[1,2-a]pyrazine-3-amine
[157] chlorhydrate de N-tert-butyl-2-(5-((3-(diméthylamino)phényl)éthynyl)thiophén-2-yl)imidazo[1,2-a]pyrazine-3-amine
[158] chlorhydrate de N-tert-butyl-2-(5-((6-méthylpyridin-2-yl)éthynyl)thiophén-2-yl)imidazo[1,2-a]pyrazine-3-amine
[159] chlorhydrate de N-tert-butyl-2-(5-((3-fluoropyridin-2-yl)éthynyl)thiophén-2-yl)imidazo[1,2-a]pyrazine-3-amine
[160] chlorhydrate de N-tert-butyl-2-(5-((3-(méthylamino)phényl)éthynyl)thiophén-2-yl)imidazo[1,2-a]pyrazine-3-amine
[161] N-tert-butyl-2-(5-(p-tolyléthynyl)thiophén-2-yl)imidazo[1,2-a]pyrazine-3-amine
[162] chlorhydrate de N-tert-butyl-2-(5-(o-tolyléthynyl)thiophén-2-yl)imidazo[1,2-a]pyrazine-3-amine
[163] chlorhydrate de N-tert-butyl-2-(4-méthyl-5-(phényléthynyl)thiophén-2-yl)imidazo[1,2-a]pyrazine-3-amine
[164] chlorhydrate de N-tert-butyl-2-(4-méthyl-5-(pyridin-2-yléthynyl)thiophén-2-yl)imidazo[1,2-a]pyrazine-3-amine
[165] N-tert-butyl-2-(5-((6-fluoropyridin-2-yl)éthynyl)thiophén-2-yl)imidazo[1,2-a]pyrazine-3-amine
[166] N-tert-butyl-2-(5-((2-nitrophényl)éthynyl)thiophén-2-yl)imidazo[1,2-a]pyrazine-3-amine
[167] N-tert-butyl-8-chloro-2-(5-(pyridin-2-yléthynyl)thiophén-2-yl)imidazo[1,2-a]pyrazine-3-amine
[168] N-tert-butyl-2-(5-((6-méthoxypyridin-2-yl)éthynyl)thiophén-2-yl)imidazo[1,2-a]pyrazine-3-amine
[169] chlorhydrate de N-tert-butyl-2-(5-((5-fluoropyridin-2-yl)éthynyl)thiophén-2-yl)imidazo[1,2-a]pyrazine-3-amine
[170] 2-(4-(3-(tert-butylamino)imidazo[1,2-a]pyrazin-2-yl)-2-(phényléthynyl)phényl)acétonitrile
[171] chlorhydrate de N-tert-butyl-2-(5-((5-méthoxypyridin-3-yl)éthynyl)thiophén-2-yl)imidazo[1,2-a]pyrazine-3-amine
[172] chlorhydrate de 5-((5-(3-(tert-butylamino)imidazo[1,2-a]pyrazin-2-yl)thiophén-2-yl)éthynyl)nicotinonitrile
[173] N-tert-butyl-2-(5-((3-(méthylthio)phényl)éthynyl)thiophén-2-yl)imidazo[1,2-a]pyrazine-3-amine
[174] benzoate de méthyl-3-((5-(3-(tert-butylamino)imidazo[1,2-a]pyrazin-2-yl)thiophén-2-yl)éthynyle)
[175] N-tert-butyl-2-(5-((3,5-difluoropyridin-2-yl)éthynyl)thiophén-2-yl)imidazo[1,2-a]pyrazine-3-amine
[176] N-tert-butyl-2-(5-(phényléthynyl)thiazol-2-yl)imidazo[1,2-a]pyrazine-3-amine
[177] N-tert-butyl-2-(2-(pyridin-4-yléthynyl)thiazol-5-yl)imidazo[1,2-a]pyrazine-3-amine
[178] 3-((5-(3-(tert-butylamino)imidazo[1,2-a]pyrazin-2-yl)thiophén-2-yl)éthynyl)benzaldéhyde
[179] 3-((5-(3-(tert-butylamino)imidazo[1,2-a]pyrazin-2-yl)thiophén-2-yl)éthynyl)-4-fluorobenzonitrile
[180] chlorhydrate de N-tert-butyl-2-(5-((3-(trifluorométhyl)phényl)éthynyl)thiophén-2-yl)imidazo[1,2-a]pyrazine-3-amine
[181] N-tert-butyl-2-(2-(pyridin-2-yléthynyl)thiazol-5-yl)imidazo[1,2-a]pyrazine-3-amine
[182] chlorhydrate de N-tert-butyl-2-(3-méthyl-5-(phényléthynyl)thiophén-2-yl)imidazo[1,2-a]pyrazine-3-amine
[183] chlorhydrate de N-tert-butyl-2-(3-méthyl-5-(pyridin-2-yléthynyl)thiophén-2-yl)imidazo[1,2-a]pyrazine-3-amine
[184] N-tert-butyl-2-(5-((3-vinylphényl)éthynyl)thiophén-2-yl)imidazo[1,2-a]pyrazine-3-amine
[185] chlorhydrate de 2-(5-((1H-imidazol-4-yl)éthynyl)thiophén-2-yl)-N-tert-butylimidazo[1,2-a]pyrazine-3-amine
[186] N-tert-butyl-2-(5-((3-méthylpyridin-2-yl)éthynyl)thiophén-2-yl)imidazo[1,2-a]pyrazine-3-amine
[187] N,N-diméthyl-2-(5-(pyridin-2-yléthynyl)thiophén-2-yl)imidazo[1,2-a]pyrazine-3-amine
[188] N-tert-butyl-2-(5-((2-(tert-butyldiphénylsilyl)thiazol-5-yl)éthynyl)thiophén-2-yl)imidazo[1,2-a]pyridine-3-amine
[189] 3-((5-(3-(tert-butylamino)imidazo[1,2-a]pyridin-2-yl)thiophén-2-yl)éthynyl)benzonitrile
[190] N-tert-butyl-2-(5-(phényléthynyl)thiazol-2-yl)imidazo[1,2-a]pyridine-3-amine
[191] N-tert-butyl-2-(5-(thiazol-5-yléthynyl)thiophén-2-yl)imidazo[1,2-a]pyridine-3-amine
[192] chlorhydrate de N-tert-butyl-2-(5-(pyridin-2-yléthynyl)thiophén-2-yl)imidazo[1,2-a]pyridine-3-amine
[193] 6-chloro-N-cyclohexyl-2-(3-((6-méthylpyridin-2-yl)éthynyl)phényl)imidazo[1,2-a]pyridine-3-amine
[194] 5,7-diméthyl-N-phénéthyl-2-(5-(phényléthynyl)thiophén-2-yl)imidazo[1,2-a]pyrimidine-3-amine
[195] N-(3-méthoxyphénéthyl)-5,7-diméthyl-2-(5-(phényléthynyl)thiophén-2-yl)imidazo[1,2-a]pyrimidine-3-amine
[196] N-(3-méthoxyphénéthyl)-5,7-diméthyl-2-(4-(phényléthynyl)thiophén-2-yl)imidazo[1,2-a]pyrimidine-3-amine
[197] N-(3-méthoxyphénéthyl)-5,7-diméthyl-2-(5-(phényléthynyl)furan-2-yl)imidazo[1,2-a]pyrimidine-3-amine
[198] N-(4-chlorobenzyl)-8-méthyl-2-(5-(phényléthynyl)thiophén-2-yl)imidazo[1,2-a]pyridine-3-amine
[199] N-(3-méthoxyphénéthyl)-5-méthyl-2-(4-(phényléthynyl)thiophén-2-yl)imidazo[1,2-a]pyridine-3-amine
[200] N-(2-méthylhexan-2-yl)-2-(4-(phényléthynyl)thiophén-2-yl)imidazo[1,2-a]pyrazine-3-amine
[201] N-phénéthyl-2-(4-(phényléthynyl)thiophén-2-yl)imidazo[1,2-a]pyrazine-3-amine
[202] N-(3-méthoxyphénéthyl)-2-(4-(phényléthynyl)thiophén-2-yl)imidazo[1,2-a]pyrazine-3-amine
[203] 2-(4-(phényléthynyl)thiophén-2-yl)-N-(2-(thiophén-2-yl)éthyl)imidazo[1,2-a]pyrazine-3-amine
[204] N-(4-chlorobenzyl)-2-(5-(phényléthynyl)thiophén-2-yl)imidazo[1,2-a]pyridine-3-amine
[205] N-(2-méthylpentan-2-yl)-2-(5-(phényléthynyl)thiophén-2-yl)imidazo[1,2-a]pyrazine-3-amine
[206] N-(cyclohexylméthyl)-2-(4-(phényléthynyl)thiophén-2-yl)imidazo[1,2-a]pyrazine-3-amine
[207] N-(2-méthoxybenzyl)-2-(5-(phényléthynyl)furan-2-yl)imidazo[1,2-a]pyrazine-3-amine
[208] N-(cyclohexylméthyl)-2-(4-(phényléthynyl)thiophén-2-yl)imidazo[1,2-a]pyridine-3-amine
[209] N-(2-méthylpentan-2-yl)-2-(4-(phényléthynyl)thiophén-2-yl)imidazo[1,2-a]pyridine-3-amine
[210] 8-bromo-6-méthyl-2-(3-((6-méthylpyridin-2-yl)éthynyl)phényl)-N-(2,4,4-triméthylpentan-2-yl)imidazo[1,2-a]pyridine-3-amine
[211] 8-bromo-N-cyclopentyl-6-méthyl-2-(3-(pyridin-2-yléthynyl)phényl)imidazo[1,2-a]pyridine-3-amine
[212] N-cyclopentyl-2-(4-(phényléthynyl)thiophén-2-yl)imidazo[1,2-a]pyrazine-3-amine
[213] N-(1-phényléthyl)-2-(4-(phényléthynyl)thiophén-2-yl)imidazo[1,2-a]pyrazine-3-amine
[214] N-(2-méthylpentan-2-yl)-2-(4-(phényléthynyl)thiophén-2-yl)imidazo[1,2-a]pyrazine-3-amine
[215] 8-bromo-N-cyclohexyl-6-méthyl-2-(5-(pyridin-2-yléthynyl)furan-2-yl)imidazo[1,2-a]pyridine-3-amine
[216] N-cyclopentyl-2-(5-(phényléthynyl)furan-2-yl)imidazo[1,2-a]pyrazine-3-amine
[217] N-(3-méthoxyphénéthyl)-7-méthyl-2-(4-(phényléthynyl)thiophén-2-yl)imidazo[1,2-a]pyrimidine-3-amine
[218] 8-(benzyloxy)-2-(5-(phényléthynyl)thiophén-2-yl)-N-(2,4,4-triméthylpentan-2-yl)imidazo[1,2-a]pyridine-3-amine
[219] 8-(benzyloxy)-N-cyclopentyl-2-(4-(phényléthynyl)thiophén-2-yl)imidazo[1,2-a]pyridine-3-amine
[220] 8-(benzyloxy)-N-(2-méthylpentan-2-yl)-2-(4-(phényléthynyl)thiophén-2-yl)imidazo[1,2-a]pyridine-3-amine
[221] 6-chloro-N-(4-fluorobenzyl)-2-(5-(phényléthynyl)furan-2-yl)imidazo[1,2-a]pyridine-3-amine
[222] 6-bromo-N-butyl-5-méthyl-2-(4-(phényléthynyl)thiophén-2-yl)imidazo[1,2-a]pyridine-3-amine
[223] N-(furan-2-yl)-8-méthyl-2-(5-(phényléthynyl)furan-2-yl)imidazo[1,2-a]pyridine-3-amine
[224] N-(furan-2-yl)-2-(5-(phényléthynyl)furan-2-yl)imidazo[1,2-a]pyridine-3-amine
[225] N-(furan-2-yl)-2-(5-(phényléthynyl)furan-2-yl)-7-propylimidazo[1,2-a]pyridine-3-amine
[226] 5,7-diméthyl-2-(4-(phényléthynyl)thiophén-2-yl)-N-(3-(trifluorométhyl)phényl)imidazo[1,2-a]pyrimidine-3-amine
[227] 6-bromo-N-(4-chlorophénéthyl)-8-méthyl-2-(4-(phényléthynyl)thiophén-2-yl)imidazo[1,2-a]pyridine-3-amine
[228] N-(4-chlorophénéthyl)-7-phényl-2-(4-(phényléthynyl)thiophén-2-yl)imidazo[1,2-a]pyridine-3-amine
[229] N-phénéthyl-7-phényl-2-(4-(phényléthynyl)thiophén-2-yl)imidazo[1,2-a]pyridine-3-amine
[230] N-(4-chlorobenzyl)-5,7-diméthyl-2-(4-(phényléthynyl)thiophén-2-yl)imidazo[1,2-a]pyridine-3-amine
[231] 6-bromo-N-(4-chlorobenzyl)-5-méthyl-2-(4-(phényléthynyl)thiophén-2-yl)imidazo[1,2-a]pyridine-3-amine
[232] 8-bromo-N-(4-chlorobenzyl)-6-méthyl-2-(4-(phényléthynyl)thiophén-2-yl)imidazo[1,2-a]pyridine-3-amine
[233] N-(3-méthoxyphénéthyl)-2-(4-(phényléthynyl)thiophén-2-yl)-5-propylimidazo[1,2-a]pyridine-3-amine
[234] 6-bromo-8-méthyl-2-(4-(phényléthynyl)thiophén-2-yl)-N-(2-(thiophén-2-yl)éthyl)imidazo[1,2-a]pyridine-3-amine
[235] 7-phényl-2-(4-(phényléthynyl)thiophén-2-yl)-N-(2-(thiophén-2-yl)éthyl)imidazo[1,2-a]pyridine-3-amine
[236] 6,8-dibromo-N-(2-méthylpentan-2-yl)-2-(5-(phényléthynyl)thiophén-2-yl)imidazo[1,2-a]pyridine-3-amine
[237] 6-bromo-N-(2,6-diméthylphényl)-8-méthyl-2-(4-(phényléthynyl)thiophén-2-yl)imidazo[1,2-a]pyridine-3-amine
[238] N-cyclohexyl-2-(3-(pyridin-2-yléthynyl)phényl)imidazo[1,2-a]pyrazine-3-amine
[239] 2-(3-(pyridin-2-yléthynyl)phényl)-N-(2,4,4-triméthylpentan-2-yl)imidazo[1,2-a]pyrazine-3-amine
[240] N-cyclopentyl-2-(4-(pyridin-2-yléthynyl)phényl)imidazo[1,2-a]pyrazine-3-amine
[241] 8-chloro-2-(4-(pyridin-2-yléthynyl)phényl)-6-(trifluorométhyl)-N-(2,4,4-triméthylpentan-2-yl)imidazo[1,2-a]pyridine-3-amine
[242] N-(4-fluorophényl)-2-(4-(pyridin-2-yléthynyl)phényl)imidazo[1,2-a]pyrazine-3-amine
[243] N-cyclopentyl-2-(2-méthyl-6-(phényléthynyl)pyridin-3-yl)imidazo[1,2-a]pyrazine-3-amine
[244] N-cyclohexyl-2-(2-méthyl-6-(phényléthynyl)pyridin-3-yl)imidazo[1,2-a]pyrazine-3-amine
[245] N-cyclopentyl-2-(5-(pyridin-2-yléthynyl)furan-2-yl)imidazo[1,2-a]pyrazine-3-amine
[246] 8-bromo-N-cyclopentyl-6-méthyl-2-(5-(pyridin-2-yléthynyl)furan-2-yl)imidazo[1,2-a]pyridine-3-amine
[247] N-cyclohexyl-2-(5-(pyridin-2-yléthynyl)furan-2-yl)imidazo[1,2-a]pyrazine-3-amine
[248] 2-(5-(pyridin-2-yléthynyl)furan-2-yl)-N-(2,4,4-triméthylpentan-2-yl)imidazo[1,2-a]pyrazine-3-amine
[249] N-cyclopentyl-2-(3-((6-méthylpyridin-2-yl)éthynyl)phényl)imidazo[1,2-a]pyrazine-3-amine
[250] 8-bromo-N-cyclopentyl-6-méthyl-2-(4-(pyridin-2-yléthynyl)phényl)imidazo[1,2-a]pyridine-3-amine
[251] N-cyclohexyl-2-(3-((6-méthylpyridin-2-yl)éthynyl)phényl)imidazo[1,2-a]pyrazine-3-amine
[252] N-(4-fluorophényl)-2-(3-((6-méthylpyridin-2-yl)éthynyl)phényl)imidazo[1,2-a]pyrazine-3-amine
[253] 2-(5-(pyridin-2-yléthynyl)thiophén-2-yl)-N-(2,4,4-triméthylpentan-2-yl)imidazo[1,2-a]pyrimidine-3-amine
[254] N-cyclohexyl-2-(3-(pyridin-2-yléthynyl)phényl)imidazo[1,2-a]pyrimidine-3-amine [255] 2-(3-(pyridin-2-yléthynyl)phényl)-N-(2,4,4-triméthylpentan-2-yl)imidazo[1,2-a]pyrimidine-3-amine
[256] 2-(6-(phényléthynyl)pyridin-3-yl)-N-(2,4,4-triméthylpentan-2-yl)imidazo[1,2-a]pyrimidine-3-amine
[257] N-cyclopentyl-2-(5-(pyridin-2-yléthynyl)furan-2-yl)imidazo[1,2-a]pyrimidine-3-amine
[258] N-tert-butyl-2-(5-(pyridin-2-yléthynyl)furan-2-yl)imidazo[1,2-a]pyrimidine-3-amine
[259] N-cyclopentyl-2-(3-((6-méthylpyridin-2-yl)éthynyl)phényl)imidazo[1,2-a]pyrimidine-3-amine
[260] N-cyclopentyl-2-(3-(pyridin-2-yléthynyl)phényl)imidazo[1,2-a]pyridine-3-amine
[261] N-tert-butyl-2-(3-(pyridin-2-yléthynyl)phényl)imidazo[1,2-a]pyridine-3-amine
[262] 2-(3-(pyridin-2-yléthynyl)phényl)-N-(2,4,4-triméthylpentan-2-yl)imidazo[1,2-a]pyridine-3-amine
[263] N-(4-fluorophényl)-2-(3-(pyridin-2-yléthynyl)phényl)imidazo[1,2-a]pyridine-3-amine
[264] N-tert-butyl-2-(4-(pyridin-2-yléthynyl)phényl)imidazo[1,2-a]pyridine-3-amine
[265] N-cyclopentyl-2-(2-méthyl-6-(phényléthynyl)pyridin-3-yl)imidazo[1,2-a]pyridine-3-amine
[266] N-(4-fluorophényl)-2-(2-méthyl-6-(phényléthynyl)pyridin-3-yl)imidazo[1,2-a]pyridine-3-amine
[267] N-cyclopentyl-6-méthyl-2-(5-(pyridin-2-yléthynyl)thiophén-2-yl)imidazo[1,2-a]pyridine-3-amine
[268] N-tert-butyl-6-méthyl-2-(5-(pyridin-2-yléthynyl)thiophén-2-yl)imidazo[1,2-a]pyridine-3-amine
[269] N-cyclohexyl-6-méthyl-2-(5-(pyridin-2-yléthynyl)thiophén-2-yl)imidazo[1,2-a]pyridine-3-amine
[270] 6-méthyl-2-(5-(pyridin-2-yléthynyl)thiophén-2-yl)-N-(2,4,4-triméthylpentan-2-yl)imidazo[1,2-a]pyridine-3-amine
[271] 6-méthyl-2-(2-méthyl-6-(phényléthynyl)pyridin-3-yl)-N-(2,4,4-triméthylpentan-2-yl)imidazo[1,2-a]pyridine-3-amine
[272] N-cyclopentyl-6-méthyl-2-(5-(pyridin-2-yléthynyl)furan-2-yl)imidazo[1,2-a]pyridine-3-amine
[273] N-cyclohexyl-6-méthyl-2-(3-((6-méthylpyridin-2-yl)éthynyl)phényl)imidazo[1,2-a]pyridine-3-amine
[274] N-cyclohexyl-7-méthyl-2-(5-(pyridin-2-yléthynyl)thiophén-2-yl)imidazo[1,2-a]pyridine-3-amine
[275] 7-méthyl-2-(5-(pyridin-2-yléthynyl)thiophén-2-yl)-N-(2,4,4-triméthylpentan-2-yl)imidazo[1,2-a]pyridine-3-amine
[276] N-tert-butyl-7-méthyl-2-(3-(pyridin-2-yléthynyl)phényl)imidazo[1,2-a]pyridine-3-amine
[277] N-(4-fluorophényl)-7-méthyl-2-(3-(pyridin-2-yléthynyl)phényl)imidazo[1,2-a]pyridine-3-amine
[278] N-tert-butyl-7-méthyl-2-(4-(pyridin-2-yléthynyl)phényl)imidazo[1,2-a]pyridine-3-amine
[279] N-(4-fluorophényl)-7-méthyl-2-(5-(pyridin-2-yléthynyl)furan-2-yl)imidazo[1,2-a]pyridine-3-amine
[280] N-cyclohexyl-8-méthyl-2-(6-(phényléthynyl)pyridin-3-yl)imidazo[1,2-a]pyridine-3-amine
[281] N-cyclopentyl-2-(6-(phényléthynyl)pyridin-3-yl)imidazo[1,2-a]pyridine-3-amine
[282] N-tert-butyl-2-(6-(phényléthynyl)pyridin-3-yl)imidazo[1,2-a]pyridine-3-amine
[283] N-cyclohexyl-2-(6-(phényléthynyl)pyridin-3-yl)imidazo[1,2-a]pyridine-3-amine
[284] 2-(6-(phényléthynyl)pyridin-3-yl)-N-(2,4,4-triméthylpentan-2-yl)imidazo[1,2-a]pyridine-3-amine
[285] N-tert-butyl-2-(5-(pyridin-2-yléthynyl)furan-2-yl)imidazo[1,2-a]pyridine-3-amine
[286] N-cyclohexyl-2-(5-(pyridin-2-yléthynyl)furan-2-yl)imidazo[1,2-a]pyridine-3-amine
[287] N-(4-fluorophényl)-2-(5-(pyridin-2-yléthynyl)furan-2-yl)imidazo[1,2-a]pyridine-3-amine
[288] N-cyclohexyl-2-(3-((6-méthylpyridin-2-yl)éthynyl)phényl)imidazo[1,2-a]pyridine-3-amine
[289] 2-(3-((6-méthylpyridin-2-yl)éthynyl)phényl)-N-(2,4,4-triméthylpentan-2-yl)imidazo[1,2-a]pyridine-3-amine
[290] N-tert-butyl-5-méthyl-2-(3-((6-méthylpyridin-2-yl)éthynyl)phényl)imidazo[1,2-a]pyridine-3-amine
[291] 5-méthyl-2-(3-((6-méthylpyridin-2-yl)éthynyl)phényl)-N-(2,4,4-triméthylpentan-2-yl)imidazo[1,2-a]pyridine-3-amine
[292] N-cyclohexyl-5,7-diméthyl-2-(3-(pyridin-2-yléthynyl)phényl)imidazo[1,2-a]pyrimidine-3-amine
[293] N-cyclohexyl-5,7-diméthyl-2-(2-méthyl-6-(phényléthynyl)pyridin-3-yl)imidazo[1,2-a]pyrimidine-3-amine
[294] N-cyclopentyl-5,7-diméthyl-2-(5-(pyridin-2-yléthynyl)furan-2-yl)imidazo[1,2-a]pyrimidine-3-amine
[295] N-tert-butyl-5,7-diméthyl-2-(5-(pyridin-2-yléthynyl)furan-2-yl)imidazo[1,2-a]pyrimidine-3-amine
[296] N-(4-fluorophényl)-8-méthyl-2-(3-(pyridin-2-yléthynyl)phényl)imidazo[1,2-a]pyridine-3-amine
[297] N-cyclohexyl-8-méthyl-2-(4-(pyridin-2-yléthynyl)phényl)imidazo[1,2-a]pyridine-3-amine
[298] N-cyclohexyl-7-éthyl-2-(5-(pyridin-2-yléthynyl)thiophén-2-yl)imidazo[1,2-a]pyridine-3-amine
[299] 7-éthyl-2-(5-(pyridin-2-yléthynyl)thiophén-2-yl)-N-(2,4,4-triméthylpentan-2-yl)imidazo[1,2-a]pyridine-3-amine
[300] N-cyclohexyl-7-éthyl-2-(3-(pyridin-2-yléthynyl)phényl)imidazo[1,2-a]pyridine-3-amine
[301] N-cyclopentyl-7-éthyl-2-(4-(pyridin-2-yléthynyl)phényl)imidazo[1,2-a]pyridine-3-amine
[302] N-cyclopentyl-7-éthyl-2-(6-(phényléthynyl)pyridin-3-yl)imidazo[1,2-a]pyridine-3-amine
[303] N-tert-butyl-7-éthyl-2-(6-(phényléthynyl)pyridin-3-yl)imidazo[1,2-a]pyridine-3-amine
[304] N-tert-butyl-7-éthyl-2-(5-(pyridin-2-yléthynyl)furan-2-yl)imidazo[1,2-a]pyridine-3-amine
[305] 7-isopropyl-2-(5-(pyridin-2-yléthynyl)thiophén-2-yl)-N-(2,4,4-triméthylpentan-2-yl)imidazo[1,2-a]pyridine-3-amine
[306] N-tert-butyl-7-isopropyl-2-(3-(pyridin-2-yléthynyl)phényl)imidazo[1,2-a]pyridine-3-amine
[307] N-tert-butyl-7-isopropyl-2-(4-(pyridin-2-yléthynyl)phényl)imidazo[1,2-a]pyridine-3-amine
[308] N-cyclohexyl-7-isopropyl-2-(5-(pyridin-2-yléthynyl)furan-2-yl)imidazo[1,2-a]pyridine-3-amine
[309] N-tert-butyl-7-isopropyl-2-(3-((6-méthylpyridin-2-yl)éthynyl)phényl)imidazo[1,2-a]pyridine-3-amine
[310] 6-chloro-N-cyclopentyl-2-(5-(pyridin-2-yléthynyl)thiophén-2-yl)imidazo[1,2-a]pyridine-3-amine
[311] N-tert-butyl-6-chloro-2-(5-(pyridin-2-yléthynyl)thiophén-2-yl)imidazo[1,2-a]pyridine-3-amine
[312] 6-chloro-N-cyclohexyl-2-(3-(pyridin-2-yléthynyl)phényl)imidazo[1,2-a]pyridine-3-amine
[313] 6-chloro-2-(3-(pyridin-2-yléthynyl)phényl)-N-(2,4,4-triméthylpentan-2-yl)imidazo[1,2-a]pyridine-3-amine
[314] N-tert-butyl-6-chloro-2-(4-(pyridin-2-yléthynyl)phényl)imidazo[1,2-a]pyridine-3-amine
[315] 6-chloro-N-cyclohexyl-2-(4-(pyridin-2-yléthynyl)phényl)imidazo[1,2-a]pyridine-3-amine
[316] N-tert-butyl-6-chloro-2-(5-(pyridin-2-yléthynyl)furan-2-yl)imidazo[1,2-a]pyridine-3-amine
[317] 6-chloro-N-cyclohexyl-2-(5-(pyridin-2-yléthynyl)furan-2-yl)imidazo[1,2-a]pyridine-3-amine
[318] 6-chloro-2-(5-(pyridin-2-yléthynyl)furan-2-yl)-N-(2,4,4-triméthylpentan-2-yl)imidazo[1,2-a]pyridine-3-amine
[319] 6-chloro-N-cyclopentyl-2-(3-((6-méthylpyridin-2-yl)éthynyl)phényl)imidazo[1,2-a]pyridine-3-amine
[320] N-tert-butyl-6-chloro-2-(3-((6-méthylpyridin-2-yl)éthynyl)phényl)imidazo[1,2-a]pyridine-3-amine
[321] bischlorhydrate de N-tert-butyl-2-(5-(pyridin-2-yléthynyl)thiophén-2-yl)imidazo[1,2-a]pyridine-3-amine
[322] N-méthyl-2-(5-(pyridin-2-yléthynyl)thiophén-2-yl)imidazo[1,2-a]pyrazine-3-amine
[323] bischlorhydrate de N-tert-butyl-2-(5-(pyridin-2-yléthynyl)thiophén-2-yl)imidazo[1,2-a]pyridine-3-amine
[324] [2-(5-pyridin-2-yléthynyl-thiophén-2-yl)-imidazo[1,2-a]pyridin-3-yl]-(1,1,3,3-tétraméthyl-butyl)-amine
[325] chlorhydrate de tert-butyl-[2-(5-pyrimidin-2-yléthynyl-thiophén-2-yl)-imidazo[1,2-a]pyridin-3-yl]-amine
[326] {2-[5-(3-amino-pyridin-2-yléthynyl)-thiophén-2-yl]-imidazo[1,2-a]pyridin-3-yl}-tert-butyl-amine
[327] tert-butyl-[2-(5-pyridin-2-yléthynyl-thiazol-2-yl)-imidazo[1,2-a]pyridin-3-yl]-amine
[328] tert-butyl-[2-(2-pyridin-2-yléthynyl-thiazol-5-yl)-imidazo[1,2-a]pyridin-3-yl]-amine
[329] chlorhydrate de tert-butyl-{2-[5-(6-fluoro-pyridin-2-yléthynyl)-thiophén-2-yl]-imidazo[1,2-a]pyridin-3-yl}-amine
[330] chlorhydrate de tert-butyl-{2-[5-(3-chloro-5-fluoro-phényléthynyl)-thiophén-2-yl]-imidazo[1,2-a]pyridin-3-yl}-amine
[331] chlorhydrate de tert-butyl-[2-(5-pyridin-2-yléthynyl-thiophén-2-yl)-imidazo[1,2-b]pyridazin-3-yl]-amine
[332] chlorhydrate de tert-butyl-[2-(5-thiazol-2-yléthynyl-thiophén-2-yl)-imidazo[1,2-a]pyridin-3-yl]-amine
[333] tert-butyl-{2-[5-(3-trifluorométhoxy-phényléthynyl)-thiophén-2-yl]-imidazo[1,2-a]pyrazin-3-yl}-amine
[334] tert-butyl-12-[5-(3-[1,3]dioxolan-2-yl-phényléthynyl)-thiophén-2-yl]-imidazo[1,2-a]pyridin-3-yl}-amine
[335] chlorhydrate de tert-butyl-{2-[5-(3,5-diméthyl-phényléthynyl)-thiophén-2-yl]-imidazo[1,2-a]pyridin-3-yl}-amine
[336] chlorhydrate de tert-butyl-{2-[5-(3-fluoro-pyridin-2-yléthynyl)-thiophén-2-yl]-imidazo[1,2-a]pyridin-3-yl}-amine
[337] chlorhydrate de tert-butyl-{2-[5-(3-méthyl-3H-imidazol-4-yléthynyl)-thiophén-2-yl]-imidazo[1,2-a]pyridin-3-yl}-amine et
[338] chlorhydrate de tert-butyl-{2-[5-(5-chloro-thiophén-2-yléthynyl)-thiophén-2-yl]-imidazo[1,2-a]pyridin-3-yl}-amine
[339] chlorhydrate de tert-butyl-{2-[5-(5-méthyl-pyridin-2-yléthynyl)-thiophén-2-yl]-imidazo[1,2-a]pyridin-3-yl}-amine
[340] chlorhydrate de 1-{3-[5-(3-tert-butylamino-imidazo[1,2-a]pyridin-2-yl)-thiophén-2-yléthynyl]-phényl}-éthanone
[341] chlorhydrate de {3-[5-(3-tert-butylamino-imidazo[1,2-a]pyridin-2-yl)-thiophén-2-yléthynyl]-phényl}-méthanol
[342] N-tert-butyl-2-(5-((3-méthoxypyridin-2-yl)éthynyl)thiophén-2-yl)imidazo[1,2-a]pyridine-3-amine
[343] chlorhydrate de N-tert-butyl-2-(5-(thiophén-2-yléthynyl)thiophén-2-yl)imidazo[1,2-a]pyridine-3-amine
[344] chlorhydrate de 5-((5-(3-(tert-butylamino)imidazo[1,2-a]pyridin-2-yl)thiophén-2-yl)éthynyl)-2-fluorobenzonitrile
[345] N-tert-butyl-2-(5-((3,4-difluorophényl)éthynyl)thiophén-2-yl)imidazo[1,2-a]pyridine-3-amine
[346] N-tert-butyl-2-(5-((3-(méthoxyméthyl)phényl)éthynyl)thiophén-2-yl)imidazo[1,2-a]pyridine-3-amine
[347] chlorhydrate de 2-(5-((3-aminophényl)éthynyl)thiophén-2-yl)-N-tert-butylimidazo[1,2-a]pyridine-3-amine
[348] chlorhydrate de N-tert-butyl-2-(5-((4-fluoro-3-méthylphényl)éthynyl)thiophén-2-yl)imidazo[1,2-a]pyridine-3-amine
[349] N-tert-butyl-2-(5-((3,5-difluorophényl)éthynyl)thiophén-2-yl)imidazo[1,2-a]pyridine-3-amine
[350] chlorhydrate de N-tert-butyl-2-(5-(thiophén-3-yléthynyl)thiophén-2-yl)imidazo[1,2-a]pyridine-3-amine
[351] chlorhydrate de N-tert-butyl-2-(5-((3-méthylpyridin-2-yl)éthynyl)thiophén-2-yl)imidazo[1,2-a]pyridine-3-amine
[352] chlorhydrate de 3-((5-(3-(tert-butylamino)imidazo[1,2-a]pyridin-2-yl)thiophén-2-yl)éthynyl)benzène-sulfonamide
[353] acide 3-((5-(3-(tert-butylamino)imidazo[1,2-a]pyridin-2-yl)thiophén-2-yl)éthynyl)benzoïque
[354] chlorhydrate de 3-((5-(3-(tert-butylamino)imidazo[1,2-a]pyridin-2-yl)thiophén-2-yl)éthynyl)benzamide
[355] N-(3-((5-(3-(tert-butylamino)imidazo[1,2-a]pyridin-2-yl)thiophén-2-yl)éthynyl)phényl)acétamide
[356] N-tert-butyl-N-méthyl-2-(5-(pyridin-2-yléthynyl)thiophén-2-yl)imidazo[1,2-a]pyridine-3-amine
[357] N,N-diméthyl-2-(5-(pyridin-2-yléthynyl)thiophén-2-yl)imidazo[1,2-a]pyridine-3-amine
[358] N-tert-butyl-N-méthyl-2-(5-(pyridin-2-yléthynyl)thiazol-2-yl)imidazo[1,2-a]pyridine-3-amine
[359] (6-((5-(3-(tert-butylamino)imidazo[1,2-a]pyridin-2-yl)thiophén-2-yl)éthynyl)pyridin-2-yl)méthanol
[360] N-(3-((5-(3-(tert-butylamino)imidazo[1,2-a]pyridin-2-yl)thiophén-2-yl)éthynyl)phényl)méthanesulfonamide
[361] chlorhydrate de N-tert-butyl-8-méthyl-2-(5-(pyridin-2-yléthynyl)thiophén-2-yl)imidazo[1,2-a]pyrazine-3-amine
[362] 2-(5-(pyridin-2-yléthynyl)thiophén-2-yl)imidazo[1,2-a]pyridine-3-amine
[363] N-tert-butyl-2-(5-((3-chlorophényl)éthynyl)thiophén-2-yl)imidazo[1,2-a]pyridine-3-amine
[364] N-tert-butyl-2-(5-((2,3-difluorophényl)éthynyl)thiophén-2-yl)imidazo[1,2-a]pyridine-3-amine et
[365] N-tert-butyl-7-chloro-2-(5-(pyridin-2-yléthynyl)thiophén-2-yl)imidazo[1,2-a]pyridine-3-amine
et à chaque fois éventuellement sous la forme des sels correspondants, notamment des chlorhydrates, ou à chaque fois éventuellement sous la forme des solvates correspondants.

25. Procédé de fabrication de composés imidazo-3-yl-amine bicycliques substitués de formule générale I selon une ou plusieurs des revendications 1 à 24, **caractérisé en ce qu'**au moins un composé de formule générale II 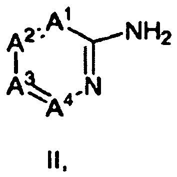 dans laquelle A¹, A², A³ et A⁴ ont la signification selon une ou plusieurs des revendications 1 à 24, est mis en réaction dans un milieu réactionnel, éventuellement en présence d'au moins un acide organique ou inorganique ou d'au moins un sel de métal de transition, avec au moins un isocyanure de formule générale III
R²-N≡C III,
dans laquelle R² a la signification selon une ou plusieurs des revendications 1 à 24, et au moins un aldéhyde de formule générale IV 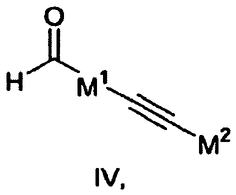 dans laquelle M¹ et M² ont la signification selon une ou plusieurs des revendications 1 à 24, et le composé ainsi obtenu de formule générale V 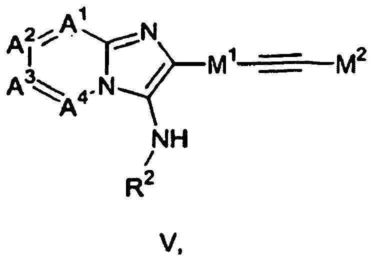 dans laquelle A¹, A², A³, A⁴, R², M¹ et M² ont la signification indiquée précédemment, est éventuellement purifié et/ou isolé, et éventuellement transformé en un sel correspondant et celui-ci est éventuellement purifié et/ou isolé,
ou au moins un composé de formule générale II 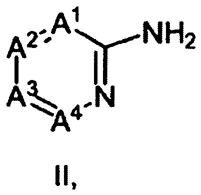 dans laquelle A¹, A², A³ et A⁴ ont la signification selon une ou plusieurs des revendications 1 à 24, est mis en réaction dans un milieu réactionnel, éventuellement en présence d'au moins un acide organique ou inorganique ou d'au moins un sel de métal de transition, avec au moins un isocyanure de formule générale III
R²-N≡C III,
dans laquelle R² a la signification selon une ou plusieurs des revendications 1 à 24, et au moins un aldéhyde de formule générale VI 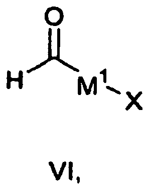 dans laquelle M¹ a la signification selon une ou plusieurs des revendications 1 à 24 et X représente un groupe partant, et le composé ainsi obtenu de formule générale VII 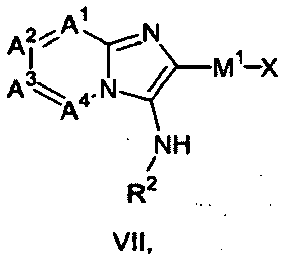 dans laquelle A¹, A², A³, A⁴, R², M¹ et X ont la signification indiquée précédemment, est éventuellement purifié et/ou isolé, et éventuellement transformé en un sel correspondant et celui-ci est éventuellement purifié et/ou isolé, et est transformé par mise en réaction avec au moins un acétylène de formule générale XI 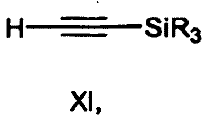 dans laquelle les R représentent chacun indépendamment les uns des autres un radical alkyle linéaire ou ramifié ou un radical phényle non substitué, dans un milieu de réaction, éventuellement en présence d'au moins un catalyseur approprié et éventuellement en présence d'au moins une base inorganique et/ou organique, en un composé substitué correspondant de formule générale XII 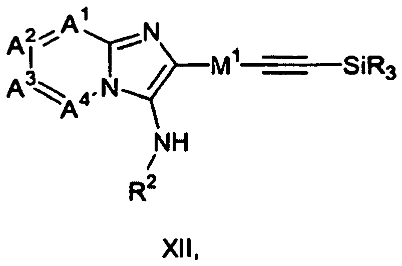 dans laquelle A¹, A², A³, A⁴, R² et M¹ ont la signification indiquée précédemment et les R représentent chacun indépendamment les uns des autres un radical alkyle linéaire ou ramifié ou un radical phényle non substitué, et éventuellement purifié et/ou isolé, et éventuellement transformé en un sel correspondant et celui-ci est éventuellement purifié et/ou isolé,
et au moins un composé de formule générale XII est transformé dans un milieu réactionnel, éventuellement en présence d'au moins une base inorganique et/ou organique, éventuellement en présence d'au moins un sel inorganique et éventuellement en présence d'au moins un sel d'ammonium, en un composé substitué correspondant de formule générale XIII 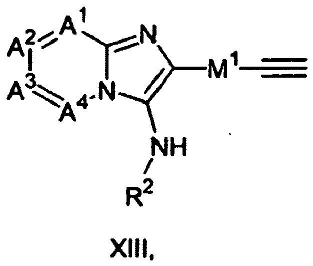 dans laquelle A¹, A², A³, A⁴, R² et M¹ ont la signification indiquée précédemment, et éventuellement purifié et/ou isolé, et éventuellement transformé en un sel correspondant et celui-ci est éventuellement purifié et/ou isolé,
et au moins un composé de formule générale XIII et/ou au moins un composé de formule générale XII est transformé par mise en réaction avec au moins un composé de formule générale M²-X, M² ayant la signification selon une ou plusieurs des revendications 1 à 24 et X représentant un groupe partant, dans un milieu réactionnel, éventuellement en présence d'au moins un catalyseur approprié, éventuellement en présence d'au moins une base inorganique et/ou organique, éventuellement en présence d'au moins un sel inorganique et éventuellement en présence d'au moins un sel d'ammonium, en un composé substitué correspondant de formule générale V 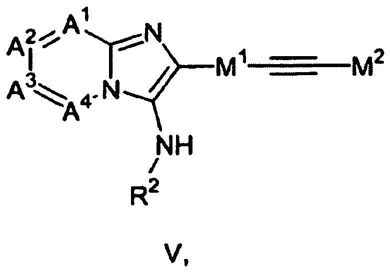 dans laquelle A¹, A², A³, A⁴, R², M¹ et M² ont la signification indiquée précédemment, et éventuellement purifié et/ou isolé, et éventuellement transformé en un sel correspondant et celui-ci est éventuellement purifié et/ou isolé,
ou un composé de formule générale VII est transformé par mise en réaction avec au moins un acétylène de formule générale VIII 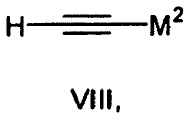 dans laquelle M² a la signification selon une ou plusieurs des revendications 1 à 24, dans un milieu réactionnel, éventuellement en présence d'au moins un catalyseur approprié et éventuellement en présence d'au moins une base inorganique et/ou organique, en un composé substitué correspondant de formule générale V 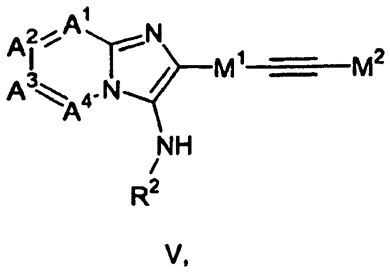 dans laquelle A¹, A², A³, A⁴, R², M¹ et M² ont la signification indiquée précédemment, et éventuellement purifié et/ou isolé, et éventuellement transformé en un sel correspondant et celui-ci est éventuellement purifié et/ou isolé,
et le composé de formule générale V est éventuellement transformé par mise en réaction avec au moins un composé de formule générale R³-X, R³ ayant la signification selon une ou plusieurs des revendications 1 à 24 et X représentant un groupe partant, dans un milieu réactionnel, en présence d'au moins une base organique ou inorganique,
ou par mise en réaction avec au moins un composé de formule générale R²⁰-C(=O)-OH, R²⁰ ayant la signification selon une ou plusieurs des revendications 1 à 24, dans un milieu réactionnel, éventuellement en présence d'au moins une base organique ou inorganique et/ou en présence d'au moins un agent de couplage,
ou par mise en réaction avec au moins un composé de formule générale R²⁰-C(=O)-X, R²⁰ ayant la signification selon une ou plusieurs des revendications 1 à 24 et X représentant un groupe partant, dans un milieu réactionnel, éventuellement en présence d'au moins une base organique ou inorganique,
ou par mise en réaction avec au moins un composé de formule générale R²⁰-C(=O)-H, R²⁰ ayant la signification selon une ou plusieurs des revendications 1 à 24, dans un milieu réactionnel, éventuellement en présence d'au moins un réducteur,
en un composé de formule générale I, éventuellement sous la forme d'un sel correspondant, 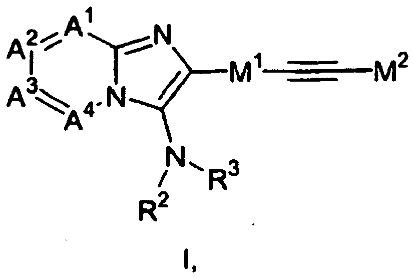 dans laquelle A¹, A², A³, A⁴, R², R³, M¹ et M² ont la signification selon une ou plusieurs des revendications 1 à 24, et celui-ci est éventuellement purifié et/ou isolé.

26. Procédé de fabrication de composés imidazo-3-yl-amine bicycliques substitués de formule générale I selon une ou plusieurs des revendications 1 à 24, **caractérisé en ce qu'**au moins un composé de formule générale V 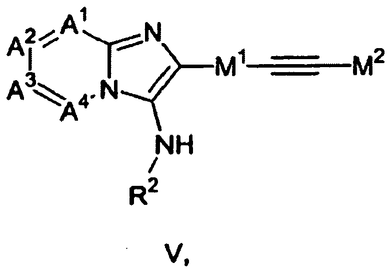 dans laquelle A¹, A², A³, A⁴, R², M¹ et M² ont la signification selon une ou plusieurs des revendications 1 à 24, est mis en réaction éventuellement dans un milieu réactionnel en présence d'au moins un acide organique ou inorganique, et le composé ainsi obtenu de formule générale IX 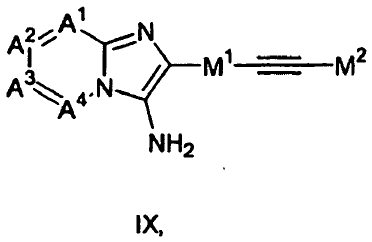 dans laquelle A¹, A², A³, A⁴, M¹ et M² ont la signification selon une ou plusieurs des revendications 1 à 24, est éventuellement purifié et/ou isolé, et éventuellement transformé en un sel correspondant et celui-ci est éventuellement purifié et/ou isolé, et ce composé/ce sel est éventuellement transformé par mise en réaction dans un milieu réactionnel, en présence d'au moins une base inorganique ou organique, avec au moins un composé de formule générale R³-X, R³ ayant la signification selon une ou plusieurs des revendications 1 à 24 et X représentant un groupe partant, ou
dans un milieu réactionnel, éventuellement en présence d'au moins une base organique ou inorganique, et/ou éventuellement en présence d'au moins un agent de couplage, avec au moins un composé de formule générale R²⁰-C(=O)-OH, R²⁰ ayant la signification selon une ou plusieurs des revendications 1 à 24,
ou dans un milieu réactionnel, éventuellement en présence d'au moins une base organique ou inorganique, avec au moins un composé de formule générale R²⁰-C(=O)-X, R²⁰ ayant la signification selon une ou plusieurs des revendications 1 à 24 et X représentant un groupe partant,
ou dans un milieu réactionnel, éventuellement en présence d'au moins un réducteur, avec au moins un composé de formule générale R²⁰-C(=O)-H, R²⁰ ayant la signification selon une ou plusieurs des revendications 1 à 24,
en un composé correspondant de formule générale X, éventuellement sous la forme d'un sel correspondant, 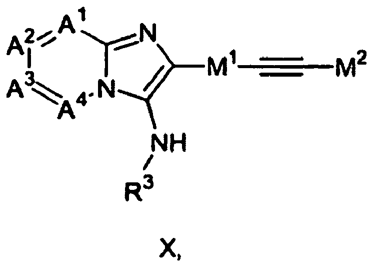 dans laquelle A¹, A², A³, A⁴, R³, M¹ et M² ont la signification selon une ou plusieurs des revendications 1 à 24, et celui-ci est éventuellement purifié et/ou isolé, et le composé de formule générale X est éventuellement transformé par mise en réaction avec au moins un composé de formule générale R²-X, R² ayant la signification selon une ou plusieurs des revendications 1 à 24 et X représentant un groupe partant, dans un milieu réactionnel, en présence d'au moins une base organique ou inorganique,
ou par mise en réaction avec au moins un composé de formule générale R²⁰-C(=O)-OH, R²⁰ ayant la signification selon une ou plusieurs des revendications 1 à 24, dans un milieu réactionnel, éventuellement en présence d'au moins une base organique ou inorganique et/ou en présence d'au moins un agent de couplage,
ou par mise en réaction avec au moins un composé de formule générale R²⁰-C(=O)-X, R²⁰ ayant la signification selon une ou plusieurs des revendications 1 à 24 et X représentant un groupe partant, dans un milieu réactionnel, éventuellement en présence d'au moins une base organique ou inorganique,
ou par mise en réaction avec au moins un composé de formule générale R²⁰-C(=O)-H, R²⁰ ayant la signification selon une ou plusieurs des revendications 1 à 24, dans un milieu réactionnel, éventuellement en présence d'au moins un réducteur,
en un composé de formule générale I, éventuellement sous la forme d'un sel correspondant, 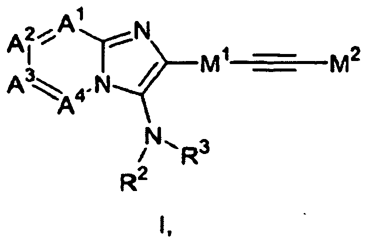 dans laquelle A¹, A², A³, A⁴, R², R³, M¹ et M² ont la signification selon une ou plusieurs des revendications 1 à 24, et celui-ci est éventuellement purifié et/ou isolé.

27. Médicament contenant au moins un composé selon une ou plusieurs des revendications 1 à 24 et éventuellement un ou plusieurs adjuvants physiologiquement compatibles.

28. Médicament selon la revendication 27 pour la prophylaxie et/ou le traitement de troubles et/ou de maladies médiés au moins en partie par les récepteurs mGluR5.

29. Médicament selon la revendication 27 ou 28, pour le traitement et/ou la prophylaxie de la douleur, de préférence de la douleur choisie dans le groupe constitué par la douleur aiguë, la douleur chronique, la douleur neuropathique et la douleur viscérale.

30. Médicament selon la revendication 27 ou 28 pour le traitement et/ou la prophylaxie de la migraine ; des dépressions ; des maladies neurodégénératives, de préférence choisies dans le groupe constitué par la maladie de Parkinson, la maladie d'Alzheimer, la maladie d'Huntington et la sclérose en plaques ; des maladies cognitives, de préférence des déficiences cognitives, de manière particulièrement préférée du trouble déficitaire de l'attention (TDA) ; des attaques de panique ; du syndrome d'anxiété ; de l'épilepsie ; de la toux ; de l'incontinence urinaire ; de la diarrhée ; du prurit ; de la schizophrénie ; des ischémies cérébrales ; des spasmes musculaires ; des crampes ; du syndrome du côlon irritable ; des troubles de l'alimentation, de préférence choisis dans le groupe constitué par la boulimie, la cachexie, l'anorexie et l'obésité ; de l'abus d'alcool et/ou de drogues (notamment la nicotine et/ou la cocaïne) et/ou de médicaments ; de la dépendance à l'alcool et/ou à des drogues (notamment la nicotine et/ou la cocaïne) et/ou à des médicaments ; de préférence pour la prophylaxie et/ou la réduction des phénomènes de sevrage en cas de dépendance à l'alcool et/ou à des drogues (notamment la nicotine et/ou la cocaïne) et/ou à des médicaments ; pour la prophylaxie et/ou la réduction des phénomènes de tolérance à des drogues et/ou des médicaments, notamment aux opioïdes ; du syndrome de reflux gastrooesophagien ; du reflux gastro-oesophagien ; pour la diurèse ; pour l'antinatriurèse ; pour la régulation du système cardiovasculaire ; pour l'anxiolyse ; pour l'augmentation de la vigilance ; pour l'augmentation de la libido ; pour la modulation de l'activité physique et pour l'anesthésie locale.

31. Utilisation d'au moins un composé selon une ou plusieurs des revendications 1 à 24 pour la fabrication d'un médicament pour la prophylaxie et/ou le traitement de troubles et/ou de maladies médiés au moins en partie par les récepteurs mGluR5.

32. Utilisation d'au moins un composé selon une ou plusieurs des revendications 1 à 24 pour la fabrication d'un médicament pour le traitement et/ou la prophylaxie de la douleur, de préférence de la douleur choisie dans le groupe constitué par la douleur aiguë, la douleur chronique, la douleur neuropathique et la douleur viscérale.

33. Utilisation d'au moins un composé selon une ou plusieurs des revendications 1 à 24 pour la fabrication d'un médicament pour le traitement et/ou la prophylaxie de la migraine ; des dépressions ; des maladies neurodégénératives, de préférence choisies dans le groupe constitué par la maladie de Parkinson, la maladie d'Alzheimer, la maladie d'Huntington et la sclérose en plaques ; des maladies cognitives, de préférence des déficiences cognitives, de manière particulièrement préférée du trouble déficitaire de l'attention (TDA) ; des attaques de panique ; du syndrome d'anxiété ; de l'épilepsie ; de la toux ; de l'incontinence urinaire ; de la diarrhée ; du prurit ; de la schizophrénie ; des ischémies cérébrales ; des spasmes musculaires ; des crampes ; du syndrome du côlon irritable ; des troubles de l'alimentation, de préférence choisis dans le groupe constitué par la boulimie, la cachexie, l'anorexie et l'obésité ; de l'abus d'alcool et/ou de drogues (notamment la nicotine et/ou la cocaïne) et/ou de médicaments, de la dépendance à l'alcool et/ou à des drogues (notamment la nicotine et/ou la cocaïne) et/ou à des médicaments, de préférence pour la prophylaxie et/ou la réduction des phénomènes de sevrage en cas de dépendance à l'alcool et/ou à des drogues (notamment la nicotine et/ou la cocaïne) et/ou à des médicaments ; du développement de phénomènes de tolérance à des drogues et/ou des médicaments, notamment aux opioïdes ; du syndrome de reflux gastro-oesophagien ; du reflux gastrooesophagien ; pour la diurèse ; pour l'antinatriurèse ; pour la régulation du système cardiovasculaire ; pour l'anxiolyse ; pour l'augmentation de la vigilance ; pour l'augmentation de la libido ; pour la modulation de l'activité physique et pour l'anesthésie locale.
